# EUROPEAN PATENT APPLICATION

(11) **EP 4 843 571 A2**
(43) Date of publication of application: **25.11.2026**
(21) Application number: 25741576.0
(22) Date of filing: 16.01.2025
(51) Int. Cl.: A61K 31/343, A61K 31/422, C07D 307/78, C07D 307/93, C07D 407/02, C07D 413/02, A61K 38/16, A61K 47/68, C07K 16/28, C07K 19/00, A61P 35/00

(54) **CONJUGATE OF PROTEIN TRANSLATION INHIBITOR AND USE THEREOF**

(30) Priority: 16.01.2024 CN 202410063661; 06.11.2024 CN 202411574559; 07.01.2025 CN 202510025596
(71) Applicant: Duality Biologics (Shanghai) Co., Ltd., Shanghai 201204 (CN)
(72) Inventor: LI, Bing, Suzhou, Jiangsu 215000 (CN); LIN, Shengchao, Suzhou, Jiangsu 215000 (CN); ZHANG, Yu, Suzhou, Jiangsu 215000 (CN); HUA, Haiqing, Suzhou, Jiangsu 215000 (CN); ZHU, Zhongyuan, Suzhou, Jiangsu 215000 (CN)
(74) Representative: J A Kemp LLP
(86) International application number: PCT/CN2025/072722
(87) International publication number: WO 2025/153015

(57) **Abstract**

The present disclosure relates to the field of pharmaceutical chemistry, and particularly relates to a conjugate of a protein translation inhibitor and use thereof in the manufacture of a medicament for treating and/or preventing diseases.

## Description

The present application is based on and claims priority to Chinese Patent Application No. 2202410063661.2 filed on January 16, 2024, Chinese Patent Application No. 202411574559.5 filed on November 6, 2024, and Chinese Patent Application No. 202510025596.9 filed on January 7, 2025, which are incorporated herein by reference in their entirety.

### TECHNICAL FIELD

The present disclosure pertains to the field of pharmaceutical chemistry, and particularly relates to a conjugate of a protein translation inhibitor and use thereof in the manufacture of a medicament for treating and/or preventing diseases.

### BACKGROUND

Abnormal messenger RNA (mRNA) translation is a common hallmark of malignancies and manifests itself as the up-regulation of oncoproteins, growth factors, and signal transduction proteins associated with proliferation, survival, and metastasis. The expression of oncogenic drivers is tightly controlled by translation, and is regulated by the eukaryotic translation initiation factor 4F (elF4F) complex. eIF4F mediates the recruitment of ribosomes to mRNA and initiates the translation of mRNA to protein. The eIF4F complex consists of three subunits: the mRNA 5' cap-binding protein eIF4E, the scaffold protein eIF4G, and the RNA helicase eIF4A. Hyperactivation of eIF4A is associated with a poor prognosis of related diseases, including lymphoma, lung cancer, colorectal cancer, liver cancer, breast cancer, and ovarian cancer. There are 3 eIF4A family members: eIF4A1, eIF4A2, and elF4A3, all of which belong to a helicase protein family called DEAD-box. The family proteins all have 9 conserved motifs that are involved in RNA binding or ATP hydrolysis.

There are reports that natural products can inhibit eIF4A-mediated translation and exhibit antiproliferative and anti-tumor phenotypes both *in vivo* and *in vitro.* For example, silvestrol and rocaglamide A have been shown to bind to and stabilize untranslated RNA/eIF4A complexes. These stable eIF4A·RocA complexes block scanning ribosomes, thereby inhibiting the translation of target mRNAs. Thus, the expression of related oncogenic factors is regulated through eIF4A-mediated translational inhibition.

The present disclosure provides ligand-drug conjugates prepared from eIF4A inhibitors with a novel structure, which are expected to meet significant clinical needs in tumor treatment.

### SUMMARY

### Ligand-Drug Conjugate

A first aspect of the present disclosure provides a ligand-drug conjugate or a racemic mixture, tautomer, stereoisomer, enantiomer, or diastereoisomer thereof, or a mixture thereof, or a pharmaceutically acceptable salt or solvate thereof, wherein the ligand-drug conjugate consists of a ligand, a linking group, and a drug group, and the drug group is selected from a structure represented by formula I-A, II-A', III-A', IV-A, or V-A: 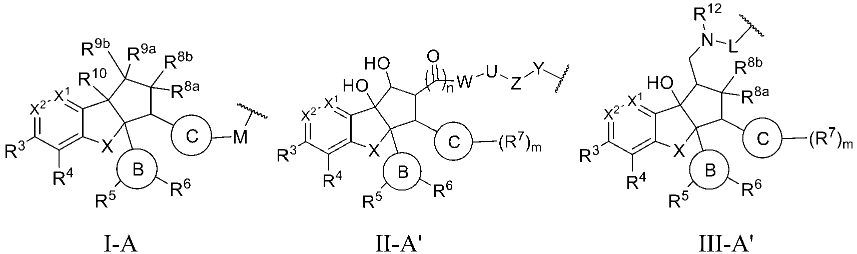 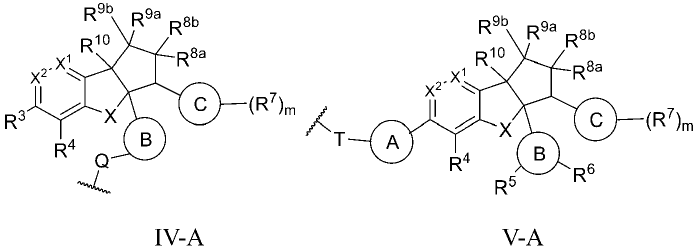 wherein:
M is selected from -N(R^{m})-, -O-, -(C₁-C₆)alkylene-O-, and -(C₁-C₆)alkylene-N(R^{m})-, wherein the -(C₁-C₆)alkylene-O- and -(C₁-C₆)alkylene-N(R^{m})- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and -N[(C₁-C₄)alkyl]₂;
n is 0 or 1;
W is absent or is selected from -(C₁-C₆)alkylene-, -O-, and -N(R^{w})-, wherein the -(C₁-C₆)alkylene-is optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and -N[(C₁-C₄)alkyl]₂;
U is selected from -(C₁-C₆)alkylene-, -(C₃-C₆)cycloalkylene-, and -(4-10 membered)heterocyclylene-, wherein the -(C₁-C₆)alkylene-, -(C₃-C₆)cycloalkylene-, and -(4-10 membered)heterocyclylene- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and - N[(C₁-C₄)alkyl]₂;
Z is absent or is selected from -(C₁-C₆)alkylene-, -(C₃-C₆)cycloalkylene-, and -(4-10 membered)heterocyclylene-;
Y is absent or is selected from -O-, -N(R^{y})-, and -N⁺(R^{y})₂-, wherein R^{y} is selected from hydrogen, -(C₁-C₆)alkyl, -O(C₁-C₆)alkyl, -(C₁-C₆)haloalkyl, and -O-(C₁-C₆)haloalkyl, or R^{y} and Z form -(4-10 membered)heterocyclylene-, wherein the -(4-10 membered)heterocyclylene- is optionally substituted with one or more (e.g., 1, 2, 3, 4, 5, 6, or 7) groups selected from hydrogen, halogen, - OH, -O-(C₁-C₄)alkyl, -NH₂, -NH(C₁-C₄)alkyl, and -N[(C₁-C₄)alkyl]₂;
R¹² is selected from hydrogen, -(C₁-C₆)alkyl, -O-(C₁-C₆)alkyl, -(C=O)-(C₁-C₆)alkyl, -(C₃-C₆)cycloalkyl, -O-(C₃-C₆)cycloalkyl, -(4-10 membered)heterocyclyl, and -O-(4-10 membered)heterocyclyl, wherein the -(C₁-C₆)alkyl, -O-(C₁-C₆)alkyl, -(C=O)-(C₁-C₆)alkyl, -(C₃-C₆)cycloalkyl, -O-(C₃-C₆)cycloalkyl, -(4-10 membered)heterocyclyl, and -O-(4-10 membered)heterocyclyl are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and - N[(C₁-C₄)alkyl]₂;
L is absent or is selected from -C(=O)-(C₁-C₆)alkylene-O-, -C(=O)-(C₁-C₆)alkylene-N(R^{L})-, - C(=O)-(C₁-C₆)alkylene-N⁺(R^{L})₂-, -C(=O)NH-(C₁-C₆)alkylene-O-, -C(=O)NH-(C₁-C₆)alkylene-N(R^{L})-, and -C(=O)NH-(C₁-C₆)alkylene-N⁺(R^{L})₂-, wherein R^{L} is selected from hydrogen, -(C₁-C₆)alkyl, -O-(C₁-C₆)alkyl, -(C₁-C₆)haloalkyl, and -O-(C₁-C₆)haloalkyl, wherein the -C(=O)-(C₁-C₆)alkylene-O-, -C(=O)-(C₁-C₆)alkylene-N(R^{L})-, -C(=O)-(C₁-C₆)alkylene-N⁺(R^{L})₂-, -C(=O)NH-(C₁-C₆)alkylene-O-, -C(=O)NH-(C₁-C₆)alkylene-N(R^{L})-, and -C(=O)NH-(C₁-C₆)alkylene-N⁺(R^{L})₂- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and -N[(C₁-C₄)alkyl]₂; Q is selected from -N(R^{q})-, -O-, -(C₁-C₆)alkylene-O-, and -(C₁-C₆)alkylene-N(R^{q})-, wherein the - (C₁-C₆)alkylene-O- and -(C₁-C₆)alkylene-N(R^{q})- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and -N[(C₁-C₄)alkyl]₂;
T is selected from -N(R^{t})-, -O-, -(C₁-C₆)alkylene-O-, and -(C₁-C₆)alkylene-N(R^{t})-, wherein the - (C₁-C₆)alkylene-O- and -(C₁-C₆)alkylene-N(R^{q})- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and -N[(C₁-C₄)alkyl]₂;
R^{m}, R^{w}, R^{q}, and R^{t} are each independently selected from hydrogen, -(C₁-C₆)alkyl, -O-(C₁-C₆)alkyl, -(C₁-C₆)haloalkyl, and -O-(C₁-C₆)haloalkyl;
X¹ is selected from N and C(R¹), wherein R¹ is selected from hydrogen, halogen, cyano, -OH, -
(C₁-C₆)alkyl, and -O-(C₁-C₆)alkyl;
X² is selected from N and C(R²), wherein R² is selected from hydrogen, halogen, cyano, -OH, -
(C₁-C₆)alkyl, and -O-(C₁-C₆)alkyl;
R³ is selected from -(C₃-C₆)cycloalkyl, -(3-8 membered)heterocyclyl, -(C₆-C₁₀)aryl, and -(5-12 membered)heteroaryl, wherein the -(C₃-C₆)cycloalkyl, -(3-8 membered)heterocyclyl, -(C₆-
C₁₀)aryl, and -(5-12 membered)heteroaryl are optionally substituted with one or more R³¹; ring A is selected from -(C₃-C₆)cycloalkylene-, -(3-8 membered)heterocyclylene-, -(C₆-C₁₀)arylene-, and -(5-12 membered)heteroarylene-, wherein the -(C₃-C₆)cycloalkylene-, -(3-8 membered)heterocyclylene-, -(C₆-C₁₀)arylene-, and -(5-12 membered)heteroarylene- are optionally substituted with one or more R³¹;
each R³¹ is independently selected from oxo, hydrogen, halogen, cyano, nitro, -OH, -SH, -NH₂, - (C₁-C₆)alkyl, -(C₁-C₆)alkylene-OH, -(C₁-C₆)alkylene-NH₂, - (C₁-C₆)alkylene-NH[(C₁-C₆)alkyl], - (C₁-C₆)alkylene-N[(C₁-C₆)alkyl]₂, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C ₃-C₆)cycloalkyl, -(4-10 membered)heterocyclyl, -O-(C₃-C₆)cycloalkyl, -0-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, - (C₆-C₁₀)aryl, -(5-12 membered)heteroaryl, -S(=O)₂-NH₂, -S(=O)₂-NH(C₁-C₆)alkyl, -S(=O)₂-N[(C₁-C₆)alkyl]₂, -NH-C(=O)H, -NH-C(=O)-(C₁-C₆)alkyl, -N[(C₁-C₆)alkyl]-C(=O)-(C₁-C₆)alkyl, N[(C₁-C₆)alkyl]-C(=O)H, -NH-S(=O)₂-(C₁-C₆)alkyl, -N[(C₁-C₆)alkyl]-S(=O)₂-(C₁-C₆)alkyl, - P(=O)[(C₁-C₆)alkyl]₂, -P(=O)[(C₁-C₆)alkyl]-NH₂, -P(=O)[(C₁-C₆)alkyl]-NH(C₁-C₆)alkyl, and - P(=O)[(C₁-C₆)alkyl]-N[(C₁-C₆)alkyl]₂, wherein the -(C₁-C₆)alkyl, -(C₁-C₆)alkylene-OH, -(C₁-C₆)alkylene-NH₂, -(C₁-C₆)alkylene-NH[(C₁-C₆)alkyl], -(C₁-C₆)alkylene-N[(C₁-C₆)alkyl]₂, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH(C₁-C₆)alkyl, - C(=O)-N[(C₁-C₆)alkyl]₂, -(C₆-C₁₀)aryl, -(5-12 membered)heteroaryl, -S(=O)₂-NH₂, -S(=O)₂-NH(C₁-C₆)alkyl, -S(=O)₂-N[(C₁-C₆)alkyl]₂, -NH-C(=O)H, -NH-C(=O)-(C₁-C₆)alkyl, -N[(C₁-C₆)alkyl]-C(=O)-(C₁-C₆)alkyl, N[(C₁-C₆)alkyl]-C(=O)H, -NH-S(=O)₂-(C₁-C₆)alkyl, -N[(C₁-C₆)alkyl]-S(=O)₂-(C₁-C₆)alkyl, -P(=O)[(C₁-C₆)alkyl]₂, -P(=O)[(C₁-C₆)alkyl]-NH₂, -P(=O)[(C₁-C₆)alkyl]-NH(C₁-C₆)alkyl, and -P(=O)[(C₁-C₆)alkyl]-N[(C₁-C₆)alkyl]₂ are optionally substituted with one or more groups selected from hydrogen, halogen, -OH, -O-(C₁-C₄)alkyl, -(C₁-C₆)alkylene-OH, -NH₂, -NH(C₁-C₄)alkyl, -N[(C₁-C₄)alkyl]₂, -(C₁-C₆)alkylene-NH₂, -(C₁-C₆)alkylene-NH[(C₁-C₆)alkyl], -(C₁-C₆)alkylene-N[(C₁-C₆)alkyl]₂, -(C₆-C₁₀)aryl, -(5-12 membered)heteroaryl, -(C₁-C₆)alkylene-O(C₁-C₆)alkyl, and oxo;
R⁴ is selected from hydrogen, halogen, cyano, -OH, -NH₂, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4-10 membered)heterocyclyl, -O-(4-10 membered)heterocyclyl, -(C₆-C₁₀)aryl, -O-(C₆-C₁₀)aryl, -(5-12 membered)heteroaryl, and -O-(5-12 membered)heteroaryl;
X is selected from O, S, NH, N[(C₁-C₆)alkyl], N[C(=O)-R^{c}], C(R^{a})(R^{b}), C(=O), C[=C(R^{a})R^{b}], S(=O), and S(=O)₂;
R^{a} and R^{b} are each independently selected from hydrogen, halogen, cyano, -OR^{c}, -SR^{c}, -N(R^{c})R^{c}, -(C₁-C₆)alkyl, and -(C₁-C₆)haloalkyl;
R^{c} is selected from hydrogen, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4-10 membered)heterocyclyl, -O-(4-10 membered)heterocyclyl, -(C₆-C₁₀)aryl, -O-(C₆-C₁₀)aryl, -(5-12 membered)heteroaryl, and -O-(5-12 membered)heteroaryl;
ring B is selected from -(C₆-C₁₀)aryl and -(5-12 membered)heteroaryl, or ring B is selected from - (C₆-C₁₀)arylene- and -(5-12 membered)heteroarylene-, wherein the -(C₆-C₁₀)arylene- and -(5-12 membered)heteroarylene- are optionally substituted with one or more groups selected from R⁵ and R⁶.
R⁵ and R⁶ are each independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, -C(=O)-H, and -C(=N-OH)-H, wherein the -(C₁-C₆)alkyl, -(C₂-C₆)alkenyl, -(C₂-C₆)alkynyl, -(C₁-C₆)haloalkyl, -O-(C₁-C₆)alkyl, - O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -
(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH(C₁-C₆)alkyl, and -C(=O)-N[(C₁-C₆)alkyl]₂ are optionally substituted with one or more groups selected from hydrogen, halogen, oxo, -OH, -O-(C₁-C₄)alkyl, -NH₂, -NH(C₁-C₄)alkyl, and N[(C₁-C₄)alkyl]₂; or
R⁵ and R⁶, together with the carbon atoms to which they are attached, form 5-membered heterocyclyl;
ring C is selected from -(C₆-C₁₀)aryl and -(5-12 membered)heteroaryl, or ring C is selected from - (C₆-C₁₀)arylene- and -(5-12 membered)heteroarylene-, wherein the -(C₆-C₁₀)arylene- and -(5-12 membered)heteroarylene- are optionally substituted with one or more R⁷;
each R⁷ is independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, and -NHC(=O)-(C₁-C₆)alkyl, wherein the -(C₁-C₆)alkyl, -(C₂-C₆)alkenyl, -(C₂-C₆)alkynyl, -(C₁-C₆)haloalkyl, -O-(C₁-C₆)alkyl, - O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, - (4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH(C₁-C₆)alkyl, -C(=O)-N[(C₁-C₆)alkyl]₂, and -NHC(=O)-(C₁-C₆)alkyl are optionally substituted with one or more groups selected from hydrogen, halogen, oxo, -OH, -O-(C₁-C₄)alkyl,
-NH₂, -NH(C₁-C₄)alkyl, and -N[(C₁-C₄)alkyl]₂;
m is 1, 2, 3, 4, or 5;
R^{8a}, R^{8b}, R^{9a}, and R^{9b} are each independently selected from hydrogen, halogen, cyano, -(C₁-C₆)alkyl, -(C₁-C₆)haloalkyl, -(C₂-C₆)alkenyl, -(C₂-C₆)alkynyl, -OR, -N(R)R, -[(C₁-C₈)alkylene]R, -[(C₁-C₈)alkylene]OR, -[(C₁-C₈)alkylene]N(R)R, -[(C₁-C₈)alkylene]N(R)C(=O)R, -[(C₁-C₈)alkylene]N(R)C(=O)N(R)R, -C(=O)R, -C(=O)N(R)R, -C(=O)[(C₁-C₈)alkylene]N(R)R, - C(=O)OR, -C(=S)N(R)R, -SR, -S(=O)R, -S(=O)₂R, -S(=O)₂N(R)R, -N(R)C(=O)R, - N(R)C(=O)N(R)R, -P(=O)(OR)(OR), -(C₆-C₁₀)aryl, -(5-12 membered)heteroaryl, -(C₃-C₆)cycloalkyl, and -(4-10 membered)heterocyclyl,
wherein each R is independently selected from hydrogen, -OH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -(C₁-C₄)alkylene-O-(C₁-C₆)alkyl, -O-(C₁-C₆)haloalkyl, -S-(C₁-C₆)alkyl, -S(=O)₂-(C₁-C₆)alkyl, -(C₃-C₆)cycloalkyl, -(4-10 membered)heterocyclyl, -O-(C₃-C₆)cycloalkyl, -O-(4-10 membered)heterocyclyl, -C(=O)-H, - C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, -[(C₁-C₆)alkylene]-(C₃-C₆)cycloalkyl,-[(C₁-C₆)alkylene]-(4-10 membered)heterocyclyl, -(C₆-C₁₀)aryl,-[(C₁-C₆)alkylene]-(C₆-C₁₀)aryl, and -(5-12 membered)heteroaryl, wherein the -(C₁-C₆)alkyl, -(C₂-C₆)alkenyl, -(C₂-C₆)alkynyl, -(C₁-C₆)haloalkyl, -O-(C₁-C₆)alkyl, -(C₁-C₄)alkylene-O-(C₁-C₆)alkyl, -O-(C₁-C₆)haloalkyl, -S-(C₁-C₆)alkyl, -S(=O)₂-(C₁-C₆)alkyl, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH(C₁-C₆)alkyl, -C(=O)-N[(C₁-C₆)alkyl]₂, -[(C₁-C₆)alkylene]-(C₃-C₆)cycloalkyl, -[(C₁-C₆)alkylene]-(4-10 membered)heterocyclyl, -(C₆-C₁₀)aryl, -[(C₁-C₆)alkylene]-(C₆-C₁₀)aryl, and - (5-12 membered)heteroaryl are optionally substituted with one or more groups selected from hydrogen, halogen, -OH, -O-(C₁-C₄)alkyl, -NH₂, -NH(C₁-C₄)alkyl, N[(C₁-C₄)alkyl]₂, and (C₁-C₄)alkyl;
or R^{8a} and R^{8b} are combined to form oxo, -(C₂-C₆)alkenyl, -(C₃-C₆)cycloalkyl, or -(4-10 membered)heterocyclyl;
or R^{9a} and R^{9b} are combined to form oxo, -(C₂-C₆)alkenyl, -(C₃-C₆)cycloalkyl, or -(4-10 membered)heterocyclyl;
or R^{8a} and R^{9a}, together with the carbon atoms to which they are attached, form (C₃-C₆)cycloalkyl, (4-10 membered)heterocyclyl, or (5-10 membered)heteroaryl, wherein the aforementioned group is optionally substituted with one or more groups selected from hydrogen, halogen, -OH, -O-(C₁-C₄)alkyl, -NH₂, -NH(C₁-C₄)alkyl, -N[(C₁-C₄)alkyl]₂, -(C₃-C₆)cycloalkyl, -(4-8 membered)heterocyclyl, -(5-6 membered)heteroaryl, and -(C₆-C₁₀)aryl;
or R^{8b} and R^{9b}, together with the carbon atoms to which they are attached, form (C₃-C₆)cycloalkyl, (4-10 membered)heterocyclyl, or (5-10 membered)heteroaryl, wherein the aforementioned group is optionally substituted with one or more groups selected from hydrogen, halogen, -OH, -O-(C₁-C₄)alkyl, -NH₂, -NH(C₁-C₄)alkyl, -N[(C₁-C₄)alkyl]₂, -(C₃-C₆)cycloalkyl, -(4-8 membered)heterocyclyl, -(5-6 membered)heteroaryl, and -(C₆-C₁₀)aryl;
R¹⁰ is selected from hydrogen, cyano, -OH, and -NH₂, or R¹⁰ and R^{9a}, together with the atoms to which they are attached, form (5-6 membered)heterocyclyl, wherein the (5-6 membered)heterocyclyl is optionally substituted with one or more groups selected from hydrogen, halogen, -OH, -(C₁-C₄)alkyl, -(C₃-C₆)cycloalkyl, -O-(C₁-C₄)alkyl, -NH₂, -NH(C₁-C₄)alkyl, and - N[(C₁-C₄)alkyl]₂.

The first aspect of the present disclosure further provides a ligand-drug conjugate or a racemic mixture, tautomer, stereoisomer, enantiomer, or diastereoisomer thereof, or a mixture thereof, or a pharmaceutically acceptable salt or solvate thereof, wherein the ligand-drug conjugate consists of a ligand, a linking group, and a drug group, and the drug group is selected from a structure represented by formula I-A, II-A, III-A, IV-A, or V-A: 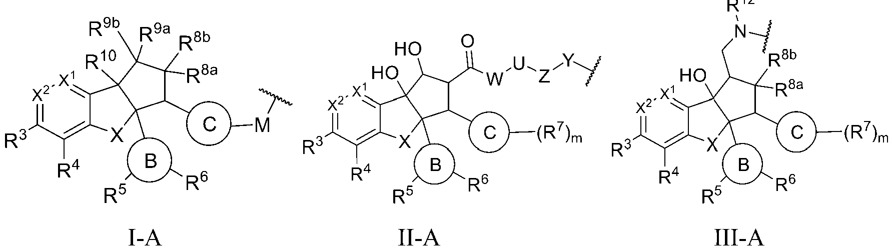 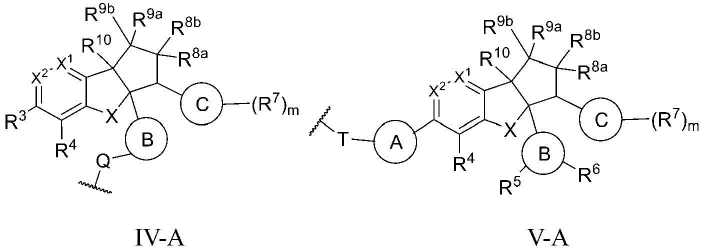 wherein:
M is selected from -N(R^{m})-, -O-, -(C₁-C₆)alkylene-O-, and -(C₁-C₆)alkylene-N(R^{m})-, wherein the -(C₁-C₆)alkylene-O- and -(C₁-C₆)alkylene-N(R^{m})- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and -N[(C₁-C₄)alkyl]₂;
W is absent or is selected from -(C₁-C₆)alkylene-, -O-, and -N(R^{w})-, wherein the -(C₁-C₆)alkylene-is optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and -N[(C₁-C₄)alkyl]₂;
U is selected from -(C₁-C₆)alkylene-, -(C₃-C₆)cycloalkylene-, and -(4-10 membered)heterocyclylene-, wherein the -(C₁-C₆)alkylene-, -(C₃-C₆)cycloalkylene-, and -(4-10 membered)heterocyclylene- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and - N[(C₁-C₄)alkyl]₂;
Z is absent or is selected from -(C₁-C₆)alkylene-, -(C₃-C₆)cycloalkylene-, and -(4-10 membered)heterocyclylene-;
Y is absent or is selected from -O- and -N(R^{y})-, wherein R^{y} is selected from hydrogen, -(C₁-C₆)alkyl, -O(C₁-C₆)alkyl, -(C₁-C₆)haloalkyl, and -O-(C₁-C₆)haloalkyl, or R^{y} and Z form -(4-10 membered)heterocyclylene-, wherein the -(4-10 membered)heterocyclylene- is optionally substituted with one or more (e.g., 1, 2, 3, 4, 5, 6, or 7) groups selected from hydrogen, halogen, - OH, -O-(C₁-C₄)alkyl, -NH₂, -NH(C₁-C₄)alkyl, and -N[(C₁-C₄)alkyl]₂;
R¹² is selected from hydrogen, -(C₁-C₆)alkyl, -O-(C₁-C₆)alkyl, -(C=O)-(C₁-C₆)alkyl, -(C₃-C₆)cycloalkyl, -O-(C₃-C₆)cycloalkyl, -(4-10 membered)heterocyclyl, and -O-(4-10 membered)heterocyclyl, wherein the -(C₁-C₆)alkyl, -O-(C₁-C₆)alkyl, -(C=O)-(C₁-C₆)alkyl, -(C₃-C₆)cycloalkyl, -O-(C₃-C₆)cycloalkyl, -(4-10 membered)heterocyclyl, and -O-(4-10 membered)heterocyclyl are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and - N[(C₁-C₄)alkyl]₂;
Q is selected from -N(R^{q})-, -O-, -(C₁-C₆)alkylene-O-, and -(C₁-C₆)alkylene-N(R^{q})-, wherein the - (C₁-C₆)alkylene-O- and -(C₁-C₆)alkylene-N(R^{q})- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and -N[(C₁-C₄)alkyl]₂;
T is selected from -N(R^{t})-, -O-, -(C₁-C₆)alkylene-O-, and -(C₁-C₆)alkylene-N(R^{t})-, wherein the - (C₁-C₆)alkylene-O- and -(C₁-C₆)alkylene-N(R^{q})- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and -N[(C₁-C₄)alkyl]₂;
R^{m}, R^{w}, R^{q}, and R^{t} are each independently selected from hydrogen, -(C₁-C₆)alkyl, -O-(C₁-C₆)alkyl, -(C₁-C₆)haloalkyl, and -O-(C₁-C₆)haloalkyl;
X¹ is selected from N and C(R¹), wherein R¹ is selected from hydrogen, halogen, cyano, -OH, -
(C₁-C₆)alkyl, and -O-(C₁-C₆)alkyl; X² is selected from N and C(R²), wherein R² is selected from hydrogen, halogen, cyano, -OH, -
(C₁-C₆)alkyl, and -O-(C₁-C₆)alkyl;
R³ is selected from -(C₃-C₆)cycloalkyl, -(3-8 membered)heterocyclyl, -(C₆-C₁₀)aryl, and -(5-12 membered)heteroaryl, and R³ is optionally substituted with one or more R³¹;
ring A is selected from -(C₃-C₆)cycloalkylene-, -(3-8 membered)heterocyclylene-, -(C₆-C₁₀)arylene-, and -(5-12 membered)heteroarylene-, and ring A is optionally substituted with one or more R³¹;
each R³¹ is independently selected from oxo, hydrogen, halogen, cyano, nitro, -OH, -SH, -NH₂, - (C₁-C₆)alkyl, -(C₁-C₆)alkylene-OH, -(C₁-C₆)alkylene-NH₂, -(C₁-C₆)alkylene-NH[(C₁-C₆)alkyl], - (C₁-C₆)alkylene-N[(C₁-C₆)alkyl]₂, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, and -C(=O)-N[(C₁-C₆)alkyl]₂, wherein the -(C₁-C₆)alkyl, -(C₁-C₆)alkylene-OH, -(C₁-C₆)alkylene-NH₂, -(C₁-C₆)alkylene-NH[(C₁-C₆)alkyl], -(C₁-C₆)alkylene-N[(C₁-C₆)alkyl]₂, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH(C₁-C₆)alkyl, and -C(=O)-N[(C₁-C₆)alkyl]₂ are optionally substituted with one or more groups selected from hydrogen, halogen, -OH, -O-(C₁-C₄)alkyl, -(C₁-C₆)alkylene-OH, -NH₂, -NH(C₁-C₄)alkyl, -N[(C₁-C₄)alkyl]₂, -(C₁-C₆)alkylene-NH₂, -(C₁-C₆)alkylene-NH[(C₁-C₆)alkyl], and -(C₁-C₆)alkylene-N[(C₁-C₆)alkyl]₂;
R⁴ is selected from hydrogen, halogen, cyano, -OH, -NH₂, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4-10 membered)heterocyclyl, -O-(4-10 membered)heterocyclyl, -(C₆-C₁₀)aryl, -O-(C₆-C₁₀)aryl, -(5-12 membered)heteroaryl, and -O-(5-12 membered)heteroaryl;
X is selected from O, S, NH, N[(C₁-C₆)alkyl], N[C(=O)-R°], C(R^{a})(R^{b}), C(=O), C[=C(R^{a})R^{b}], S(=O), and S(=O)₂;
R^{a} and R^{d} are each independently selected from hydrogen, halogen, cyano, -OR^{c}, -SR^{c}, -N(R^{c})R^{c}, -(C₁-C₆)alkyl, and -(C₁-C₆)haloalkyl;
R^{c} is selected from hydrogen, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4-10 membered)heterocyclyl, -O-(4-10 membered)heterocyclyl, -(C₆-C₁₀)aryl, -O-(C₆-C₁₀)aryl, -(5-12 membered)heteroaryl, and -O-(5-12 membered)heteroaryl;
ring B is selected from -(C₆-C₁₀)aryl and -(5-12 membered)heteroaryl, or ring B is selected from - (C₆-C₁₀)arylene- and -(5-12 membered)heteroarylene-, wherein the -(C₆-C₁₀)arylene- and -(5-12 membered)heteroarylene- are optionally substituted with one or more groups selected from R⁵ and R⁶.
R⁵ and R⁶ are each independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, -C(=O)-H, and -C(=N-OH)-H, wherein the -(C₁-C₆)alkyl, -(C₂-C₆)alkenyl, -(C₂-C₆)alkynyl, -(C₁-C₆)haloalkyl, -O-(C₁-C₆)alkyl, - O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, - (4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH(C₁-C₆)alkyl, and -C(=O)-N[(C₁-C₆)alkyl]₂ are optionally substituted with one or more groups selected from hydrogen, halogen, -OH, -O-(C₁-C₄)alkyl, -NH₂, -NH(C₁-C₄)alkyl, and N[(C₁-C₄)alkyl]₂; or
R⁵ and R⁶, together with the carbon atoms to which they are attached, form 5-membered heterocyclyl;
ring C is selected from -(C₆-C₁₀)aryl and -(5-12 membered)heteroaryl, or ring C is selected from - (C₆-C₁₀)arylene- and -(5-12 membered)heteroarylene-, wherein the -(C₆-C₁₀)arylene- and -(5-12 membered)heteroarylene- are optionally substituted with one or more R⁷;
each R⁷ is independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, and -C(=O)-N[(C₁-C₆)alkyl]₂, wherein the -(C₁-C₆)alkyl, - (C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH(C₁-C₆)alkyl, and -C(=O)-N[(C₁-C₆)alkyl]₂ are optionally substituted with one or more groups selected from hydrogen, halogen, -OH, -O-(C₁-C₄)alkyl, -NH₂, -NH(C₁-C₄)alkyl, and -N[(C₁-C₄)alkyl]₂;
m is 1, 2, 3, 4, or 5;
R^{8a}, R^{8b}, R^{9a}, and R^{9b} are each independently selected from hydrogen, halogen, cyano, -(C₁-C₆)alkyl, -(C₁-C₆)haloalkyl, -(C₂-C₆)alkenyl, -(C₂-C₆)alkynyl, -OR, -N(R)R, -[(C₁-C₈)alkylene]R, -[(C₁-C₈)alkylene]OR, -[(C₁-C₈)alkylene]N(R)R, -C(=O)R, -C(=O)N(R)R, -C(=O)[(C₁-C₈)alkylene]N(R)R, -C(=O)OR, -C(=S)N(R)R, -SR, -S(=O)R, -S(=O)₂R, -S(=O)₂N(R)R, - N(R)C(=O)R, -N(R)C(=O)N(R)R, -P(=O)(OR)(OR), -(C₆-C₁₀)aryl, -(5-12 membered)heteroaryl, -(C₃-C₆)cycloalkyl, and -(4-10 membered)heterocyclyl,
wherein each R is independently selected from hydrogen, -OH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -(C₃-C₆)cycloalkyl, -(4-10 membered)heterocyclyl, -O-(C₃-C₆)cycloalkyl, -O-(4-10 membered)heterocyclyl, -C(=O)-H, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, -[(C₁-C₆)alkylene]-(4-10 membered)heterocyclyl, -(C₆-C₁₀)aryl, -[(C₁-C₆)alkylene]-(C₆-C₁₀)aryl, and -(5-12 membered)heteroaryl, wherein the -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH(C₁-C₆)alkyl, -C(=O)-N[(C₁-C₆)alkyl]₂, -[(C₁-C₆)alkylene]-(4-10 membered)heterocyclyl, -(C₆-C₁₀)aryl, -[(C₁-C₆)alkylene]-(C₆-C₁₀)aryl, and - (5-12 membered)heteroaryl are optionally substituted with one or more groups selected from hydrogen, halogen, -OH, -O-(C₁-C₄)alkyl, -NH₂, -NH(C₁-C₄)alkyl, and N[(C₁-C₄)alkyl]₂; or R^{8a} and R^{8b}, and R^{9a} and R^{9b} are independently combined to form oxo, -(C₂-C₆)alkenyl, -(C₃-C₆)cycloalkyl, or -(4-10 membered)heterocyclyl;
or R^{8a} and R^{9a}, and R^{8b} and R^{9b}, together with the carbon atoms to which they are attached, form (C₃-C₆)cycloalkyl, (4-10 membered)heterocyclyl, or (5-10 membered)heteroaryl, wherein the aforementioned group is optionally substituted with one or more groups selected from hydrogen, halogen, -OH, -O-(C₁-C₄)alkyl, -NH₂, -NH(C₁-C₄)alkyl, -N[(C₁-C₄)alkyl]₂, -(C₃-C₆)cycloalkyl, - (4-8 membered)heterocyclyl, -(5-6 membered)heteroaryl, and -(C₆-C₁₀)aryl;
R¹⁰ is selected from hydrogen, cyano, -OH, and -NH₂, or R¹⁰ and R^{9a}, together with the atoms to which they are attached, form (5-6 membered)heterocyclyl, wherein the (5-6 membered)heterocyclyl is optionally substituted with one or more groups selected from hydrogen, halogen, -OH, -(C₁-C₄)alkyl, -(C₃-C₆)cycloalkyl, -O-(C₁-C₄)alkyl, -NH₂, -NH(C₁-C₄)alkyl, and - N[(C₁-C₄)alkyl]₂.

In certain embodiments, the drug group is selected from a structure represented by formula I-A: 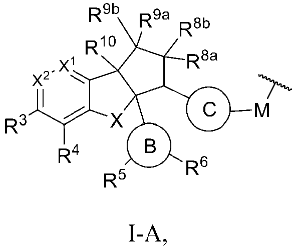 wherein X, X¹, X², ring B, ring C, R³, R⁴, R⁵, R⁶, R^{8a}, R^{8b}, R^{9a}, R^{9b}, R¹⁰, and M are defined as described in any one of the embodiments of the present disclosure.

In certain embodiments, R^{m} is selected from hydrogen, -(C₁-C₄)alkyl, -O-(C₁-C₄)alkyl, -(C₁-C₄)haloalkyl, and -O-(C₁-C₄)haloalkyl.

In certain embodiments,R^{m} is selected from hydrogen, methyl, ethyl, propyl, -O-methyl, -O-ethyl, and -O-propyl.

In certain embodiments,R^{m} is hydrogen.

In certain embodiments, M is selected from -NH-, -O-, -(C₁-C₆)alkylene-O-, and -(C₁-C₆)alkylene-NH-, wherein the -(C₁-C₆)alkylene-O- and -(C₁-C₆)alkylene-NH- are optionally substituted with 1, 2, or 3 groups selected from hydrogen, halogen, -OH, -O-methyl, -NH₂, -NH-methyl, - N(methyl)₂, and -N(ethyl)₂.

In certain embodiments, M is selected from -NH-, -O-, -(C₁-C₆)alkylene-O-, and -(C₁-C₆)alkylene-NH-.

In certain embodiments, M is selected from -NH-, -O-, -(C₁-C₄)alkylene-O-, and -(C₁-C₄)alkylene-NH-.

In certain embodiments, M is selected from -NH-, -O-, -CH₂O-, and -CH₂NH-.

In certain embodiments, M is selected from -NH-, -O-, *-CH₂O-, and *-CH₂NH-, wherein the * end is attached to ring C.

In certain embodiments, M is selected from -NH-.

In certain embodiments, ring C is selected from -(C₆-C₁₀)arylene- and -(5-10 membered)heteroarylene-, wherein the -(C₆-C₁₀)arylene- and -(5-10 membered)heteroarylene- are optionally substituted with 1, 2, or 3 R⁷.

In certain embodiments, ring C is selected from phenylene, naphthylene, and (5-6 membered)heteroarylene, wherein the phenylene, naphthylene, and (5-6 membered)heteroarylene are optionally substituted with 1 or 2 R⁷.

In certain embodiments, ring C is phenylene, wherein the phenylene is optionally substituted with 1 R⁷.

In certain embodiments, each R⁷ is independently selected from hydrogen, halogen, cyano, nitro, -OH, -SH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, and -O-(4-8 membered)heterocyclyl, wherein the -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, and -O-(4-8 membered)heterocyclyl are optionally substituted with 1, 2, 3, 4, or 5 groups selected from hydrogen, halogen, -OH, -O-(C₁-C₄)alkyl, -NH₂, -NH(C₁-C₄)alkyl, and -N[(C₁-C₄)alkyl]₂.

In certain embodiments, each R⁷ is independently selected from hydrogen, halogen, cyano, -OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, and -(C₁-C₄)alkylene-NH₂.

In certain embodiments, each R⁷ is independently selected from hydrogen, fluorine, chlorine, bromine, -OH, -NH₂, -CH₂OH, and -CH₂NH₂.

In certain embodiments, each R⁷ is independently selected from hydrogen.

In certain embodiments, ring C is 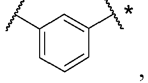 wherein the * end is attached to M.

In certain embodiments, the drug group is selected from a structure represented by formula II-A': 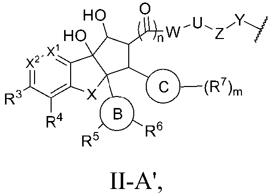 wherein X, X¹, X², ring B, ring C, R³, R⁴, R⁵, R⁶, R⁷, m, W, U, Z, and Y are defined as described in any one of the embodiments of the present disclosure, and n is 0 or 1.

In certain embodiments, the drug group is selected from a structure represented by formula II-A: 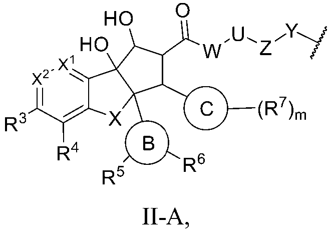 wherein X, X¹, X², ring B, ring C, R³, R⁴, R⁵, R⁶, R⁷, m, W, U, Z, and Y are defined as described in any one of the embodiments of the present disclosure.

In certain embodiments,R^{w} and R^{y} are each independently selected from hydrogen, -(C₁-C₄)alkyl, -O-(C₁-C₄)alkyl, -(C₁-C₄)haloalkyl, and -O-(C₁-C₄)haloalkyl.

In certain embodiments,R" and R^{y}are each independently selected from hydrogen, methyl, ethyl, propyl, -O-methyl, -O-ethyl, and -O-propyl.

In certain embodiments,R^{w} is selected from hydrogen and methyl.

In certain embodiments,R^{y} is hydrogen.

In certain embodiments, W is absent or is selected from -(C₁-C₆)alkylene-, -NH-, -N[(C₁-C₆)alkyl]-, and -N[O(C₁-C₆)alkyl]-.

In certain embodiments, W is absent or is selected from -(C₁-C₄)alkylene, -NH-, and -N[-(C₁-C₄)alkyl]-.

In certain embodiments, W is absent or is selected from methylene, ethylene, -NH-, -N(Me)-, and -N(Et)-.

In certain embodiments, W is absent or is selected from -NH- and -N(Me)-.

In certain embodiments, U is selected from -(C₁-C₆)alkylene-, -(C₃-C₆)cycloalkylene-, and -(4-10 membered)heterocyclylene-.

In certain embodiments, U is selected from -(C₁-C₄)alkylene-, -(C₃-C₆)cycloalkylene-, and -(4-6 membered)heterocyclylene-.

In certain embodiments, U is selected from methylene, ethylene, cyclopropylene, azetidinylene, and pyrrolidinylene.

In certain embodiments, U is selected from methylene, ethylene, and

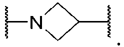

In certain embodiments, U is selected from methylene, ethylene, 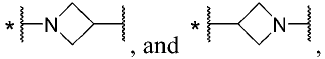 wherein the * end is attached to W, and when W is absent, the * end is attached to carbonyl. In certain embodiments, Z is absent or is selected from -(C₁-C₄)alkylene-, -(C₃-C₆)cycloalkylene-, and -(4-6 membered)heterocyclylene-.

In certain embodiments, Z is absent or is selected from methylene, ethylene, cyclopropylene, azetidinylene, and pyrrolidinylene.

In certain embodiments, Z is absent.

In certain embodiments, Y is absent or is selected from -O-, -N(R^{y})-, and -N⁺(R^{y})₂-, wherein R^{y} is selected from hydrogen, -(C₁-C₆)alkyl, and -O(C₁-C₆)alkyl, or R^{y} and Z form -(4-10 membered)heterocyclylene-.

In certain embodiments, Y is absent or is selected from -O-, -N(R^{y})-, and -N⁺(R^{y})₂-, wherein R^{y} is selected from hydrogen, -(C₁-C₄)alkyl, and -O(C₁-C₄)alkyl, or R^{y} and Z form -(4-6 membered)heterocyclylene-.

In certain embodiments, Y is absent or is selected from -O-, -N(R^{y})-, and -N⁺(R^{y})₂-, wherein R^{y} is selected from hydrogen, methyl, ethyl, and -O-methyl.

In certain embodiments, Y is selected from -O-, -NH-, -N(CH₃)-, and -N⁺(CH₃)₂-.

In certain embodiments, Y is absent or is selected from -O- and -N(R^{y})-, wherein R^{y} is selected from hydrogen, -(C₁-C₆)alkyl, and -O(C₁-C₆)alkyl, or R^{y} and Z form -(4-10 membered)heterocyclylene-.

In certain embodiments, Y is absent or is selected from -O- and -N(R^{y})-, wherein R^{y} is selected from hydrogen, -(C₁-C₄)alkyl, and -O(C₁-C₄)alkyl, or R^{y} and Z form -(4-6 membered)heterocyclylene-.

In certain embodiments, Y is absent or is selected from -O- and -N(R^{y})-, wherein R^{y} is selected from hydrogen, methyl, ethyl, and -O-methyl.

In certain embodiments, Y is selected from -O- and -NH-.

In certain embodiments, the structural unit -W-U-Z-Y- is selected from -N(R^{w})-(C₁-C₆)alkylene-N(R^{y})-, -N(R^{w})-(C₁-C₆)alkylene-N⁺(R^{y})₂-, -N(R^{w})-(C₁-C₆)alkylene-O-, -(C₁-C₆)alkylene-N(R^{y})-, - (C₁-C₆)alkylene-O-, -(C₃-C₆)cycloalkylene-N(R^{y})-, -(C₁-C₆)alkylene-N⁺(R^{y})₂-, -(C₃-C₆)cycloalkylene-O-, -(4-6 membered)heterocyclylene-O-, -(4-6 membered)heterocyclylene-N(R^{y})-, and -N(R^{w})-(4-6 membered)heterocyclylene-, wherein the -N(R^{w})-(C₁-C₆)alkylene-N(R^{y})-, -N(R^{w})-(C₁-C₆)alkylene-N⁺(R^{y})₂-, -N(R^{w})-(C₁-C₆)alkylene-O-, -(C₁-C₆)alkylene-N(R^{y})-, -(C₁-C₆)alkylene-N⁺(R^{y})₂-, -(C₁-C₆)alkylene-O-, -(C₃-C₆)cycloalkylene-N(R^{y})-, -(C₃-C₆)cycloalkylene-O-, -(4-6 membered)heterocyclylene-O-, -(4-6 membered)heterocyclylene-N(R^{y})-, and -N(R^{w})-(4-6 membered)heterocyclylene- are optionally substituted with one or more (e.g., 1, 2, 3, 4, 5, 6, or 7) groups selected from hydrogen, halogen, -OH, -O-(C₁-C₄)alkyl, -NH₂, - NH(C₁-C₄)alkyl, and -N[(C₁-C₄)alkyl]₂.

In certain embodiments, the structural unit -W-U-Z-Y- is selected from -N(R^{w})-(C₁-C₆)alkylene-N(R^{y})-, -N(R^{w})-(C₁-C₆)alkylene-N⁺(R^{y})₂-, -N(R^{w})-(C₁-C₆)alkylene-O-, -(C₁-C₆)alkylene-N(R^{y})-, - (C₁-C₆)alkylene-N⁺(R^{y})₂-, -(C₁-C₆)alkylene-O-, -(4-6 membered)heterocyclylene-O-, -(4-6 membered)heterocyclylene-N(R^{y})-, and -N(R^{w})-(4-6 membered)heterocyclylene-.

In certain embodiments, the structural unit -W-U-Z-Y- is selected from *-NH-CH₂-CH₂-NH-, *-N(CH₃)-CH₂-CH₂-NH-, *-NH-CH₂-CH₂-O-, *-CH₂-O-, *-CH₂-NH-, *-CH₂-N(CH₃)-, *-CH₂-N⁺(CH₃)₂-, 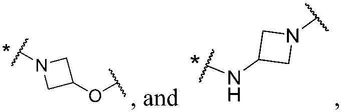 wherein the * end is attached to carbonyl.

In certain embodiments, the structural unit -W-U-Z-Y- is selected from -N(R^{w})-(C₁-C₆)alkylene-N(R^{y})-, -N(R^{w})-(C₁-C₆)alkylene-O-, -(C₁-C₆)alkylene-N(R^{y})-, -(C₁-C₆)alkylene-O-, -(C₃-C₆)cycloalkylene-N(R^{y})-, -(C₃-C₆)cycloalkylene-O-, -(4-6 membered)heterocyclylene-O-, -(4-6 membered)heterocyclylene-N(R^{y})-, and -N(R^{w})-(4-6 membered)heterocyclylene-, wherein the - N(R^{w})-(C₁-C₆)alkylene-N(R^{y})-, -N(R^{w})-(C₁-C₆)alkylene-O-, -(C₁-C₆)alkylene-N(R^{y})-, -(C₁-C₆)alkylene-O-, -(C₃-C₆)cycloalkylene-N(R^{y})-, -(C₃-C₆)cycloalkylene-O-, -(4-6 membered)heterocyclylene-O-, -(4-6 membered)heterocyclylene-N(R^{y})-, and -N(R^{w})-(4-6 membered)heterocyclylene- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and - N[(C₁-C₄)alkyl]₂.

In certain embodiments, the structural unit -W-U-Z-Y- is selected from -N(R^{w})-(C₁-C₆)alkylene-N(R^{y})-, -N(R^{w})-(C₁-C₆)alkylene-O-, -(C₁-C₆)alkylene-N(R^{y})-, -(C₁-C₆)alkylene-O-, -(4-6 membered)heterocyclylene-O-, -(4-6 membered)heterocyclylene-N(R^{y})-, and -N(R^{w})-(4-6 membered)heterocyclylene-.

In certain embodiments, the structural unit -W-U-Z-Y- is selected from *-NH-CH₂-CH₂-NH-, *-N(CH₃)-CH₂-CH₂-NH-, *-NH-CH₂-CH₂-O-, *-CH₂-O-, *-CH₂-NH-, 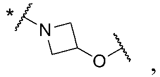 and 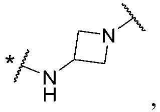 wherein the * end is attached to carbonyl.For example, the structural unit -W-U-Z-Y- is selected from *-NH-CH₂-CH₂-NH- and 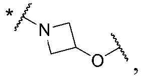 wherein the * end is attached to carbonyl.

In certain embodiments, the drug group is selected from a structure represented by formula III-A': 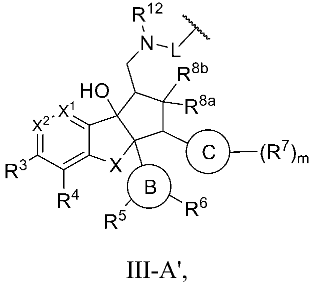 wherein X, X¹, X², ring B, ring C, L, R³, R⁴, R⁵, R⁶, R⁷, m, R^{8a}, R^{8b}, and R¹² are defined as described in any one of the embodiments of the present disclosure.

In certain embodiments, the drug group is selected from a structure represented by formula III-A: 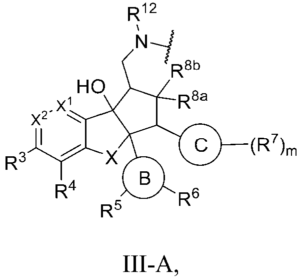 wherein X, X¹, X², ring B, ring C, R³, R⁴, R⁵, R⁶, R⁷, m, R^{8a}, R^{8b}, and R¹² are defined as described in any one of the embodiments of the present disclosure.

In certain embodiments, R¹² is selected from hydrogen, -(C₁-C₆)alkyl, -O-(C₁-C₆)alkyl, -(C=O)-(C₁-C₆)alkyl, -(C₃-C₆)cycloalkyl, -O-(C₃-C₆)cycloalkyl, -(4-10 membered)heterocyclyl, and -O-(4-10 membered)heterocyclyl.

In certain embodiments, R¹² is selected from hydrogen, -(C₁-C₄)alkyl, -O-(C₁-C₄)alkyl, -(C=O)-(C₁-C₄)alkyl, -(C₃-C₆)cycloalkyl, and -O-(C₃-C₆)cycloalkyl.

In certain embodiments, R¹² is selected from hydrogen, -(C₁-C₄)alkyl, -O-(C₁-C₄)alkyl, and -(C₃-C₆)cycloalkyl.

In certain embodiments, R¹² is selected from hydrogen and -(C₁-C₄)alkyl.

In certain embodiments, R¹² is selected from hydrogen, methyl, and ethyl.

In certain embodiments, R¹² is selected from hydrogen and methyl.

In certain embodiments, R¹² is selected from hydrogen.

In certain embodiments, L is absent or is selected from -C(=O)-(C₁-C₄)alkylene-O-, -C(=O)-(C₁-C₄)alkylene-N(R^{L})-, -C(=O)-(C₁-C₄)alkylene-N⁺(R^{L})₂-, -C(=O)NH-(C₁-C₄)alkylene-O-, - C(=O)NH-(C₁-C₄)alkylene-N(R^{L})-, and -C(=O)NH-(C₁-C₄)alkylene-N⁺(R^{L})₂-, wherein R^{L} is selected from hydrogen, -(C₁-C₄)alkyl, and -(C₁-C₄)haloalkyl.

In certain embodiments, L is absent or is selected from -C(=O)-(C₁-C₄)alkylene-O-, -C(=O)-(C₁-C₄)alkylene-N(R^{L})-, and -C(=O)-(C₁-C₄)alkylene-N⁺(R^{L})₂-, wherein R^{L} is selected from hydrogen, methyl, and ethyl.

In certain embodiments, L is absent or is selected from -C(=O)CH₂O-, -C(=O)CH₂CH₂O-, - C(=O)CH₂N(R^{L})-, -C(=O)CH₂CH₂N(R^{L})- -C(=O)CH₂N⁺(R^{L})₂-, and -C(=O)CH₂CH₂N⁺(R^{L})₂-, wherein R^{L} is selected from hydrogen, methyl, and ethyl.

In certain embodiments, L is absent or is selected from *-C(=O)CH₂O-, *-C(=O)CH₂N(CH₃)-, and *-C(=O)CH₂N⁺(CH₃)₂-, wherein the * end is attached to N.

In certain embodiments, L is absent.

In certain embodiments, the drug group is selected from a structure represented by formula IV-A: 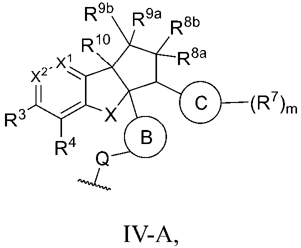 wherein X, X¹, X², ring B, ring C, R³, R⁴, Q, R⁷, m, R^{8a}, R^{8b}, R^{9a}, R^{9b}, and R¹⁰ are defined as described in any one of the embodiments of the present disclosure.

In certain embodiments,R^{q} is selected from hydrogen, -(C₁-C₄)alkyl, -O-(C₁-C₄)alkyl, -(C₁-C₄)haloalkyl, and -O-(C₁-C₄)haloalkyl.

In certain embodiments,R^{q} is selected from hydrogen, methyl, ethyl, propyl, -O-methyl, -O-ethyl, and -O-propyl.

In certain embodiments,R^{q} is hydrogen.

In certain embodiments, Q is selected from -NH-, -O-, -(C₁-C₆)alkylene-O-, and -(C₁-C₆)alkylene-NH-, wherein the -(C₁-C₆)alkylene-O- and -(C₁-C₆)alkylene-NH- are optionally substituted with 1, 2, or 3 groups selected from hydrogen, halogen, -OH, -O-methyl, -NH₂, -NH-methyl, - N(methyl)₂, and -N(ethyl)₂.

In certain embodiments, Q is selected from -NH-, -O-, -(C₁-C₆)alkylene-O-, and -(C₁-C₆)alkylene-NH-.

In certain embodiments,Q is selected from -NH-, -O-, -(C₁-C₄)alkylene-O-, and -(C₁-C₄)alkylene-NH-.

In certain embodiments,Q is selected from -NH-, -O-, -CH₂O-, and -CH₂NH-.

In certain embodiments,Q is selected from -NH-, -O-, *-CH₂O-, and *-CH₂NH-, wherein the * end is attached to ring B.

In certain embodiments, Q is selected from -NH-.

In certain embodiments, ring B is selected from -(C₆-C₁₀)arylene- and (5-10 membered)heteroarylene-, wherein the -(C₆-C₁₀)arylene- and (5-10 membered)heteroarylene- are optionally substituted with 1, 2, or 3 groups selected from R⁵ and R⁶.

In certain embodiments, ring B is selected from phenylene, naphthylene, and (5-6 membered)heteroarylene, wherein the phenylene, naphthylene, and (5-6 membered)heteroarylene are optionally substituted with 1 or 2 groups selected from R⁵ and R⁶.

In certain embodiments, ring B is phenylene, wherein the phenylene is optionally substituted with 1 R⁵ or R⁶.

In certain embodiments, R⁵ and R⁶ are each independently selected from hydrogen, halogen, cyano, nitro, -OH, -SH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, and -O-(4-8 membered)heterocyclyl, wherein the -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, and -O-(4-8 membered)heterocyclyl are optionally substituted with 1, 2, 3, 4, or 5 groups selected from hydrogen, halogen, -OH, -O-(C₁-C₄)alkyl, -NH₂, -NH(C₁-C₄)alkyl, and N[(C₁-C₄)alkyl]₂.

In certain embodiments, R⁵ and R⁶ are each independently selected from hydrogen, halogen, cyano, -OH, -NH₂, -(C₁-C₄)alkyl, -(C₁-C₄)alkyl-OH, -(C₁-C₄)alkyl-NH₂, -(C₁-C₄)haloalkyl, -O-(C₁-C₄)alkyl, -O-(C₁-C₄)haloalkyl, and -(C₃-C₆)cycloalkyl.

In certain embodiments, R⁵ is selected from hydrogen, fluorine, chlorine, bromine, cyano, -OH, - NH₂, and -CH₂NH₂.

In certain embodiments, R⁵ is selected from hydrogen, -OH, -NH₂, and -CH₂NH₂.

In certain embodiments, R⁵ is selected from hydrogen.

In certain embodiments, R⁶ is selected from hydrogen, fluorine, chlorine, bromine, cyano, -OH, - NH₂, -O-methyl, and cyclopropyl.

In certain embodiments, R⁶ is selected from hydrogen, fluorine, chlorine, bromine, cyano, -OH, - NH₂, and -O-methyl.

In certain embodiments, R⁶ is selected from hydrogen, cyano, and -O-methyl.

In certain embodiments, R⁶ is selected from cyano and -O-methyl.

In certain embodiments, R⁶ is cyano.

In certain embodiments, R⁶ is hydrogen.

In certain embodiments, ring B is 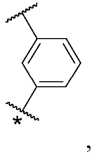 wherein the * end is attached to Q.

In certain embodiments, the drug group is selected from a structure represented by formula V-A: 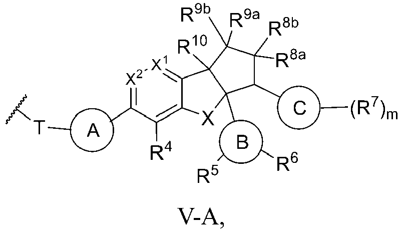 wherein X, X¹, X², T, ring A, ring B, ring C, R⁴, R⁵, R⁶, R⁷, m, R^{8a}, R^{8b}, R^{9a}, R^{9b}, and R¹⁰ are defined as described in any one of the embodiments of the present disclosure.

In certain embodiments,R^{t} is selected from hydrogen, -(C₁-C₄)alkyl, -O-(C₁-C₄)alkyl, -(C₁-C₄)haloalkyl, and -O-(C₁-C₄)haloalkyl.

In certain embodiments,R^{t} is selected from hydrogen, methyl, ethyl, propyl, -O-methyl, -O-ethyl, and -O-propyl.

In certain embodiments,R^{t} is hydrogen.

In certain embodiments, T is selected from -NH-, -O-, -(C₁-C₆)alkylene-O-, and -(C₁-C₆)alkylene-NH-, wherein the -(C₁-C₆)alkylene-O- and -(C₁-C₆)alkylene-NH- are optionally substituted with 1, 2, or 3 groups selected from hydrogen, halogen, -OH, -O-methyl, -NH₂, -NH-methyl, - N(methyl)₂, and -N(ethyl)₂.

In certain embodiments, T is selected from -NH-, -O-, -(C₁-C₆)alkylene-O-, and -(C₁-C₆)alkylene-NH-.

In certain embodiments, T is selected from -NH-, -O-, -(C₁-C₄)alkylene-O-, and -(C₁-C₄)alkylene-NH-.

In certain embodiments, T is selected from -NH-, -O-, -CH₂O-, and -CH₂NH-.

In certain embodiments, T is selected from -NH-, -O-, *-CH₂O-, and *-CH₂NH-, wherein the * end is attached to ring A.

In certain embodiments, T is selected from -NH-, *-CH₂O-, and *-CH₂NH-, wherein the * end is attached to ring A.

In certain embodiments, T is selected from -NH- and *-CH₂NH-, wherein the * end is attached to ring A.

In certain embodiments, T is selected from -NH-.

In certain embodiments,ring A is selected from -(C₃-C₆)cycloalkylene-, -(4-6 membered)heterocyclylene-, -(C₆-C₁₀)arylene-, and -(5-12 membered)heteroarylene-, wherein the -(C₃-C₆)cycloalkylene-, -(4-6 membered)heterocyclylene-, -(C₆-C₁₀)arylene-, and -(5-12 membered)heteroarylene are optionally substituted with one or more R³¹.

In certain embodiments, theR³¹ is selected from oxo, hydrogen, halogen, cyano, nitro, -OH, -SH, -NH₂, -(C₁-C₄)alkyl, -(C₁-C₄)alkylene-OH, -(C₁-C₄)alkylene-NH₂, -(C₁-C₄)alkylene-NH(C₁-C₄)alkyl, -(C₁-C₄)alkylene-N[(C₁-C₄)alkyl]₂, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, -O-(4-8 membered)heterocyclyl, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, -C(=O)-NH₂, -C(=O)-NH(C₁-C₄)alkyl, and -C(=O)-N[(C₁-C₄)alkyl]₂.

In certain embodiments, R³¹ is selected from oxo, hydrogen, halogen, cyano, -OH, -SH, -NH₂, - (C₁-C₄)alkyl, -(C₁-C₄)haloalkyl, -O-(C₁-C₄)alkyl, -NH-(C₁-C₄)alkyl, -(C₁-C₄)alkylene-OH, -(C₁-C₄)alkylene-NH₂, -(C₁-C₄)alkylene-NH(C₁-C₄)alkyl, and -(C₁-C₄)alkylene-N[(C₁-C₄)alkyl]₂. In certain embodiments, R³¹ is selected from oxo, hydrogen, fluorine, chlorine, cyano, -OH, -SH, -NH₂, methyl, halomethyl, -O-methyl, -CH₂OH, -CH₂NH₂, and -CH₂CH₂N(CH₃)₂.

In certain embodiments, R³¹ is selected from oxo, hydrogen, fluorine, cyano, -NH₂, -CH₂OH, - CH₂NH₂, methyl, trifluoromethyl, and -O-methyl.

In certain embodiments, R³¹ is selected from hydrogen, -NH₂, -CH₂OH, and -CH₂NH₂.

In certain embodiments, R³¹ is hydrogen.

In certain embodiments, ring A is selected from phenylene, naphthylene, and -(5-10 membered)heteroarylene-, wherein the phenylene, naphthylene, and -(5-10 membered)heteroarylene are optionally substituted with one or more R³¹, wherein R³¹ is defined as described in any one of the embodiments of the present disclosure.

In certain embodiments, ring A is selected from -(5-9 membered)heteroarylene-, wherein the -(5-9 membered)heteroarylene- is optionally substituted with 1, 2, 3, or 4 R³¹, wherein R³¹ is defined as described in any one of the embodiments of the present disclosure.

In certain embodiments, ring A is selected from -(5-6 membered)heteroarylene-, wherein the -(5-6 membered)heteroarylene- is optionally substituted with 1, 2, 3, or 4 R³¹, wherein R³¹ is defined as described in any one of the embodiments of the present disclosure.

In certain embodiments, ring A is selected from oxazolylene, isoxazolylene, thiazolylene, isothiazolylene, pyrazolylene, imidazolylene, triazolylene, tetrazolylene, pyridinylene, pyrazinylene, pyridazinylene, pyrimidinylene, pyridazinoimidazolylene, pyrazinoimidazolylene, and pyrimidinonylene; for example, ring A is selected from oxazolylene, imidazolylene, or pyrimidinylene; for example, ring A is selected from oxazolylene; ring A is optionally substituted with 1, 2, or 3 R³¹, wherein R³¹ is defined as described in any one of the embodiments of the present disclosure.

In certain embodiments, ring A is selected from 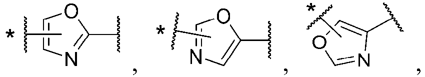 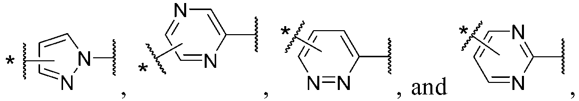 wherein the * end is attached to T.For example, ring A is

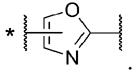

In certain embodiments, ring A is selected from 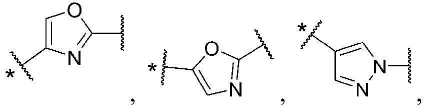 and 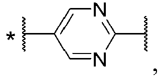 wherein the * end is attached to T.

In certain embodiments, ring A is selected from 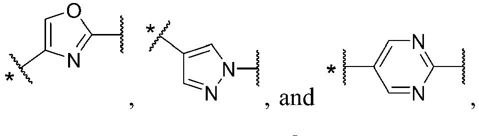 wherein the * end is attached to T. For example, ring A is selected from

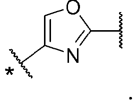

In certain embodiments,R¹ is selected from hydrogen, halogen, cyano, -OH, -(C₁-C₄)alkyl, and - O-(C₁-C₄)alkyl.

In certain embodiments, R¹ is selected from hydrogen, fluorine, chlorine, cyano, -OH, methyl, ethyl, propyl, -O-methyl, -O-ethyl, and -O-propyl.

In certain embodiments, R¹ is selected from hydrogen and -O-methyl.

In certain embodiments, R¹ is -O-methyl.

In certain embodiments, X¹ is selected from N, CH, and C(OCH₃).

In certain embodiments, X¹ is selected from N and C(OCH₃).

In certain embodiments, X¹ is selected from C(OCH₃).

In certain embodiments, X¹ is selected from N.

In certain embodiments,R² is selected from hydrogen, halogen, cyano, -OH, -(C₁-C₄)alkyl, and - O-(C₁-C₄)alkyl.

In certain embodiments, R² is selected from hydrogen, fluorine, chlorine, cyano, -OH, methyl, ethyl, propyl, -O-methyl, -O-ethyl, and -O-propyl.

In certain embodiments, R² is hydrogen.

In certain embodiments, X² is selected from N and CH.

In certain embodiments, X² is C(R²).

In certain embodiments, X² is selected from CH.

In certain embodiments,R³ is selected from -(C₃-C₆)cycloalkyl, -(4-6 membered)heterocyclyl, - (C₆-C₁₀)aryl, and -(5-12 membered)heteroaryl, wherein the -(C₃-C₆)cycloalkyl, -(4-6 membered)heterocyclyl, -(C₆-C₁₀)aryl, and -(5-12 membered)heteroaryl are optionally substituted with one or more R³¹.

In certain embodiments, theR³¹ is selected from oxo, hydrogen, halogen, cyano, nitro, -OH, -SH, -NH₂, -(C₁-C₄)alkyl, -(C₁-C₄)alkylene-OH, -(C₁-C₄)alkylene-NH₂, -(C₁-C₄)alkylene-NH(C₁-C₄)alkyl, -(C₁-C₄)alkylene-N[(C₁-C₄)alkyl]₂, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, -O-(4-8 membered)heterocyclyl, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, -C(=O)-NH₂, -C(=O)-NH(C₁-C₄)alkyl, and -C(=O)-N[(C₁-C₄)alkyl]₂.

In certain embodiments, R³¹ is selected from oxo, hydrogen, halogen, cyano, -OH, -SH, -NH₂, - (C₁-C₄)alkyl, -(C₁-C₄)haloalkyl, -O-(C₁-C₄)alkyl, -NH-(C₁-C₄)alkyl, -(C₁-C₄)alkylene-OH, -(C₁-C₄)alkylene-NH₂, -(C₁-C₄)alkylene-NH(C₁-C₄)alkyl, and -(C₁-C₄)alkylene-N[(C₁-C₄)alkyl]₂.

In certain embodiments, R³¹ is selected from oxo, hydrogen, fluorine, chlorine, cyano, -OH, -SH, -NH₂, methyl, halomethyl, -O-methyl, -CH₂OH, -CH₂NH₂, and -CH₂CH₂N(CH₃)₂.

In certain embodiments, R³¹ is selected from oxo, hydrogen, fluorine, cyano, -NH₂, -CH₂OH, - CH₂NH₂, methyl, trifluoromethyl, and -O-methyl.

In certain embodiments, R³¹ is selected from hydrogen, -NH₂, -CH₂OH, and -CH₂NH₂.

In certain embodiments, R³¹ is selected from hydrogen.

In certain embodiments, R³ is selected from -(C₃-C₆)cycloalkyl and -(4-6 membered)heterocyclyl, wherein the -(C₃-C₆)cycloalkyl and -(4-6 membered)heterocyclyl are optionally substituted with one or more R³¹, wherein R³¹ is defined as described in any one of the embodiments of the present disclosure.

In certain embodiments, R³ is selected from cyclopropyl, oxetanyl, tetrahydrofuranyl, azetidinyl, pyrrolidinyl, morpholinyl, morpholinonyl, piperidinyl, piperazinyl, and piperazinonyl, wherein the cyclopropyl, oxetanyl, tetrahydrofuranyl, azetidinyl, pyrrolidinyl, morpholinyl, morpholinonyl, piperidinyl, piperazinyl, and piperazinonyl are optionally substituted with 1, 2, or 3 R³¹, wherein R³¹ is defined as described in any one of the embodiments of the present disclosure.

In certain embodiments, R³ is selected from

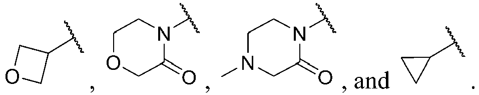

In certain embodiments, R³ is selected from phenyl, naphthyl, and -(5-10 membered)heteroaryl, wherein the phenyl, naphthyl, and -(5-10 membered)heteroaryl are optionally substituted with one or more R³¹, wherein R³¹ is defined as described in any one of the embodiments of the present disclosure.

In certain embodiments, R³ is selected from -(5-9 membered)heteroaryl, wherein the -(5-9 membered)heteroaryl is optionally substituted with 1, 2, 3, or 4 R³¹, wherein R³¹ is defined as described in any one of the embodiments of the present disclosure.

In certain embodiments, R³ is selected from oxazolyl, isoxazolyl, thiazolyl, isothiazolyl, pyrazolyl, imidazolyl, triazolyl, tetrazolyl, pyridinyl, pyrazinyl, pyridazinyl, pyrimidinyl, pyridazinoimidazolyl, pyrazinoimidazolyl, and pyrimidinonyl; for example, R³ is selected from oxazolyl, imidazolyl, or pyrimidinyl; for example, R³ is selected from oxazolyl; R³ is optionally substituted with 1, 2, or 3 R³¹, wherein R³¹ is defined as described in any one of the embodiments of the present disclosure. 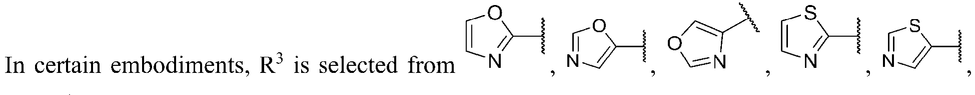 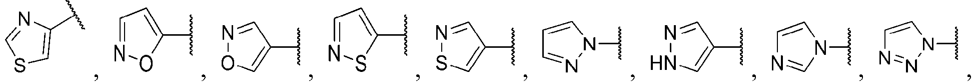 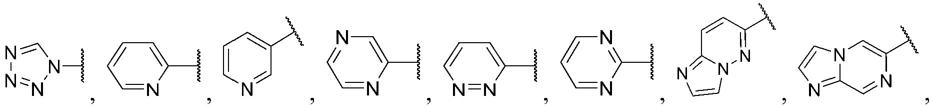 and 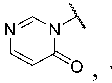 wherein the 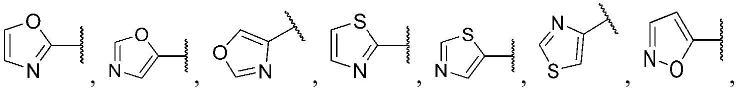 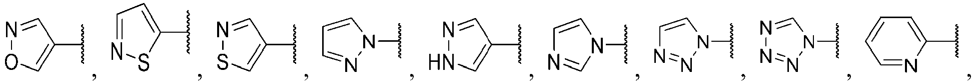 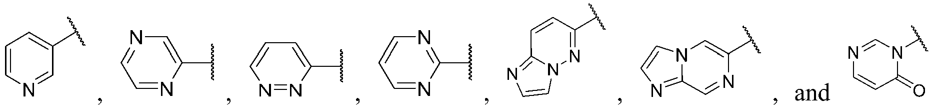 are optionally substituted with 1, 2, or 3 R³¹, wherein R³¹ is defined as described in any one of the embodiments of the present disclosure.

In certain embodiments, R³ is selected from 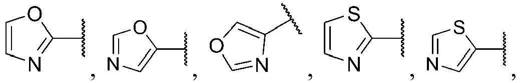 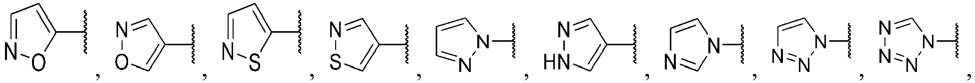 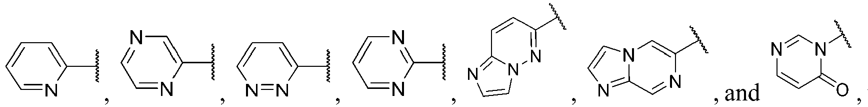 wherein R³ is optionally substituted with 1, 2, or 3 R³¹.

In certain embodiments, R³ is selected from 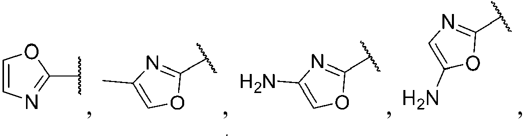 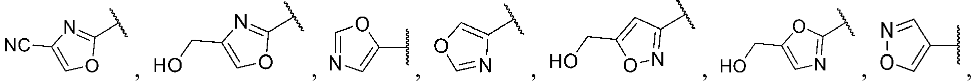 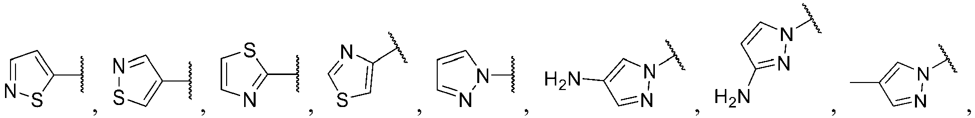 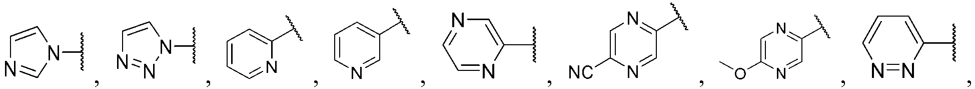 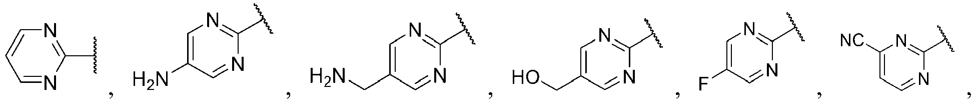 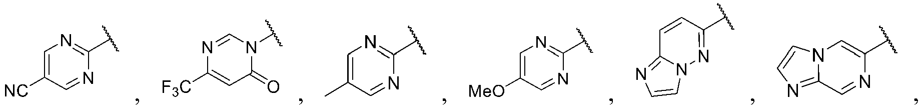 and

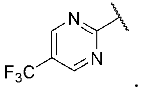

In certain embodiments, R³ is selected from

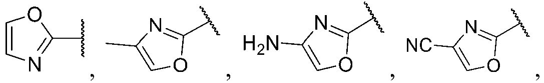

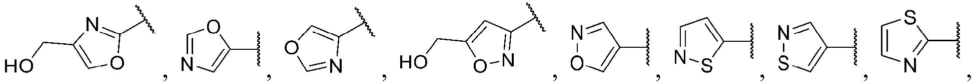

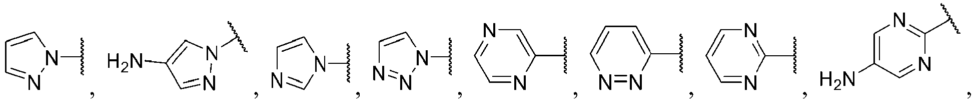

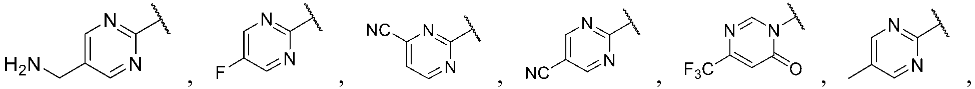

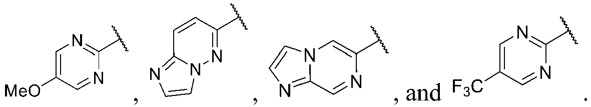

In certain embodiments, R³ is selected from

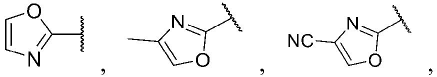

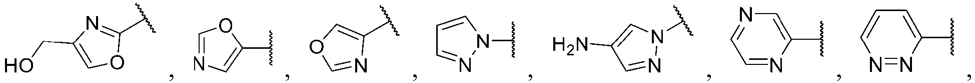

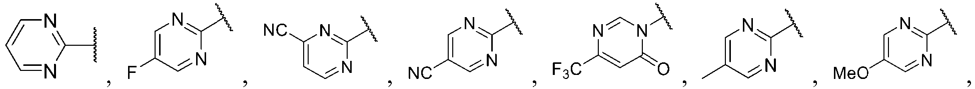

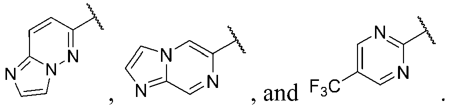

In certain embodiments, R³ is selected from 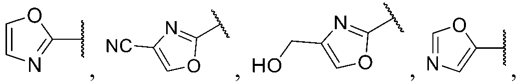 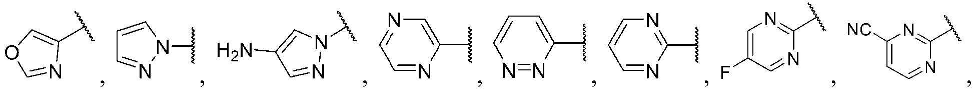 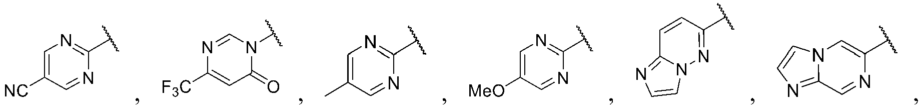 and

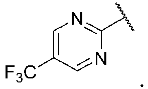

In certain embodiments, R³ is selected from 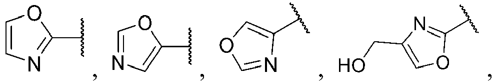 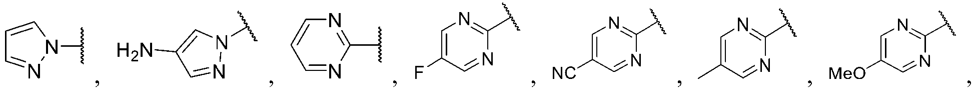 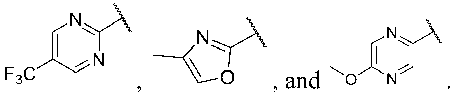 In certain embodiments, R³ is selected from

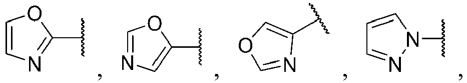

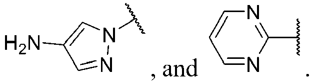

In certain embodiments, R³ is selected from

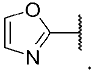

In certain embodiments, R⁴ is selected from hydrogen, halogen, cyano, -OH, -NH₂, -(C₁-C₄)alkyl, -(C₁-C₄)haloalkyl, -O-(C₁-C₄)alkyl, and -O-(C₁-C₄)haloalkyl.

In certain embodiments, R⁴ is selected from hydrogen, fluorine, chlorine, cyano, -OH, -NH₂, and methyl.

In certain embodiments, R⁴ is hydrogen.

In certain embodiments, X is selected from O, S, NH, N[(C₁-C₄)alkyl], N[C(=O)-R°], C(R^{a})(R^{b}), C(=O), C[=C(R^{a})R^{b}], S(=O), and S(=O)₂, wherein R^{a} and R^{b} are each independently selected from hydrogen, halogen, cyano, -OR^{c}, -SR^{c}, -N(R^{c})R^{c}, -(C₁-C₄)alkyl, and -(C₁-C₄)haloalkyl, wherein R^{c} is selected from hydrogen, -(C₁-C₄)alkyl, -(C₁-C₄)haloalkyl, -O-(C₁-C₄)alkyl, -O-(C₁-C₄)haloalkyl, and -(C₃-C₆)cycloalkyl.

In certain embodiments, R^{c} is selected from hydrogen, methyl, ethyl, halomethyl, -O-methyl, and cyclopropyl.

In certain embodiments, R^{a} and R^{b} are each independently selected from hydrogen, fluorine, chlorine, cyano, -OH, -O-methyl, -SH, -S-methyl, -NH₂, -N(methyl)₂, -N(methyl)-ethyl, - N(ethyl)₂, methyl, ethyl, propyl, halomethyl, haloethyl, and halopropyl.

In certain embodiments, X is selected from O, S, NH, and N[(C₁-C₄)alkyl].

In certain embodiments, X is selected from O, S, and NH.

In certain embodiments, X is selected from O and S.

In certain embodiments, X is selected from O.

In certain embodiments, ring B is selected from -(C₆-C₁₀)aryl and (5-10 membered)heteroaryl.

In certain embodiments, ring B is selected from phenyl, naphthyl, and (5-6 membered)heteroaryl.

In certain embodiments, the structural unit

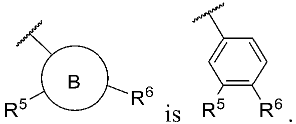

In certain embodiments, R⁵ and R⁶ are each independently selected from hydrogen, halogen, cyano, nitro, -OH, -SH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, and -O-(4-8 membered)heterocyclyl, wherein the -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, and -O-(4-8 membered)heterocyclyl are optionally substituted with 1, 2, 3, 4, or 5 groups selected from hydrogen, halogen, -OH, -O-(C₁-C₄)alkyl, -NH₂, -NH(C₁-C₄)alkyl, and N[(C₁-C₄)alkyl]₂.

In certain embodiments, R⁵ and R⁶ are each independently selected from hydrogen, halogen, cyano, -OH, -NH₂, -(C₁-C₄)alkyl, -(C₁-C₄)alkyl-OH, -(C₁-C₄)alkyl-NH₂, -(C₁-C₄)haloalkyl, -O-(C₁-C₄)alkyl, -O-(C₁-C₄)haloalkyl, and -(C₃-C₆)cycloalkyl.

In certain embodiments, R⁵ is selected from hydrogen, fluorine, chlorine, bromine, cyano, -OH, - NH₂, and -CH₂NH₂.

In certain embodiments, R⁵ is selected from hydrogen, -OH, -NH₂, and -CH₂NH₂.

In certain embodiments, R⁵ is selected from hydrogen.

In certain embodiments, R⁶ is selected from hydrogen, fluorine, chlorine, bromine, cyano, -OH, - NH₂, -O-methyl, and cyclopropyl.

In certain embodiments, R⁶ is selected from hydrogen, fluorine, chlorine, bromine, cyano, -OH, - NH₂, and -O-methyl.

In certain embodiments, R⁶ is selected from hydrogen, cyano, and -O-methyl.

In certain embodiments, R⁶ is selected from cyano and -O-methyl.

In certain embodiments, R⁶ is cyano.

In certain embodiments, R⁵ and R⁶, together with the atoms to which they are attached, form 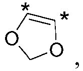 wherein * indicates the position of the attached atom.

In certain embodiments, ring C is selected from -(C₆-C₁₀)aryl and (5-10 membered)heteroaryl.

In certain embodiments, ring C is selected from phenyl, naphthyl, and (5-6 membered)heteroaryl.

In certain embodiments, the structural unit 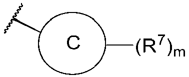 is

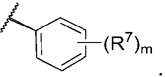

In certain embodiments, the structural unit 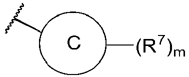 is 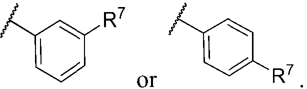 In certain embodiments, each R⁷ is independently selected from hydrogen, halogen, cyano, nitro, -OH, -SH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, -O-(4-8 membered)heterocyclyl, and -NHC(=O)-(C₁-C₄)alkyl, wherein the -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, - NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, -O-(4-8 membered)heterocyclyl, and -NHC(=O)-(C₁-C₄)alkyl are optionally substituted with 1, 2, 3, 4, or 5 groups selected from hydrogen, halogen, -OH, -O-(C₁-C₄)alkyl, - NH₂, -NH(C₁-C₄)alkyl, and -N[(C₁-C₄)alkyl]₂.

In certain embodiments, each R⁷ is independently selected from hydrogen, halogen, cyano, nitro, -OH, -SH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, and -O-(4-8 membered)heterocyclyl, wherein the -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, and -O-(4-8 membered)heterocyclyl are optionally substituted with 1, 2, 3, 4, or 5 groups selected from hydrogen, halogen, -OH, -O-(C₁-C₄)alkyl, -NH₂, -NH(C₁-C₄)alkyl, and -N[(C₁-C₄)alkyl]₂.

In certain embodiments, each R⁷ is independently selected from hydrogen, halogen, cyano, -OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, -(C₁-C₄)alkylene-NH₂, and -NHC(=O)-(C₁-C₄)alkylene-NH₂.

In certain embodiments, each R⁷ is independently selected from hydrogen, halogen, cyano, -OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, and -(C₁-C₄)alkylene-NH₂.

In certain embodiments, each R⁷ is independently selected from hydrogen, fluorine, chlorine, bromine, -OH, -NH₂, -CH₂OH, -CH₂NH₂, -NHC(=O)CH₂NH₂, and -NHC(=O)CH₂CH₂NH₂.

In certain embodiments, each R⁷ is independently selected from hydrogen, fluorine, chlorine, bromine, -OH, -NH₂, -CH₂OH, and -CH₂NH₂.

In certain embodiments, each R⁷ is independently selected from hydrogen.

In certain embodiments, m is 1, 2, or 3.

In certain embodiments, m is 1.

In certain embodiments, R^{8a}, R^{8b}, R^{9a}, and R^{9b} are each independently selected from hydrogen, halogen, cyano, -(C₁-C₄)alkyl, -(C₁-C₄)haloalkyl, -(C₂-C₄)alkenyl, -(C₂-C₄)alkynyl, -OR, -N(R)R, -[(C₁-C₆)alkylene]R, -[(C₁-C₆)alkylene]OR, -[(C₁-C₆)alkylene]N(R)R, -[(C₁-C₆)alkylene]N(R)C(=O)R, -[(C₁-C₆)alkylene]N(R)C(=O)N(R)R, -C(=O)R, -C(=O)N(R)R, - C(=O)[(C₁-C₆)alkylene]N(R)R, -C(=O)OR, -C(=S)N(R)R, -SR, -S(=O)R, -S(=O)₂R, - S(=O)₂N(R)R, -N(R)C(=O)R, -N(R)C(=O)N(R)R, and -P(=O)(OR)(OR), wherein each R is independently selected from hydrogen, -OH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -(C₁-C₄)alkylene-O-(C₁-C₄)alkyl, -S-(C₁-C₄)alkyl, -S(=O)₂ -(C₁-C₄)alkyl, -(C₃-C₆)cycloalkyl, -(4-8 membered)heterocyclyl, -O-(C₃-C₆)cycloalkyl, -O-(4-8 membered)heterocyclyl, -C(=O)-H, - C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, -C(=O)-NH₂, -C(=O)-NH(C₁-C₄)alkyl, -[(C₁-C₄)alkylene]-(C₃-C₆)cycloalkyl, - C(=O)-N[(C₁-C₄)alkyl]₂, -[(C₁-C₄)alkylene]-(4-8 membered)heterocyclyl, -(C₆-C₁₀)aryl, -[(C₁-C₄)alkylene]-(C₆-C₁₀)aryl, and -(5-10 membered)heteroaryl; the -(C₁-C₄)alkyl, -(C₂-C₄)alkenyl, - (C₂-C₄)alkynyl, -(C₁-C₄)haloalkyl, -O-(C₁-C₄)alkyl, -O-(C₁-C₄)haloalkyl, -(C₁-C₄)alkylene-O-(C₁-C₄)alkyl, -S-(C₁-C₄)alkyl, -S(=O)₂-(C₁-C₄)alkyl, -(C₃-C₆)cycloalkyl, -(4-8 membered)heterocyclyl, -O-(C₃-C₆)cycloalkyl, -O-(4-8 membered)heterocyclyl, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, - C(=O)-NH(C₁-C₄)alkyl, -[(C₁-C₄)alkylene]-(C₃-C₆)cycloalkyl, -C(=O)-N[(C₁-C₄)alkyl]₂, -[(C₁-C₄)alkylene]-(4-8 membered)heterocyclyl, -(C₆-C₁₀)aryl, -[(C₁-C₄)alkylene]-(C₆-C₁₀)aryl, and - (5-10 membered)heteroaryl are optionally substituted with 1, 2, 3, 4, 5, 6, or 7 groups selected from hydrogen, halogen, -OH, -O-(C₁-C₄)alkyl, -NH₂, -NH(C₁-C₄)alkyl, N[(C₁-C₄)alkyl]₂, and (C₁-C₄)alkyl.

In certain embodiments, R^{8a}, R^{8b}, R^{9a}, and R^{9b} are each independently selected from hydrogen, halogen, cyano, -(C₁-C₄)alkyl, -(C₁-C₄)haloalkyl, -(C₂-C₄)alkenyl, -(C₂-C₄)alkynyl, -OR, -N(R)R, -[(C₁-C₆)alkylene]R, -[(C₁-C₆)alkylene]OR, -[(C₁-C₆)alkylene]N(R)R, -C(=O)R, -C(=O)N(R)R, - C(=O)[(C₁-C₆)alkylene]N(R)R, -C(=O)OR, -C(=S)N(R)R, -SR, -S(=O)R, -S(=O)₂R, - S(=O)₂N(R)R, -N(R)C(=O)R, -N(R)C(=O)N(R)R, and -P(=O)(OR)(OR); each R is independently selected from hydrogen, -OH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -(C₃-C₆)cycloalkyl, -(4-8 membered)heterocyclyl, -O-(C₃-C₆)cycloalkyl, -O-(4-8 membered)heterocyclyl, -C(=O)-H, - C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, -C(=O)-NH₂, -C(=O)-NH(C₁-C₄)alkyl, -C(=O)-N[(C₁-C₄)alkyl]₂, -[(C₁-C₄)alkylene]-(4-8 membered)heterocyclyl, -(C₆-C₁₀)aryl, -[(C₁-C₄)alkylene]-(C₆-C₁₀)aryl, and - (5-10 membered)heteroaryl, wherein the -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -(C₃-C₆)cycloalkyl, -(4-8 membered)heterocyclyl, -O-(C₃-C₆)cycloalkyl, -O-(4-8 membered)heterocyclyl, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, - C(=O)-NH(C₁-C₄)alkyl, -C(=O)-N[(C₁-C₄)alkyl]₂, -[(C₁-C₄)alkylene]-(4-8 membered)heterocyclyl, -(C₆-C₁₀)aryl, -[(C₁-C₄)alkylene]-(C₆-C₁₀)aryl, and -(5-10 membered)heteroaryl are optionally substituted with 1, 2, 3, 4, 5, 6, or 7 groups selected from hydrogen, halogen, -OH, -O-(C₁-C₄)alkyl, -NH₂, -NH(C₁-C₄)alkyl, and N[(C₁-C₄)alkyl]₂.

In certain embodiments, each R is independently selected from hydrogen, -OH, -NH₂, -(C₁-C₄)alkyl, -(C₁-C₄)haloalkyl, -O-(C₁-C₄)alkyl, -(C₁-C₄)alkylene-O-(C₁-C₆)alkyl, -O-(C₁-C₄)haloalkyl, -S(=O)₂-(C₁-C₄)alkyl, -(C₃-C₆)cycloalkyl, -(4-8 membered)heterocyclyl, -C(=O)-H, -C(=O)-(C₁-C₄)alkyl, -C(=O)-(C₃-C₆)cycloalkyl, -[(C₁-C₄)alkylene]-(C₃-C₆)cycloalkyl, and -[(C₁-C₄)alkylene]-(4-8 membered)heterocyclyl, wherein the -(C₁-C₄)alkyl, -(C₁-C₄)haloalkyl, -O-(C₁-C₄)alkyl, -(C₁-C₄)alkylene-O-(C₁-C₆)alkyl, -O-(C₁-C₄)haloalkyl, -S(=O)₂-(C₁-C₄)alkyl, -(C₃-C₆)cycloalkyl, -(4-8 membered)heterocyclyl, -C(=O)-(C₁-C₄)alkyl, -C(=O)-(C₃-C₆)cycloalkyl, - [(C₁-C₄)alkylene]-(C₃-C₆)cycloalkyl, and -[(C₁-C₄)alkylene]-(4-8 membered)heterocyclyl are optionally substituted with 1, 2, 3, 4, or 5 groups selected from hydrogen, halogen, -OH, -O-(C₁-C₄)alkyl, -NH₂, -NH(C₁-C₄)alkyl, N[(C₁-C₄)alkyl]₂, methyl, and ethyl.

In certain embodiments, each R is independently selected from hydrogen, -OH, -NH₂, -(C₁-C₄)alkyl, -(C₁-C₄)haloalkyl, -O-(C₁-C₄)alkyl, -O-(C₁-C₄)haloalkyl, -(C₃-C₆)cycloalkyl, -(4-8 membered)heterocyclyl, -C(=O)-H, -C(=O)-(C₁-C₄)alkyl, -C(=O)-(C₃-C₆)cycloalkyl, and -[(C₁-C₄)alkylene]-(4-8 membered)heterocyclyl, wherein the -(C₁-C₄)alkyl, -(C₁-C₄)haloalkyl, -O-(C₁-C₄)alkyl, -O-(C₁-C₄)haloalkyl, -(C₃-C₆)cycloalkyl, -(4-8 membered)heterocyclyl, -C(=O)-(C₁-C₄)alkyl, -C(=O)-(C₃-C₆)cycloalkyl, and -[(C₁-C₄)alkylene]-(4-8 membered)heterocyclyl are optionally substituted with 1, 2, 3, 4, or 5 groups selected from hydrogen, halogen, -OH, -O-(C₁-C₄)alkyl, -NH₂, -NH(C₁-C₄)alkyl, and N[(C₁-C₄)alkyl]₂.

In certain embodiments, each R is independently selected from hydrogen, -OH, -NH₂, -CH₃, - CH₂CH₃, -CH₂OH, -CH₂CH₂OH, -CH₂OCH₃, -CH₂NH₂, -CH₂CH₂NH₂, -CH₂CH₂NHCH₃, - CH₂N(CH₃)₂, -CH(N(CH₃)₂)CH₂OH, -CH₂OCH₂CH₂OH, -OCH₃, -C(=O)-H, -C(=O)CH₃, - C(=O)CH₂OH, -S(=O)₂CH₃, cyclobutyl-OH, oxetanyl, azetidinyl, azetidinyl-OH, pyrrolidinyl, piperazinyl, azetidinyl-CH₃, piperazinyl-CH₃, and -CH₂-azetidinyl.

In certain embodiments, each R is independently selected from hydrogen, -OH, -NH₂, -CH₃, - CH₂OH, -CH₂CH₂OH, -CH₂NH₂, -CH₂CH₂NH₂, -OCH₃, -C(=O)-H, -C(=O)CH₂OH, oxetanyl, azetidinyl, and azetidinyl-OH.

In certain embodiments, each R is independently selected from hydrogen, -OH, -NH₂, -(C₁-C₄)alkyl, -O-(C₁-C₄)alkyl, and -(4-8 membered)heterocyclyl, wherein the -(C₁-C₄)alkyl, -O-(C₁-C₄)alkyl, and -(4-8 membered)heterocyclyl are optionally substituted with 1, 2, 3, 4, or 5 groups selected from hydrogen, halogen, -OH, -NH₂, -NH(C₁-C₄)alkyl, -N[(C₁-C₄)alkyl]₂, and -O-(C₁-C₄)alkyl.

In certain embodiments, each R is independently selected from hydrogen, -OH, -(C₁-C₄)alkyl, -O-(C₁-C₄)alkyl, and -(4-8 membered)heterocyclyl, wherein the -(C₁-C₄)alkyl, -O-(C₁-C₄)alkyl, and - (4-8 membered)heterocyclyl are optionally substituted with 1, 2, or 3 groups selected from hydrogen, halogen, -OH, -NH₂, -NH-methyl, and -N(methyl)₂.

In certain embodiments, each R is independently selected from methyl, ethyl, propyl, -O-methyl, -O-ethyl, -O-propyl, oxetanyl, and azetidinyl, wherein the aforementioned groups are each independently and optionally substituted with 1, 2, or 3 groups selected from -OH and -NH₂.

In certain embodiments, R^{8a}, R^{8b}, R^{9a}, and R^{9b} are each independently selected from hydrogen, cyano, -OR, -N(R)R, -[(C₁-C₄)alkylene]R, -[(C₁-C₄)alkylene]OR, -[(C₁-C₄)alkylene]N(R)R, - [(C₁-C₄)alkylene]N(R)C(=O)R, -[(C₁-C₄)alkylene]N(R)C(=O)N(R)R, -C(=O)R, -C(=O)N(R)R, - C(=O)[(C₁-C₄)alkylene]N(R)R, -C(=O)OR, -C(=S)N(R)R, -S(=O)R, -S(=O)₂R, -S(=O)₂N(R)R, - N(R)C(=O)R, and -N(R)C(=O)N(R)R, wherein R is defined as described in any one of the embodiments of the present disclosure.

In certain embodiments, R^{8a}, R^{8b}, R^{9a}, and R^{9b} are each independently selected from hydrogen, - OR, -N(R)R, -[(C₁-C₄)alkylene]R, -[(C₁-C₄)alkylene]OR, -[(C₁-C₄)alkylene]N(R)R, -C(=O)R, - C(=O)N(R)R, -C(=O)[(C₁-C₄)alkylene]N(R)R, -C(=O)OR, -C(=S)N(R)R, -S(=O)R, -S(=O)₂R, - S(=O)₂N(R)R, -N(R)C(=O)R, and -N(R)C(=O)N(R)R, wherein R is defined as described in any one of the embodiments of the present disclosure.

In certain embodiments, R^{8a} and R^{8b} are each independently selected from hydrogen, -methylene-R, -ethylene-R, -propylene-R, -methylene-OR, -ethylene-OR, -propylene-OR, -methylene-N(R)R, -ethylene-N(R)R, -propylene-N(R)R, -C(=O)R, -C(=O)N(R)R, and -C(=O)OR, wherein R is defined as described in any one of the embodiments of the present disclosure.

In certain embodiments, R^{8a} and R^{8b} are each independently selected from hydrogen, -methylene-R, -methylene-OR, -methylene-N(R)R, -C(=O)R, -C(=O)OR, and -C(=O)N(R)R, wherein R is defined as described in any one of the embodiments of the present disclosure; for example, each R is independently selected from hydrogen, -OH, -NH₂, -(C₁-C₄)alkyl, -O-(C₁-C₄)alkyl, -C(=O)-(C₁-C₄)alkyl, -S(=O)₂(C₁-C₄)alkyl, and -(4-8 membered)heterocyclyl, wherein the -(C₁-C₄)alkyl and -(4-8 membered)heterocyclyl are each optionally substituted with 1, 2, 3, 4, or 5 groups selected from hydrogen, halogen, -OH, -NH₂, -NHCH₃, and CH₃.

In certain embodiments, R^{8a} and R^{8b} are each independently selected from hydrogen, -methylene-R, -methylene-OR, -methylene-N(R)R, -C(=O)R, -C(=O)OR, and -C(=O)N(R)R, wherein R is defined as described in any one of the embodiments of the present disclosure.

In certain embodiments, R^{8a} is hydrogen.

In certain embodiments, R^{8a} is

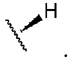

In certain embodiments, R^{8b} is selected from hydrogen,

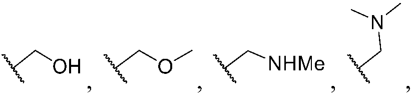

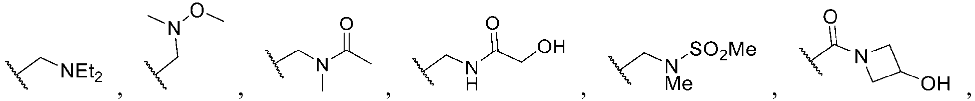

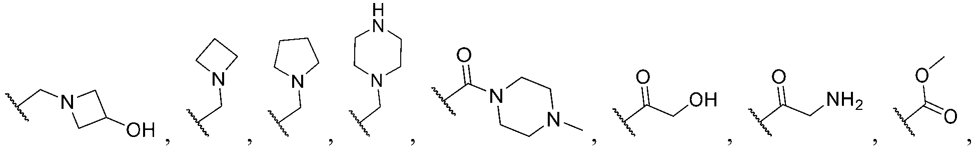

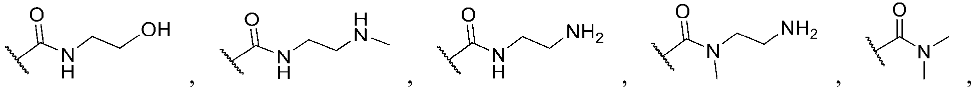

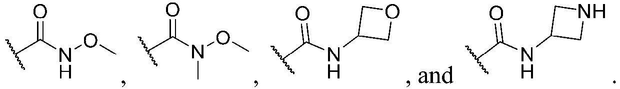

In certain embodiments, R^{8b} is selected from hydrogen, 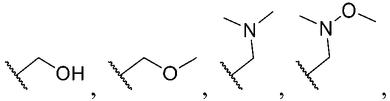 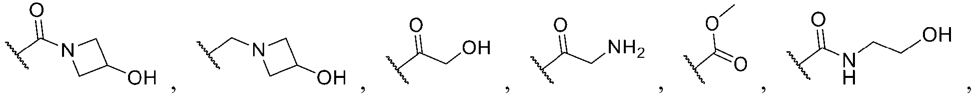 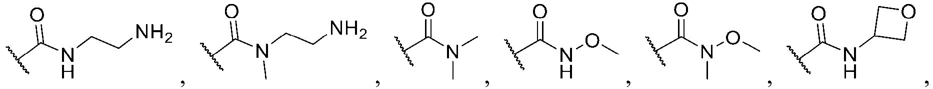 and

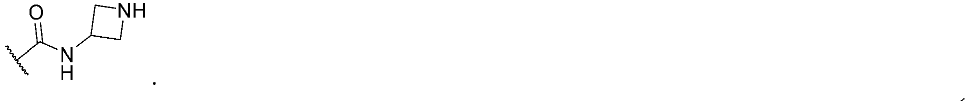

In certain embodiments, R^{8b} is selected from hydrogen,

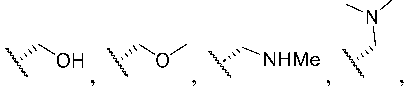

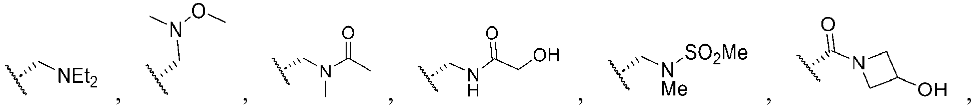

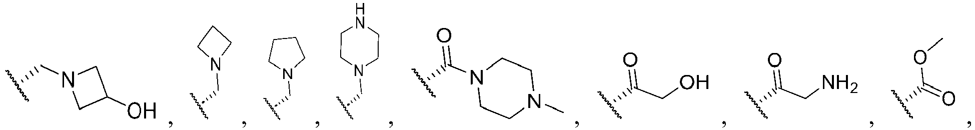

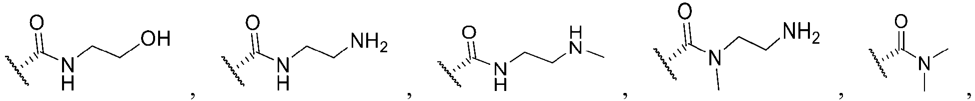

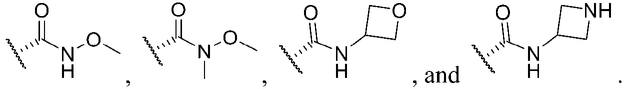

In certain embodiments, R^{8b} is selected from hydrogen, 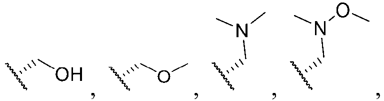 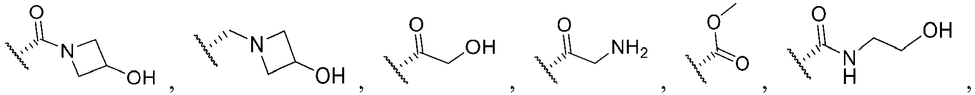 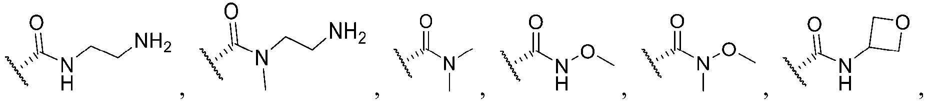 and

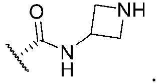

In certain embodiments, each R^{8b} is independently selected from hydrogen, -methylene-R, - methylene-OR, -methylene-N(R)R, and -C(=O)N(R)R, wherein each R is independently selected from hydrogen, -(C₁-C₄)alkyl, and -O-(C₁-C₄)alkyl.

In certain embodiments, R^{8b} is selected from hydrogen, 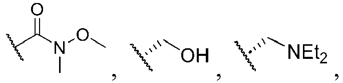 and

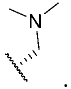

In certain embodiments, R^{8b} is selected from hydrogen,

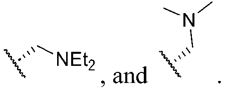

In certain embodiments, R^{8b} is selected from hydrogen,

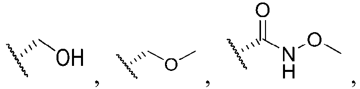

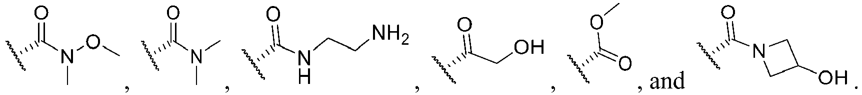

In certain embodiments, R^{8b} is selected from hydrogen,

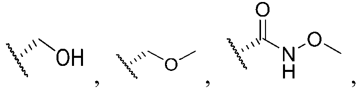

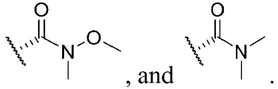

In certain embodiments, R^{9a} and R^{9b} are each independently selected from hydrogen, -CN, -OR, - NHR, -N(R)R, -NHC(=O)R, -methylene-R, -ethylene-R, -propylene-R, -methylene-N(R)R, - ethylene-N(R)R, -propylene-N(R)R, -methylene-NHC(=O)R, and -methylene-NHC(=O)NHR, wherein R is defined as described in any one of the embodiments of the present disclosure.

In certain embodiments, R^{9a} and R^{9b} are each independently selected from hydrogen, -OR, -NHR, -methylene-R, -ethylene-R, -propylene-R, -methylene-N(R)R, -ethylene-N(R)R, -propylene-N(R)R, and -NHC(=O)R, wherein R is defined as described in any one of the embodiments of the present disclosure.

In certain embodiments, R^{9a} and R^{9b} are each independently selected from hydrogen, -CN, -OR, - N(R)R, -NHC(=O)R, -methylene-N(R)R, -methylene-NHC(=O)R, and -methylene-NHC(=O)NHR, wherein R is defined as described in any one of the embodiments of the present disclosure; for example, each R is independently selected from hydrogen, -OH, -(C₁-C₄)alkyl, - (C₁-C₄)alkylene-O-(C₁-C₄)alkyl, -S(=O)₂(C₁-C₄)alkyl, -(C₃-C₆)cycloalkyl, -(4-8 membered)heterocyclyl, -methylene-(C₃-C₆)cycloalkyl, and -methylene-(4-8 membered)heterocyclyl, wherein the -(C₁-C₄)alkyl, -(C₃-C₆)cycloalkyl, and -(4-8 membered)heterocyclyl are each optionally substituted with 1, 2, 3, 4, or 5 groups selected from hydrogen, halogen, -OH, -NH₂, -NH(C₁-C₄)alkyl, -N[(C₁-C₄)alkyl]₂, -O-(C₁-C₄)alkyl, and -(C₁-C₄)alkyl.

In certain embodiments, R^{9a} and R^{9b} are each independently selected from hydrogen, -OR, -N(R)R, -methylene-N(R)R, and -NHC(=O)R, wherein R is defined as described in any one of the embodiments of the present disclosure.

In certain embodiments, R^{9a} is selected from hydrogen, -CN, -N(R)R, -NHC(=O)R, -methylene-N(R)R, -methylene-NHC(=O)R, and -methylene-NHC(=O)NHR, wherein R is defined as described in any one of the embodiments of the present disclosure; for example, each R is independently selected from hydrogen, -OH, methyl, ethyl, -methylene-O-ethyl, -S(=O)₂-methyl, cyclobutyl, azetidinyl, -methylene-cyclobutyl, and -methylene-azetidinyl, wherein the aforementioned groups are each independently and optionally substituted with 1, 2, or 3 groups selected from -OH, OCH₃, NHCH₃, N(CH₃)₂, and CH₃.

In certain embodiments, R^{9a} is selected from hydrogen and -methylene-N(R)R, wherein R is defined as described in any one of the embodiments of the present disclosure.

In certain embodiments, R^{9a} is selected from hydrogen, -CN, -NHCH₃, -N(CH₃)₂, -NHC(=O)H, - NHC(=O)CH₂OH, -NHC(=O)CH₂OCH₃, -NHC(=O)CH₂NHCH₃, -NHC(=O)CH₂N(CH₃)₂, - CH₂NH₂, -CH₂NHCH₃, -CH₂N(CH₃)₂, -CH₂N(CH₂CH₃)₂, -CH₂N(CH₃)(CH₂CH₃), - CH₂NHC(=O)CH₂OH, -CH₂NHC(=O)CH₂OCH₃, -CH₂NHC(=O)CH₂NHCH₃, - CH₂NHC(=O)CH₂N(CH₃)₂, -CH₂NHC(=O)H, -CH₂NHC(=O)CH₃, -CH₂NHC(=O)NHOH, - CH₂NHC(=O)NHCH₂CH₂OH, -CH₂NHC(=O)CH(N(CH₃)₂)CH₂OH, -CH₂NHC(=O)CH₂NHCH₃, - CH₂NHC(=O)CH₂N(CH₃)₂, -CH₂NHC(=O)CH₂N(CH₂CH₃)₂, -CH₂NHC(=O)CH₂OCH₂CH₂OH, - CH₂NHS(=O)₂CH₃, -CH₂NHC(=O)cyclobutyl-OH, -CH₂NHC(=O)-azetidinyl-CH₃, and - CH₂NHC(=O)CH₂-azetidinyl.

In certain embodiments, R^{9a} is selected from hydrogen, -CH₂NH₂, -CH₂NHCH₃, -NHC(=O)H, - NHC(=O)CH₂OH, and -CH₂NHC(=O)CH₂OH.

In certain embodiments, R^{9a} is selected from hydrogen, 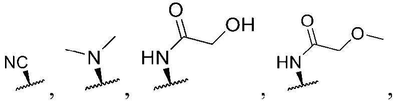 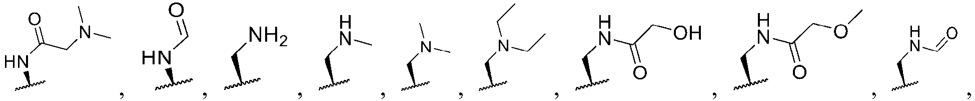 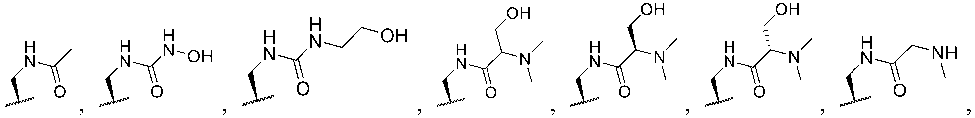 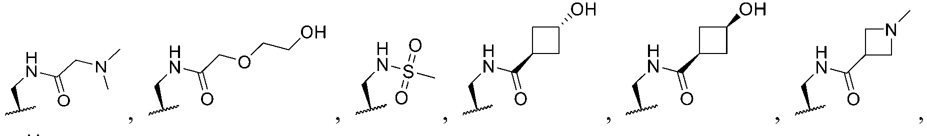 and

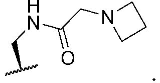

In certain embodiments, R^{9a} is selected from hydrogen,

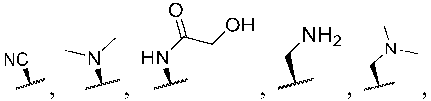

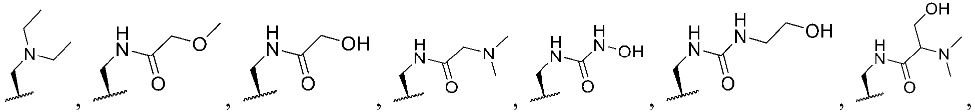

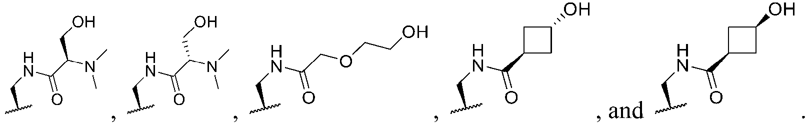

In certain embodiments, R^{9a} is selected from hydrogen,

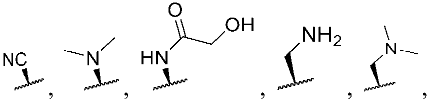

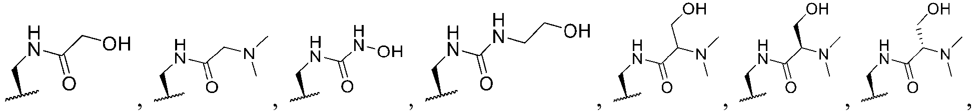

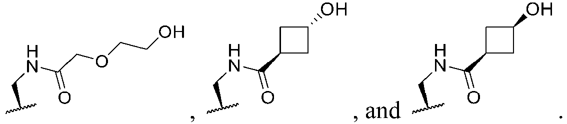

In certain embodiments, R^{9a} is selected from hydrogen, -CN, -methylene-N(R)R, and -methylene-NHC(=O)R, wherein each R is independently selected from hydrogen, -(C₁-C₄)alkyl, -methylene-O-(C₁-C₄)alkyl, -methylene-NH-(C₁-C₄)alkyl, -methylene-N[(C₁-C₄)alkyl]₂, and -S(=O)₂-(C₁-C₄)alkyl.

In certain embodiments, R^{9a} is selected from hydrogen,

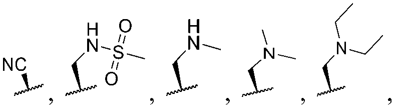

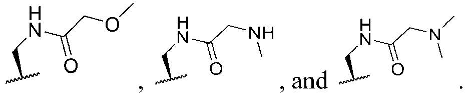

In certain embodiments, R^{9a} is selected from hydrogen,

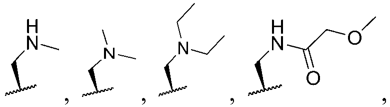

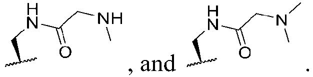

In certain embodiments, R^{9a} is selected from hydrogen, 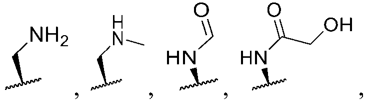 and

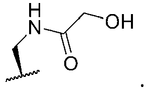

In certain embodiments, R^{9a} is selected from hydrogen,

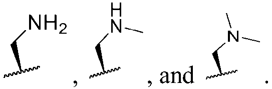

In certain embodiments, R^{9a} is selected from hydrogen,

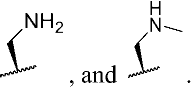

In certain embodiments, R^{9a} is hydrogen.

In certain embodiments, R^{9a} is

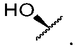

In certain embodiments, R^{9b} is selected from hydrogen and

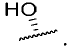

In certain embodiments, R^{9b} is -OH.

In certain embodiments, R^{9b} is

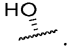

In certain embodiments, R^{9b} is selected from hydrogen,

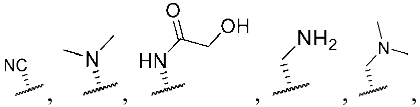

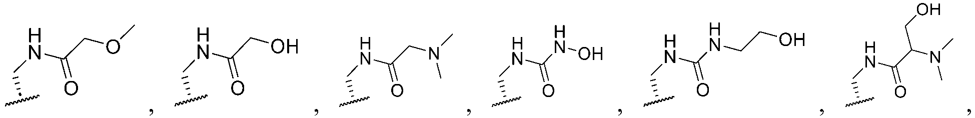

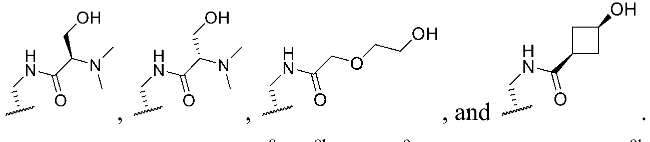

In certain embodiments, R^{8a}, R^{8b}, and R^{9a} are hydrogen, and R^{9b} is as described in any one of the embodiments of the present disclosure.

In certain embodiments, R^{8a}, R^{8b}, and R^{9b} are hydrogen, and R^{9a} is as described in any one of the embodiments of the present disclosure.

In certain embodiments, R^{8a} and R^{9a} are hydrogen, R^{8b} and R^{9b} are as described in any one of the embodiments of the present disclosure, and neither R^{8b} nor R^{9b} is hydrogen. In certain embodiments, R^{8a} and R^{8b}, and R^{9a} and R^{9b} are independently combined to form oxo, -(C₂-C₄)alkenyl, -(C₃-C₆)cycloalkyl, or -(4-8 membered)heterocyclyl.

In certain embodiments, R^{8a} and R^{8b} are combined to form oxo, -(C₂-C₄)alkenyl, -(C₃-C₆)cycloalkyl, or -(4-8 membered)heterocyclyl.

In certain embodiments, R^{9a} and R^{9b} are combined to form oxo, -(C₂-C₄)alkenyl, -(C₃-C₆)cycloalkyl, or -(4-8 membered)heterocyclyl.

In certain embodiments, R^{8a} and R^{8b}, and R^{9a} and R^{9b} are independently combined to form oxo, vinyl, propenyl, cyclopropyl, cyclobutyl, cyclopentyl, oxetanyl, tetrahydrofuranyl, tetrahydropyranyl, azetidinyl, pyrrolidinyl, piperidinyl, or piperazinyl.

In certain embodiments, R^{8a} and R^{9a}, together with the carbon atoms to which they are attached, form (C₃-C₆)cycloalkyl, (4-8 membered)heterocyclyl, or (5-9 membered)heteroaryl, wherein the aforementioned group is optionally substituted with 1, 2, 3, 4, 5, 6, or 7 groups selected from hydrogen, halogen, -OH, -O-(C₁-C₄)alkyl, -NH₂, -NH(C₁-C₄)alkyl, -N[(C₁-C₄)alkyl]₂, -(C₃-C₆)cycloalkyl, and -(C₆-C₁₀)aryl.

In certain embodiments, R^{8b} and R^{9b}, together with the carbon atoms to which they are attached, form (C₃-C₆)cycloalkyl, (4-8 membered)heterocyclyl, or (5-9 membered)heteroaryl, wherein the aforementioned group is optionally substituted with 1, 2, 3, 4, 5, 6, or 7 groups selected from hydrogen, halogen, -OH, -O-(C₁-C₄)alkyl, -NH₂, -NH(C₁-C₄)alkyl, -N[(C₁-C₄)alkyl]₂, -(C₃-C₆)cycloalkyl, and -(C₆-C₁₀)aryl.

In certain embodiments, R^{8a} and R^{9a}, and R^{8b} and R^{9b}, together with the carbon atoms to which they are attached, form (C₃-C₆)cycloalkyl, (4-8 membered)heterocyclyl, or (5-9 membered)heteroaryl, wherein the aforementioned group is optionally substituted with 1, 2, 3, 4, 5, 6, or 7 groups selected from hydrogen, halogen, -OH, -O-(C₁-C₄)alkyl, -NH₂, -NH(C₁-C₄)alkyl, -N[(C₁-C₄)alkyl]₂, -(C₃-C₆)cycloalkyl, and -(C₆-C₁₀)aryl.

In certain embodiments, R^{8a} and R^{9a}, together with the carbon atoms to which they are attached, form (4-8 membered)heterocyclyl or (5-9 membered)heteroaryl, wherein the aforementioned group is optionally substituted with 1, 2, 3, 4, or 5 groups selected from hydrogen, fluorine, chlorine, -OH, -O-methyl, -O-ethyl, -NH₂, -NH-methyl, -NH-ethyl, -N(methyl)₂, -N(methyl)-ethyl, -N(ethyl)₂, cyclopropyl, cyclobutyl, cyclopentyl, phenyl, and naphthyl.

In certain embodiments, R^{8b} and R^{9b}, together with the carbon atoms to which they are attached, form (4-8 membered)heterocyclyl or (5-9 membered)heteroaryl, wherein the aforementioned group is optionally substituted with 1, 2, 3, 4, or 5 groups selected from hydrogen, fluorine, chlorine, -OH, -O-methyl, -O-ethyl, -NH₂, -NH-methyl, -NH-ethyl, -N(methyl)₂, -N(methyl)-ethyl, -N(ethyl)₂, cyclopropyl, cyclobutyl, cyclopentyl, phenyl, and naphthyl.

In certain embodiments, R^{8a} and R^{9a}, and R^{8b} and R^{9b}, together with the carbon atoms to which they are attached, form (4-8 membered)heterocyclyl or (5-9 membered)heteroaryl, wherein the aforementioned group is optionally substituted with 1, 2, 3, 4, or 5 groups selected from hydrogen, fluorine, chlorine, -OH, -O-methyl, -O-ethyl, -NH₂, -NH-methyl, -NH-ethyl, -N(methyl)₂, - N(methyl)-ethyl, -N(ethyl)₂, cyclopropyl, cyclobutyl, cyclopentyl, phenyl, and naphthyl.

In certain embodiments, R^{8a} and R^{9a}, together with the carbon atoms to which they are attached, form (4-8 membered)heterocyclyl or (5-9 membered)heteroaryl, wherein the aforementioned group is optionally substituted with 1, 2, 3, 4, or 5 groups selected from hydrogen, fluorine, chlorine, -OH, -O-methyl, -O-ethyl, -NH₂, -NH-methyl, -NH-ethyl, -N(methyl)₂, -N(methyl)-ethyl, -N(ethyl)₂, cyclopropyl, cyclobutyl, cyclopentyl, phenyl, and naphthyl.

In certain embodiments, R^{8b} and R^{9b}, together with the carbon atoms to which they are attached, form (4-8 membered)heterocyclyl or (5-9 membered)heteroaryl, wherein the aforementioned group is optionally substituted with 1, 2, 3, 4, or 5 groups selected from hydrogen, fluorine, chlorine, -OH, -O-methyl, -O-ethyl, -NH₂, -NH-methyl, -NH-ethyl, -N(methyl)₂, -N(methyl)-ethyl, -N(ethyl)₂, cyclopropyl, cyclobutyl, cyclopentyl, phenyl, and naphthyl.

In certain embodiments, R^{8a} and R^{9a} are absent, and R^{8b} and R^{9b}, together with the atoms to which they are attached, form 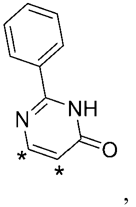 wherein * indicates the position of the attached atom.

In certain embodiments, R¹⁰ is selected from -OH.

In certain embodiments, R¹⁰ and R^{9a}, together with the atoms to which they are attached, form 5-membered nitrogen-containing heterocyclyl, wherein the 5-membered nitrogen-containing heterocyclyl is optionally substituted with 1, 2, 3, 4, or 5 groups selected from hydrogen, halogen, -OH, -(C₁-C₄)alkyl, -NH₂, -NH(C₁-C₄)alkyl, and -N[(C₁-C₄)alkyl]₂.

In certain embodiments, R¹⁰ and R^{9a}, together with the atoms to which they are attached, form imidazolinyl, wherein the imidazolinyl is optionally substituted with 1, 2, or 3 groups selected from halogen, -OH, methyl, -NH₂, -NH-methyl, and -N(methyl)₂.

In certain embodiments, R¹⁰ and R^{9a}, together with the atoms to which they are attached, form 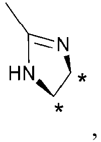 wherein * indicates the position of the attached atom.

In certain embodiments, R¹⁰ is -OH.

In certain embodiments, R¹⁰ is

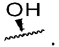

In certain embodiments, the drug group is selected from a structure represented by formula I-A-1, II-A-1', II-A-1, III-A-1', III-A-1, IV-A-1, or V-A-1: 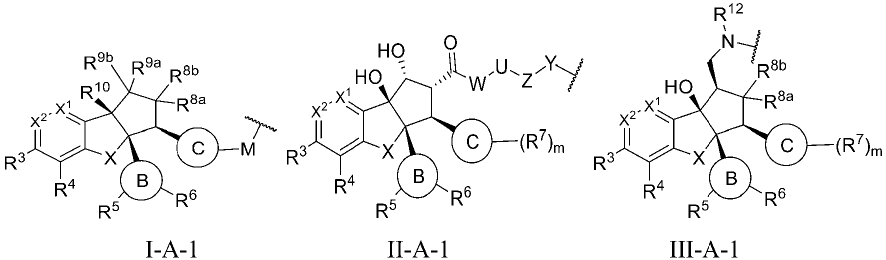 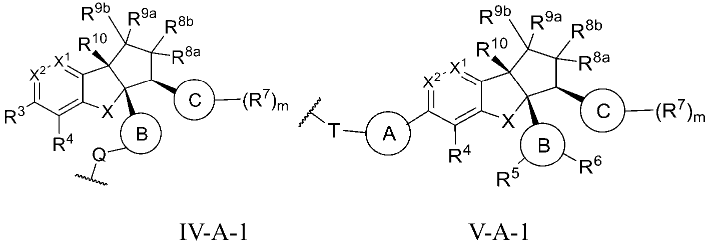 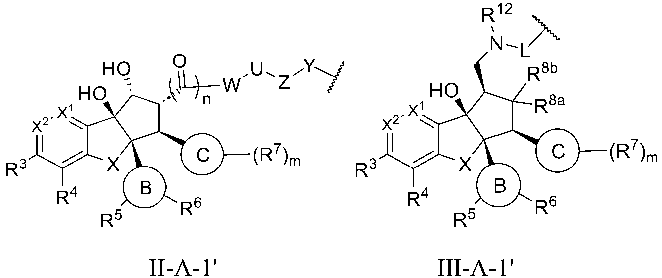 wherein X, X¹, X², ring A, ring B, ring C, R³, R⁴, R⁵, R⁶, R⁷, R^{8a}, R^{8b}, R^{9a}, R^{9b}, R¹⁰, R¹², m, n, M, W, U, Z, Y, L, Q, and T are defined as described in any one of the embodiments of the present disclosure.

In certain embodiments, the drug group is selected from a structure represented by formula I-A-2, II-A-2', II-A-2, III-A-2', III-A-2, IV-A-2, or V-A-2: 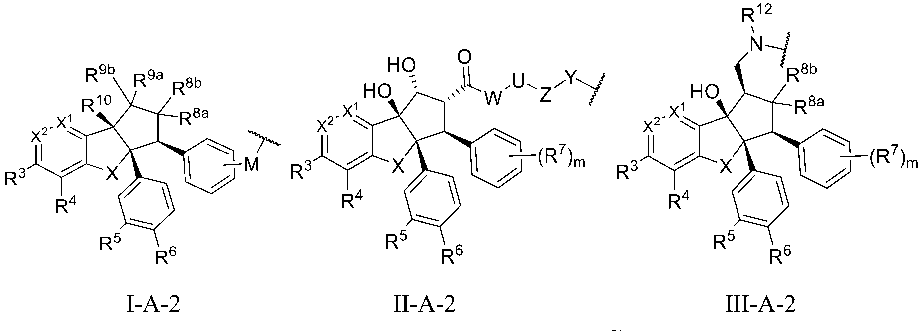 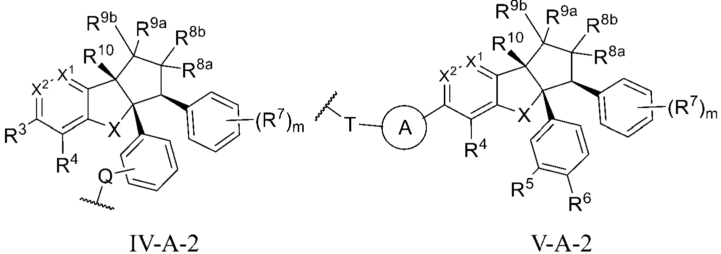 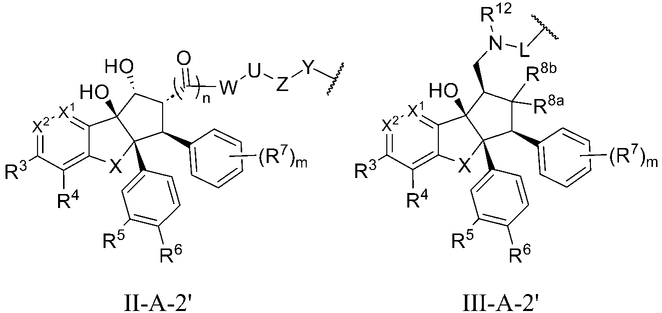 wherein X, X¹, X², ring A, R³, R⁴, R⁵, R⁶, R⁷, R^{8a}, R^{8b}, R^{9a}, R^{9b}, R¹⁰, R¹², m, n, M, W, U, Z, Y, L, Q, and T are defined as described in any one of the embodiments of the present disclosure.

In certain embodiments, the drug group is selected from a structure represented by formula I-A-3, III-A-3', III-A-3, IV-A-3, or V-A-3: 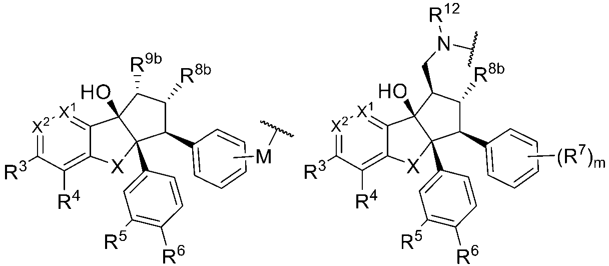 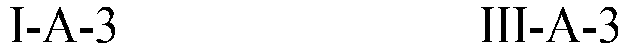 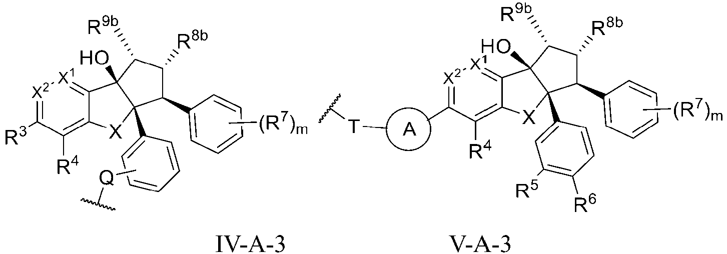 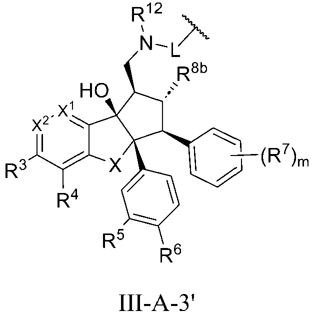 wherein X, X¹, X², ring A, R³, R⁴, R⁵, R⁶, R⁷, R^{8b}, R^{9b}, R¹², m, M, L, Q, and T are defined as described in any one of the embodiments of the present disclosure.

In certain embodiments, the drug group is selected from a structure represented by formula I-A-4, II-A-4', II-A-4, III-A-4', III-A-4, IV-A-4, or V-A-4: 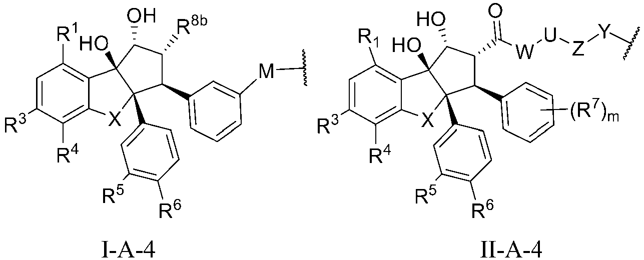 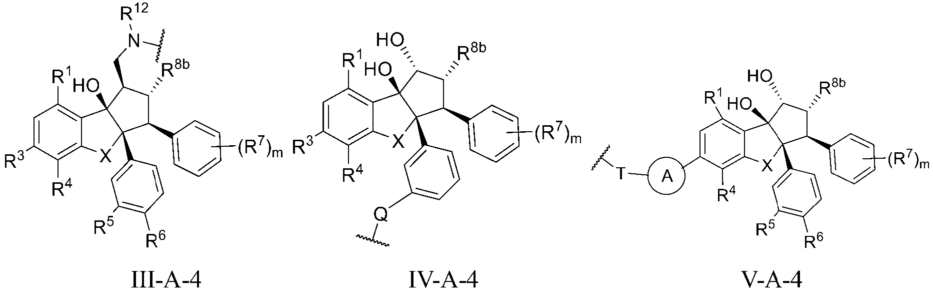 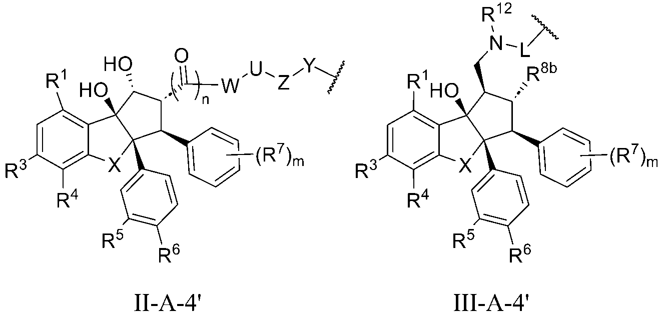 wherein X, R¹, ring A, R³, R⁴, R⁵, R⁶, R⁷, R^{8b}, R¹², m, n, M, W, U, Z, Y, L, Q, and T are defined as described in any one of the embodiments of the present disclosure.

In certain embodiments, provided is a compound of the structure represented by the general formula described above, wherein:
M is selected from -NH-, -O-, -(C₁-C₄)alkylene-O-, and -(C₁-C₄)alkylene-NH-;
n is 0 or 1;
W is absent or is selected from -(C₁-C₄)alkylene, -NH-, and -N[-(C₁-C₄)alkyl]-;
U is selected from -(C₁-C₄)alkylene-, -(C₃-C₆)cycloalkylene-, and -(4-6 membered)heterocyclylene-;
Z is absent or is selected from -(C₁-C₄)alkylene-, -(C₃-C₆)cycloalkylene-, and -(4-6 membered)heterocyclylene-;
Y is absent or is selected from -O-, -N(R^{y})-, and -N⁺(R^{y})₂-, wherein R^{y} is selected from hydrogen, -(C₁-C₄)alkyl, and -O(C₁-C₄)alkyl, or R^{y} and Z form -(4-6 membered)heterocyclylene-;
R¹² is selected from hydrogen, -(C₁-C₄)alkyl, -O-(C₁-C₄)alkyl, and -(C₃-C₆)cycloalkyl;
L is absent or is selected from -C(=O)-(C₁-C₄)alkylene-O-, -C(=O)-(C₁-C₄)alkylene-N(R^{L})-, and - C(=O)-(C₁-C₄)alkylene-N⁺(R^{L})₂-, wherein R^{L} is selected from hydrogen, -(C₁-C₄)alkyl, and -(C₁-C₄)haloalkyl;
Q is selected from -NH-, -O-, -(C₁-C₄)alkylene-O-, and -(C₁-C₄)alkylene-NH-;
T is selected from -NH-, -O-, -(C₁-C₄)alkylene-O-, and -(C₁-C₄)alkylene-NH-;
X¹ is selected from N and C(R¹), wherein R¹ is selected from hydrogen, halogen, cyano, -OH, -
(C₁-C₄)alkyl, and -O-(C₁-C₄)alkyl;
X² is selected from C(R²), wherein R² is selected from hydrogen, halogen, cyano, -OH, -(C₁-C₄)alkyl, and -O-(C₁-C₄)alkyl;
R³¹ is selected from oxo, hydrogen, fluorine, chlorine, cyano, -OH, -SH, -NH₂, -(C₁-C₄)alkyl, -O-(C₁-C₄)alkyl, -(C₁-C₄)alkylene-OH, -(C₁-C₄)haloalkyl, -(C₁-C₄)alkylene-NH₂, -(C₁-C₄)alkylene-NH(C₁-C₄)alkyl, and -(C₁-C₄)alkylene-N[(C₁-C₄)alkyl]₂;
R³ is selected from -(5-9 membered)heteroaryl, -(C₃-C₆)cycloalkyl, and -(4-6 membered)heterocyclyl, and R³ is optionally substituted with 1, 2, 3, or 4 R³¹;
ring A is selected from -(5-9 membered)heteroarylene-, and ring A is optionally substituted with 1, 2, 3, or 4 R³¹;
R⁴ is selected from hydrogen, fluorine, chlorine, cyano, -OH, -NH₂, methyl, halomethyl, -O-methyl, -NH-methyl, and cyclopropyl;
X is selected from O, S, NH, and N[(C₁-C₄)alkyl];
ring B is phenyl or phenylene, wherein the phenylene is optionally substituted with 1 R⁵ or R⁶; R⁵ and R⁶ are each independently selected from hydrogen, fluorine, chlorine, bromine, cyano, - OH, -NH₂, -(C₁-C₄)alkyl, -(C₁-C₄)alkyl-OH, -(C₁-C₄)alkyl-NH₂, -(C₁-C₄)haloalkyl, -O-(C₁-C₄)alkyl, -O-(C₁-C₄)haloalkyl, and -(C₃-C₆)cycloalkyl; or R⁵ and R⁶, together with the atoms to
which they are attached, form 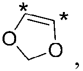 wherein * indicates the position of the attached atom; ring C is phenyl or phenylene, wherein the phenylene is optionally substituted with 1 R⁷; each R⁷ is independently selected from hydrogen, fluorine, chlorine, bromine, cyano, -OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, and -(C₁-C₄)alkylene-NH₂;
m is 1, 2, or 3;
each R is independently selected from hydrogen, -OH, NH₂, -(C₁-C₄)alkyl, -(C₁-C₄)haloalkyl, -O-(C₁-C₄)alkyl, -O-(C₁-C₄)haloalkyl, -(C₃-C₆)cycloalkyl, -(4-8 membered)heterocyclyl, -C(=O)-H, - C(=O)-(C₁-C₄)alkyl, -C(=O)-(C₃-C₆)cycloalkyl, and -[(C₁-C₄)alkylene]-(4-8 membered)heterocyclyl, wherein the -(C₁-C₄)alkyl, -(C₁-C₄)haloalkyl, -O-(C₁-C₄)alkyl, -O-(C₁-C₄)haloalkyl, -(C₃-C₆)cycloalkyl, -(4-8 membered)heterocyclyl, -C(=O)-(C₁-C₄)alkyl, -C(=O)-(C₃-C₆)cycloalkyl, and -[(C₁-C₄)alkylene]-(4-8 membered)heterocyclyl are optionally substituted with 1, 2, 3, 4, or 5 groups selected from hydrogen, halogen, -OH, -O-methyl, -O-ethyl, -O-propyl, -NH₂, -NH-methyl, -NH-ethyl, -NH-propyl, -N(methyl)₂, -N(methyl)-ethyl, -N(methyl)-propyl, - N(ethyl)₂, -N(ethyl)-propyl, methyl, and ethyl;
R^{8a} is 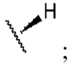
R^{8b} is selected from hydrogen, -methylene-R, -ethylene-R, -propylene-R, -methylene-OR, - ethylene-OR, -propylene-OR, -methylene-N(R)R, -ethylene-N(R)R, -propylene-N(R)R, -C(=O)R, -C(=O)N(R)R, and -C(=O)OR;
R^{9a} is selected from hydrogen, -OR, -N(R)R, -methylene-N(R)R, -methylene-NHC(=O)R, and - NHC(=O)R;
R^{9b} is selected from hydrogen and 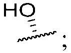
R¹⁰ is

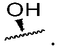

In certain embodiments, provided is a compound of the structure represented by the general formula described above, wherein:
M is selected from -NH-, -O-, -CH₂O-, and -CH₂NH-;
n is 0 or 1;
the structural unit -W-U-Z-Y- is selected from -N(R^{w})-(C₁-C₆)alkylene-N(R^{y})-, -N(R^{w})-(C₁-C₆)alkylene-N⁺(R^{y})₂-, -N(R^{w})-(C₁-C₆)alkylene-O-, -(C₁-C₆)alkylene-N(R^{y})-, -(C₁-C₆)alkylene-N⁺(R^{y})₂-, -(C₁-C₆)alkylene-O-, -(4-6 membered)heterocyclylene-O-, and -(4-6 membered)heterocyclylene-N(R^{y})-;
R¹² is selected from hydrogen and -(C₁-C₄)alkyl;
L is absent or is selected from -C(=O)CH₂O-, -C(=O)CH₂CH₂O-, -C(=O)CH₂N(R^{L})-, - C(=O)CH₂CH₂N(R^{L})-, -C(=O)CH₂N⁺(R^{L})₂-, and -C(=O)CH₂CH₂N⁺(R^{L})₂-, wherein R^{L} is selected from hydrogen, methyl, and ethyl;
Q is selected from -NH-, -O-, -CH₂O-, and -CH₂NH-;
T is selected from -NH-, -O-, -CH₂O-, and -CH₂NH-;
X¹ is selected from N and C(R¹), wherein R¹ is selected from hydrogen, fluorine, chlorine, cyano, -OH, methyl, ethyl, propyl, -O-methyl, -O-ethyl, and -O-propyl;
X² is selected from C(R²), wherein R² is selected from hydrogen, fluorine, chlorine, cyano, -OH, methyl, ethyl, propyl, -O-methyl, -O-ethyl, and -O-propyl;
R³¹ is selected from oxo, hydrogen, fluorine, chlorine, cyano, -OH, -SH, -NH₂, methyl, halomethyl, -O-methyl, -CH₂OH, -CH₂NH₂, and -CH₂CH₂N(CH₃)₂;
R³ is selected from cyclopropyl, oxetanyl, morpholinyl, morpholinonyl, piperidinyl, piperazinyl, piperazinonyl, oxazolyl, isoxazolyl, thiazolyl, isothiazolyl, pyrazolyl, imidazolyl, triazolyl, tetrazolyl, pyridinyl, pyrazinyl, pyridazinyl, pyrimidinyl, pyridazinoimidazolyl, pyrazinoimidazolyl, and pyrimidinonyl, and R³ is optionally substituted with 1, 2, or 3 R³¹;
ring A is selected from oxazolylene, isoxazolylene, thiazolylene, isothiazolylene, pyrazolylene, imidazolylene, triazolylene, tetrazolylene, pyridinylene, pyrazinylene, pyridazinylene, pyrimidinylene, pyridazinoimidazolylene, pyrazinoimidazolylene, and pyrimidinonylene, and ring A is optionally substituted with 1, 2, or 3 R³¹;
R⁴ is selected from hydrogen, fluorine, chlorine, cyano, -OH, and -NH₂;
X is selected from O;
the structural unit 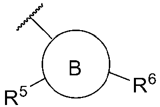 is 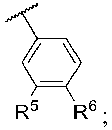
R⁵ is selected from hydrogen, -OH, -NH₂, and -CH₂NH₂;
R⁶ is selected from hydrogen, fluorine, chlorine, bromine, cyano, -OH, -NH₂, cyclopropyl, and - O-methyl;
or R⁵ and R⁶, together with the atoms to which they are attached, form 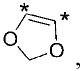 wherein * indicates the position of the attached atom;
or ring B is 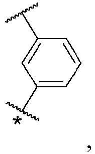 wherein the * end is attached to Q;
the structural unit 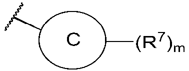 is 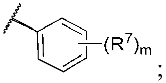 each R⁷ is independently selected from hydrogen, fluorine, chlorine, bromine, cyano, -OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, and -(C₁-C₄)alkylene-NH₂;
m is 1, 2, or 3;
or ring C is 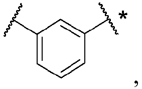 wherein the * end is attached to M;
R^{8a} is 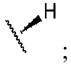
R^{8b} is selected from hydrogen, -methylene-R, -methylene-N(R)R, -C(=O)OR, -C(=O)N(R)R, and -C(=O)R, wherein R is defined as described in any one of the embodiments of the present disclosure; for example, each R is independently selected from hydrogen, -OH, methyl, ethyl, -O-methyl, -C(=O)-methyl, -S(=O)₂-methyl, azetidinyl, pyrrolidinyl, and piperazinyl, wherein the aforementioned groups are each independently and optionally substituted with 1, 2, or 3 groups selected from -OH and -NH₂;
R^{9a} is selected from hydrogen, -CN, -N(R)R, -NHC(=O)R, -methylene-N(R)R, -methylene-NHC(=O)R, and -methylene-NHC(=O)NHR, wherein R is defined as described in any one of the embodiments of the present disclosure; for example, each R is independently selected from hydrogen, -OH, methyl, ethyl, -methylene-O-ethyl, -S(=O)₂-methyl, cyclobutyl, azetidinyl, - methylene-cyclobutyl, and -methylene-azetidinyl, wherein the aforementioned groups are each independently and optionally substituted with 1, 2, or 3 groups selected from -OH, OCH₃, NHCH₃, N(CH₃)₂, and CH₃;
R^{9b} is selected from hydrogen and 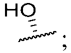
R¹⁰ is

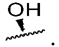

In certain embodiments, provided is a compound of the structure represented by the general formula described above, wherein:
M is selected from -NH-, -O-, -CH₂O-, and -CH₂NH-;
n is 0 or 1;
W is absent or is selected from methylene, ethylene, -NH-, -N(Me)-, and -N(Et)-;
U is selected from methylene, ethylene, cyclopropyl, azetidinylene, and pyrrolidinylene;
Z is absent or is selected from methylene, ethylene, cyclopropyl, azetidinylene, and pyrrolidinylene;
Y is selected from -O-, -N(R^{y})-, and -N⁺(R^{y})₂-, wherein R^{y} is selected from hydrogen, methyl, ethyl, and -O-methyl;
R¹² is selected from hydrogen, methyl, and ethyl;
L is absent or is selected from -C(=O)CH₂O-, -C(=O)CH₂CH₂O-, -C(=O)CH₂N(R^{L})-, - C(=O)CH₂CH₂N(R^{L})-, -C(=O)CH₂N⁺(R^{L})₂-, and -C(=O)CH₂CH₂N⁺(R^{L})2-, wherein R^{L} is selected from hydrogen, methyl, and ethyl;
Q is selected from -NH-;
T is selected from -NH-;
X¹ is C(R¹) and N;
R¹ is selected from hydrogen, fluorine, chlorine, cyano, -OH, methyl, ethyl, propyl, -O-methyl, - O-ethyl, and -O-propyl;
X² is selected from CH;
R³¹ is selected from hydrogen, fluorine, chlorine, cyano, -OH, -SH, -NH₂, -CH₂OH, -CH₂NH₂, methyl, trifluoromethyl, and -O-methyl;
R³ is selected from 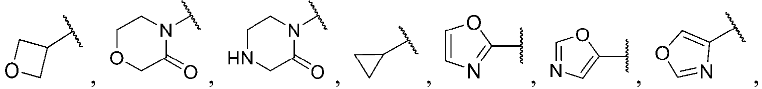 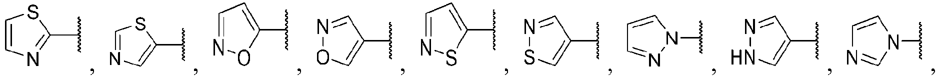 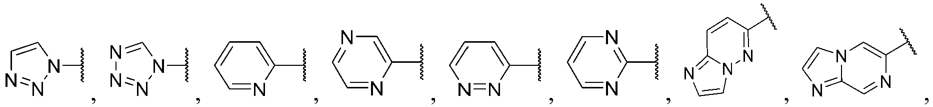 and 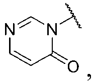 and R³ is optionally substituted with 1, 2, or 3 R³¹;
ring A is selected from 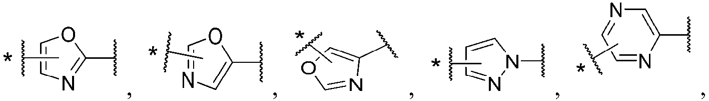 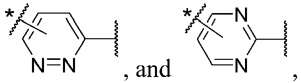 wherein the * end is attached to T;
R⁴ is hydrogen;
X is O;
the structural unit 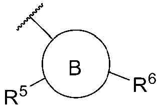 is 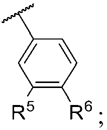 R⁵ is selected from hydrogen and -NH₂;
R⁶ is selected from hydrogen, fluorine, chlorine, bromine, cyano, -OH, -NH₂, cyclopropyl, and - O-methyl;
or R⁵ and R⁶, together with the atoms to which they are attached, form 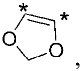 wherein * indicates the position of the attached atom;
or ring B is 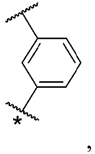 wherein the * end is attached to Q;
the structural unit 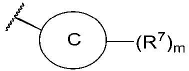 is 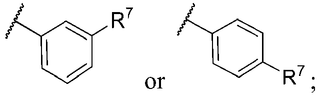
or ring C is 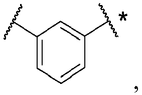 wherein the * end is attached to M;
each R⁷ is independently selected from hydrogen, fluorine, chlorine, bromine, -OH, and -NH₂;
R^{8a} is 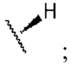
R^{8b} is selected from hydrogen, 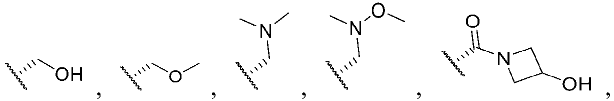 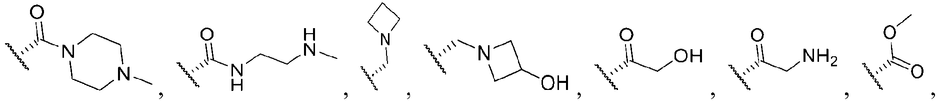 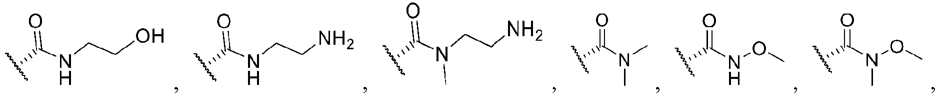 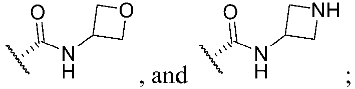
R^{9a} is selected from hydrogen, 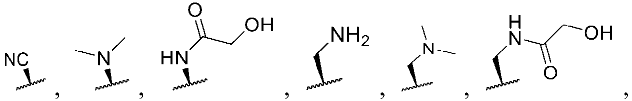 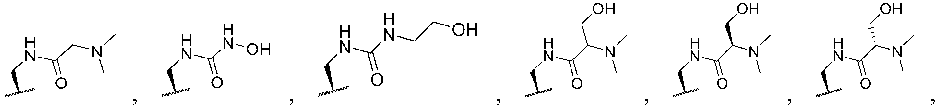 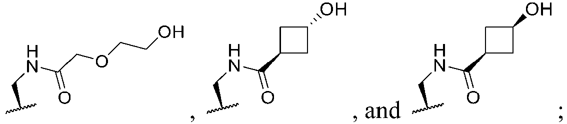
R^{9b} is selected from hydrogen and 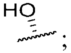
R¹⁰ is

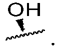

In certain embodiments, provided is a compound of the structure represented by the general formula described above, wherein:
M is selected from -NH-, -O-, *-CH₂O-, and *-CH₂NH-, wherein the * end is attached to ring C;
n is 0 or 1;
W is absent or is selected from -NH- and -N(Me)-;
U is selected from methylene, ethylene, and 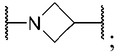
Z is absent;
Y is selected from -O-, -NH-, -N(CH₃)-, and -N⁺(CH₃)₂-;
the structural unit -W-U-Z-Y- is selected from *-NH-CH₂-CH₂-NH-, *-NH-CH₂-CH₂-O-, *-CH₂-
O-, *-CH₂-NH-, *-CH₂-N(CH₃)-, *-CH₂-N⁺(CH₃)₂-, and 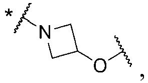 wherein the * end is attached to carbonyl;
R¹² is selected from hydrogen and methyl;
L is absent or is selected from *-C(=O)CH₂O-, *-C(=O)CH₂N(CH₃)-, and *-C(=O)CH₂N⁺(CH₃)₂-, wherein the * end is attached to N;
Q is selected from -NH-;
T is selected from -NH-;
X¹ is selected from C(R¹) and N;
R¹ is selected from -O-methyl;
X² is selected from CH;
R³ is selected from 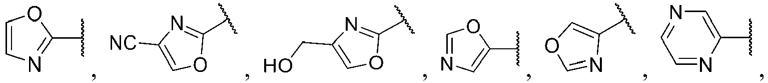 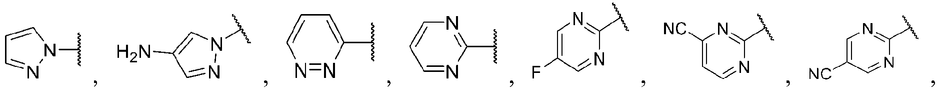 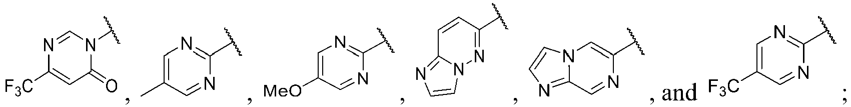
ring A is selected from 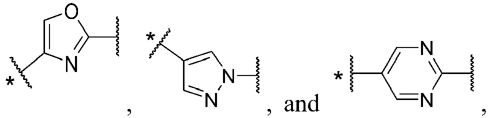 wherein the * end is attached to T;
R⁴ is hydrogen;
X is O;
the structural unit 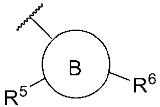 is
R⁵ is selected from hydrogen;
R⁶ is selected from cyano and -O-methyl;
or R⁵ and R⁶, together with the atoms to which they are attached, form 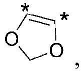 wherein * indicates the position of the attached atom;
or ring B is 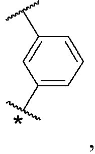 wherein the * end is attached to Q;
the structural unit 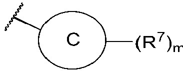 is 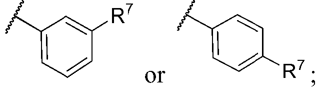
R⁷ is hydrogen;
or ring C is 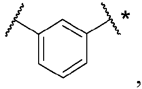 wherein the * end is attached to M;
R^{8a} is 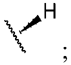
R^{8b} is selected from hydrogen, 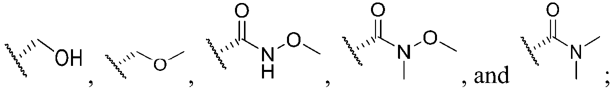
R^{9a} is hydrogen, 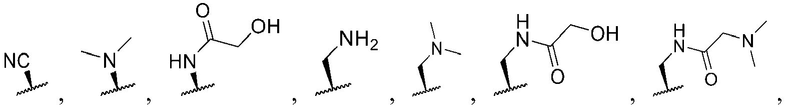 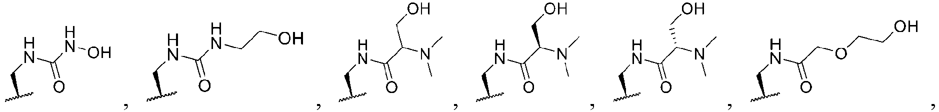 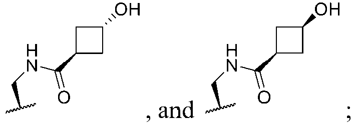
R^{9b} is 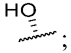
R¹⁰ is

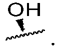

In certain embodiments, the drug group is selected from a structure represented by formula I-A-5, II-A-5, III-A-5, IV-A-5, or V-A-5: 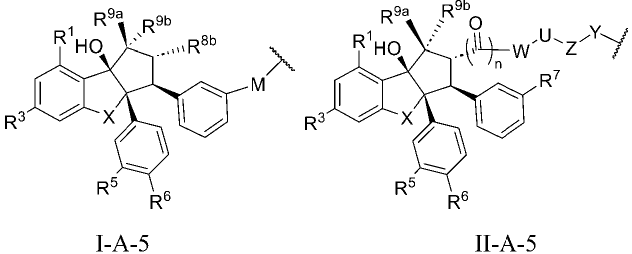 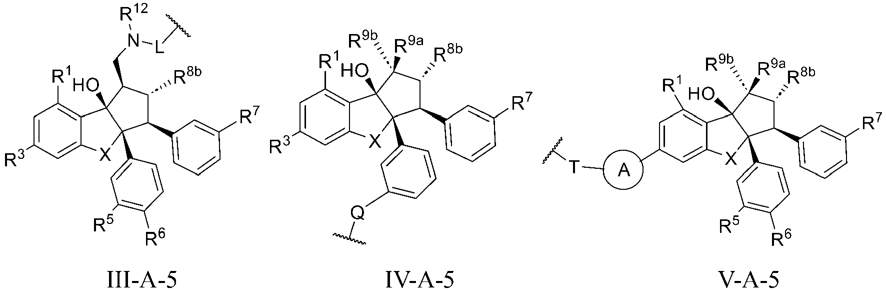 wherein X, R¹, ring A, R³, R⁵, R⁶, R⁷, R^{8b}, R^{9a}, R^{9b}, R¹², n, M, W, U, Z, Y, L, Q, and T are defined as described in any one of the embodiments of the present disclosure.

In certain embodiments, provided is a compound of the structure represented by formula I-A-5, II-A-5, III-A-5, IV-A-5, or V-A-5, wherein:
M is selected from -NH-, -O-, *-CH₂O-, and *-CH₂NH-, wherein the * end is attached to ring C;
n is 0 or 1;
W is absent or is selected from -NH- and -N(Me)-;
U is selected from methylene, ethylene, and 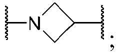
Z is absent;
Y is selected from -O-, -NH-, -N(CH₃)-, and -N⁺(CH₃)₂-;
the structural unit -W-U-Z-Y- is selected from *-NH-CH₂-CH₂-NH-, *-NH-CH₂-CH₂-O-, *-CH₂-
O-, *-CH₂-NH-, *-CH₂-N(CH₃)-, *-CH₂-N⁺(CH₃)₂-, and 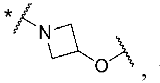 wherein the * end is attached to carbonyl;
R¹² is selected from hydrogen and methyl;
L is absent or is selected from *-C(=O)CH₂O-, *-C(=O)CH₂N(CH₃)-, and *-C(=O)CH₂N⁺(CH₃)₂-,
wherein the * end is attached to N;
Q is selected from -NH-;
T is selected from -NH-;
X is O;
R¹ is selected from hydrogen, halogen, cyano, -OH, -(C₁-C₄)alkyl, and -O-(C₁-C₄)alkyl; in certain embodiments, R¹ is selected from hydrogen, fluorine, chlorine, cyano, -OH, methyl, ethyl, propyl, -O-methyl, -O-ethyl, and -O-propyl;
R³ is selected from oxazolyl, pyrazolyl, and pyrimidinyl; in certain embodiments, R³ is selected from 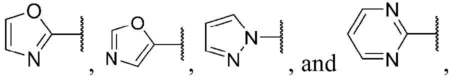 and R³ is optionally substituted with 1, 2, or 3 R³¹;
ring A is selected from -(5-6 membered)heteroarylene-, wherein the -(5-6 membered)heteroarylene- is optionally substituted with 1, 2, 3, or 4 R³¹;
each R³¹ is independently selected from hydrogen, fluorine, chlorine, cyano, -OH, -SH, -NH₂, - CH₂OH, -CH₂NH₂, -CH₃, -CF₃, and -OCH₃;
or R³ is selected from 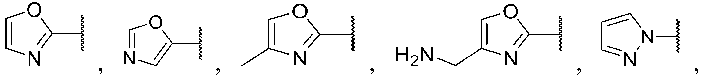 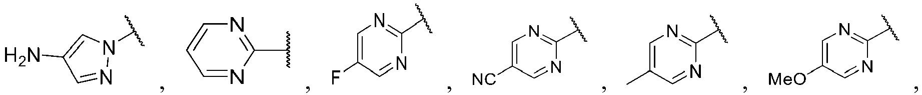 and 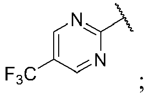
or ring A is selected from 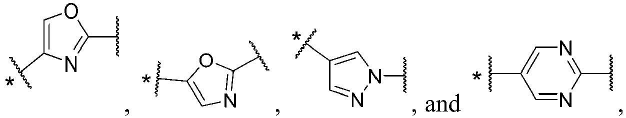 wherein the * end is attached to T;
R⁵ is hydrogen or -NH₂;
R⁶ is selected from hydrogen, fluorine, chlorine, bromine, cyano, -OH, -NH₂, cyclopropyl, and - O-methyl;
or R⁵ and R⁶, together with the atoms to which they are attached, form 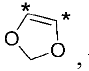 wherein * indicates the position of the attached atom;
each R⁷ is independently selected from hydrogen, fluorine, chlorine, bromine, -OH, and -NH₂;
R^{8b} is selected from hydrogen, -methylene-R, -methylene-OR, -methylene-N(R)R, -C(=O)R, - C(=O)OR, and -C(=O)N(R)R, wherein R is defined as described in any one of the embodiments of the present disclosure; for example, each R is independently selected from hydrogen, -OH, - NH₂, -(C₁-C₄)alkyl, -O-(C₁-C₄)alkyl, -C(=O)-(C₁-C₄)alkyl, -S(=O)₂(C₁-C₄)alkyl, and -(4-8 membered)heterocyclyl, wherein the -(C₁-C₄)alkyl and -(4-8 membered)heterocyclyl are each optionally substituted with 1, 2, 3, 4, or 5 groups selected from hydrogen, halogen, -OH, -NH₂, - NHCH₃, and CH₃; in certain embodiments, R^{8b} is selected from hydrogen, 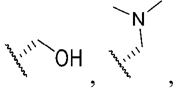 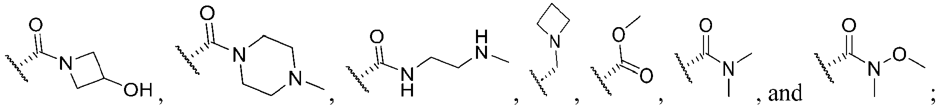
R^{9a} is selected from hydrogen, -CN, -N(R)R, -NHC(=O)R, -methylene-N(R)R, -methylene-NHC(=O)R, and -methylene-NHC(=O)NHR, wherein R is defined as described in any one of the embodiments of the present disclosure; for example, each R is independently selected from hydrogen, -OH, methyl, ethyl, -methylene-O-ethyl, -S(=O)₂-methyl, cyclobutyl, azetidinyl, - methylene-cyclobutyl, and -methylene-azetidinyl, wherein the aforementioned groups are each independently and optionally substituted with 1, 2, or 3 groups selected from -OH, OCH₃, NHCH₃, N(CH₃)₂, and CH₃; in certain embodiments, R^{9a} is selected from hydrogen, 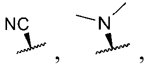 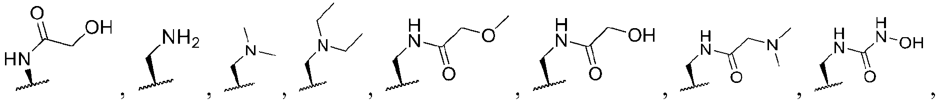 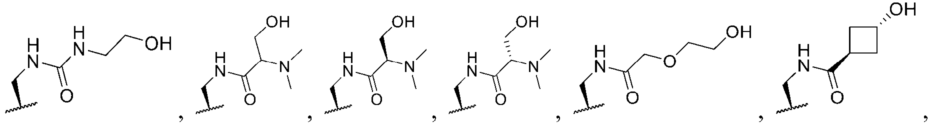 and 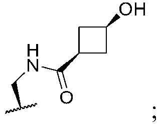
R^{9b} is selected from hydrogen and

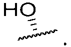

In certain embodiments, the drug group is selected from structures represented by formulas I-A-6 and I-A-7: 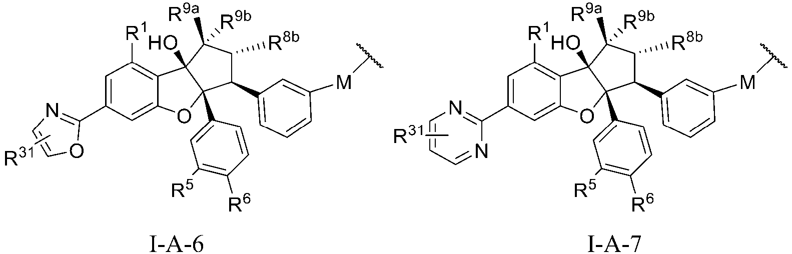 wherein R¹, R³¹, R⁵, R⁶, R^{8b}, R^{9a}, R^{9b}, and M are defined as described in any one of the embodiments of the present disclosure.

In certain embodiments, R^{8b} and R^{9a} are hydrogen, and R^{9b} is as described in any one of the embodiments of the present disclosure.

In certain embodiments, R^{8b} and R^{9b} are hydrogen, and R^{9a} is as described in any one of the embodiments of the present disclosure.

In certain embodiments, R^{9a} is hydrogen, R^{8b} and R^{9b} are as described in any one of the embodiments of the present disclosure, and neither R^{8b} nor R^{9b} is hydrogen.

In certain embodiments, R³¹ is hydrogen.

In certain embodiments, the drug group is selected from the structures shown in Table A:

**Table A**

| No. | Structure |
|---|---|
| I-A1 | 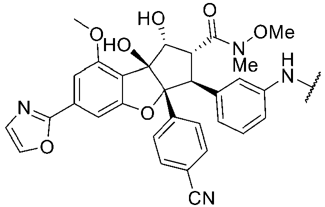 |
| I-A2 | 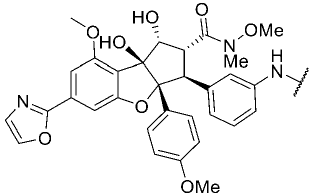 |
| I-A3 | 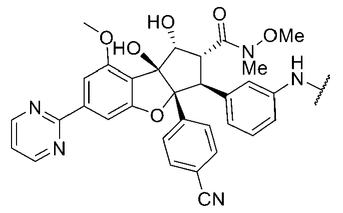 |
| I-A4 | 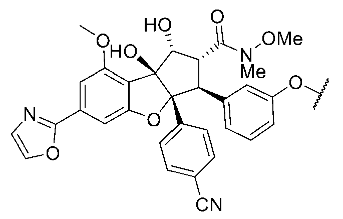 |
| I-A5 | 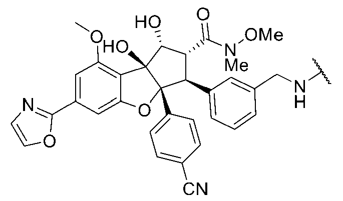 |
| I-A6 | 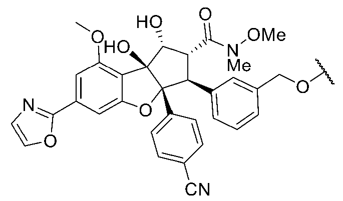 |
| I-A7 | 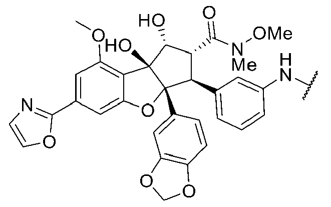 |
| I-A8 | 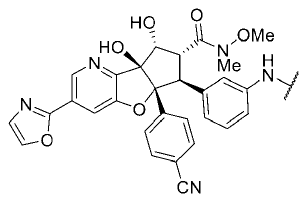 |
| I-A9 | 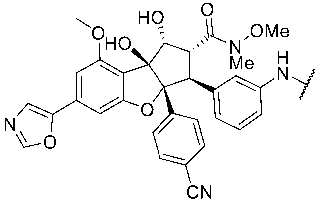 |
| I-A10 | 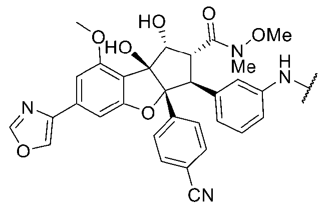 |
| I-A11 | 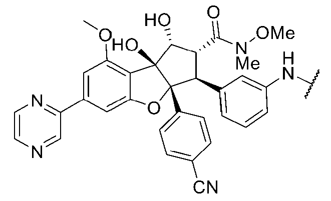 |
| I-A12 | 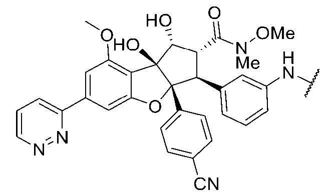 |
| I-A13 | 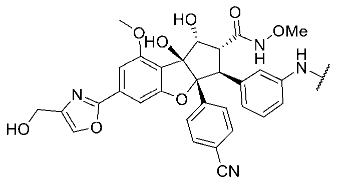 |
| I-A14 | 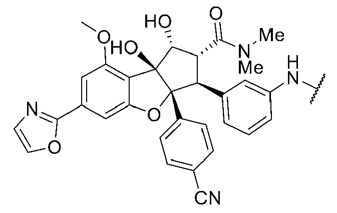 |
| I-A15 | 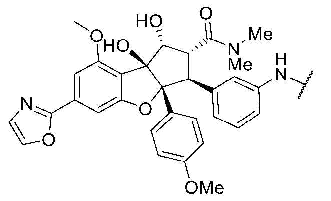 |
| I-A16 | 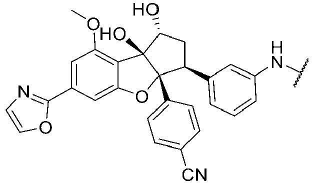 |
| I-A17 | 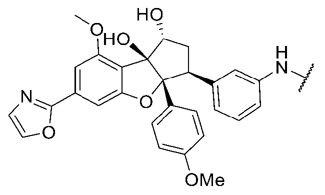 |
| I-A18 | 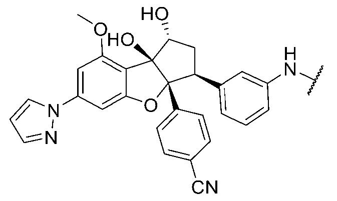 |
| I-A19 | 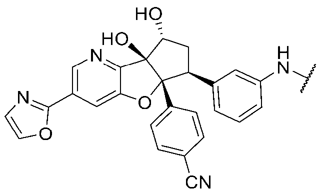 |
| I-A20 | 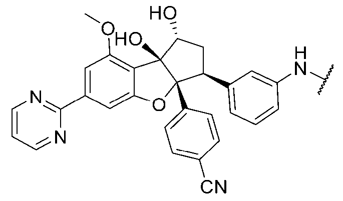 |
| I-A21 | 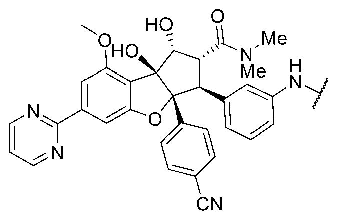 |
| I-A22 | 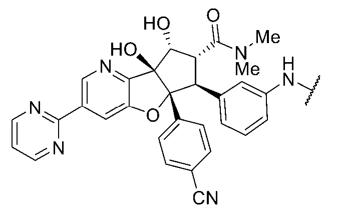 |
| I-A23 | 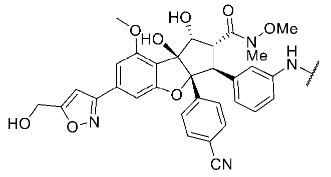 |
| I-A24 | 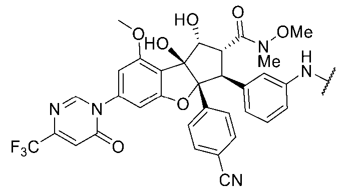 |
| I-A25 | 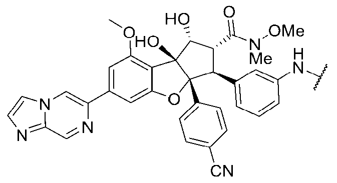 |
| I-A26 | 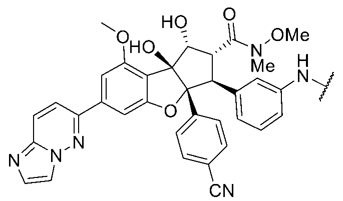 |
| I-A27 | 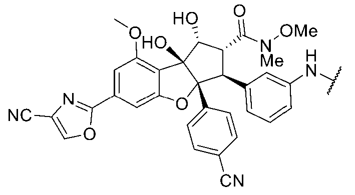 |
| I-A28 | 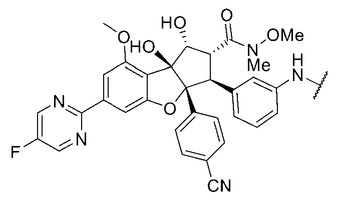 |
| I-A29 | 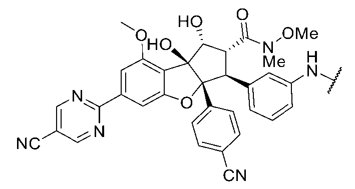 |
| I-A30 | 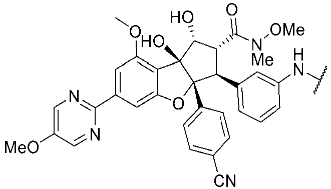 |
| I-A31 | 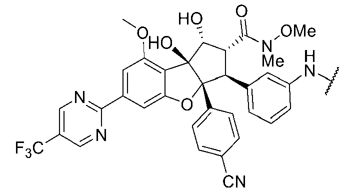 |
| I-A32 | 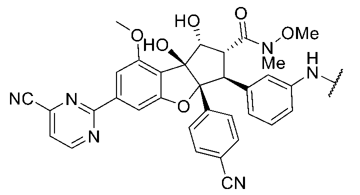 |
| I-A33 | 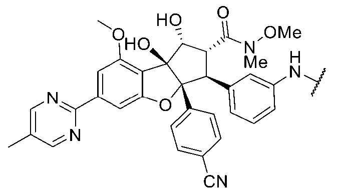 |
| I-A34 | 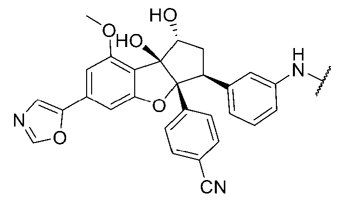 |
| I-A35 | 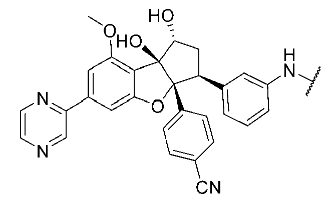 |
| I-A36 | 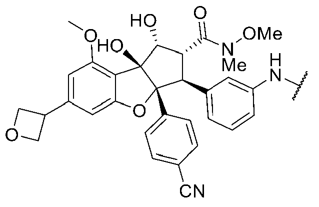 |
| I-A37 | 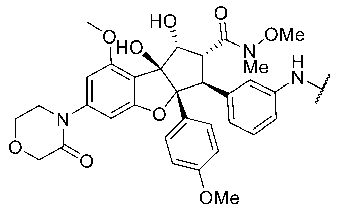 |
| I-A38 | 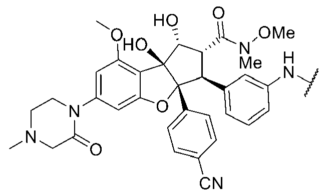 |
| I-A39 | 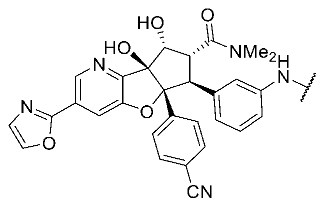 |
| I-A40 | 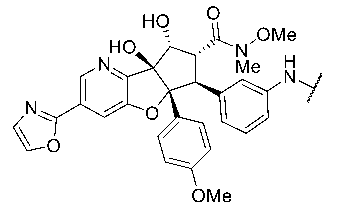 |
| I-A41 | 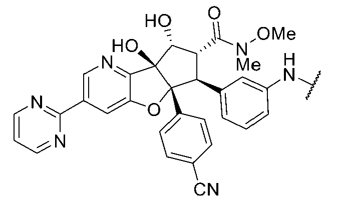 |
| I-A42 | 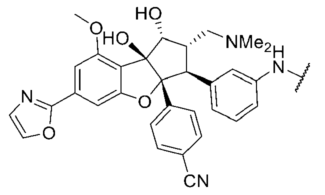 |
| I-A43 | 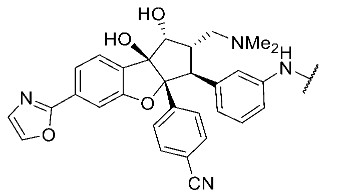 |
| I-A44 | 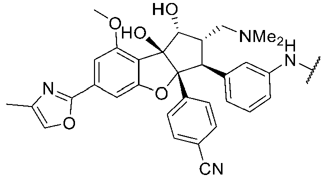 |
| I-A45 | 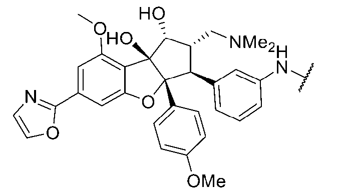 |
| I-A46 | 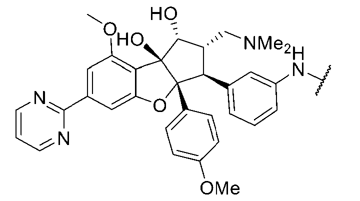 |
| I-A47 | 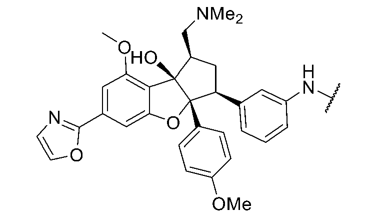 |
| I-A48 | 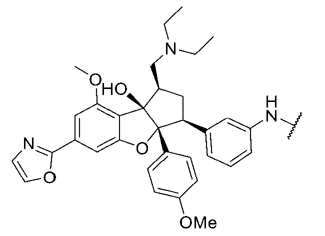 |
| I-A49 | 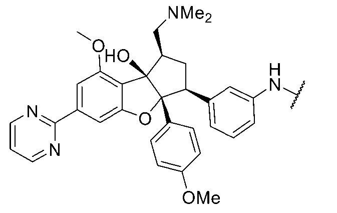 |
| I-A50 | 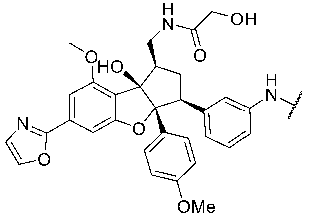 |
| I-A51 | 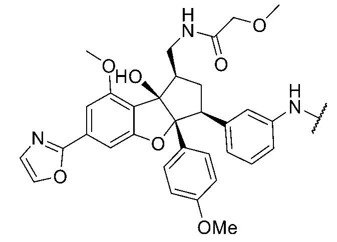 |
| I-A52 | 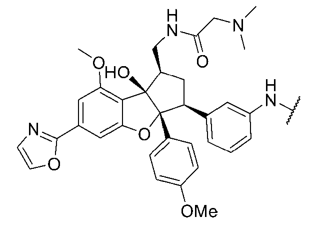 |
| I-A53 | 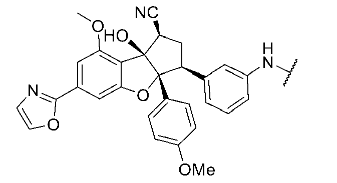 |
| I-A54 | 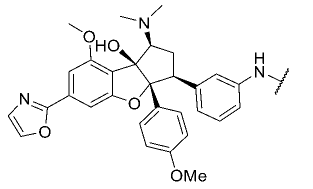 |
| I-A55 | 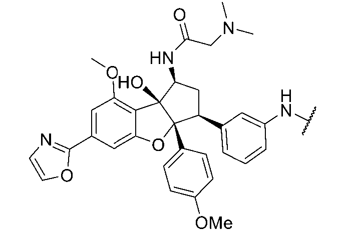 |
| I-A56 | 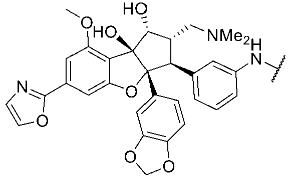 |
| I-A57 | 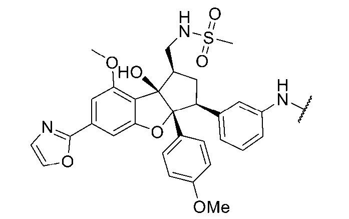 |
| II-A1 | 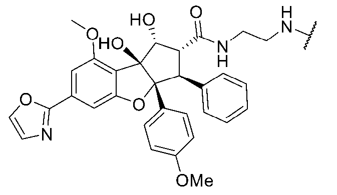 |
| II-A2 | 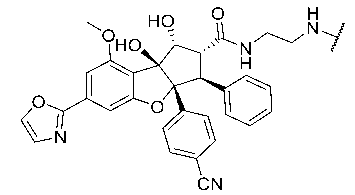 |
| II-A3 | 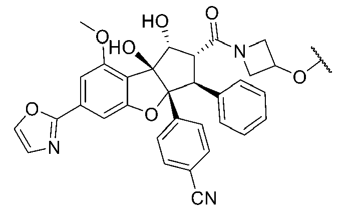 |
| II-A4 | 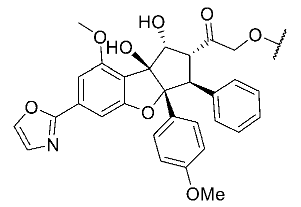 |
| II-A5 | 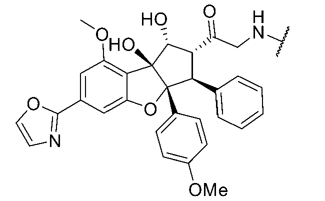 |
| II-A6 | 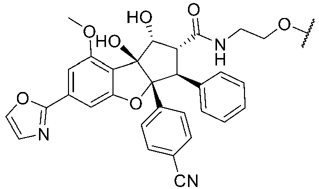 |
| II-A7 | 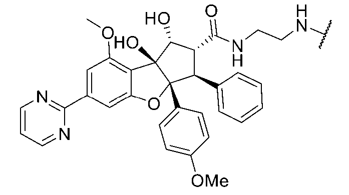 |
| II-A8 | 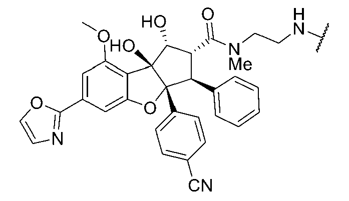 |
| II-A9 | 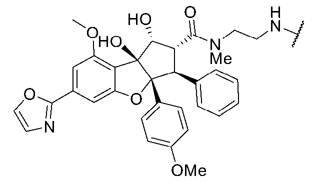 |
| II-A10 | 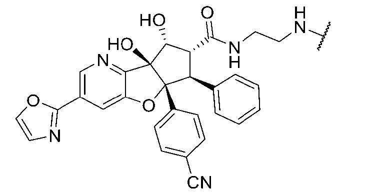 |
| II-A11 | 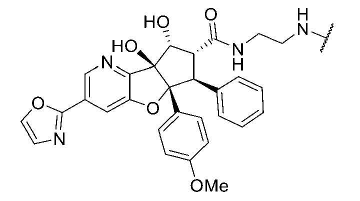 |
| II-A12 | 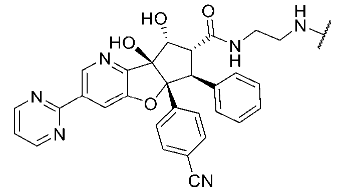 |
| II-A13 | 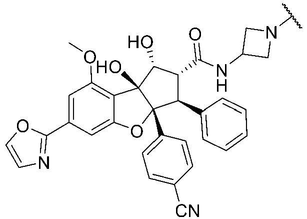 |
| II-A14 | 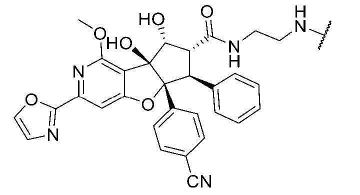 |
| II-A15 | 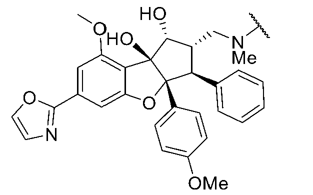 |
| II-A16 | 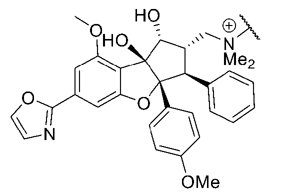 |
| III-A1 | 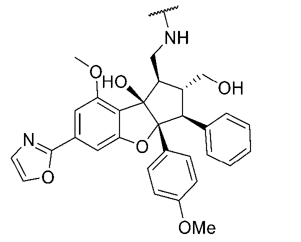 |
| III-A2 | 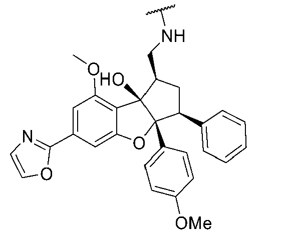 |
| III-A3 | 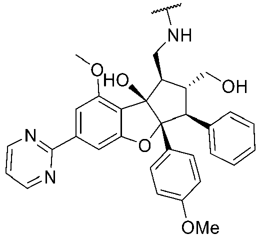 |
| III-A4 | 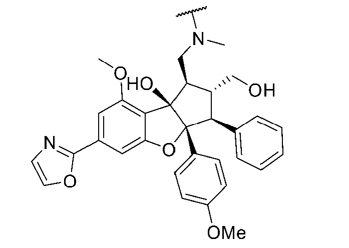 |
| III-A5 | 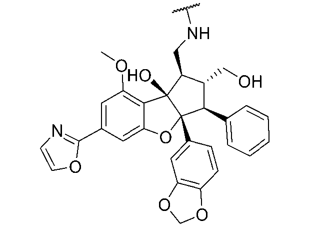 |
| III-A6 | 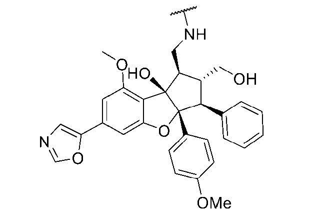 |
| III-A7 | 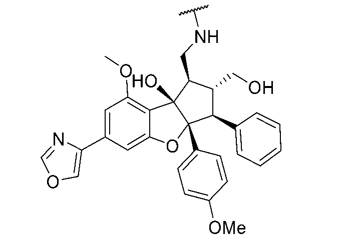 |
| III-A8 | 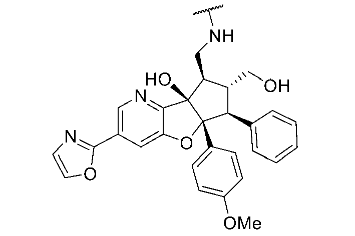 |
| III-A9 | 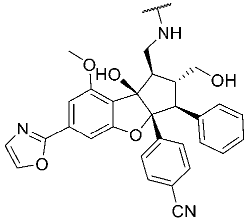 |
| III-A10 | 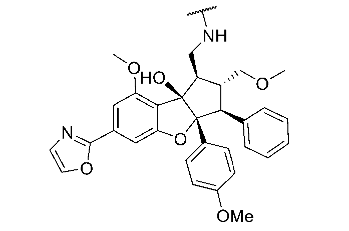 |
| III-A11 | 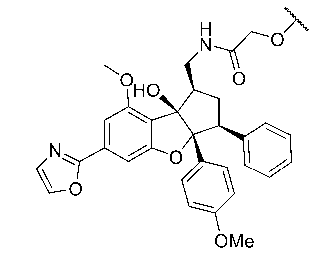 |
| III-A12 | 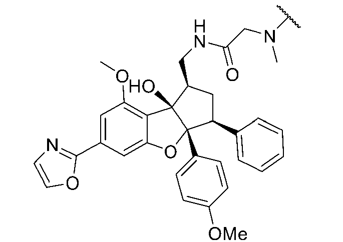 |
| III-A13 | 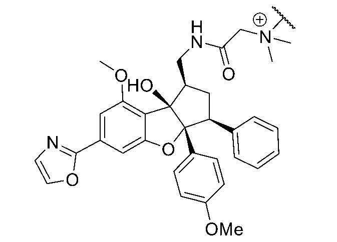 |
| IV-A1 | 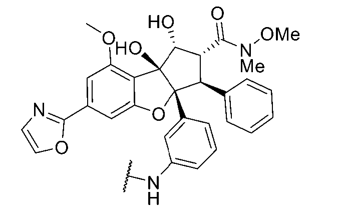 |
| IV-A2 | 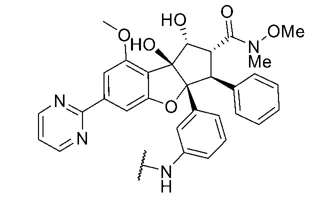 |
| IV-A3 | 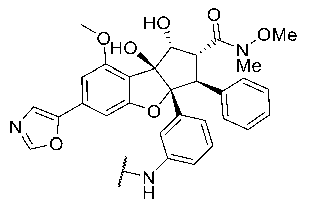 |
| IV-A4 | 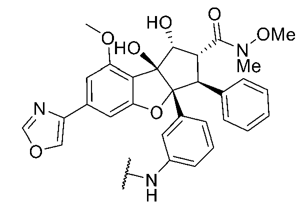 |
| IV-A5 | 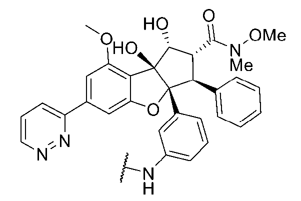 |
| IV-A6 | 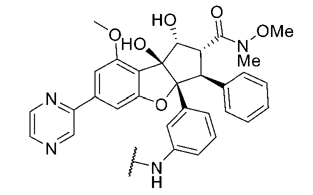 |
| IV-A7 | 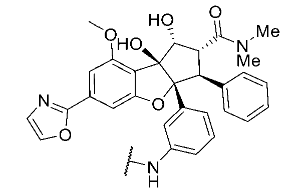 |
| IV-A8 | 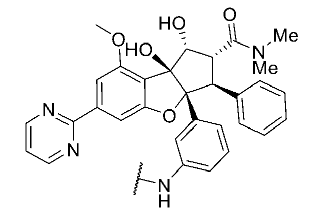 |
| IV-A9 | 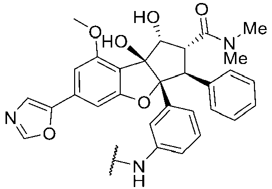 |
| IV-A10 | 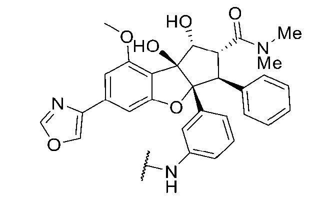 |
| IV-A11 | 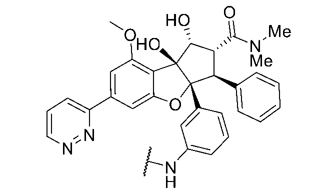 |
| IV-A12 | 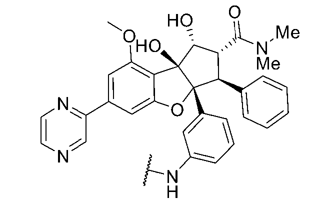 |
| IV-A13 | 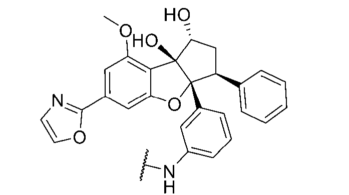 |
| IV-A14 | 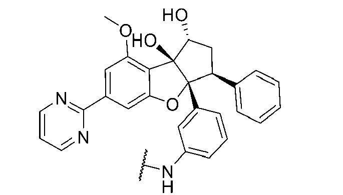 |
| IV-A15 | 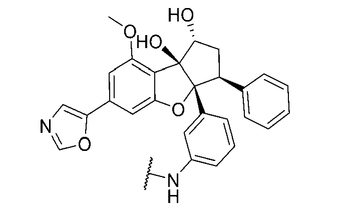 |
| IV-A16 | 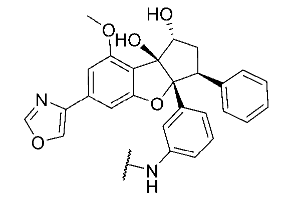 |
| IV-A17 | 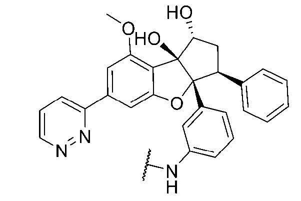 |
| IV-A18 | 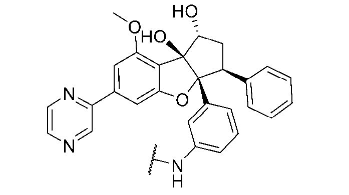 |
| IV-A19 | 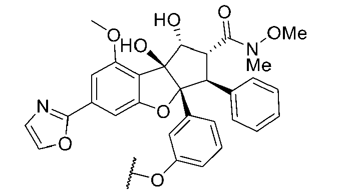 |
| IV-A20 | 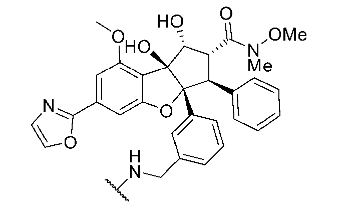 |
| IV-A21 | 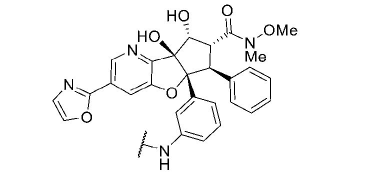 |
| IV-A22 | 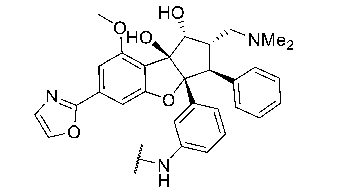 |
| V-A1 | 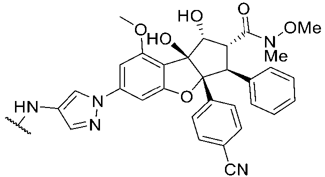 |
| V-A2 | 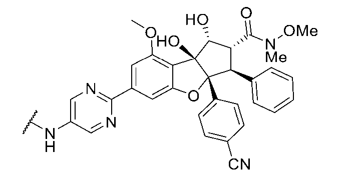 |
| V-A3 | 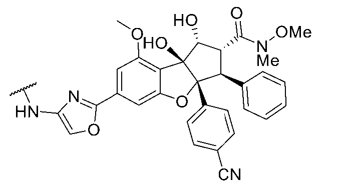 |
| V-A4 | 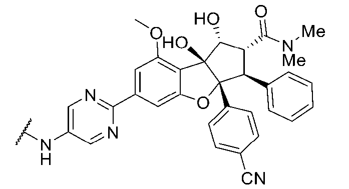 |
| V-A5 | 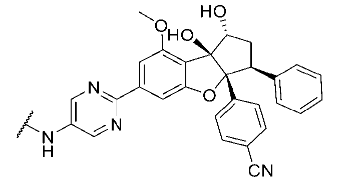 |
| V-A6 | 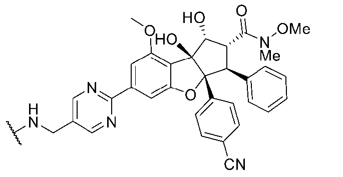 |
| V-A7 | 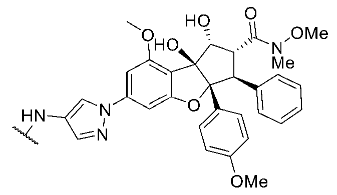 |
| V-A8 | 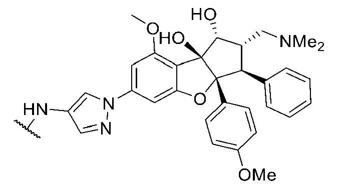 |
| V-A9 | 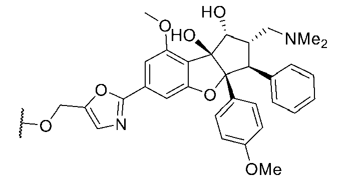 |
| V-A10 | 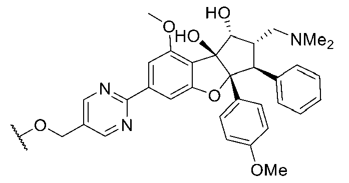 |
| V-A11 | 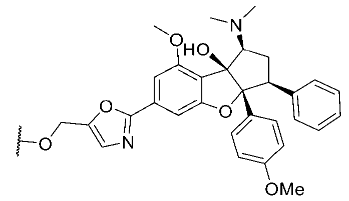 |
| V-A12 | 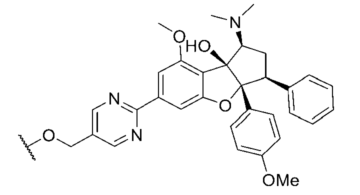 |

In certain embodiments, provided is the ligand-drug conjugate or the racemic mixture, tautomer, stereoisomer, enantiomer, or diastereoisomer thereof, or the mixture thereof, or the pharmaceutically acceptable salt or solvate thereof, wherein the linking group is -Tr-L³-L²-L¹-, wherein:
Tr is absent, or Tr is any group;
L³ is absent or is selected from a polypeptide fragment and a peptide fragment analog;
L² is absent or is selected from a linker fragment;
L¹ is a linking unit.

In certain embodiments, provided is the ligand-drug conjugate or the racemic mixture, tautomer, stereoisomer, enantiomer, or diastereoisomer thereof, or the mixture thereof, or the pharmaceutically acceptable salt or solvate thereof, wherein the linking group is -Tr-L³-L²-L¹-, wherein:
Tr is absent, or Tr is any group;
L³ is absent or is selected from a polypeptide fragment;
L² is absent or is selected from a linker fragment;
L¹ is a linking unit.

In certain embodiments, the linking group and the drug group are linked to form a structure selected from a structure represented by formula I-B: 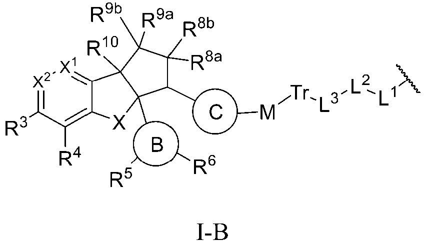 wherein:
X, X¹, X², ring B, ring C, R³, R⁴, R⁵, R⁶, R^{8a}, R^{8b}, R^{9a}, R^{9b}, R¹⁰, M, Tr, L³, L², and L¹ are defined as described in any one of the embodiments of the present disclosure;
the wavy line indicates linking to the ligand by a nitrogen atom or a carbon atom on the L¹ group.

In certain embodiments, the linking group and the drug group are linked to form a structure selected from a structure represented by formula II-B': 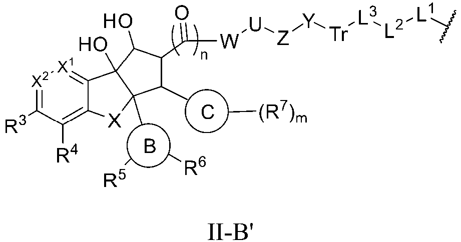 wherein:
X, X¹, X², ring B, ring C, R³, R⁴, R⁵, R⁶, R⁷, m, n, W, U, Z, Y, Tr, L³, L², and L¹ are defined as described in any one of the embodiments of the present disclosure;
the wavy line indicates linking to the ligand by a nitrogen atom or a carbon atom on the L¹ group.

In certain embodiments, the linking group and the drug group are linked to form a structure selected from a structure represented by formula II-B: 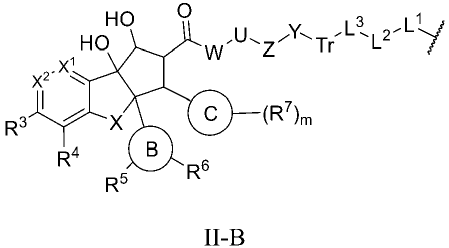 wherein:
X, X¹, X², ring B, ring C, R³, R⁴, R⁵, R⁶, R⁷, m, W, U, Z, Y, Tr, L³, L², and L¹ are defined as described in any one of the embodiments of the present disclosure;
the wavy line indicates linking to the ligand by a nitrogen atom or a carbon atom on the L¹ group.

In certain embodiments, the linking group and the drug group are linked to form a structure selected from a structure represented by formula III-B': 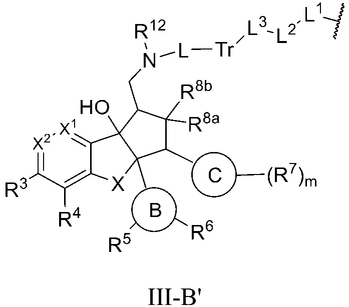 wherein:
X, X¹, X², ring B, ring C, R³, R⁴, R⁵, R⁶, R⁷, R^{8a}, R^{8b}, R¹², L, m, Tr, L³, L², and L¹ are defined as described in any one of the embodiments of the present disclosure;
the wavy line indicates linking to the ligand by a nitrogen atom or a carbon atom on the L¹ group.

In certain embodiments, the linking group and the drug group are linked to form a structure selected from a structure represented by formula III-B: 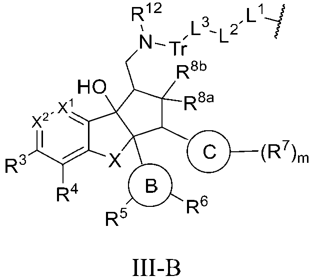 wherein:
X, X¹, X², ring B, ring C, R³, R⁴, R⁵, R⁶, R⁷, R^{8a}, R^{8b}, R¹², m, Tr, L³, L², and L¹ are defined as described in any one of the embodiments of the present disclosure;
the wavy line indicates linking to the ligand by a nitrogen atom or a carbon atom on the L¹ group.

In certain embodiments, the linking group and the drug group are linked to form a structure selected from a structure represented by formula IV-B: 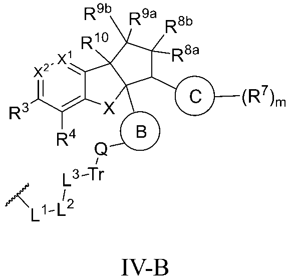 wherein:
X, X¹, X², ring B, ring C, R³, R⁴, R⁷, R^{8a}, R^{8b}, R^{9a}, R^{9b}, R¹⁰, m, Q, Tr, L³, L², and L¹ are defined as described in any one of the embodiments of the present disclosure;
the wavy line indicates linking to the ligand by a nitrogen atom or a carbon atom on the L¹ group.

In certain embodiments, the linking group and the drug group are linked to form a structure selected from a structure represented by formula V-B: 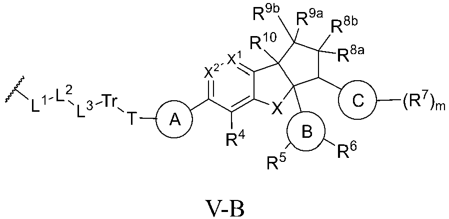 wherein:
X, X¹, X², ring A, ring B, ring C, R⁴, R⁵, R⁶, R⁷, R^{8a}, R^{8b}, R^{9a}, R^{9b}, R¹⁰, m, T, Tr, L³, L², and L¹ are defined as described in any one of the embodiments of the present disclosure;
the wavy line indicates linking to the ligand by a nitrogen atom or a carbon atom on the L¹ group.

In certain embodiments, provided is a structure formed by linking the linking group and the drug group, wherein:
L¹ is a linking unit;
L² is -(C(R^{L21})₂)ᵣ-, wherein r is a natural number from 0 to 50;
any C(R^{L21})₂ unit in L² may be independently replaced with the following structural unit: -Cy-, - C(=O)-, -NR^{L22}-, -O-, -s-, -S(=O)-, -S(=O)₂-, -P(R^{L22})-, -P(=O)(R^{L22})-, -C(=S)-, -C(=NR^{L22})-, _ N=N-, -CH=N-, -N=CH-, 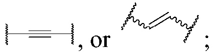 -Cy- is selected from -phenylene-, -(5-8 membered)heteroarylene-, -(4-10 membered)heterocyclylene-, and -(C₃-C₁₀)cycloalkylene-, and -Cy- is unsubstituted, or each -Cy-is independently substituted with one or more R^{Cx};
R^{L21}, R^{L22}, and R^{Cx} are each independently selected from hydrogen, deuterium, halogen, nitro, - CN, -OR^{L2a}, -SR^{L2a}, -N(R^{L2a})₂, -N⁺(R^{L2a})₃, -C(=O)R^{L2a}, -C(=O)OR^{L2a}, -C(=O)-C(=O)R ^{L2a}, - C(=O)CH₂C(=O)R^{L2a}, -S(=O)R^{L2a}, -S(=O)₂R^{L2a}, -C(=O)N(R^{L2a})₂, -S(=O)₂N(R^{L2a})₂, -OC(=O)R^{L2a}, -N(R^{L2a})S(=O)₂R^{L2b}, -N(R^{L2a})C(=O)R^{L2b}, -(CH2)_{y}-C(=O)-(N(Me)CH₂C(=O))ₛ-OR^{L2a}, -(CH₂)_{y}-C(=O)-(N(Me)CH₂C(=O))ₛ-NHR^{L2a}, -(CH₂)_{y}-C(=O)-(N(Me)CH₂C(=O))ₛ-N⁺(R^{L2a})₃, -(CH₂)_{y}-NHC(=O)CH₂(OCH₂CH₂)OR^{L2a}, -(CH₂)_{y}-NH(C(=O)CH₂(N(Me))ₛ-R^{L2a}, -(CH₂)_{y}-C(=O)NH-(CH₂CH₂O)ₛ-R^{L2a}, -(CH₂)_{y}-NHC(=O)-(CH₂CH₂O)ₛ-R ^{L2a}, -(CH₂CH₂O)ₛ-R^{L2a}, - (C(=O)CH₂N(Me))ₛ-R^{L2a}, -C(=O)CH₂(OCH₂CH₂)ₛ-OR^{L2a}, -C(=O)-(CH₂CH₂O)ₘ-R^{L2a}, -C(=O)-(CH₂)_{y}-C(=O)NH-(CH₂CH₂O)ₛ-R^{L2a}, -C(=O)-(CH₂)_{y}-NHC(=O)-(CH₂CH₂O)ₛ-R^{L2a}, -(C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl, wherein the -(C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl are optionally substituted with one or more R^{L2a};
s and y are each independently a natural number from 0 to 50;
R^{L2a} and R^{L2b} are each independently selected from hydrogen, deuterium, halogen, nitro, -CN, - OH, -SH, -NH₂, -N(Me)₂, -C(=O)OH, -S(=O)₂Me, -S(=O)₂OH, -C(=O)NH₂, -S(=O)₂NH₂, -(C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl;
L³ is absent or is selected from an amino acid residue, a short peptide consisting of 2-10 amino acid residues, -C(=O)-(C₁-C₆)alkylene-NH-, 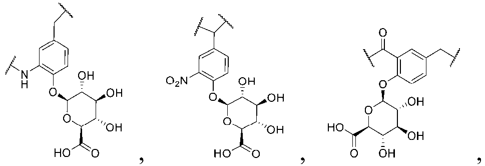 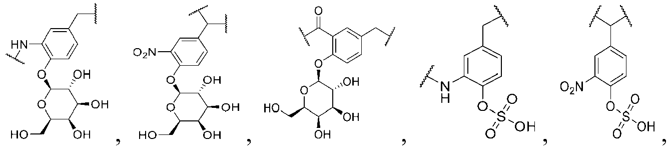 and any combination of the aforementioned groups, wherein the amino acid residues are natural amino acid residues or non-natural amino acid residues, and L³ is optionally modified by one or more protecting groups; in certain embodiments, the protecting groups are selected from a polyethylene glycol fragment and -OtBu, wherein the polyethylene glycol fragment is linked to L³ by the structural unit -NH-; Tr is absent or is selected from 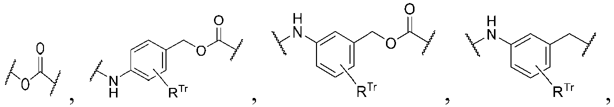 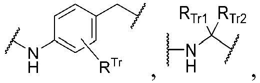 and any combination of the aforementioned groups; R^{Tr}, R^{Tr1}, and R^{Tr2} are each independently selected from hydrogen, deuterium, halogen, nitro, -CN, -OH, -NH₂, -SH, -OC(=O)NH₂, -C(=O)OH, -C(=O)NH₂, -S(=O)₂OH, -S(=O)₂NH₂, -CH₂C(=O)-(N(Me)CH₂C(=O))_{z}-OR^{Tra}, -CH₂C(=O)-(N(Me)CH₂C(=O))_{z}-NHR^{Tra}, -(CH₂CH₂O)_{z}-R^{Tra}, - C(=O)NH-(CH₂CH₂O)_{z}-R^{Tra}, -(C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl, wherein the - (C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl are optionally substituted with one or more R^{Tra};
each R^{Tra} is independently hydrogen, deuterium, halogen, nitro, -CN, -OH, -NH₂, -SH, -N(Me)₂, - C(=O)OH, -C(=O)NH₂, -S(=O)₂OH, -S(=O)₂NH₂, -S(=O)₂Me, -(C₁-C₆)alkyl, -(C₂-C₆)alkenyl, - (C₂-C₆)alkynyl, -(C₃-C₈)cycloalkyl, -(4-10 membered)heterocycloalkyl, -(C₆-C₁₀)aryl, and -(5-10 membered)heteroaryl;
each z is independently a natural number from 0 to 50.

In certain embodiments, provided is a structure formed by linking the linking group and the drug group, wherein:
L¹ is a linking unit;
L² is -(C(R^{L21})₂)ᵣ-, wherein r is a natural number from 0 to 50;
any C(R^{L21})₂ unit in L² may be independently replaced with the following structural unit: -Cy-, - C(=O)-, -NR^{L22}-, -O-, -S-, -S(=O)-, -S(=O)₂-, -P(R^{L22})-, -P(=OXR^{L22})-, -C(=S)-, -C(=NR^{L22})-, - N=N-, -CH=N-, -N=CH-, 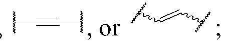 -Cy- is selected from -phenylene-, -(5-8 membered)heteroarylene-, -(4-10 membered)heterocyclylene-, and -(C₃-C₁₀)cycloalkylene-, and -Cy- is unsubstituted, or each -Cy-is independently substituted with one or more R^{Cx};
R^{L21}, R^{L22}, and R^{Cx} are each independently selected from hydrogen, deuterium, halogen, nitro, - CN, -OR^{L2a}, -SR^{L2a}, -N(R^{L2a})₂, -N⁺(R^{L2a})₃, -C(=O)R^{L2a}, -C(=O)OR^{L2a}, -C(=O)-C(=O)R^{L2a}, - C(=O)CH₂C(=O)R^{L2a}, -S(=O)R^{L2a}, -S(=O)₂R^{L2a}, -C(=O)N(R^{L2a})₂, -S(=O)₂N(R^{L2a})₂, -OC(=O)R^{L2a}, -N(R^{L2a})S(=O)₂R^{L2b}, -N(R^{L2a})C(=O)R^{L2b}, -(CH2)_{y}-C(=O)-(N(Me)CH₂C(=O))ₛ-OR^{L2a}, -(CH₂)_{y}-C(=O)-(N(Me)CH₂C(=O))ₛ-NHR^{L2a}, -(CH2)_{y}-C(=O)-(N(Me)CH₂C(=O))ₛ-N⁺(R^{L2a})₃, -(CH₂)_{y}-NHC(=O)CH₂(OCH₂CH₂)OR^{L2a}, -(CH₂)_{y}-NH(C(=O)CH₂(N(Me))ₛ-R^{L2a}, -(CH₂)_{y}-C(=O)NH-(CH₂CH₂O)ₛ-R^{L2a}, -(CH₂)_{y}-NHC(=O)-(CH₂CH₂O)ₛ-R^{L2a}, -(CH₂CH₂O)ₛ-R^{L2a}, - (C(=O)CH₂N(Me))ₛ-R^{L2a}, -C(=O)CH₂(OCH₂CH₂)ₛ-OR^{L2a}, -C(=O)-(CH₂CH₂O)ₘ-R^{L2a}, -C(=O)-(CH₂)_{y}-C(=O)NH-(CH₂CH₂O)ₛ-R^{L2a}, -C(=O)-(CH₂)_{y}-NHC(=O)-(CH₂CH₂O)ₛ-R^{L2a}, -(C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl, wherein the -(C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl are optionally substituted with one or more R^{L2a};
s and y are each independently a natural number from 0 to 50;
R^{L2a} and R^{L2b} are each independently selected from hydrogen, deuterium, halogen, nitro, -CN, - OH, -SH, -NH₂, -N(Me)₂, -C(=O)OH, -S(=O)₂Me, -S(=O)₂OH, -C(=O)NH₂, -S(=O)₂NH₂, -(C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl;
L³ is absent or is selected from an amino acid residue, a short peptide consisting of 2-10 amino acid residues, 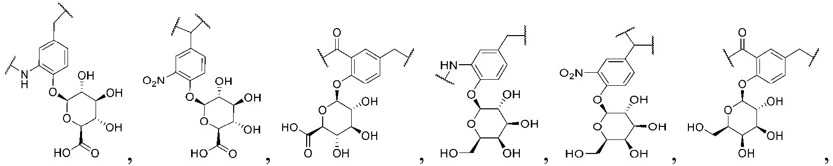 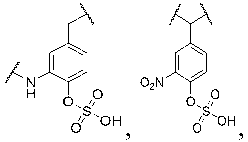 and any combination of the aforementioned groups, wherein the amino acid residues are natural amino acid residues or non-natural amino acid residues, and L³ is optionally modified by one or more protecting groups; in certain embodiments, the protecting groups are selected from a polyethylene glycol fragment and -OtBu, wherein the polyethylene glycol fragment is linked to L³ by the structural unit -NH-;
Tr is absent or is selected from 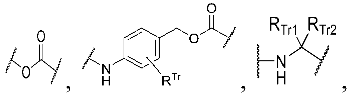 and any combination of the aforementioned groups; R^{Tr}, R^{Tr1}, and R^{Tr2} are each independently selected from hydrogen, deuterium, halogen, nitro, -CN, -OH, -NH₂, -SH, -OC(=O)NH₂, -C(=O)OH, -C(=O)NH₂, -S(=O)₂OH, -S(=O)₂NH₂, -CH₂C(=O)-(N(Me)CH₂C(=O))_{z}-OR^{Tra}, -CH₂C(=O)-(N(Me)CH₂C(=O))_{z}-NHR^{Tra}, -(CH₂CH₂O)_{z}-R^{Tra}, - C(=O)NH-(CH₂CH₂O)_{z}-R^{Tra}, -(C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl, wherein the - (C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl are optionally substituted with one or more R^{Tra};
each R^{Tra} is independently hydrogen, deuterium, halogen, nitro, -CN, -OH, -NH₂, -SH, -N(Me)₂, - C(=O)OH, -C(=O)NH₂, -S(=O)₂OH, -S(=O)₂NH₂, -S(=O)₂Me, -(C₁-C₆)alkyl, -(C₂-C₆)alkenyl, -
(C₂-C₆)alkynyl, -(C₃-C₈)cycloalkyl, -(4-10 membered)heterocycloalkyl, -(C₆-C₁₀)aryl, and -(5-10 membered)heteroaryl;
each z is independently a natural number from 0 to 50.

In certain embodiments, L¹ is selected from carbonyl, -(C₁-C₈)alkylene-C(=O)-, -(4-8 membered)heterocyclylene-, -(5-10 membered)heteroarylene-, and -(C₁-C₈)alkylene-(5-10 membered)heteroarylene-, wherein the -(C₁-C₈)alkylene-C(=O)-, -(4-8 membered)heterocyclylene-, -(5-10 membered)heteroarylene-, and -(C₁-C₈)alkylene-(5-10 membered)heteroarylene- are optionally substituted with 1, 2, or 3 oxo, -(C₁-C₄)alkylene-, and - (C₁-C₄)alkyl groups.

In certain embodiments, L¹ is selected from carbonyl, -(C₁-C₄)alkylene-C(=O)-, -(5-6 membered)heterocyclylene-, -(5-6 membered)heteroarylene-, and -(C₁-C₄)alkylene-(5-6 membered)heteroarylene-, wherein the -(C₁-C₄)alkylene-C(=O)-, -(5-6 membered)heterocyclylene-, -(5-6 membered)heteroarylene-, and -(C₁-C₄)alkylene-(5-6 membered)heteroarylene- are optionally substituted with 1, 2, or 3 oxo, -(C₁-C₄)alkylene-, and - (C₁-C₄)alkyl groups.

In certain embodiments, L¹ is selected from

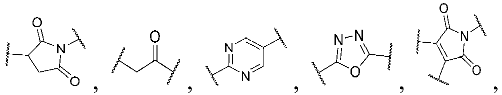

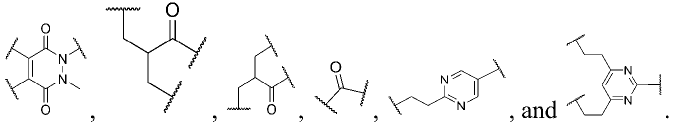

In certain embodiments, L¹ is selected from

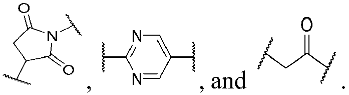

In certain embodiments, L¹ is selected from 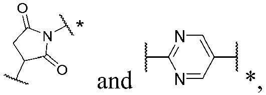 wherein the * end is attached to L².

In certain embodiments, L¹ is selected from 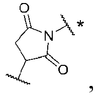 wherein the * end is attached to L². In certain embodiments, L² is -(C(R^{L21})₂)ᵣ-, wherein r is a natural number from 0 to 10 (e.g., 1, 2, 3, 4, 5, 6, 7, 8, or 9);
any C(R^{L21})₂ unit in L² may be independently replaced with the following structural unit: -Cy-, - C(=O)-, -NR^{L22}-, -O-, -S-, or -S(=O)-; -Cy- is selected from -phenylene-, -4-6 membered heterocyclylene-, and -(C₃-C₆)cycloalkylene-, and -Cy- is unsubstituted, or each -Cy- is independently substituted with 1, 2, 3, 4, 6, or 7 R^{Cx}; R^{L21}, R^{L22}, and R^{Cx} are each independently selected from hydrogen, deuterium, halogen, nitro, - CN, -OR^{L2a}, -SR^{L2a}, -N(R^{L2a})2, -C(=O)R^{L2a}, -C(=O)OR^{L2a}, -C(=O)-C(=O)R^{L2a}, - C(=O)CH₂C(=O)R^{L2a}, -S(=O)R^{L2a}, -S(=O)₂R^{L2a}, -C(=O)N(R^{L2a})₂, -S(=O)₂N(R^{L2a})₂, -(CH₂)₂-C(=O)NH-(CH₂CH₂O)ₜ-R^{L2a}, -(C₁-C₆)alkyl, and -(C₂-C₆)alkenyl, wherein the -(C₁-C₆)alkyl and -
(C₂-C₆)alkenyl are optionally substituted with 1, 2, 3, 4, 5, 6, or 7 R^{L2a};
t is a natural number from 0 to 30;
R^{L2a} is selected from hydrogen, deuterium, fluorine, chlorine, bromine, iodine, nitro, -CN, -OH, - SH, -NH₂, -N(Me)₂, -C(=O)OH, -S(=O)₂Me, -S(=O)₂OH, -C(=O)₂OH, -C(=O)NH₂, -S(=O)₂NH₂, methyl, ethyl, and vinyl.

In certain embodiments, L² is -(C(R^{L21})₂)ᵣ-, wherein r is a natural number from 0 to 10 (e.g., 1, 2, 3, 4, 5, 6, 7, or 8);
any C(R^{L21})₂ unit in L² may be independently replaced with the following structural unit: -Cy-, - C(=O)-, -NR^{L22}-, -O-, -S-, or -S(=O)-;
-Cy- is selected from -phenylene-, -4-6 membered heterocyclylene-, and -(C₃-C₆)cycloalkylene-, and -Cy- is unsubstituted, or each -Cy- is independently substituted with 1, 2, 3, 4, 6, or 7 R^{Cx}; R^{L21}, R^{L22}, and R^{Cx}; are each independently selected from hydrogen, deuterium, halogen, nitro, - CN, -OR^{L2a}, -SR^{L2a}, -N(R^{L2a})2, -C(=O)R^{L2a}, -C(=O)OR^{L2a}, -C(=O)-C(=O)R^{L2a}, - C(=O)CH₂C(=O)R^{L2a}, -S(=O)R^{L2a}, -S(=O)₂^{RL2a}, -C(=O)N(R^{L2a})₂, -S(=O)₂N(R^{L2a})₂, -(CH₂)₂-C(=O)NH-(CH₂CH₂O)ₜ-R^{L2a}, -(C₁-C₆)alkyl, and -(C₂-C₆)alkenyl, wherein the -(C₁-C₆)alkyl and -
(C₂-C₆)alkenyl are optionally substituted with 1, 2, 3, 4, 5, 6, or 7 R^{L2a}; t is a natural number from 0 to 30;
R^{L2a} is selected from hydrogen, deuterium, fluorine, chlorine, bromine, iodine, nitro, -CN, -OH, - SH, -NH₂, -N(Me)₂, -C(=O)OH, -S(=O)₂Me, -S(=O)₂OH, -C(=O)₂OH, -C(=O)NH₂, -S(=O)₂NH₂, methyl, ethyl, and vinyl.

In certain embodiments, L² is -(C(R^{L21})₂)ᵣ-, wherein r is 2, 3, 4, 5, 6, 7, 8, or 9, and any C(R^{L21})₂ unit in L² may be independently replaced with the following structural unit: -Cy-, -C(=O)-, -NR^{L22}-, -O-, -S-, or -S(=O)-;
-Cy- is selected from cyclopropylene, cyclobutylene, cyclopentylene, azetidinylene, pyrrolidinylene, piperazinylene, or piperidinylene, and -Cy- is unsubstituted, or each -Cy- is independently substituted with 1, 2, 3, 4, or 5 R^{Cx}; for example, -Cy- is 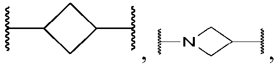 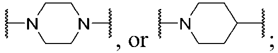
R^{L21}, R^{L22}, and R^{Cx} are each independently selected from hydrogen, deuterium, fluorine, chlorine, bromine, iodine, nitro, -CN, -OH, -SH, -NH₂, -C(=O)OH, -C(=O)NH, -(CH₂)₂-C(=O)NH-(CH₂CH₂O)₁₁-CH₃, methyl, ethyl, propyl, deuterated methyl, deuterated ethyl, halomethyl, haloethyl, methyl-OH, ethyl-OH, propyl-OH, vinyl-OH, vinyl, deuterated vinyl, and halovinyl. In certain embodiments, L² is -(C(R^{L21})₂)ᵣ-, wherein r is 2, 3, 4, 5, 6, 7, or 8;
any C(R^{L21})₂ unit in L² may be independently replaced with the following structural unit: -Cy-, - C(=O)-, -NR^{L22}-, -O-, -S-, or -S(=O)-;
-Cy- is selected from cyclopropylene, cyclobutylene, cyclopentylene, azetidinylene, pyrrolidinylene, piperazinylene, or piperidinylene, and -Cy- is unsubstituted, or each -Cy- is independently substituted with 1, 2, 3, 4, or 5 R^{Cx}; for example, -Cy- is 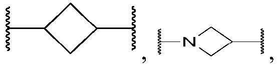 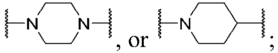
R^{L21} R^{L22}, and R^{Cx} are each independently selected from hydrogen, deuterium, fluorine, chlorine, bromine, iodine, nitro, -CN, -OH, -SH, -NH₂, -C(=O)OH, -C(=O)NH, -(CH₂)₂-C(=O)NH-(CH₂CH₂O)₁₁-CH₃, methyl, ethyl, propyl, deuterated methyl, deuterated ethyl, halomethyl, haloethyl, methyl-OH, ethyl-OH, propyl-OH, vinyl-OH, vinyl, deuterated vinyl, and halovinyl.

In certain embodiments, L² is selected from

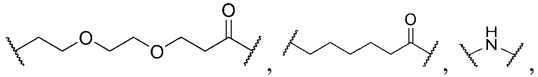

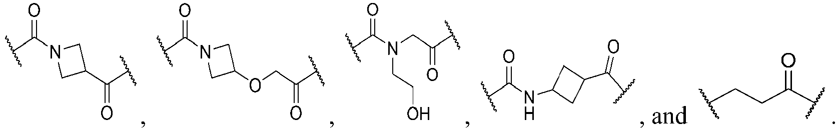

In certain embodiments, L² is selected from

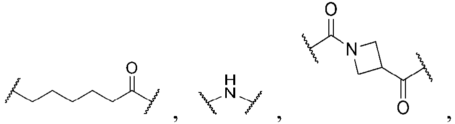

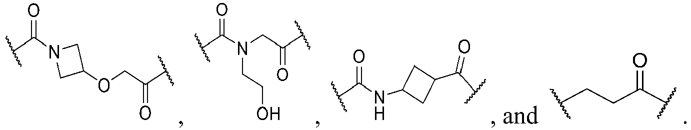

In certain embodiments, L² is selected from 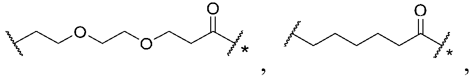 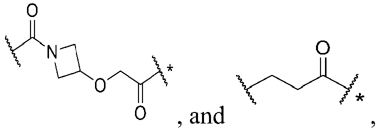 wherein the * end is attached to L₃.

In certain embodiments, L² is selected from 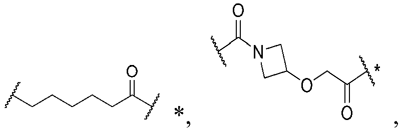 and 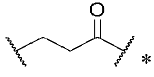 , wherein the * end is attached to L₃.

In certain embodiments, L² is selected from 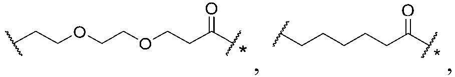 and 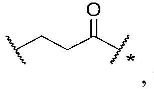 wherein the * end is attached to L₃.

In certain embodiments, L² is selected from 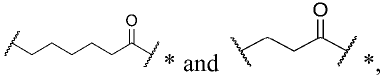 wherein the * end is attached to L₃.

In certain embodiments, -L¹-L²- is selected from

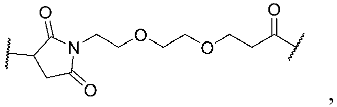

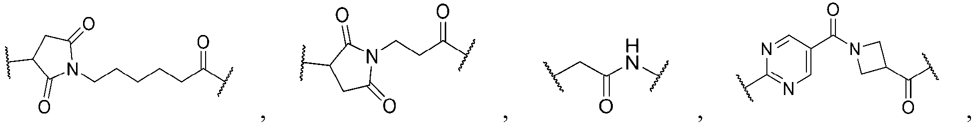

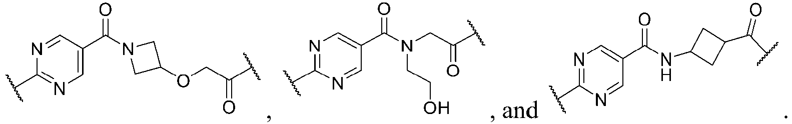

In certain embodiments, -L¹-L²- is selected from 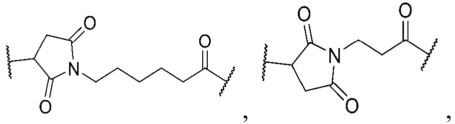 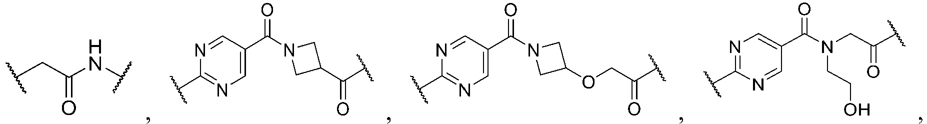 and

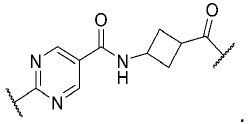

In certain embodiments, -L¹-L²- is selected from 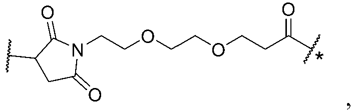 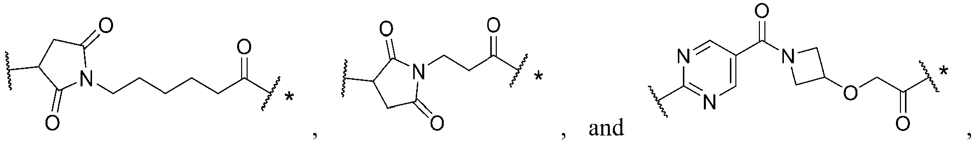 wherein the * end is attached to L₃.

In certain embodiments, -L¹-L²- is selected from 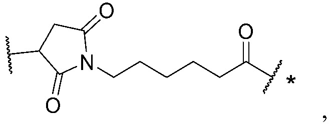 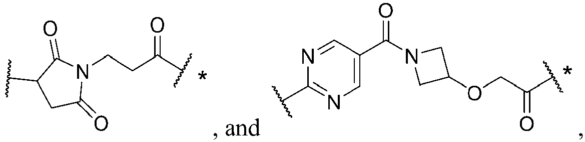 wherein the * end is attached to L₃.

In certain embodiments, -L¹-L²- is selected from 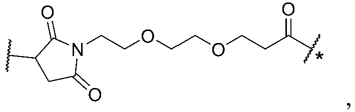 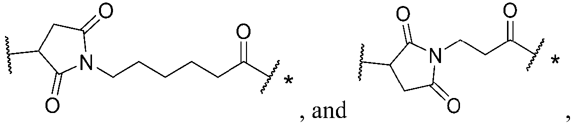 wherein the * end is attached to L₃.

In certain embodiments, -L¹-L²- is selected from 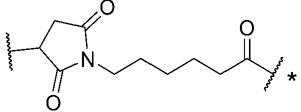 and 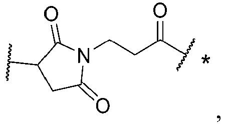 wherein the * end is attached to L₃.

In certain embodiments, the amino acid residue is selected from Val, D-Val, Phe, Lys, Leu, Ile, Gly, Ala, D-Ala, Cit, Asp, Asn, Glu, Gln, Arg, and Ser;
the short peptide consisting of 2-10 amino acid residues is selected from -Arg-Val-, -Arg-Leu-, - Val-Cit-, -Val-Ala-, -Val-Lys-, -Val-Lys(AC)-, -Phe-Lys-, -Phe-Lys(AC)-, -Leu-Lys-, -Leu-Lys(AC)-, -Ala-Ala-, -Ala-Lys-, -(D-Ala)-Ala-, -Gly-Glu-, -Gly-Asp-, -Gly-Asn-, -Val-Glu-, -Val-Asp-, -Asn-Asn-, -Asp-Asp-, -Asp-Glu-, -Ser-Val-, -Ser-Ala-, -Ser-Gly-, -Ser-Glu-, -Ser-Gln-, - Ser-Asp-, -Gly-Gln-, -Asn-Gly-, -Asn-Ala-, -Ala-Ala-Glu-, -Gly-Gly-Glu-, -Gly-Gly-Asp-, -Gly-Gly-Asn-, -Gly-Ala-Ala-, -Gly-Val-Ala-, -Gly-Val-Cit-, -Glu-Val-Cit-, -Ala-Ala-Ala-, -Ala-(D-Ala)-Ala-, -Ala-Ala-Asn-, -Ala-(D-Ala)-Asn-, -Ala-Ala-Asp-, -Val-Lys-Gly-, -(D-Val)-Leu-Lys-, -Gly-Gly-Arg-, -Gly-Gly-Gly-, -Lys-Ala-Asn-, -Gly-Phe-Gly-, -Gly-Gly-Phe-, -Asn-Pro-Val-, - Ala-Lys-Gly-, -Gly-Lys-Gly-, -Gly-Gly-Gly-Gly- (SEQ ID NO: 61), -Gly-Gly-Phe-Gly- (SEQ ID NO: 62), -Gly-Gly-Glu-Gly- (SEQ ID NO: 63), -Lys-Ala-Ala-Asn- (SEQ ID NO: 64), -Lys-Ala-Ala-Asp- (SEQ ID NO: 65), -Ala-Ala-Pro-Val- (SEQ ID NO: 66), and -Ala-Ala-Pro-Nva- (SEQ ID NO: 67);
the polyethylene glycol fragment is selected from -(CH₂CH₂O)ₜ-R^{L3}, wherein R^{L3} is -(C₁-C₆)alkyl, and t is an integer from 6 to 30.

In certain embodiments, the amino acid residue is selected from Val, D-Val, Phe, Lys, Leu, Ile, Gly, Ala, D-Ala, Cit, Asp, Asn, Glu, Gln, Arg, and Ser;
the short peptide consisting of 2-10 amino acid residues is selected from -Arg-Val-, -Arg-Leu-, - Val-Cit-, -Val-Ala-, -Val-Lys-, -Val-Lys(AC)-, -Phe-Lys-, -Phe-Lys(AC)-, -Leu-Lys-, -Leu-Lys(AC)-, -Ala-Ala-, -Ala-Lys-, -(D-Ala)-Ala-, -Gly-Glu-, -Gly-Asp-, -Gly-Asn-, -Val-Glu-, -Val-Asp-, -Asn-Asn-, -Asp-Asp-, -Asp-Glu-, -Ser-Ala-, -Ser-Gly-, -Ser-Glu-, -Ser-Asp-, -Gly-Gln-, - Ala-Ala-Glu-, -Gly-Gly-Glu-, -Gly-Gly-Asp-, -Gly-Gly-Asn-, -Gly-Ala-Ala-, -Gly-Val-Ala-, - Gly-Val-Cit-, -Glu-Val-Cit-, -Ala-Ala-Ala-, -Ala-(D-Ala)-Ala-, -Ala-Ala-Asn-, -Ala-(D-Ala)-Asn-, -Ala-Ala-Asp-, -Val-Lys-Gly-, -(D-Val)-Leu-Lys-, -Gly-Gly-Arg-, -Gly-Gly-Gly-, -Lys-Ala-Asn-, -Gly-Phe-Gly-, -Gly-Gly-Phe-, -Asn-Pro-Val-, -Ala-Lys-Gly-, -Gly-Lys-Gly-, -Gly-Gly-Gly-Gly-, -Gly-Gly-Phe-Gly-, -Gly-Gly-Glu-Gly-, -Lys-Ala-Ala-Asn-, -Lys-Ala-Ala-Asp-, - Ala-Ala-Pro-Val-, and -Ala-Ala-Pro-Nva-;
the polyethylene glycol fragment is selected from -(CH₂CH₂O)ₜ-R^{L3}, wherein R^{L3} is -(C₁-C₆)alkyl, and t is an integer from 6 to 30.

In certain embodiments, L³ is selected from Val, D-Val, Phe, Lys, Leu, Ile, Gly, Ala, D-Ala, Cit, Asp, Asn, Glu, Gln, Arg, Ser, -Arg-Val-, -Arg-Leu-, -Val-Cit-, -Val-Ala-, -Val-Lys-, -Val-Lys(AC)-, -Phe-Lys-, -Phe-Lys(AC)-, -Leu-Lys-, -Leu-Lys(AC)-, -Ala-Ala-, -Ala-Lys-, -(D-Ala)-Ala-, -Gly-Glu-, -Gly-Asp-, -Gly-Asn-, -Val-Glu-, -Val-Asp-, -Asn-Asn-, -Asp-Asp-, -Asp-Glu-, -Ser-Val-, - Ser-Ala-, -Ser-Gly-, -Ser-Glu-, -Ser-Gln-, -Ser-Asp-, -Gly-Gln-, -Asn-Gly-, -Asn-Ala-, -Ala-Ala-Glu-, -Gly-Gly-Glu-, -Gly-Gly-Asp-, -Gly-Gly-Asn-, -Gly-Ala-Ala-, -Gly-Val-Ala-, -Gly-Val-Cit-, -Glu-Val-Cit-, -Ala-Ala-Ala-, -Ala-(D-Ala)-Ala-, -Ala-Ala-Asn-, -Ala-(D-Ala)-Asn-, -Ala-Ala-Asp-, -Val-Lys-Gly-, -(D-Val)-Leu-Lys-, -Gly-Gly-Arg-, -Gly-Gly-Gly-, -Lys-Ala-Asn-, -Gly-Phe-Gly-, -Gly-Gly-Phe-, -Asn-Pro-Val-, -Ala-Lys-Gly-, -Gly-Lys-Gly-, -Gly-Gly-Gly-Gly-, - Gly-Gly-Phe-Gly-, -Gly-Gly-Glu-Gly-, -Lys-Ala-Ala-Asn-, -Lys-Ala-Ala-Asp-, -Ala-Ala-Pro-Val-, -Ala-Ala-Pro-Nva-, 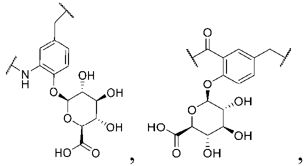 -C(=O)-(C₁-C₄)alkylene-NH-, and any combination of the aforementioned groups, wherein Asp and Glu are each independently and optionally modified by -(CH₂CH₂O)ₜ-R^{L3}, wherein -(CH₂CH₂O)ₜ-R^{L3} is linked to Asp or Glu by the structural unit -NH-, R^{L3} is -(C₁-C₄)alkyl, and t is an integer from 6 to 30 (e.g., 8, 9, 10, 11, 12, 13, 14, 15, 20, 21, 22, 23, 24, or 25).

In certain embodiments, L³ is selected from Val, D-Val, Phe, Lys, Leu, Ile, Gly, Ala, D-Ala, Cit, Asp, Asn, Glu, Gln, Arg, -Arg-Val-, -Arg-Leu-, -Val-Cit-, -Val-Ala-, -Val-Lys-, -Val-Lys(AC)-, - Phe-Lys-, -Phe-Lys(AC)-, -Leu-Lys-, -Leu-Lys(AC)-, -Ala-Ala-, -Ala-Lys-, -D-Ala-Ala-, -Gly-Glu-, -Gly-Asp-, -Gly-Asn-, -Val-Glu-, -Val-Asp-, -Asn-Asn-, -Asp-Asp-, -Asp-Glu-, -Ser-Ala-, - Ser-Gly-, -Ser-Glu-, -Ser-Asp-, -Gly-Gln-, -Ala-Ala-Glu-, -Gly-Gly-Glu-, -Gly-Gly-Asp-, -Gly-Gly-Asn-, -Gly-Ala-Ala-, -Gly-Val-Ala-, -Gly-Val-Cit-, -Glu-Val-Cit-, -Ala-Ala-Ala-, -Ala-(D-Ala)-Ala-, -Ala-Ala-Asn-, -Ala-(D-Ala)-Asn-, -Ala-Ala-Asp-, -Val-Lys-Gly-, -D-Val-Leu-Lys-, - Gly-Gly-Arg-, -Gly-Gly-Gly-, -Lys-Ala-Asn-, -Gly-Phe-Gly-, -Gly-Gly-Phe-, -Asn-Pro-Val-, - Ala-Lys-Gly-, -Gly-Lys-Gly-, -Gly-Gly-Gly-Gly-, -Gly-Gly-Phe-Gly-, -Gly-Gly-Glu-Gly-, -Lys-Ala-Ala-Asn-, -Lys-Ala-Ala-Asp-, -Ala-Ala-Pro-Val-, -Ala-Ala-Pro-Nva-, 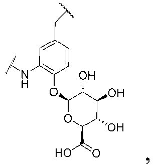 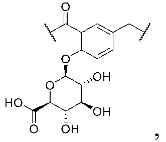 and any combination of the aforementioned groups, wherein Asp and Glu are each independently and optionally modified by -(CH₂CH₂O)ₜR^{L3}, wherein (CH₂CH₂O)ₜ-R^{L3} is linked to Asp or Glu by the structural unit -NH-, R^{L3} is -(C₁-C₄)alkyl, and t is an integer from 6 to 30 (e.g., 8, 9, 10, 11, 12, 13, 14, 15, 20, 21, 22, 23, 24, or 25).

In certain embodiments, L³ is selected from -Val-Cit-, -Arg-Val-, -Ala-Ala-, -Val-Ala-, -Gly-Glu-, -Ser-Val-, -Ser-Ala-, -Ser-Gly-, -Ser-Glu-, -Ser-Gln-, -Ser-Asp-, -Gly-Gln-, -Gly-Asp-, -Gly-Asn-, -Asp-Asp-, -Asp-Glu-, -Asn-Gly-, -Asn-Ala-, -Ala-Ala-Ala-, -Ala-Ala-Glu-, -Gly-Ala-Ala-, -Gly-Gly-Glu-, -Gly-Gly-Phe-Gly-, 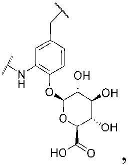 and -C(=O)-(C₁-C₄)alkylene-NH-, wherein Asp and Glu are each independently and optionally modified by -(CH₂CH₂O)ₜ-R^{L3}, wherein (CH₂CH₂O)ₜ-R^{L3} is linked to Asp or Glu by the structural unit -NH-, R^{L3} is -(C₁-C₄)alkyl, and t is an integer from 6 to 30 (e.g., 8, 9, 10, 11, 12, 13, 14, 15, 20, 21, 22, 23, 24, or 25).

In certain embodiments, L³ is selected from -Val-Cit-, -Arg-Val-, -Ala-Ala-, -Val-Ala-, -Gly-Glu-, -Ser-Ala-, -Ser-Gly-, -Ser-Glu-, -Ser-Asp-, -Gly-Gln-, -Gly-Asp-, -Gly-Asn-, -Asp-Asp-, -Asp-Glu-, -Ala-Ala-Ala-, -Ala-Ala-Glu-, -Gly-Ala-Ala-, -Gly-Gly-Glu-, -Gly-Gly-Phe-Gly-, or 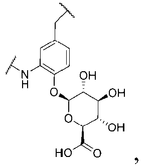 wherein Asp and Glu are each independently and optionally modified by - (CH₂CH₂O)ₜ-R^{L3}, wherein (CH₂CH₂O)ₜ-R^{L3} is linked to Asp or Glu by the structural unit -NH-, R^{L3} is -(C₁-C₄)alkyl, and t is an integer from 6 to 30 (e.g., 8, 9, 10, 11, 12, 13, 14, 15, 20, 21, 22, 23, 24, or 25).

In certain embodiments, L³ is selected from -Val-Cit-, -Arg-Val-, -Ala-Ala-, -Val-Ala-, -Gly-Glu-, -Ser-Val-, -Ser-Ala-, -Ser-Gly-, -Ser-Glu-, -Ser-Gln-, -Ser-Asp-, -Gly-Gln-, -Asn-Gly-, -Asn-Ala-, -Ala-Ala-Glu-, -Gly-Ala-Ala-, -Gly-Gly-Glu-, -Gly-Gly-Phe-Gly-, -Ala-Ala-Glu(NH-(CH₂CH₂O)ₜ-R^{L3})-, and -C(=O)-CH₂CH₂-NH-, wherein R^{L3} is -(C₁-C₄)alkyl, and t is an integer from 6 to 30; an example is -Ala-Ala-Glu(NH-(CH₂CH₂O)₁₁-CH₃)- or -Ala-Ala-Glu(NH-(CH₂CH₂O)₂₄-CH₃)-.

In certain embodiments, L³ is selected from -Val-Cit-, -Arg-Val-, -Ala-Ala-, -Gly-Glu-, -Ser-Ala-, -Ser-Gly-, -Ser-Glu-, -Ser-Asp-, -Gly-Gln-, -Ala-Ala-Glu-, -Gly-Ala-Ala-, -Gly-Gly-Glu-, -Gly-Gly-Phe-Gly-, and -Ala-Ala-Glu(NH-(CH₂CH₂O)ₜ-R^{L3})-, wherein R^{L3} is -(C₁-C₄)alkyl, and t is an integer from 6 to 30; an example is -Ala-Ala-Glu(NH-(CH₂CH₂O)₁₁-CH₃)- or -Ala-Ala-Glu(NH-(CH₂CH₂O)₂₄-CH₃)-.

In certain embodiments, L³ is selected from -Val-Cit-*, *-Ala-Ala-, -Val-Ala-*, *-Gly-Glu-, *-Ser-Val-, *-Ser-Ala-, *-Ser-Gly-, *-Ser-Glu-, *-Ser-Gln-, *-Ser-Asp-, -Gly-Gln-*, *-Gly-Gln-, *-Asn-Gly-, *-Asn-Ala-, *-Gly-Ala-Ala-, *-Gly-Gly-Glu-, -Gly-Gly-Phe-Gly-*, *-Ala-Ala-Glu(NH-(CH₂CH₂O)₁₁-CH₃)-, and 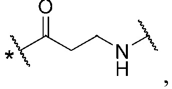 wherein the * end is attached to Tr. In certain embodiments, L³ is selected from -Val-Cit-*, *-Ala-Ala-, *-Gly-Glu-, *-Ser-Ala-, *-Ser-Gly-, *-Ser-Glu-, *-Ser-Asp-, -Gly-Gln-*, *-Gly-Ala-Ala-, *-Gly-Gly-Glu-, -Gly-Gly-Phe-Gly-*, and *-Ala-Ala-Glu(NH-(CH₂CH₂O)₁₁-CH₃)-, wherein the * end is attached to Tr.

In certain embodiments, L³ is selected from -Val-Cit-*, *-Ala-Ala-, -Val-Ala-*, *-Gly-Glu-, *-Ser-Val-, *-Ser-Ala-, *-Ser-Gly-, *-Ser-Glu-, *-Ser-Gln-, *-Ser-Asp-, -Gly-Gln-*, *-Gly-Gln-, *-Asn-Gly-, *-Asn-Ala-, *-Gly-Ala-Ala-, *-Gly-Gly-Glu-, -Gly-Gly-Phe-Gly-*, *Ala-Ala-C(=O)-CH[CH₂CH₂-C(=O)-NH-(CH₂CH₂O)₁₁-CH₃]-NH-, and 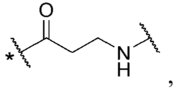 wherein the * end is attached to Tr.

In certain embodiments, L³ is selected from -Val-Cit-*, *-Ala-Ala-, *-Gly-Glu-, *-Ser-Ala-, *-Ser-Gly-, *-Ser-Glu-, *-Ser-Asp-, -Gly-Gln-*, *-Gly-Ala-Ala-, *-Gly-Gly-Glu-, -Gly-Gly-Phe-Gly-*, and *-Ala-Ala-C(=O)-CH[CH₂CH₂-C(=O)-NH-(CH₂CH₂O)₁₁-CH₃]-NH-, wherein the * end is attached to Tr.

In certain embodiments, L³ is selected from -Glu-Ser-, -Ala-Ser-, -Gly-Ser-, and -Asp-Ser-.

In certain embodiments, L³ is selected from *-Ala-Ala-, *-Gly-Glu-, and *-Gly-Gly-Glu-, wherein the * end is attached to Tr.

In certain embodiments, L³ is selected from *-Ala-Ala- and 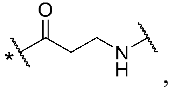 wherein the * end is attached to Tr.

In certain embodiments, L³ is selected from 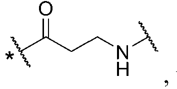 wherein the * end is attached to Tr.

In certain embodiments, Tr is absent or is selected from 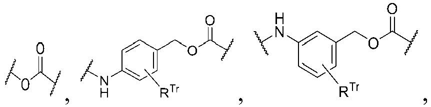 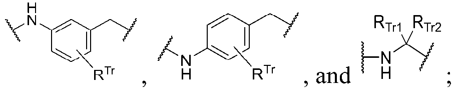 R^{Tr}, R^{Tr1}, and R^{Tr2} are each independently selected from hydrogen, deuterium, halogen, nitro, -CN, -OH, -NH₂, -SH, -OC(=O)NH₂, -C(=O)OH, -C(=O)NH₂, -S(=O)₂OH, -S(=O)₂NH₂, -(CH₂CH₂O)_{z}-R^{Tra}, -C(=O)NH-(CH₂CH₂O)_{z}-R^{Tra}, gluconolactone, glucuronic acid, -(C₁-C₄)alkyl, and -(C₂-C₄)alkenyl, wherein the -(C₁-C₄)alkyl and -(C₂-C₄)alkenyl are optionally substituted with 1, 2, 3, 4, or 5 R^{Tra};
each R^{Tra} is independently hydrogen, deuterium, halogen, nitro, -CN, -OH, -NH₂, -SH, -N(Me)₂, - C(=O)OH, -C(=O)NH₂, -S(=O)₂OH, -S(=O)₂NH₂, -S(=O)₂Me, -(C₁-C₄)alkyl, or -(C₂-C₄)alkenyl; each z is independently an integer from 6 to 30.

In certain embodiments, Tr is absent or is selected from 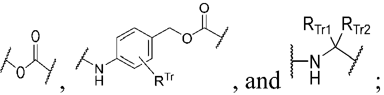 R^{Tr}, R^{Tr1}, and R^{Tr2} are each independently selected from hydrogen, deuterium, halogen, nitro, -CN, -OH, -NH₂, -SH, -OC(=O)NH₂, -C(=O)OH, -C(=O)NH₂, -S(=O)₂OH, -S(=O)₂NH₂, -(CH₂CH₂O)_{z}-R^{Tra}, -C(=O)NH-(CH₂CH₂O)_{z}-R^{Tra}, -(C₁-C₄)alkyl, and -(C₂-C₄)alkenyl, wherein the -(C₁-C₄)alkyl and -(C₂-C₄)alkenyl are optionally substituted with 1, 2, 3, 4, or 5 R^{Tra};
each R^{Tra} is independently hydrogen, deuterium, halogen, nitro, -CN, -OH, -NH₂, -SH, -N(Me)₂, - C(=O)OH, -C(=O)NH₂, -S(=O)₂OH, -S(=O)₂NH₂, -S(=O)₂Me, -(C₁-C₄)alkyl, or -(C₂-C₄)alkenyl; each z is independently an integer from 6 to 30.

In certain embodiments, R^{Tr}, R^{Tr1}, and R^{Tr2} are each independently selected from hydrogen, deuterium, fluorine, chlorine, bromine, iodine, nitro, -CN, -OH, -NH₂, -SH, -OC(=O)NH₂, - C(=O)OH, -C(=O)NH₂, -S(=O)₂OH, -S(=O)₂NH₂, -(CH₂CH₂O)_{z}-R^{Tra}, -C(=O)NH-(CH₂CH₂O)_{z}-R^{Tra}, gluconolactone, glucuronic acid, methyl, ethyl, -ethylene-OH, and -CH₂-COOH;
each R^{Tra} is independently hydrogen, deuterium, fluorine, chlorine, bromine, iodine, -CN, -OH, - NH₂, -SH, -N(Me)₂, -C(=O)OH, -C(=O)NH₂, methyl, or ethyl;
each z is independently an integer from 6 to 30 (e.g., 8, 9, 10, 11, 12, 13, 14, 15, 20, 21, 22, 23, 24, or 25).

In certain embodiments, R^{Tr}, R^{Tr1}, and R^{Tr2} are each independently selected from hydrogen, deuterium, fluorine, chlorine, bromine, iodine, nitro, -CN, -OH, -NH₂, -SH, -OC(=O)NH₂, - C(=O)OH, -C(=O)NH₂, -S(=O)₂OH, -S(=O)₂NH₂, -(CH₂CH₂O)_{z}-R^{Tra}, -C(=O)NH-(CH₂CH₂O)_{z}-R^{Tra}, methyl, ethyl, -ethylene-OH, and -CH₂-COOH;
each R^{Tra} is independently hydrogen, deuterium, fluorine, chlorine, bromine, iodine, -CN, -OH, - NH₂, -SH, -N(Me)₂, -C(=O)OH, -C(=O)NH₂, methyl, or ethyl;
each z is independently an integer from 6 to 30 (e.g., 8, 9, 10, 11, 12, 13, 14, 15, 20, 21, 22, 23, 24, or 25).

In certain embodiments, R^{Tr} is selected from hydrogen, -CH₂-COOH, and

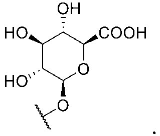

In certain embodiments, R^{Tr} is selected from hydrogen and -CH₂-COOH.

In certain embodiments, Tr is absent or is selected from

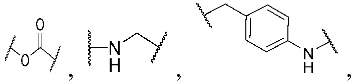

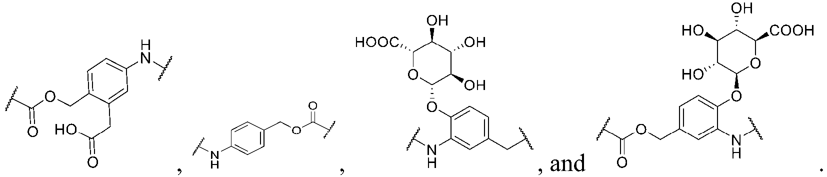

In certain embodiments, Tr is absent or is selected from 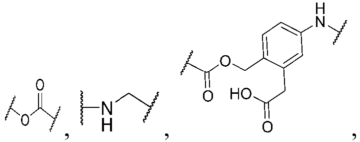 and

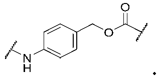

In certain embodiments, Tr is absent or is selected from 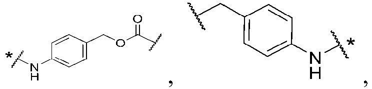 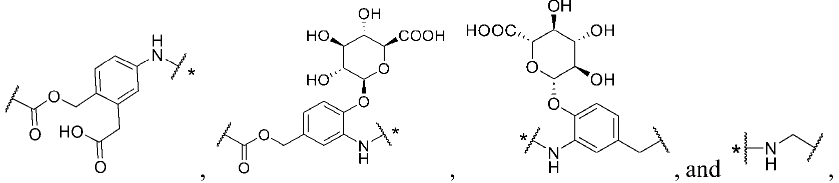 wherein the * end is attached to L³.

In certain embodiments, Tr is absent or is selected from 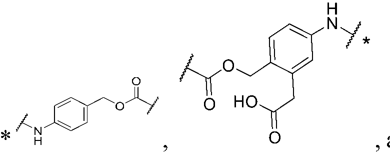 and 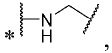 wherein the * end is attached to L₃.

In certain embodiments, Tr is absent or is 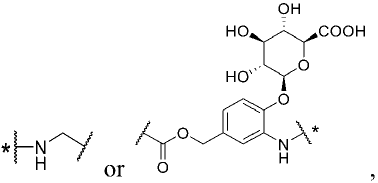 wherein the * end is attached to L₃.

In certain embodiments, Tr is absent or is 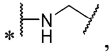 wherein the * end is attached to L³.

In certain embodiments, -L³-L²-L¹- is selected from 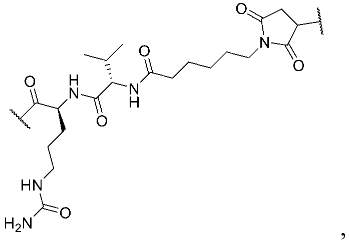 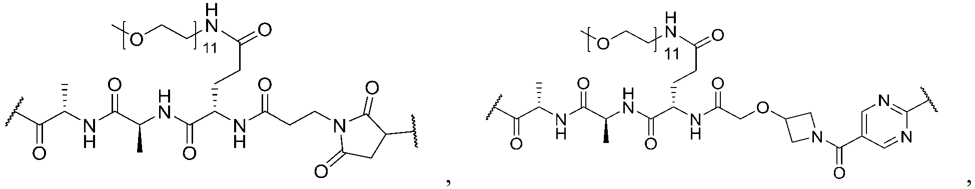 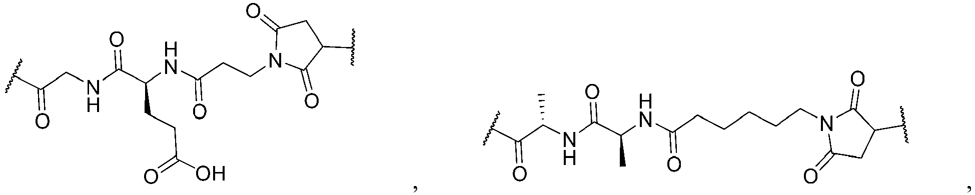 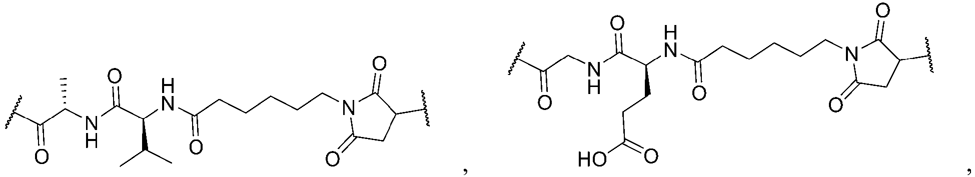 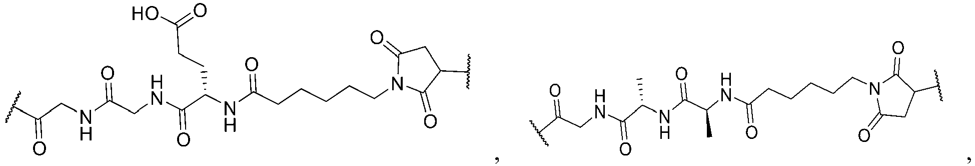 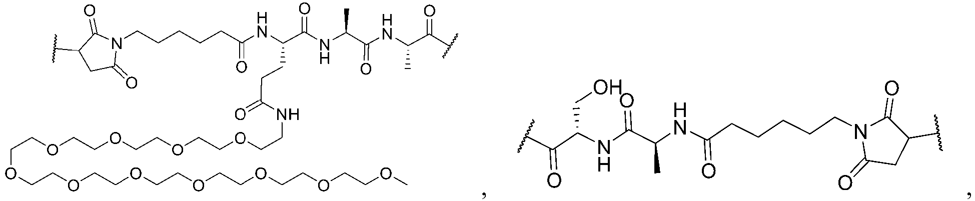 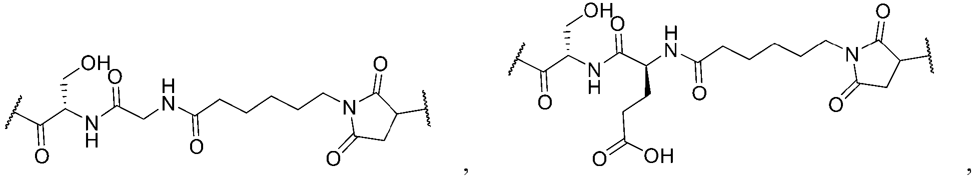 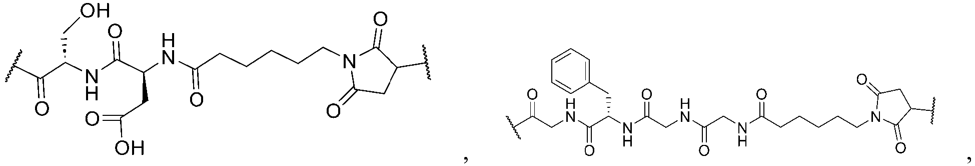 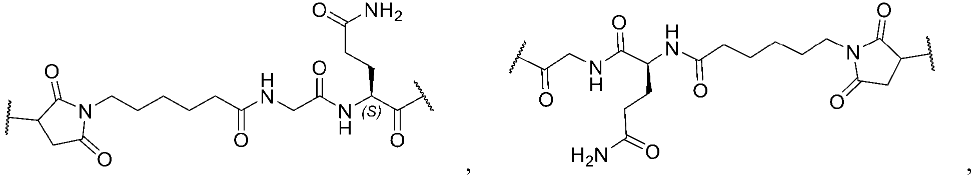 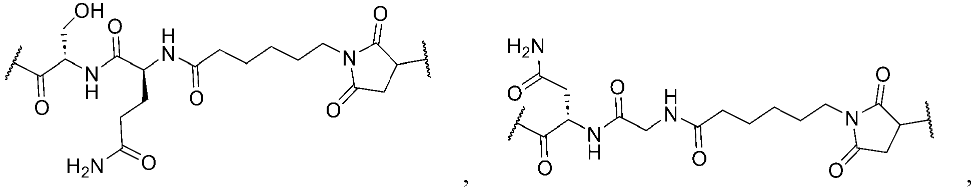 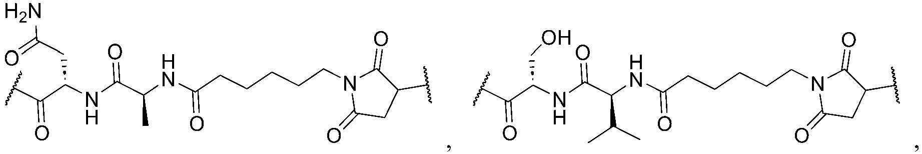 and

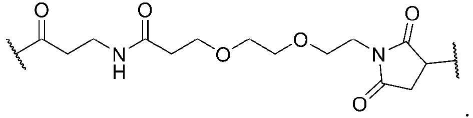

In certain embodiments, -L³-L²-L¹- is selected from 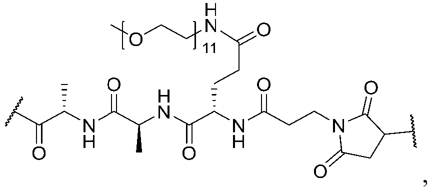 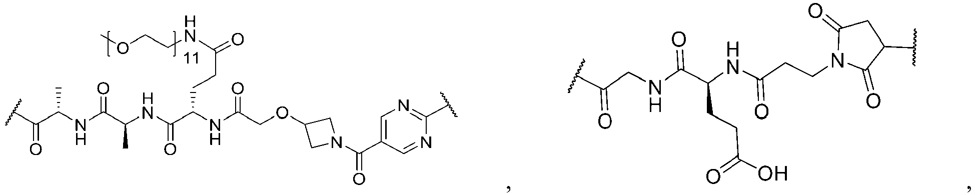 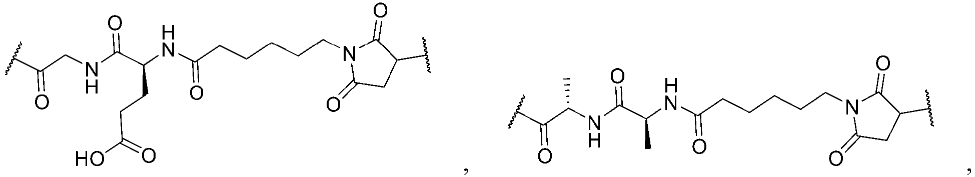 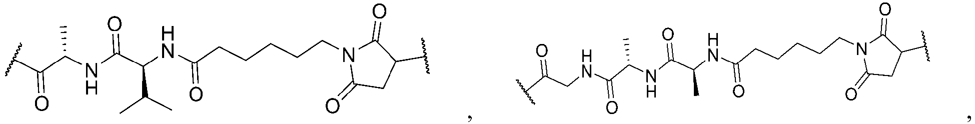 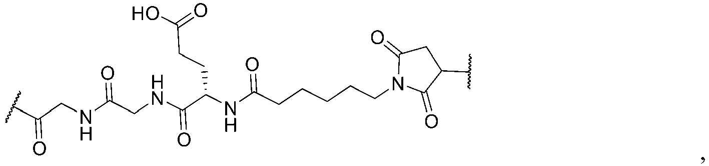 and

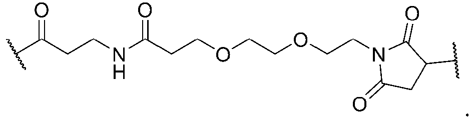

In certain embodiments, -L³-L²-L¹- is selected from 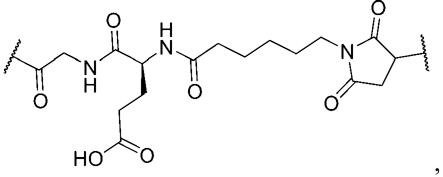 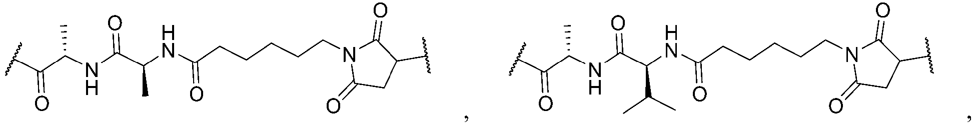 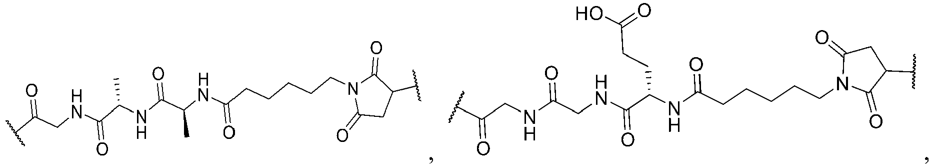 and

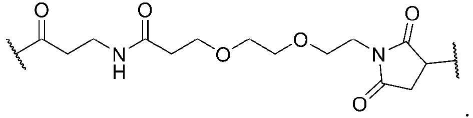

In certain embodiments, -L³-L²-L¹- is selected from

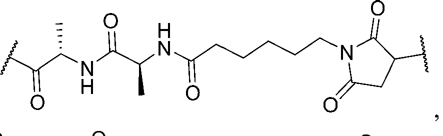

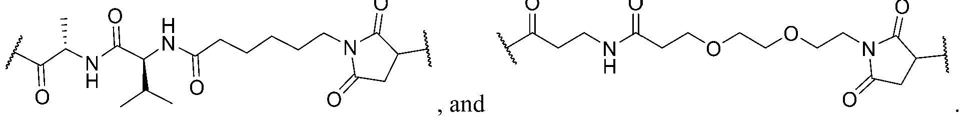

In certain embodiments, -Tr-L³-L²-L¹- is selected from 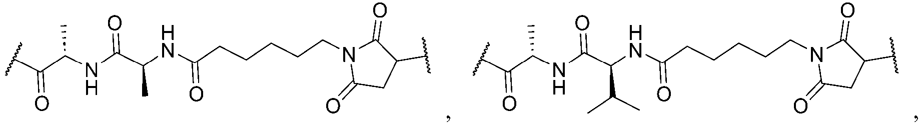 and

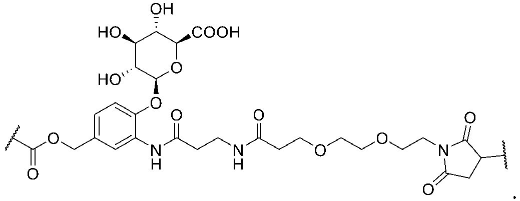

In certain embodiments, -Tr-L³-L²-L¹- is

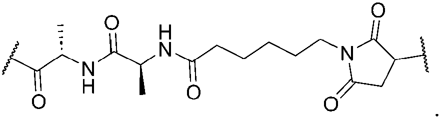

In certain embodiments, -Tr-L³-L²-L¹- is

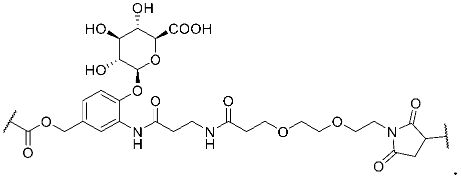

In certain embodiments, -L¹-L²-L³- is selected from

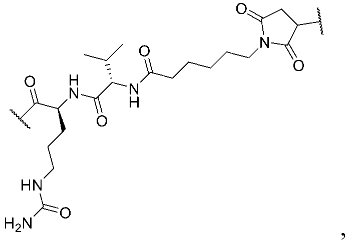

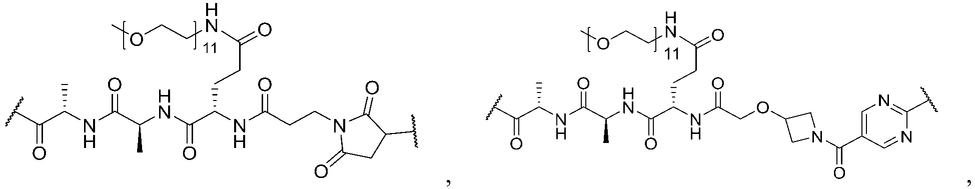

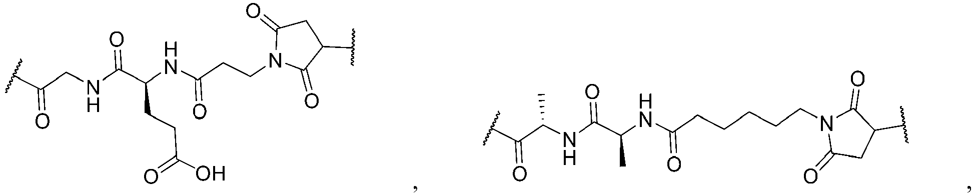

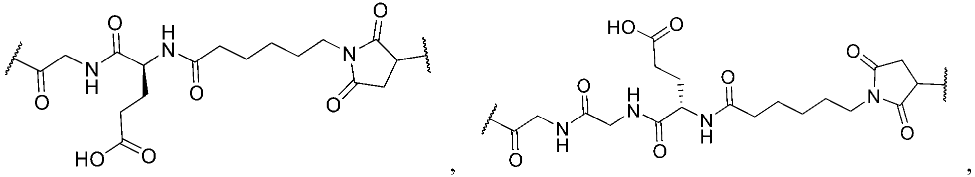

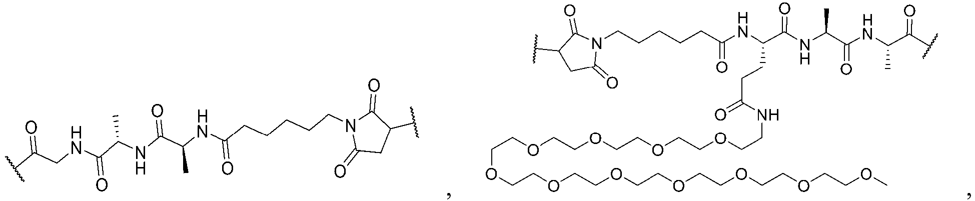

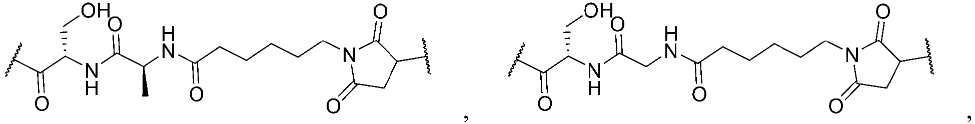

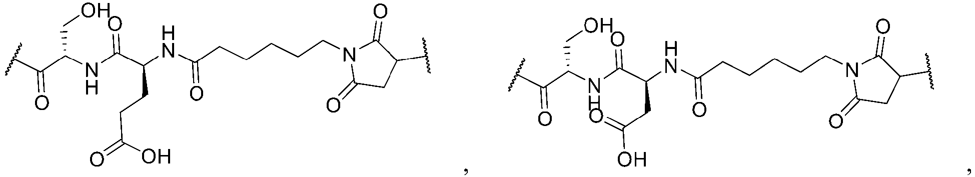

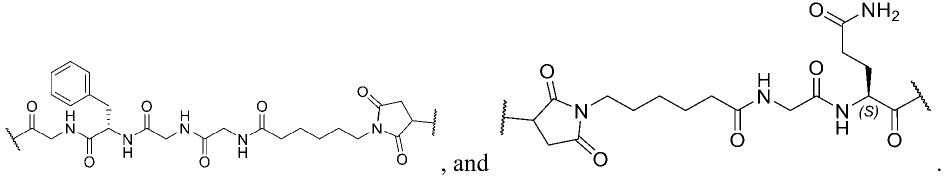

In certain embodiments, -L¹-L²-L³- is selected from

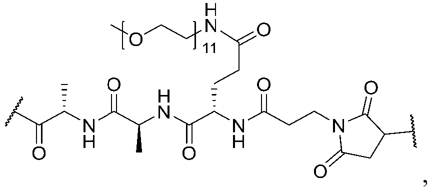

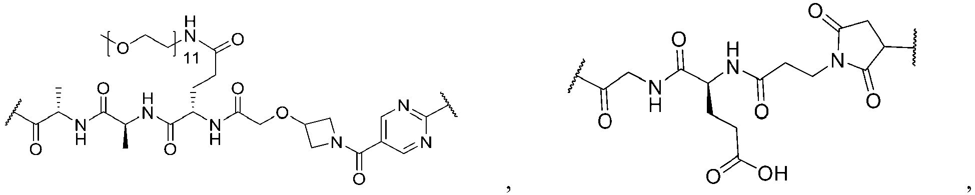

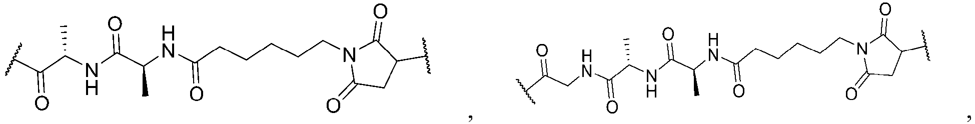

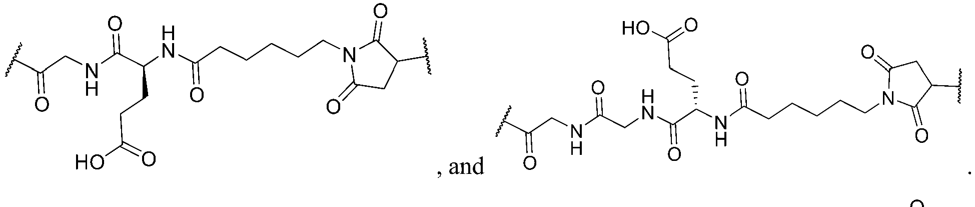

In certain embodiments, -L¹-L²-L³- is selected from

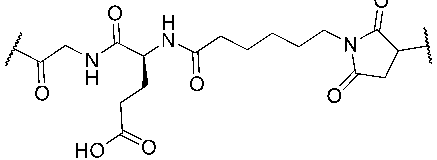

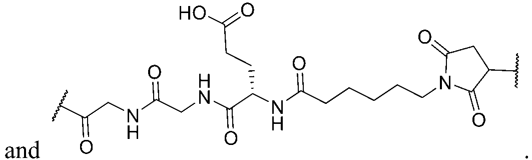

In certain embodiments, -L¹-L²-L³- is selected from 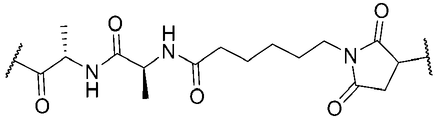 and

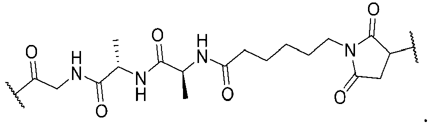

In certain embodiments, the linking group and the drug group are linked to form a structure selected from a structure represented by formula I-B-1, II-B-1', II-B-1, III-B-1', III-B-1, IV-B-1, or V-B-1: 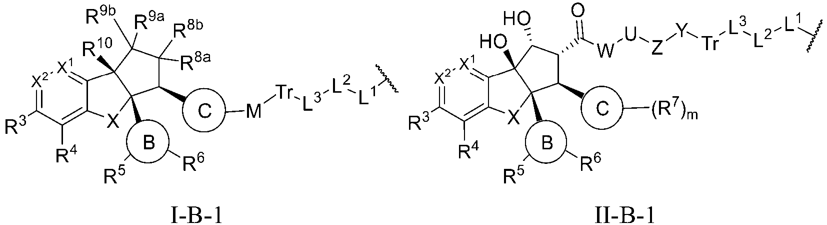 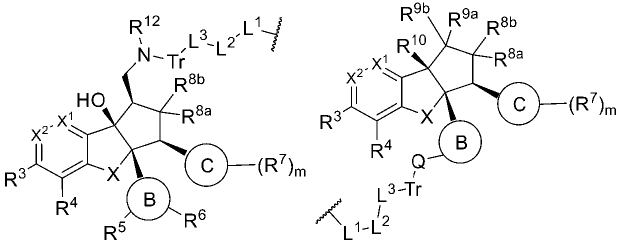 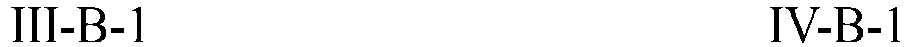 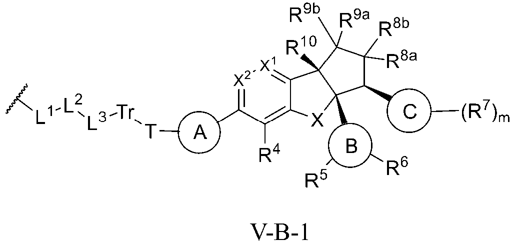 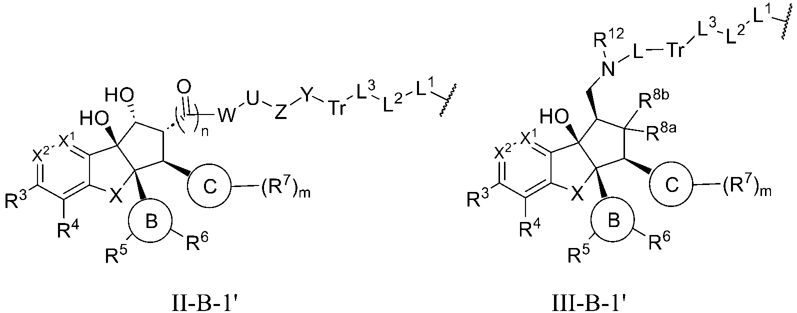 wherein X, X¹, X², ring A, ring B, ring C, R³, R⁴, R⁵, R⁶, R⁷, R^{8a}, R^{8b}, R^{9a}, R^{9b}, R¹⁰, R¹², m, n, M, W, U, Z, Y, L, Q, T, Tr, L¹, L², and L³ are defined as described in any one of the embodiments of the present disclosure.

In certain embodiments, the linking group and the drug group are linked to form a structure selected from a structure represented by formula I-B-2, II-B-2', II-B-2, III-B-2', III-B-2, IV-B-2, or V-B-2: 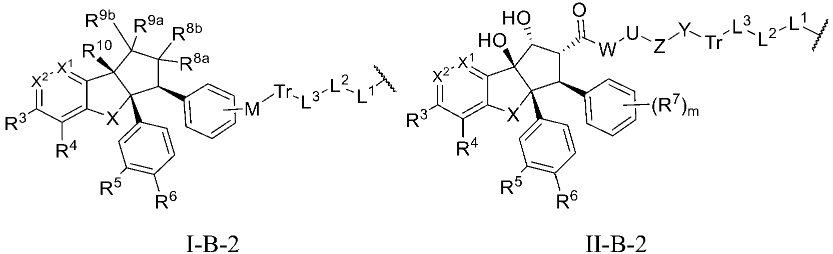 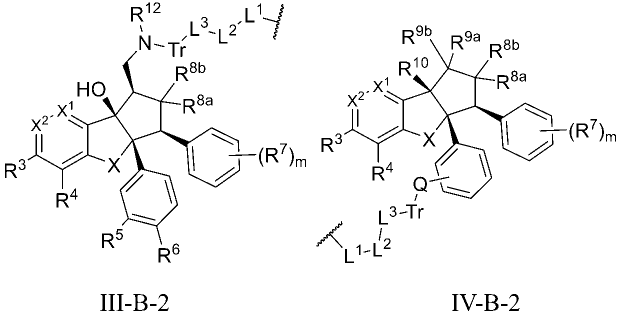 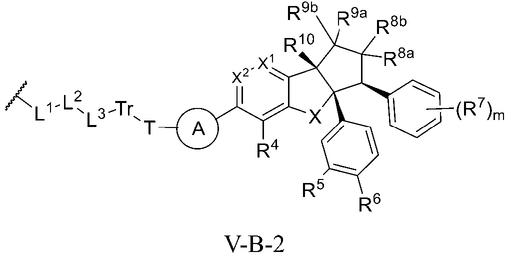 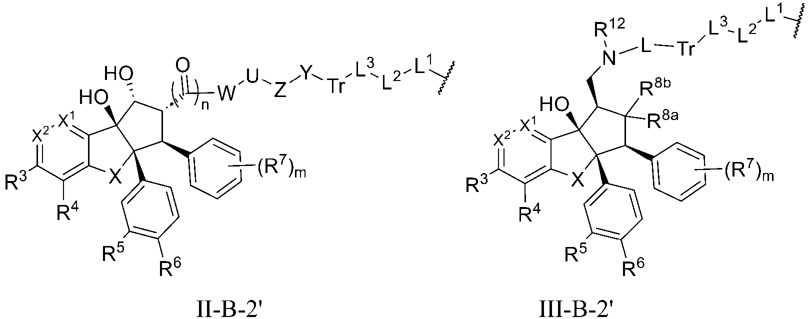 wherein X, X¹, X² , ring A, R³, R⁴, R⁵, R⁶, R⁷, R^{8a}, R^{8b}, R^{9a}, R^{9b}, R¹⁰, R¹², m, n, M, W, U, Z, Y, L, Q, T, Tr, L¹, L², and L³ are defined as described in any one of the embodiments of the present disclosure.

In certain embodiments, the linking group and the drug group are linked to form a structure selected from a structure represented by formula I-B-3, III-B-3', III-B-3, IV-B-3, or V-B-3: 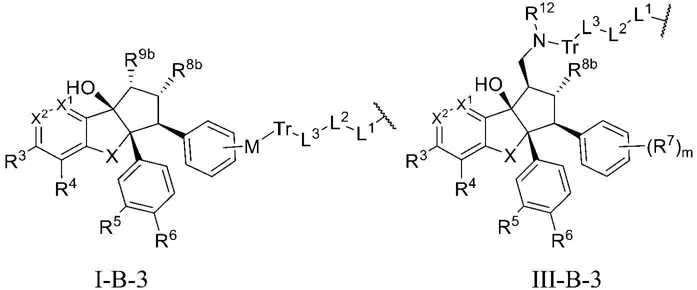 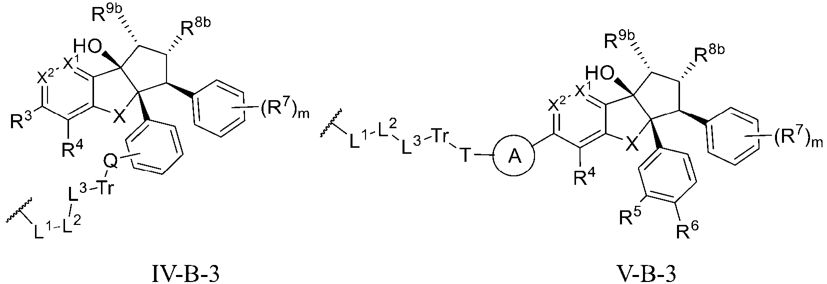 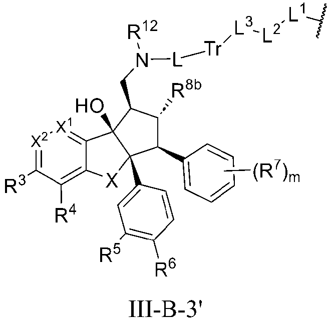 wherein X, X¹, X², ring A, R³, R⁴, R⁵, R⁶, R⁷, R^{8b}, R^{9b}, R¹², m, M, L, Q, T, Tr, L¹, L², and L³ are defined as described in any one of the embodiments of the present disclosure.

In certain embodiments, the linking group and the drug group are linked to form a structure selected from a structure represented by formula I-B-4, II-B-4', II-B-4, III-B-4', III-B-4, IV-B-4, or V-B-4: 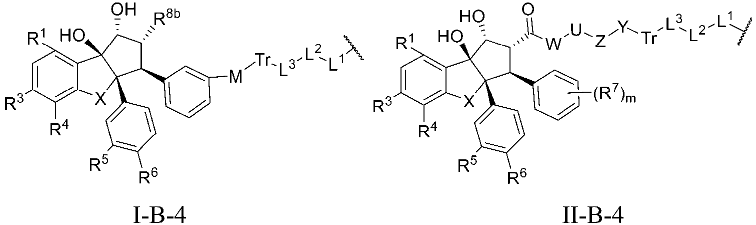 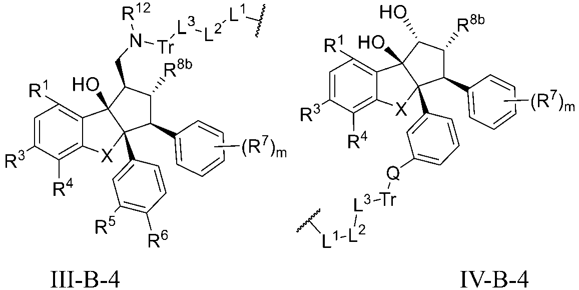 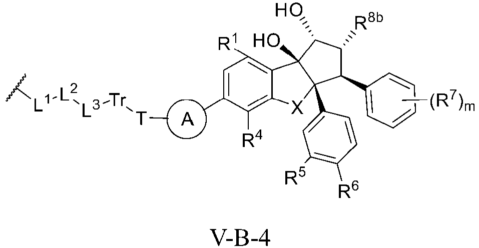 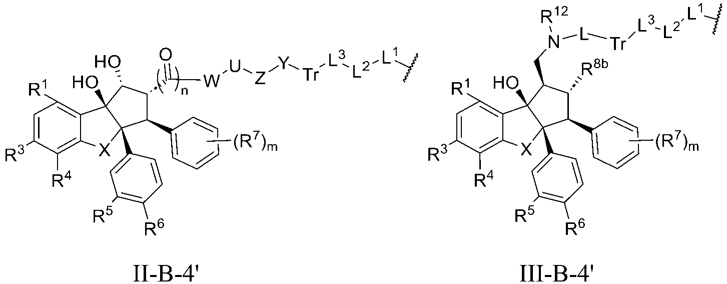 wherein X, R¹, ring A, R³, R⁴, R⁵, R⁶, R⁷, R^{8b}, R¹², m, n, M, W, U, Z, Y, L, Q, T, Tr, L¹, L², and L³ are defined as described in any one of the embodiments of the present disclosure.

In certain embodiments, the linking group and the drug group are linked to form a structure selected from a structure represented by formula I-B-5, II-B-5, III-B-5, IV-B-5, or V-B-5: 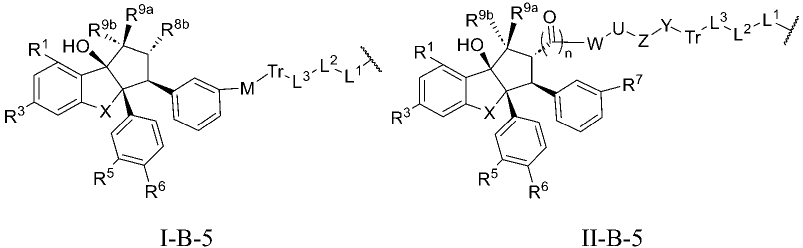 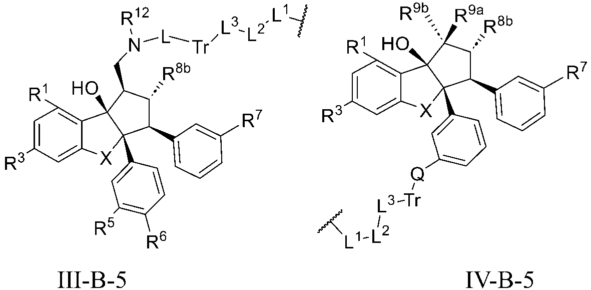 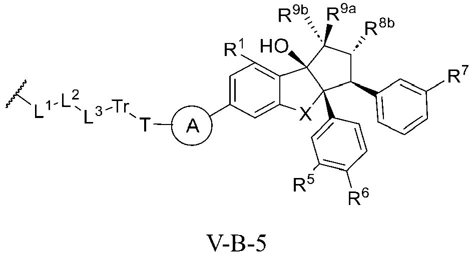 wherein X, R¹, ring A, R³, R⁵, R⁶, R⁷, R^{8b}, R^{9a}, R^{9b}, R¹², n, M, W, U, Z, Y, L, Q, T, Tr, L¹, L², and L³ are defined as described in any one of the embodiments of the present disclosure.

In certain embodiments, the linking group and the drug group are linked to form a structure selected from structures represented by formulas I-B-6 and I-B-7: 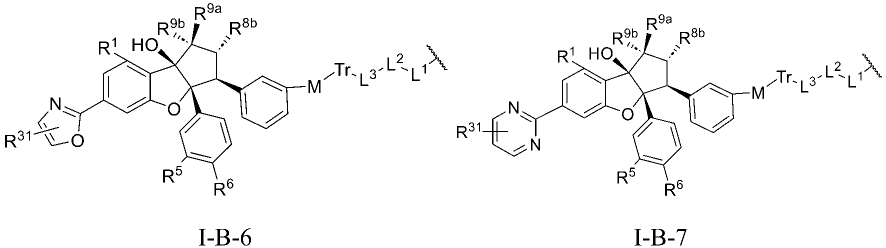 wherein R¹, R³¹, R⁵, R⁶, R^{8b}, R^{9a}, R^{9b}, M, Tr, L¹, L², and L³ are defined as described in any one of the embodiments of the present disclosure.

In certain embodiments, the linking group and the drug group are linked to form a structure selected from the structures shown in Table B below:

**Table B**

| No. | Structure |
|---|---|
| I-B1 | 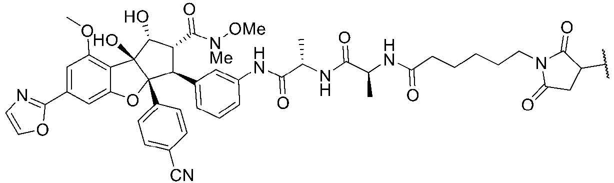 |
| I-B2 | 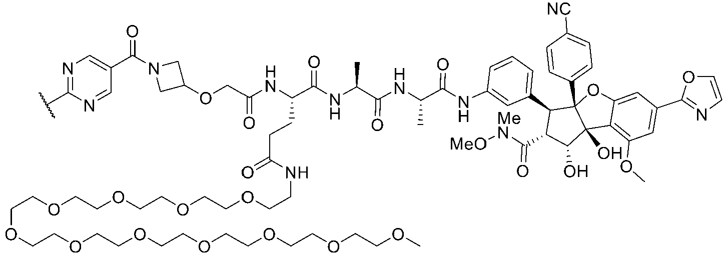 |
| I-B3 | 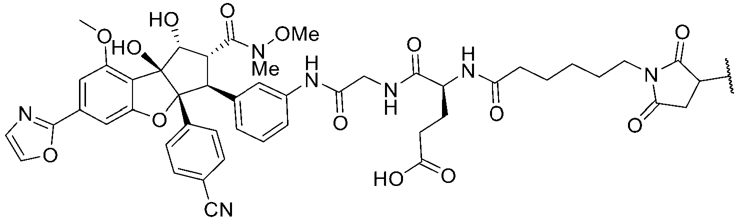 |
| I-B4 | 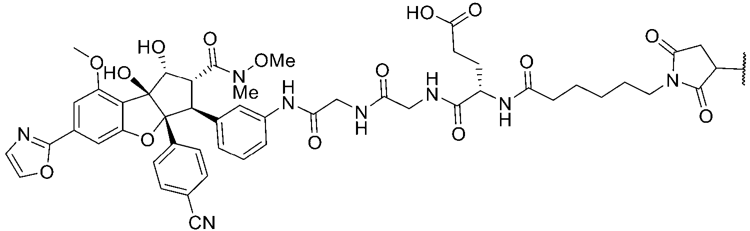 |
| I-B5 | 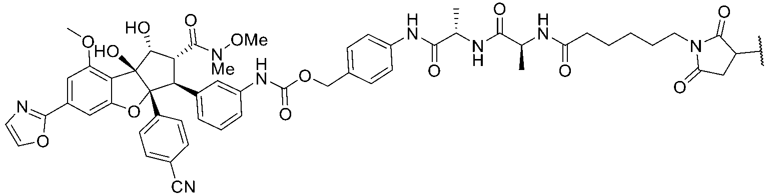 |
| I-B6 | 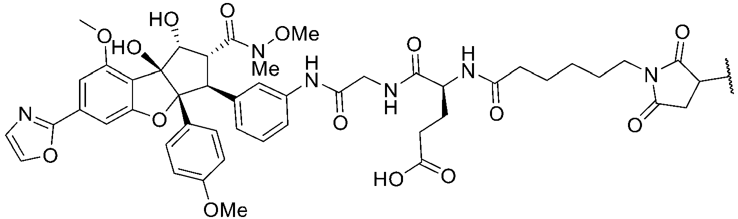 |
| I-B7 | 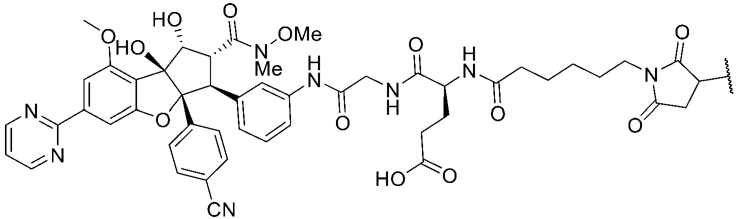 |
| I-B8 | 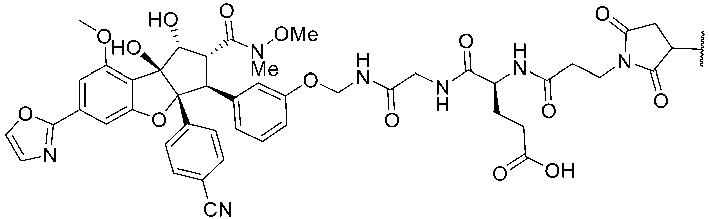 |
| I-B9 | 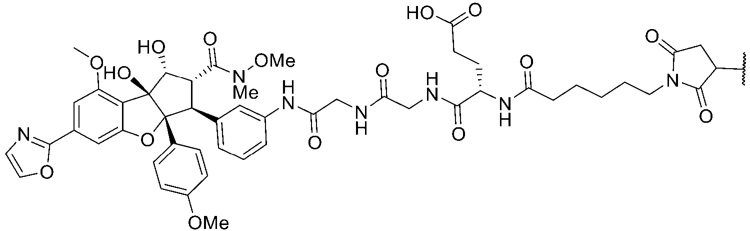 |
| I-B10 | 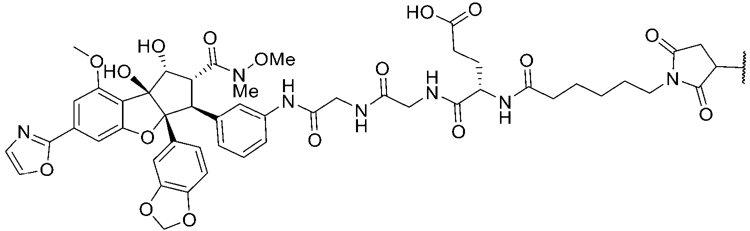 |
| I-B11 | 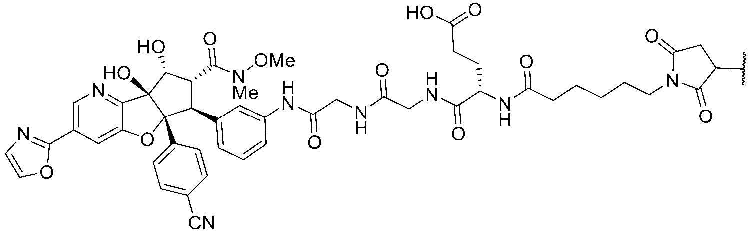 |
| I-B12 | 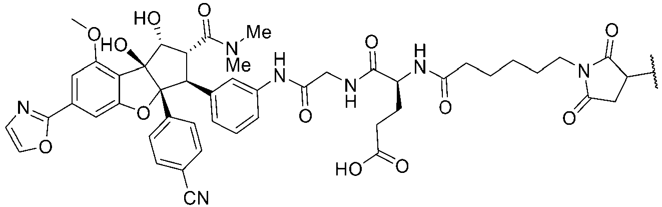 |
| I-B13 | 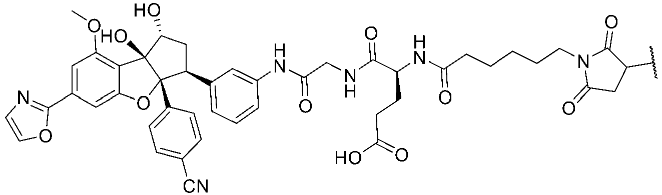 |
| I-B14 | 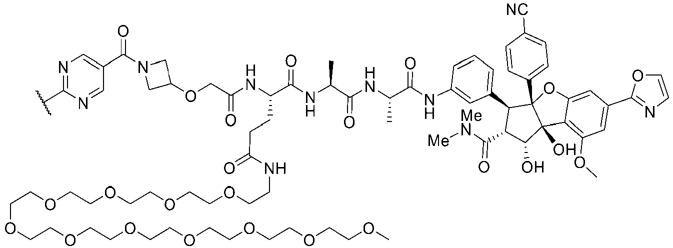 |
| I-B15 | 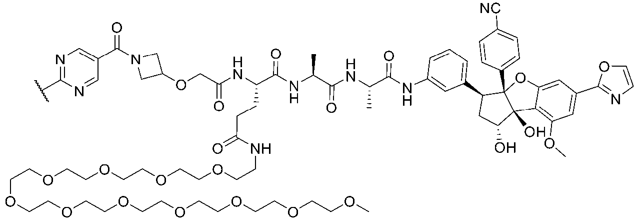 |
| I-B 16 | 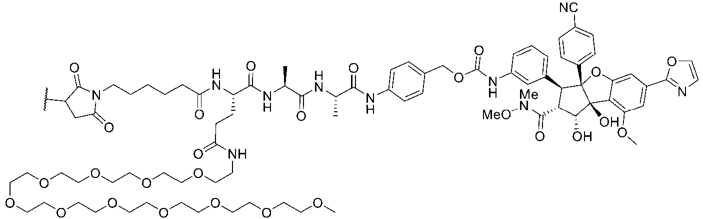 |
| I-B17 | 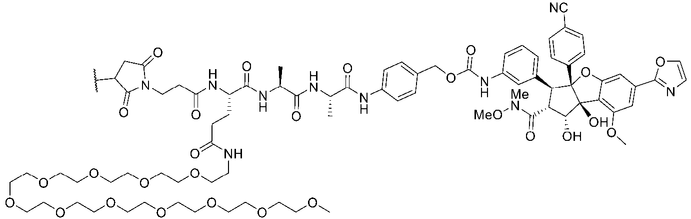 |
| I-B18 | 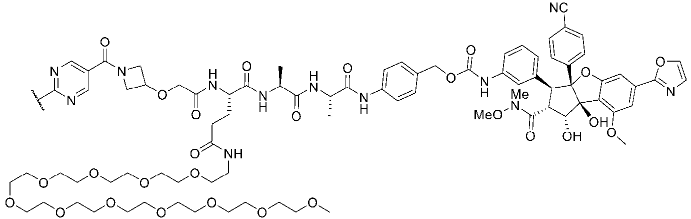 |
| I-B19 | 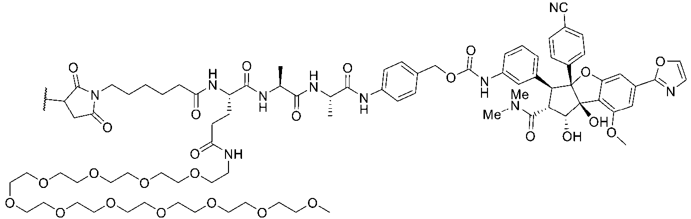 |
| I-B20 | 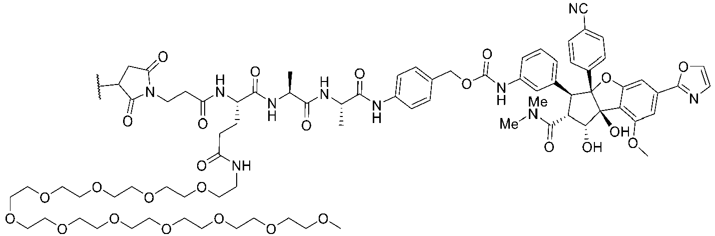 |
| I-B21 | 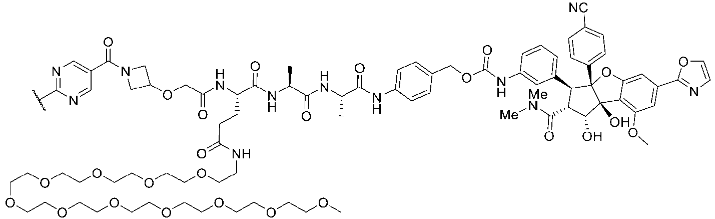 |
| I-B22 | 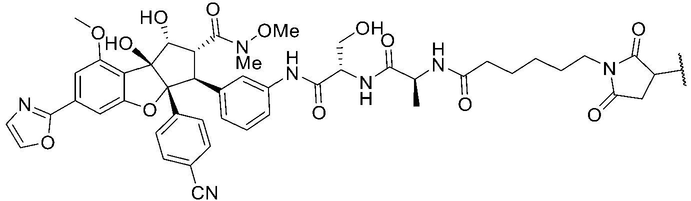 |
| I-B23 | 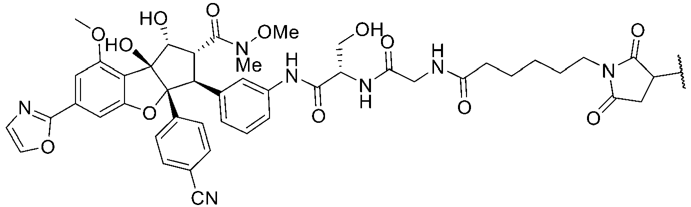 |
| I-B24 | 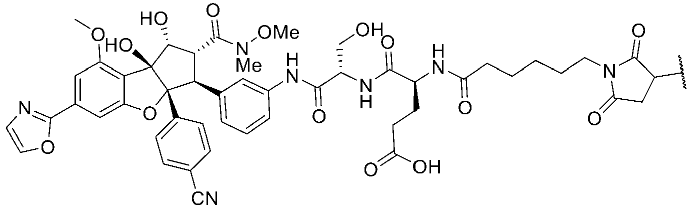 |
| I-B25 | 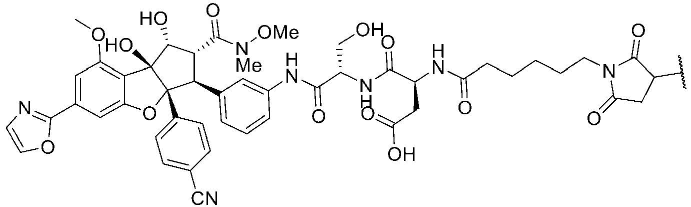 |
| I-B26 | 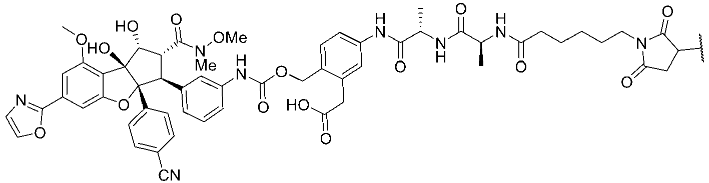 |
| I-B27 | 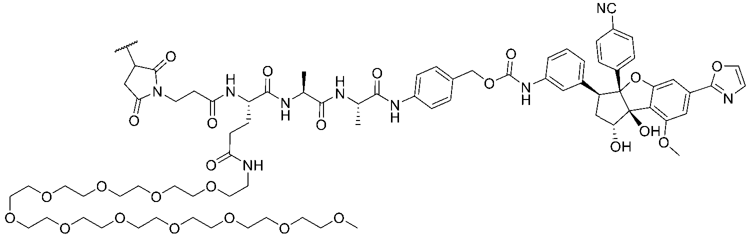 |
| I-B28 | 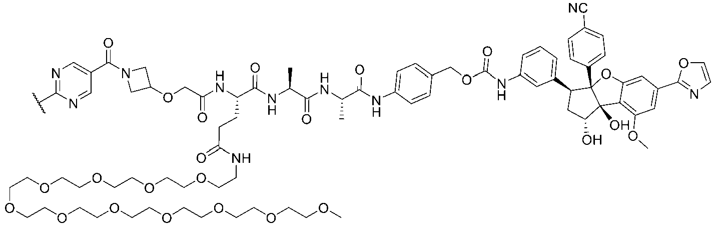 |
| I-B29 | 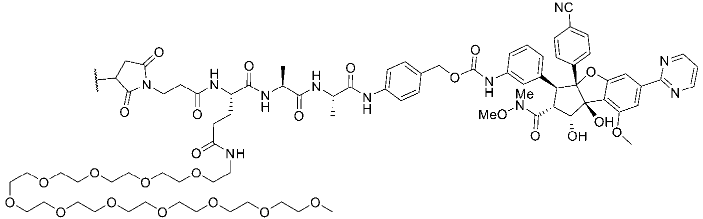 |
| I-B30 | 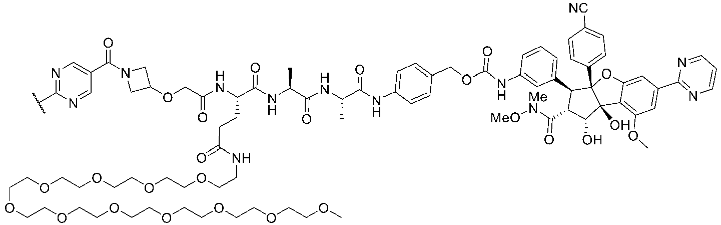 |
| I-B31 | 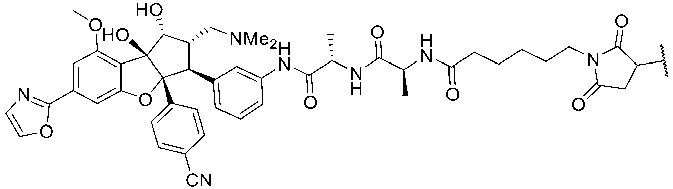 |
| I-B32 | 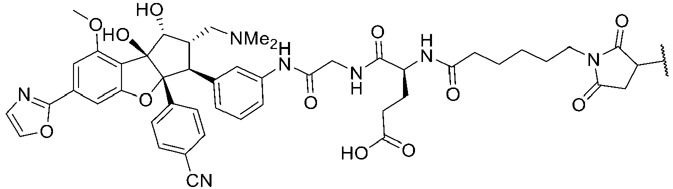 |
| I-B33 | 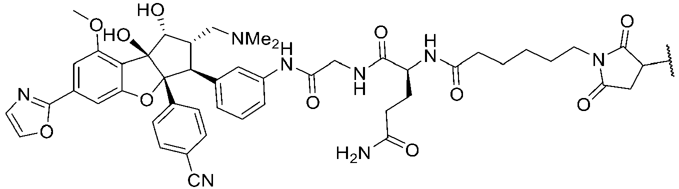 |
| I-B34 | 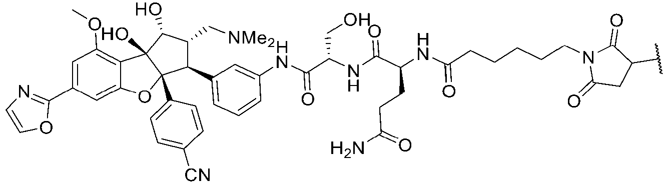 |
| I-B35 | 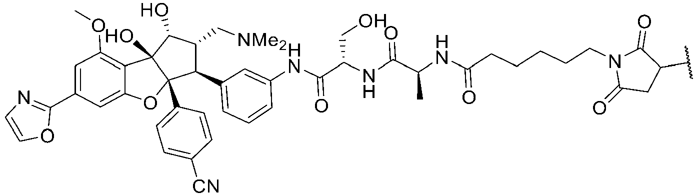 |
| I-B36 | 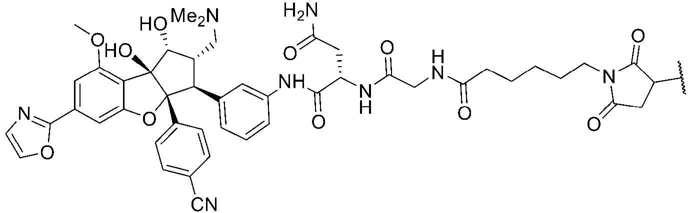 |
| I-B37 | 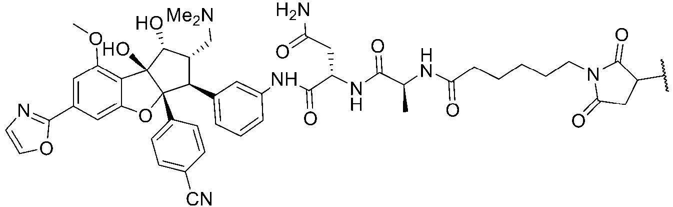 |
| I-B38 | 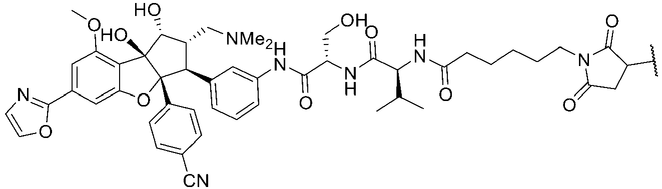 |
| I-B39 | 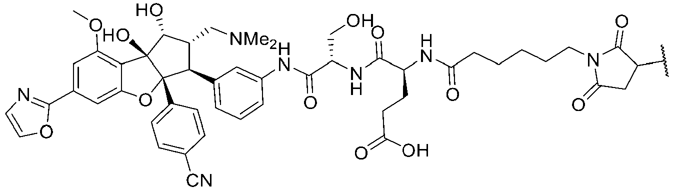 |
| I-B40 | 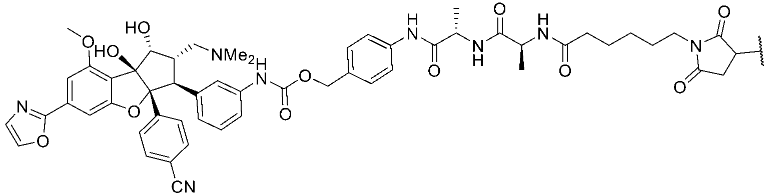 |
| I-B41 | 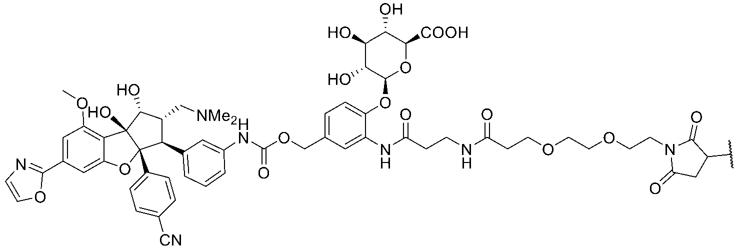 |
| I-B42 | 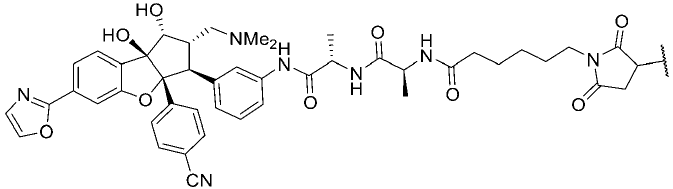 |
| I-B43 | 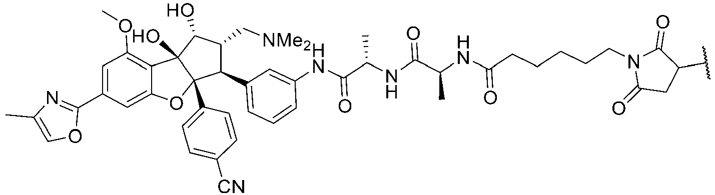 |
| I-B44 | 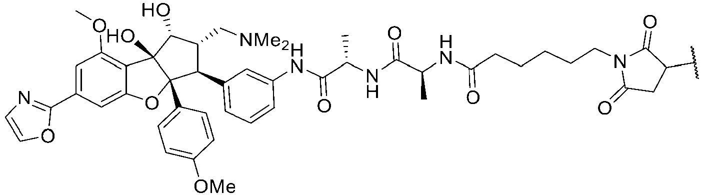 |
| I-B45 | 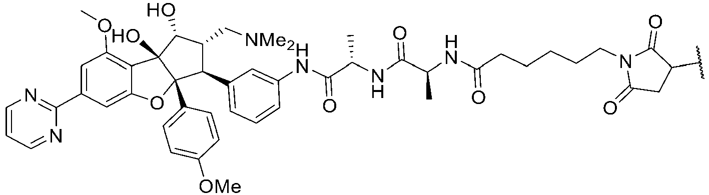 |
| I-B46 | 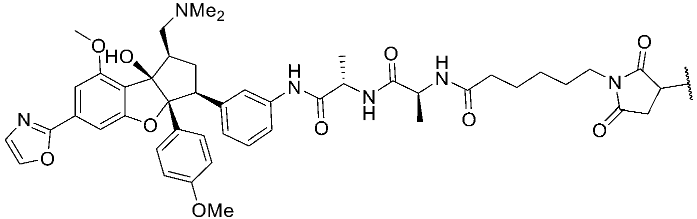 |
| I-B47 | 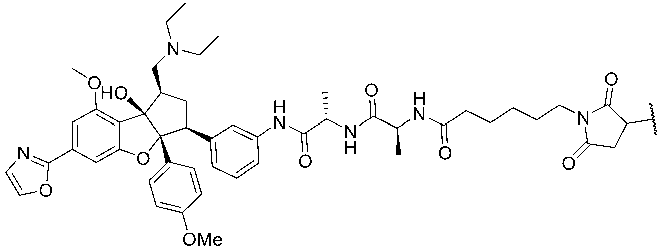 |
| I-B48 | 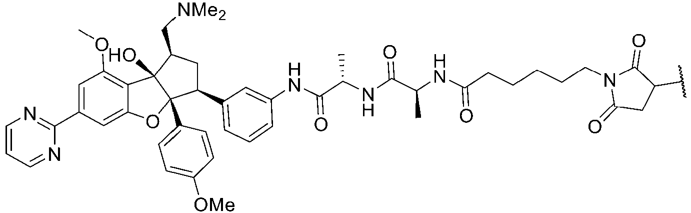 |
| I-B49 | 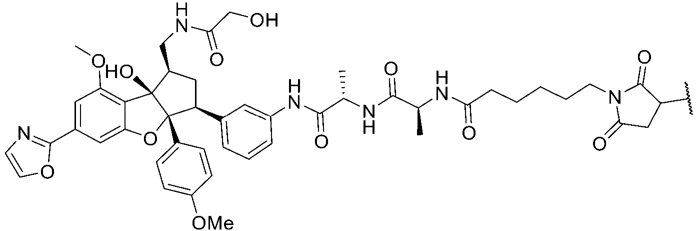 |
| I-B50 | 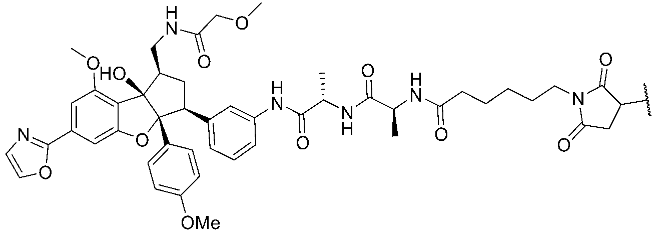 |
| I-B51 | 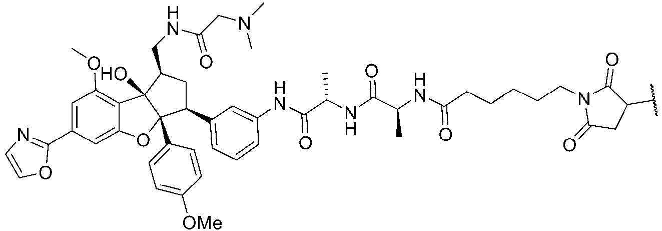 |
| I-B52 | 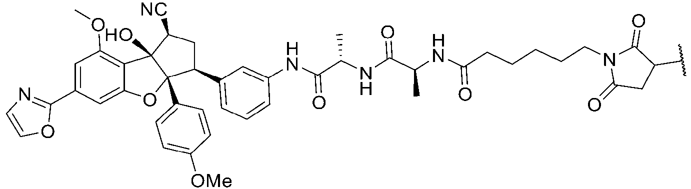 |
| I-B53 | 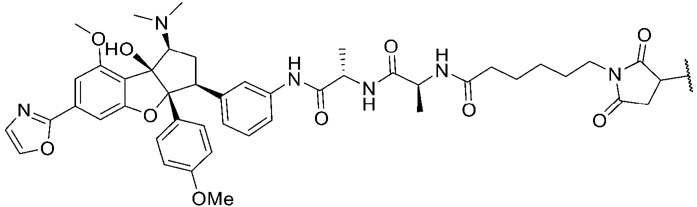 |
| I-B54 | 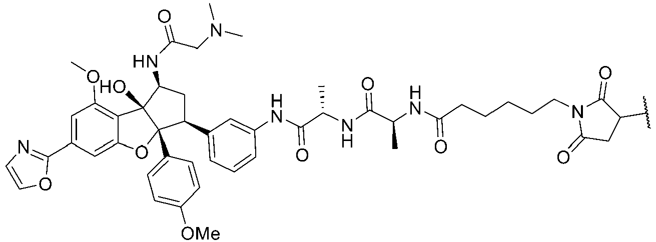 |
| I-B55 | 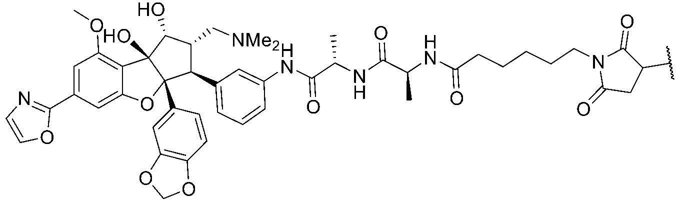 |
| I-B56 | 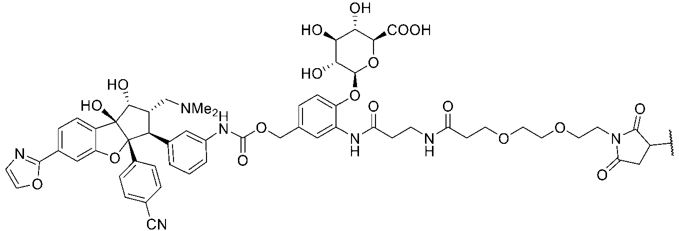 |
| I-B57 | 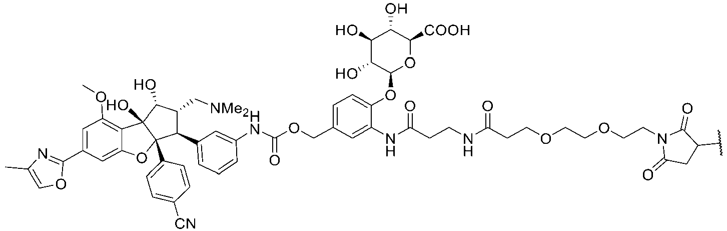 |
| I-B58 | 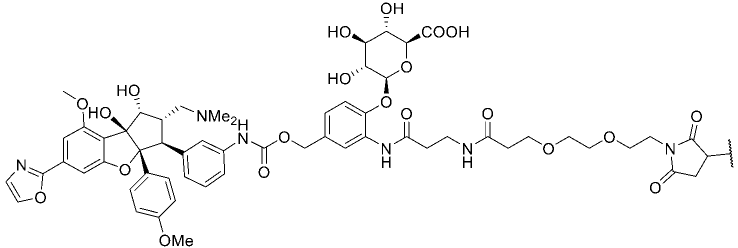 |
| I-B59 | 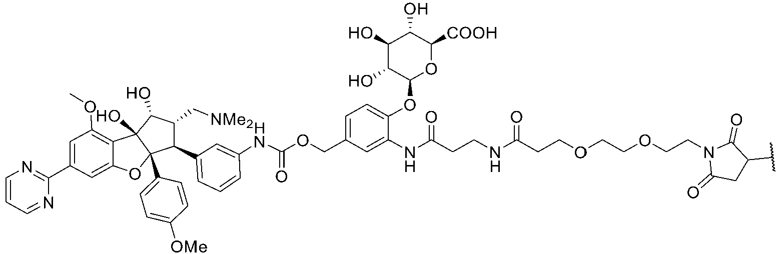 |
| I-B60 | 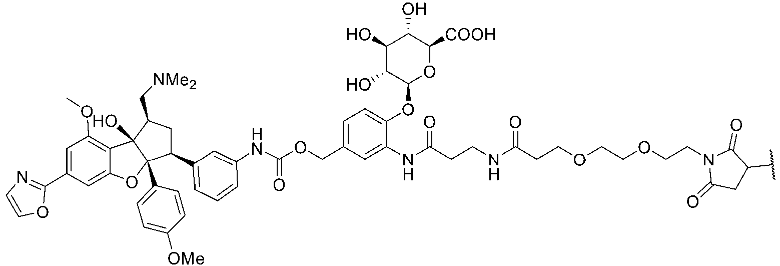 |
| I-B61 | 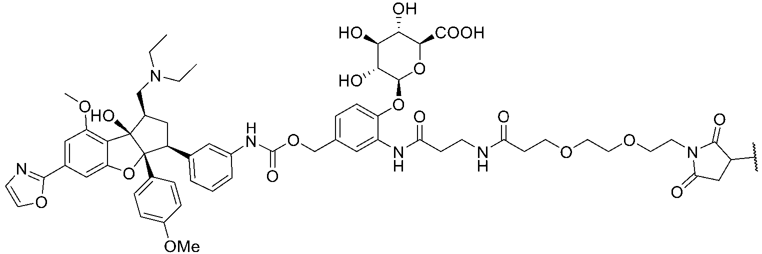 |
| I-B62 | 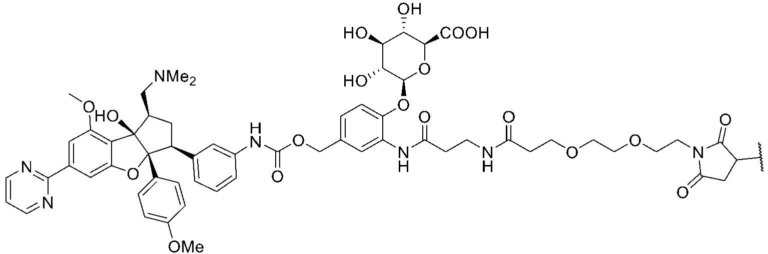 |
| I-B63 | 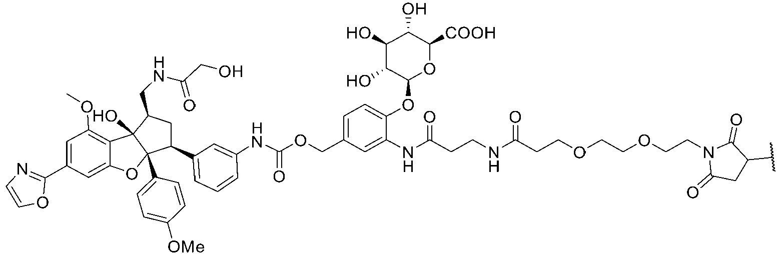 |
| I-B64 | 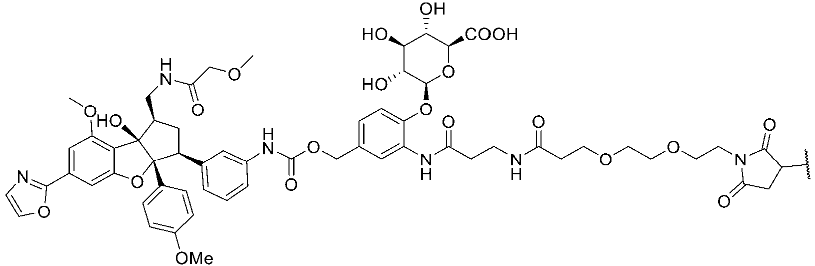 |
| I-B65 | 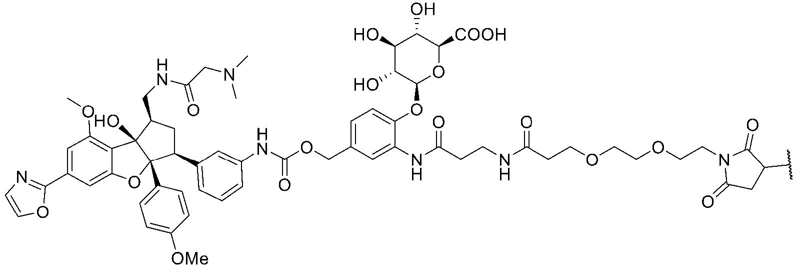 |
| I-B66 | 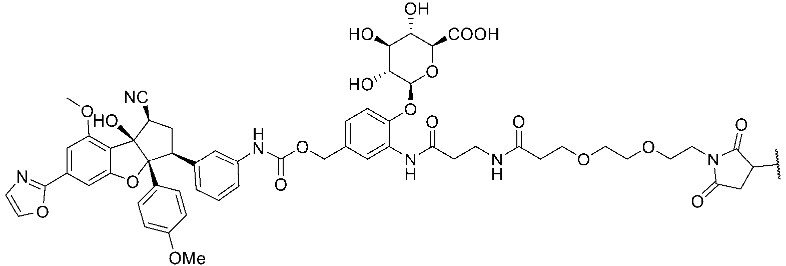 |
| I-B67 | 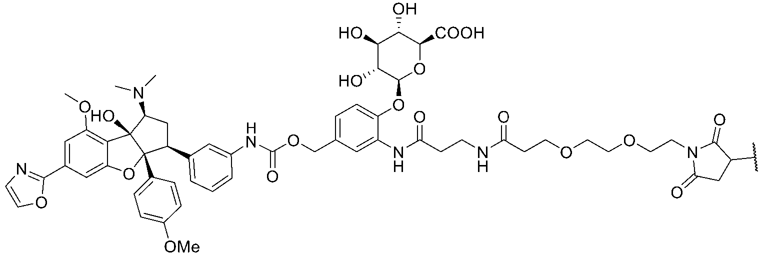 |
| I-B68 | 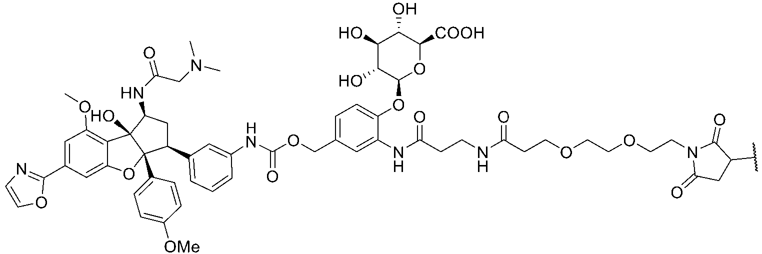 |
| I-B69 | 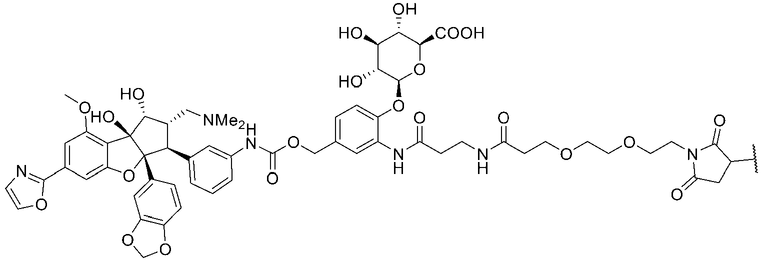 |
| I-B70 | 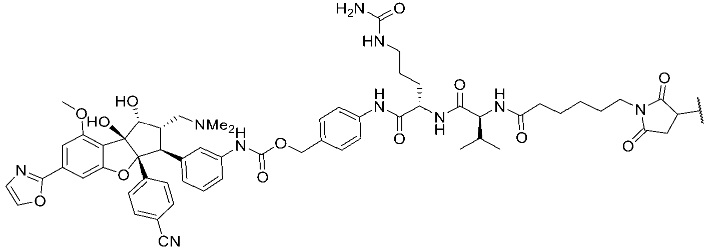 |
| I-B71 | 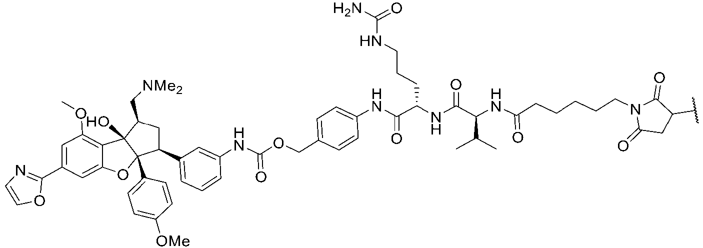 |
| I-B72 | 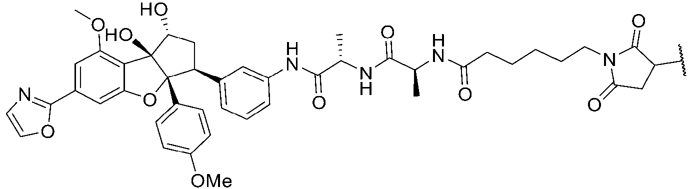 |
| I-B73 | 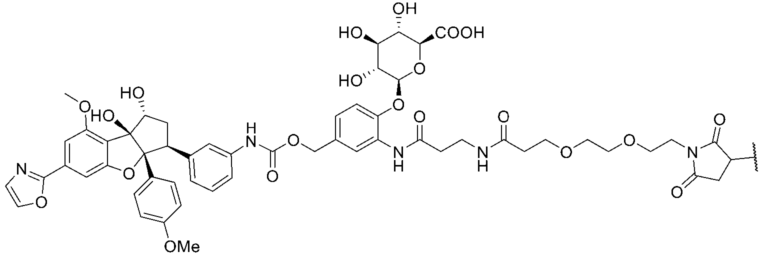 |
| I-B74 | 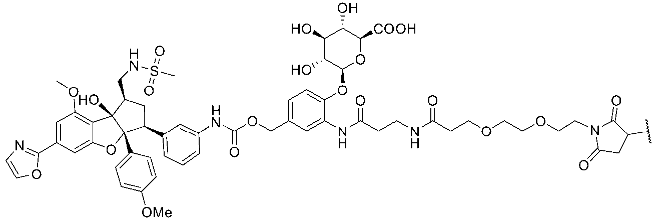 |
| II-B1 | 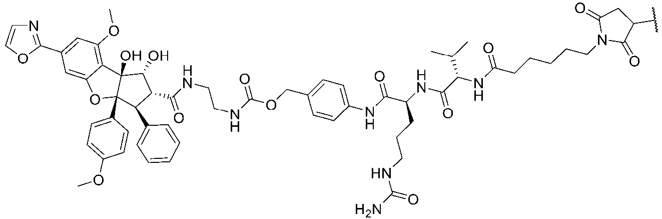 |
| II-B2 | 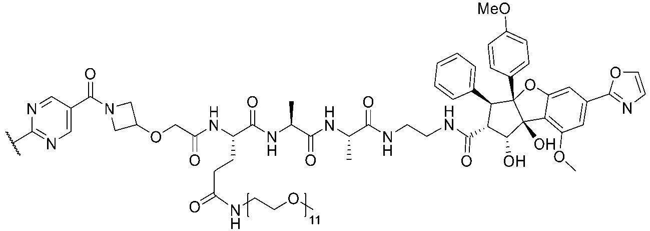 |
| II-B3 | 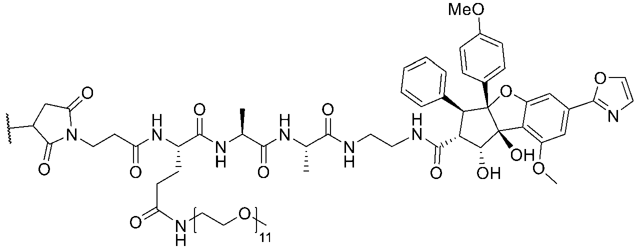 |
| II-B4 | 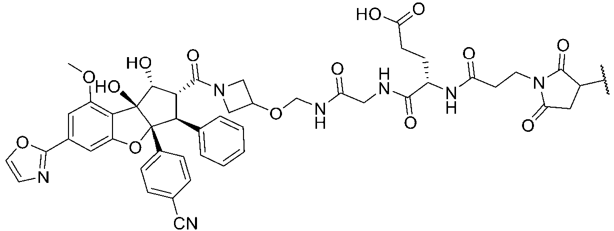 |
| II-B5 | 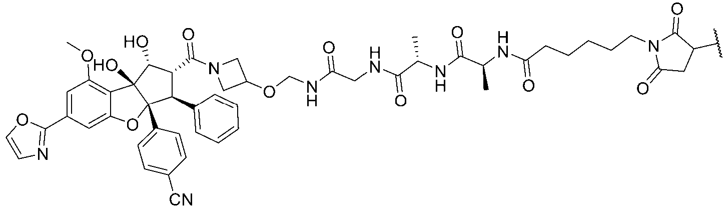 |
| II-B6 | 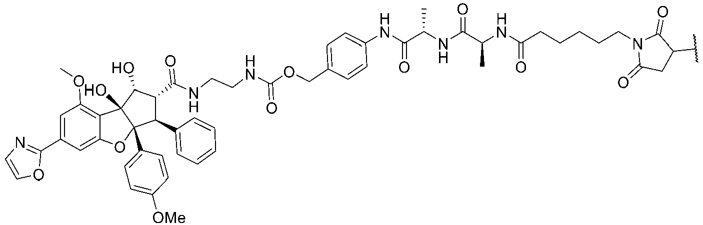 |
| II-B7 | 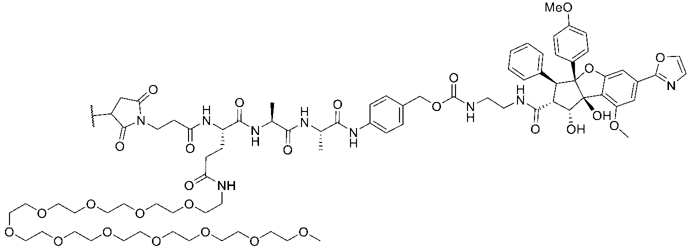 |
| II-B8 | 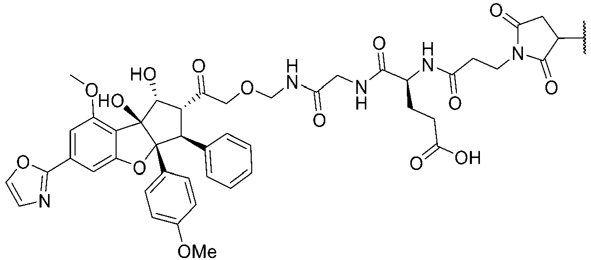 |
| II-B9 | 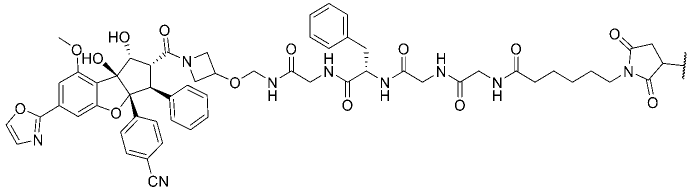 |
| II-B10 | 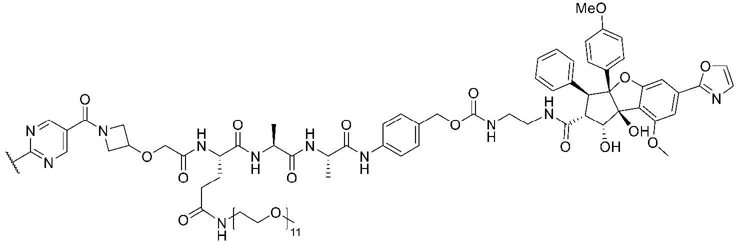 |
| II-B11 | 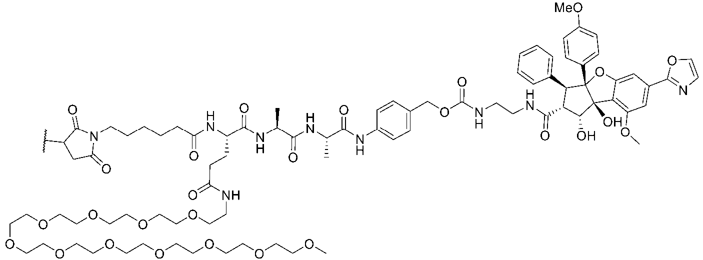 |
| II-B12 | 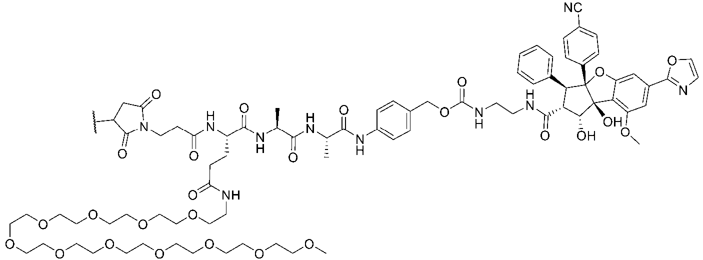 |
| II-B13 | 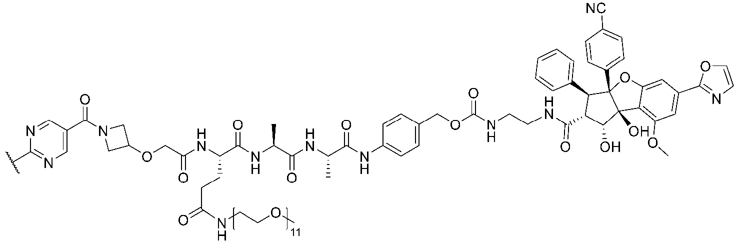 |
| II-B14 | 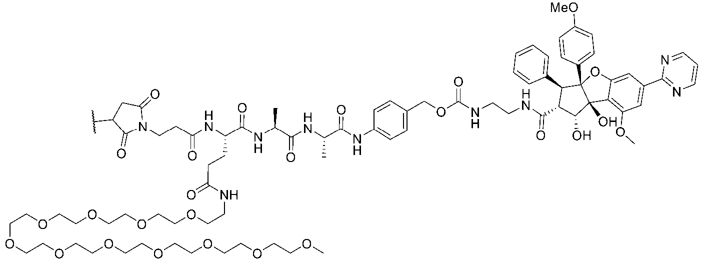 |
| II-B15 | 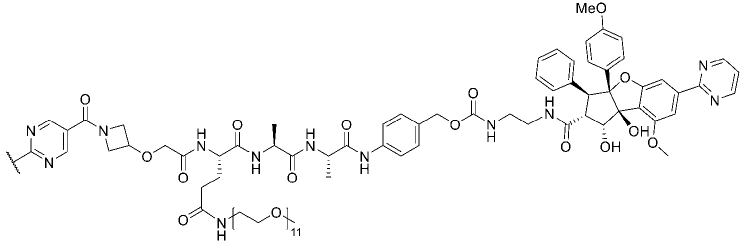 |
| II-B16 | 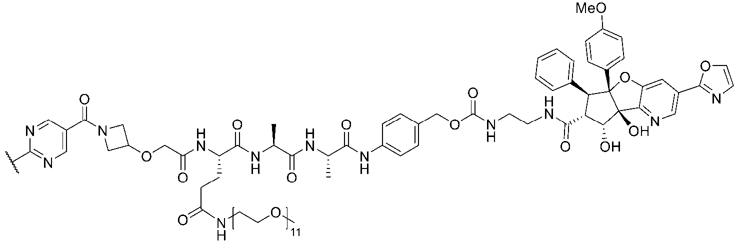 |
| II-B17 | 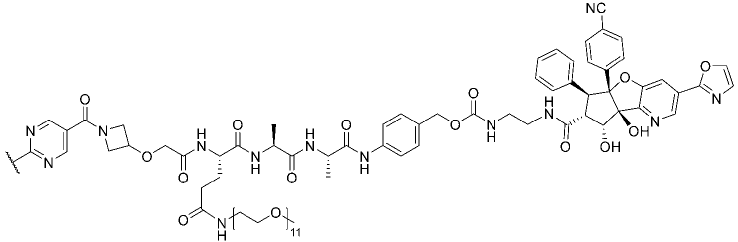 |
| II-B18 | 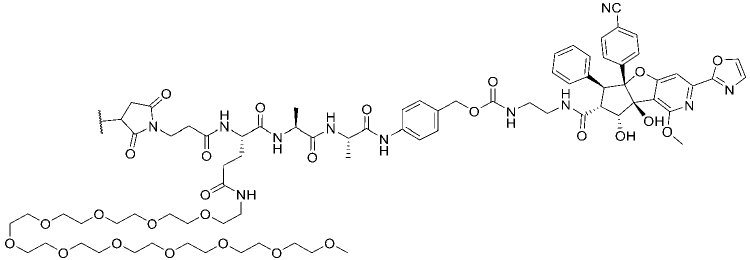 |
| II-B19 | 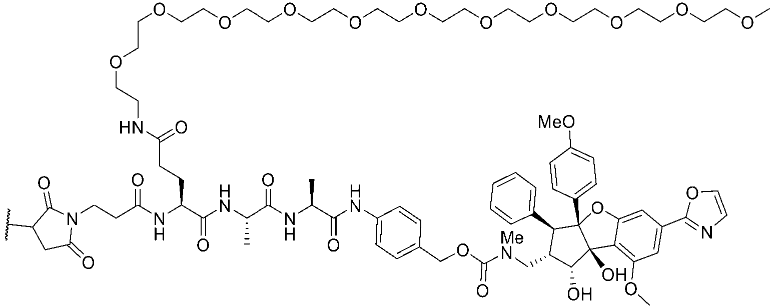 |
| II-B20 | 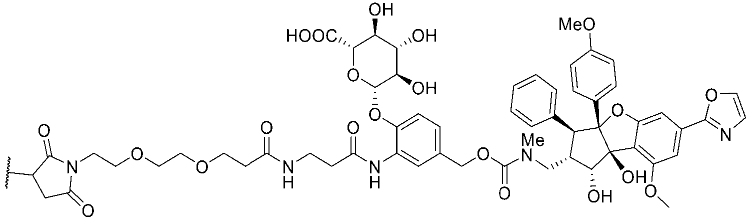 |
| II-D21 | 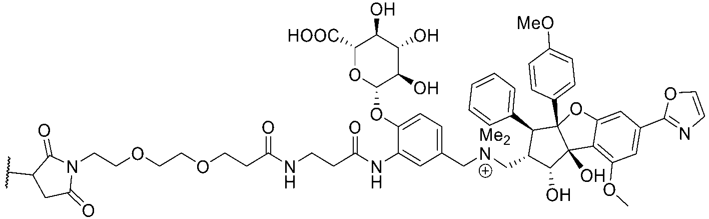 |
| III-B1 | 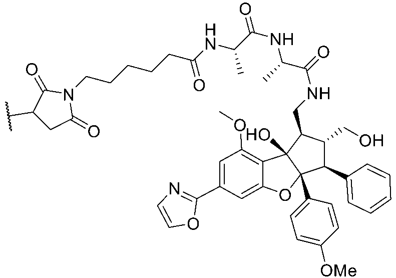 |
| III-B2 | 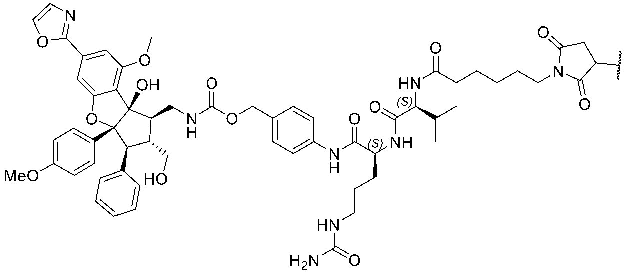 |
| III-B3 | 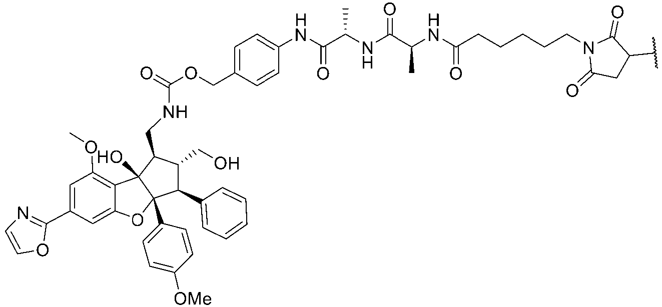 |
| III-B4 | 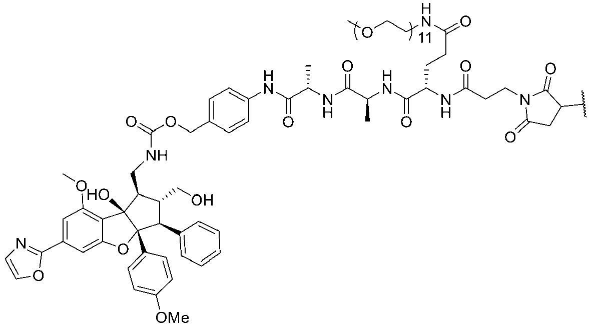 |
| III-B5 | 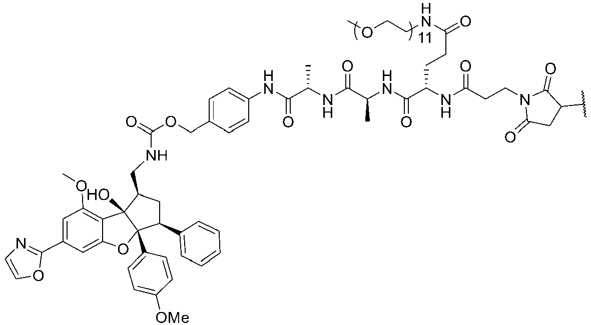 |
| III-B6 | 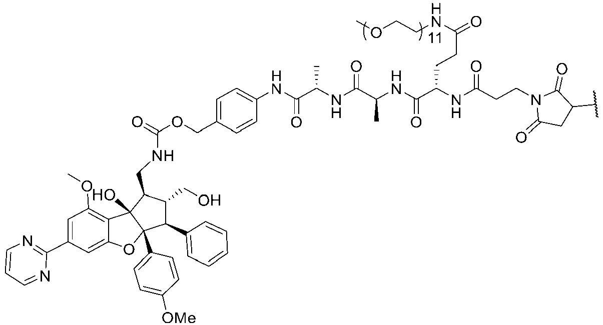 |
| III-B7 | 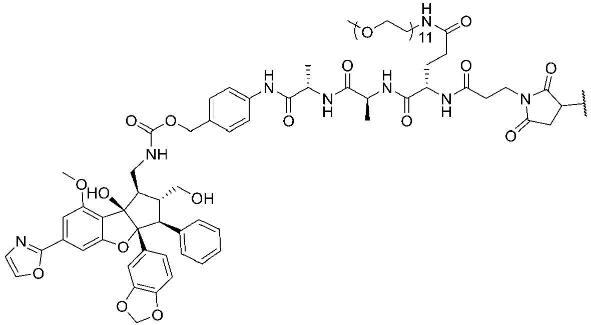 |
| III-B8 | 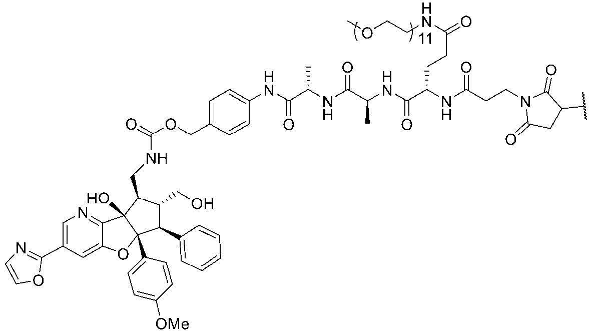 |
| III-B9 | 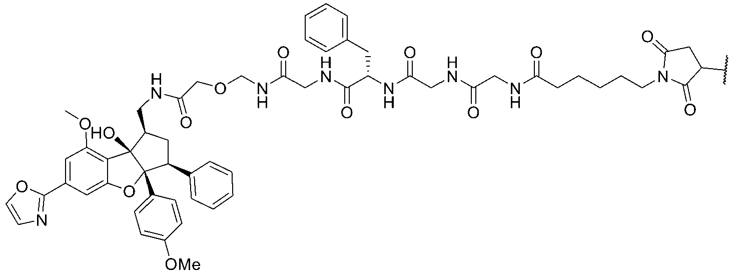 |
| III-B10 | 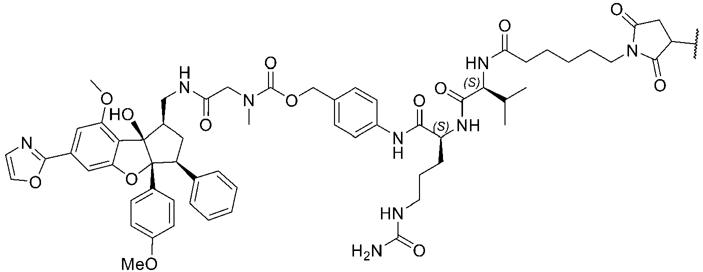 |
| III-B11 | 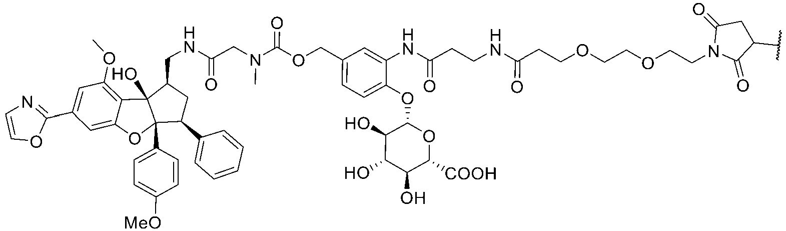 |
| III-B12 | 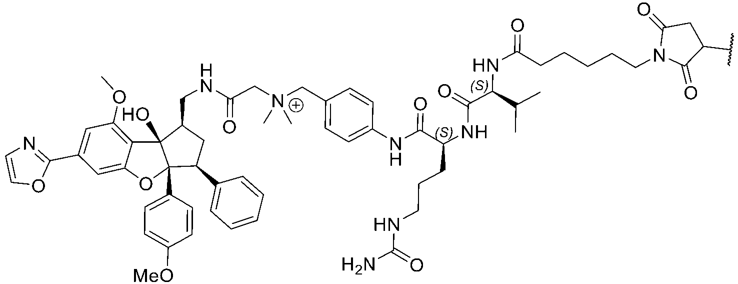 |
| III-B13 | 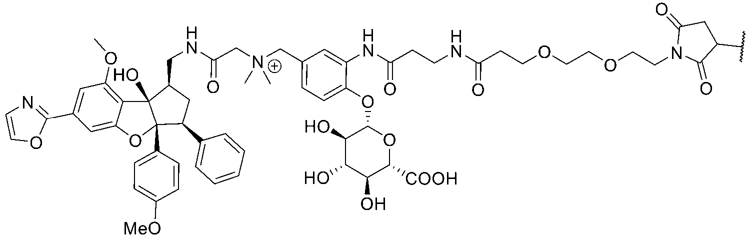 |
| IV-B1 | 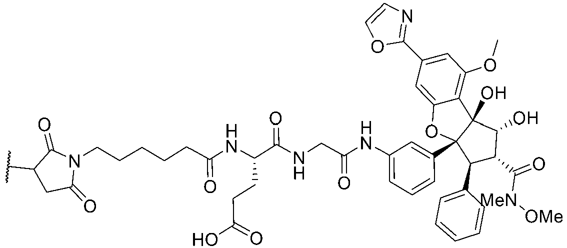 |
| IV-B2 | 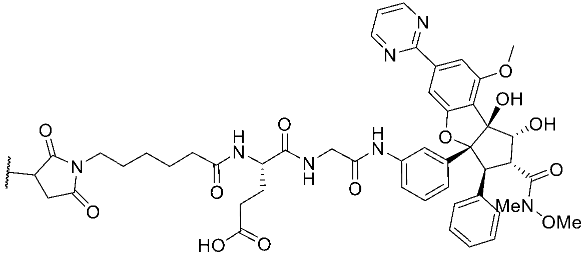 |
| IV-B3 | 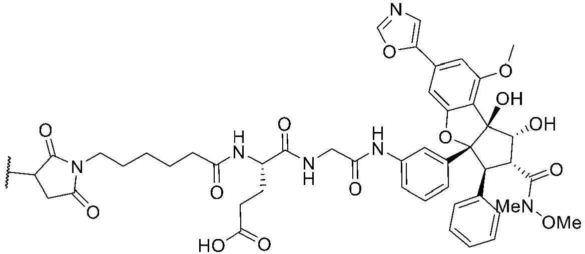 |
| IV-B4 | 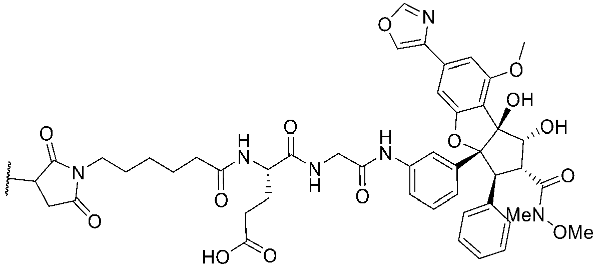 |
| IV-B5 | 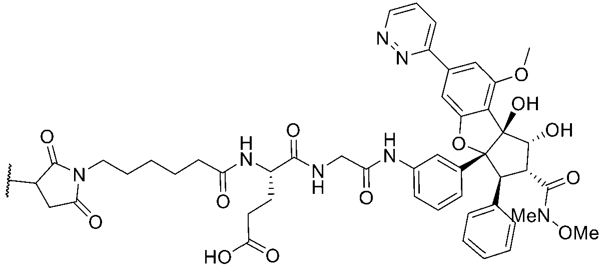 |
| IV-B6 | 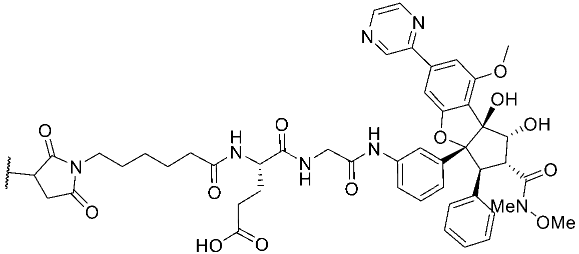 |
| IV-B7 | 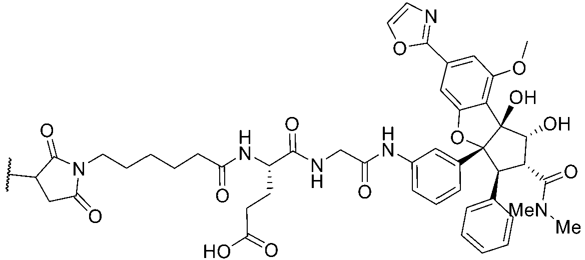 |
| IV-B8 | 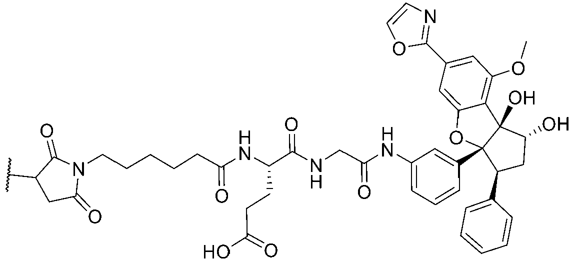 |
| IV-B9 | 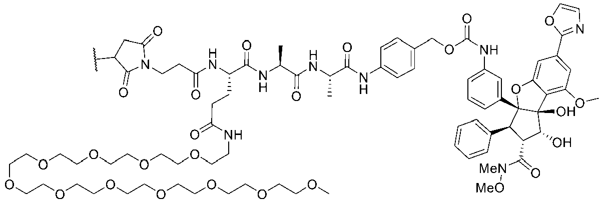 |
| IV-B10 | 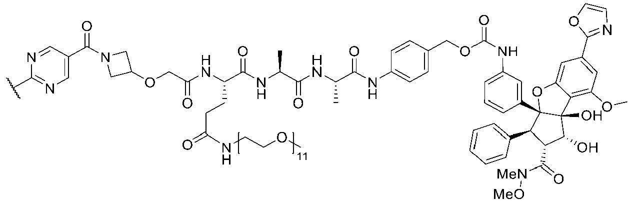 |
| IV-B11 | 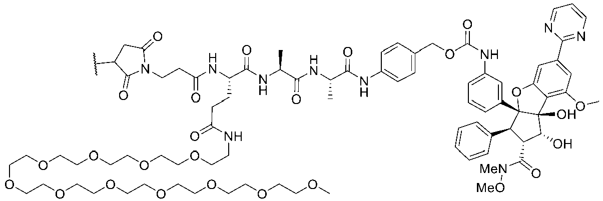 |
| IV-B12 | 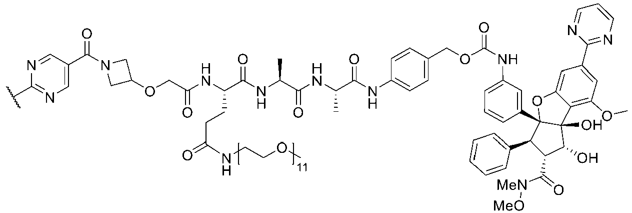 |
| IV-B13 | 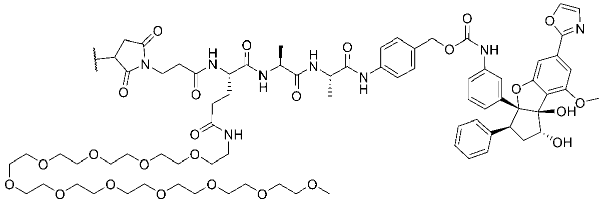 |
| IV-B14 | 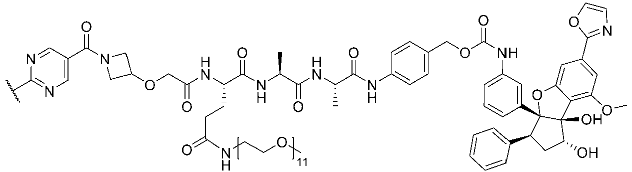 |
| IV-B15 | 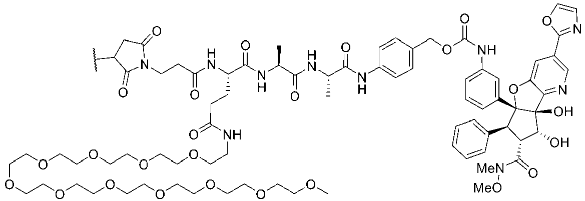 |
| IV-B16 | 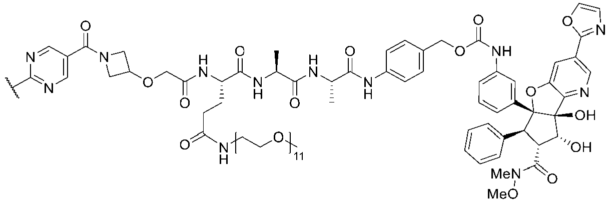 |
| IV-B17 | 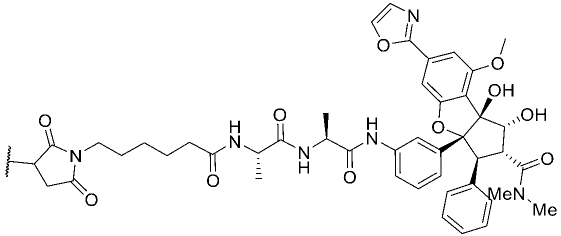 |
| IV-B18 | 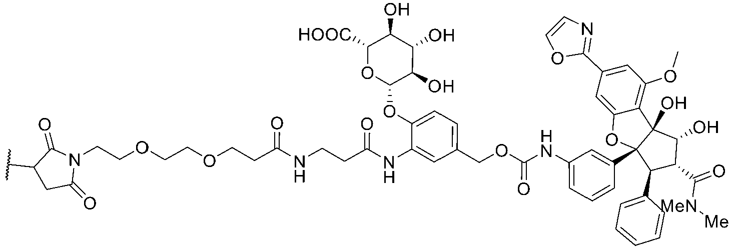 |
| IV-B19 | 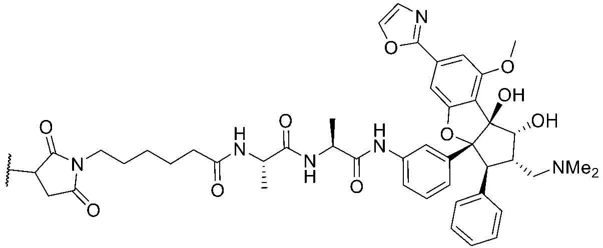 |
| IV-B20 | 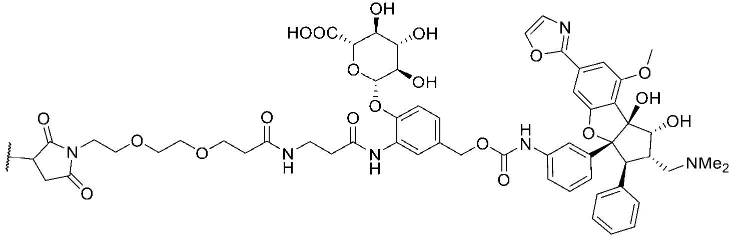 |
| V-B1 | 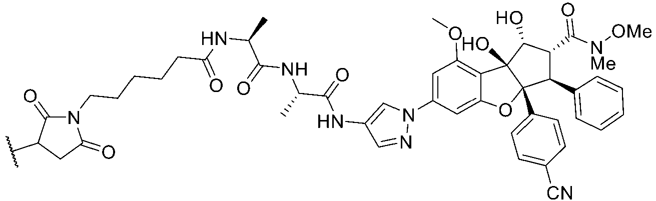 |
| V-B2 | 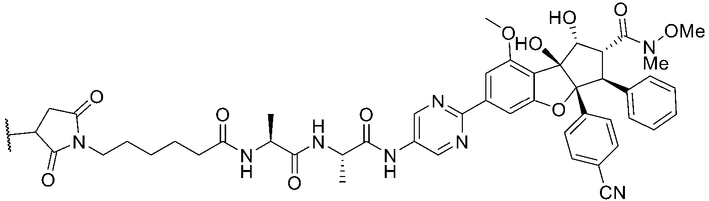 |
| V-B3 | 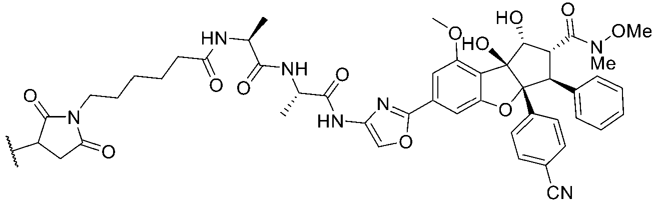 |
| V-B4 | 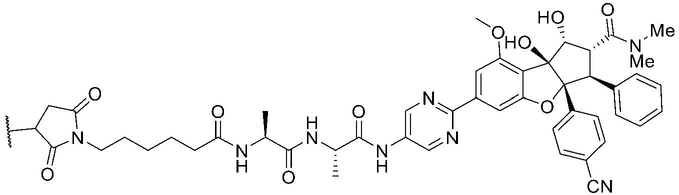 |
| V-B5 | 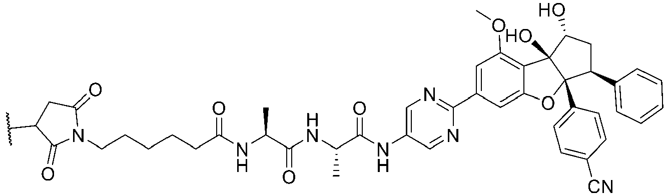 |
| V-B6 | 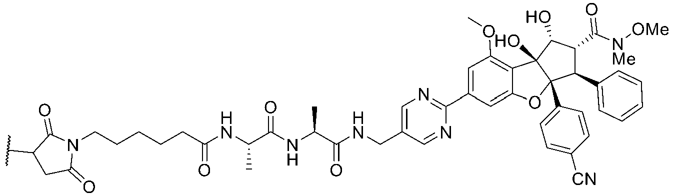 |
| V-B7 | 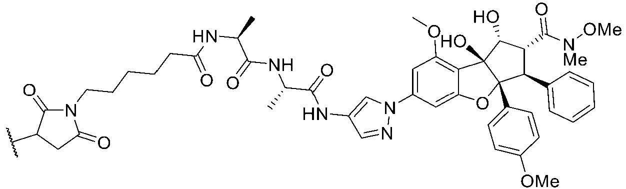 |
| V-B8 | 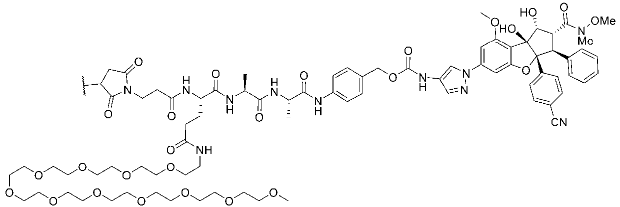 |
| V-B9 | 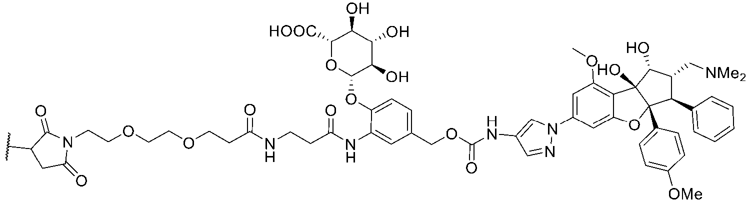 |
| V-B10 | 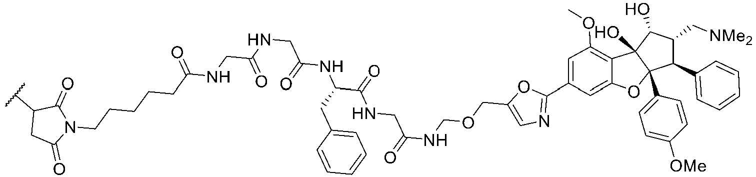 |
| V-B11 | 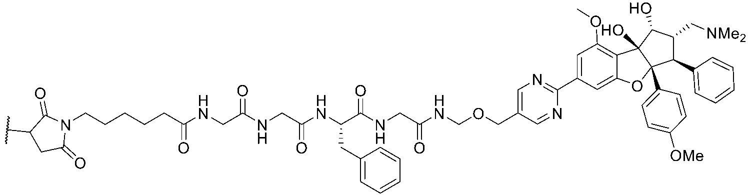 |
| V-B12 | 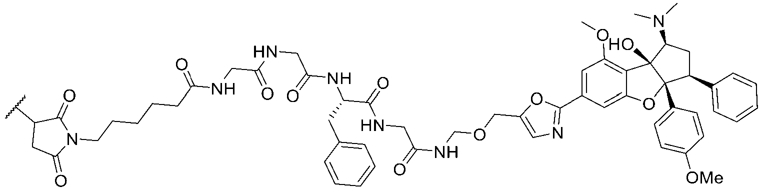 |
| V-B13 | 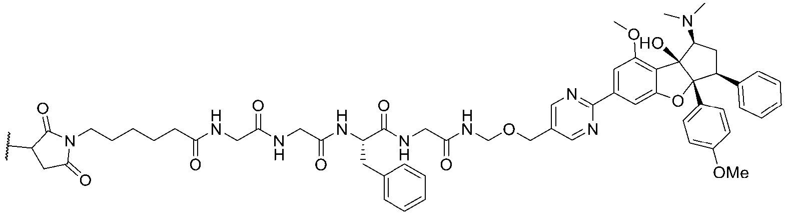 |

In certain embodiments, provided is the ligand-drug conjugate or the racemic mixture, tautomer, stereoisomer, enantiomer, or diastereoisomer thereof, or the mixture thereof, or the pharmaceutically acceptable salt or solvate thereof, wherein the ligand-drug conjugate is selected from a compound represented by formula I-C: 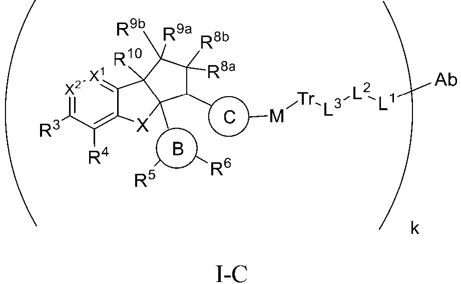 wherein:
X, X¹, X², ring B, ring C, R³, R⁴, R⁵, R⁶, R^{8a}, R^{8b}, R^{9a}, R^{9b}, R¹⁰, M, Tr, L¹, L², and L³ are defined as described in any one of the embodiments of the present disclosure;
Ab is a ligand that binds to a target;
k is a drug loading and is an integer or a decimal from 1 to 16.

In certain embodiments, provided is the ligand-drug conjugate or the racemic mixture, tautomer, stereoisomer, enantiomer, or diastereoisomer thereof, or the mixture thereof, or the pharmaceutically acceptable salt or solvate thereof, wherein the ligand-drug conjugate is selected from a compound represented by formula II-C': 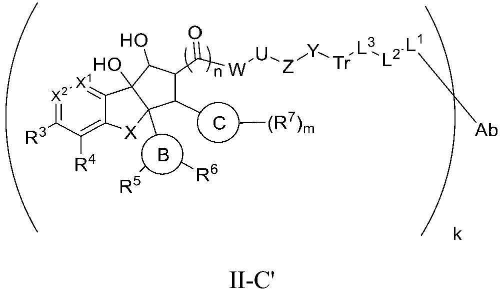 wherein:
X, X¹, X², ring B, ring C, R³, R⁴, R⁵, R⁶, R⁷, m, n, W, U, Z, Y, Tr, L¹, L², and L³ are defined as described in any one of the embodiments of the present disclosure;
Ab is a ligand that binds to a target;
k is a drug loading and is an integer or a decimal from 1 to 16.

In certain embodiments, provided is the ligand-drug conjugate or the racemic mixture, tautomer, stereoisomer, enantiomer, or diastereoisomer thereof, or the mixture thereof, or the pharmaceutically acceptable salt or solvate thereof, wherein the ligand-drug conjugate is selected from a compound represented by formula II-C: 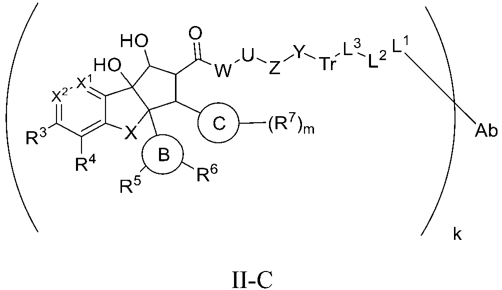 wherein:
X, X¹, X², ring B, ring C, R³, R⁴, R⁵, R⁶, R⁷, m, W, U, Z, Y, Tr, L¹, L², and L³ are defined as described in any one of the embodiments of the present disclosure;
Ab is a ligand that binds to a target;
k is a drug loading and is an integer or a decimal from 1 to 16.

In certain embodiments, provided is the ligand-drug conjugate or the racemic mixture, tautomer, stereoisomer, enantiomer, or diastereoisomer thereof, or the mixture thereof, or the pharmaceutically acceptable salt or solvate thereof, wherein the ligand-drug conjugate is selected from a compound represented by formula III-C': 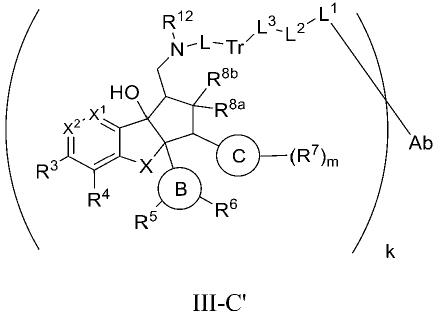 wherein:
X, X¹, X², ring B, ring C, R³, R⁴, R⁵, R⁶, R⁷, R^{8a}, R^{8b}, R¹², L, m, Tr, L¹, L², and L³ are defined as described in any one of the embodiments of the present disclosure;
Ab is a ligand that binds to a target;
k is a drug loading and is an integer or a decimal from 1 to 16.

In certain embodiments, provided is the ligand-drug conjugate or the racemic mixture, tautomer, stereoisomer, enantiomer, or diastereoisomer thereof, or the mixture thereof, or the pharmaceutically acceptable salt or solvate thereof, wherein the ligand-drug conjugate is selected from a compound represented by formula III-C: 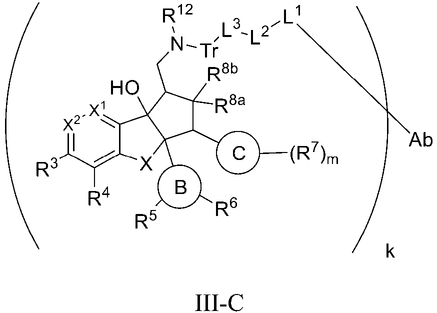 wherein:
X, X¹, X², ring B, ring C, R³, R⁴, R⁵, R⁶, R⁷, R^{8a}, R^{8b}, R¹², m, Tr, L¹, L², and L³ are defined as described in any one of the embodiments of the present disclosure;
Ab is a ligand that binds to a target;
k is a drug loading and is an integer or a decimal from 1 to 16.

In certain embodiments, provided is the ligand-drug conjugate or the racemic mixture, tautomer, stereoisomer, enantiomer, or diastereoisomer thereof, or the mixture thereof, or the pharmaceutically acceptable salt or solvate thereof, wherein the ligand-drug conjugate is selected from a compound represented by formula IV-C: 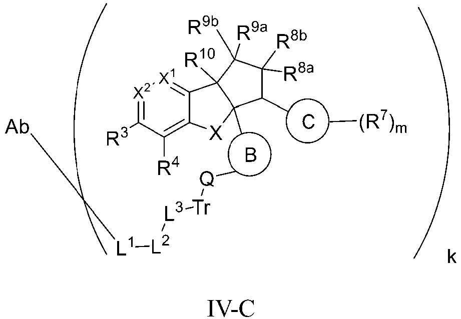 wherein:
X, X¹, X², ring B, ring C, R³, R⁴, R⁷, R^{8a}, R^{8b}, R^{9a}, R^{9b}, R¹⁰, m, Q, Tr, L¹, L², and L³ are defined as described in any one of the embodiments of the present disclosure;
Ab is a ligand that binds to a target;
k is a drug loading and is an integer or a decimal from 1 to 16.

In certain embodiments, provided is the ligand-drug conjugate or the racemic mixture, tautomer, stereoisomer, enantiomer, or diastereoisomer thereof, or the mixture thereof, or the pharmaceutically acceptable salt or solvate thereof, wherein the ligand-drug conjugate is selected from a compound represented by formula V-C: 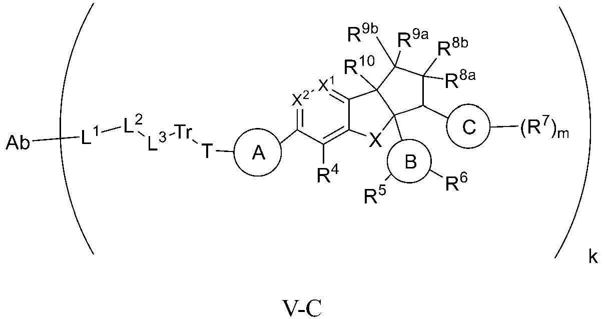 wherein:
X, X¹, X², ring A, ring B, ring C, R⁴, R⁵, R⁶, R⁷, R^{8a} , R^{8b}, R^{9a}, R^{9b}, R¹⁰, m, T, Tr, L¹, L², and L³ are defined as described in any one of the embodiments of the present disclosure;
Ab is a ligand that binds to a target;
k is a drug loading and is an integer or a decimal from 1 to 16.

In certain embodiments, Ab is conjugated to L¹ by an S atom.

In certain embodiments, Ab is a polypeptide, or an antibody or an antigen-binding fragment thereof, particularly an antibody or an antigen-binding fragment thereof, capable of specifically binding to a target.

In certain embodiments, the antibody is selected from one or more of the following:
(1) a fully human antibody, a humanized antibody, a murine antibody, and a chimeric antibody,
(2) a probody,
(3) a bispecific antibody and a multispecific antibody,
(4) a monoclonal antibody and a polyclonal antibody, and
(5) an IgG antibody.

In certain embodiments, the antigen-binding fragment is selected from an antigen-binding fragment of a Fab, a Fab', a F(ab')₂, an Fv, an scFv, an Fd, a dAb, a VHH, and a complementarity-determining region (CDR) fragment.

In certain embodiments, Ab is a monoclonal antibody, particularly a monoclonal antibody that takes sulfhydryl as a conjugation site, or a monoclonal antibody that has undergone site-directed mutagenesis or modification and takes sulfhydryl as a conjugation site.

In certain embodiments, Ab targets an antigen selected from the following group: HER2, HER3, B7H3, B7H4, DLL3, TROP2, Claudin18.2, CD30, CD33, CD70, EGFR, GPC-3, ADAM9, BCMA, CEACAM5, and c-met.

In certain embodiments, Ab targets an antigen selected from the following group: HER2, HER3, B7H3, B7H4, DLL3, TROP2, Claudin18.2, CD30, CD33, CD70, EGFR, GPC-3, ADAM9, BCMA, and CEACAM5.

In certain embodiments, Ab targets an antigen selected from the following group: HER2, HER3, B7H3, B7H4, DLL3, TROP2, Claudin18.2, CD30, CD33, CD70, EGFR, GPC-3, ADAM9, and CEACAM5.

In certain embodiments, Ab is selected from antibodies and antigen-binding fragments thereof against HER2, HER3, B7H3, TROP2, Claudin18.2, CD30, CD33, CD70, GPC-3, ADAM9, BCMA, CEACAM5, and c-met.

In certain embodiments, Ab is selected from antibodies and antigen-binding fragments thereof against HER2, HER3, B7H3, TROP2, Claudin18.2, CD30, CD33, CD70, GPC-3, ADAM9, BCMA, and CEACAM5.

In certain embodiments, Ab is selected from antibodies and antigen-binding fragments thereof against HER2, HER3, B7H3, TROP2, Claudin18.2, CD30, CD33, CD70, GPC-3, ADAM9, and CEACAM5.

In certain embodiments, Ab is selected from tusamitamab, DB1001, DB1002, DB1003, DB1004, DB1005, cetuximab, and panitumumab, and biosimilars of the antibodies and antigen-binding fragments thereof.

In certain embodiments, Ab is selected from tusamitamab, DB1001, DB1002, DB1003, DB1004, cetuximab, and panitumumab, and biosimilars thereof and antigen-binding fragments thereof.

In certain embodiments, Ab is selected from tusamitamab, DB1001, DB1002, cetuximab, and panitumumab, and biosimilars thereof and antigen-binding fragments thereof.

In certain embodiments, Ab is selected from tusamitamab, DB1001, DB1002, DB1003, DB1004, and DB1005, and antigen-binding fragments of the antibodies.

In certain embodiments, Ab is selected from tusamitamab, DB1001, DB1002, DB1003, or DB1004 and an antigen-binding fragment thereof.

In certain embodiments, Ab is selected from tusamitamab, DB1001, or DB1002 and an antigen-binding fragment thereof.

In certain embodiments, Ab comprises the sequences of a CDR1, a CDR2, and a CDR3 contained in a heavy chain set forth in SEQ ID NO: 1, and/or the sequences of a CDR1, a CDR2, and a CDR3 contained in a light chain set forth in SEQ ID NO: 2.

In certain embodiments, Ab comprises the sequences of a CDR1, a CDR2, and a CDR3 contained in a heavy chain set forth in SEQ ID NO: 3, and/or the sequences of a CDR1, a CDR2, and a CDR3 contained in a light chain set forth in SEQ ID NO: 4.

In certain embodiments, Ab comprises the sequences of a CDR1, a CDR2, and a CDR3 contained in a heavy chain set forth in SEQ ID NO: 5, and/or the sequences of a CDR1, a CDR2, and a CDR3 contained in a light chain set forth in SEQ ID NO: 6.

In certain embodiments, Ab comprises the sequences of a CDR1, a CDR2, and a CDR3 contained in a heavy chain set forth in SEQ ID NO: 25, and/or the sequences of a CDR1, a CDR2, and a CDR3 contained in a light chain set forth in SEQ ID NO: 26.

In certain embodiments, Ab comprises the sequences of a CDR1, a CDR2, and a CDR3 contained in a heavy chain set forth in SEQ ID NO: 35, and/or the sequences of a CDR1, a CDR2, and a CDR3 contained in a light chain set forth in SEQ ID NO: 36.

In certain embodiments, Ab comprises the sequences of a CDR1, a CDR2, and a CDR3 contained in a heavy chain set forth in SEQ ID NO: 15, and/or the sequences of a CDR1, a CDR2, and a CDR3 contained in a light chain set forth in SEQ ID NO: 16.

In certain embodiments, Ab comprises a heavy chain variable region CDR1 set forth in SEQ ID NO: 7, a heavy chain variable region CDR2 set forth in SEQ ID NO: 8, and a heavy chain variable region CDR3 set forth in SEQ ID NO: 9, and/or a light chain variable region CDR1 set forth in SEQ ID NO: 10, a light chain variable region CDR2 set forth in SEQ ID NO: 11, and a light chain variable region CDR3 set forth in SEQ ID NO: 12.

In certain embodiments, Ab comprises a heavy chain variable region CDR1 set forth in SEQ ID NO: 17, a heavy chain variable region CDR2 set forth in SEQ ID NO: 18, and a heavy chain variable region CDR3 set forth in SEQ ID NO: 19, and/or a light chain variable region CDR1 set forth in SEQ ID NO: 20, a light chain variable region CDR2 set forth in SEQ ID NO: 21, and a light chain variable region CDR3 set forth in SEQ ID NO: 22.

In certain embodiments, Ab comprises a heavy chain variable region CDR1 set forth in SEQ ID NO: 27, a heavy chain variable region CDR2 set forth in SEQ ID NO: 28, and a heavy chain variable region CDR3 set forth in SEQ ID NO: 29, and/or a light chain variable region CDR1 set forth in SEQ ID NO: 30, a light chain variable region CDR2 set forth in SEQ ID NO: 31, and a light chain variable region CDR3 set forth in SEQ ID NO: 32.

In certain embodiments, Ab comprises a heavy chain variable region CDR1 set forth in SEQ ID NO: 37, a heavy chain variable region CDR2 set forth in SEQ ID NO: 38, and a heavy chain variable region CDR3 set forth in SEQ ID NO: 39, and/or a light chain variable region CDR1 set forth in SEQ ID NO: 40, a light chain variable region CDR2 set forth in SEQ ID NO: 41, and a light chain variable region CDR3 set forth in SEQ ID NO: 42.

In certain embodiments, Ab comprises a heavy chain variable region CDR1 set forth in SEQ ID NO: 45, a heavy chain variable region CDR2 set forth in SEQ ID NO: 46, and a heavy chain variable region CDR3 set forth in SEQ ID NO: 47, and/or a light chain variable region CDR1 set forth in SEQ ID NO: 48, a light chain variable region CDR2 set forth in SEQ ID NO: 49, and a light chain variable region CDR3 set forth in SEQ ID NO: 50.

In certain embodiments, Ab comprises a heavy chain variable region CDR1 set forth in SEQ ID NO: 53, a heavy chain variable region CDR2 set forth in SEQ ID NO: 54, and a heavy chain variable region CDR3 set forth in SEQ ID NO: 55, and/or a light chain variable region CDR1 set forth in SEQ ID NO: 56, a light chain variable region CDR2 set forth in SEQ ID NO: 57, and a light chain variable region CDR3 set forth in SEQ ID NO: 58.

In certain embodiments, Ab comprises the sequence of a heavy chain variable region contained in a heavy chain set forth in SEQ ID NO: 1, and/or the sequence of a light chain variable region contained in a light chain set forth in SEQ ID NO: 2.

In certain embodiments, Ab comprises the sequence of a heavy chain variable region contained in a heavy chain set forth in SEQ ID NO: 3, and/or the sequence of a light chain variable region contained in a light chain set forth in SEQ ID NO: 4.

In certain embodiments, Ab comprises the sequence of a heavy chain variable region contained in a heavy chain set forth in SEQ ID NO: 5, and/or the sequence of a light chain variable region contained in a light chain set forth in SEQ ID NO: 6.

In certain embodiments, Ab comprises the sequence of a heavy chain variable region contained in a heavy chain set forth in SEQ ID NO: 25, and/or the sequence of a light chain variable region contained in a light chain set forth in SEQ ID NO: 26.

In certain embodiments, Ab comprises the sequence of a heavy chain variable region contained in a heavy chain set forth in SEQ ID NO: 35, and/or the sequence of a light chain variable region contained in a light chain set forth in SEQ ID NO: 36.

In certain embodiments, Ab comprises the sequence of a heavy chain variable region contained in a heavy chain set forth in SEQ ID NO: 15, and/or the sequence of a light chain variable region contained in a light chain set forth in SEQ ID NO: 16.

In certain embodiments, Ab comprises the sequence of a heavy chain variable region set forth in SEQ ID NO: 14 and/or the sequence of a light chain variable region set forth in SEQ ID NO: 13. In certain embodiments, Ab comprises the sequence of a heavy chain variable region set forth in SEQ ID NO: 24 and/or the sequence of a light chain variable region set forth in SEQ ID NO: 23. In certain embodiments, Ab comprises the sequence of a heavy chain variable region set forth in SEQ ID NO: 34 and/or the sequence of a light chain variable region set forth in SEQ ID NO: 33. In certain embodiments, Ab comprises the sequence of a heavy chain variable region set forth in SEQ ID NO: 44 and/or the sequence of a light chain variable region set forth in SEQ ID NO: 43. In certain embodiments, Ab comprises the sequence of a heavy chain variable region set forth in SEQ ID NO: 52 and/or the sequence of a light chain variable region set forth in SEQ ID NO: 51. In certain embodiments, Ab comprises the sequence of a heavy chain variable region set forth in SEQ ID NO: 60 and/or the sequence of a light chain variable region set forth in SEQ ID NO: 59. In certain embodiments, the amino acid sequence of a heavy chain of Ab is set forth in SEQ ID NO: 1, and the amino acid sequence of a light chain of Ab is set forth in SEQ ID NO: 2.

In certain embodiments, the amino acid sequence of a heavy chain of Ab is set forth in SEQ ID NO: 3, and the amino acid sequence of a light chain of Ab is set forth in SEQ ID NO: 4.

In certain embodiments, the amino acid sequence of a heavy chain of Ab is set forth in SEQ ID NO: 5, and the amino acid sequence of a light chain of Ab is set forth in SEQ ID NO: 6.

In certain embodiments, the amino acid sequence of a heavy chain of Ab is set forth in SEQ ID NO: 25, and the amino acid sequence of a light chain of Ab is set forth in SEQ ID NO: 26.

In certain embodiments, the amino acid sequence of a heavy chain of Ab is set forth in SEQ ID NO: 35, and the amino acid sequence of a light chain of Ab is set forth in SEQ ID NO: 36.

In certain embodiments, the amino acid sequence of a heavy chain of Ab is set forth in SEQ ID NO: 15, and the amino acid sequence of a light chain of Ab is set forth in SEQ ID NO: 16.

In certain embodiments, Ab is an anti-CEACAM5 antibody or an antigen-binding fragment thereof, e.g., tusamitamab or an antigen-binding fragment thereof or a variant thereof.

In certain embodiments, Ab comprises a heavy chain and a light chain of the anti-CEACAM5 antibody or the antigen-binding fragment thereof, wherein the amino acid sequence of the heavy chain is a sequence having at least 80%, 85%, 90%, 95%, 96%, 97%, 98%, or 99% identity to SEQ ID NO: 1 or is set forth in SEQ ID NO: 1, and the amino acid sequence of the light chain is a sequence having at least 80%, 85%, 90%, 95%, 96%, 97%, 98%, or 99% identity to SEQ ID NO: 2 or is set forth in SEQ ID NO: 2.

In certain embodiments, Ab is an anti-HER3 antibody or an antigen-binding fragment thereof, e.g., DB1001 or an antigen-binding fragment thereof or a variant thereof.

In certain embodiments, Ab comprises a heavy chain and a light chain of the anti-HER3 antibody or the antigen-binding fragment thereof, wherein the amino acid sequence of the heavy chain is a sequence having at least 80%, 85%, 90%, 95%, 96%, 97%, 98%, or 99% identity to SEQ ID NO: 3 or is set forth in SEQ ID NO: 3, and the amino acid sequence of the light chain is a sequence having at least 80%, 85%, 90%, 95%, 96%, 97%, 98%, or 99% identity to SEQ ID NO: 4 or is set forth in SEQ ID NO: 4.

In certain embodiments, Ab is an anti-GPC-3 antibody or an antigen-binding fragment thereof, e.g., DB 1002 or an antigen-binding fragment thereof or a variant thereof.

In certain embodiments, Ab comprises a heavy chain and a light chain of the anti-GPC-3 antibody or the antigen-binding fragment thereof, wherein the amino acid sequence of the heavy chain is a sequence having at least 80%, 85%, 90%, 95%, 96%, 97%, 98%, or 99% identity to SEQ ID NO: 5 or is set forth in SEQ ID NO: 5, and the amino acid sequence of the light chain is a sequence having at least 80%, 85%, 90%, 95%, 96%, 97%, 98%, or 99% identity to SEQ ID NO: 6 or is set forth in SEQ ID NO: 6.

In certain embodiments, Ab is an anti-ADAM9 antibody or an antigen-binding fragment thereof, e.g., DB 1003 or an antigen-binding fragment thereof or a variant thereof.

In certain embodiments, Ab comprises a heavy chain and a light chain of the anti-ADAM9 antibody or the antigen-binding fragment thereof, wherein the amino acid sequence of the heavy chain is a sequence having at least 80%, 85%, 90%, 95%, 96%, 97%, 98%, or 99% identity to SEQ ID NO: 25 or is set forth in SEQ ID NO: 25, and the amino acid sequence of the light chain is a sequence having at least 80%, 85%, 90%, 95%, 96%, 97%, 98%, or 99% identity to SEQ ID NO: 26 or is set forth in SEQ ID NO: 26.

In certain embodiments, Ab is an anti-BCMA antibody or an antigen-binding fragment thereof, e.g., DB 1004 or an antigen-binding fragment thereof or a variant thereof.

In certain embodiments, Ab comprises a heavy chain and a light chain of the anti-BCMA antibody or the antigen-binding fragment thereof, wherein the amino acid sequence of the heavy chain is a sequence having at least 80%, 85%, 90%, 95%, 96%, 97%, 98%, or 99% identity to SEQ ID NO: 35 or is set forth in SEQ ID NO: 35, and the amino acid sequence of the light chain is a sequence having at least 80%, 85%, 90%, 95%, 96%, 97%, 98%, or 99% identity to SEQ ID NO: 36 or is set forth in SEQ ID NO: 36.

In certain embodiments, Ab is an anti-c-met antibody or an antigen-binding fragment thereof, e.g., DB1005 or an antigen-binding fragment thereof or a variant thereof.

In certain embodiments, Ab comprises a heavy chain and a light chain of the anti-c-met antibody or the antigen-binding fragment thereof, wherein the amino acid sequence of the heavy chain is a sequence having at least 80%, 85%, 90%, 95%, 96%, 97%, 98%, or 99% identity to SEQ ID NO: 15 or is set forth in SEQ ID NO: 15, and the amino acid sequence of the light chain is a sequence having at least 80%, 85%, 90%, 95%, 96%, 97%, 98%, or 99% identity to SEQ ID NO: 16 or is set forth in SEQ ID NO: 16.

In certain embodiments, k is selected from integers or decimals from 1 to 10.

In certain embodiments, k is selected from integers or decimals from 2 to 9.

In certain embodiments, k is selected from integers or decimals from 2 to 8.

In certain embodiments, k is selected from integers or decimals from 4 to 8 (e.g., about 4, about 4.5, about 5, about 5.5, about 6, about 6.5, about 7, about 7.5, or about 8).

In certain embodiments, k is selected from integers or decimals from 6 to 8 (e.g., about 6.1, about 6.3, about 6.5, about 6.7, about 6.9, about 7.1, about 7.3, about 7.5, about 7.7, or about 8).

In certain embodiments, k is selected from integers or decimals from 7 to 9 (e.g., about 7.1, about 7.2, about 7.3, about 7.4, about 7.5, about 7.6, about 7.7, about 7.8, about 7.9, about 8, about 8.1, about 8.2, about 8.3, about 8.4, or about 8.5).

In certain embodiments, k is selected from integer or decimals from 7.5 to 8.1 (e.g., 7.81, 7.82, 7.83, 7.84, 7.85, 7.86, 7.87, 7.88, 7.89, 7.90, 7.91, 7.92, 7.93, 7.94, 7.95, 7.96, 7.97, 7.98, 7.99, 8.00, 8.01, 8.02, 8.03, 8.04, 8.05, 8.06, 8.07, 8.08, 8.09, or 8.10).

In certain embodiments, k is selected from integers or decimals from 7 to 8 (e.g., about 7.1, about 7.2, about 7.3, about 7.4, about 7.5, about 7.6, about 7.7, about 7.8, about 7.9, or about 8).

In certain embodiments, k is selected from integers or decimals from 7.5 to 8.

In certain embodiments, k is selected from a number from 2 to 8, e.g., 2, 3, 4, 5, 6, 7, or 8. In certain embodiments, k is selected from 6 and 8.

In certain embodiments, k is selected from integers from 1 to 10. In certain embodiments, k is selected from integers from 2 to 8 (e.g., 2, 3, 4, 5, 6, 7, or 8), e.g., integers from 4 to 8 (e.g., 4, 5, 6, 7, or 8), e.g., integers from 6 to 8 (e.g., 6, 7, or 8), e.g., integers from 7 to 8.

In certain embodiments, the ligand-drug conjugate is selected from a compound represented by formula I-C-1, II-C-1', II-C-1, III-C-1', III-C-1, IV-C-1, or V-C-1: 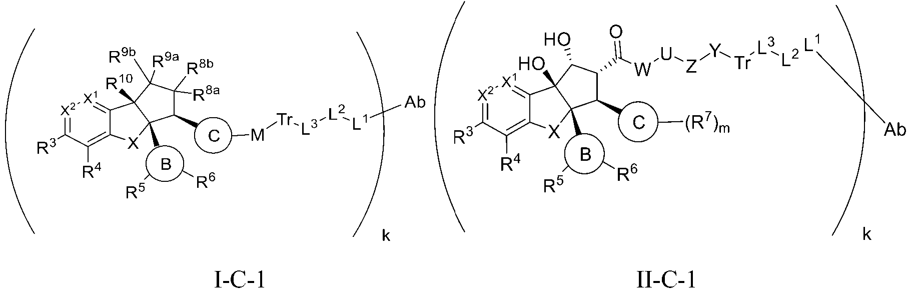 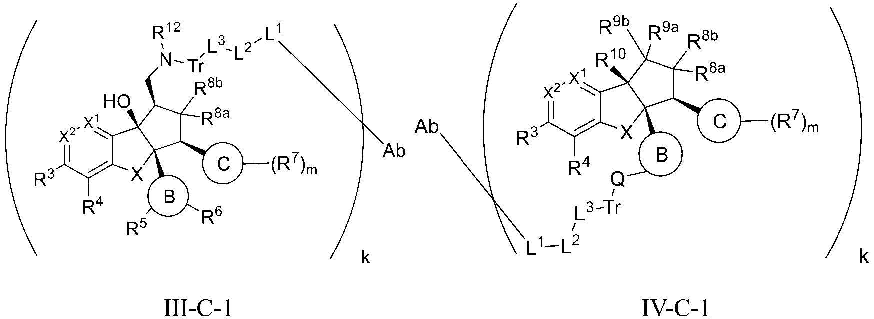 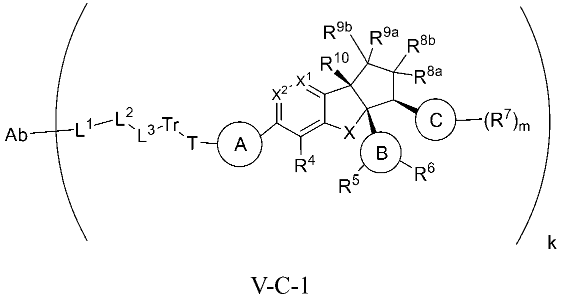 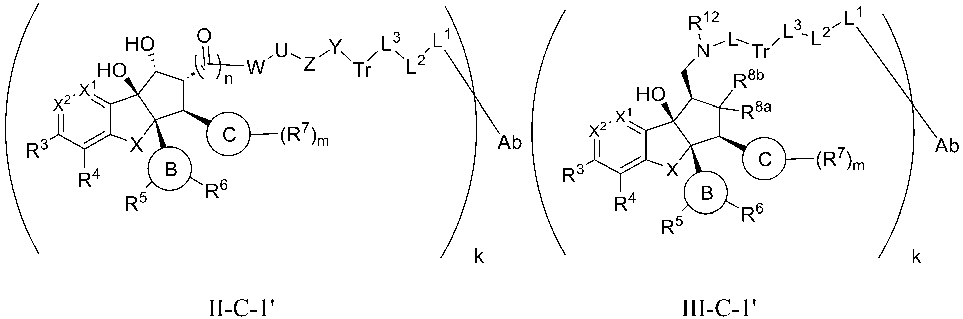 wherein X, X¹, X², ring A, ring B, ring C, R³, R⁴, R⁵, R⁶, R⁷, R^{8a}, R^{8b}, R^{9a}, R^{9b}, R¹⁰, R¹², m, n, M, W, U, Z, Y, L, Q, T, Tr, L¹, L², L³, Ab, and k are defined as described in any one of the embodiments of the present disclosure.

In certain embodiments, the ligand-drug conjugate is selected from a compound represented by formula I-C-2, II-C-2', II-C-2, III-C-2', III-C-2, IV-C-2, or V-C-2: 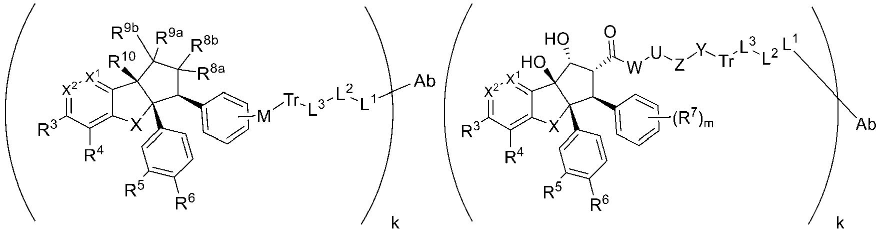 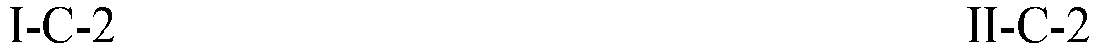 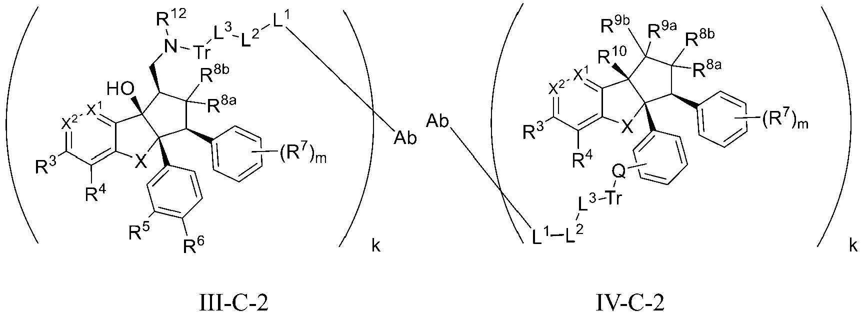 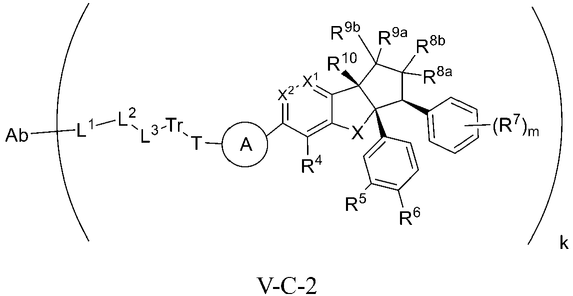 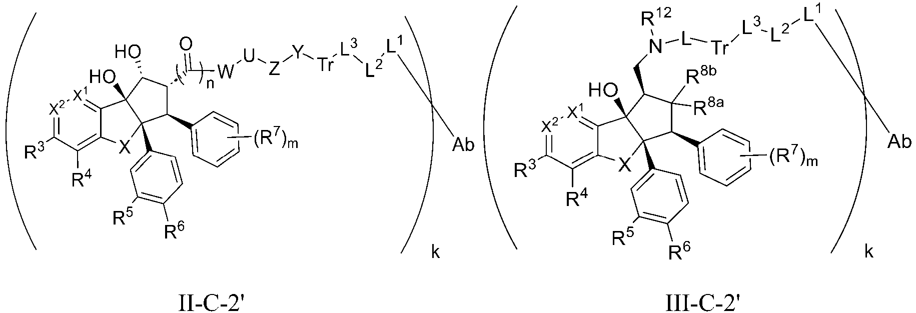 wherein X, X¹, X², ring A, R³, R⁴, R⁵, R⁶, R⁷, R^{8a}, R^{8b}, R^{9a}, R^{9b}, R¹⁰, R¹², m, n, M, W, U, Z, Y, L, Q, T, Tr, L¹, L², L³, Ab, and k are defined as described in any one of the embodiments of the present disclosure.

In certain embodiments, the ligand-drug conjugate is selected from a compound represented by formula I-C-3, III-C-3', III-C-3, IV-C-3, or V-C-3: 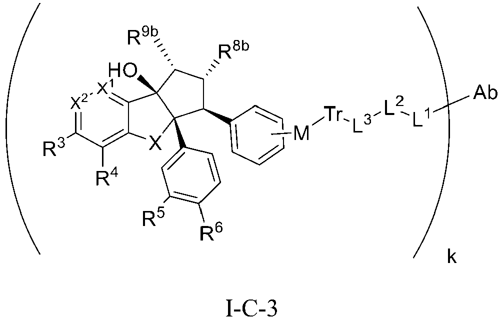 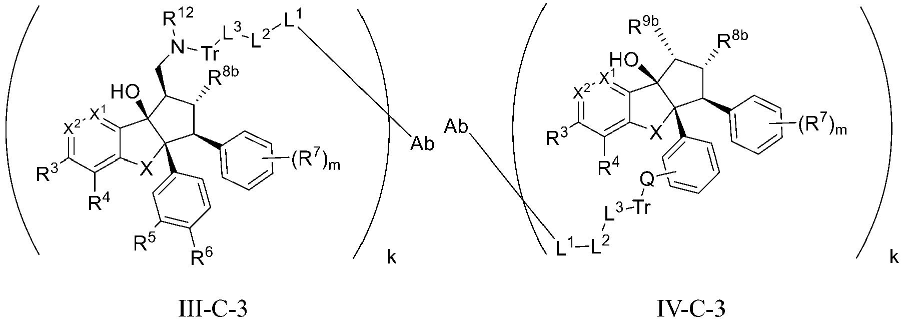 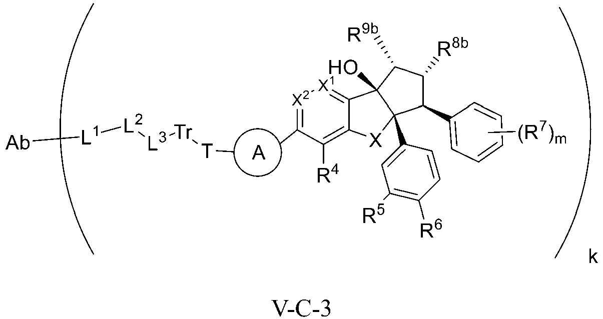 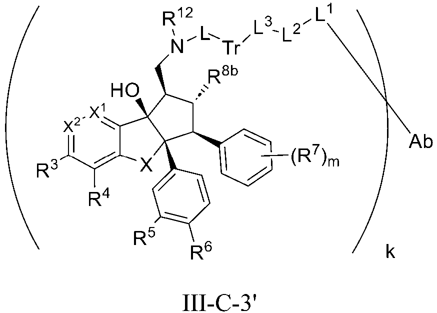 wherein X, X¹, X², ring A, R³, R⁴, R⁵, R⁶, R⁷, R^{8b}, R^{9b}, R¹², m, M, L, Q, T, Tr, L¹, L², L³, Ab, and k are defined as described in any one of the embodiments of the present disclosure.

In certain embodiments, the ligand-drug conjugate is selected from a compound represented by formula I-C-4, II-C-4', II-C-4, III-C-4', III-C-4, IV-C-4, or V-C-4: 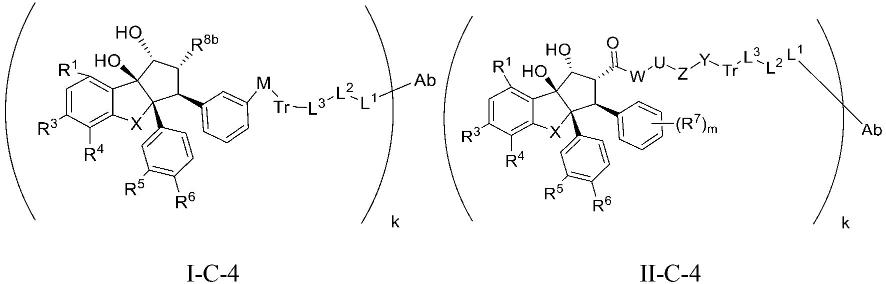 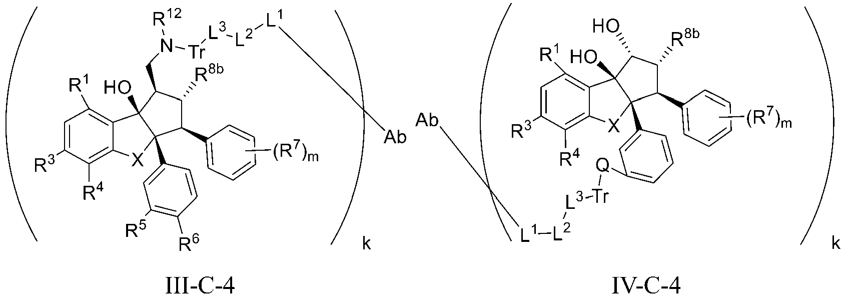 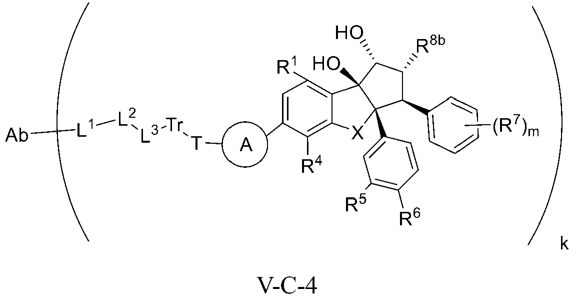 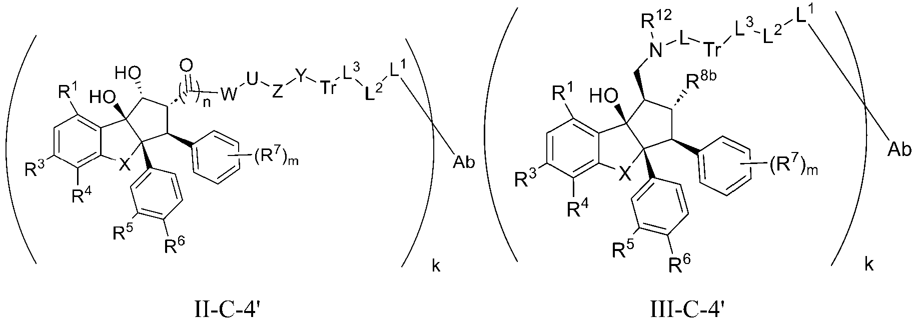 wherein X, R¹, ring A, R³, R⁴, R⁵, R⁶, R⁷, R^{8b}, R¹², m, n, M, W, U, Z, Y, L, Q, T, Tr, L¹, L², L³, Ab, and k are defined as described in any one of the embodiments of the present disclosure.

In certain embodiments, the ligand-drug conjugate is selected from a compound represented by formula I-C-5, II-C-5, III-C-5, IV-C-5, or V-C-5: 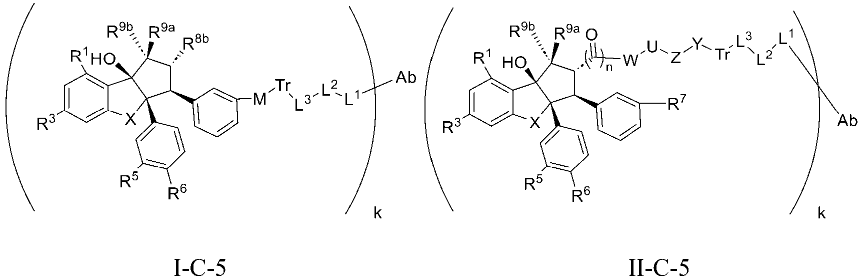 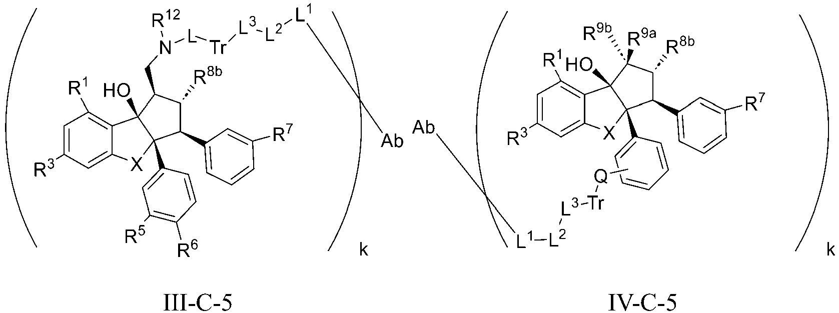 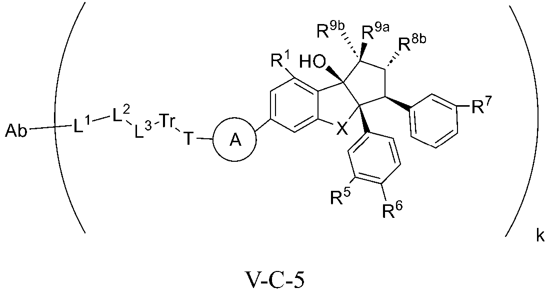 wherein X, R¹, ring A, R³, R⁵, R⁶, R⁷, R^{8b}, R^{9a}, R^{9b}, R¹², n, M, W, U, Z, Y, L, Q, T, Tr, L¹, L², L³, Ab, and k are defined as described in any one of the embodiments of the present disclosure.

In certain embodiments, the ligand-drug conjugate is selected from compounds represented by formulas I-C-6 and I-C-7: 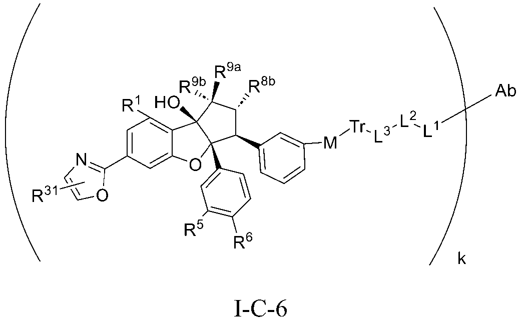 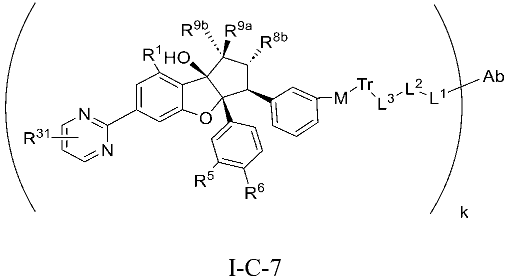 wherein R¹, R³¹, R⁵, R⁶, R^{8b}, R^{9a}, R^{9b}, M, Tr, L¹, L², L³, Ab, and k are defined as described in any one of the embodiments of the present disclosure.

In certain embodiments, the ligand-drug conjugate is selected from the structures shown in Table C:

**Table C**

| No. | Structure |
|---|---|
| I-C1 | 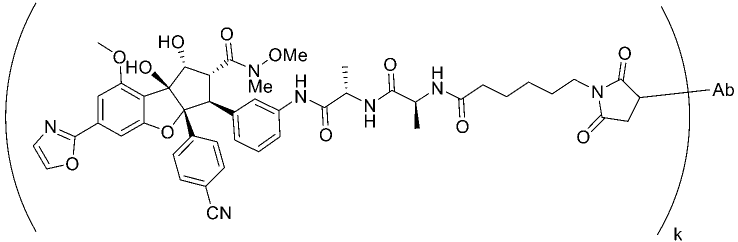 |
| I-C2 | 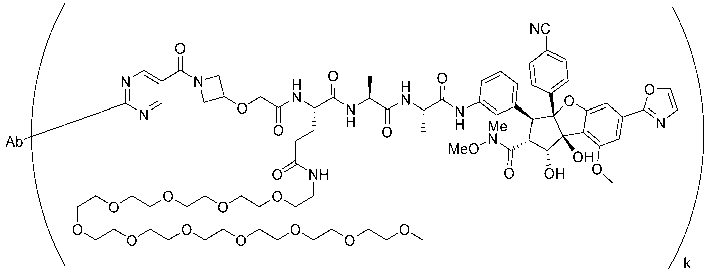 |
| I-C3 | 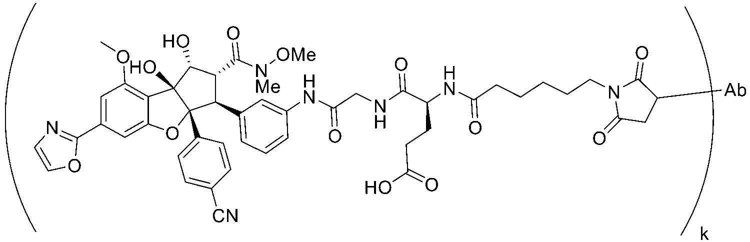 |
| I-C4 | 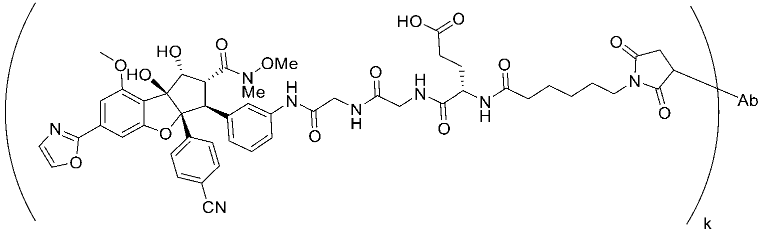 |
| I-C5 | 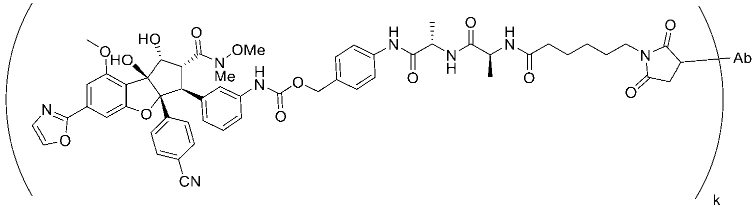 |
| I-C6 | 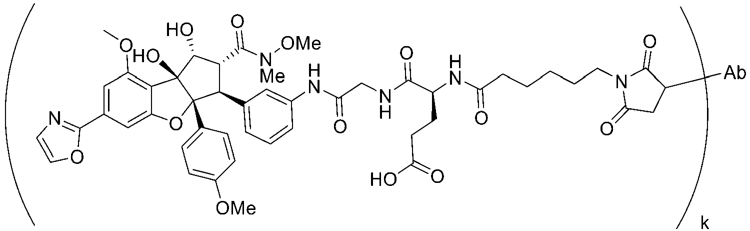 |
| I-C7 | 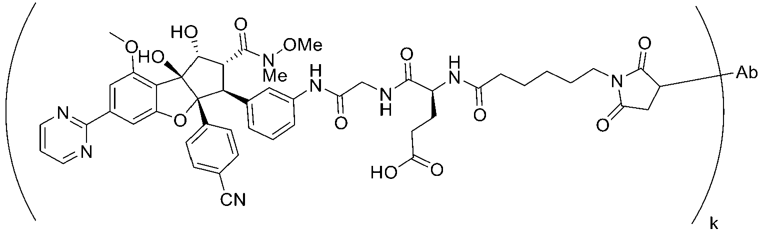 |
| I-C8 | 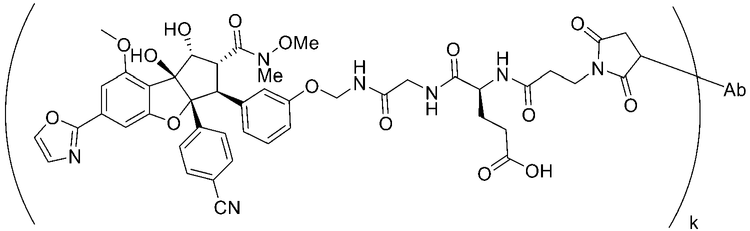 |
| I-C9 | 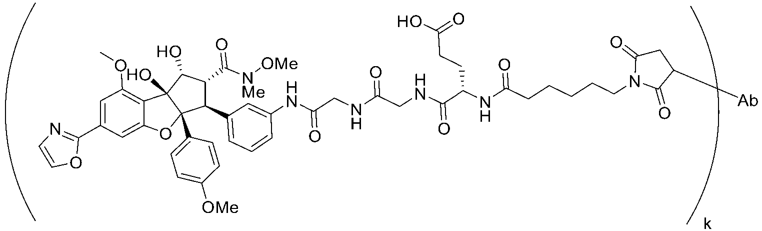 |
| I-C10 | 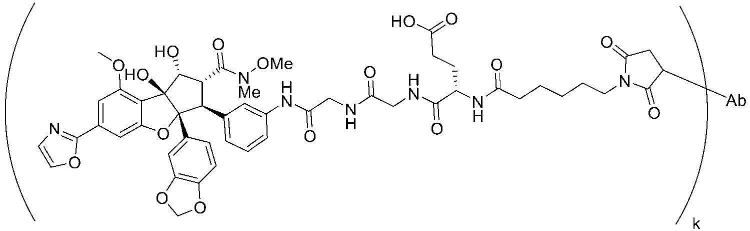 |
| I-C11 | 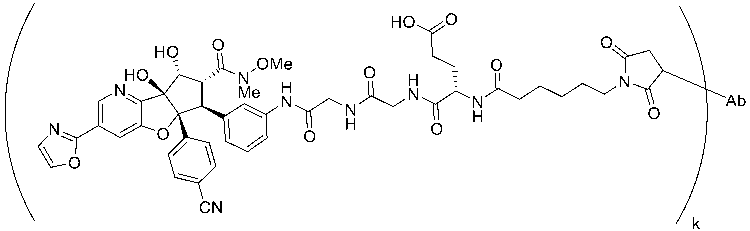 |
| I-C12 | 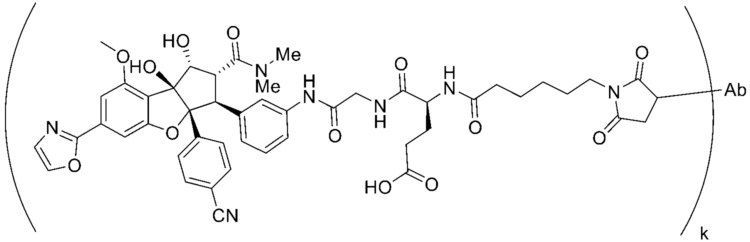 |
| I-C13 | 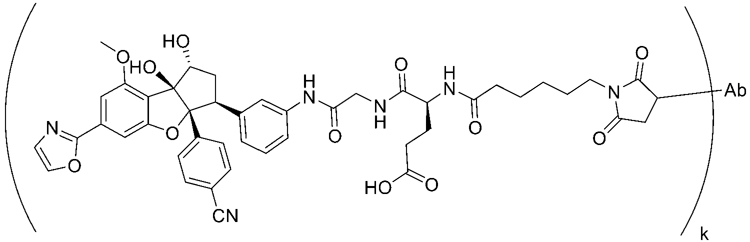 |
| I-C14 | 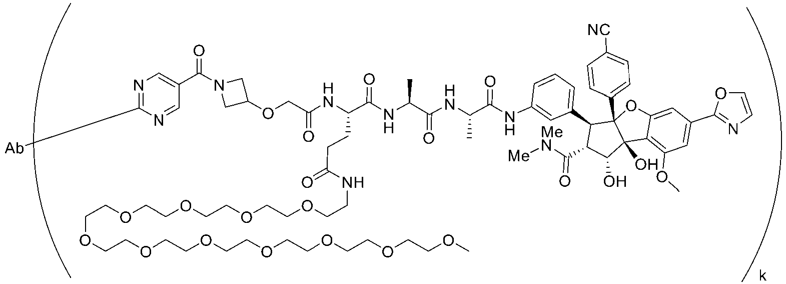 |
| I-C15 | 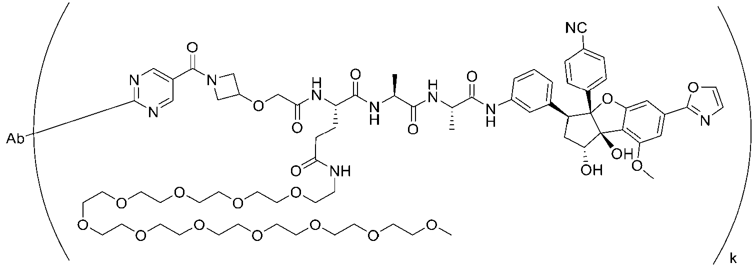 |
| I-C16 | 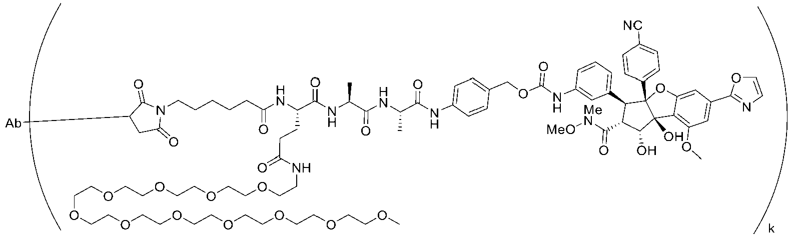 |
| I-C17 | 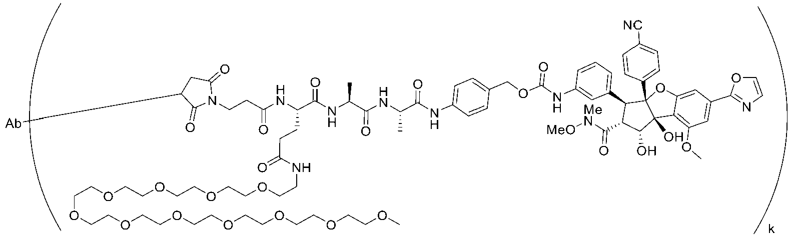 |
| I-C18 | 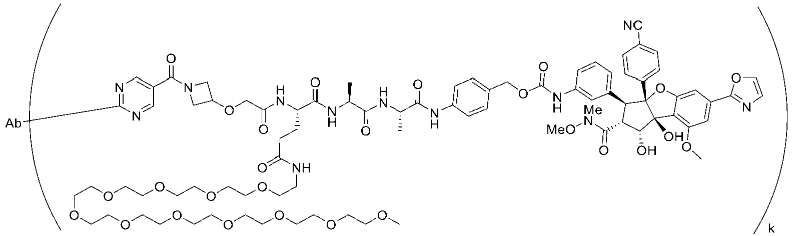 |
| I-C19 | 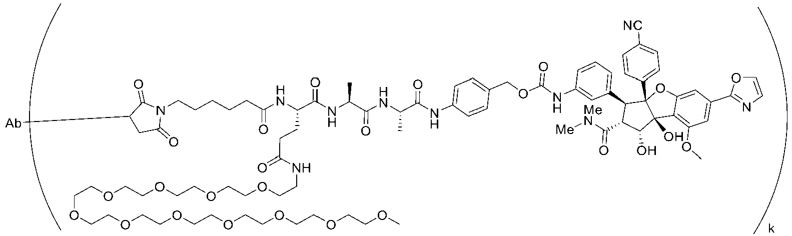 |
| I-C20 | 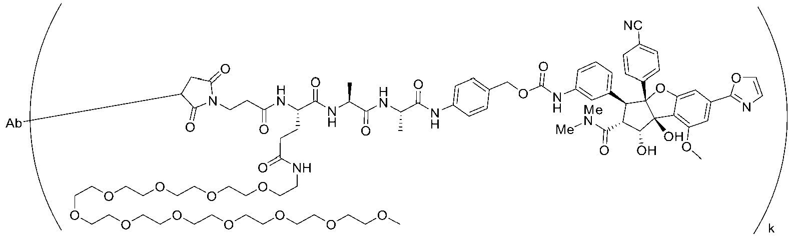 |
| I-C21 | 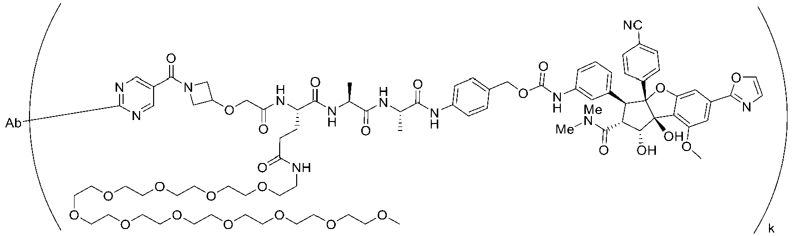 |
| I-C22 | 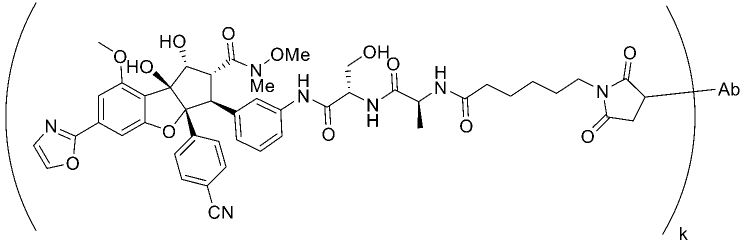 |
| I-C23 | 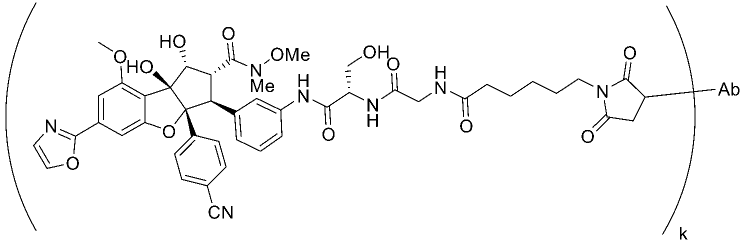 |
| I-C24 | 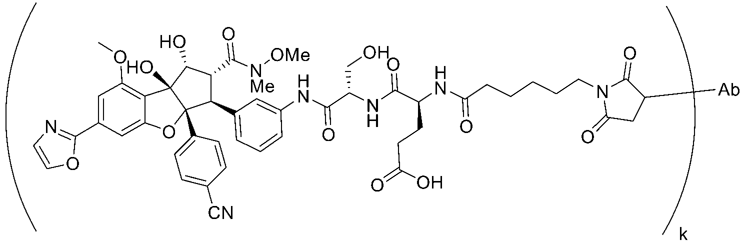 |
| I-C25 | 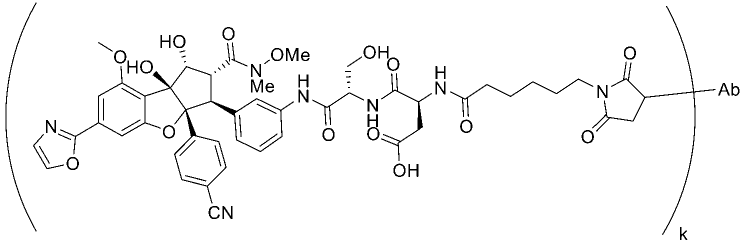 |
| I-C26 | 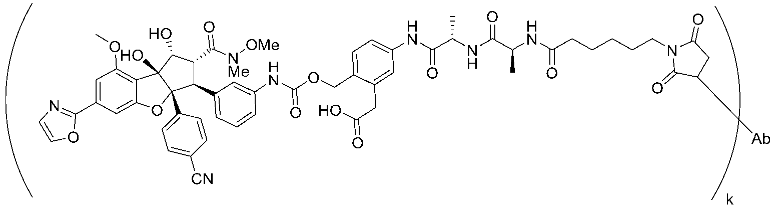 |
| I-C27 | 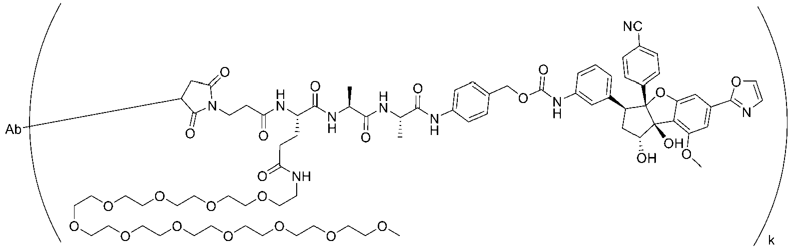 |
| I-C28 | 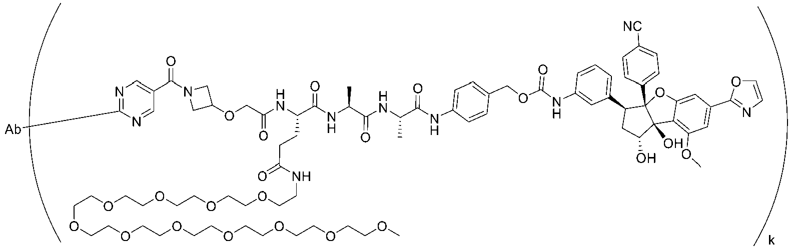 |
| I-C29 | 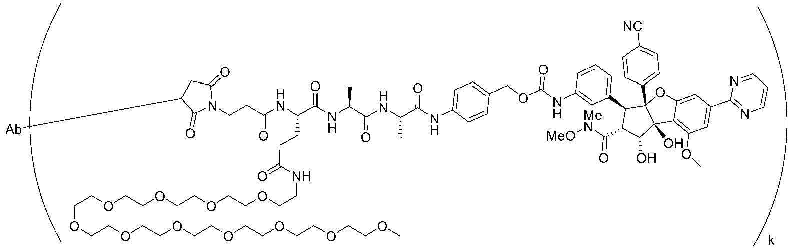 |
| I-C30 | 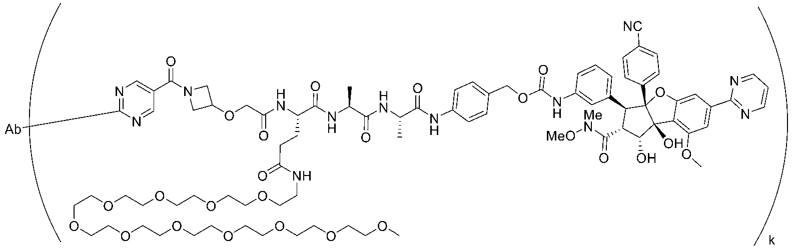 |
| I-C31 | 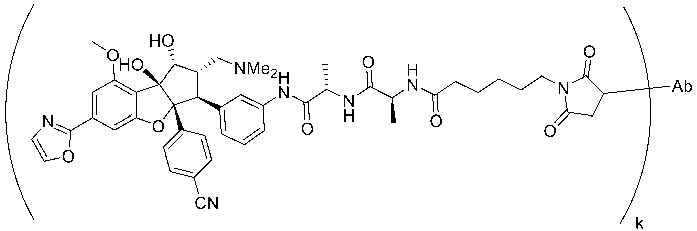 |
| I-C32 | 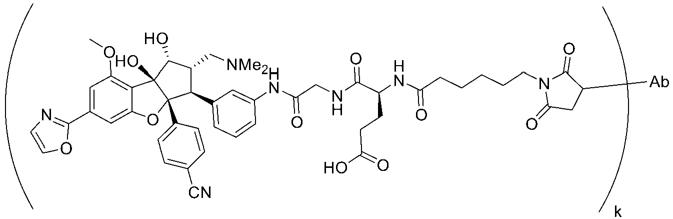 |
| I-C33 | 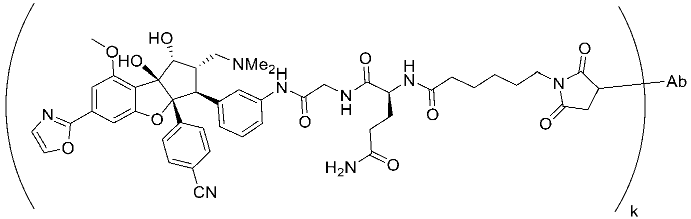 |
| I-C34 | 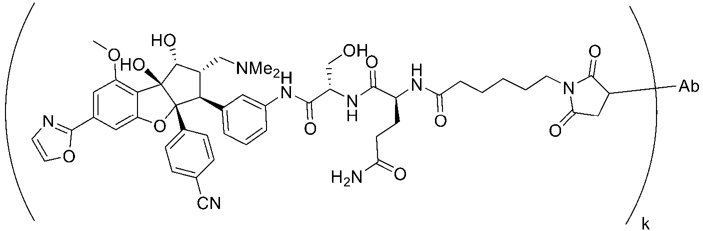 |
| I-C35 | 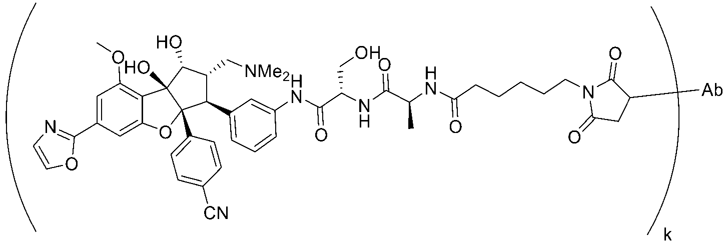 |
| I-C36 | 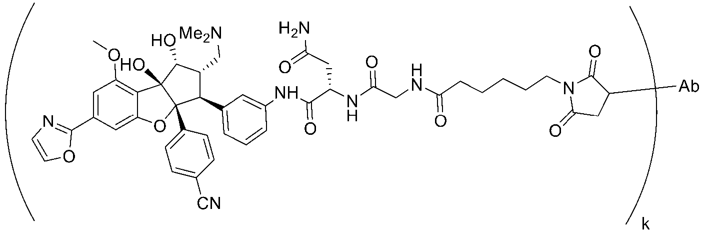 |
| I-C37 | 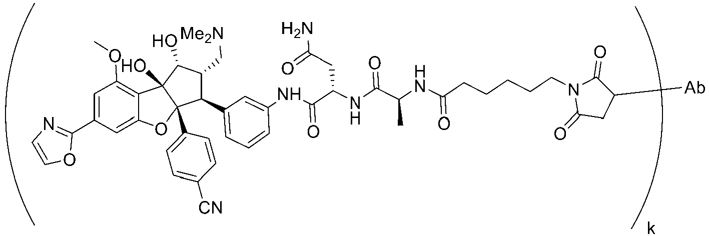 |
| I-C38 | 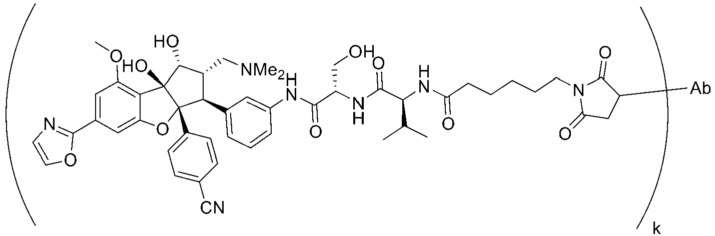 |
| I-C39 | 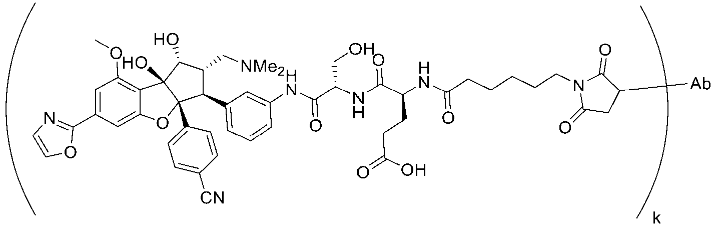 |
| I-C40 | 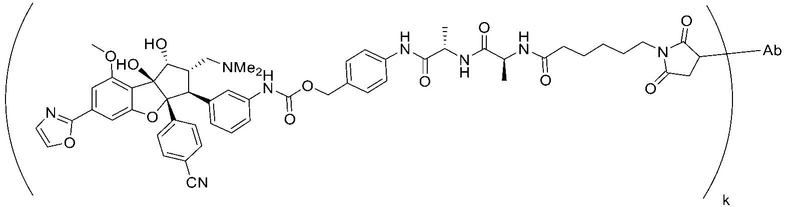 |
| I-C41 | 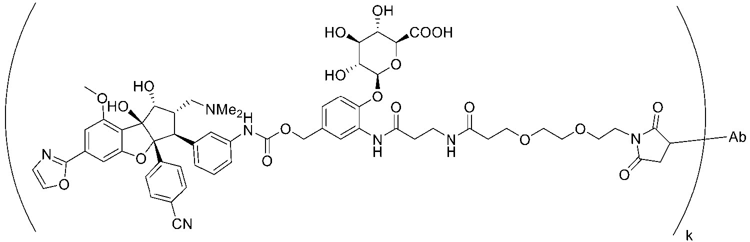 |
| I-C42 | 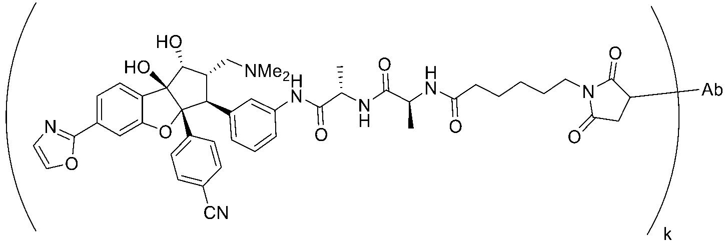 |
| I-C43 | 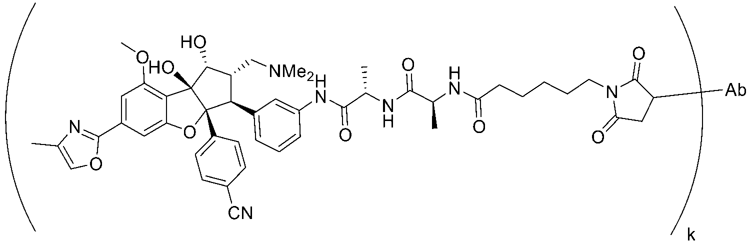 |
| I-C44 | 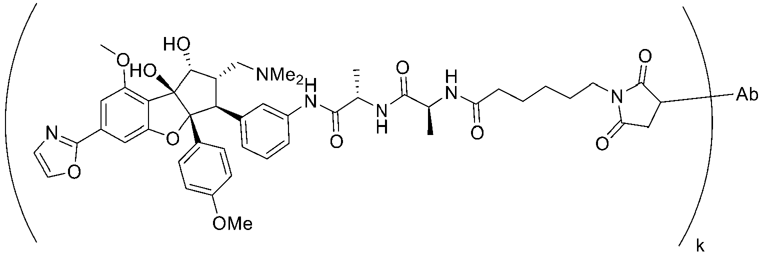 |
| I-C45 | 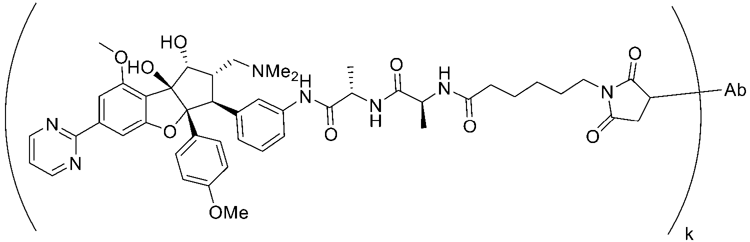 |
| I-C46 | 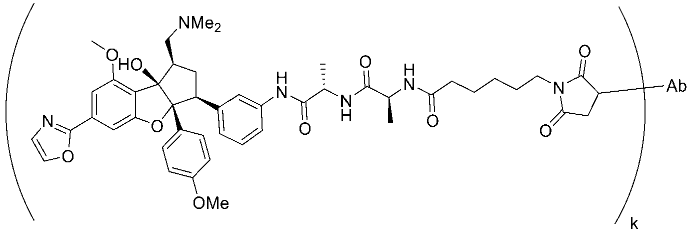 |
| I-C47 | 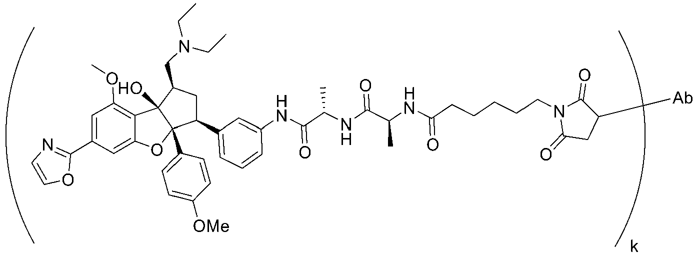 |
| I-C48 | 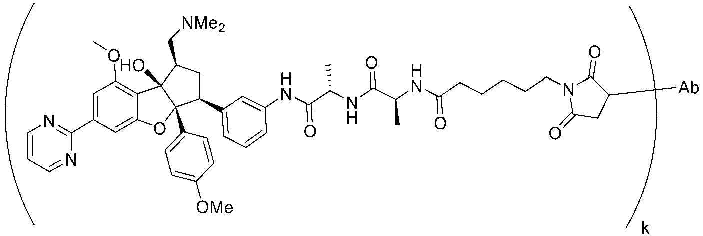 |
| I-C49 | 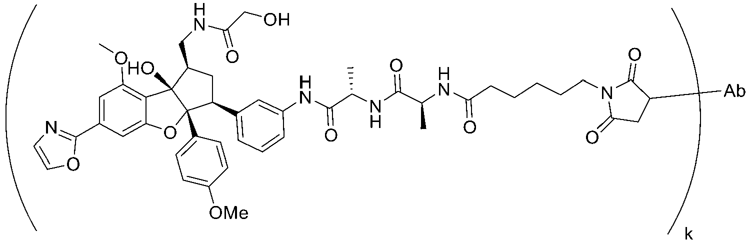 |
| I-C50 | 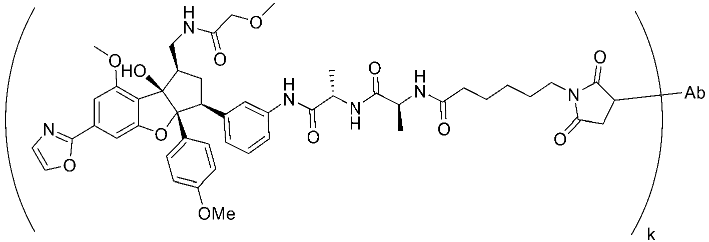 |
| I-C51 | 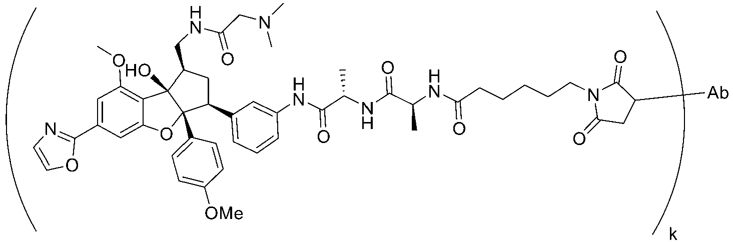 |
| I-C52 | 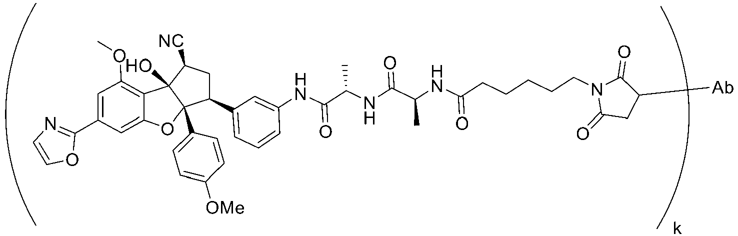 |
| I-C53 | 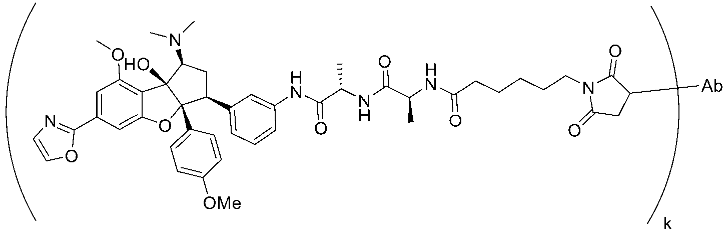 |
| I-C54 | 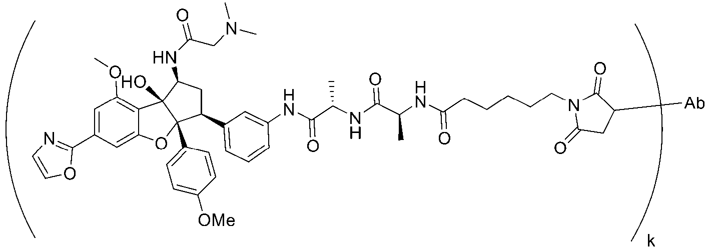 |
| I-C55 | 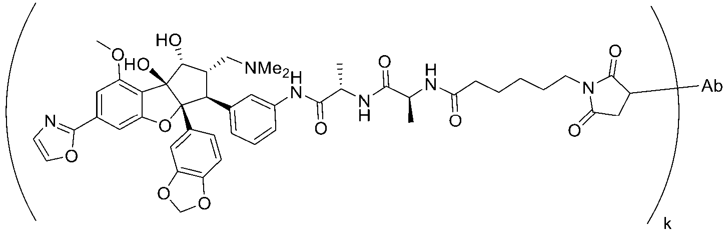 |
| I-C56 | 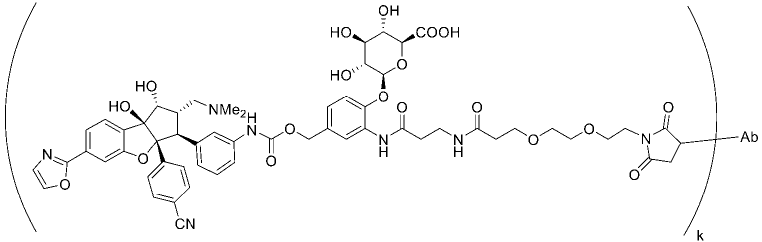 |
| I-C57 | 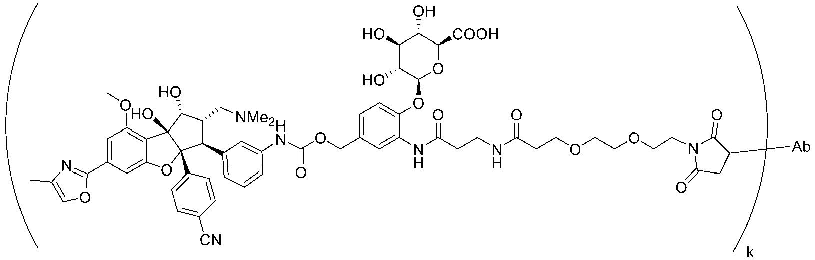 |
| I-C58 | 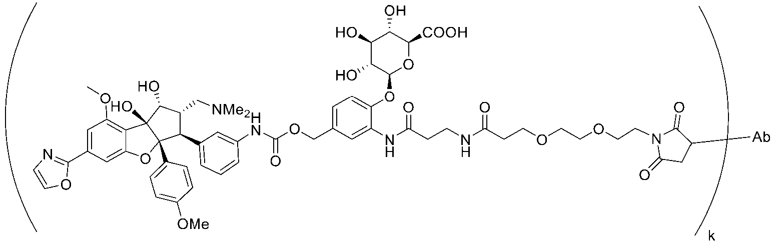 |
| I-C59 | 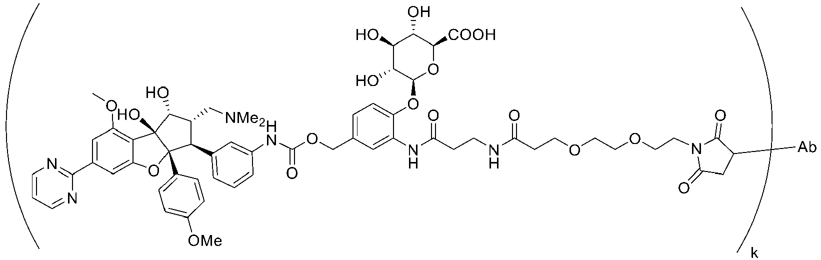 |
| I-C60 | 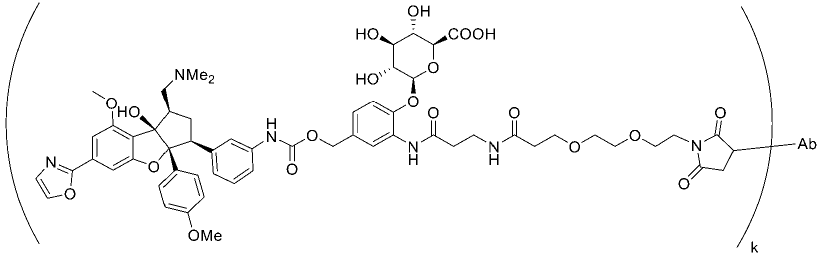 |
| I-C61 | 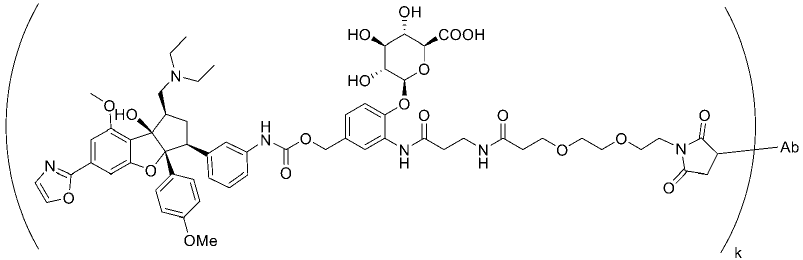 |
| I-C62 | 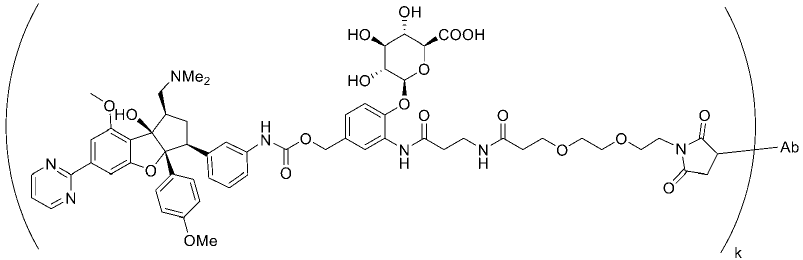 |
| I-C63 | 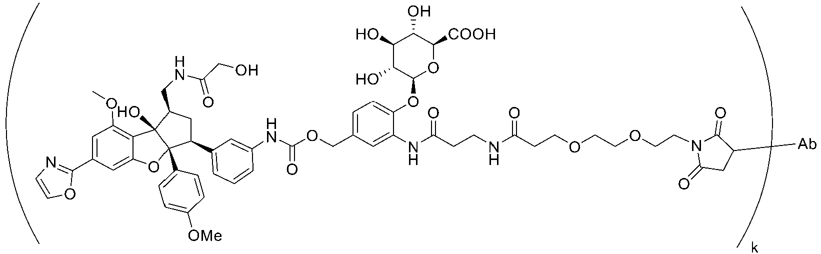 |
| I-C64 | 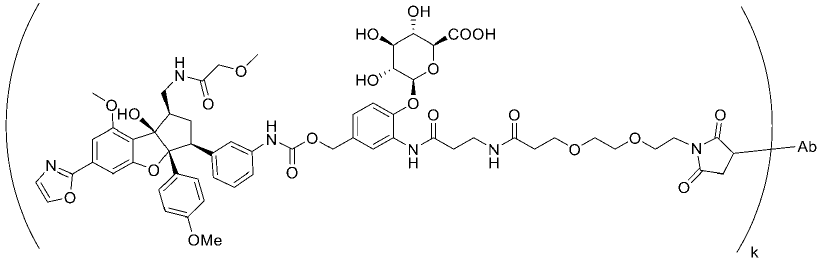 |
| I-C65 | 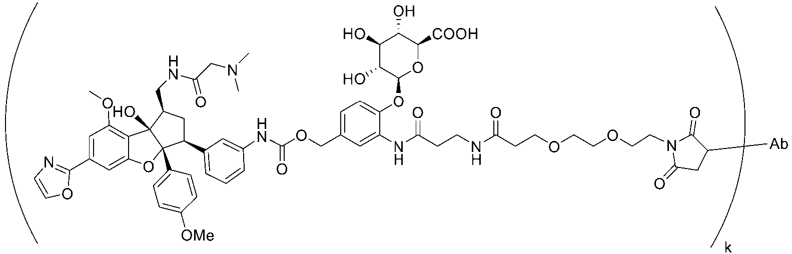 |
| I-C66 | 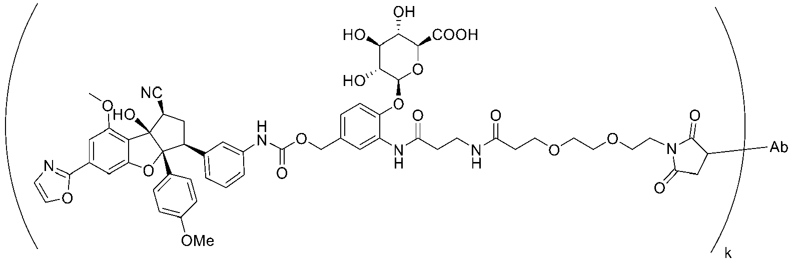 |
| I-C67 | 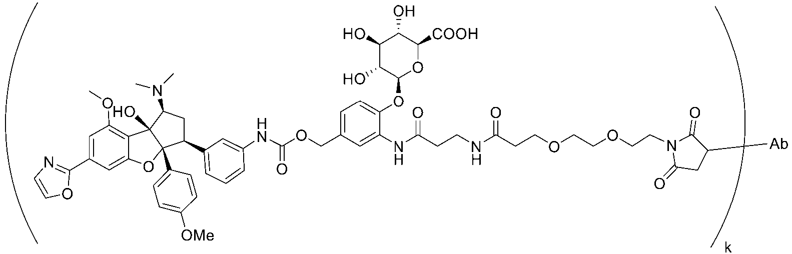 |
| I-C68 | 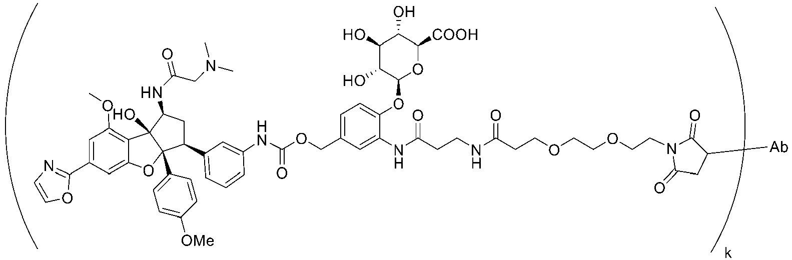 |
| I-C69 | 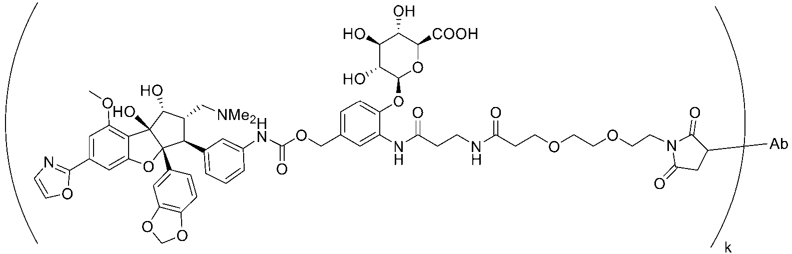 |
| I-C70 | 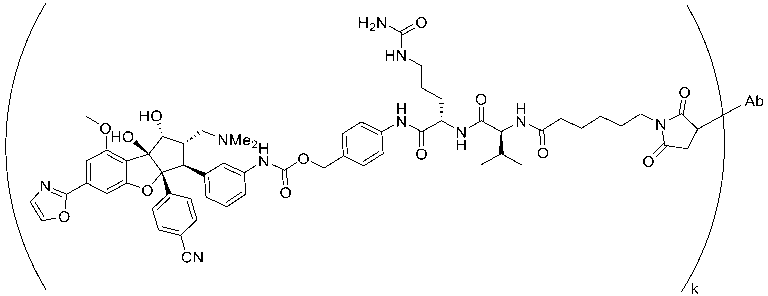 |
| I-C71 | 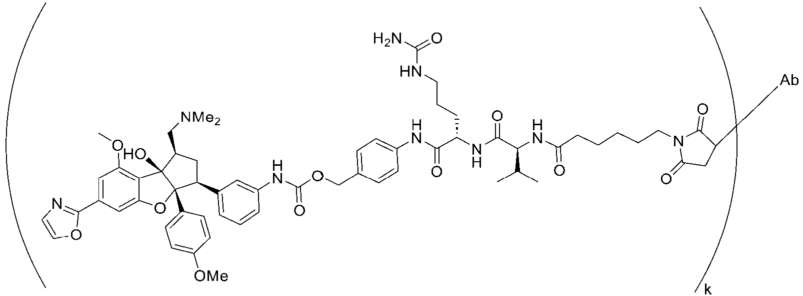 |
| I-C72 | 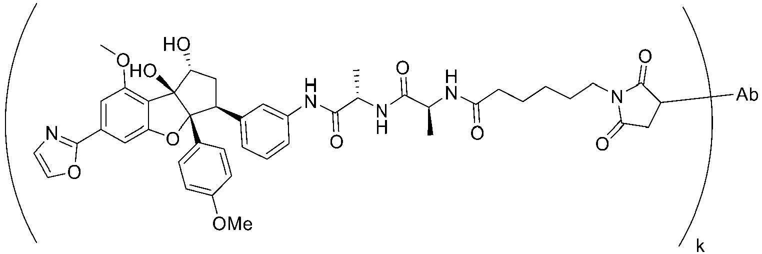 |
| I-C73 | 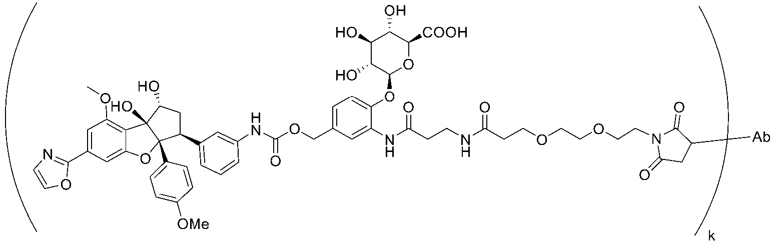 |
| I-C74 | 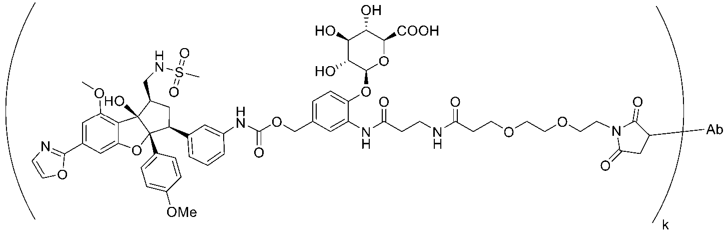 |
| II-C1 | 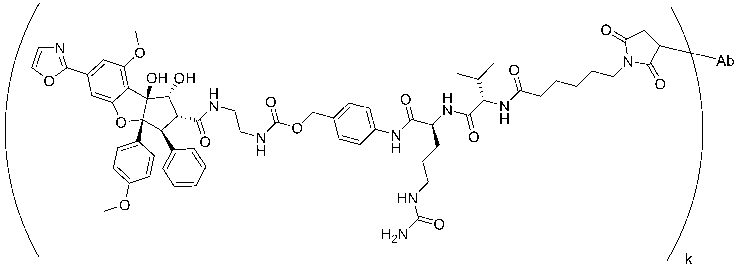 |
| II-C2 | 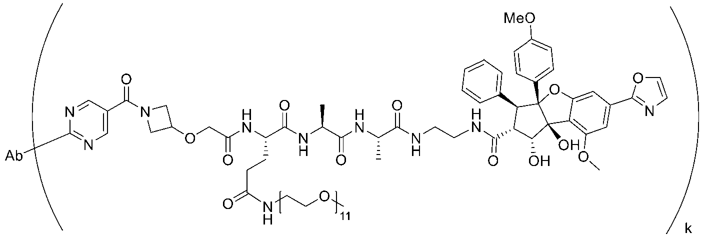 |
| II-C3 | 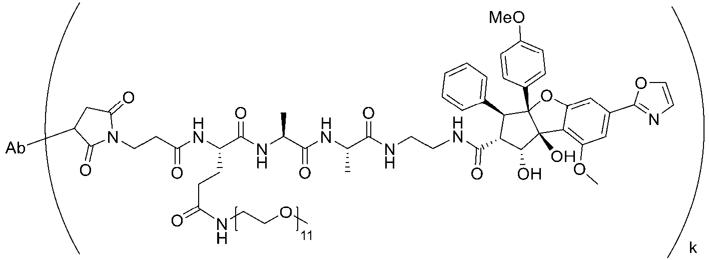 |
| II-C4 | 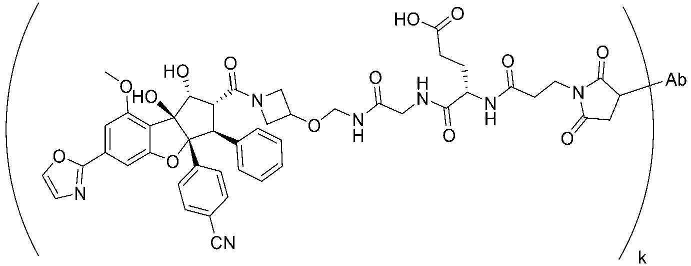 |
| II-C5 | 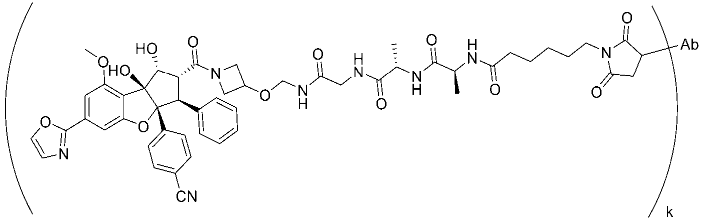 |
| II-C6 | 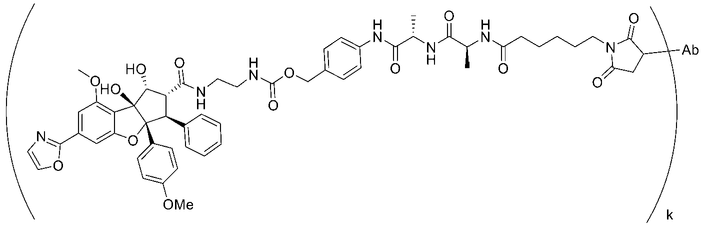 |
| II-C7 | 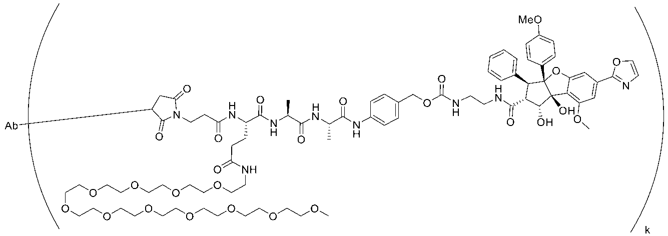 |
| II-C8 | 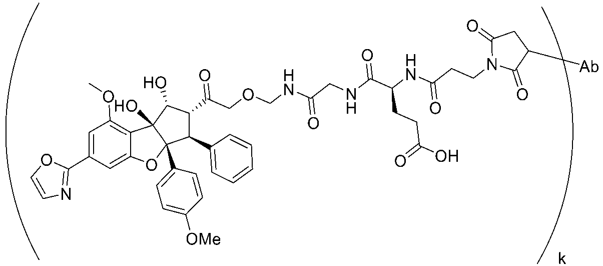 |
| II-C9 | 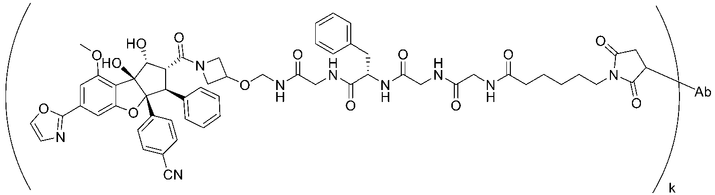 |
| II-C10 | 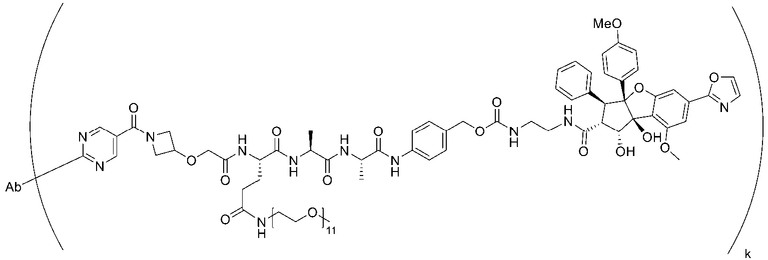 |
| II-C11 | 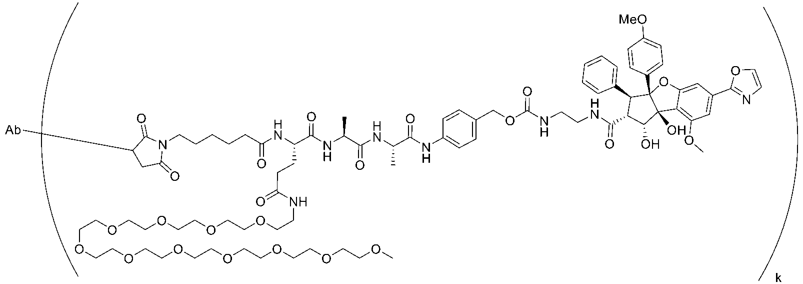 |
| II-C12 | 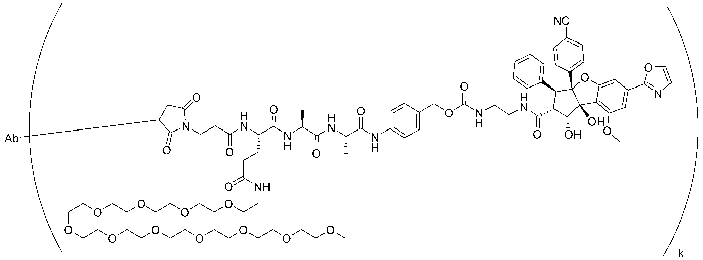 |
| II-C13 | 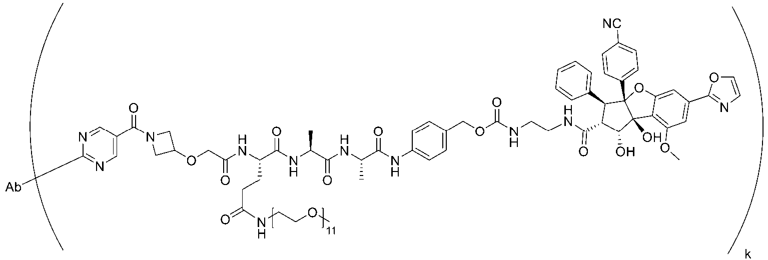 |
| II-C14 | 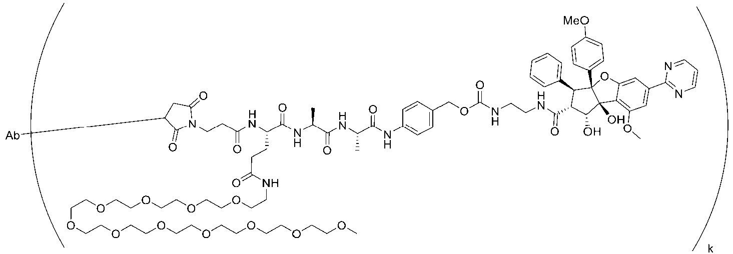 |
| II-C15 | 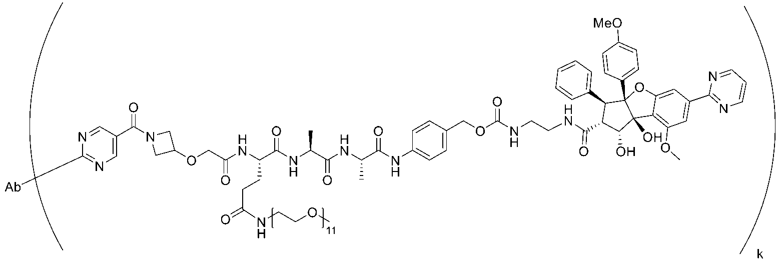 |
| II-C16 | 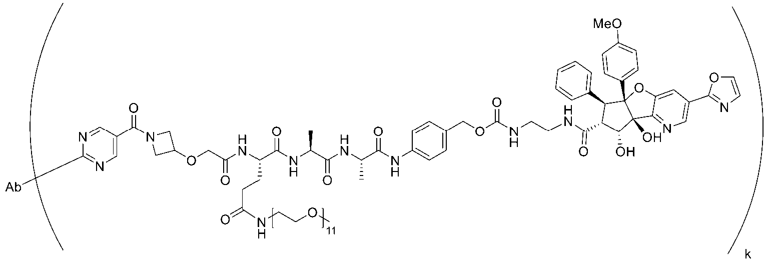 |
| II-C17 | 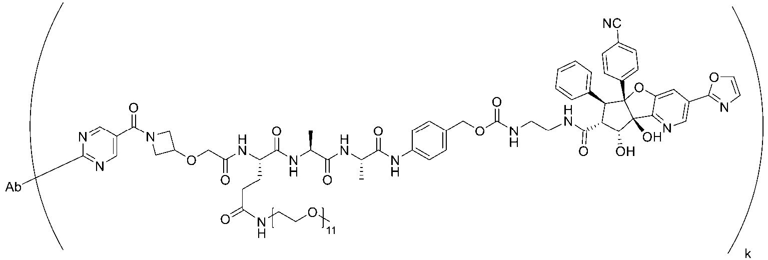 |
| II-C18 | 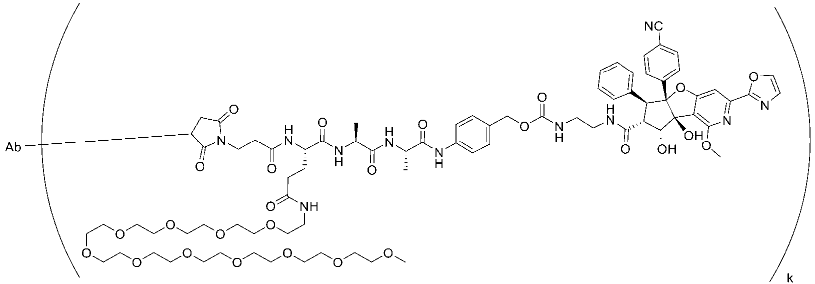 |
| II-C19 | 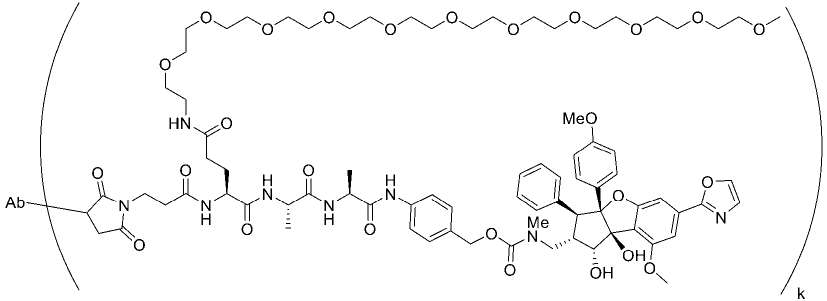 |
| II-C20 | 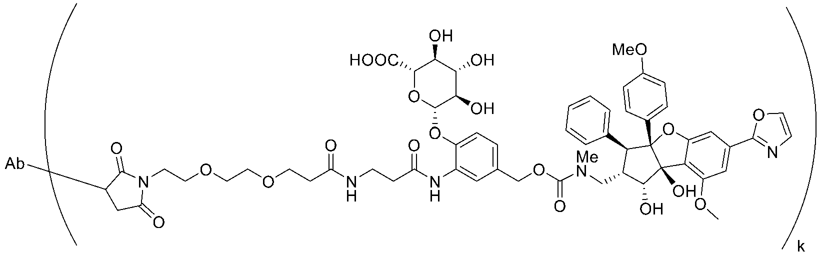 |
| II-C21 | 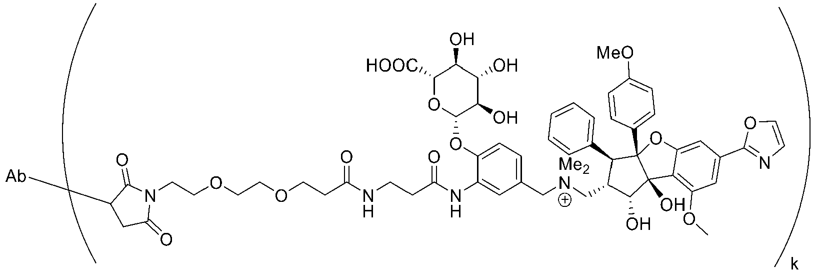 |
| III-C1 | 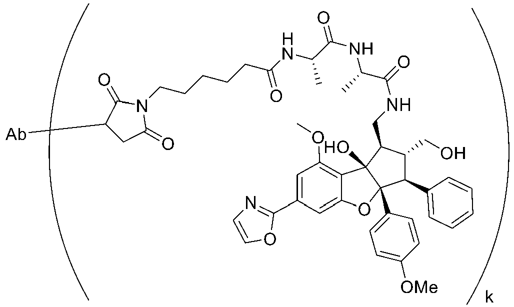 |
| III-C2 | 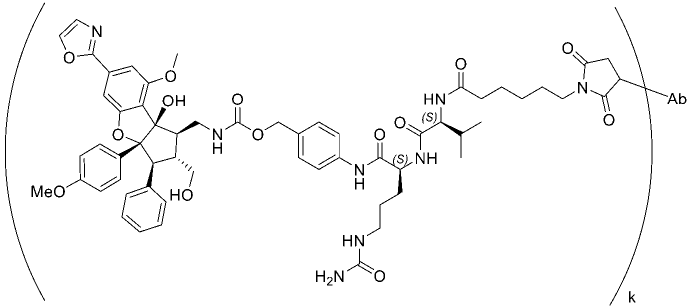 |
| III-C3 | 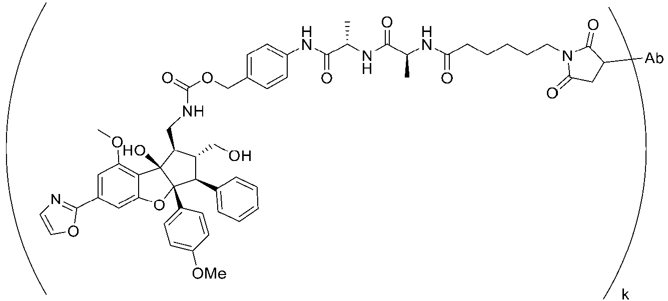 |
| III-C4 | 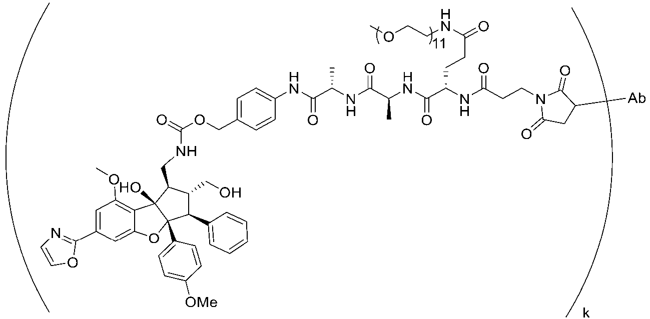 |
| III-C5 | 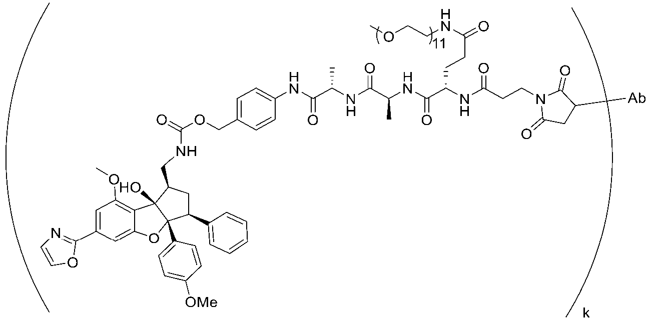 |
| III-C6 | 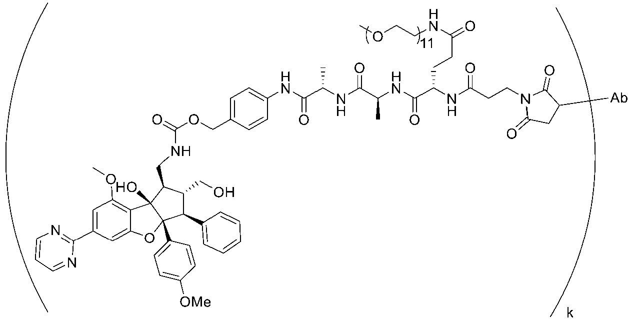 |
| III-C7 | 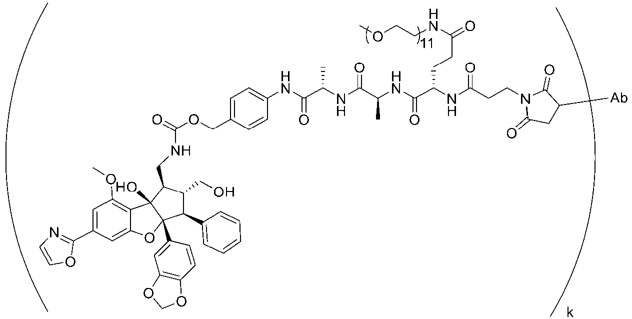 |
| III-C8 | 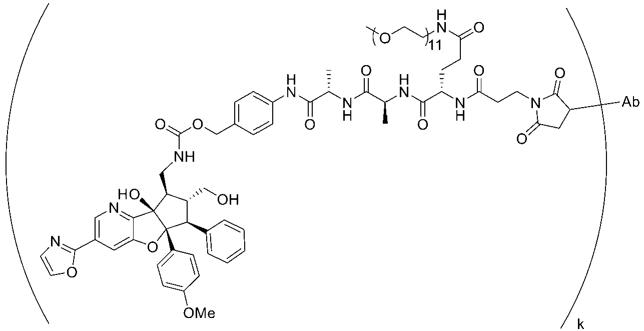 |
| III-C9 | 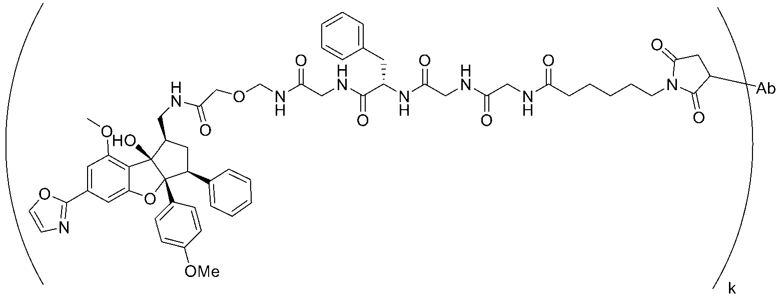 |
| III-C10 | 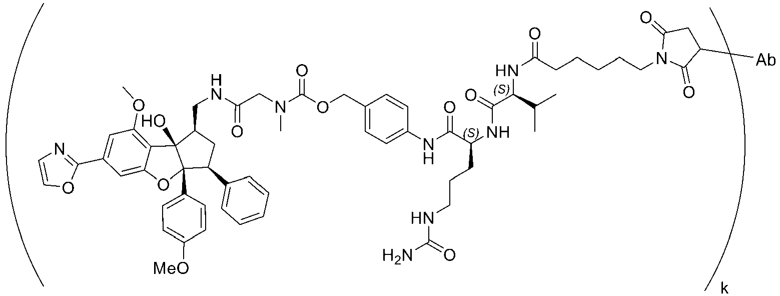 |
| III-C11 | 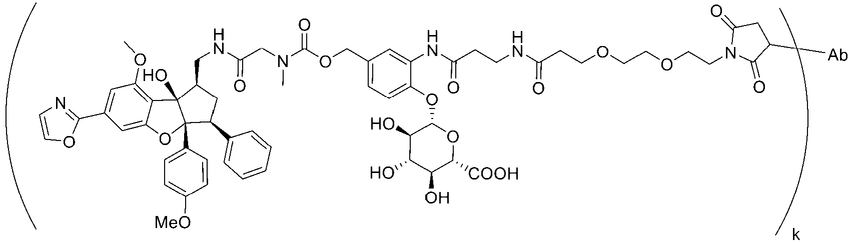 |
| III-C12 | 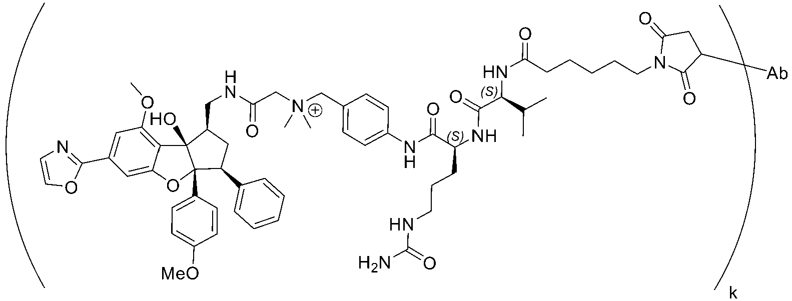 |
| III-C13 | 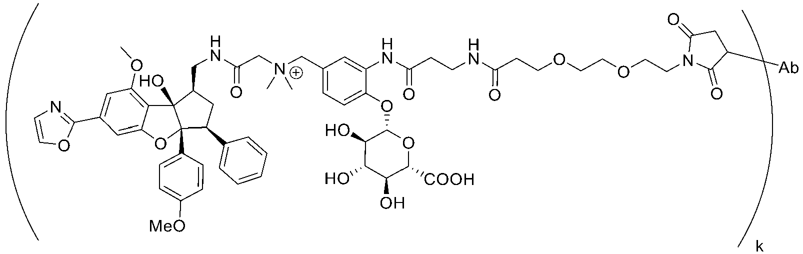 |
| IV-C1 | 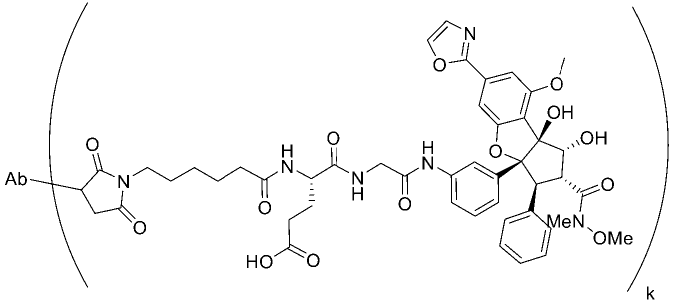 |
| IV-C2 | 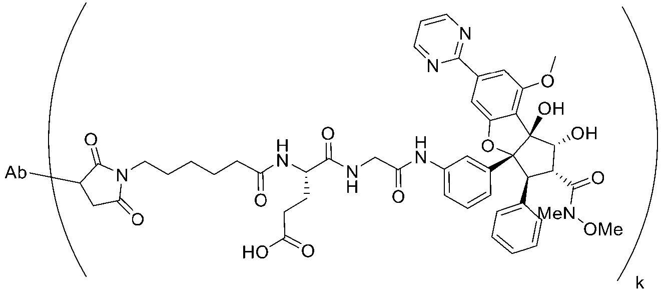 |
| IV-C3 | 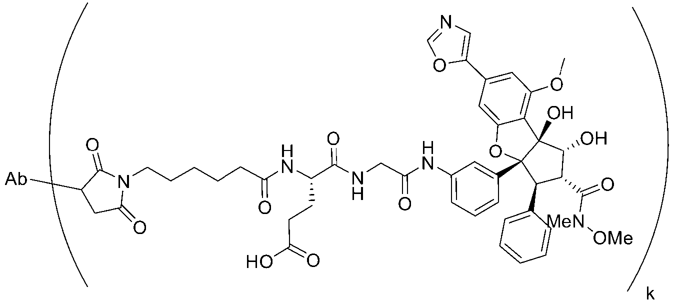 |
| IV-C4 | 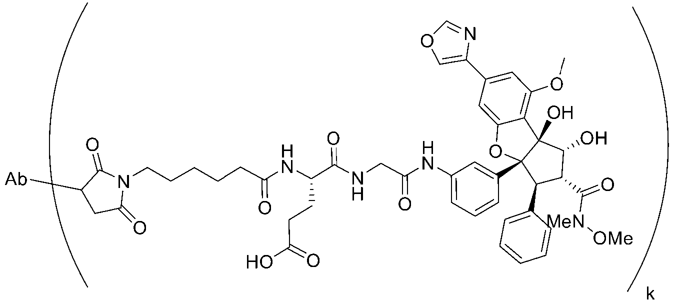 |
| IV-C5 | 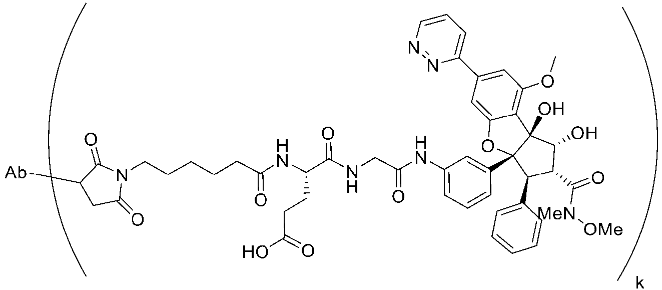 |
| IV-C6 | 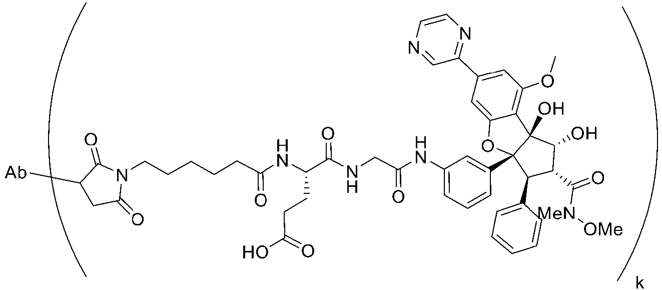 |
| IV-C7 | 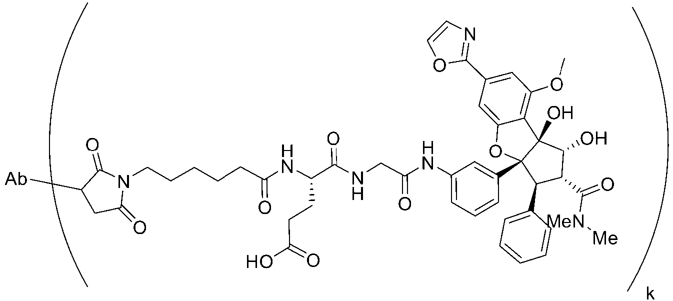 |
| IV-C8 | 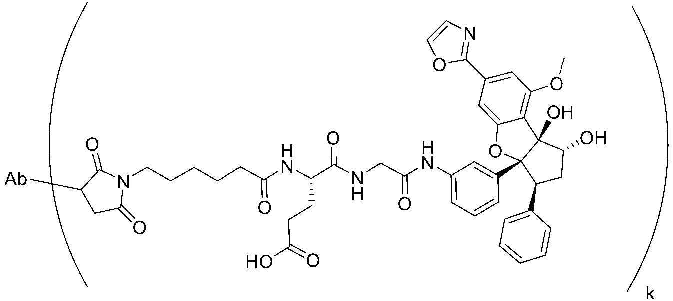 |
| IV-C9 | 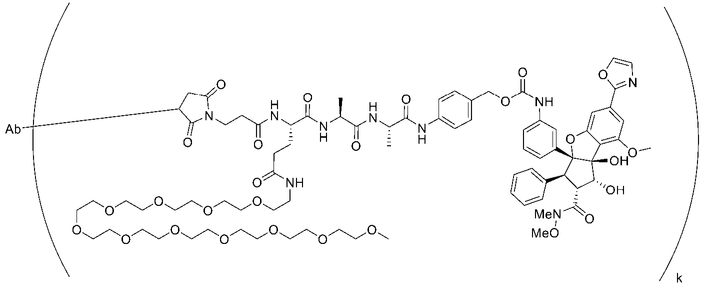 |
| IV-C10 | 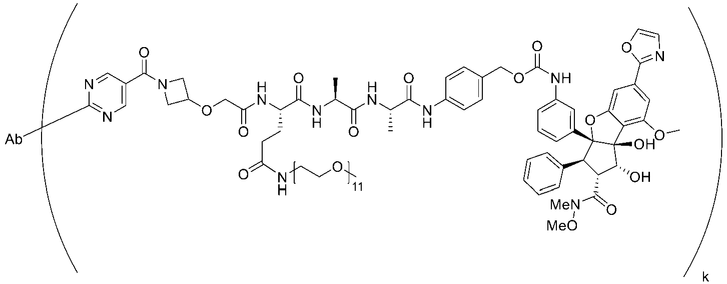 |
| IV-C11 | 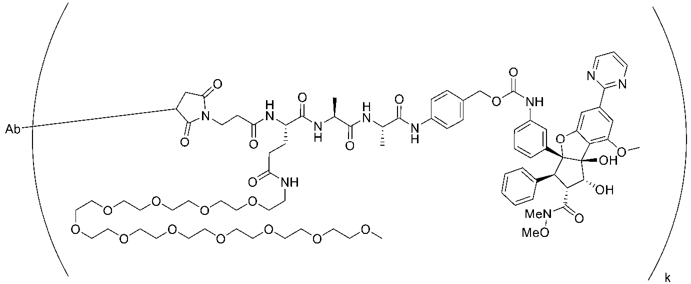 |
| IV-C12 | 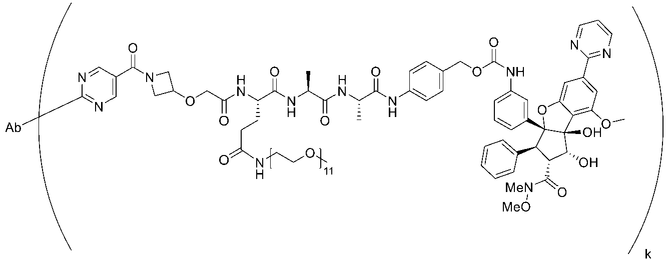 |
| IV-C13 | 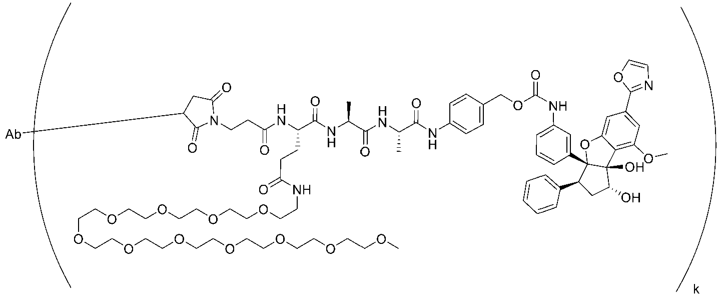 |
| IV-C14 | 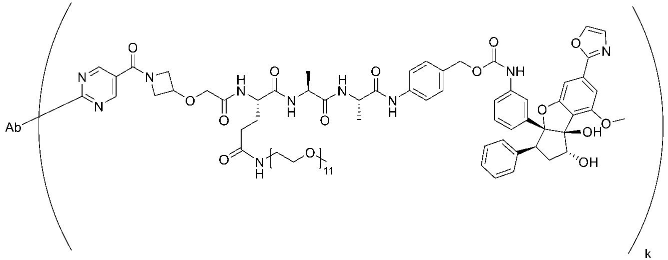 |
| IV-C15 | 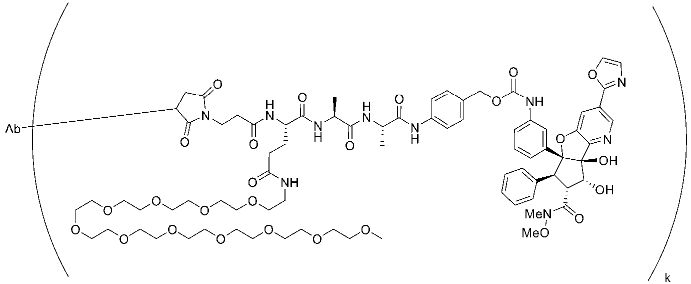 |
| IV-C16 | 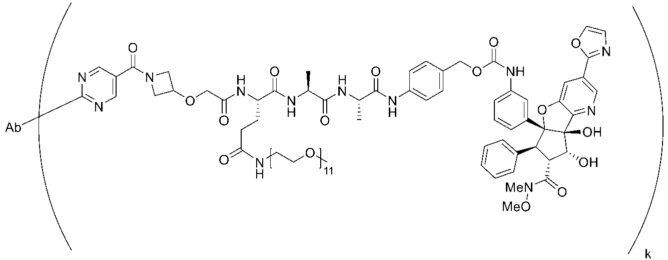 |
| IV-C17 | 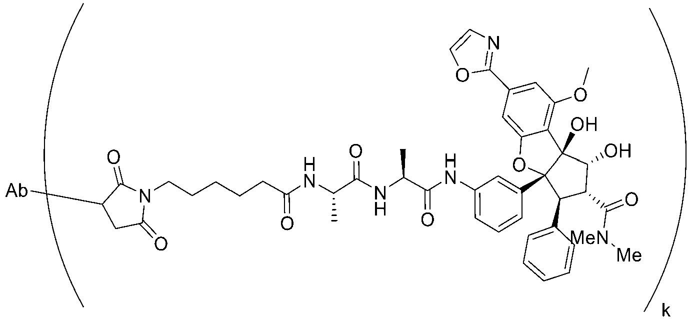 |
| IV-C18 | 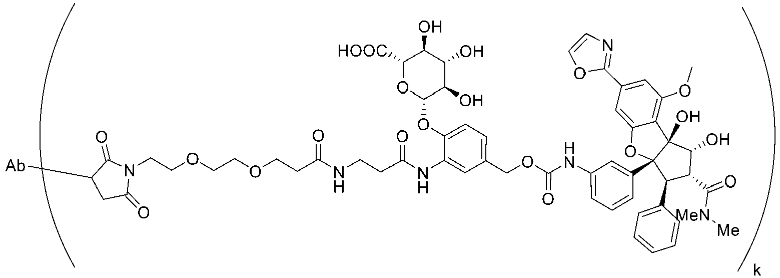 |
| IV-C19 | 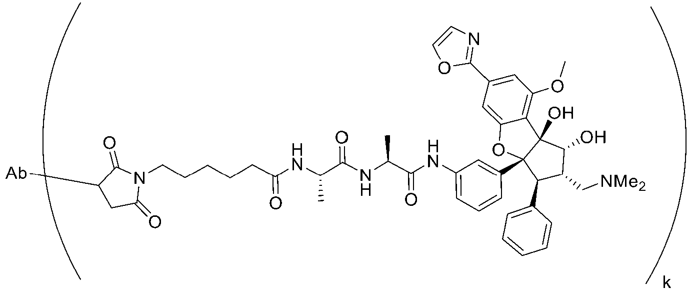 |
| IV-C20 | 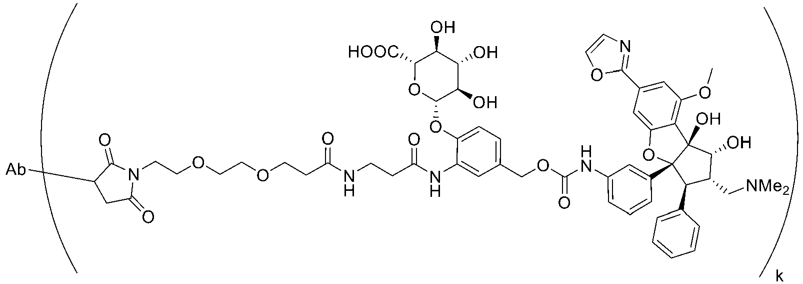 |
| V-C1 | 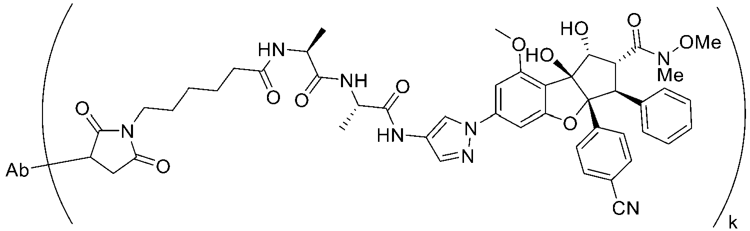 |
| V-C2 | 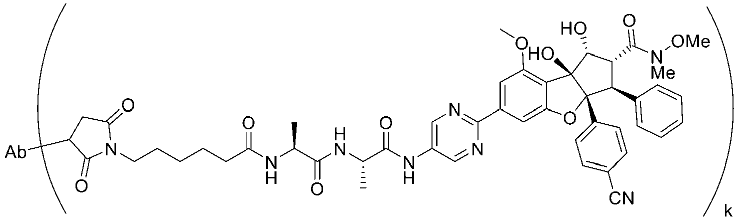 |
| V-C3 | 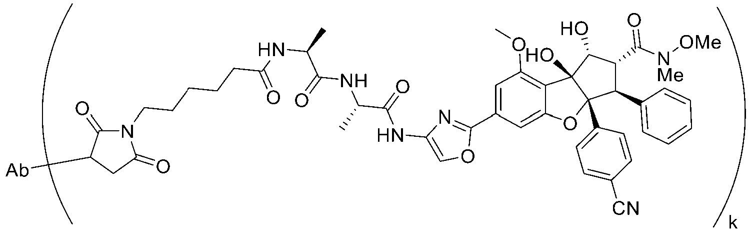 |
| V-C4 | 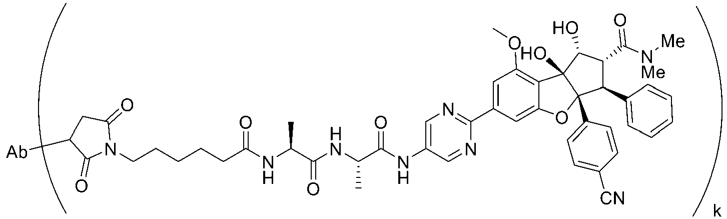 |
| V-C5 | 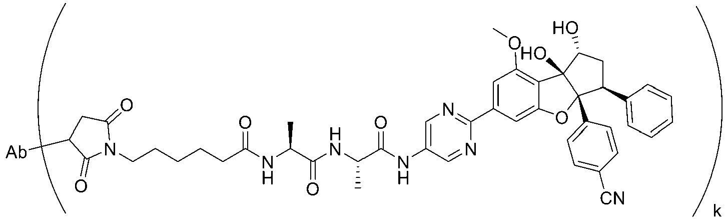 |
| V-C6 | 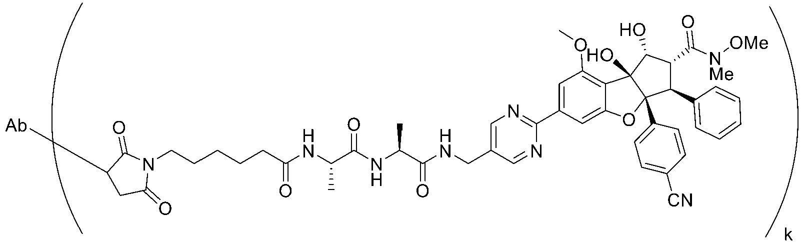 |
| V-C7 | 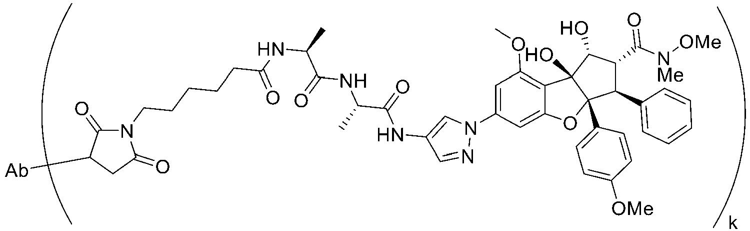 |
| V-C8 | 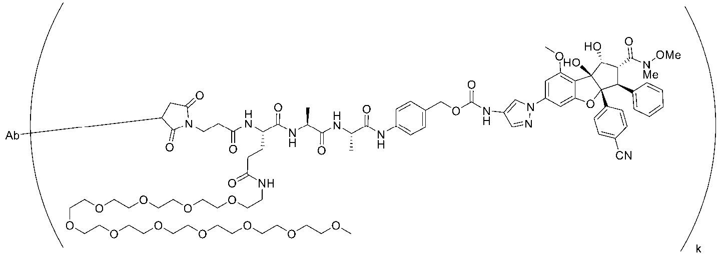 |
| V-C9 | 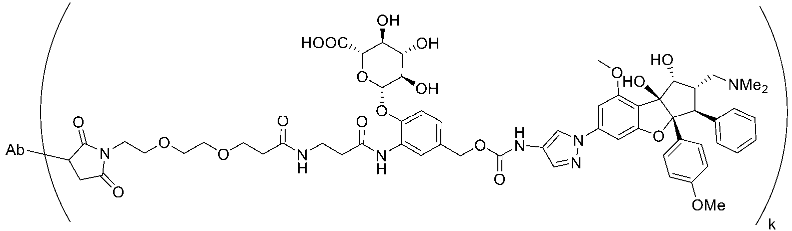 |
| V-C10 | 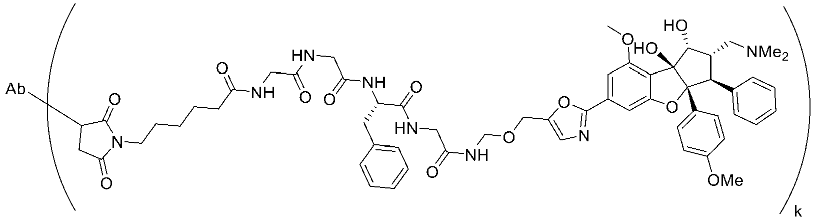 |
| V-C11 | 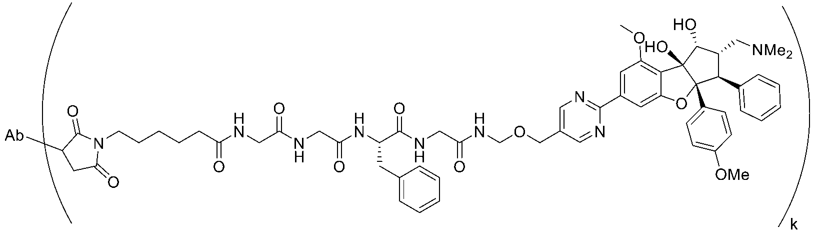 |
| V-C12 | 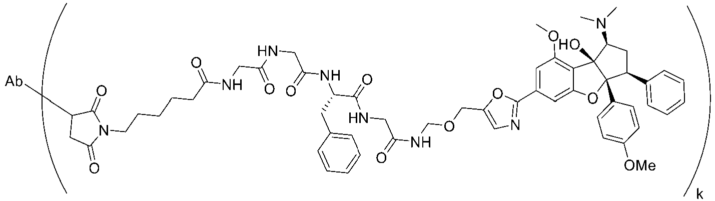 |
| V-C13 | 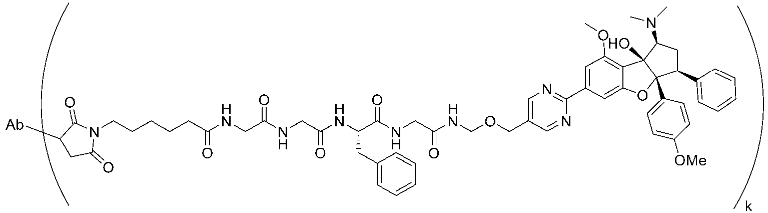 |

wherein Ab and k are as described in any one of the embodiments of the present disclosure.

In certain embodiments, the ligand-drug conjugate is selected from the structures shown in Table E:

**Table E**

| No. | Structure |
|---|---|
| I-E1 | 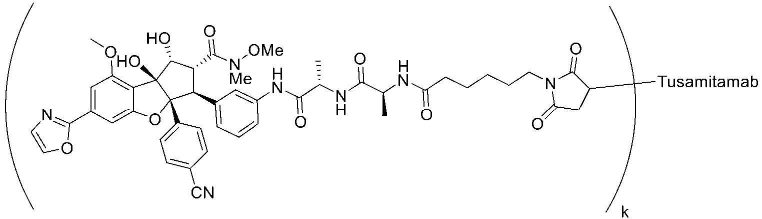 |
| I-E2 | 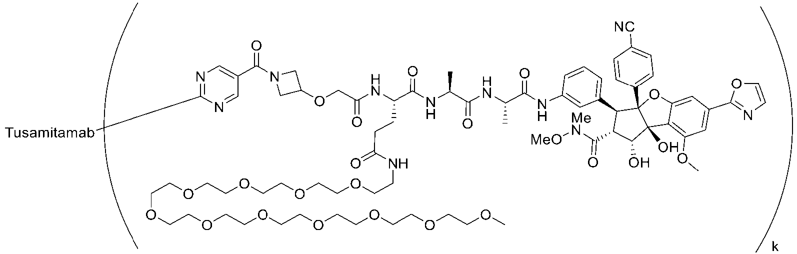 |
| I-E3 | 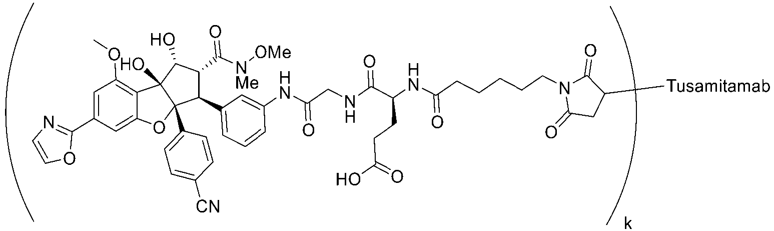 |
| I-E4 | 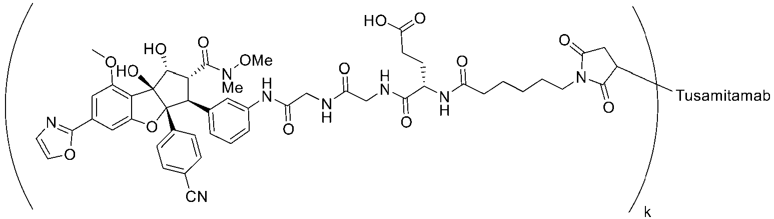 |
| I-E5 | 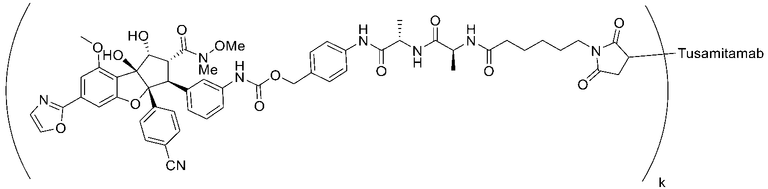 |
| I-E6 | 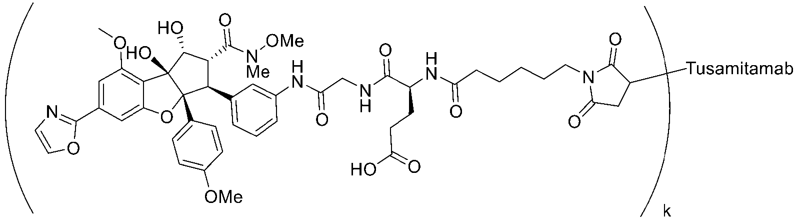 |
| I-E7 | 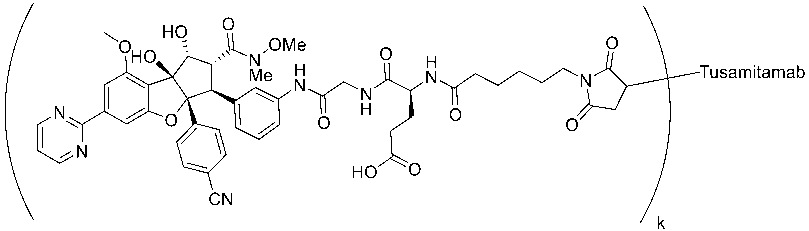 |
| I-E8 | 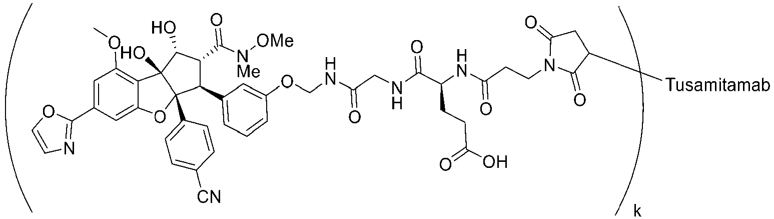 |
| I-E9 | 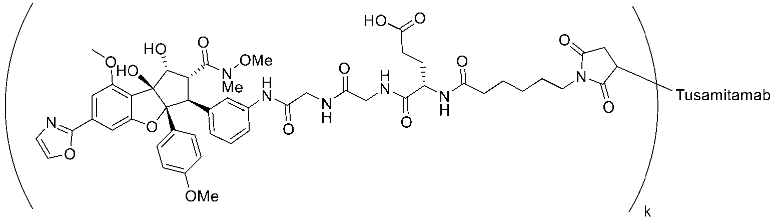 |
| I-E10 | 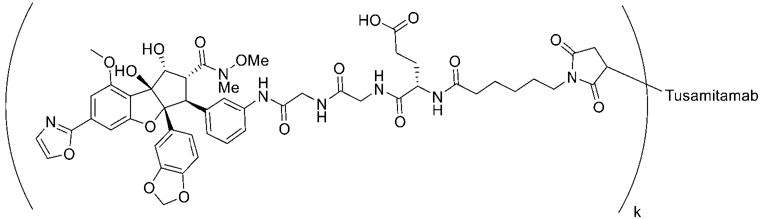 |
| I-E11 | 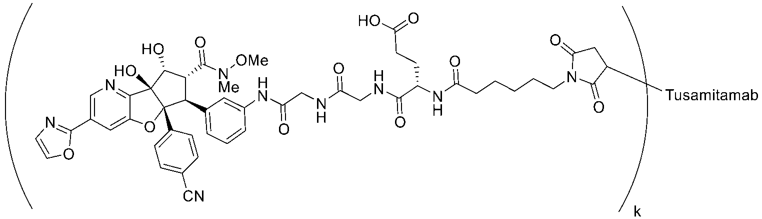 |
| I-E12 | 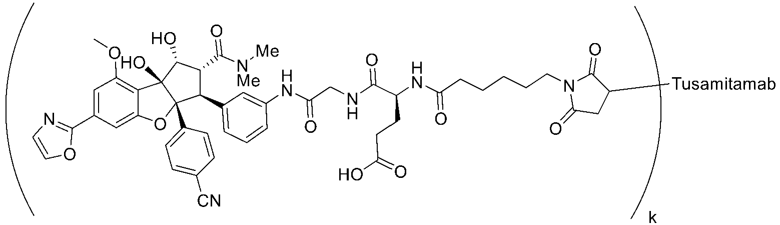 |
| I-E13 | 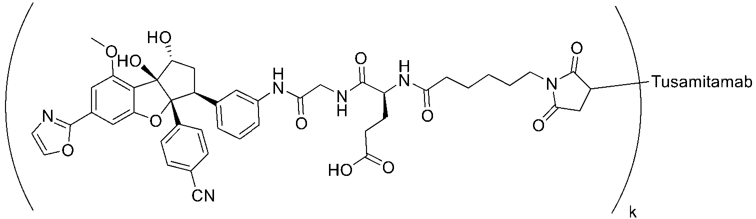 |
| I-E14 | 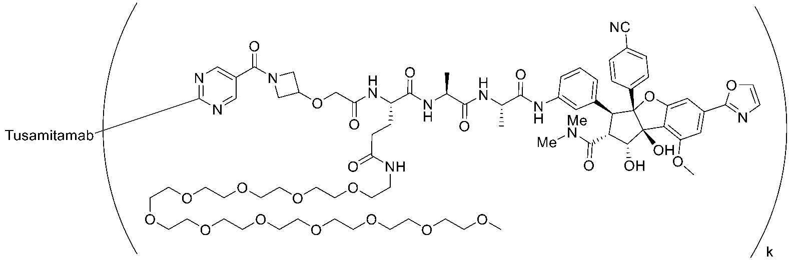 |
| I-E15 | 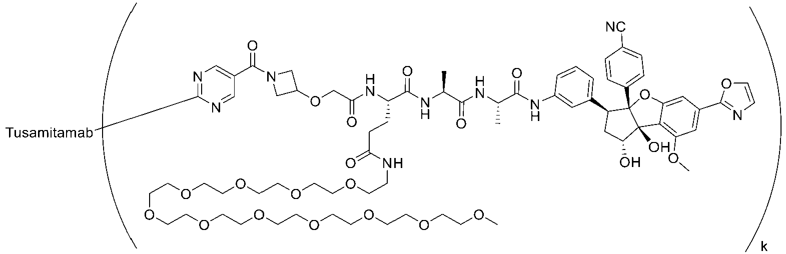 |
| I-E16 | 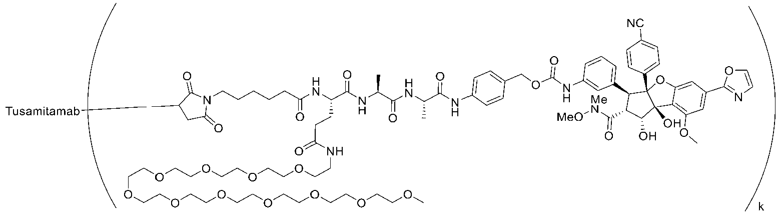 |
| I-E17 | 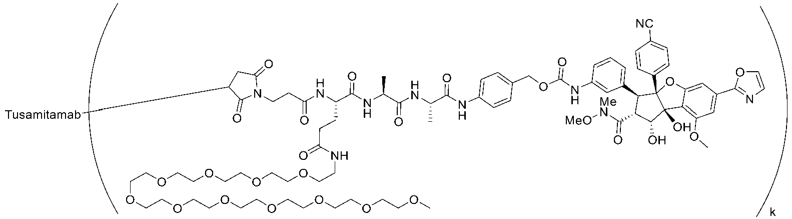 |
| I-E18 | 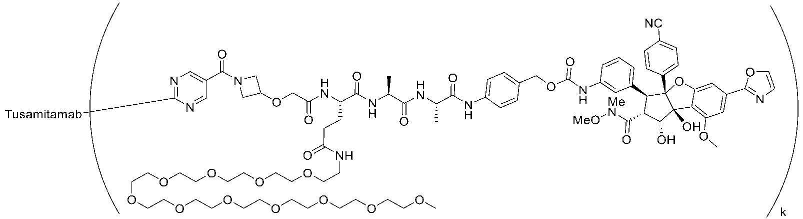 |
| I-E19 | 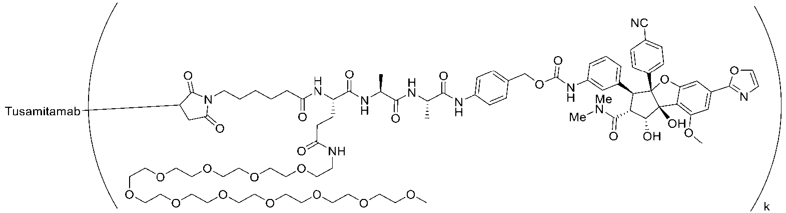 |
| I-E20 | 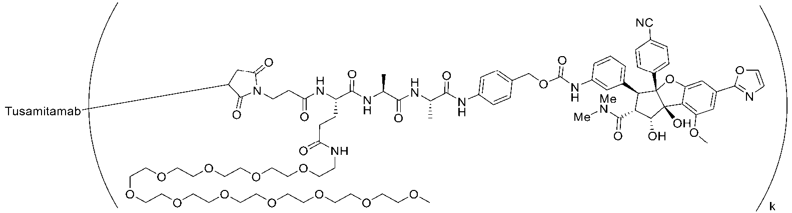 |
| I-E21 | 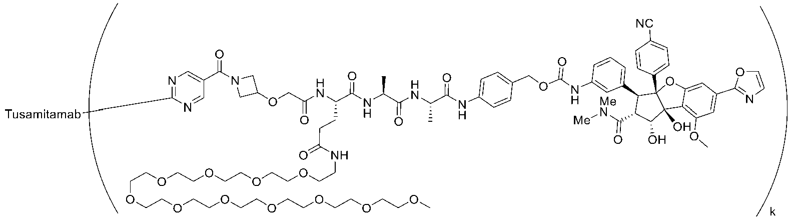 |
| I-E22 | 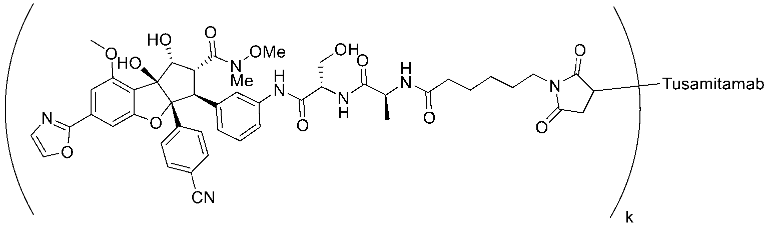 |
| I-E23 | 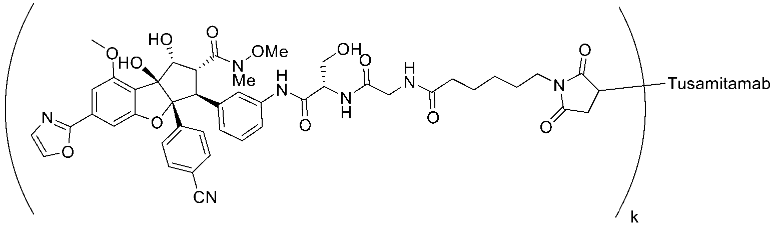 |
| I-E24 | 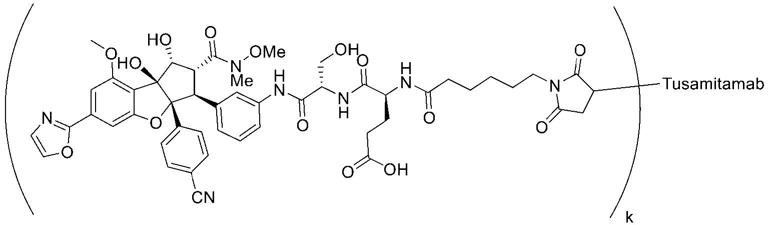 |
| I-E25 | 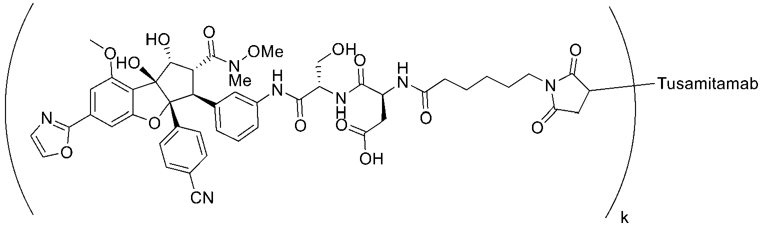 |
| I-E26 | 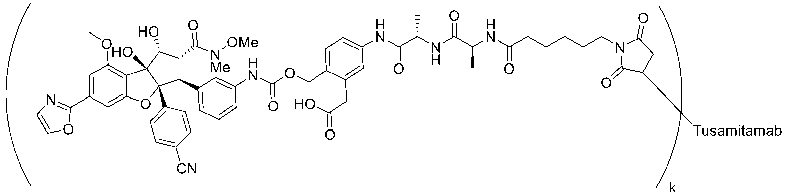 |
| I-E27 | 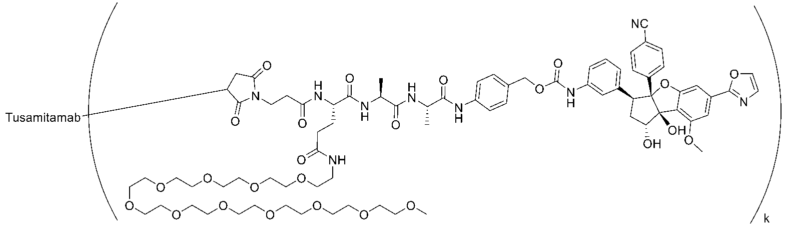 |
| I-E28 | 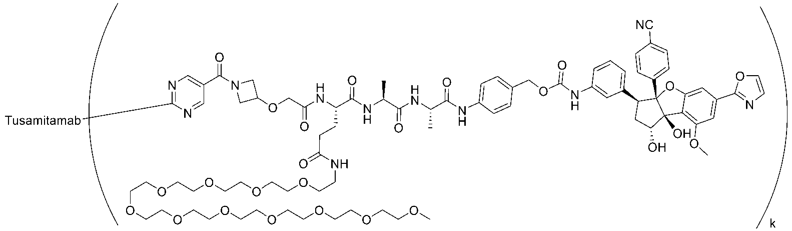 |
| I-E29 | 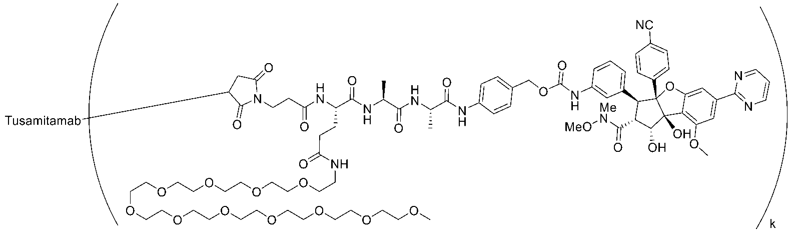 |
| I-E30 | 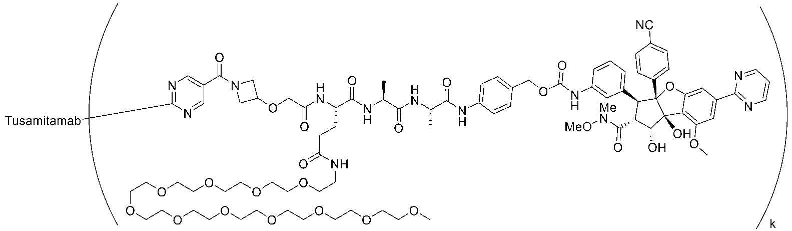 |
| I-E31 | 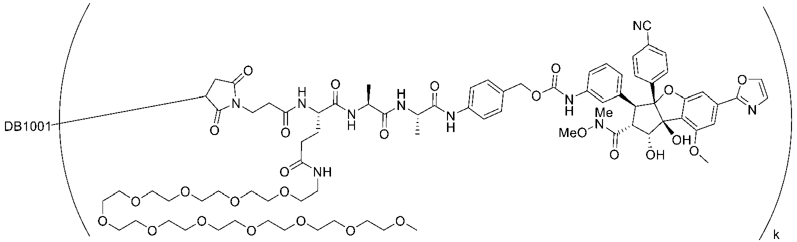 |
| I-E32 | 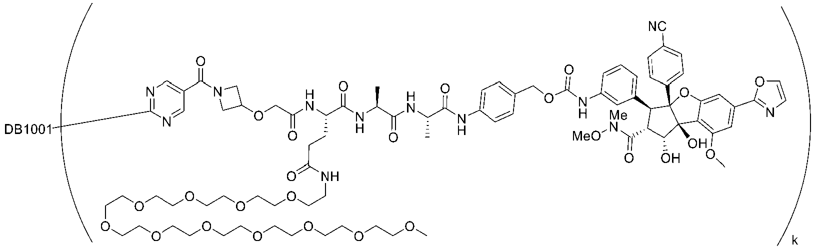 |
| I-E33 | 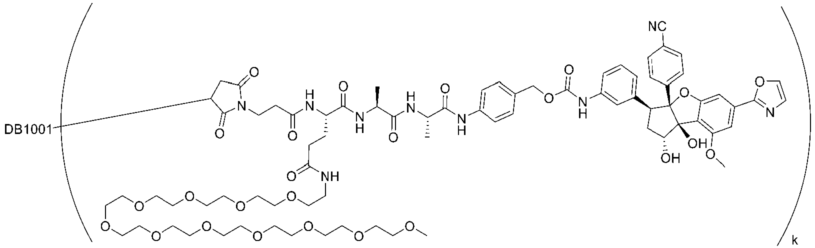 |
| I-E34 | 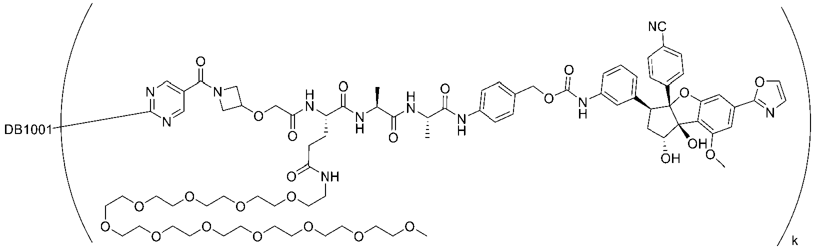 |
| I-E35 | 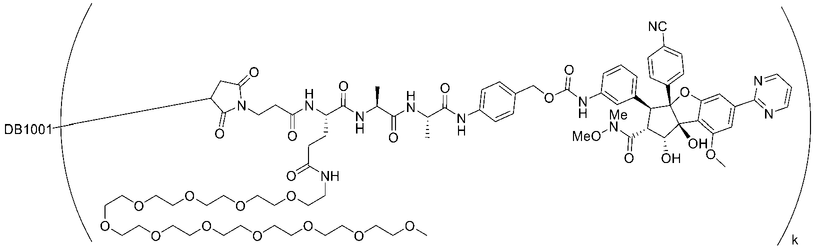 |
| I-E36 | 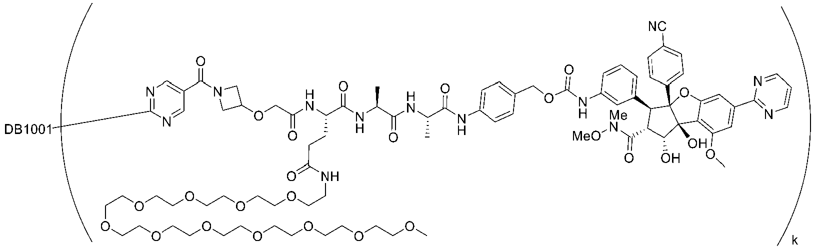 |
| I-E37 | 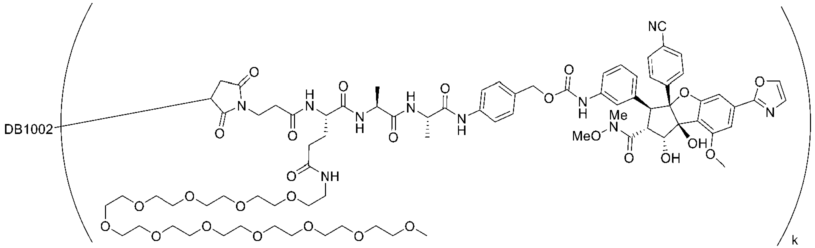 |
| I-E38 | 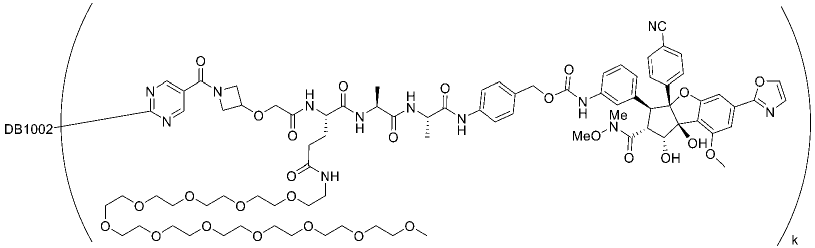 |
| I-E39 | 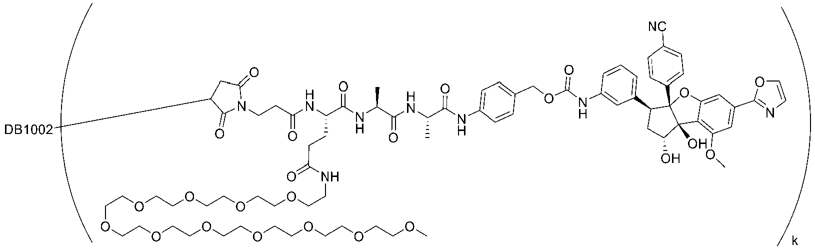 |
| I-E40 | 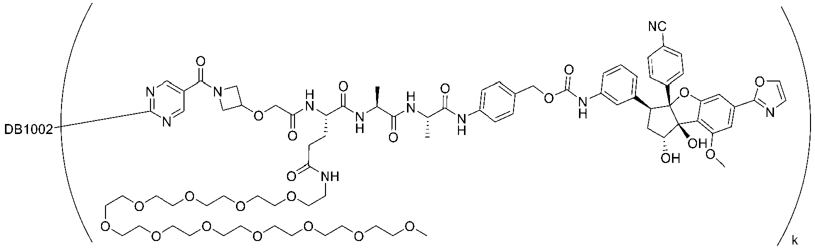 |
| I-E41 | 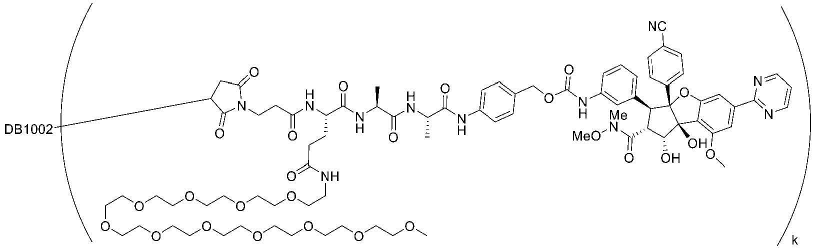 |
| I-E42 | 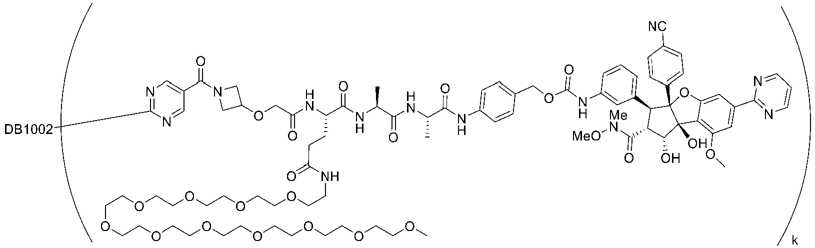 |
| I-E43 | 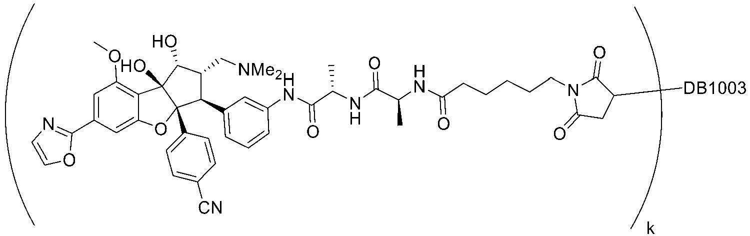 |
| I-E44 | 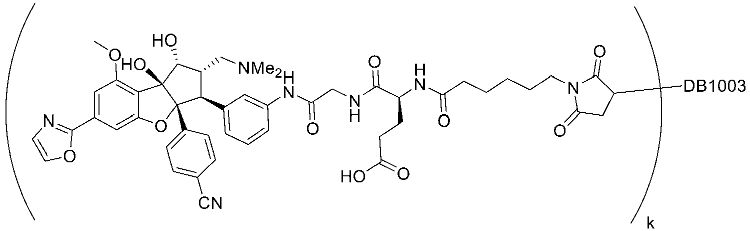 |
| I-E45 | 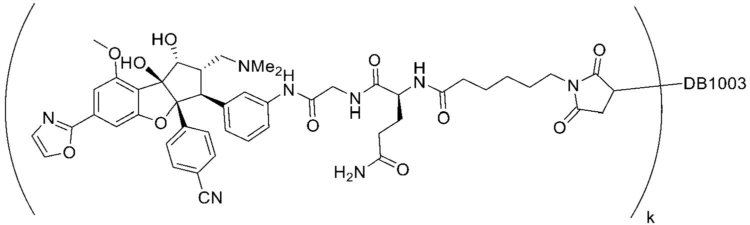 |
| I-E46 | 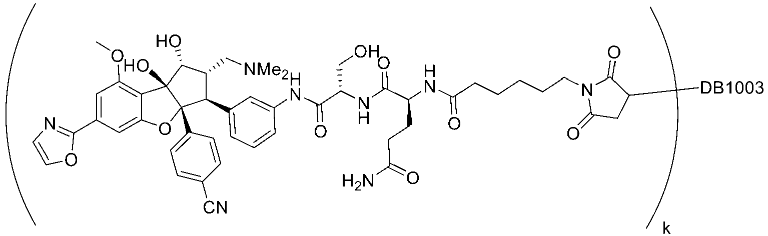 |
| I-E47 | 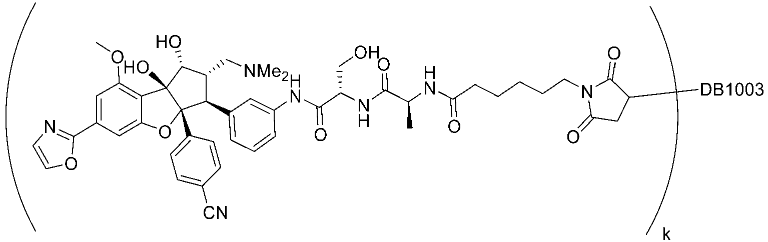 |
| I-E48 | 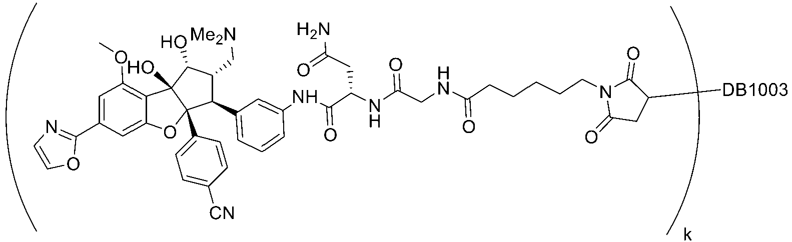 |
| I-E49 | 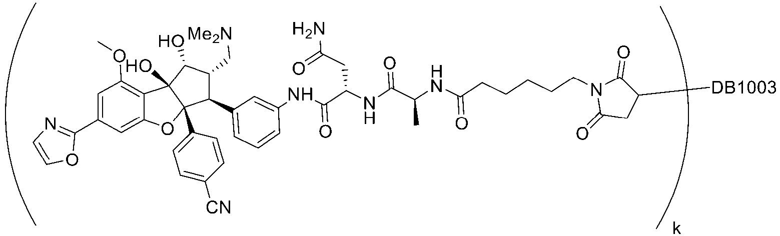 |
| I-E50 | 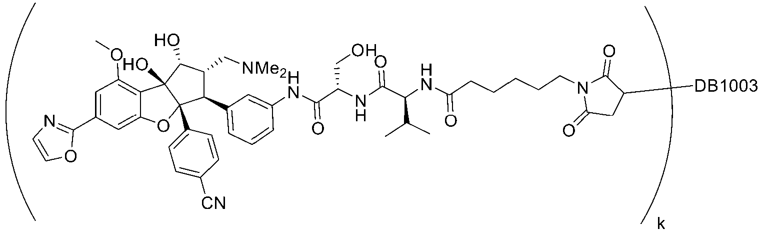 |
| I-E51 | 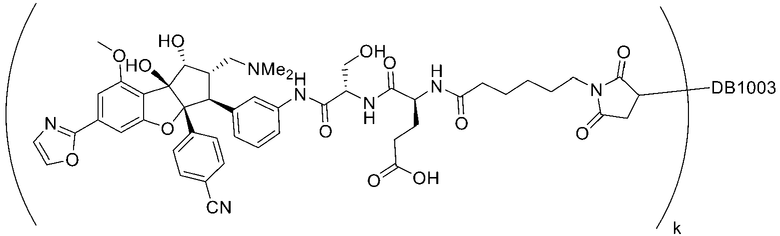 |
| I-E52 | 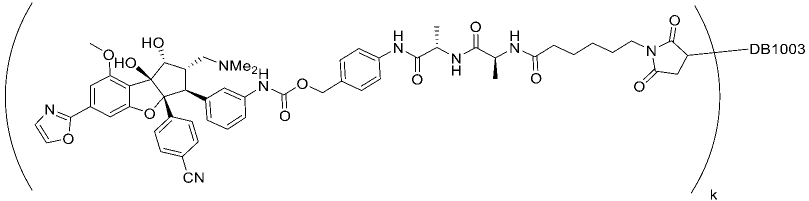 |
| I-E53 | 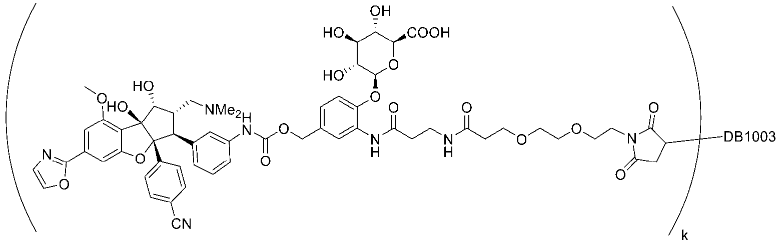 |
| I-E54 | 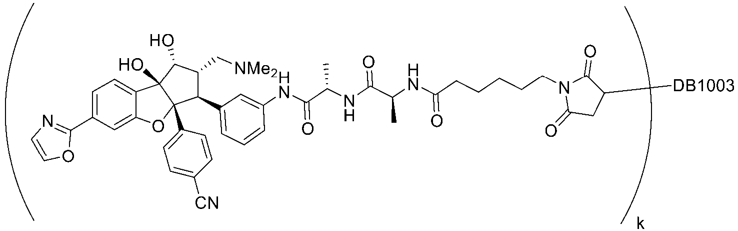 |
| I-E55 | 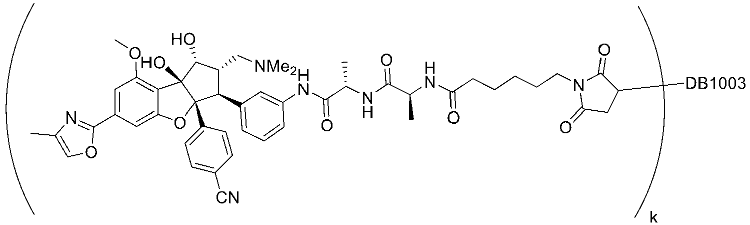 |
| I-E56 | 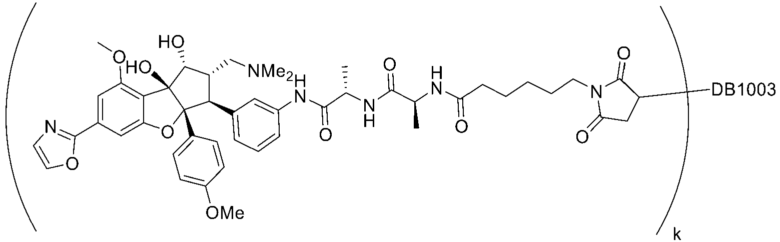 |
| I-E57 | 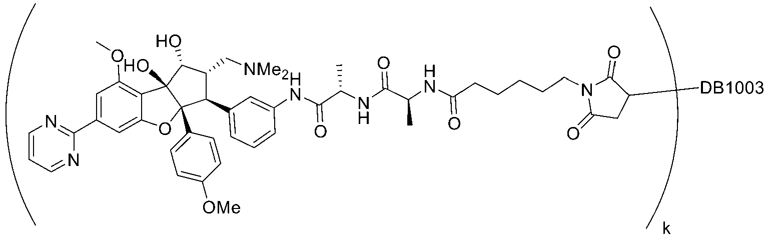 |
| I-E58 | 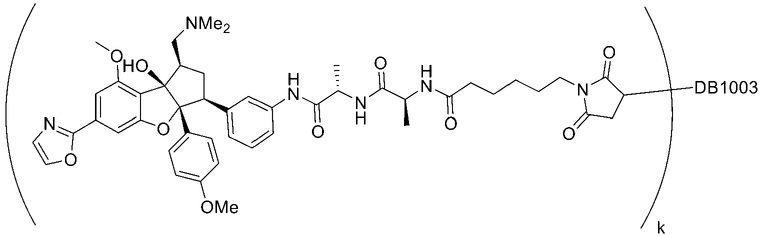 |
| I-E59 | 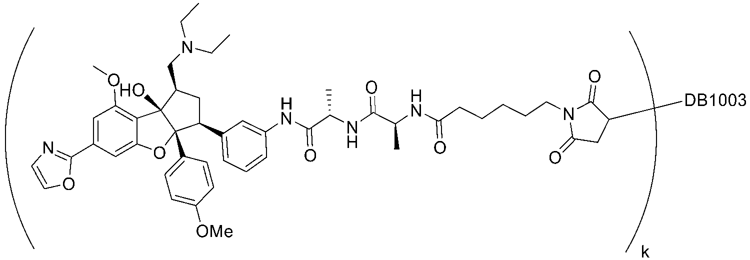 |
| I-E60 | 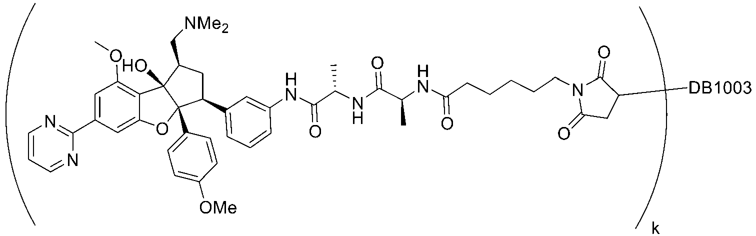 |
| I-E61 | 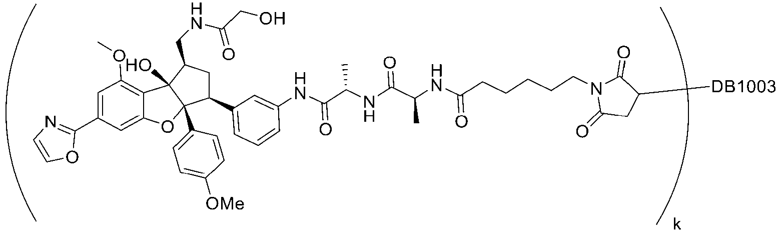 |
| I-E62 | 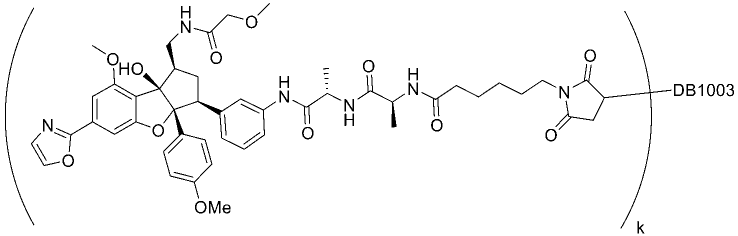 |
| I-E63 | 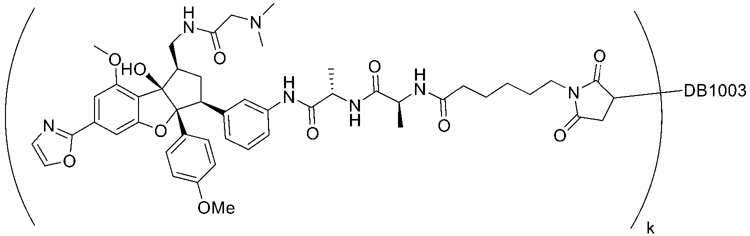 |
| I-E64 | 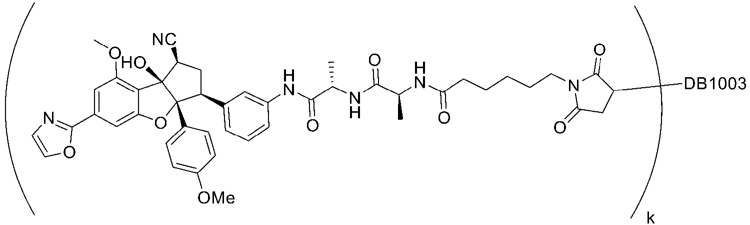 |
| I-E65 | 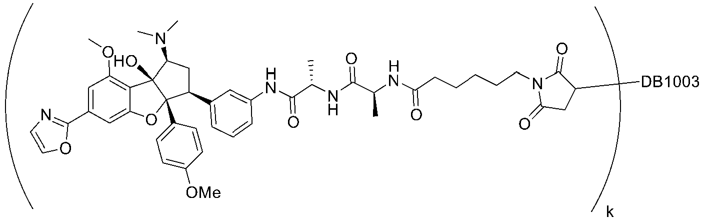 |
| I-E66 | 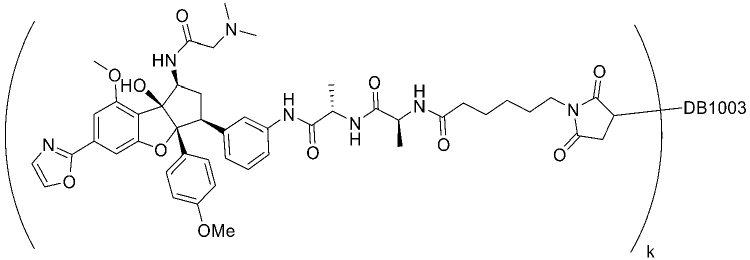 |
| I-E67 | 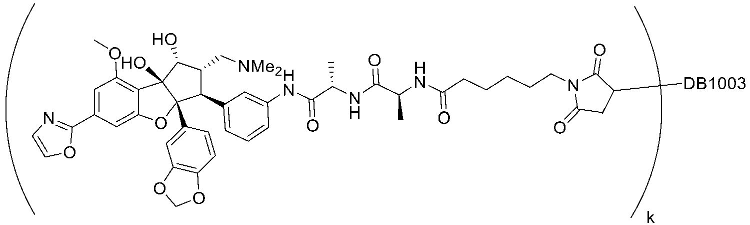 |
| I-E68 | 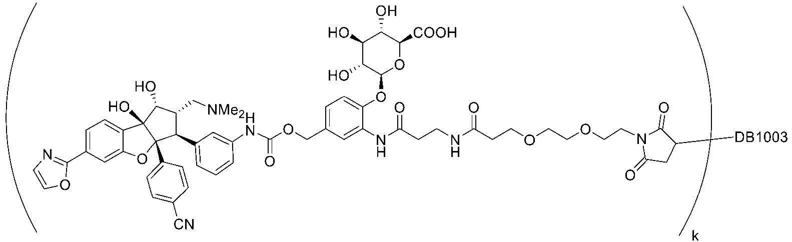 |
| I-E69 | 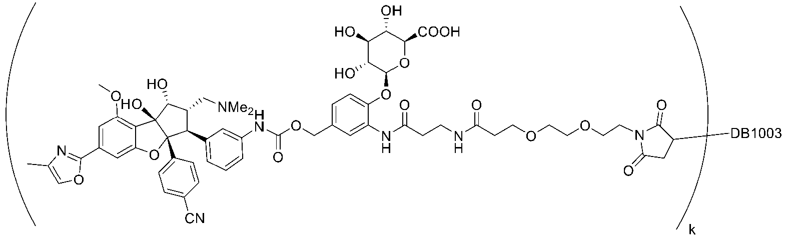 |
| I-E70 | 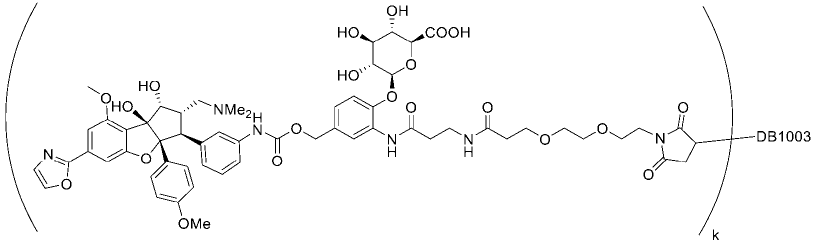 |
| I-E71 | 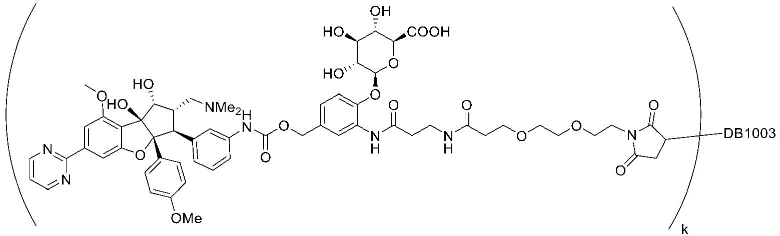 |
| I-E72 | 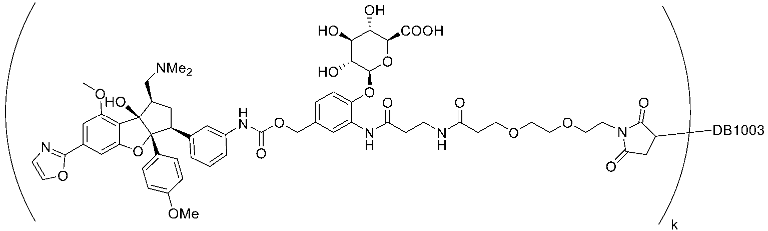 |
| I-E73 | 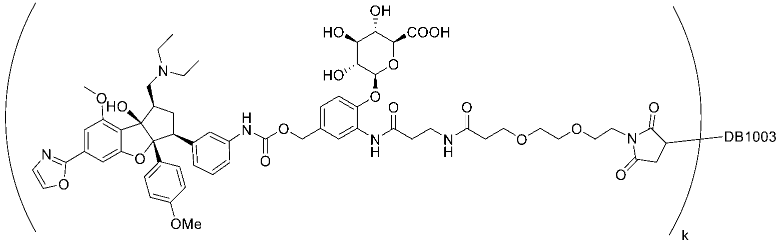 |
| I-E74 | 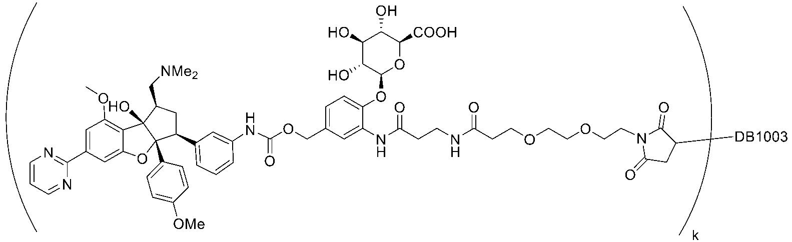 |
| I-E75 | 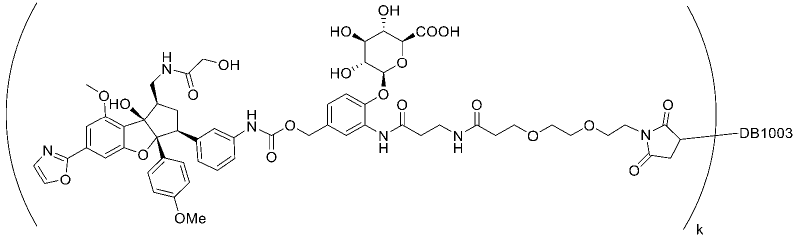 |
| I-E76 | 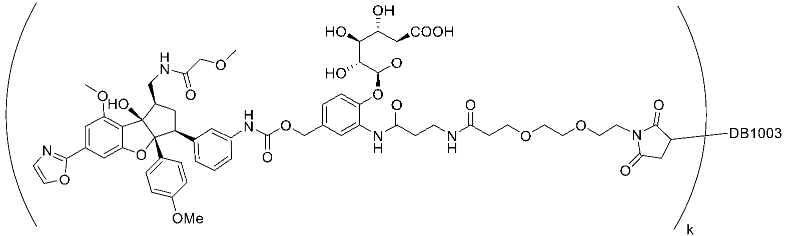 |
| I-E77 | 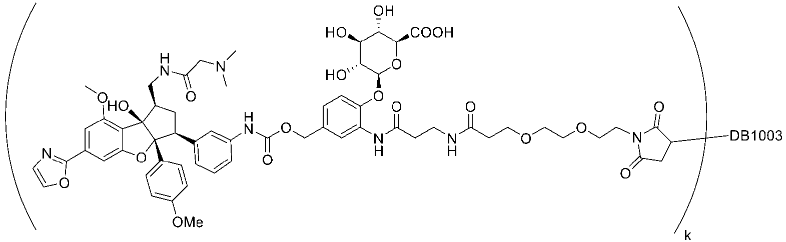 |
| I-E78 | 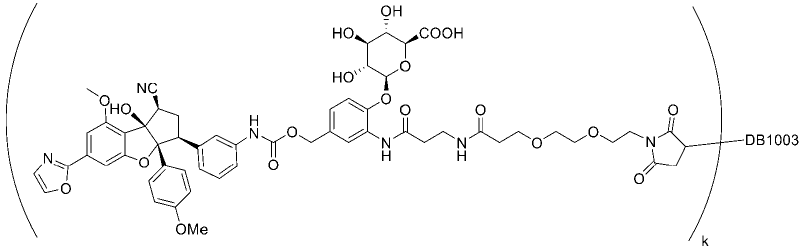 |
| I-E79 | 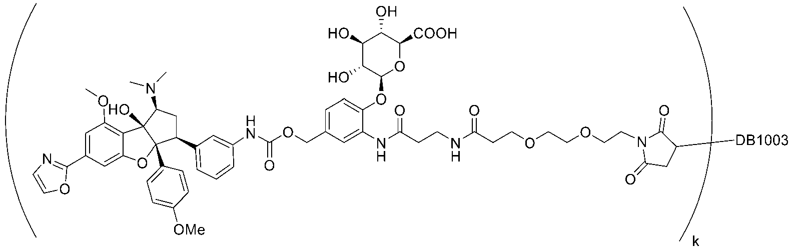 |
| I-E80 | 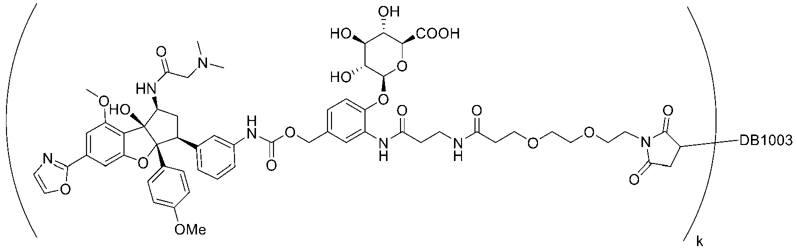 |
| I-E81 | 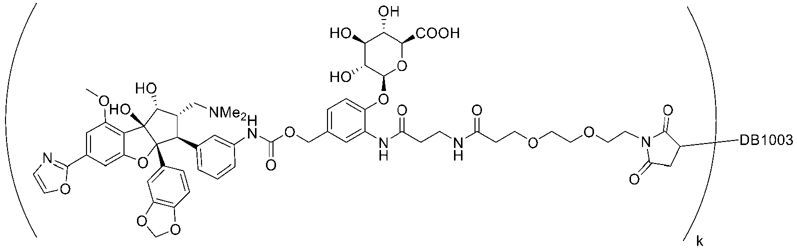 |
| I-E82 | 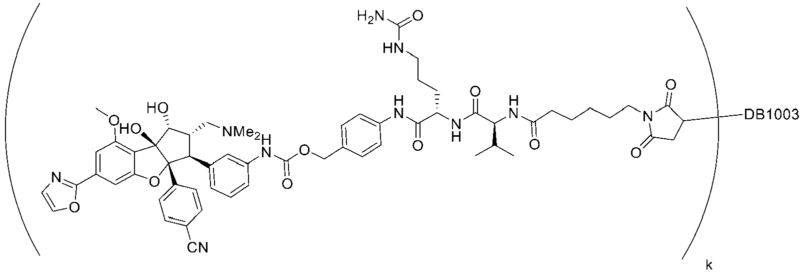 |
| I-E83 | 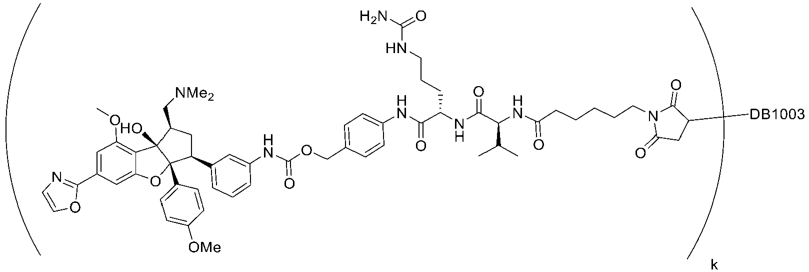 |
| I-E84 | 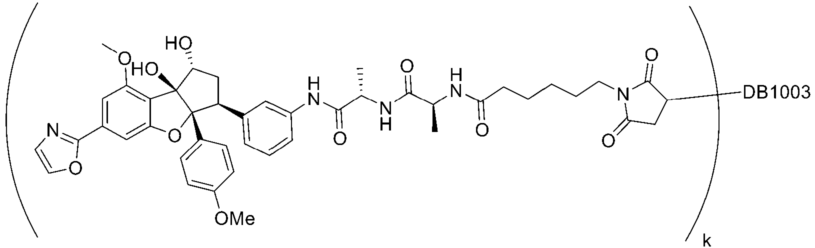 |
| I-E85 | 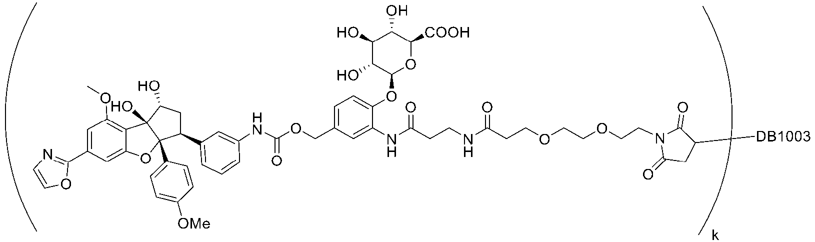 |
| I-E86 | 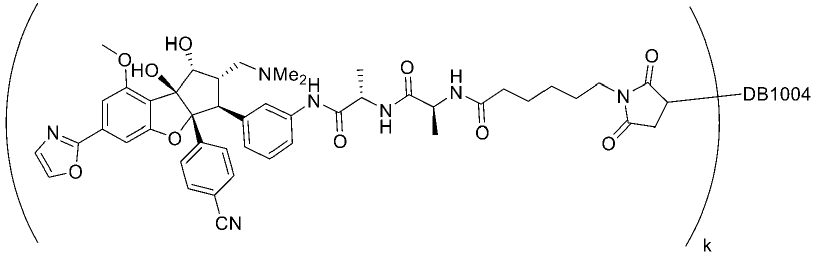 |
| I-E87 | 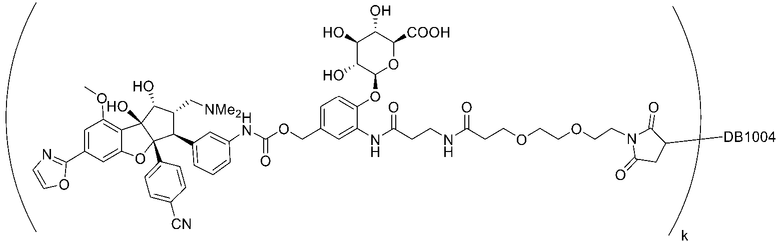 |
| I-E88 | 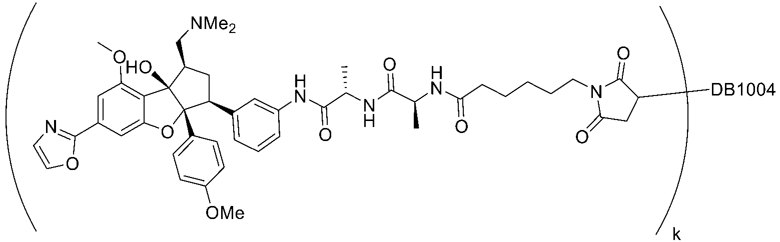 |
| I-E89 | 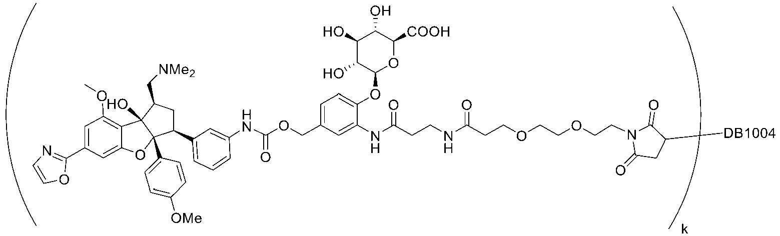 |
| I-E90 | 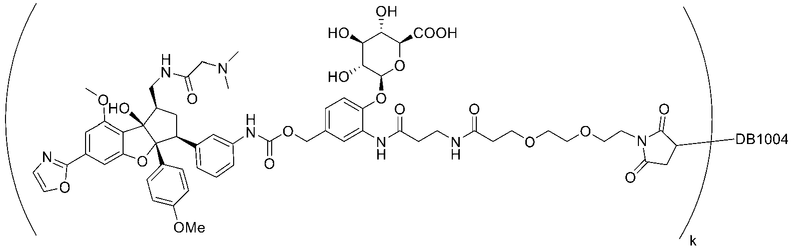 |
| I-E91 | 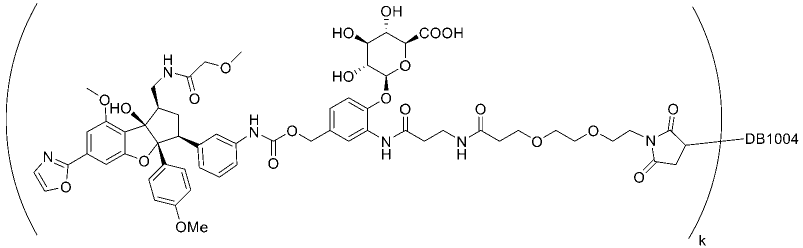 |
| I-E92 | 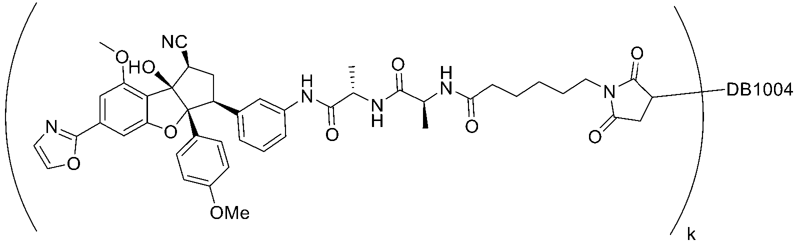 |
| I-E93 | 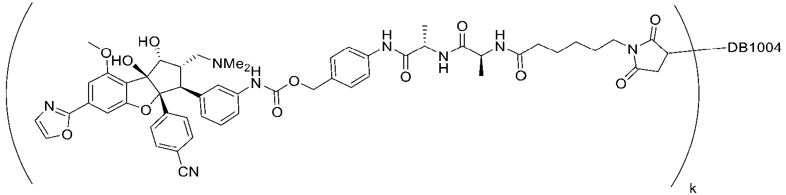 |
| I-E94 | 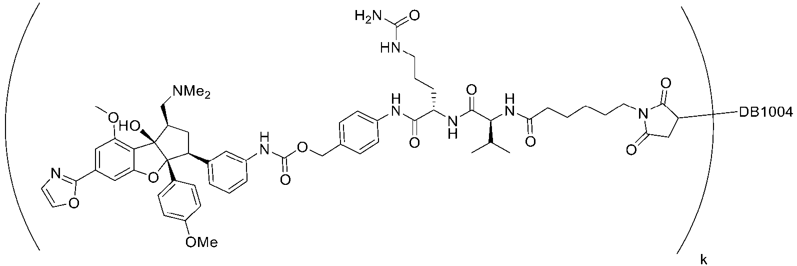 |
| I-E95 | 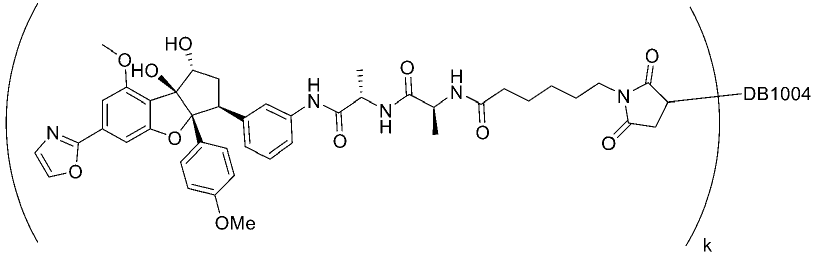 |
| I-E96 | 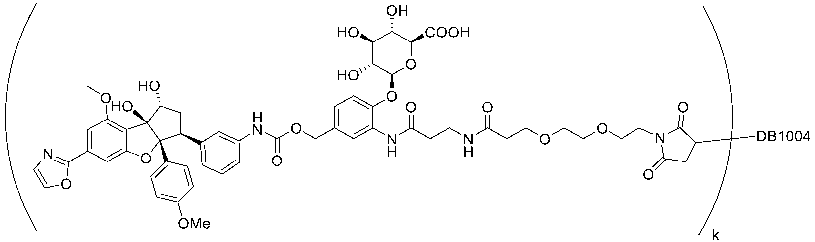 |
| I-E97 | 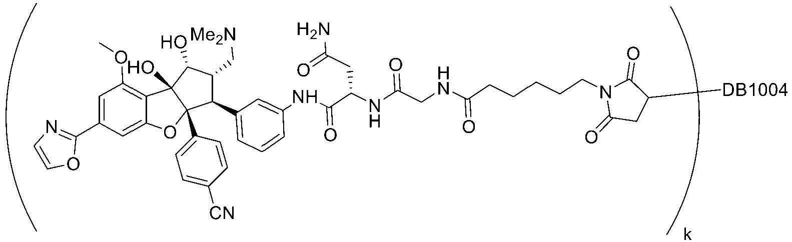 |
| I-E98 | 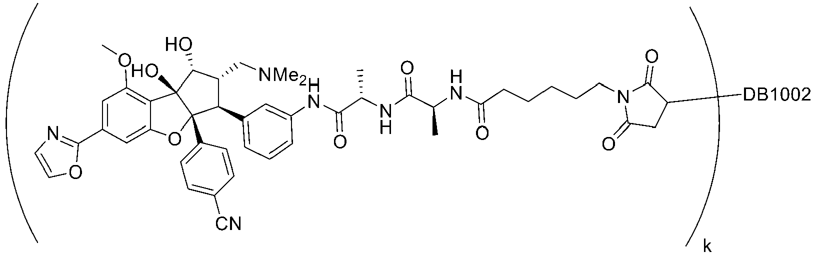 |
| I-E99 | 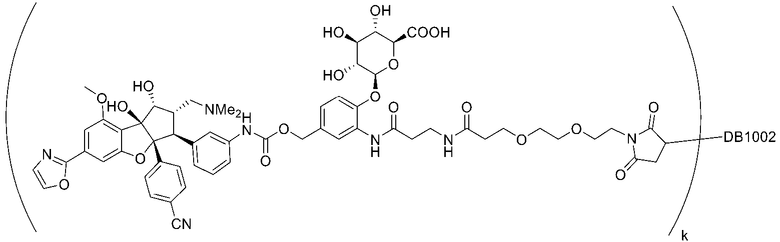 |
| I-E100 | 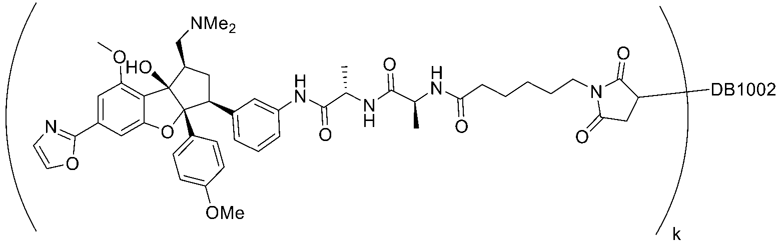 |
| I-E101 | 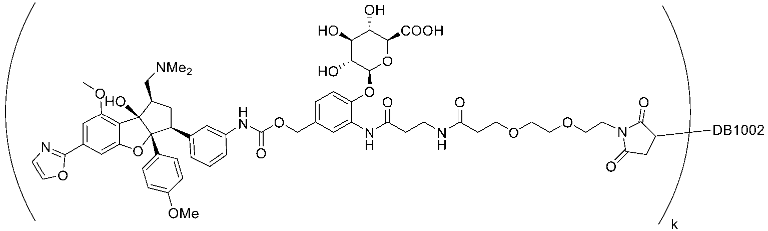 |
| I-E102 | 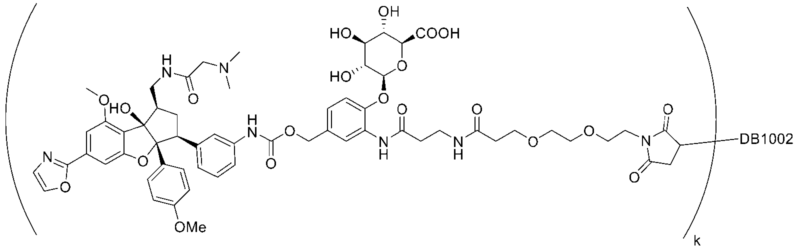 |
| I-E103 | 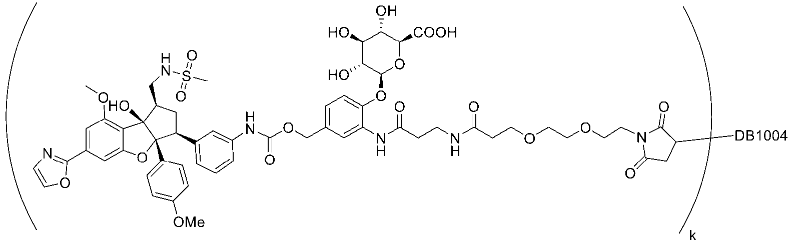 |
| I-E 104 | 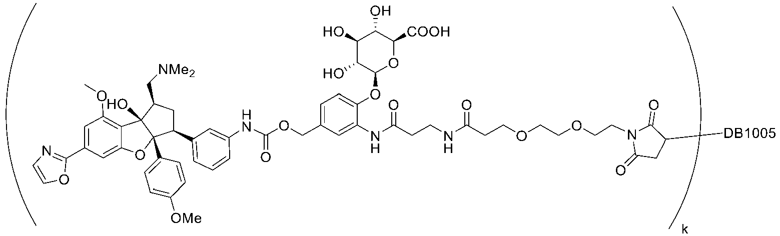 |
| I-E105 | 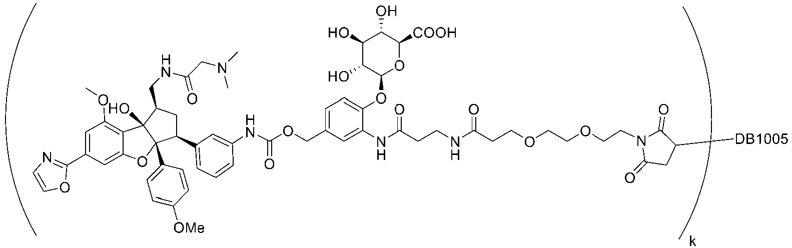 |
| I-E106 | 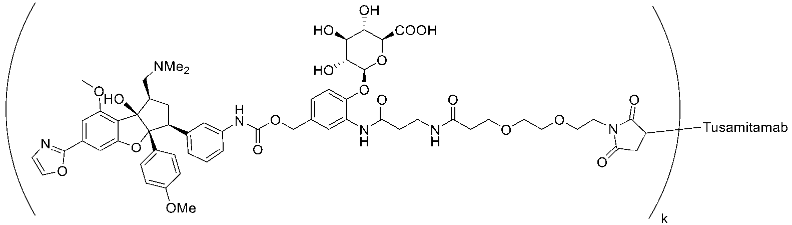 |
| I-E107 | 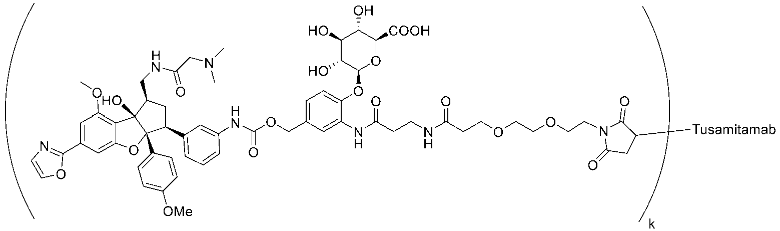 |
| I-E108 | 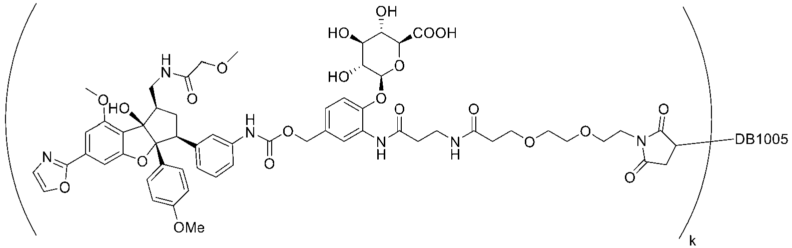 |
| I-E109 | 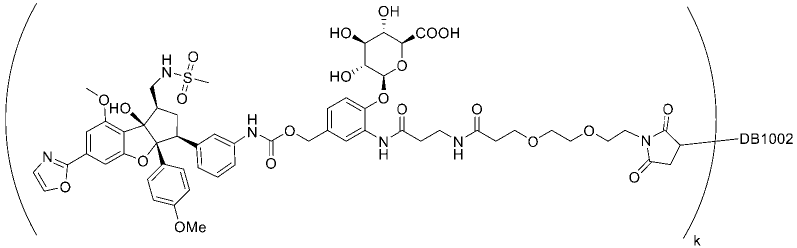 |
| II-E1 | 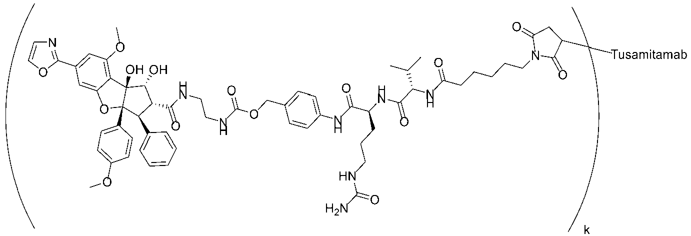 |
| II-E2 | 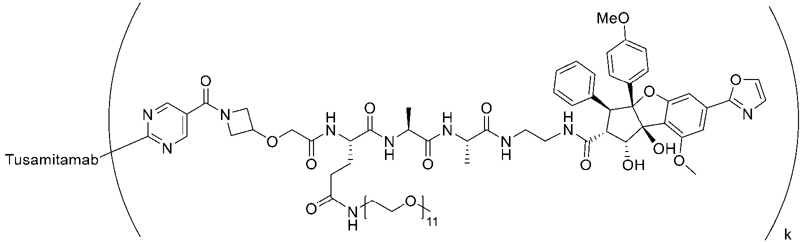 |
| II-E3 | 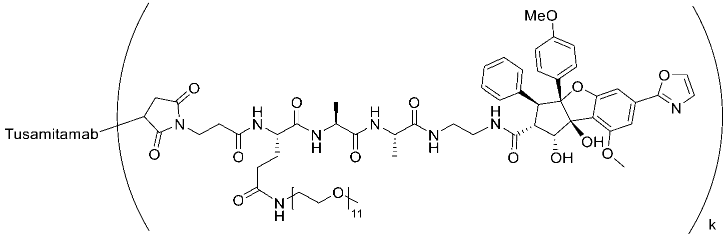 |
| II-E4 | 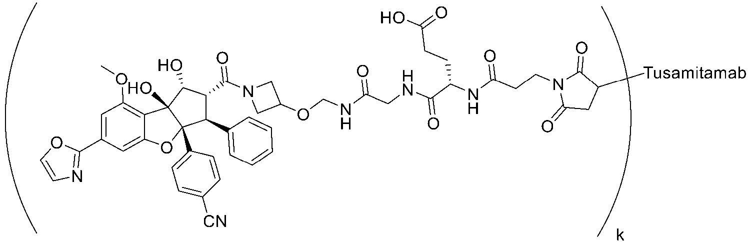 |
| II-E5 | 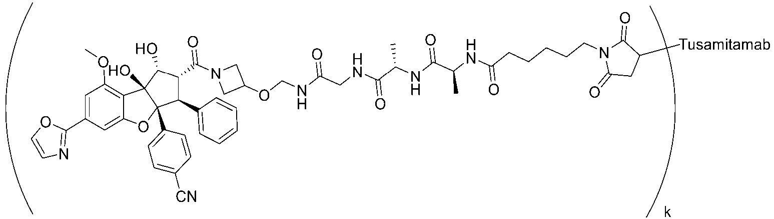 |
| II-E6 | 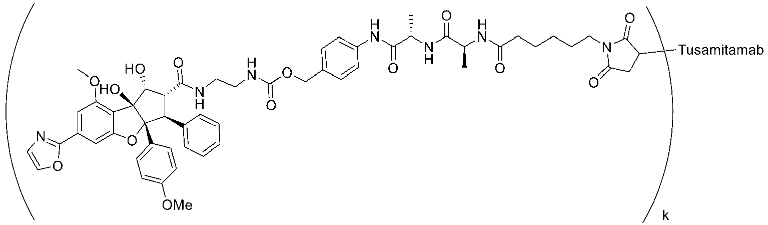 |
| II-E7 | 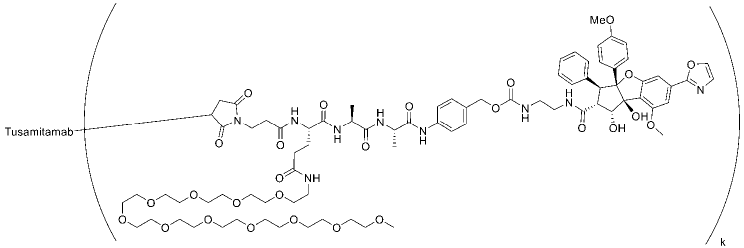 |
| II-E8 | 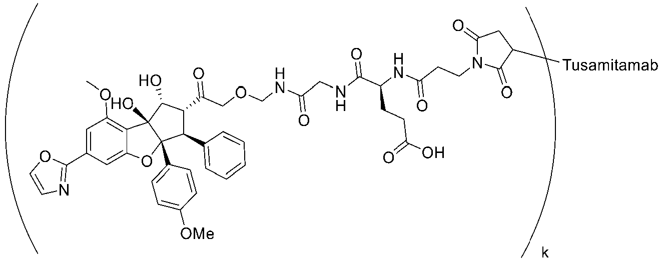 |
| II-E9 | 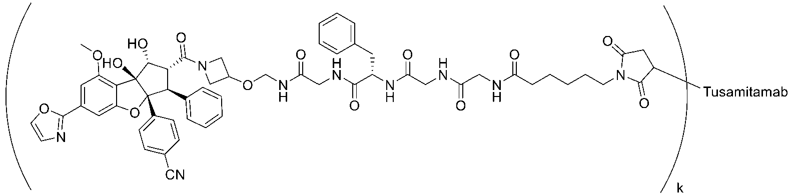 |
| II-E10 | 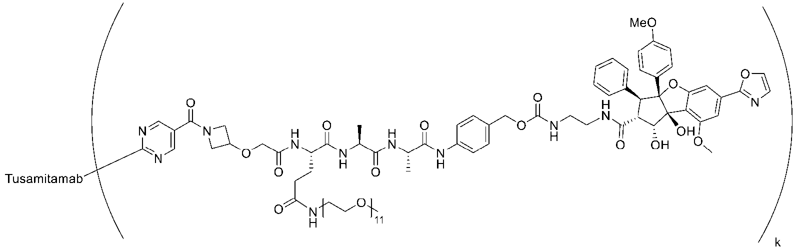 |
| II-E11 | 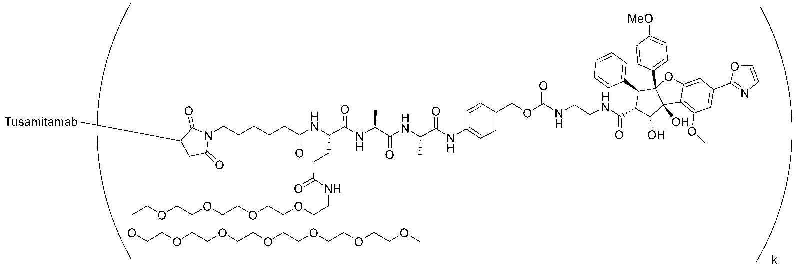 |
| II-E12 | 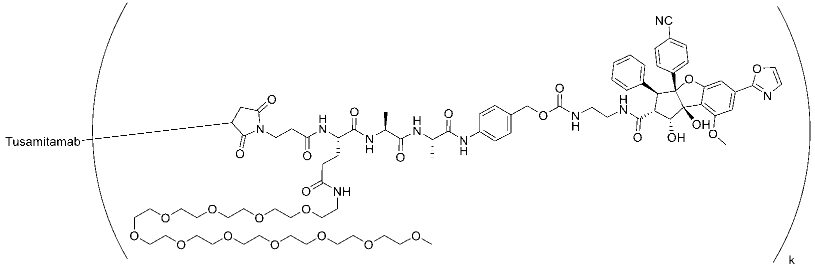 |
| II-E13 | 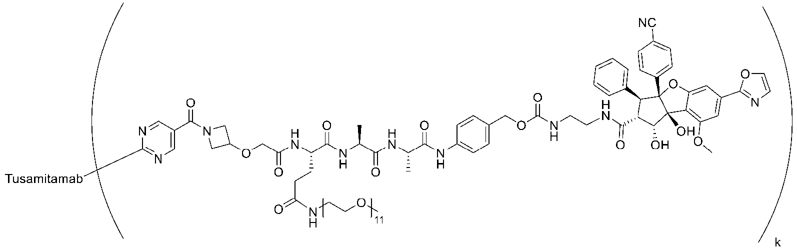 |
| II-E14 | 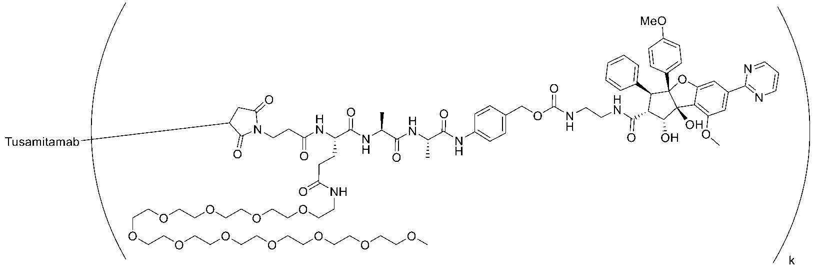 |
| II-E15 | 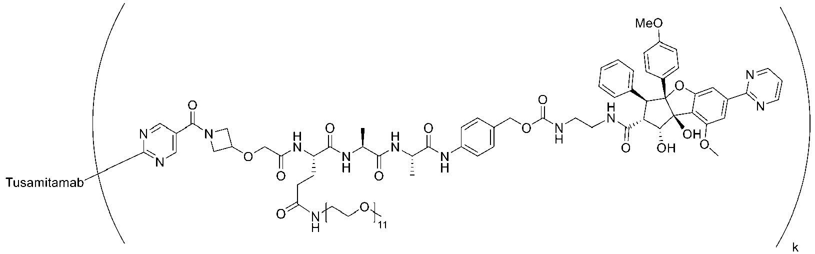 |
| II-E16 | 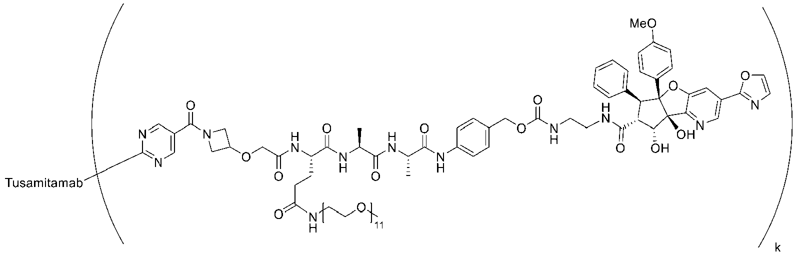 |
| II-E17 | 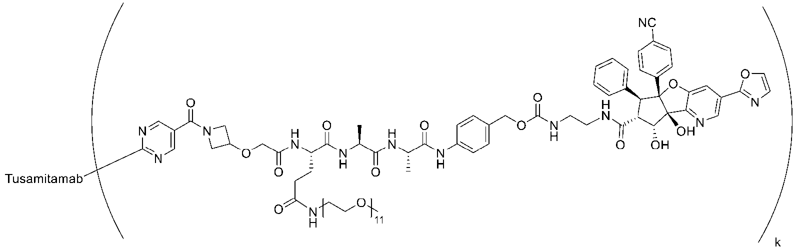 |
| II-E18 | 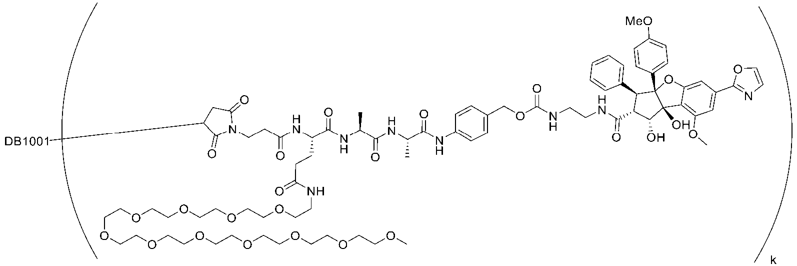 |
| II-E19 | 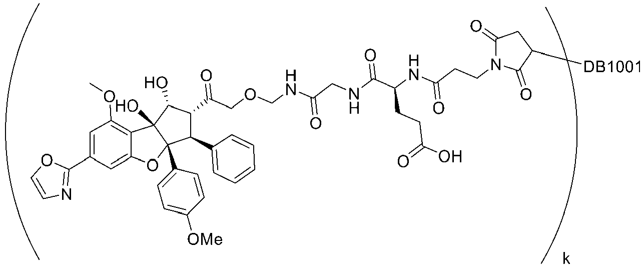 |
| II-E20 | 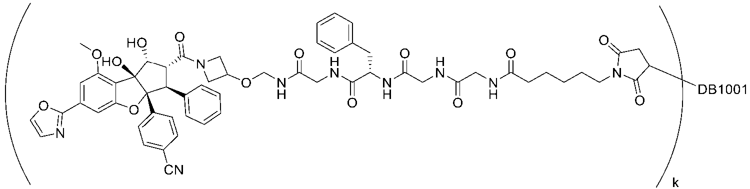 |
| II-E21 | 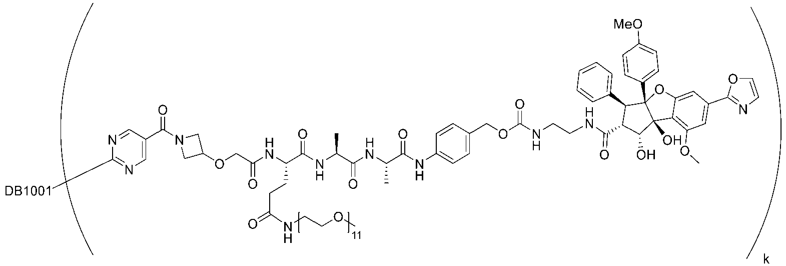 |
| II-E22 | 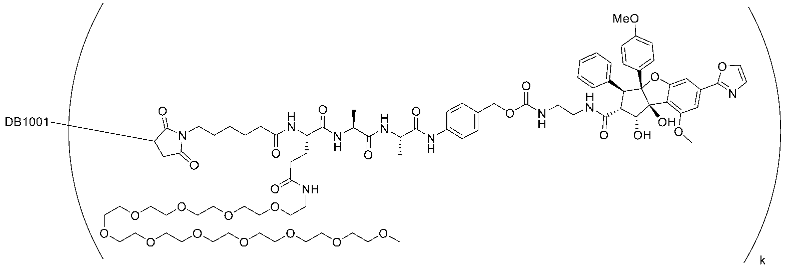 |
| II-E23 | 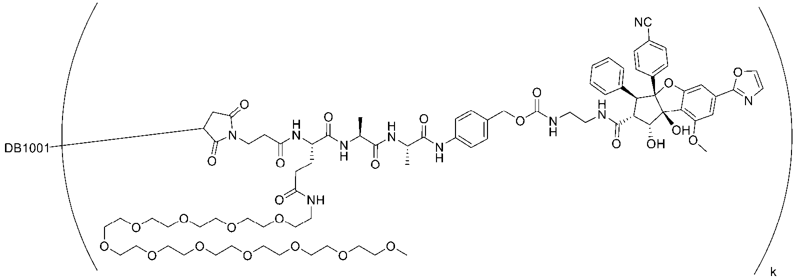 |
| II-E24 | 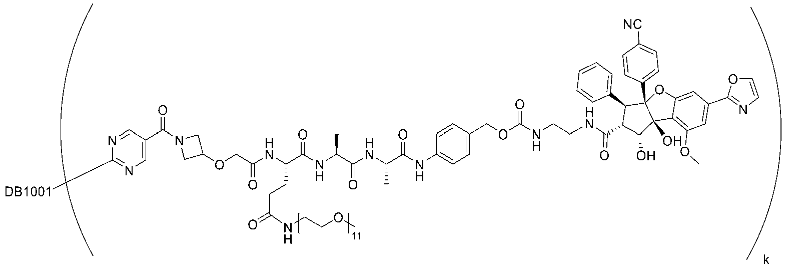 |
| II-E25 | 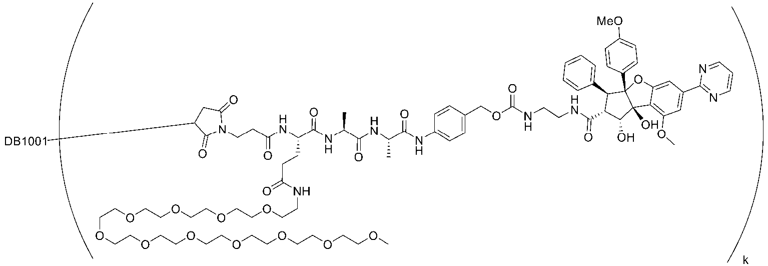 |
| II-E26 | 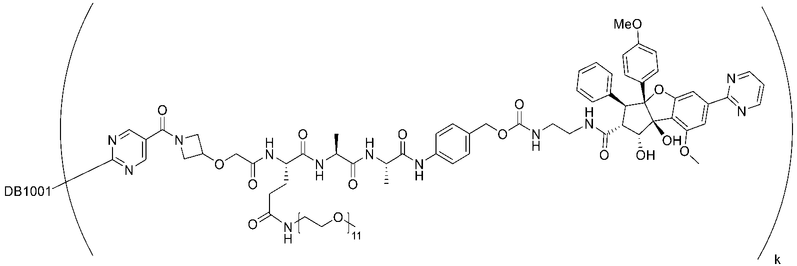 |
| II-E27 | 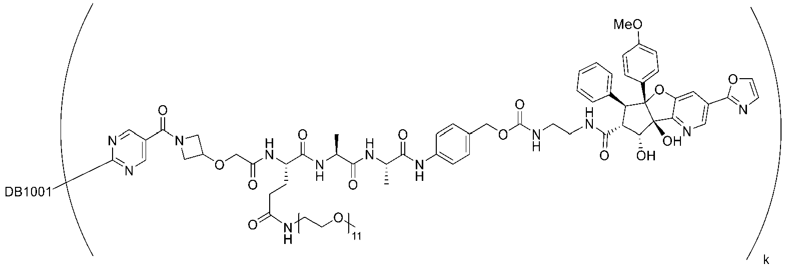 |
| II-E28 | 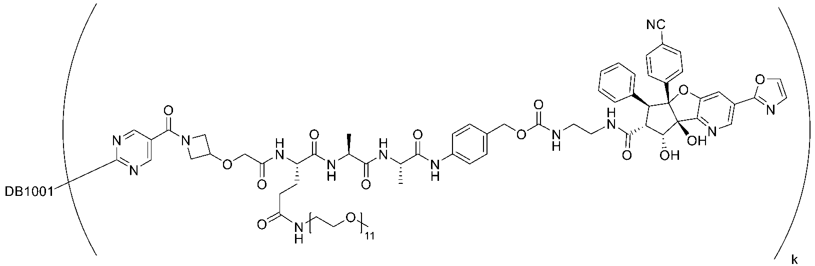 |
| II-E29 | 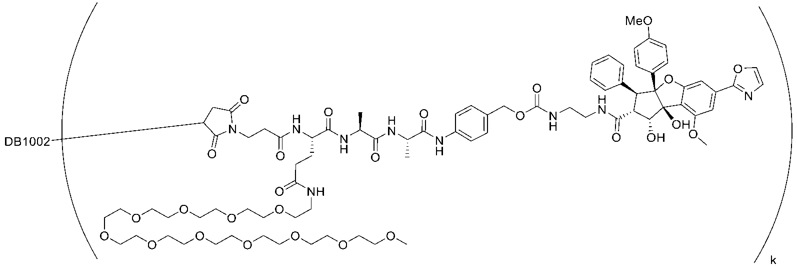 |
| II-E30 | 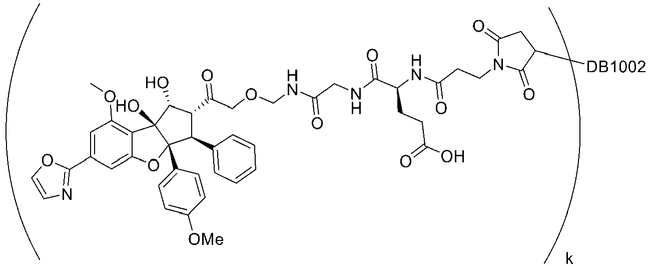 |
| II-E31 | 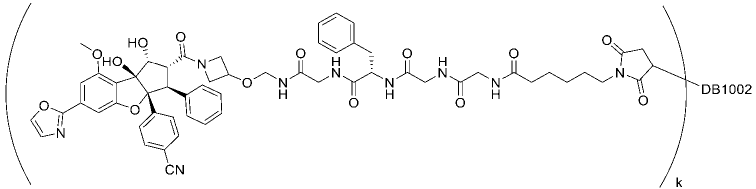 |
| II-E32 | 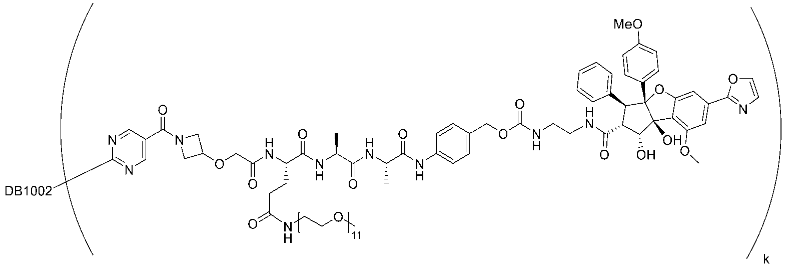 |
| II-E33 | 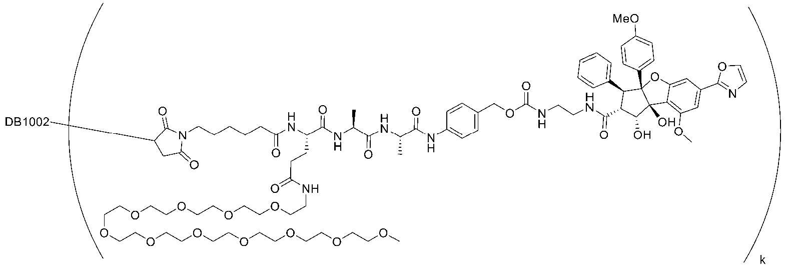 |
| II-E34 | 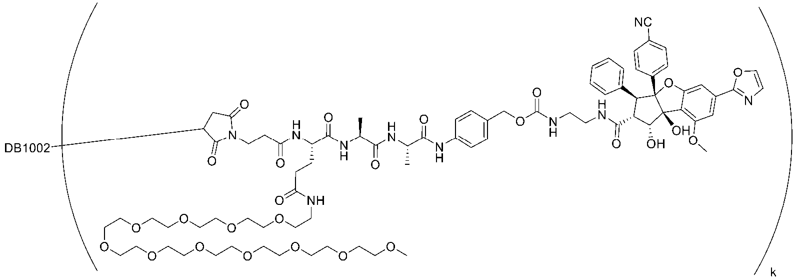 |
| II-E35 | 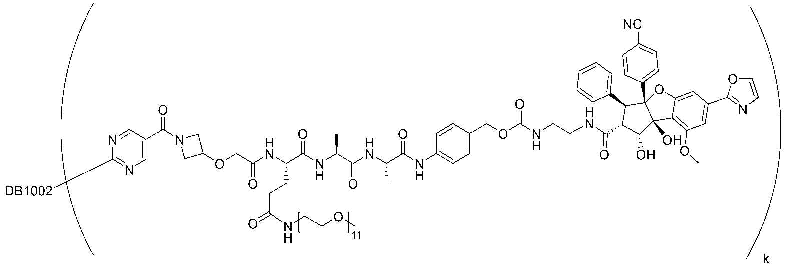 |
| II-E36 | 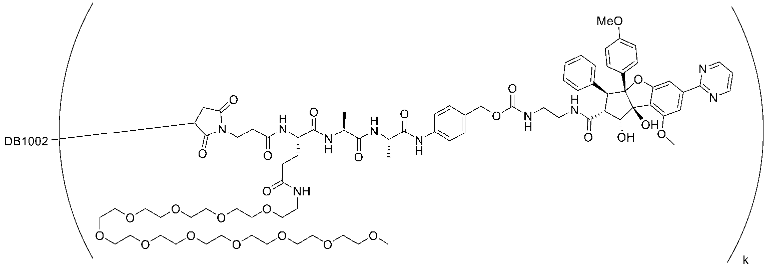 |
| II-E37 | 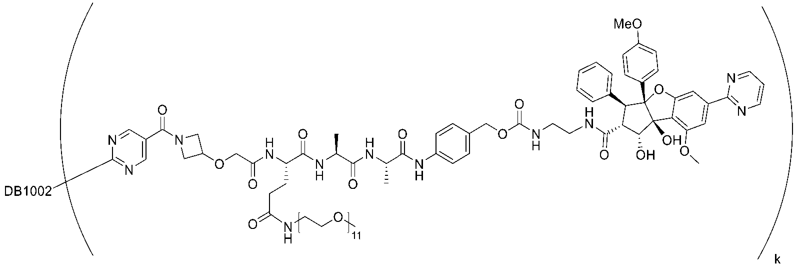 |
| II-E38 | 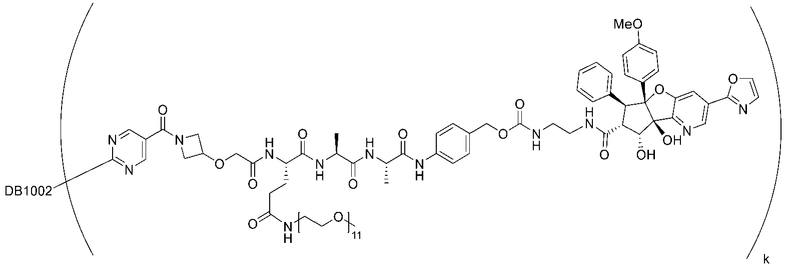 |
| II-E39 | 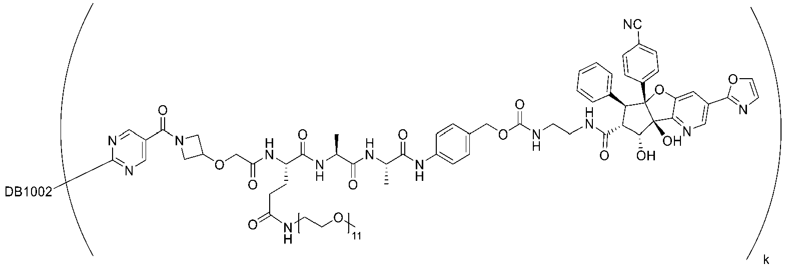 |
| II-E40 | 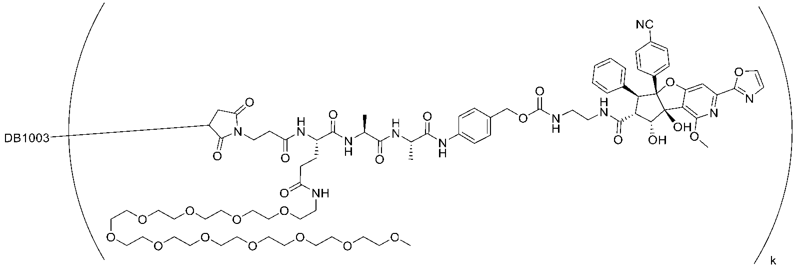 |
| II-E41 | 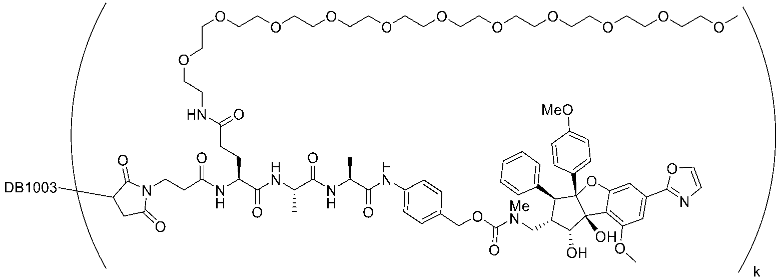 |
| II-E42 | 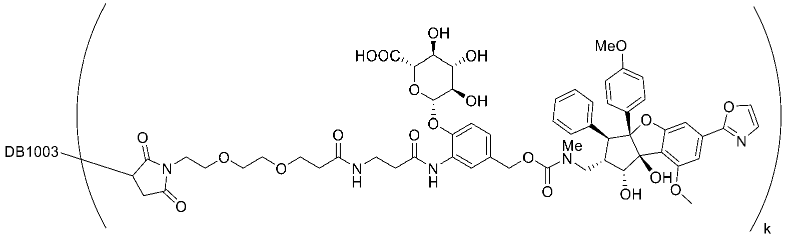 |
| II-E43 | 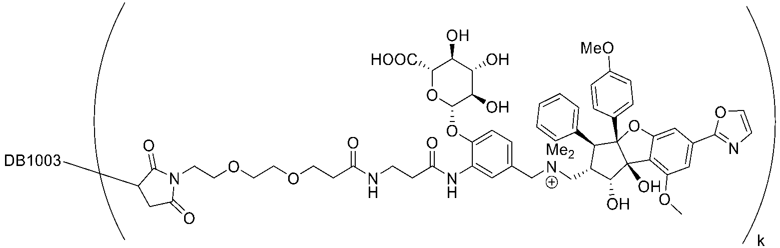 |
| II-E44 | 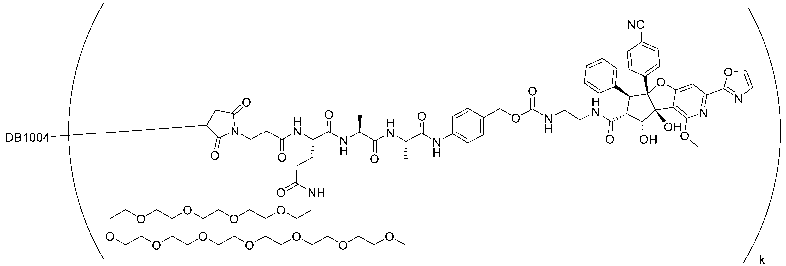 |
| II-E45 | 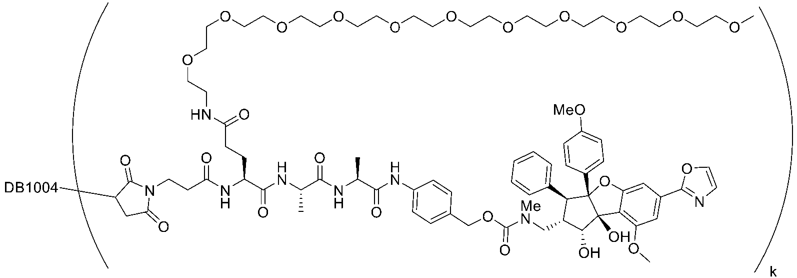 |
| II-E46 | 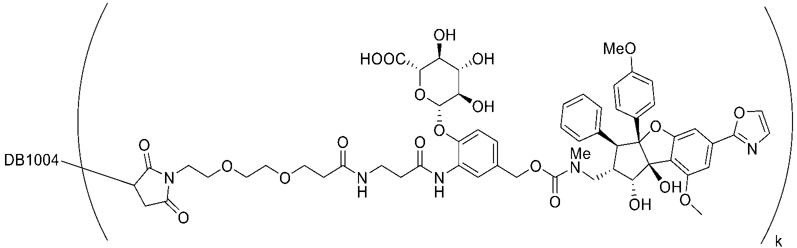 |
| II-E47 | 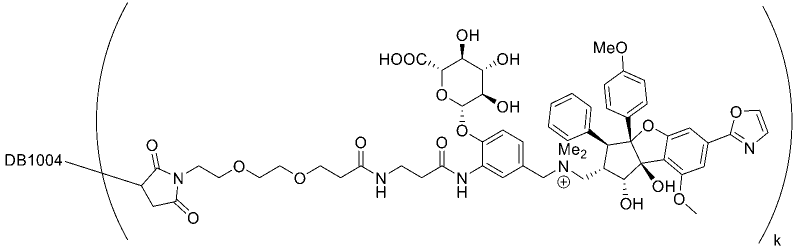 |
| III-E1 | 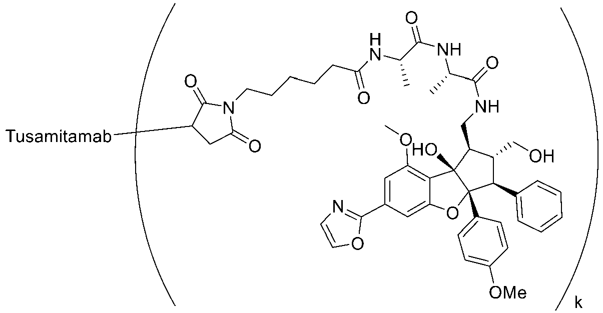 |
| III-E2 | 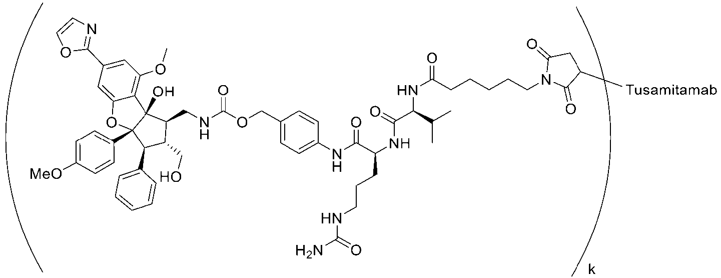 |
| III-E3 | 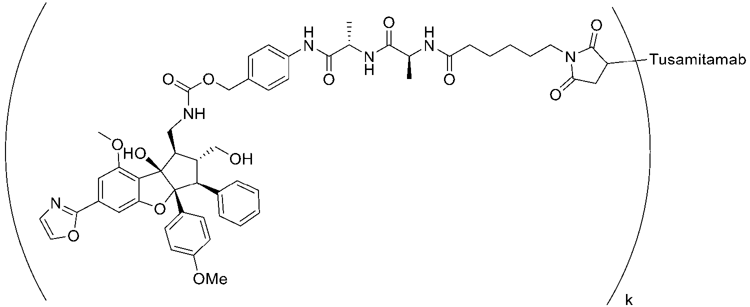 |
| III-E4 | 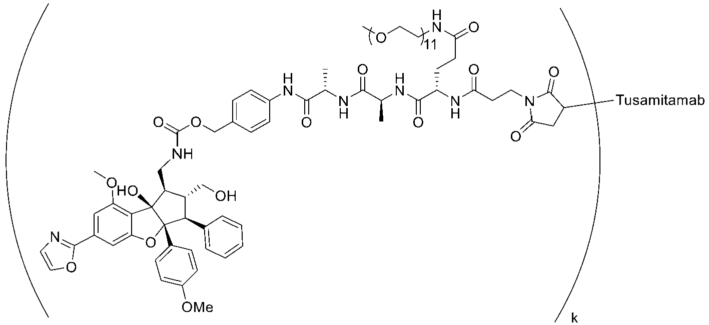 |
| III-E5 | 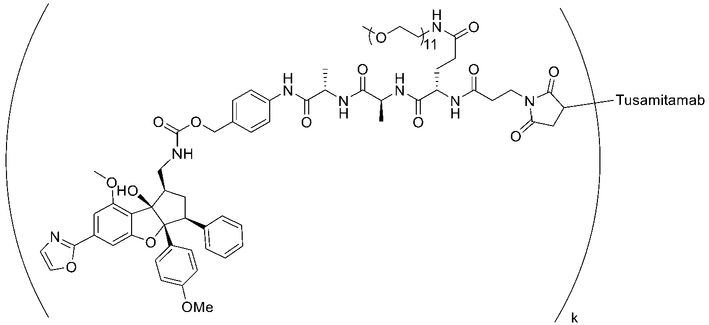 |
| III-E6 | 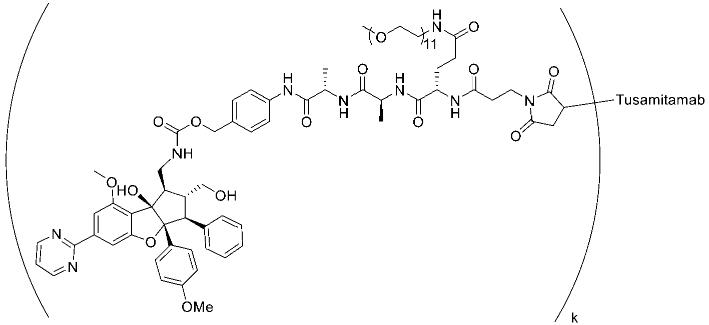 |
| III-E7 | 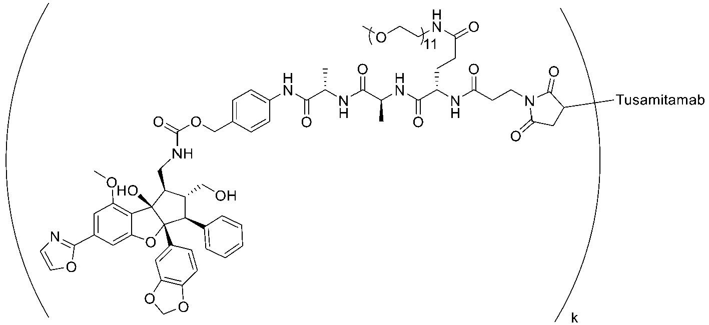 |
| III-E8 | 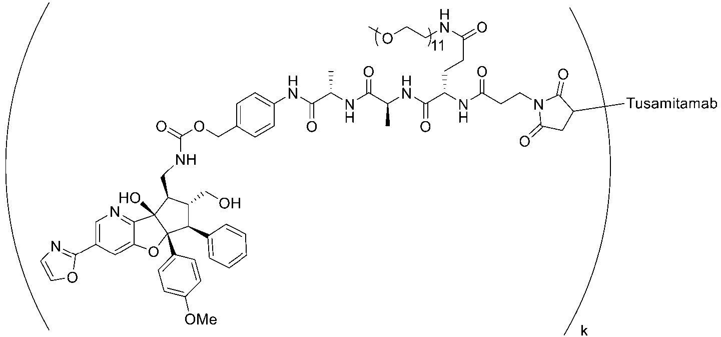 |
| III-E9 | 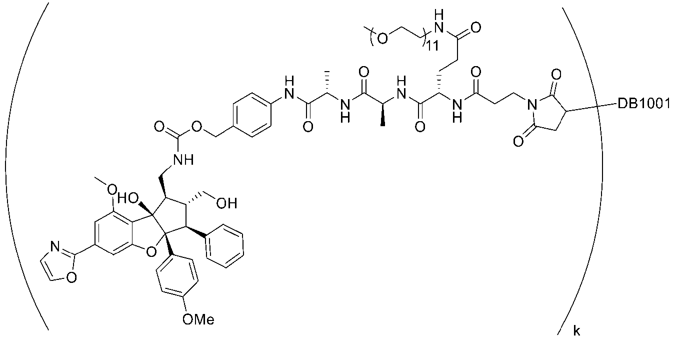 |
| III-E10 | 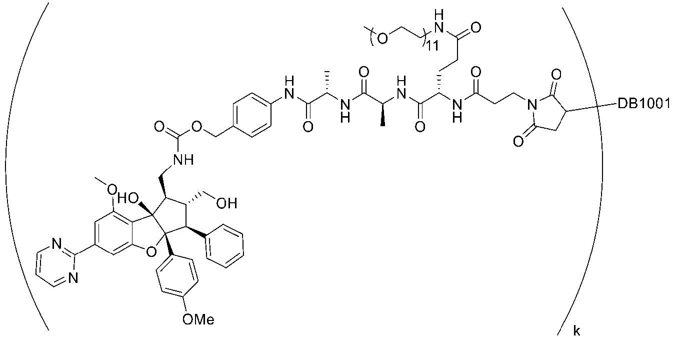 |
| III-E11 | 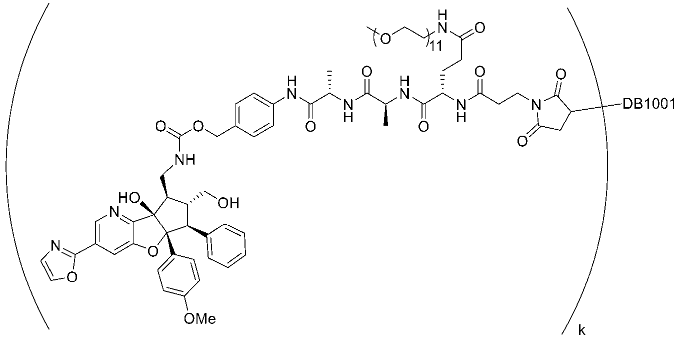 |
| III-E12 | 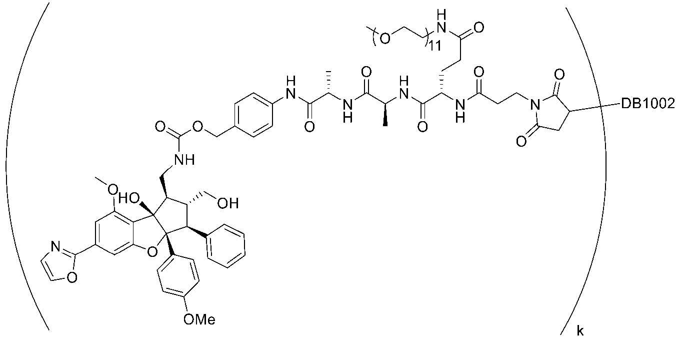 |
| III-E13 | 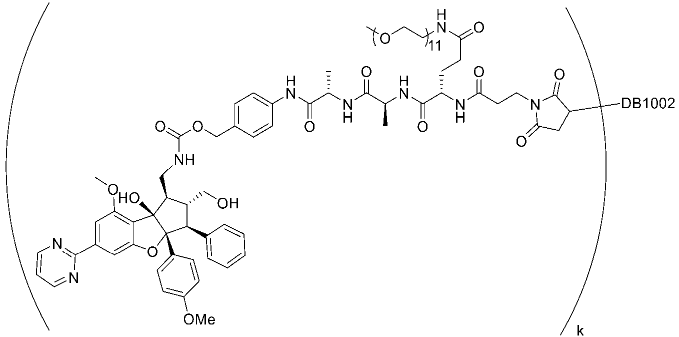 |
| III-E14 | 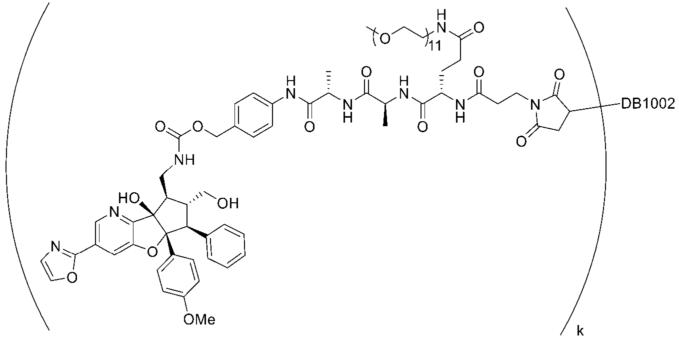 |
| III-E15 | 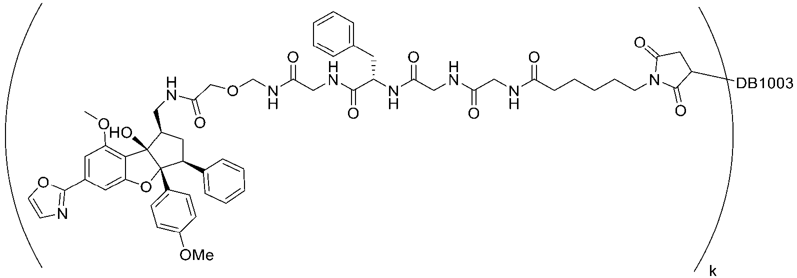 |
| III-E16 | 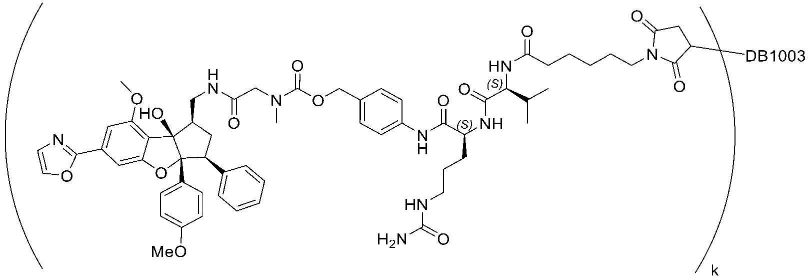 |
| III-E17 | 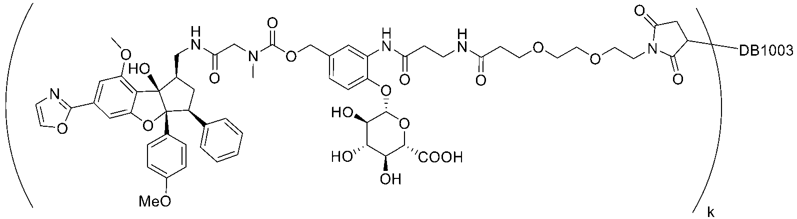 |
| III-E18 | 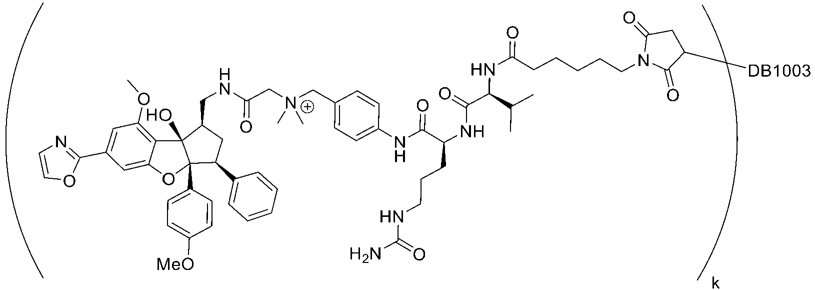 |
| III-E19 | 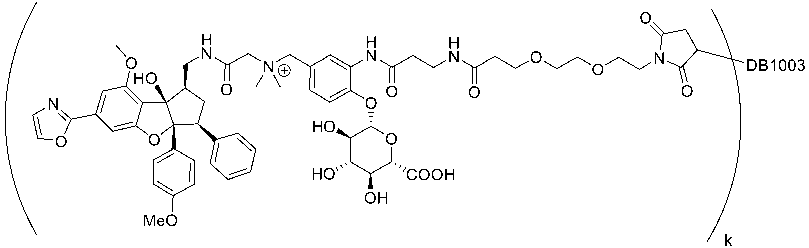 |
| III-E20 | 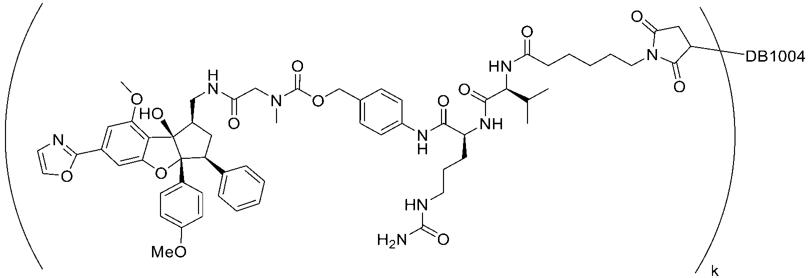 |
| III-E21 | 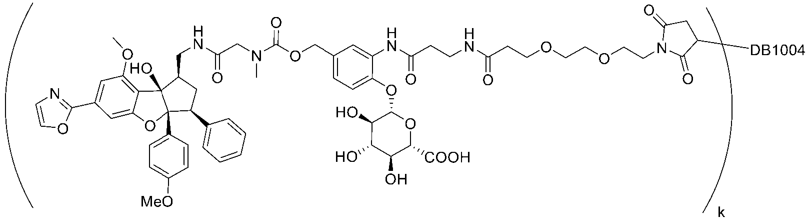 |
| III-E22 | 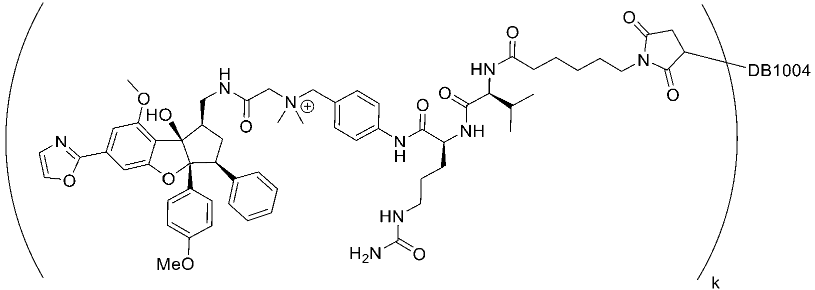 |
| III-E23 | 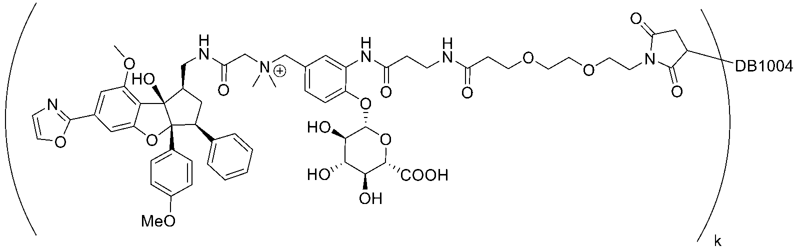 |
| IV-E1 | 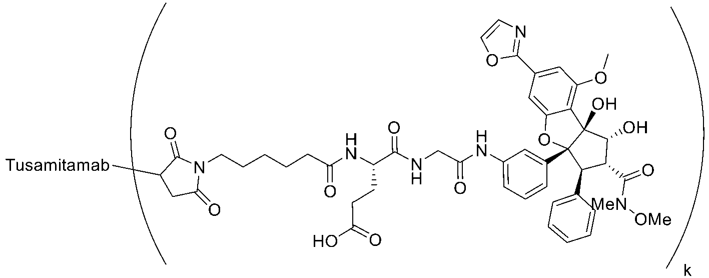 |
| IV-E2 | 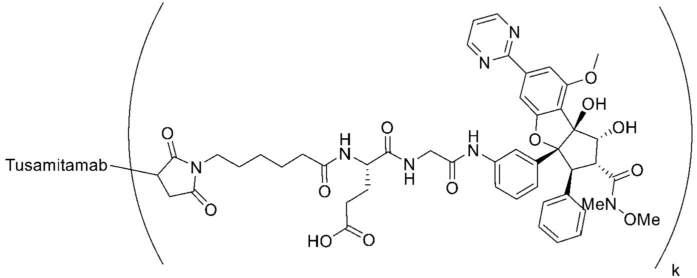 |
| IV-E3 | 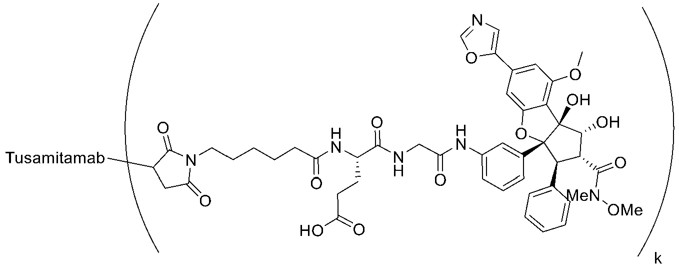 |
| IV-E4 | 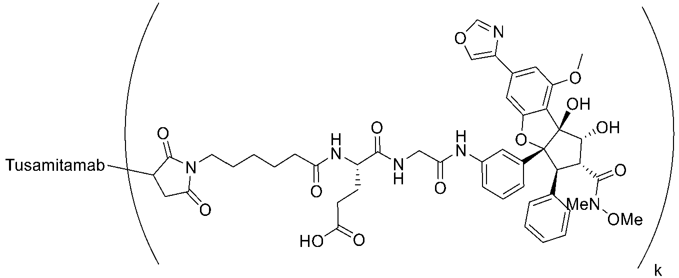 |
| IV-E5 | 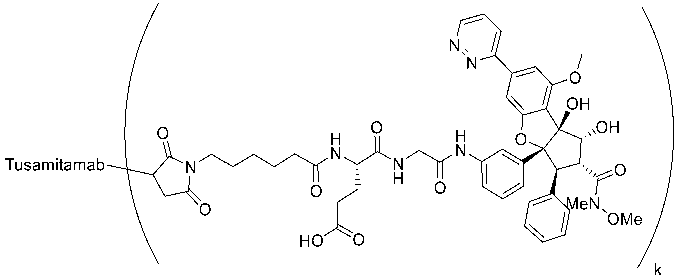 |
| IV-E6 | 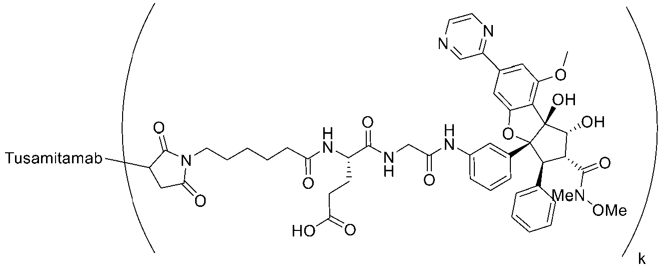 |
| IV-E7 | 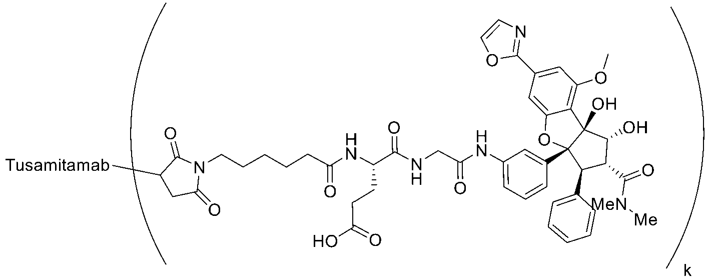 |
| IV-E8 | 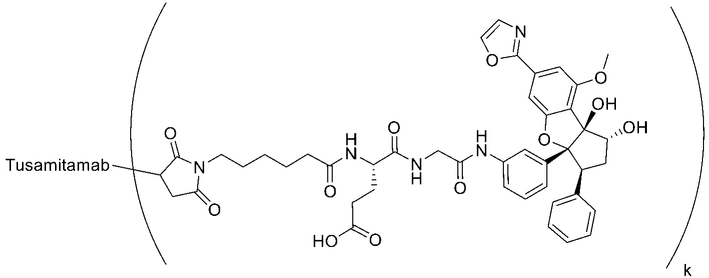 |
| IV-E9 | 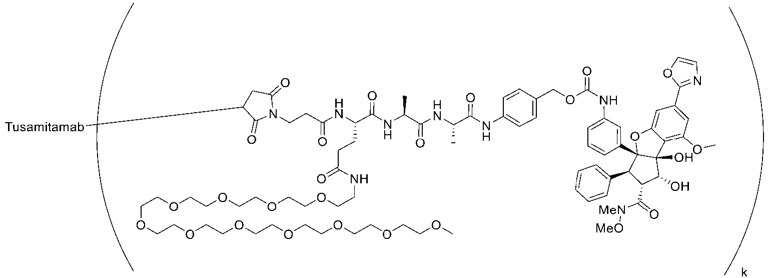 |
| IV-E10 | 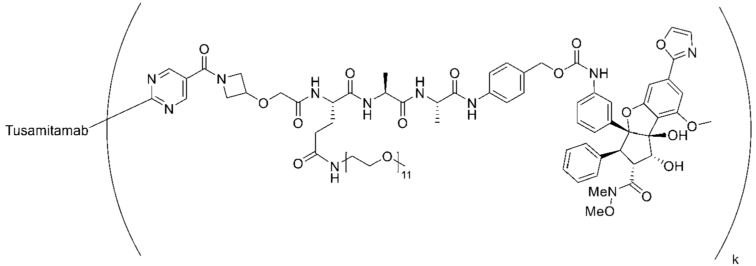 |
| IV-E11 | 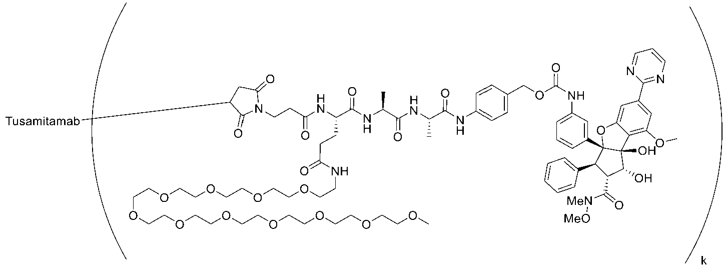 |
| IV-E12 | 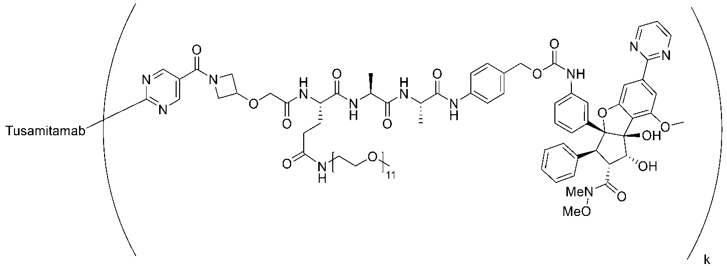 |
| IV-E13 | 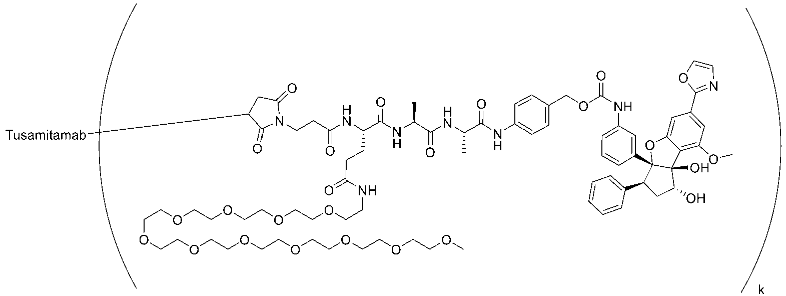 |
| IV-E14 | 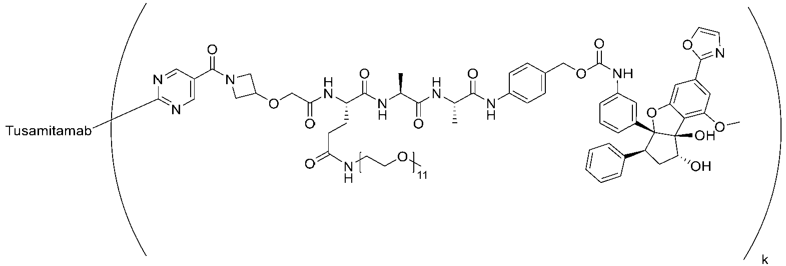 |
| IV-E15 | 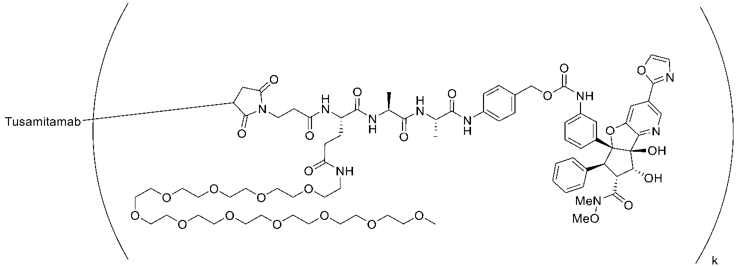 |
| IV-E16 | 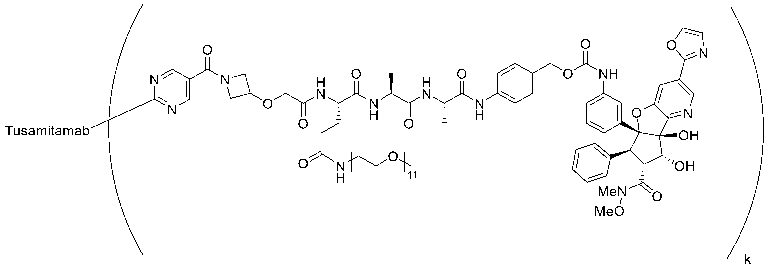 |
| IV-E17 | 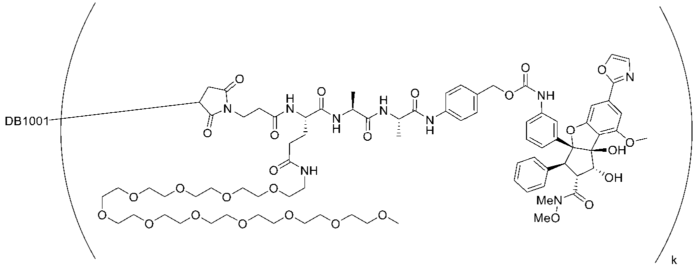 |
| IV-E18 | 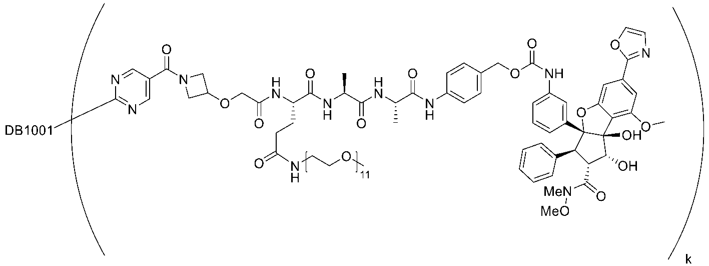 |
| IV-E19 | 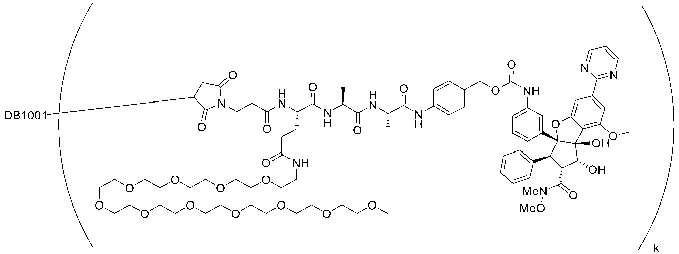 |
| IV-E20 | 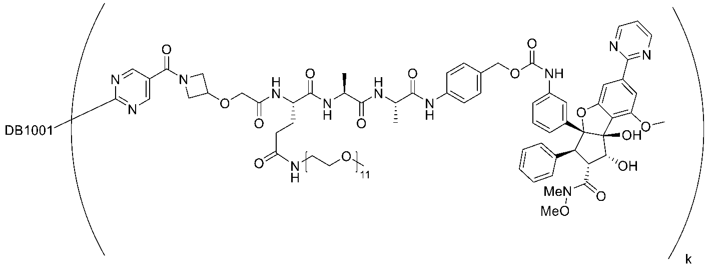 |
| IV-E21 | 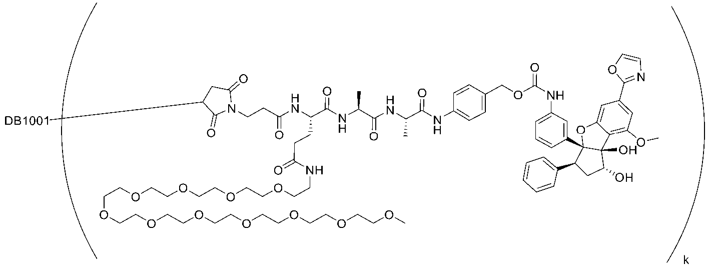 |
| IV-E22 | 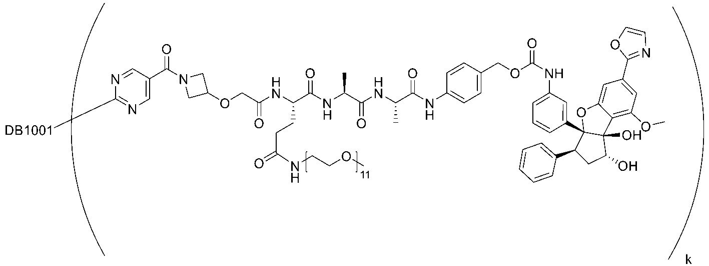 |
| IV-E23 | 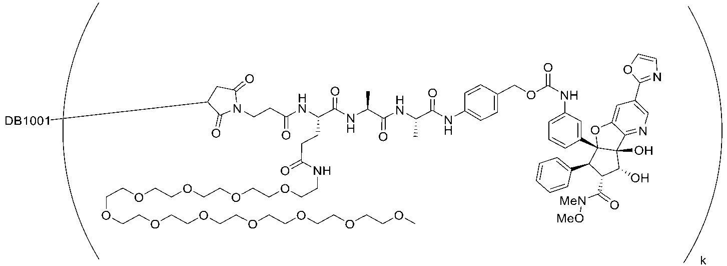 |
| IV-E24 | 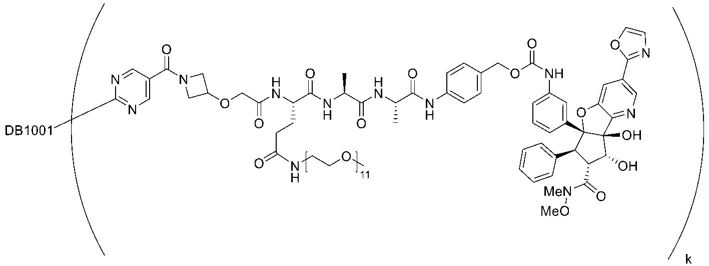 |
| IV-E25 | 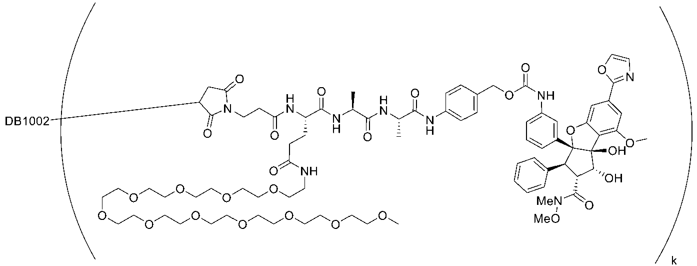 |
| IV-E26 | 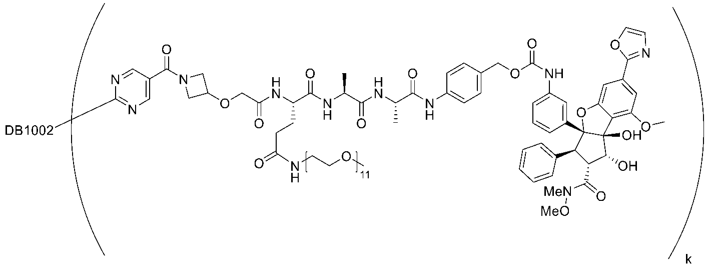 |
| IV-E27 | 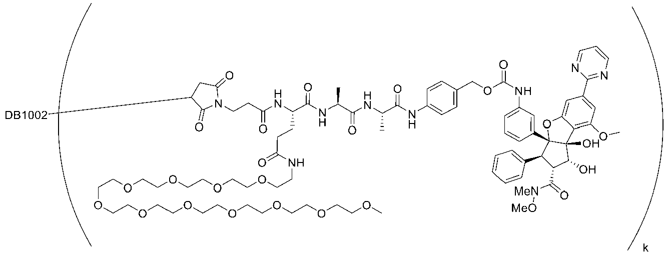 |
| IV-E28 | 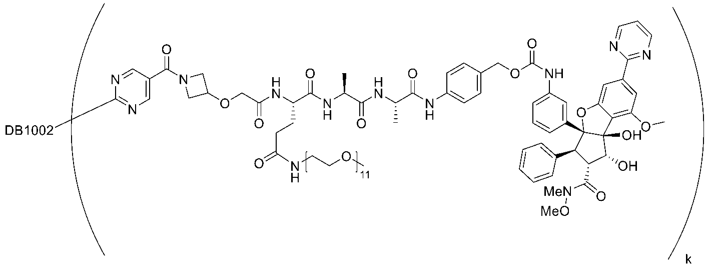 |
| IV-E29 | 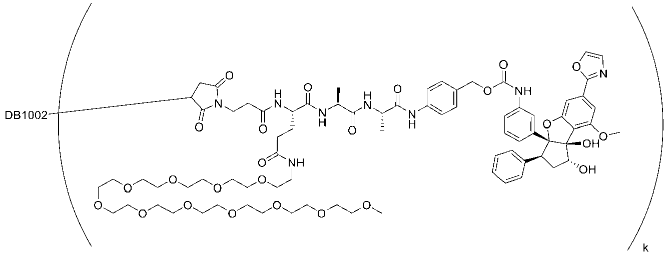 |
| IV-E30 | 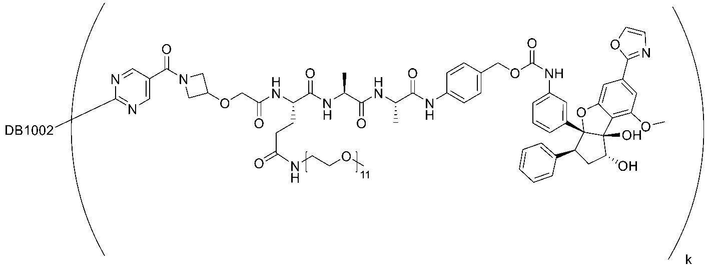 |
| IV-E31 | 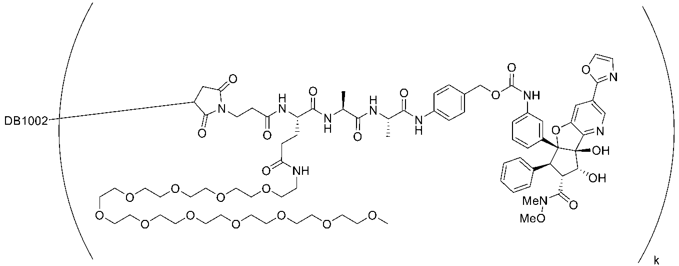 |
| IV-E32 | 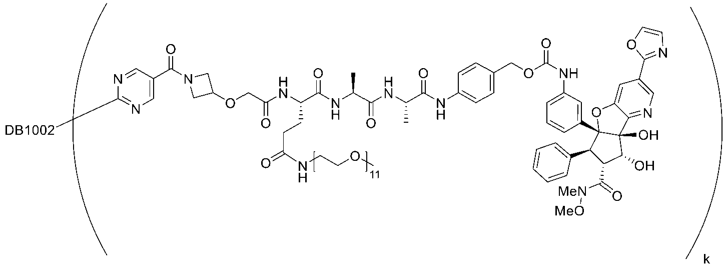 |
| IV-E33 | 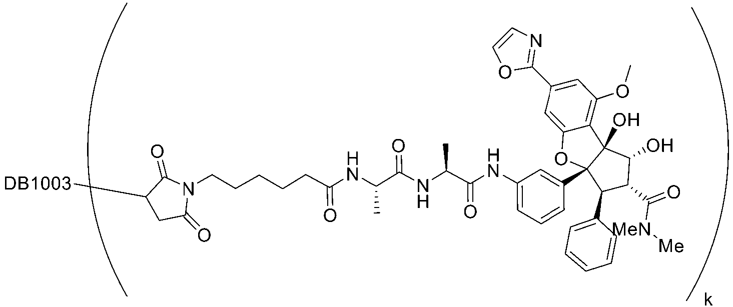 |
| IV-E34 | 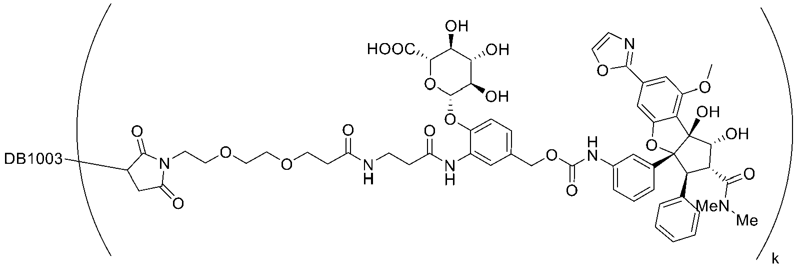 |
| IV-E35 | 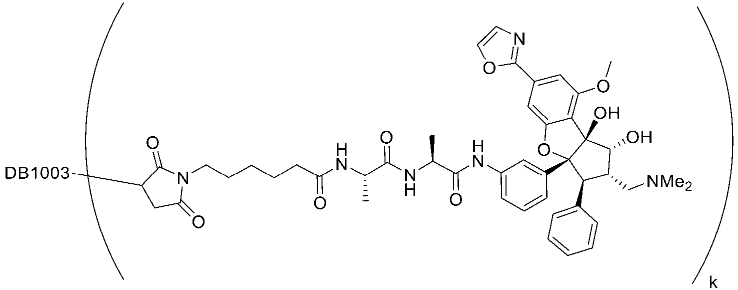 |
| IV-E36 | 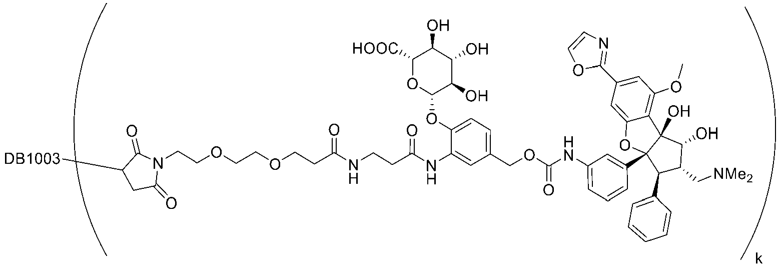 |
| IV-E37 | 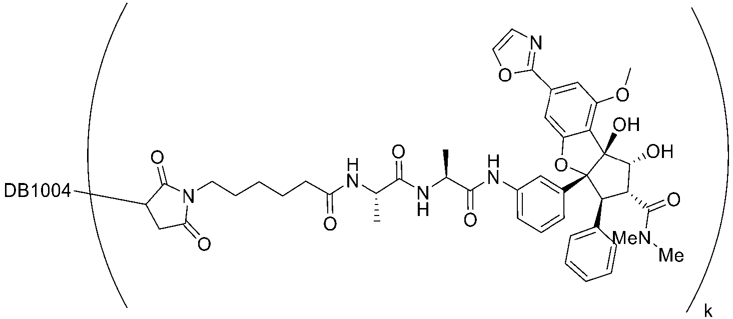 |
| IV-E38 | 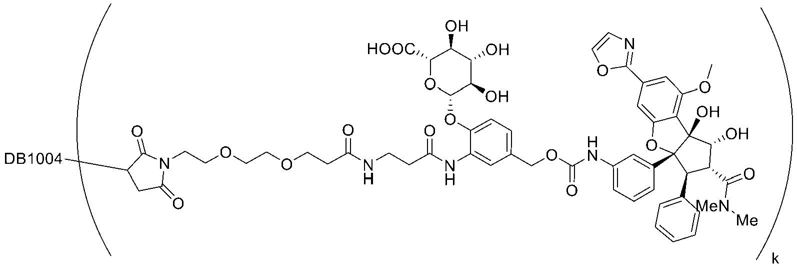 |
| V-E1 | 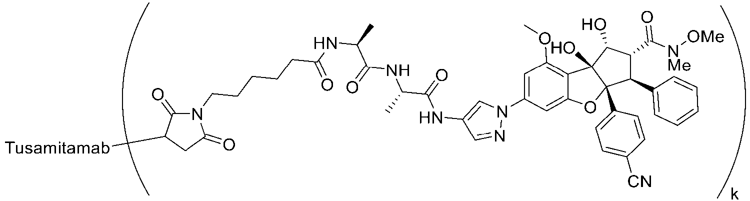 |
| V-E2 | 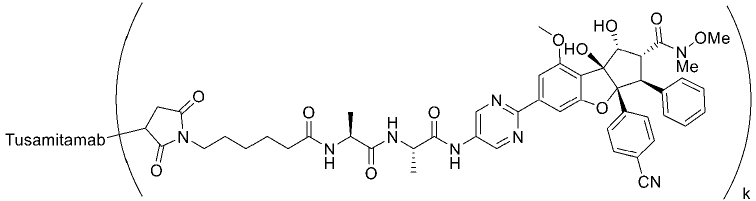 |
| V-E3 | 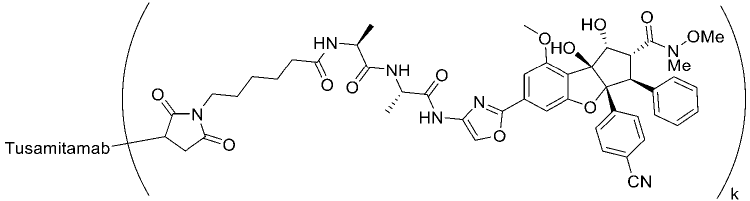 |
| V-E4 | 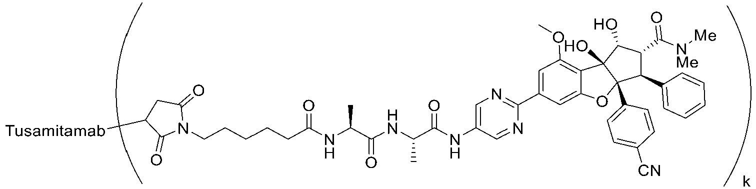 |
| V-E5 | 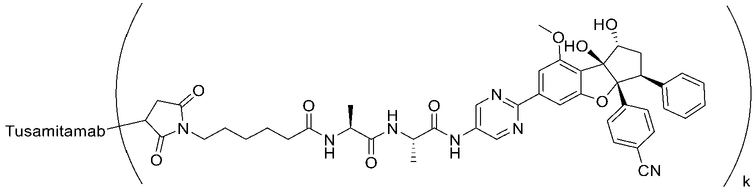 |
| V-E6 | 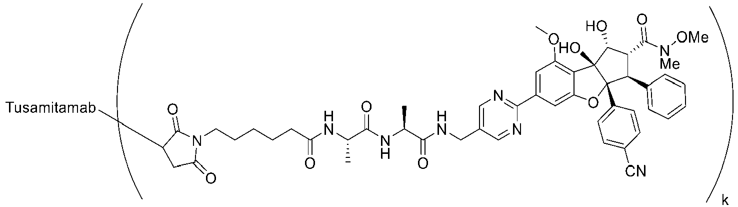 |
| V-E7 | 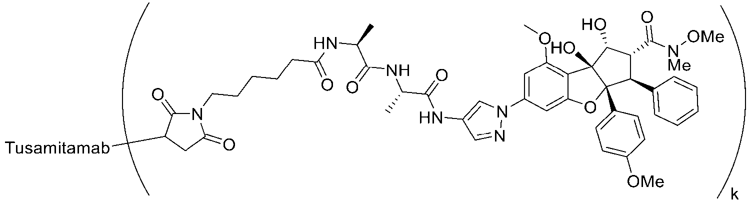 |
| V-E8 | 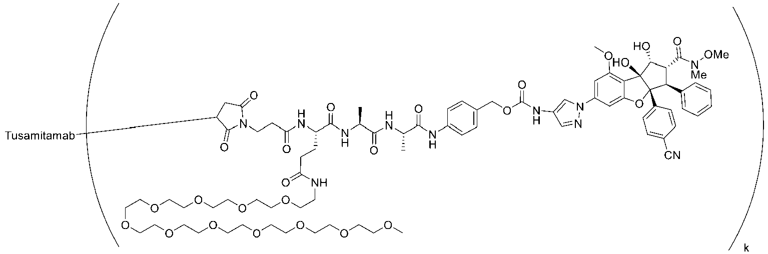 |
| V-E9 | 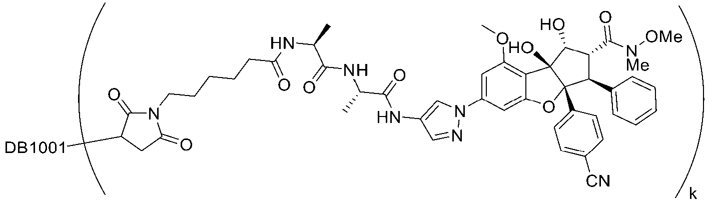 |
| V-E10 | 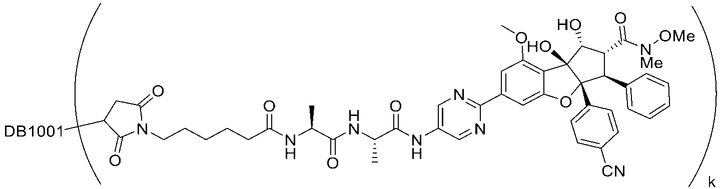 |
| V-E11 | 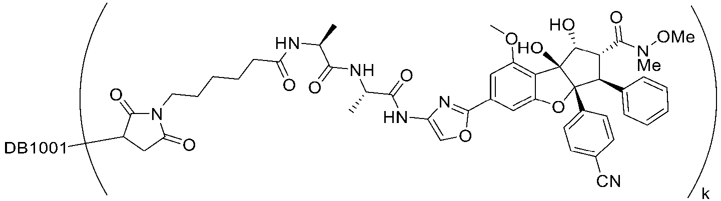 |
| V-E12 | 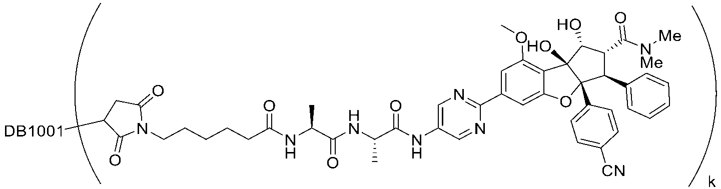 |
| V-E13 | 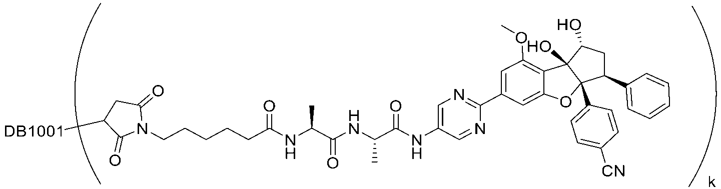 |
| V-E14 | 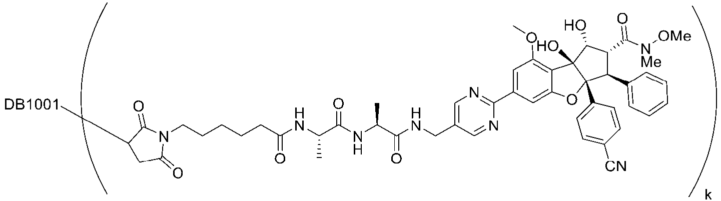 |
| V-E15 | 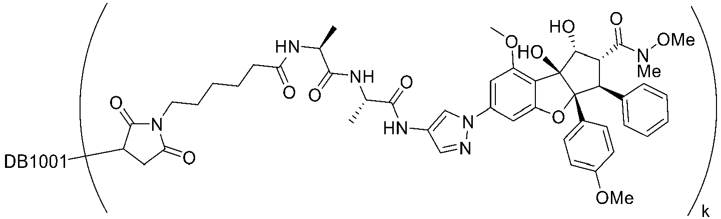 |
| V-E16 | 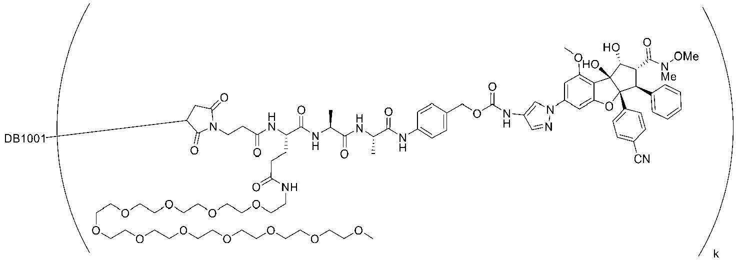 |
| V-E17 | 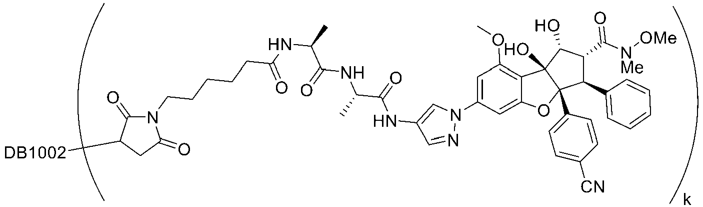 |
| V-E18 | 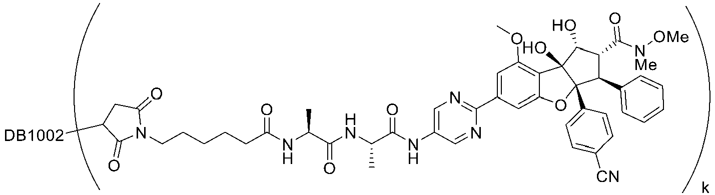 |
| V-E19 | 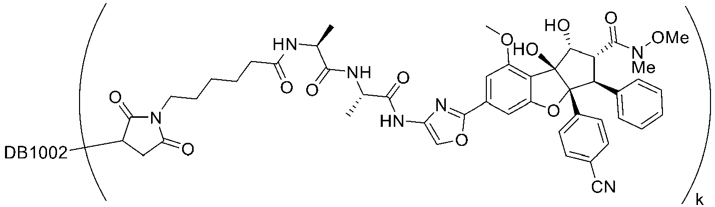 |
| V-E20 | 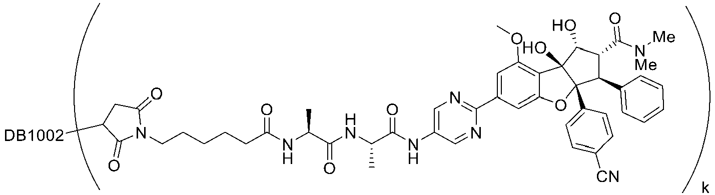 |
| V-E21 | 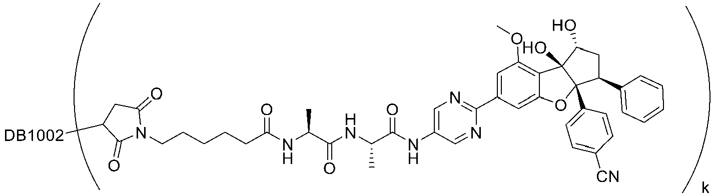 |
| V-E22 | 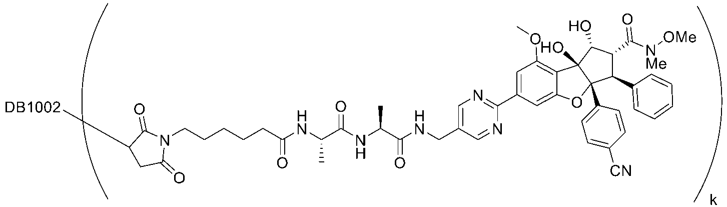 |
| V-E23 | 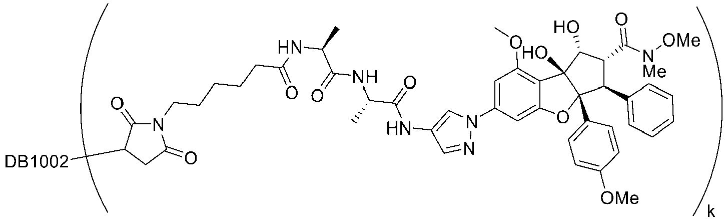 |
| V-E24 | 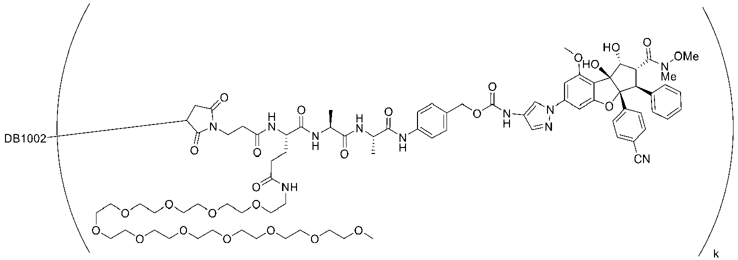 |
| V-E25 | 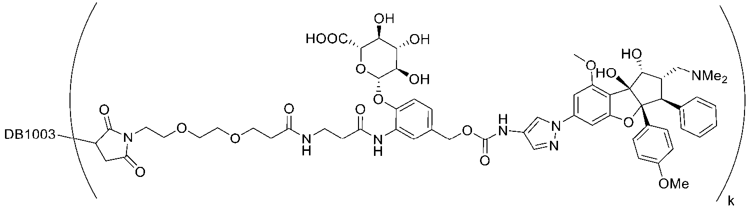 |
| V-E26 | 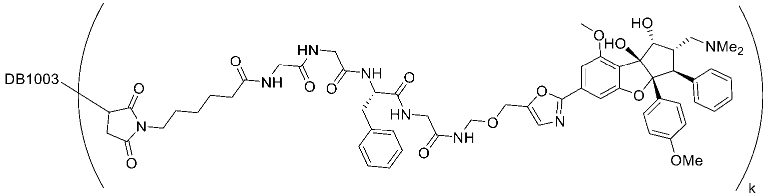 |
| V-E27 | 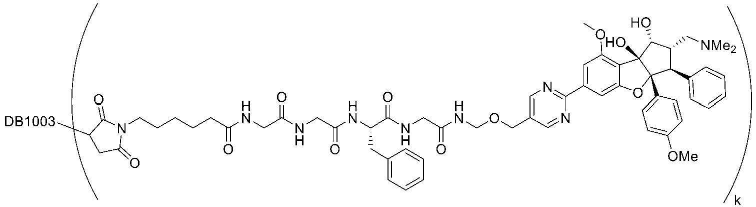 |
| V-E28 | 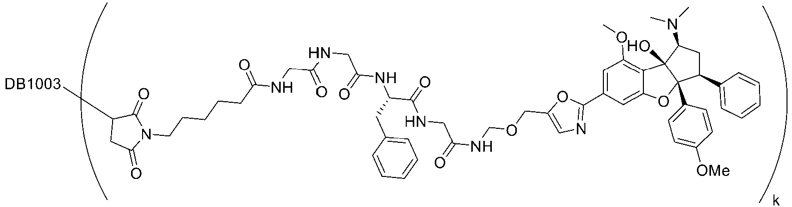 |

| | |
|---|---|
| V-E29 | 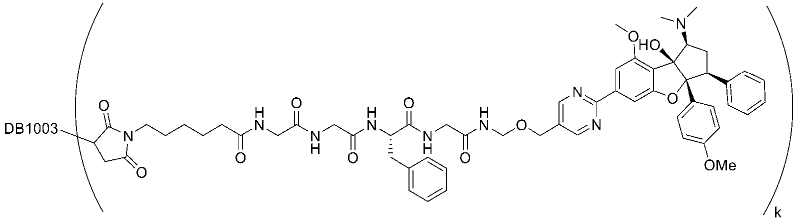 |
| V-E30 | 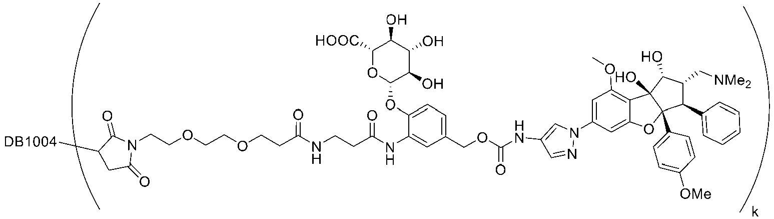 |

wherein k is as described in any one of the embodiments of the present disclosure.

A second aspect of the present disclosure provides a mixture of ligand-drug conjugates, comprising or consisting of the aforementioned ligand-drug conjugate, the tautomer thereof, the enantiomer thereof, the diastereoisomer thereof, the pharmaceutically acceptable salt thereof, or the solvate thereof, wherein the ligand-drug conjugate has one, two, or more k values.

In some embodiments, the mixture of ligand-drug conjugates has an average drug-to-ligand ratio (DAR) selected from integers or decimals from 1 to 32. In certain embodiments, the DAR value is selected from integers or decimals from 1 to 16. In certain embodiments, the DAR value is selected from integers or decimals from 2 to 9, preferably integers or decimals from 2 to 8, e.g., 2, 3, 4, 5, 6, 7, 8, 3.01, 3.22, 3.75, 3.76, 3.87, 3.89, 3.95, 3.98, 4.05, 4.06, 4.08, 4.11, 4.16, 4.22, 4.29, 5.11, 5.58, 6.17, 6.46, 6.56, 6.82, 6.88, 6.98, 7.02, 7.06, 7.08, 7.12, 7.18, 7.22, 7.25, 7.28, 7.31, 7.32, 7.33, 7.41, 7.42, 7.43, 7.45, 7.48, 7.52, 7.53, 7.56, 7.57, 7.58, 7.62, 7.64, 7.65, 7.71, 7.73, 7.78, 7.81, 7.82, 7.87, 7.88, or 7.93, e.g., 7.83, 7.84, 7.85, 7.86, 7.89, 7.90, 7.91, 7.92, 7.94, 7.95, 7.96, 7.97, 7.98, 7.99, 8.00, 8.01, 8.02, 8.03, 8.04, 8.05, 8.06, 8.07, 8.08, 8.09, or 8.10.

### Drug-Linker

A third aspect of the present disclosure provides a compound or a racemic mixture, tautomer, stereoisomer, enantiomer, or diastereoisomer thereof, or a mixture thereof, or a pharmaceutically acceptable salt or solvate thereof, wherein the compound is selected from a structure represented by formula I-D, II-D', III-D', IV-D, or V-D: 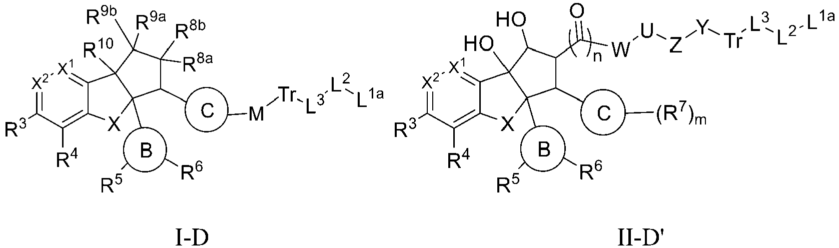 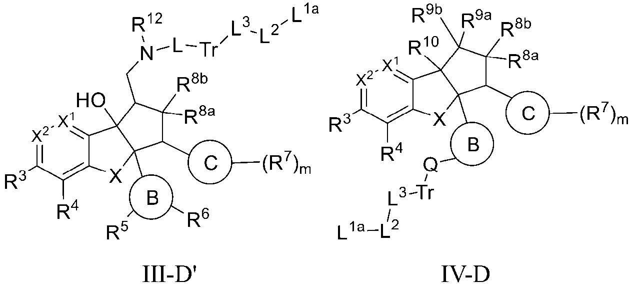 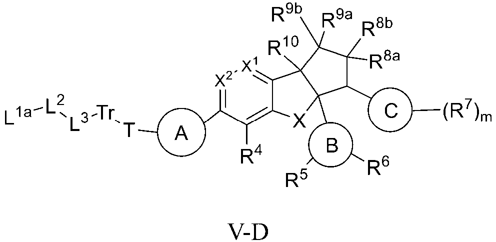 wherein:
X, X¹, X², ring A, ring B, ring C, R³, R⁴, R⁵, R⁶, R⁷, R^{8a}, R^{8b}, R^{9a}, R^{9b}, R¹⁰, R¹², m, n, M, W, U, Z, Y, L, Q, T, Tr, L², and L³ are defined as described in any one of the embodiments of the first aspect of the present disclosure;
L^{1a} is a linking unit.

The third aspect of the present disclosure further provides a compound or a racemic mixture, tautomer, stereoisomer, enantiomer, or diastereoisomer thereof, or a mixture thereof, or a pharmaceutically acceptable salt or solvate thereof, wherein the compound is selected from a structure represented by formula I-D, II-D, III-D, IV-D, or V-D: 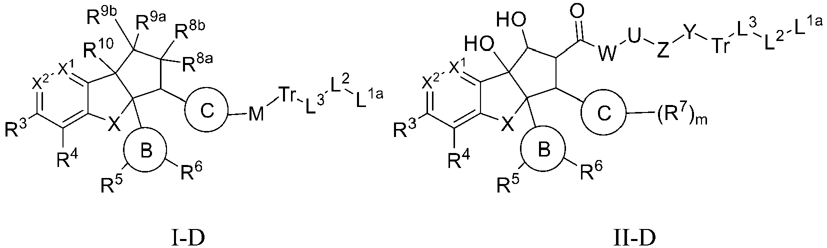 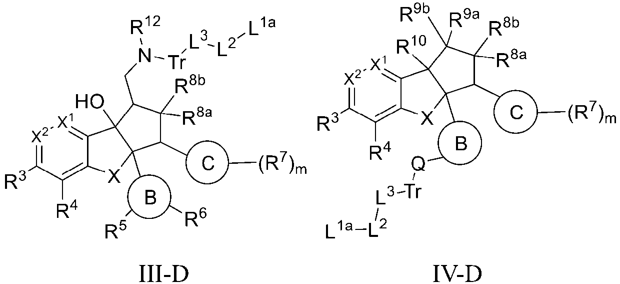 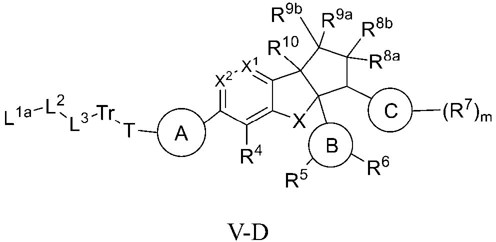 wherein:
X, X¹, X², ring A, ring B, ring C, R³, R⁴, R⁵, R⁶, R⁷, R^{8a}, R^{8b}, R^{9a}, R^{9b}, R¹⁰, R¹², m, M, W, U, Z, Y, Q, T, Tr, L², and L³ are defined as described in any one of the embodiments of the first aspect of the present disclosure;
L^{1a} is a linking unit.

In certain embodiments, the compound is selected from a structure represented by formula I-D: 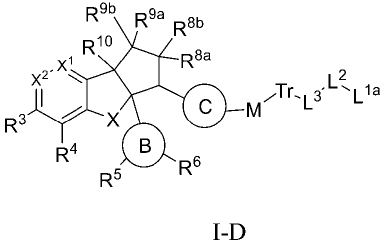 wherein X, X¹, X², ring B, ring C, R³, R⁴, R⁵, R⁶, R^{8a}, R^{8b}, R^{9a}, R^{9b}, R¹⁰, M, Tr, L^{1a}, L², and L³ are defined as described in any one of the embodiments of the present disclosure.

In certain embodiments, the compound is selected from a structure represented by formula II-D': 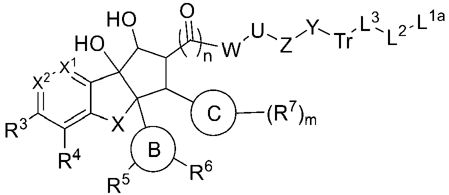 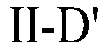 wherein X, X¹, X², ring B, ring C, R³, R⁴, R⁵, R⁶, R⁷, m, n, W, U, Z, Y, Tr, L^{1a}, L², and L³ are defined as described in any one of the embodiments of the present disclosure.

In certain embodiments, the compound is selected from a structure represented by formula II-D: 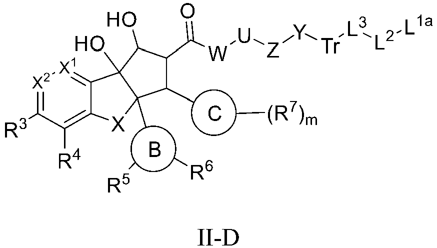 wherein X, X¹, X², ring B, ring C, R³, R⁴, R⁵, R⁶, R⁷, m, W, U, Z, Y, Tr, L^{1a}, L², and L³ are defined as described in any one of the embodiments of the present disclosure.

In certain embodiments, the compound is selected from a structure represented by formula III-D': 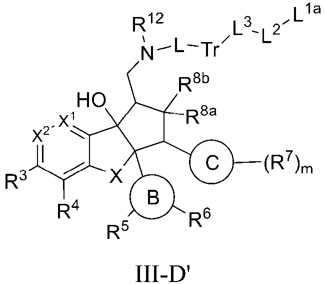 wherein X, X¹, X², ring B, ring C, R³, R⁴, R⁵, R⁶, R⁷, R^{8a}, R^{8b}, R¹², L, m, Tr, L^{1a}, L², and L³ are defined as described in any one of the embodiments of the present disclosure.

In certain embodiments, the compound is selected from a structure represented by formula III-D: 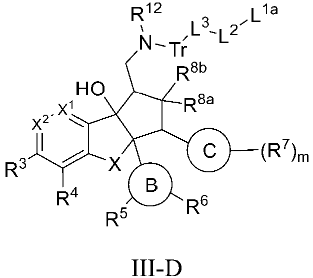 wherein X, X¹, X², ring B, ring C, R³, R⁴, R⁵, R⁶, R⁷, R^{8a}, R^{8b}, R¹², m, Tr, L^{1a}, L², and L³ are defined as described in any one of the embodiments of the present disclosure.

In certain embodiments, the compound is selected from a structure represented by formula IV-D: 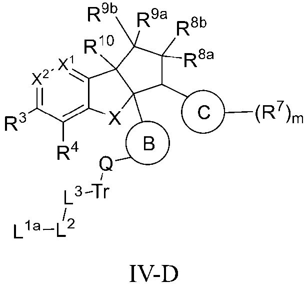 wherein X, X¹, X², ring B, ring C, R³, R⁴, R⁷, R^{8a}, R^{8b}, R^{9a}, R^{9b}, R¹⁰, m, Q, Tr, L^{1a}, L², and L³ are defined as described in any one of the embodiments of the present disclosure.

In certain embodiments, the compound is selected from a structure represented by formula V-D: 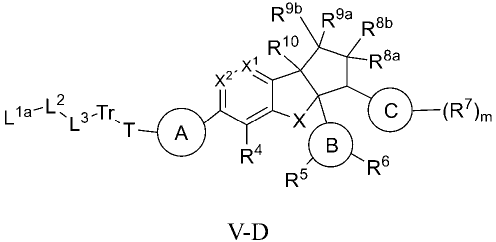 wherein X, X¹, X², ring A, ring B, ring C, R⁴, R⁵, R⁶, R⁷, R^{8a}, R^{8b}, R^{9a}, R^{9b}, R¹⁰, m, T, Tr, L^{1a}, L², and L³ are defined as described in any one of the embodiments of the present disclosure.

In certain embodiments, L^{1a} is selected from -C(=O)-(C₁-C₈)alkyl, -C(=O)O-(4-8 membered)heterocyclyl, -C(=O)O-(C₆-C₁₀)aryl, (4-8 membered)heterocyclyl, and (5-10 membered)heteroaryl, wherein the -C(=O)-(C₁-C₈)alkyl, -C(=O)O-(4-8 membered)heterocyclyl, - C(=O)O-(C₆-C₁₀ membered)aryl, (4-8 membered)heterocyclyl, and (5-10 membered)heteroaryl are optionally substituted with 1, 2, 3, 4, or 5 oxo, halogen, =CH₂, -(C₂-C₄)alkenyl, -S-CH₂-phenyl, -S(=O)₂-(C₁-C₄)alkyl, -O-S(=O)₂-phenyl-(C₁-C₄)alkyl, and -(C₁-C₄)alkyl groups. In certain embodiments, L^{1a} is selected from -C(=O)-(C₁-C₄)alkyl, -C(=O)O-(5-6 membered)heterocyclyl, -C(=O)O-phenyl, (5-6 membered)heterocyclyl, and (5-6 membered)heteroaryl, wherein the -C(=O)-(C₁-C₄)alkyl, -C(=O)O-(5-6 membered)heterocyclyl, - C(=O)O-phenyl, (5-6 membered)heterocyclyl, and (5-6 membered)heteroaryl are optionally substituted with 1, 2, 3, 4, or 5 oxo, fluorine, bromine, =CH₂, vinyl, -S-CH₂-phenyl, -S(=O)₂CH₃, -O-S(=O)₂-phenyl-CH₃, and methyl groups.

In certain embodiments, L^{1a} is selected from

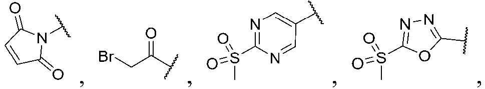

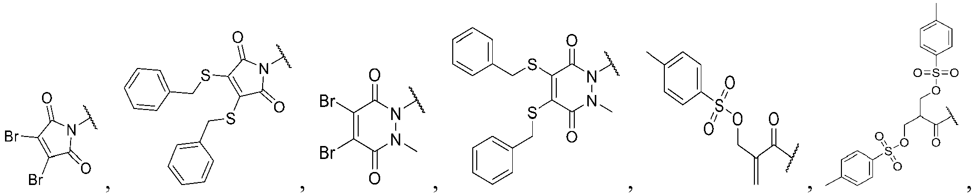

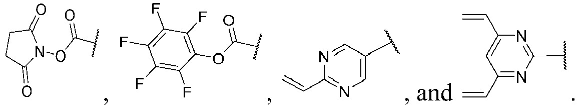

In certain embodiments, L^{1a} is selected from

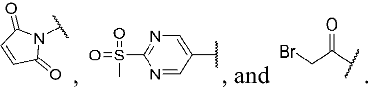

In certain embodiments, L^{1a} is selected from

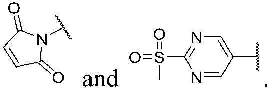

In certain embodiments, L^{1a} is selected from

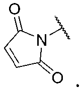

In certain embodiments, L^{1a}-L²- is selected from 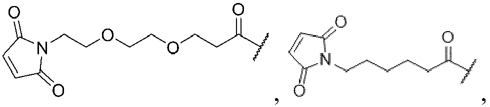 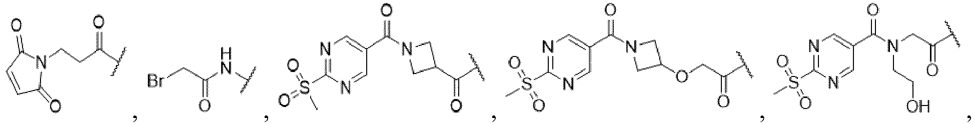 and

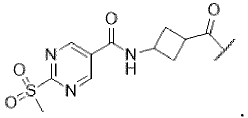

In certain embodiments, L^{1a}-L²- is selected from 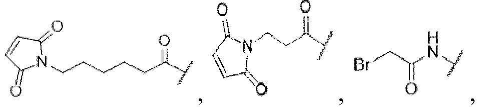 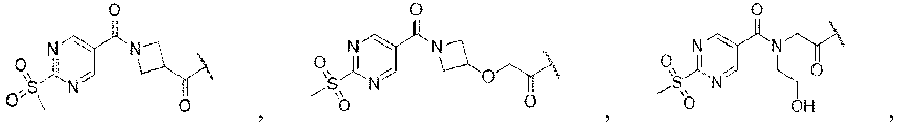 and

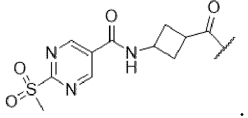

In certain embodiments, L^{1a}-L²- is selected from

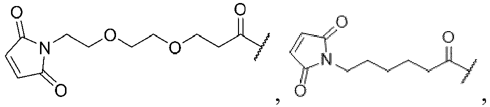

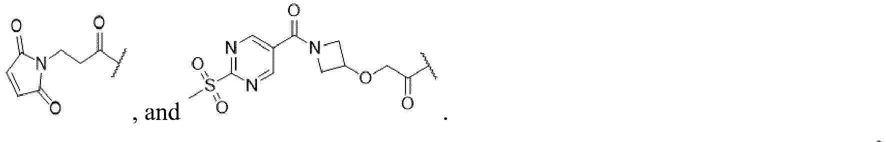

In certain embodiments, L^{1a}-L²- is selected from 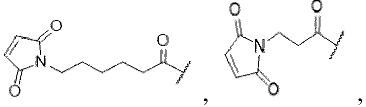 and

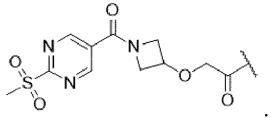

In certain embodiments, L^{1a}-L²- is selected from 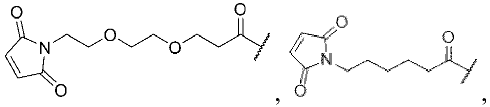 and

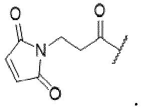

In certain embodiments, L^{1a}-L²- is selected from

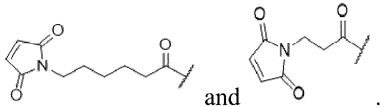

In certain embodiments, L^{1a}-L²-L³- is selected from 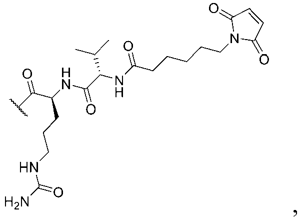 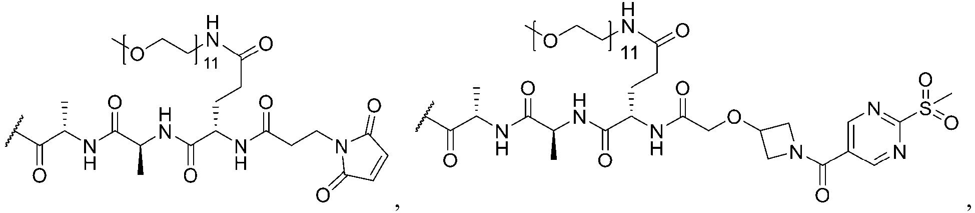 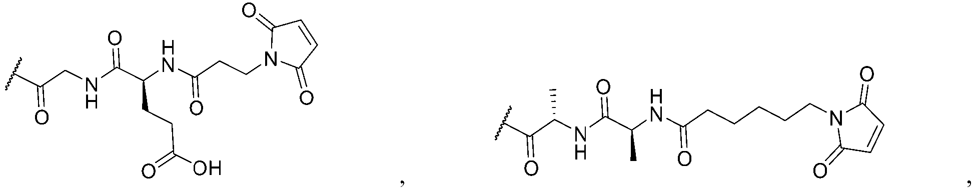 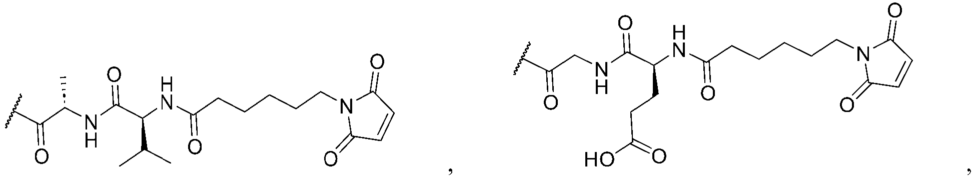 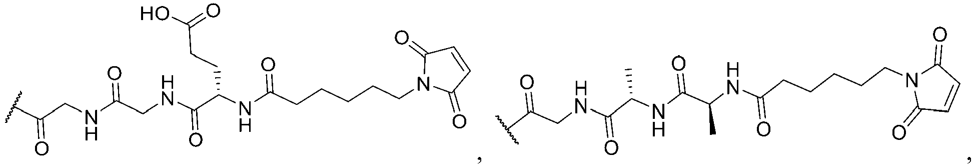 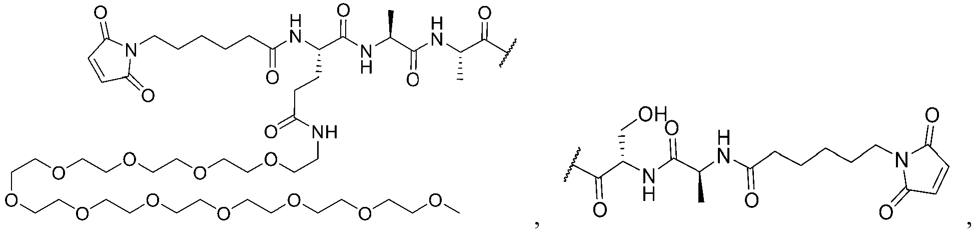 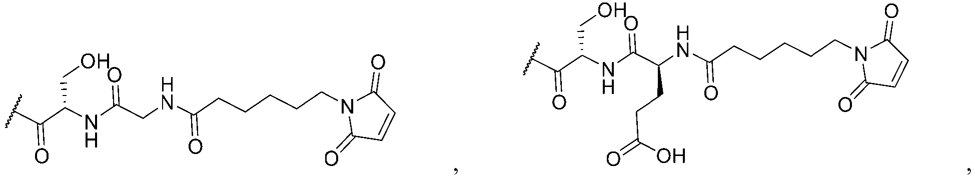 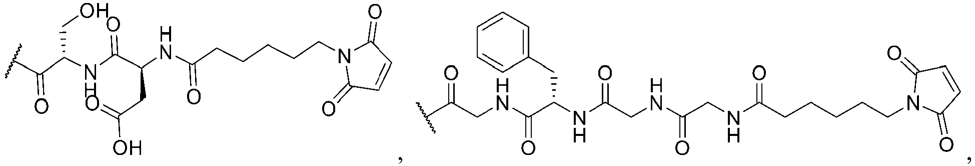 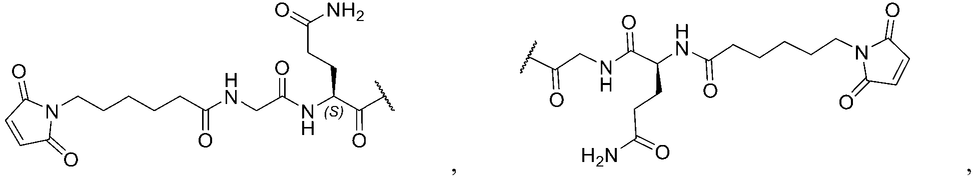 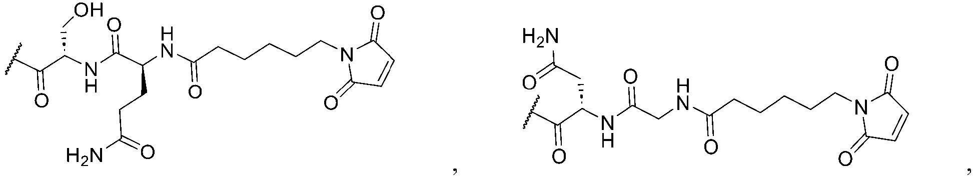 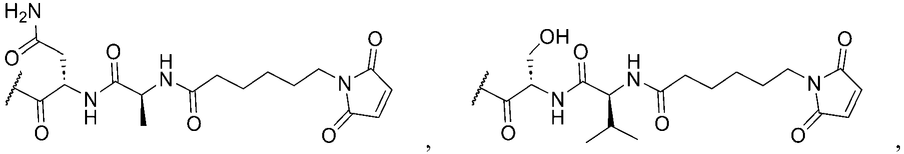 and

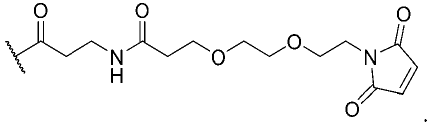

In certain embodiments, L^{1a}-L²-L³- is selected from

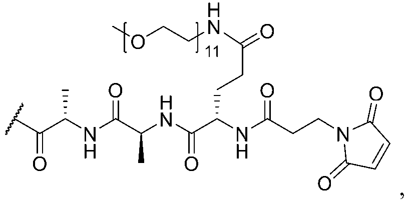

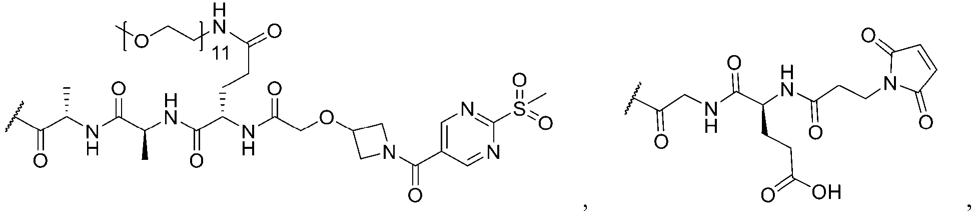

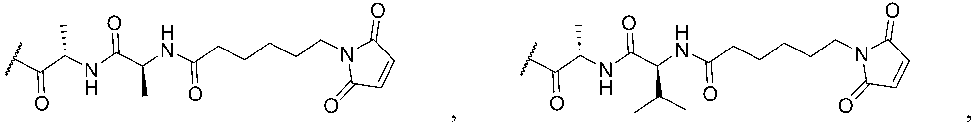

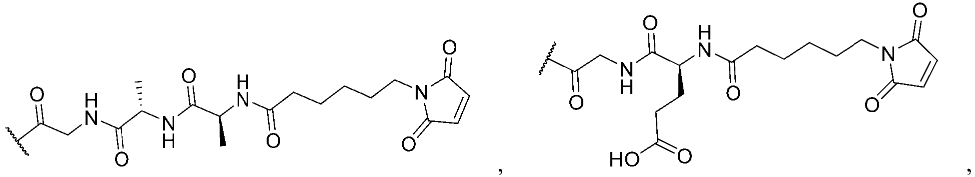

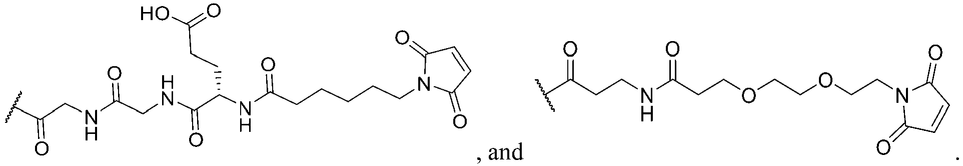

In certain embodiments, L^{1a}-L²-L³- is selected from 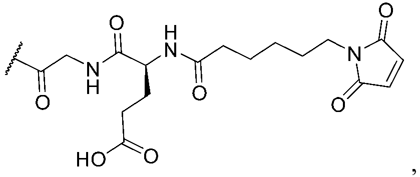 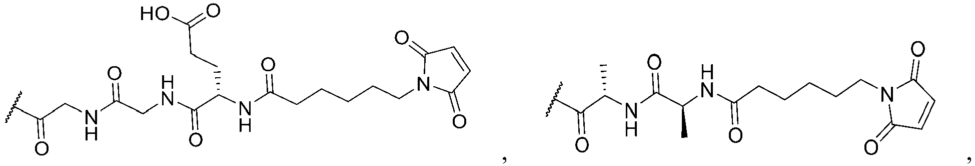 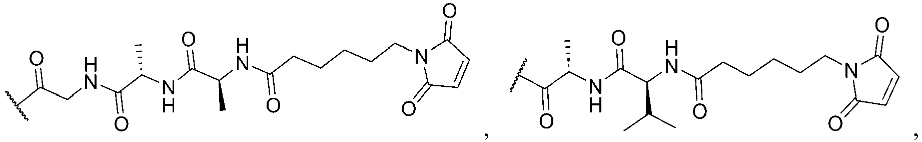 and

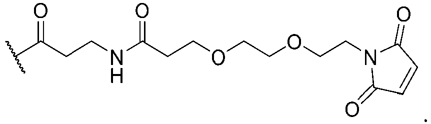

In certain embodiments, L^{1a}-L²-L³- is selected from

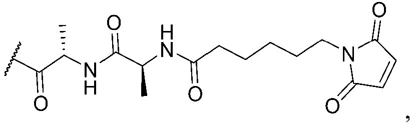

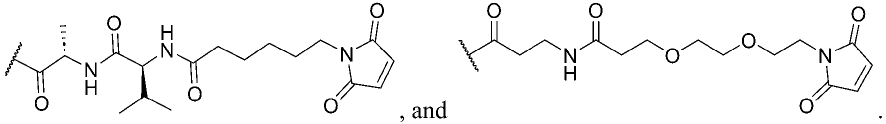

In certain embodiments, L^{1a}-L²-L³-Tr- is selected from

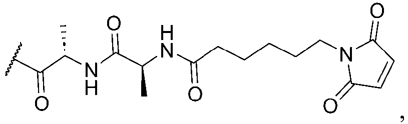

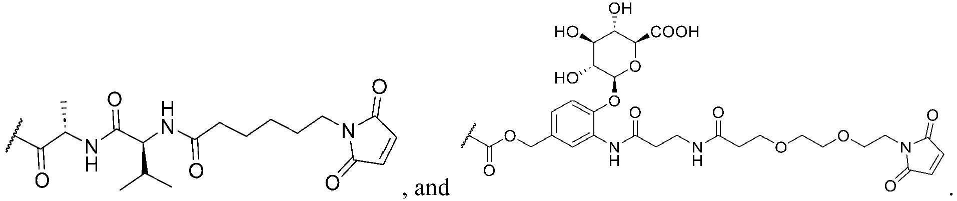

In certain embodiments, L^{1a}-L²-L³- is selected from 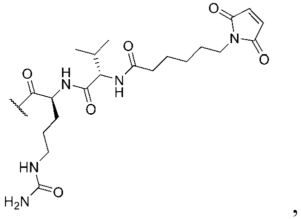 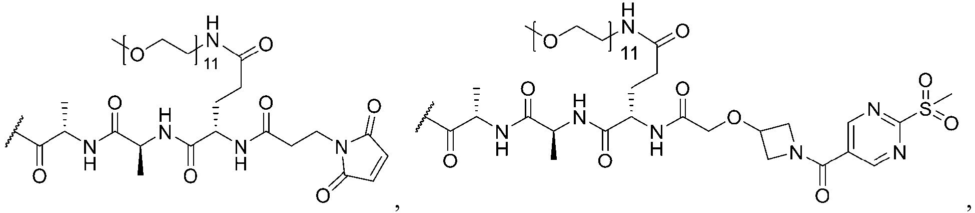 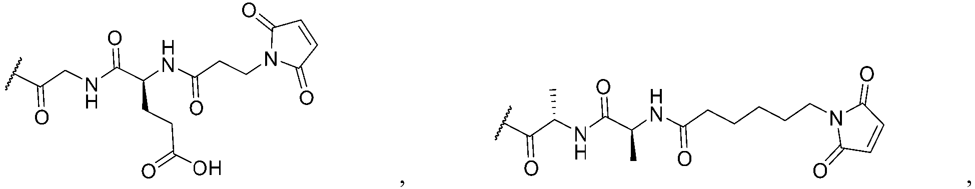 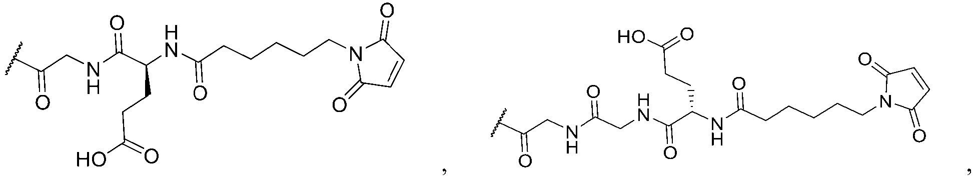 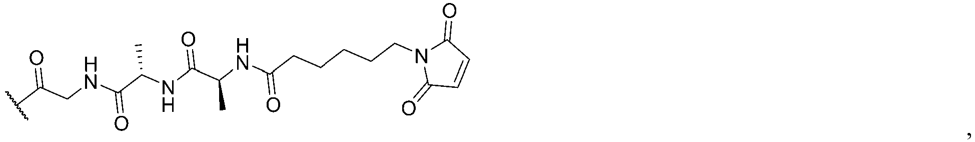 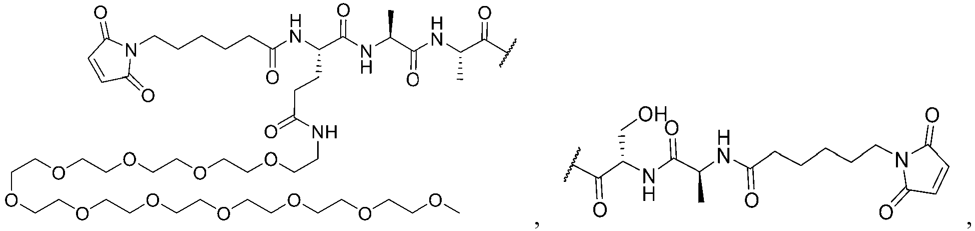 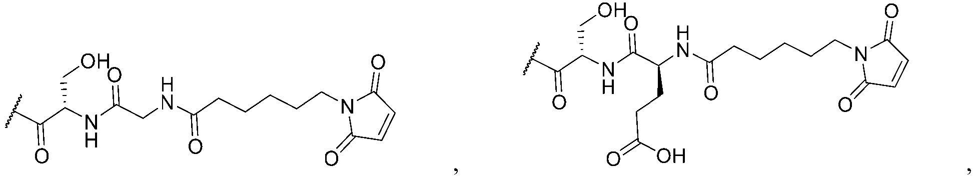 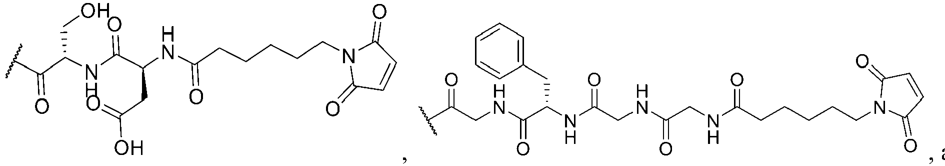 and

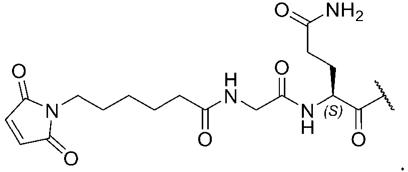

In certain embodiments, L^{1a}-L²-L³- is selected from

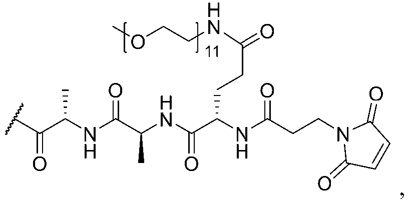

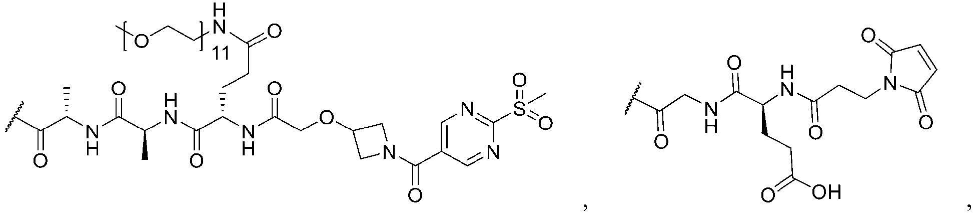

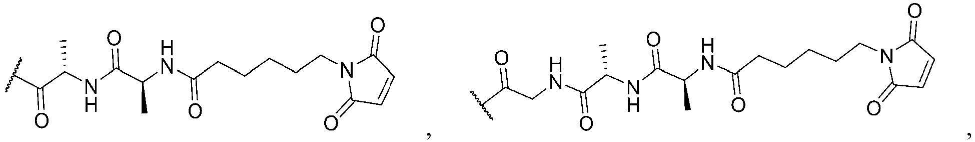

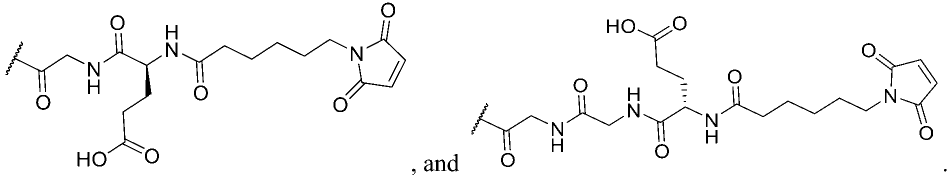

In certain embodiments, L^{1a}-L²-L³- is selected from 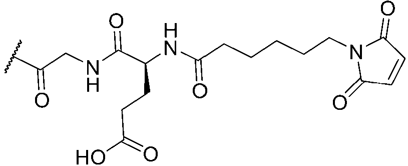 and

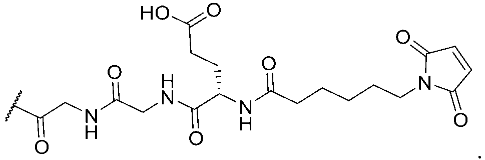

In certain embodiments, L^{1a}-L²-L³- is selected from 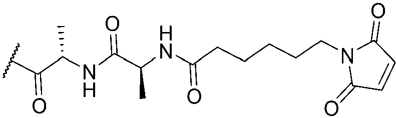 and

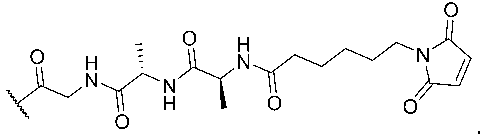

In certain embodiments, the compound is selected from a structure represented by formula I-D-3, III-D-3', III-D-3, IV-D-3, or V-D-3: 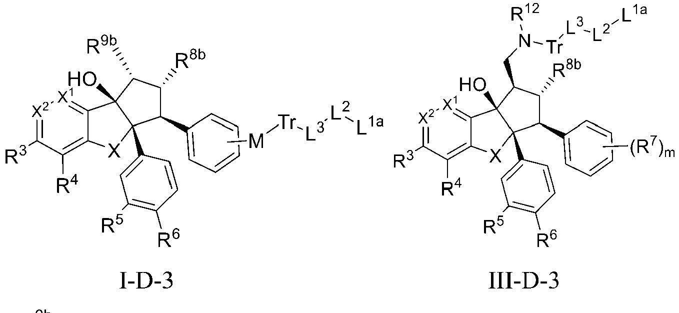 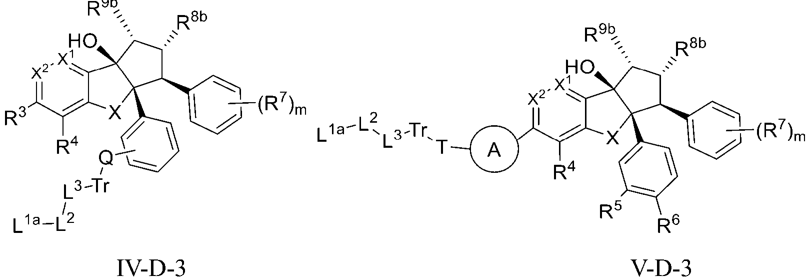 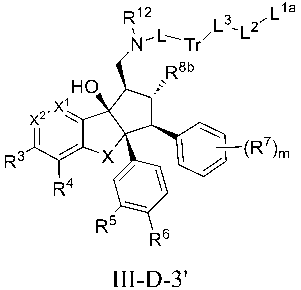 wherein X, X¹, X², ring A, R³, R⁴, R⁵, R⁶, R⁷, R^{8b}, R^{9b}, R¹², m, M, L, Q, T, Tr, L^{1a}, L², and L³ are defined as described in any one of the embodiments of the present disclosure.

In certain embodiments, the compound is selected from a structure represented by formula I-D-4, II-D-4', II-D-4, III-D-4', III-D-4, IV-D-4, or V-D-4: 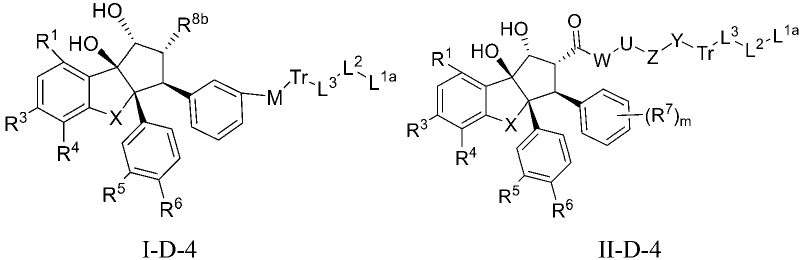 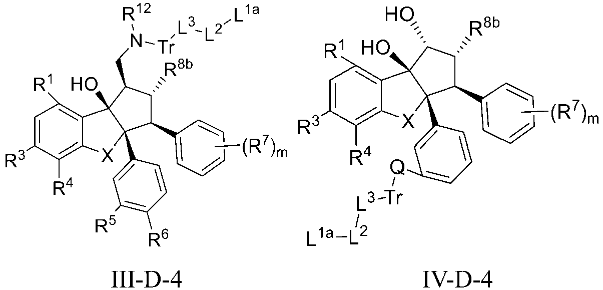 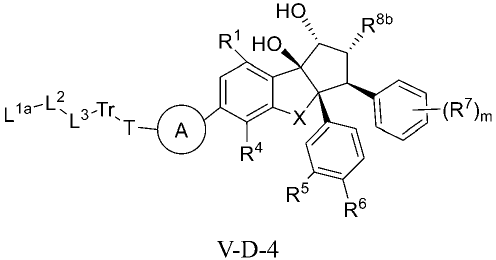 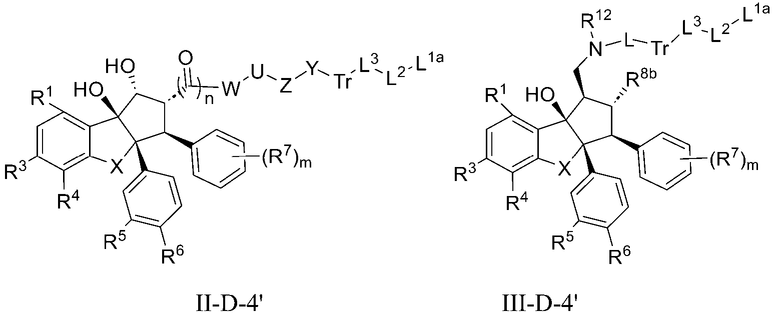 wherein X, R¹, ring A, R³, R⁴, R⁵, R⁶, R⁷, R^{8b}, R¹², m, n, M, W, U, Z, Y, Q, T, Tr, L, L^{1a}, L², and L³ are defined as described in any one of the embodiments of the present disclosure.

In certain embodiments, the compound is selected from a structure represented by formula I-D-5, II-D-5, III-D-5, IV-D-5, or V-D-5: 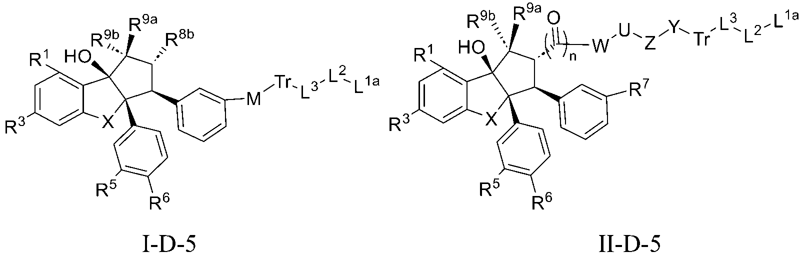 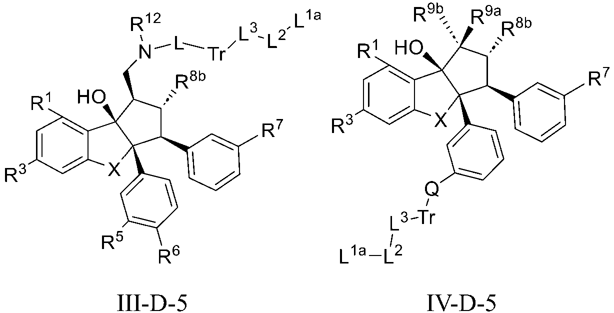 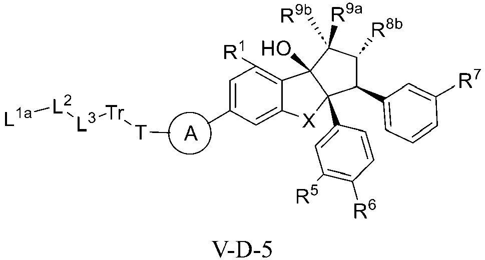 wherein X, R¹, ring A, R³, R⁵, R⁶, R⁷, R^{8b}, R^{9a}, R^{9b}, R¹², n, M, W, U, Z, Y, Q, T, Tr, L, L^{1a}, L², and L³ are defined as described in any one of the embodiments of the present disclosure.

In certain embodiments, the compound is selected from structures represented by formulas I-D-6 and I-D-7: 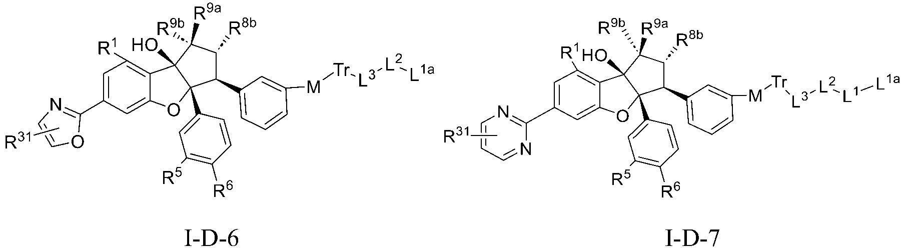 wherein R¹, R³, R⁵, R⁶, R^{8b}, R^{9a}, R^{9b}, M, Tr, L, L^{1a}, L², and L³ are defined as described in any one of the embodiments of the present disclosure.

In certain embodiments, the compound is selected from the structures shown in Table D:

**Table D**

| No. | Structure |
|---|---|
| I-D1 | 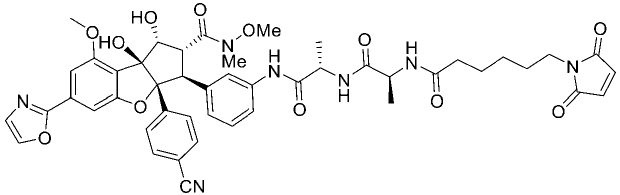 |
| I-D2 | 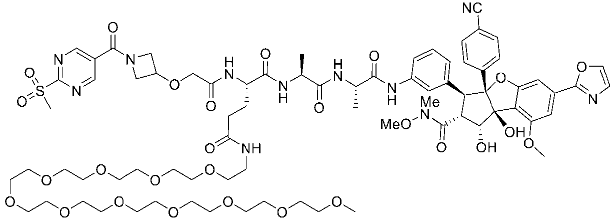 |
| I-D3 | 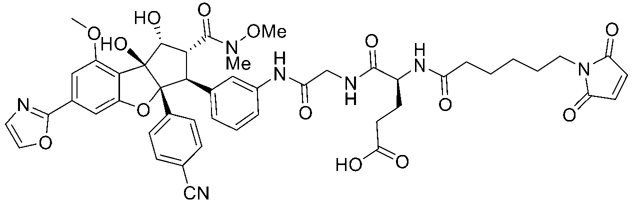 |
| I-D4 | 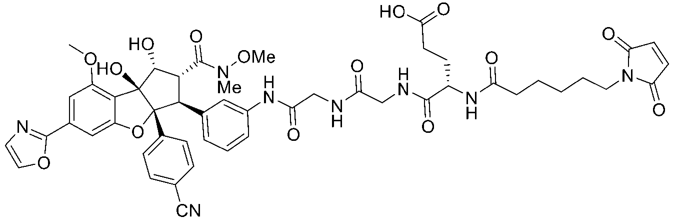 |
| I-D5 | 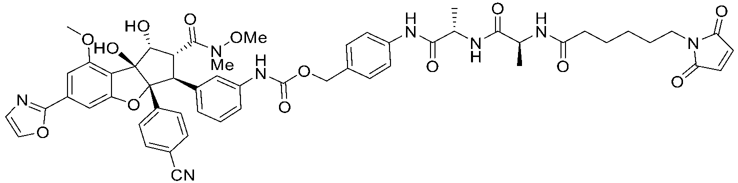 |
| I-D6 | 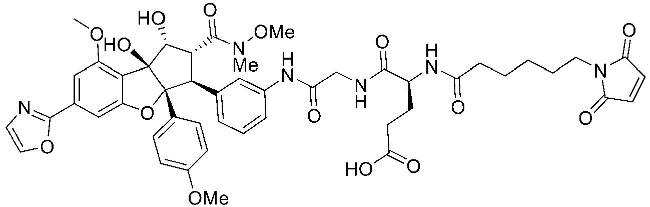 |
| I-D7 | 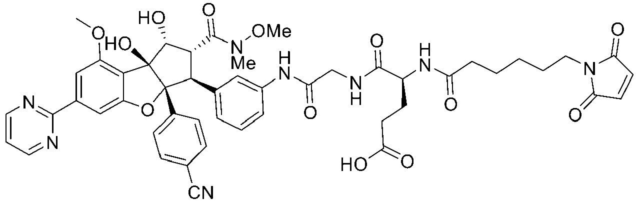 |
| I-D8 | 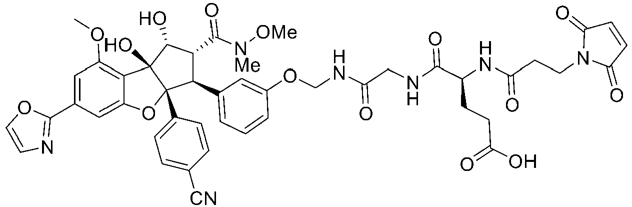 |
| I-D9 | 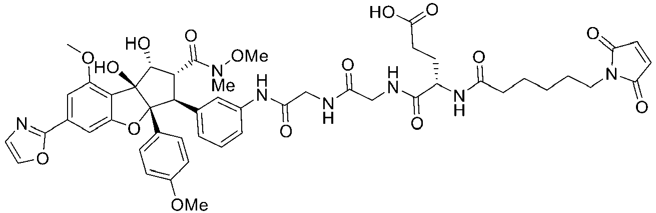 |
| I-D10 | 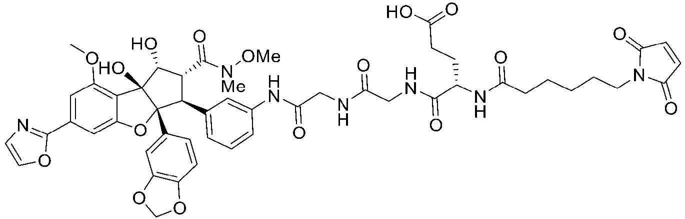 |
| I-D11 | 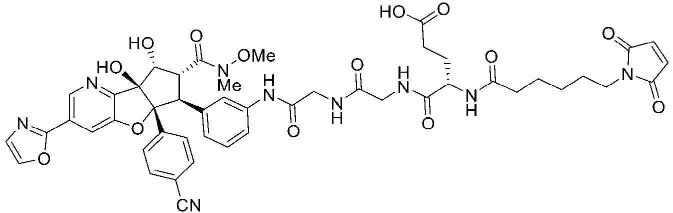 |
| I-D12 | 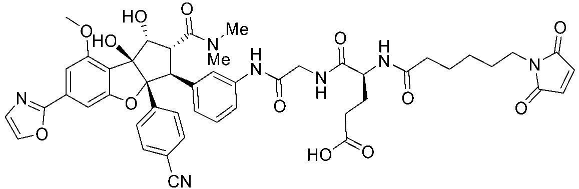 |
| I-D13 | 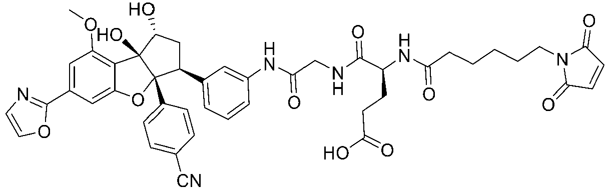 |
| I-D14 | 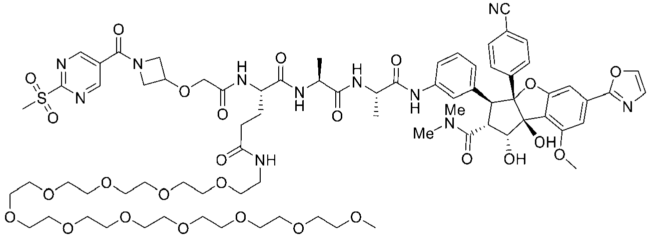 |
| I-D15 | 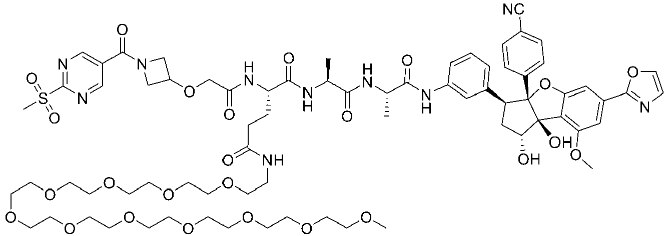 |
| I-D16 | 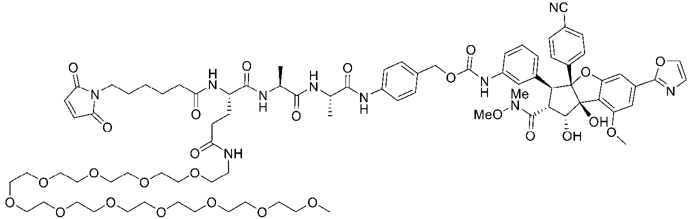 |
| I-D17 | 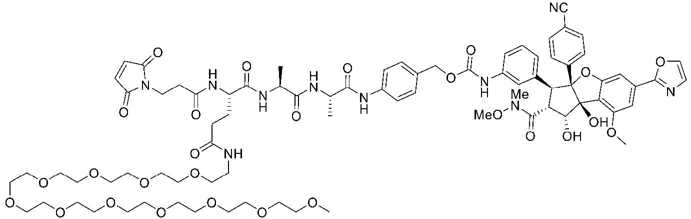 |
| I-D18 | 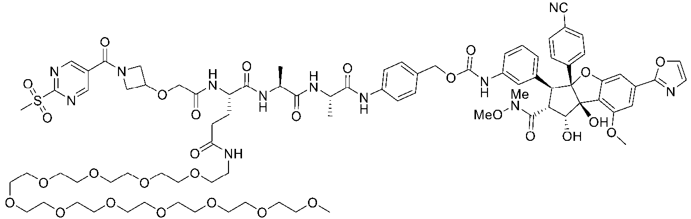 |
| I-D19 | 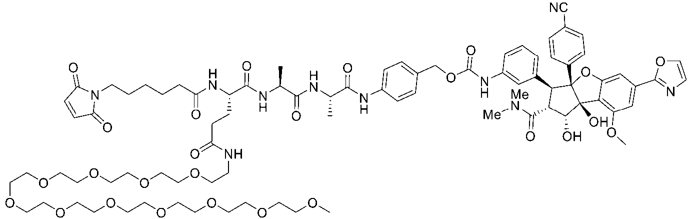 |
| I-D20 | 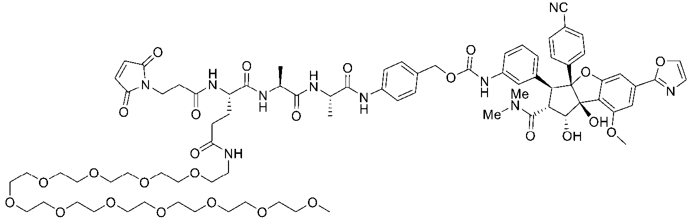 |
| I-D21 | 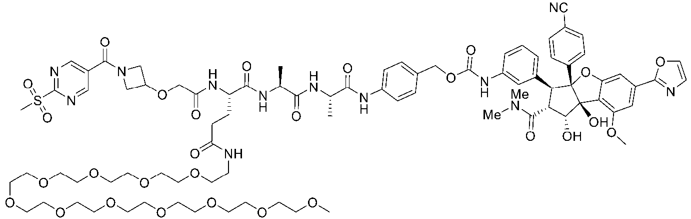 |
| I-D22 | 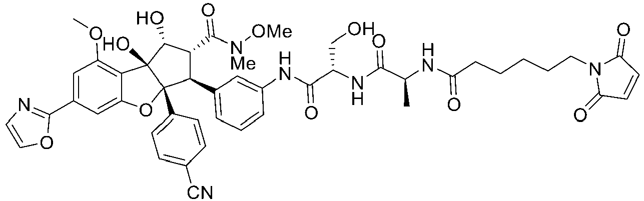 |
| I-D23 | 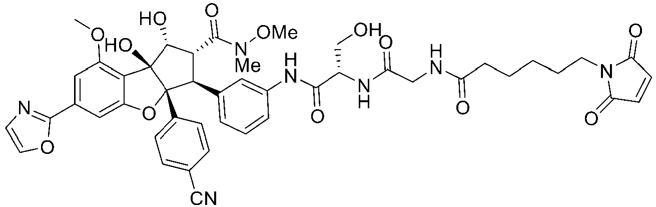 |
| I-D24 | 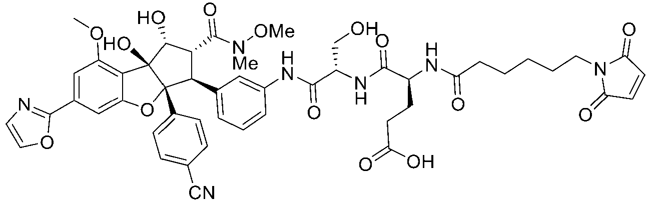 |
| I-D25 | 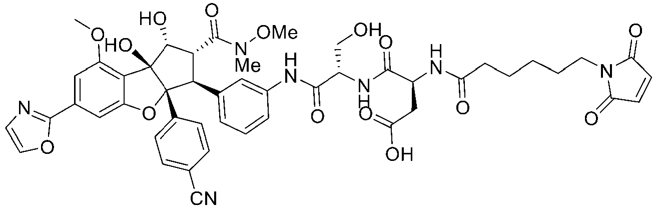 |
| I-D26 | 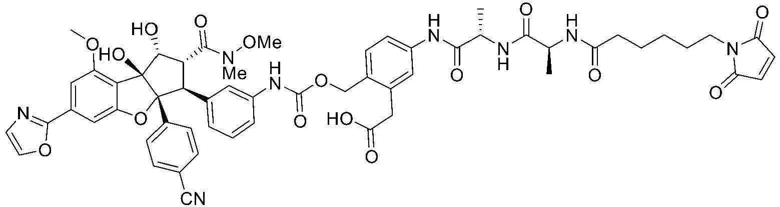 |
| I-D27 | 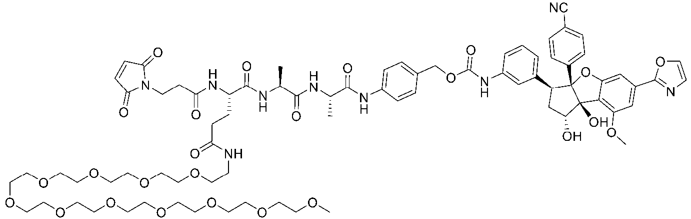 |
| I-D28 | 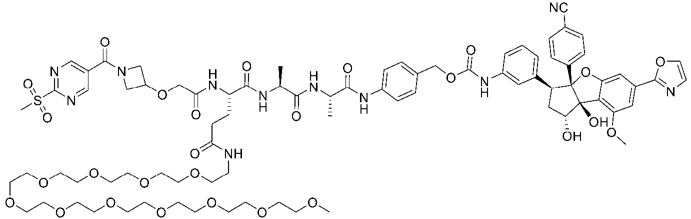 |
| I-D29 | 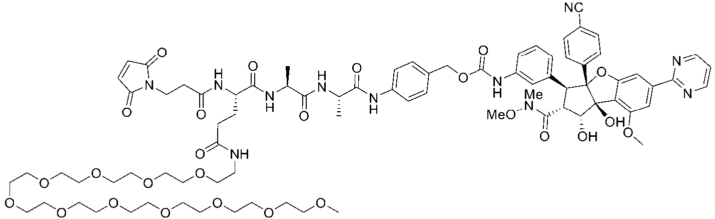 |
| I-D30 | 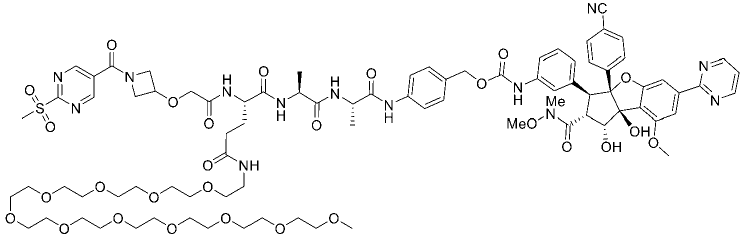 |
| I-D31 | 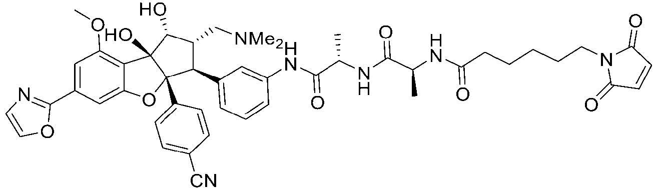 |
| I-D32 | 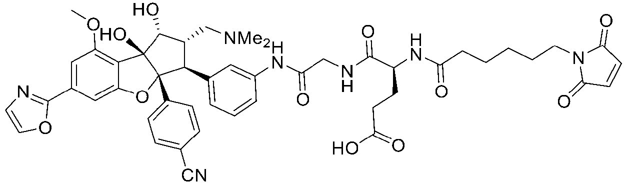 |
| I-D33 | 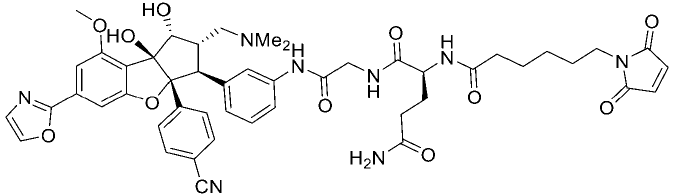 |
| I-D34 | 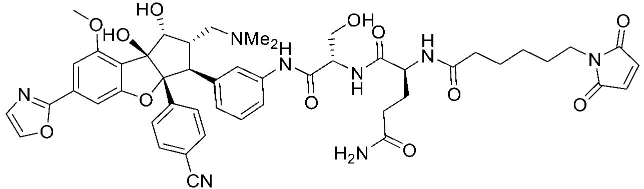 |
| I-D35 | 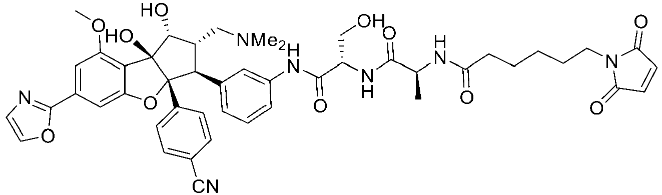 |
| I-D36 | 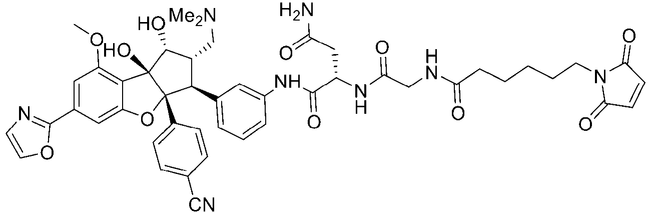 |
| I-D37 | 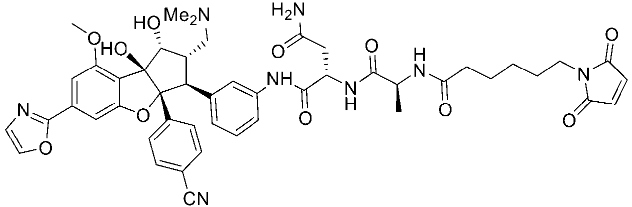 |
| I-D38 | 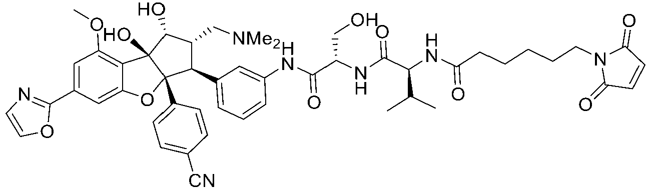 |
| I-D39 | 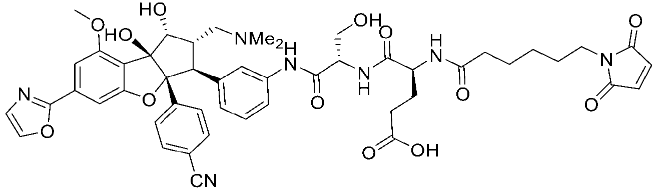 |
| I-D40 | 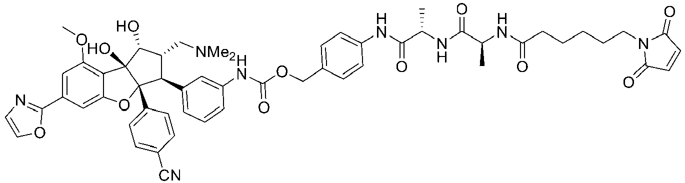 |
| I-D41 | 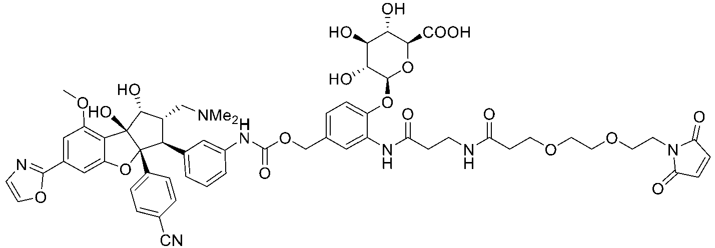 |
| I-D42 | 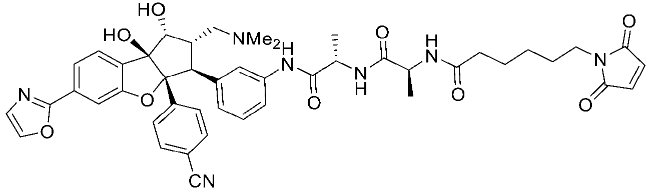 |
| I-D43 | 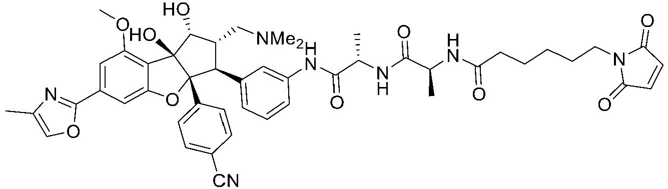 |
| I-D44 | 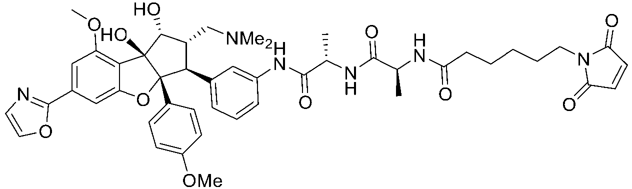 |
| I-D45 | 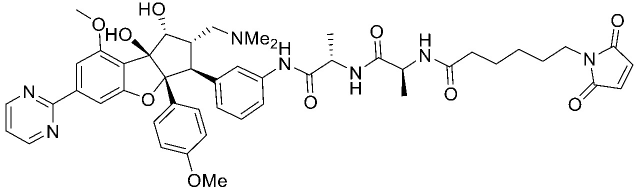 |
| I-D46 | 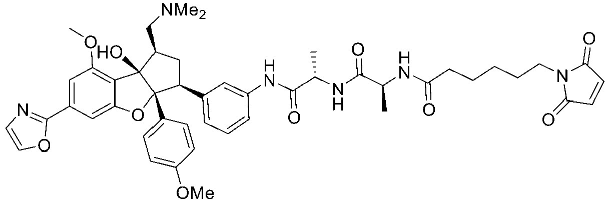 |
| I-D47 | 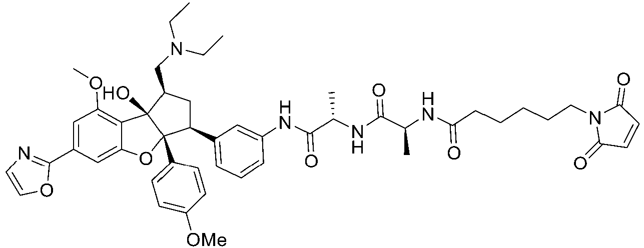 |
| I-D48 | 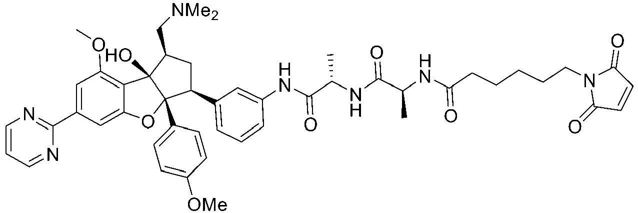 |
| I-D49 | 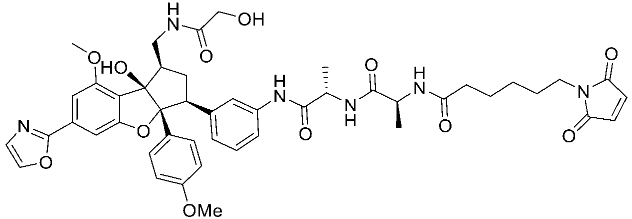 |
| I-D50 | 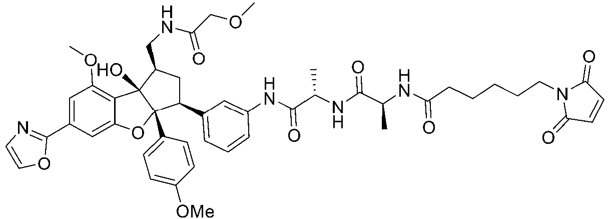 |
| I-D51 | 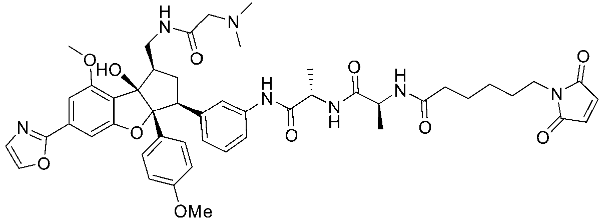 |
| I-D52 | 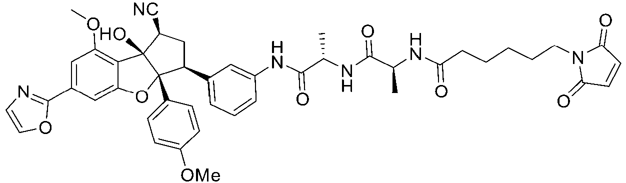 |
| I-D53 | 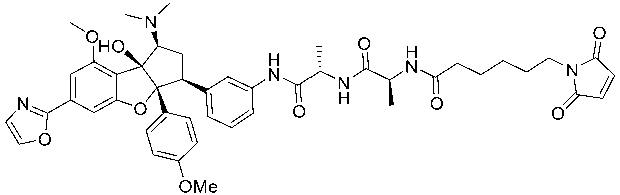 |
| I-D54 | 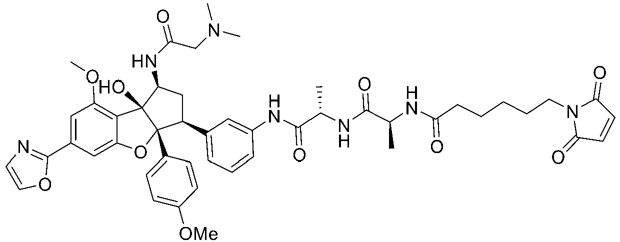 |
| I-D55 | 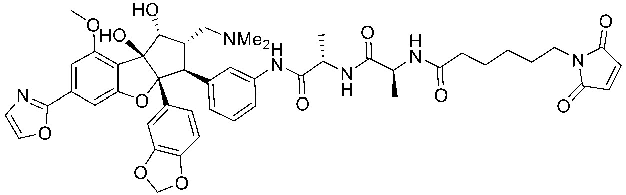 |
| I-D56 | 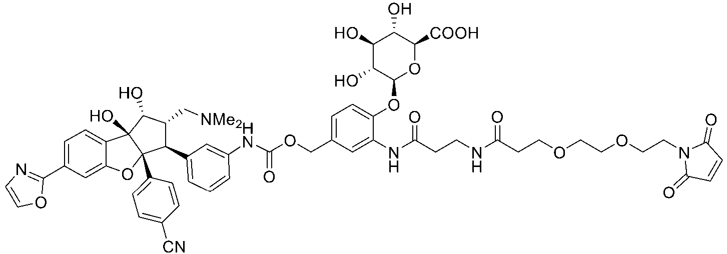 |
| I-D57 | 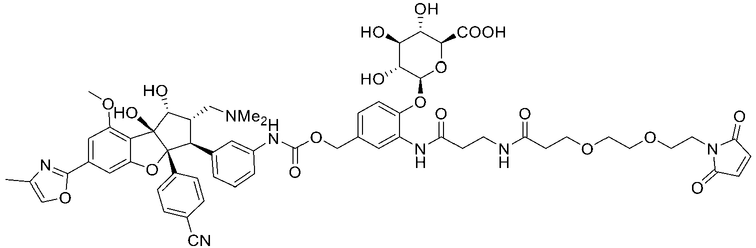 |
| I-D58 | 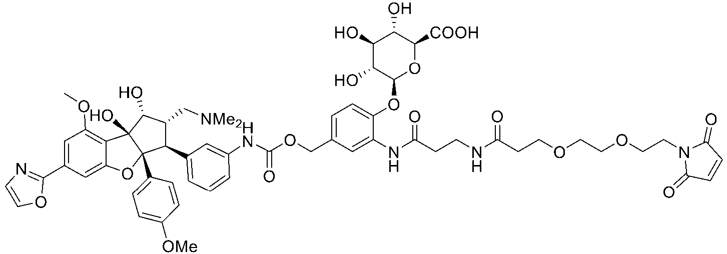 |
| I-D59 | 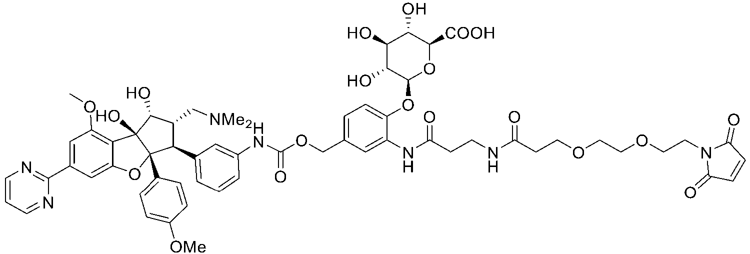 |
| I-D60 | 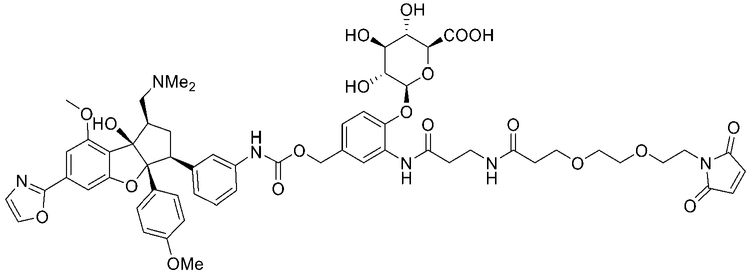 |
| I-D61 | 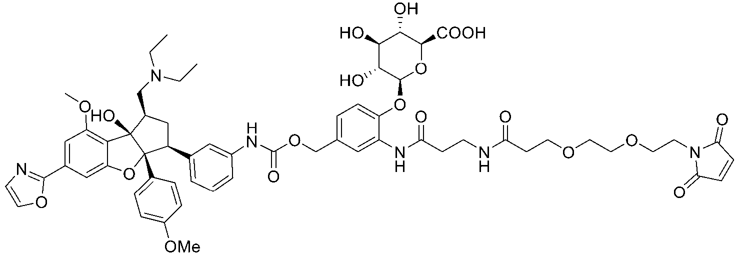 |
| I-D62 | 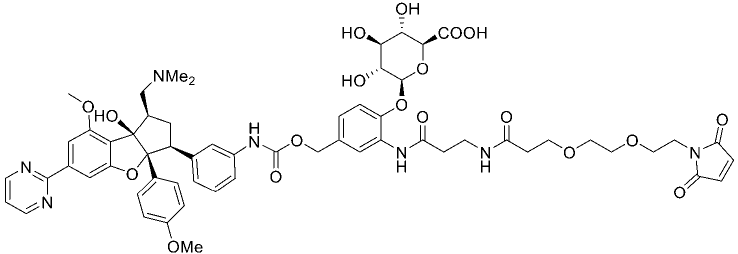 |
| I-D63 | 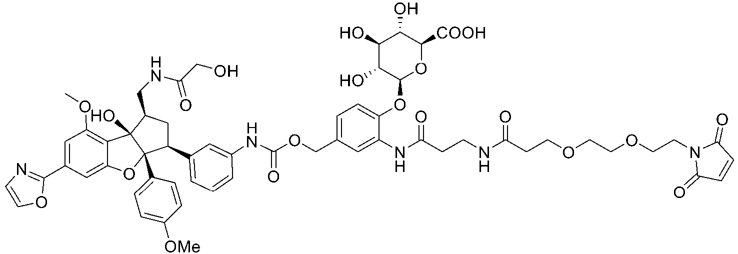 |
| I-D64 | 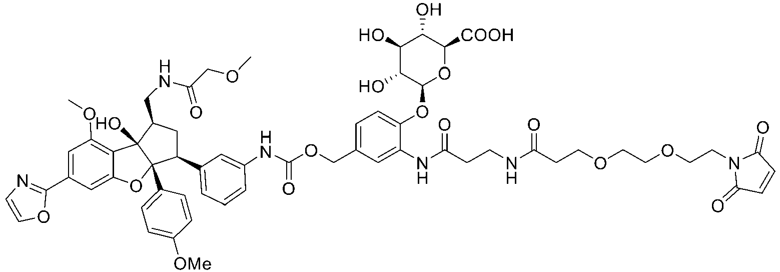 |
| I-D65 | 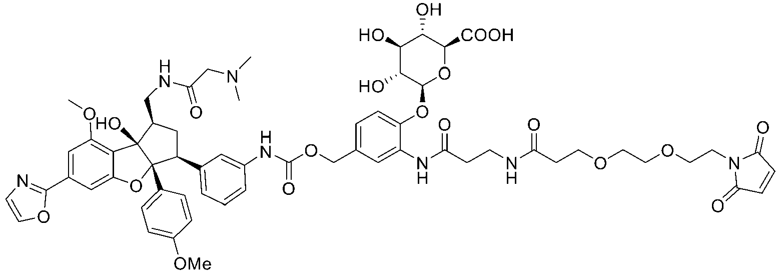 |
| I-D66 | 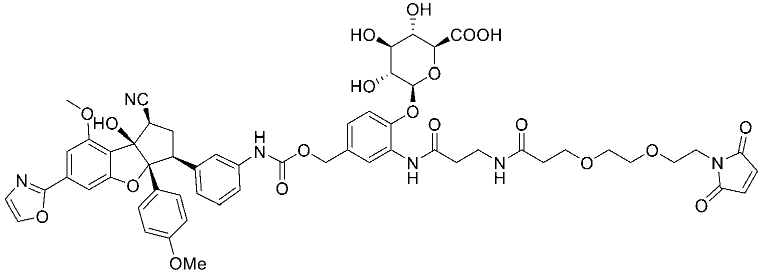 |
| I-D67 | 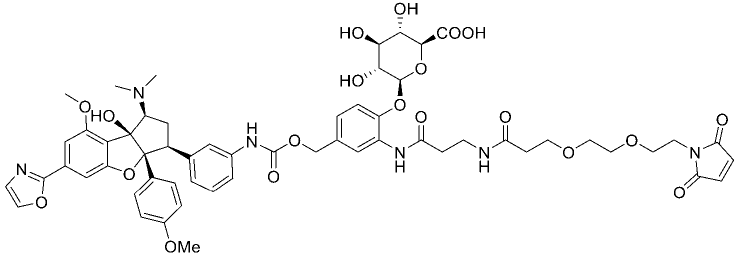 |
| I-D68 | 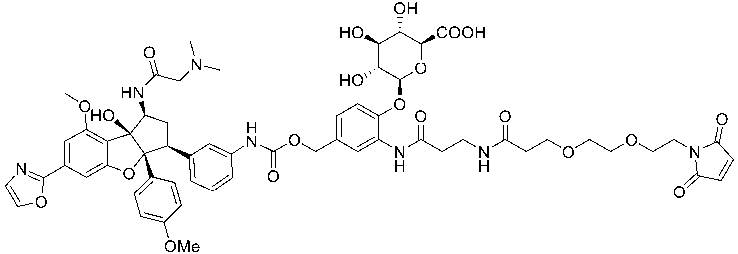 |
| I-D69 | 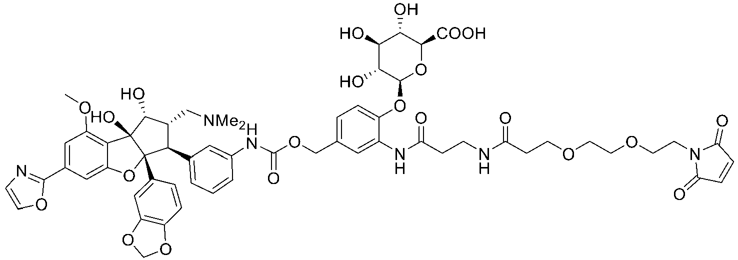 |
| I-D70 | 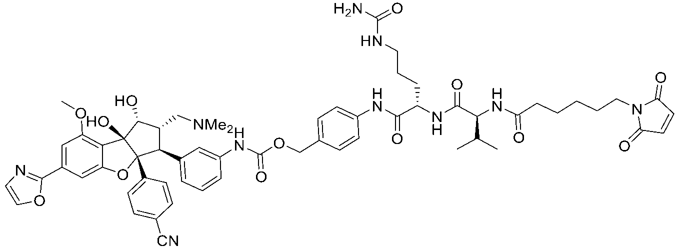 |
| I-D71 | 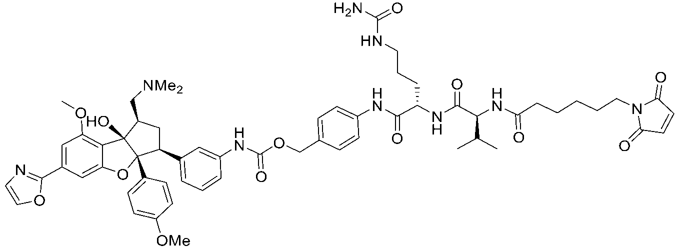 |
| I-D72 | 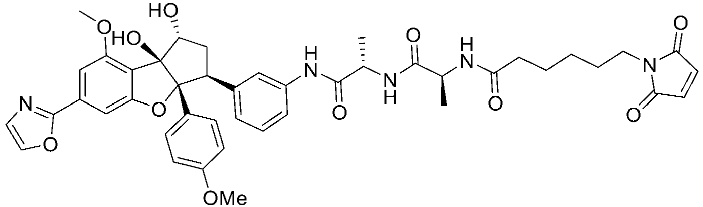 |
| I-D73 | 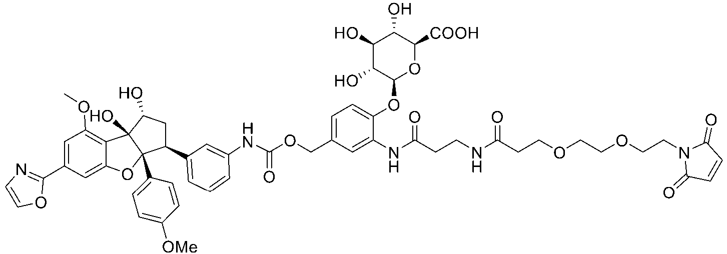 |
| I-D74 | 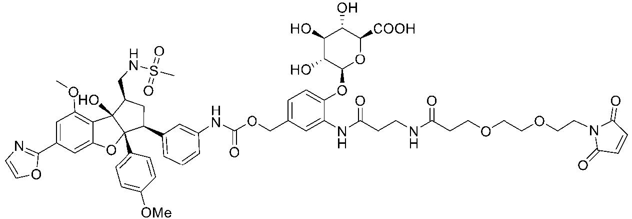 |
| II-D1 | 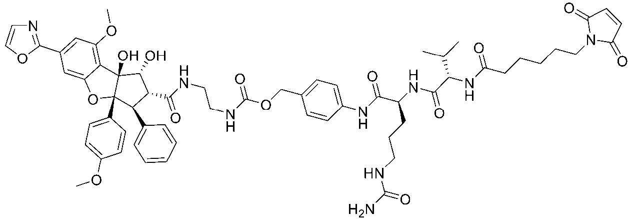 |
| II-D2 | 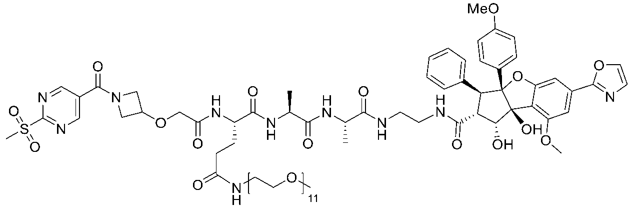 |
| II-D3 | 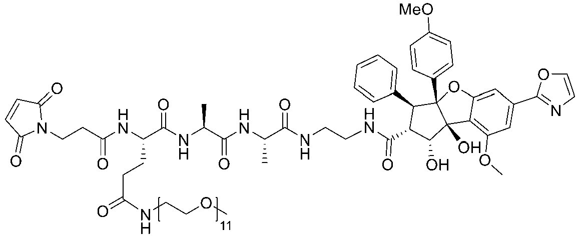 |
| II-D4 | 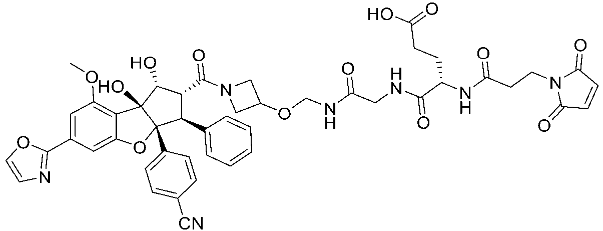 |
| II-D5 | 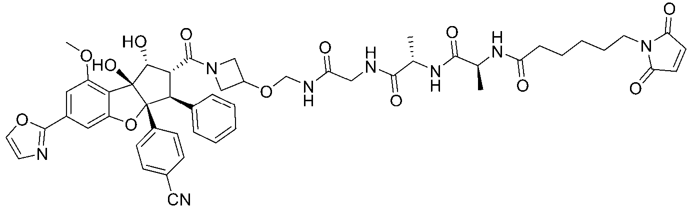 |
| II-D6 | 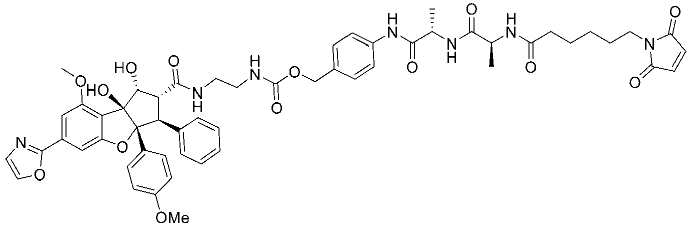 |
| II-D7 | 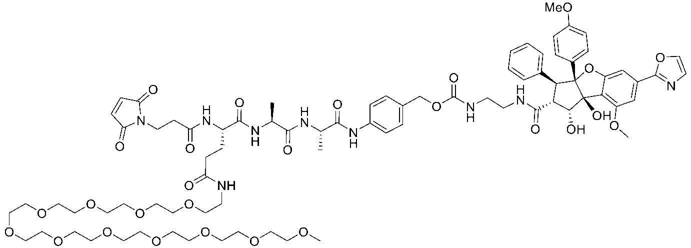 |
| II-D8 | 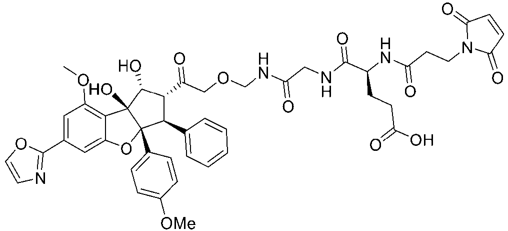 |
| II-D9 | 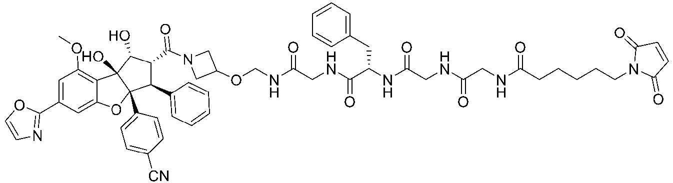 |
| II-D10 | 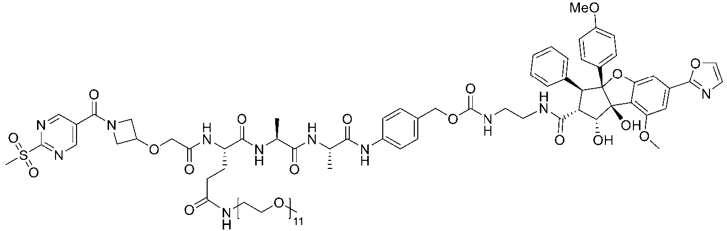 |
| II-D 11 | 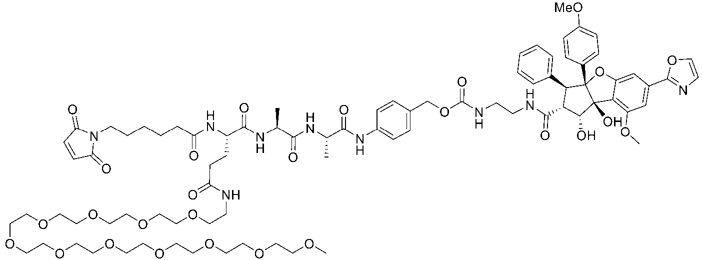 |
| II-D12 | 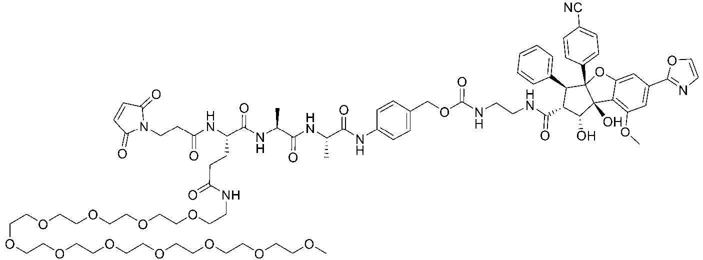 |
| II-D13 | 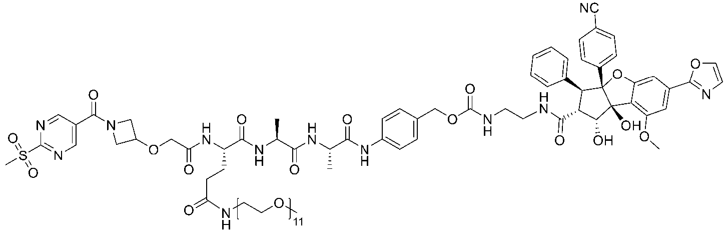 |
| II-D14 | 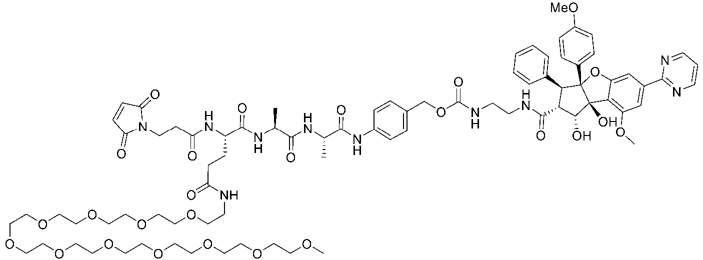 |
| II-D15 | 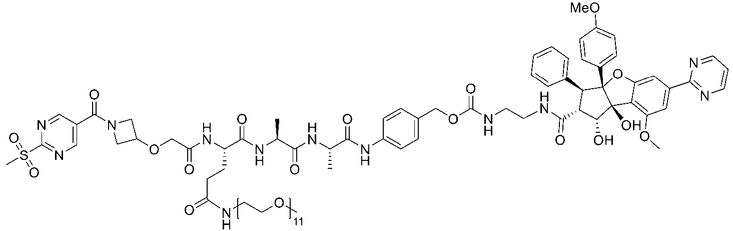 |
| II-D16 | 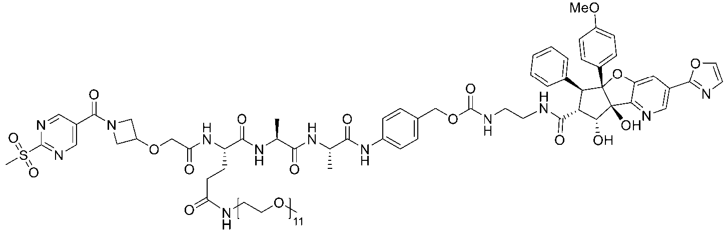 |
| II-D17 | 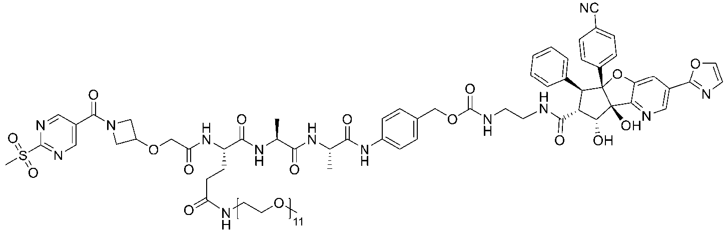 |
| II-D18 | 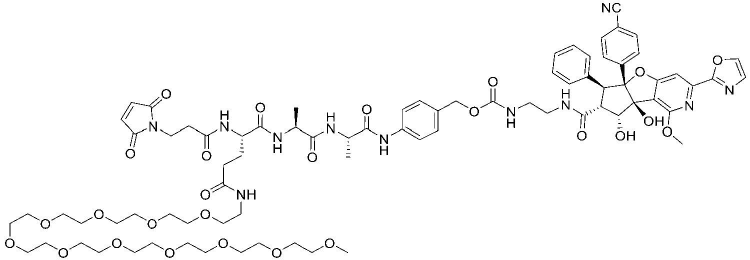 |
| II-D19 | 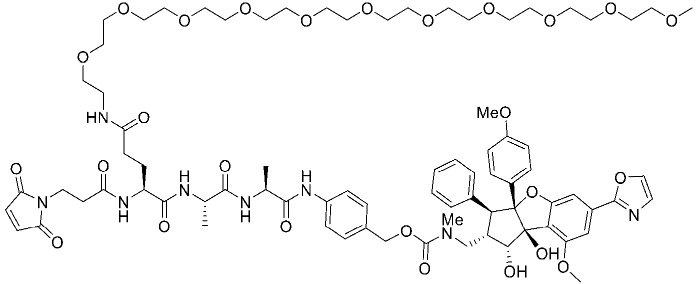 |
| II-D20 | 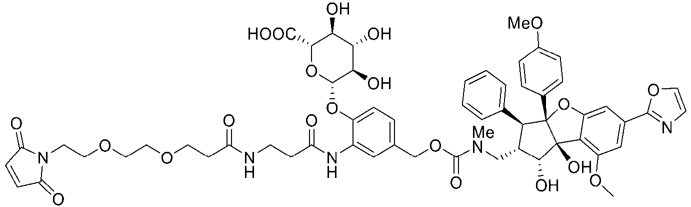 |
| II-D21 | 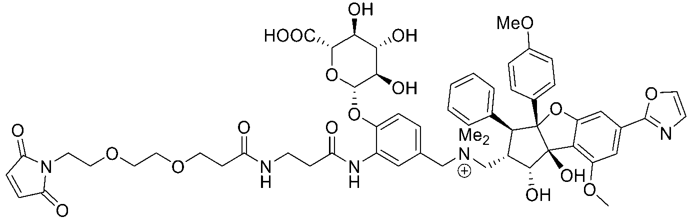 |
| III-D1 | 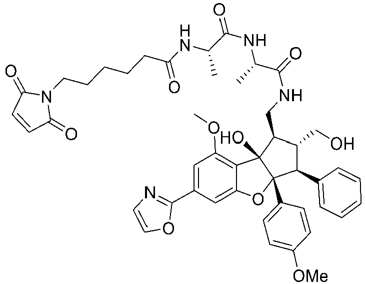 |
| III-D2 | 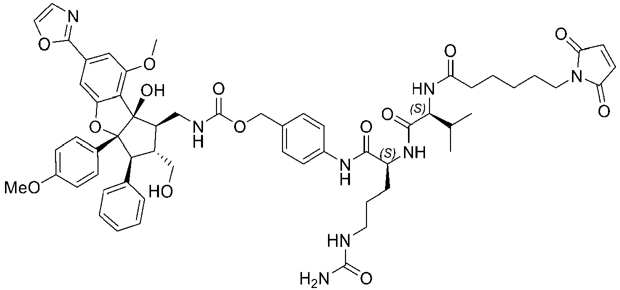 |
| III-D3 | 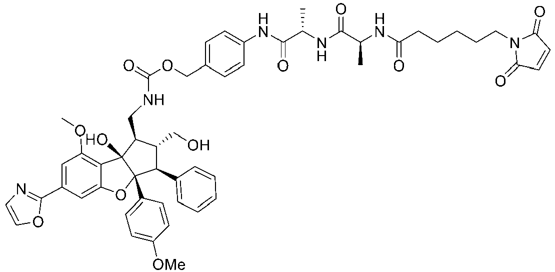 |
| III-D4 | 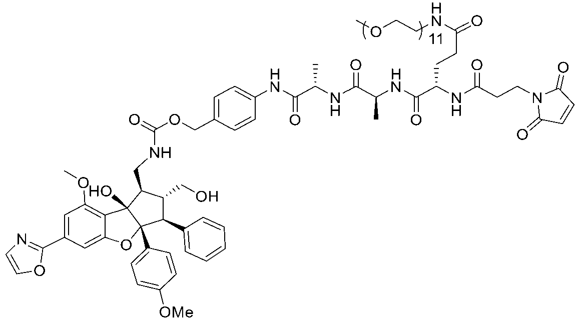 |
| III-D5 | 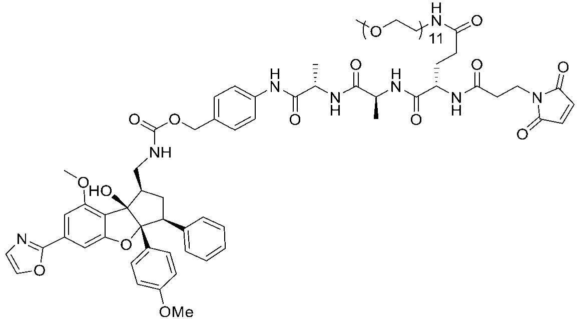 |
| III-D6 | 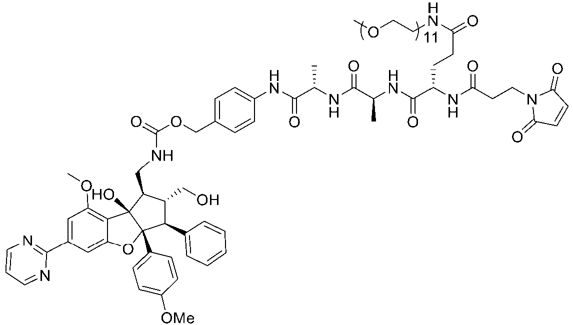 |
| III-D7 | 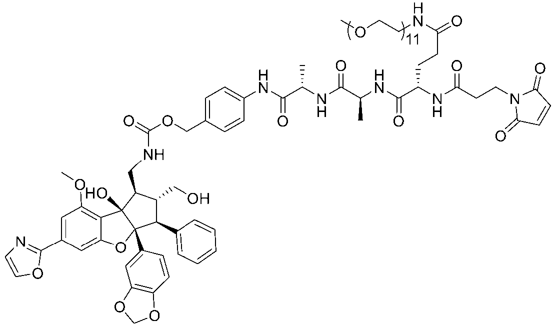 |
| III-D8 | 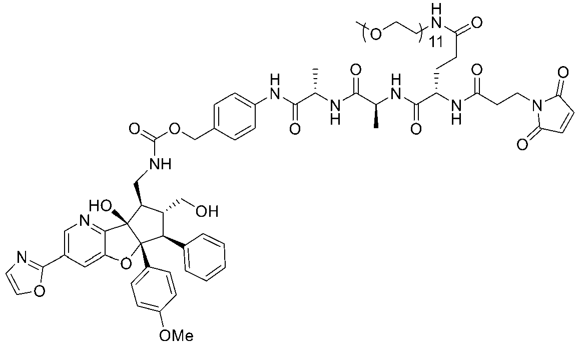 |
| III-D9 | 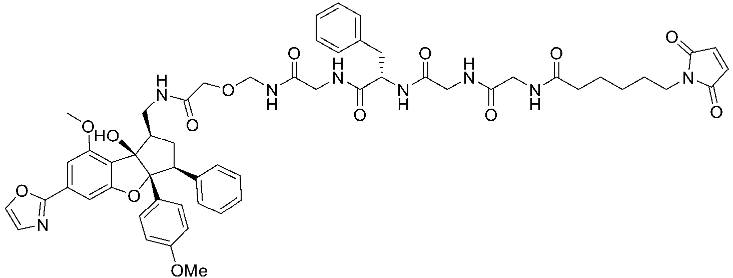 |
| III-D10 | 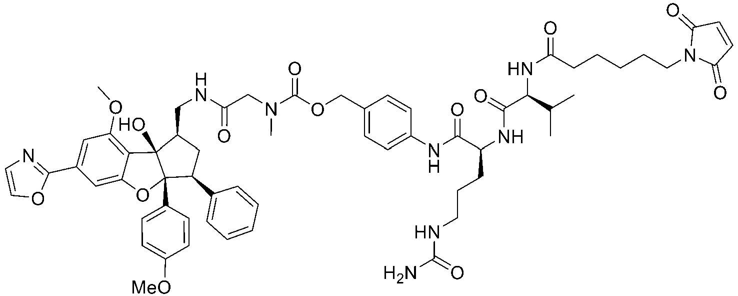 |
| III-D11 | 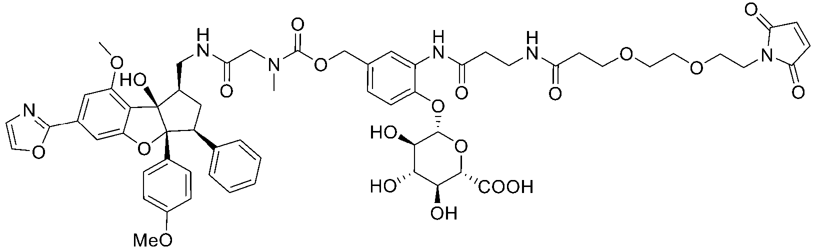 |
| III-D12 | 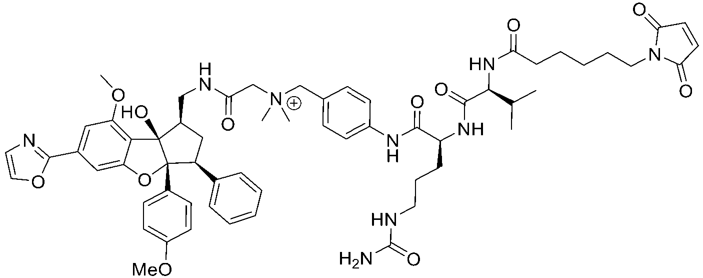 |
| III-D13 | 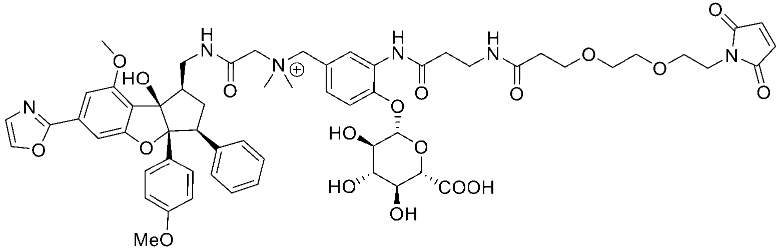 |
| IV-D1 | 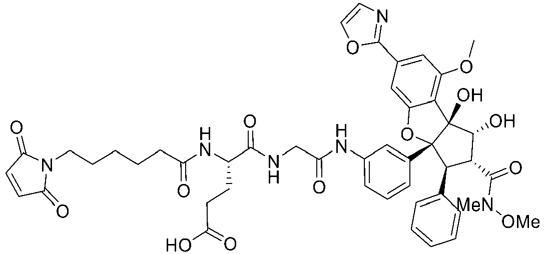 |
| IV-D2 | 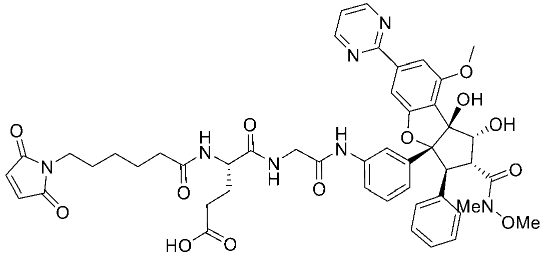 |
| IV-D3 | 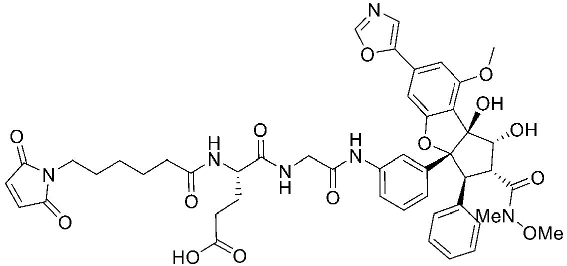 |
| IV-D4 | 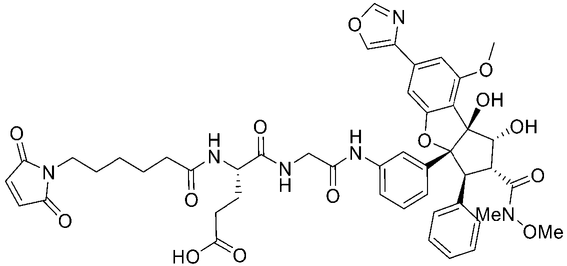 |
| IV-D5 | 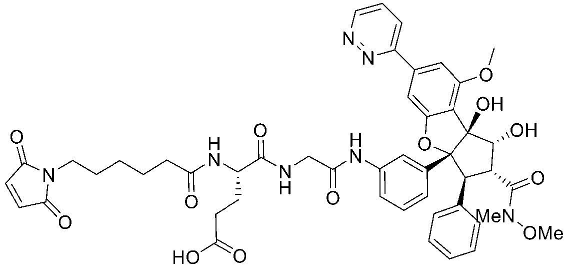 |
| IV-D6 | 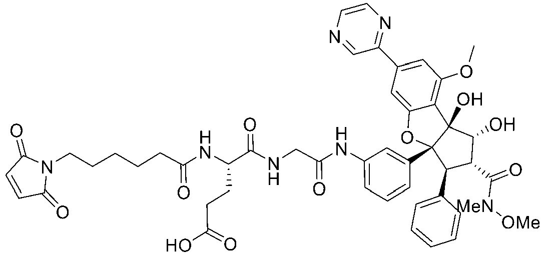 |
| IV-D7 | 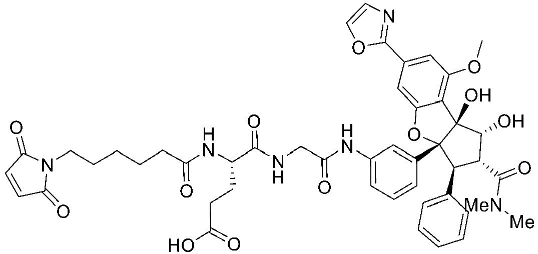 |
| IV-D8 | 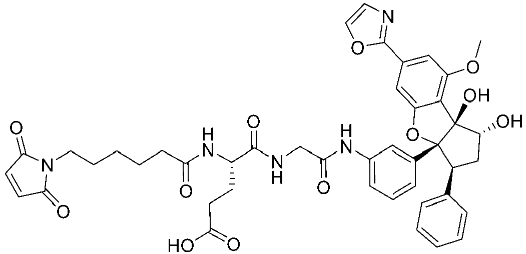 |
| IV-D9 | 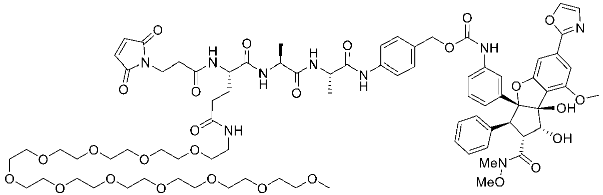 |
| IV-D10 | 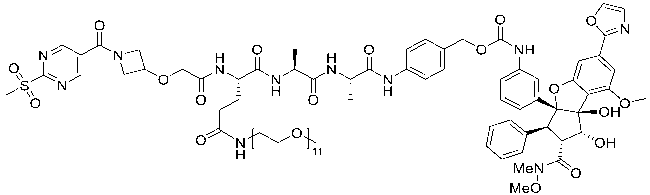 |
| IV-D11 | 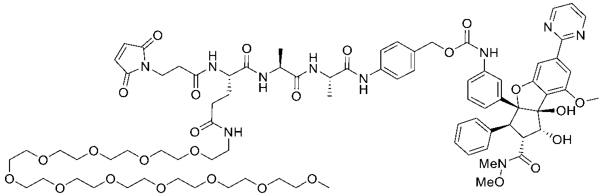 |
| IV-D12 | 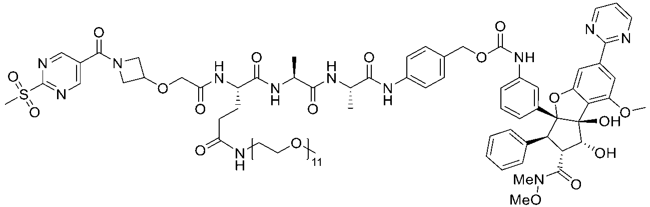 |
| IV-D13 | 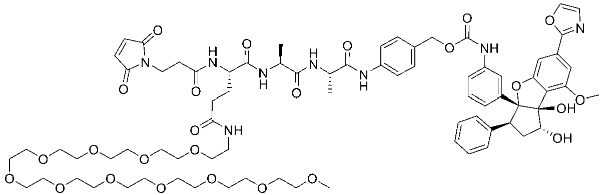 |
| IV-D14 | 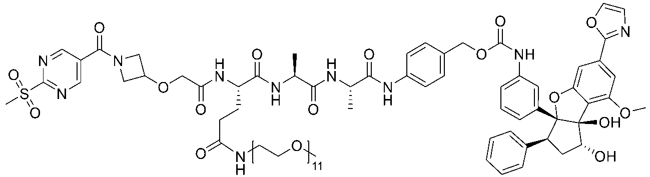 |
| IV-D15 | 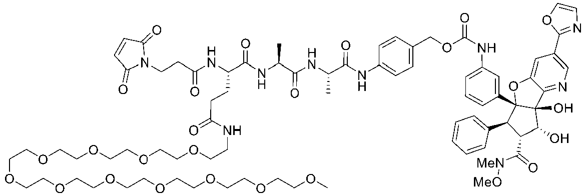 |
| IV-D16 | 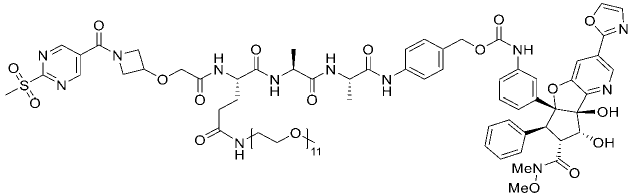 |
| IV-D17 | 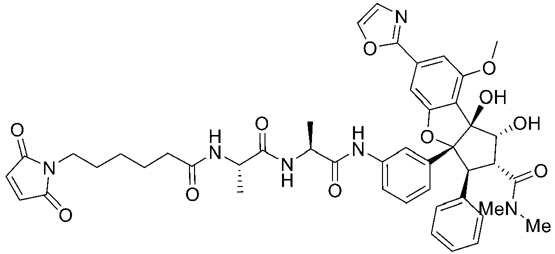 |
| IV-D18 | 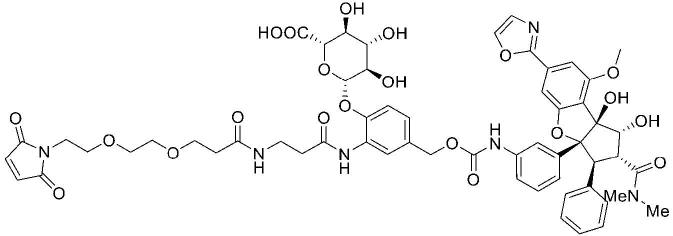 |
| IV-D19 | 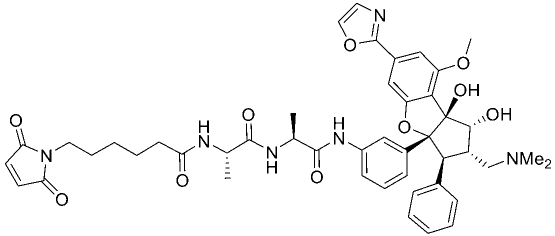 |
| IV-D20 | 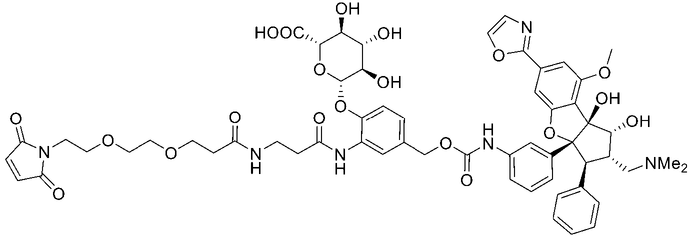 |
| V-D1 | 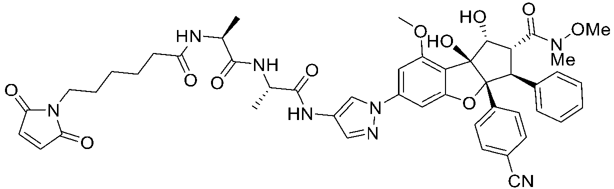 |
| V-D2 | 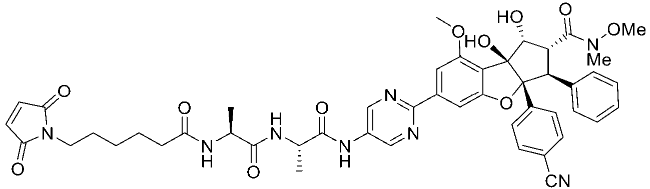 |
| V-D3 | 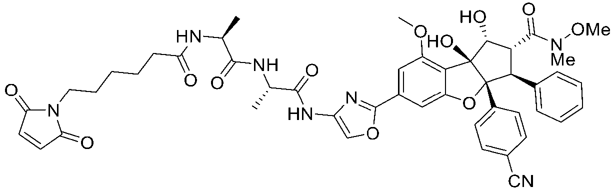 |
| V-D4 | 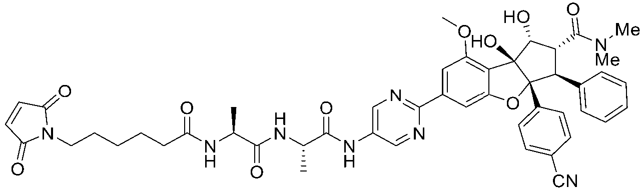 |
| V-D5 | 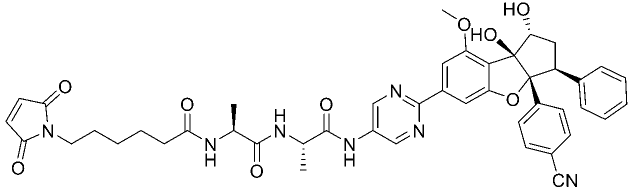 |
| V-D6 | 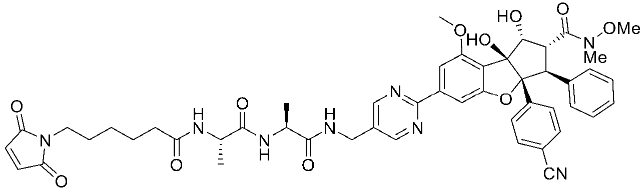 |
| V-D7 | 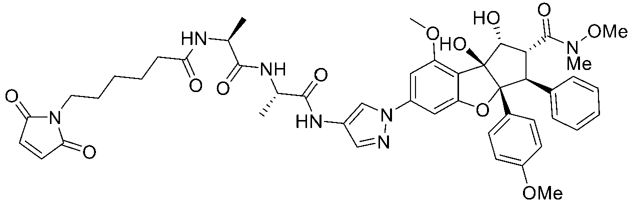 |
| V-D8 | 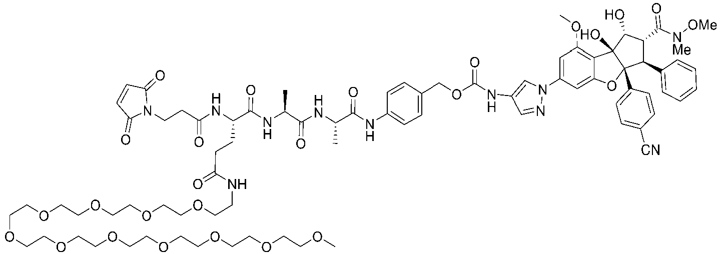 |
| V-D9 | 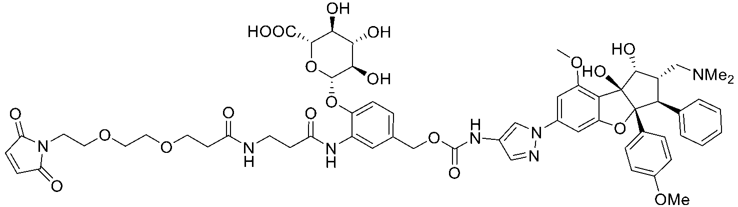 |
| V-D10 | 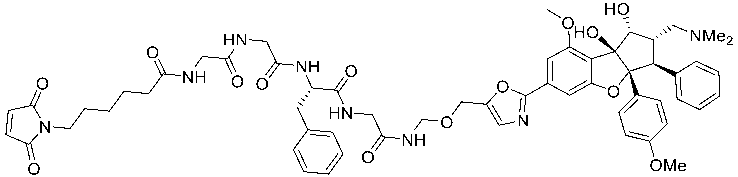 |
| V-D11 | 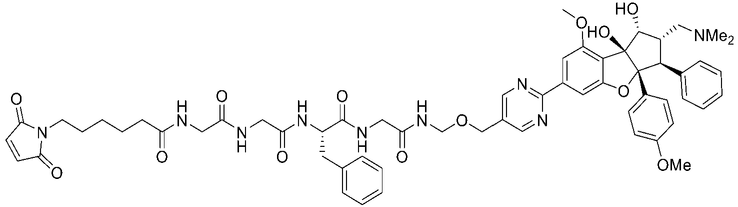 |
| V-D12 | 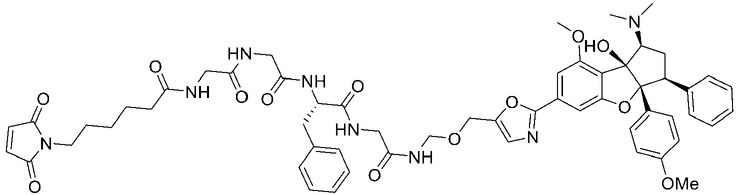 |
| V-D13 | 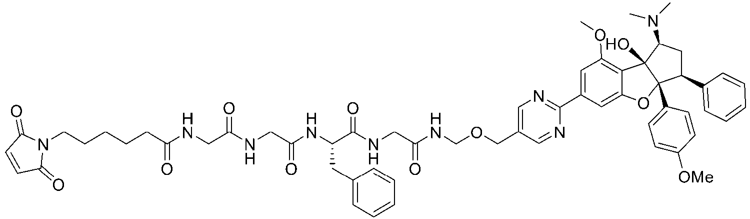 |

A fourth aspect of the present disclosure provides use of the compound or the racemic mixture, tautomer, stereoisomer, enantiomer, or diastereoisomer thereof, or the mixture thereof, or the pharmaceutically acceptable salt or solvate thereof according to any one of the embodiments of the third aspect of the present disclosure in the manufacture of the ligand-drug conjugate according to any one of the embodiments of the first aspect of the present disclosure.

### Pharmaceutical Composition

A fifth aspect of the present disclosure provides a pharmaceutical composition comprising at least one of the ligand-drug conjugate or the racemic mixture, tautomer, stereoisomer, enantiomer, or diastereoisomer thereof, or the mixture thereof, or the pharmaceutically acceptable salt or solvate thereof according to any one of the embodiments of the first aspect of the present disclosure, or at least one of the compound or the racemic mixture, tautomer, stereoisomer, enantiomer, or diastereoisomer thereof, or the mixture thereof, or the pharmaceutically acceptable salt or solvate thereof according to any one of the embodiments of the third aspect of the present disclosure, and one or more pharmaceutically acceptable carriers and/or excipients.

A sixth aspect of the present disclosure provides a pharmaceutical composition comprising at least one of the mixture of ligand-drug conjugates according to any one of the embodiments of the second aspect of the present disclosure, and one or more pharmaceutically acceptable carriers and/or excipients.

### Use

A seventh aspect of the present disclosure provides use of the ligand-drug conjugate or the racemic mixture, tautomer, stereoisomer, enantiomer, or diastereoisomer thereof, or the mixture thereof, or the pharmaceutically acceptable salt or solvate thereof according to any one of the embodiments of the first aspect of the present disclosure, or the compound or the racemic mixture, tautomer, stereoisomer, enantiomer, or diastereoisomer thereof or the mixture thereof according to any one of the embodiments of the third aspect of the present disclosure, or the pharmaceutical composition according to any one of the embodiments of the fifth aspect of the present disclosure in the manufacture of a medicament for treating and/or preventing a disease or disorder or reducing the severity of the disease or disorder, wherein the disease or disorder is a tumor or cancer.

The seventh aspect of the present disclosure further provides use of the mixture of ligand-drug conjugates according to any one of the embodiments of the second aspect of the present disclosure, or the pharmaceutical composition according to any one of the embodiments of the sixth aspect of the present disclosure in the manufacture of a medicament for treating and/or preventing a disease or disorder or reducing the severity of the disease or disorder, wherein the disease or disorder is a tumor or cancer.

The seventh aspect of the present disclosure further provides the ligand-drug conjugate or the racemic mixture, tautomer, stereoisomer, enantiomer, or diastereoisomer thereof, or the mixture thereof, or the pharmaceutically acceptable salt or solvate thereof according to any one of the embodiments of the first aspect of the present disclosure, or the compound or the racemic mixture, tautomer, stereoisomer, enantiomer, or diastereoisomer thereof or the mixture thereof according to any one of the embodiments of the third aspect of the present disclosure, or the pharmaceutical composition according to any one of the embodiments of the fifth aspect of the present disclosure for use in treating and/or preventing a disease or disorder or reducing the severity of the disease or disorder, wherein the disease or disorder is a tumor or cancer.

The seventh aspect of the present disclosure further provides the mixture of ligand-drug conjugates according to any one of the embodiments of the second aspect of the present disclosure, or the pharmaceutical composition according to any one of the embodiments of the sixth aspect of the present disclosure for use in treating and/or preventing a disease or disorder or reducing the severity of the disease or disorder, wherein the disease or disorder is a tumor or cancer.

The seventh aspect of the present disclosure further provides a method for treating and/or preventing a disease or disorder or reducing the severity of the disease or disorder, comprising administering to an individual in need thereof an effective amount of the ligand-drug conjugate or the racemic mixture, tautomer, stereoisomer, enantiomer, or diastereoisomer thereof, or the mixture thereof, or the pharmaceutically acceptable salt or solvate thereof according to any one of the embodiments of the first aspect of the present disclosure, or the compound or the racemic mixture, tautomer, stereoisomer, enantiomer, or diastereoisomer thereof or the mixture thereof according to any one of the embodiments of the third aspect of the present disclosure, or the pharmaceutical composition according to any one of the embodiments of the fifth aspect of the present disclosure, wherein the disease or disorder is a tumor or cancer.

The seventh aspect of the present disclosure further provides a method for treating and/or preventing a disease or disorder or reducing the severity of the disease or disorder, comprising administering to an individual in need thereof an effective amount of the mixture of ligand-drug conjugates according to any one of the embodiments of the second aspect of the present disclosure, or the pharmaceutical composition according to any one of the embodiments of the sixth aspect of the present disclosure.

In certain embodiments, the tumor or cancer is selected from leukemia, malignant lymphoma, multiple myeloma, gastric cancer, colorectal cancer, pancreatic cancer, colon cancer, breast cancer, ovarian cancer, liver cancer, and lung cancer.

In certain embodiments, the tumor or cancer is selected from leukemia, malignant lymphoma, multiple myeloma, gastric cancer, colorectal cancer, breast cancer, ovarian cancer, liver cancer, and lung cancer.

In certain embodiments, the tumor or cancer is selected from gastric cancer, colorectal cancer, breast cancer, ovarian cancer, and liver cancer.

In certain embodiments, the colorectal cancer is selected from colorectal adenocarcinoma and colon cancer.

In certain embodiments, the tumor or cancer is selected from leukemia, malignant lymphoma, and multiple myeloma.

In certain embodiments, the breast cancer is selected from ductal breast cancer.

### Sequence Information

The amino acid sequence of the heavy chain oftusamitamab

EVQLQESGPGLVKPGGSLSLSCAASGFVFSSYDMSWVRQTPERGLEWVAYISSGGGITYA
PSTVKGRFTVSRDNAKNTLYLQMNSLTSEDTAVYYCAAHYFGSSGPFAYWGQGTLVTVS
SASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKS
RWQQGNVFSCSVMHEALHNHYTQKSLSLSPG (SEQ ID NO: 1)

The amino acid sequence of the light chain oftusamitamab

DIQMTQSPASLSASVGDRVTITCRASENIFSYLAWYQQKPGKSPKLLVYNTRTLAEGVPS
RFSGSGSGTDFSLTISSLQPEDFATYYCQHHYGTPFTFGSGTKLEIKRTVAAPSVFIFPPSDE
QLKSGTASVVCLLNNFYPREAKVQWKVDNALQSGNSQESVTEQDSKDSTYSLSSTLTLS
KADYEKHKVYACEVTHQGLSSPVTKSFNRGEC (SEQ ID NO: 2)

The sequences of the complementarity-determining regions and variable regions of tusamitamab are shown in the table below.

| No. | Description | Sequence |
|---|---|---|
| 53 | HCDR1 | SYDMS |
| 54 | HCDR2 | YISSGGGITYAPSTVKG |
| 55 | HCDR3 | HYFGSSGPFAY |
| 56 | LCDR1 | RASENIFSYLA |
| 57 | LCDR2 | NTRTLAE |
| 58 | LCDR3 | QHHYGTPFT |
| 59 | VL | |
| 60 | VH | |

The CDR sequences in the table above are defined using the Kabat numbering system. Any other CDR sequence determination method well-known in the art can also be used to identify the amino acid residues of the CDRs in the variable regions.

The amino acid sequence of the heavy chain of the anti-HER3 antibody DB1001

QVQLQQWGAGLLKPSETLSLTCAVYGGSFSGYYWSWIRQPPGKGLEWIGEINHSGSTNY
NPSLKSRVTISVETSKNQFSLKLSSVTAADTAVYYCARDKWTWYFDLWGRGTLVTVSSA
STKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPELLGGPS
VFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSR
WQQGNVFSCSVMHEALHNHYTQKSLSLSPGK (SEQ ID NO: 3)

The amino acid sequence of the light chain of the anti-HER3 antibody DB1001

DIEMTQSPDSLAVSLGERATINCRSSQSVLYSSSNRNYLAWYQQNPGQPPKLLIYWASTR
ESGVPDRFSGSGSGTDFTLTISSLQAEDVAVYYCQQYYSTPRTFGQGTKVEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGE (SEQ ID NO: 4)

The sequences of the complementarity-determining regions and variable regions of the anti-HER3 antibody DB1001are shown in the table below.

| No. | Description | Sequence |
|---|---|---|
| 7 | HCDR1 | GYYWS |
| 8 | HCDR2 | EINHSGSTNYNPSLKS |
| 9 | HCDR3 | DKWTWYFDL |
| 10 | LCDR1 | RSSQSVLYSSSNRNYLA |
| 11 | LCDR2 | WASTRES |
| 12 | LCDR3 | QQYYSTPRT |
| 13 | VL | |
| 14 | VH | |

The CDR sequences in the table above are defined using the Kabat numbering system. Any other CDR sequence determination method well-known in the art can also be used to identify the amino acid residues of the CDRs in the variable regions.

The amino acid sequence of the heavy chain of the anti-GPC-3 antibody DB1002

QVQLVQSGAEVKKPGASVKVSCKASGYTFTDYEMHWVRQAPGQGLEWMGALDPKTG
DTAYSQKFKGRVTLTADKSTSTAYMELSSLTSEDTAVYYCTRFYSYTYWGQGTLVTVSSA
STKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLTVDKSR
WQQGNVFSCSVMHEALHNHYTQKSLSLSPGK (SEQ ID NO: 5)

The amino acid sequence of the light chain of the anti-GPC-3 antibody DB1002

DVVMTQSPLSLPVTPGEPASISCRSSQSLVHSNRNTYLHWYLQKPGQSPQLLIYKVSNRF
SGVPDRFSGSGSGTDFTLKISRVEAEDVGVYYCSQNTHVPPTFGQGTKLEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC (SEQ ID NO: 6)

The sequences of the complementarity-determining regions and variable regions of the anti-GPC-3 antibody DB1002are shown in the table below.

| No. | Description | Sequence |
|---|---|---|
| 17 | HCDR1 | DYEMH |
| 18 | HCDR2 | ALDPKTGDTAYSQKFKG |
| 19 | HCDR3 | FYSYTY |
| 20 | LCDR1 | RSSQSLVHSNRNTYLH |
| 21 | LCDR2 | KVSNRFS |
| 22 | LCDR3 | SQNTHVPPT |
| 23 | VL | DVVMTQSPLSLPVTPGEPASISCRSSQSLVHSNRNTYLHWYLQKPGQSPQLLI YKVSNRFSGVPDRFSGSGSGTDFTLKISRVEAEDVGVYYCSQNTHVPPTFGQ GTKLEIK |
| 24 | VH | QVQLVQSGAEVKKPGASVKVSCKASGYTFTDYEMHWVRQAPGQGLEWMG ALDPKTGDTAYSQKFKGRVTLTADKSTSTAYMELSSLTSEDTAVYYCTRFYSY TYWGQGTLVTVSS |

The CDR sequences in the table above are defined using the Kabat numbering system. Any other CDR sequence determination method well-known in the art can also be used to identify the amino acid residues of the CDRs in the variable regions.

The amino acid sequence of the heavy chain of the anti-ADAM9antibody DB1003

QVQLQQPGTEVVKPGASVKLSCKASGFTFPLYWMHWVRQRPGRGLEWIGRIIPIFGHTK
YNEKFKNKATLTADESSSTAYMELRSLTSDDSAVYYCARGGYYYYPREGFLDYWGQGT
SVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPA
PELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSKL
TVDKSRWQQGNVFSCSVMHEALHNHYTQKSLSLSPGK (SEQ ID NO: 25)

The amino acid sequence of the light chain of the anti-ADAM9antibody DB 1003

DIVMTQSPASLSVSPGERASISCRSSQSVDYSGDSYMNWYQQKPGQSPRLLIYAASDRES
GVPDRFSGSGSGTDFTLKISRVEEEDAGTYYCQQSREDPFTFGQGTKVDIKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC (SEQ ID NO: 26)

The sequences of the complementarity-determining regions and variable regions of the anti-ADAM9antibody DB1003are shown in the table below.

| No. | Description | Sequence |
|---|---|---|
| 27 | HCDR1 | LYWMH |
| 28 | HCDR2 | RIIPIFGHTKYNEKFKN |
| 29 | HCDR3 | GGYYYYPREGFLDY |
| 30 | LCDR1 | RSSQSVDYSGDSYMN |
| 31 | LCDR2 | AASDRES |
| 32 | LCDR3 | QQSREDPFT |
| 33 | VL | DIVMTQSPASLSVSPGERASISCRSSQSVDYSGDSYMNWYQQKPGQSPRLLIY AASDRESGVPDRFSGSGSGTDFTLKISRVEEEDAGTYYCQQSREDPFTFGQGT KVDIK |
| 34 | VH | QVQLQQPGTEVVKPGASVKLSCKASGFTFPLYWMHWVRQRPGRGLEWIGRI IPIFGHTKYNEKFKNKATLTADESSSTAYMELRSLTSDDSAVYYCARGGYYYY PREGFLDYWGQGTSVTVSS |

The CDR sequences in the table above are defined using the Kabat numbering system. Any other CDR sequence determination method well-known in the art can also be used to identify the amino acid residues of the CDRs in the variable regions.

The amino acid sequence of the heavy chain of the anti-BCMAantibody DB1004 (i.e., belantamab)

QVQLVQSGAEVKKPGSSVKVSCKASGGTFSNYWMHWVRQAPGQGLEWMGATYRGHS
DTYYNQKFKGRVTITADKSTSTAYMELSSLRSEDTAVYYCARGAIYDGYDVLDNWGQG
TLVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQV
YTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGK (SEQ ID NO: 35)

The amino acid sequence of the light chain of the anti-BCMAantibody DB 1004 (i.e., belantamab)

DIQMTQSPSSLSASVGDRVTITCSASQDISNYLNWYQQKPGKAPKLLIYYTSNLHSGVPS
RFSGSGSGTDFTLTISSLQPEDFATYYCQQYRKLPWTFGQGTKLEIKRTVAAPSVFIFPPSD
EQLKSGTASVVCLLNNFYPREAKVQWKVDNALQSGNSQESVTEQDSKDSTYSLSSTLTL
SKADYEKHKVYACEVTHQGLSSPVTKSFNRGEC (SEQ ID NO: 36)

The sequences of the complementarity-determining regions and variable regions of the anti-BCMAantibody DB1004are shown in the table below.

| No. | Description | Sequence |
|---|---|---|
| 37 | HCDR1 | NYWMH |
| 38 | HCDR2 | ATYRGHSDTYYNQKFKG |
| 39 | HCDR3 | GAIYDGYDVLDN |
| 40 | LCDR1 | SASQDISNYLN |
| 41 | LCDR2 | YTSNLHS |
| 42 | LCDR3 | QQYRKLPWT |
| 43 | VL | DIQMTQSPSSLSASVGDRVTITCSASQDISNYLNWYQQKPGKAPKL LIYYTSNLHSGVPSRFSGSGSGTDFTLTISSLQPEDFATYYCQQYRK LPWTFGQGTKLEIK |
| 44 | VH | QVQLVQSGAEVKKPGSSVKVSCKASGGTFSNYWMHWVRQAPGQ GLEWMGATYRGHSDTYYNQKFKGRVTITADKSTSTAYMELSSLRS EDTAVYYCARGAIYDGYDVLDNWGQGTLVTVSS |

The CDR sequences in the table above are defined using the Kabat numbering system. Any other CDR sequence determination method well-known in the art can also be used to identify the amino acid residues of the CDRs in the variable regions.

The amino acid sequence of the heavy chain of the anti-c-met antibody DB1005

EVQLVESGGGLVQPGGSLRLSCAASGFTFTDYYMSWVRQAPGKGLEWLGFIRNKANGY
TTEYSASVKGRFTISRDNSKNTLYLQMNSLRAEDTAVYYCARDNWFAYWGQGTLVTVS
SASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDHCPPCPAPELLGGPS
VFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSR
WQQGNVFSCSVMHEALHNHYTQKSLSLSPG (SEQ ID NO: 15)

The amino acid sequence of the light chain of the anti-c-met antibody DB1005

DIQMTQSPSSLSASVGDRVTITCKSSQSLLASGNQNNYLAWYQQKPGKAPKMLIIWASTR
VSGVPSRFSGSGSGTDFTLTISSLQPEDFATYYCQQSYSRPYTFGQGTKVEIKRTVAAPSVF
IFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC (SEQ ID NO: 16)

The sequences of the complementarity-determining regions and variable regions of the anti-c-met antibody DB1005are shown in the table below.

| No. | Description | Sequence |
|---|---|---|
| 45 | HCDR1 | DYYMS |
| 46 | HCDR2 | FIRNKANGYTTEYSASVKG |
| 47 | HCDR3 | DNWFAY |
| 48 | LCDR1 | KSSQSLLASGNQNNYLA |
| 49 | LCDR2 | WASTRVS |
| 50 | LCDR3 | QQSYSRPYT |
| 51 | VL | DIQMTQSPSSLSASVGDRVTITCKSSQSLLASGNQNNYLAWYQQKP GKAPKMLIIWASTRVSGVPSRFSGSGSGTDFTLTISSLQPEDFATYYC QQSYSRPYTFGQGTKVEIK |
| 52 | VH | EVQLVESGGGLVQPGGSLRLSCAASGFTFTDYYMSWVRQAPGKG LEWLGFIRNKANGYTTEYSASVKGRFTISRDNSKNTLYLQMNSLR AEDTAVYYCARDNWFAYWGQGTLVTVSS |

The CDR sequences in the table above are defined using the Kabat numbering system. Any other CDR sequence determination method well-known in the art can also be used to identify the amino acid residues of the CDRs in the variable regions.

### The sequences of short peptides

**Note: X represents norvaline (Nva).**

| No. | Description | Sequence |
|---|---|---|
| 61 | Short peptide | GGGG |
| 62 | Short peptide | GGFG |
| 63 | Short peptide | GGEG |
| 64 | Short peptide | KAAN |
| 65 | Short peptide | KAAD |
| 66 | Short peptide | AAPV |
| 67 | Short peptide | AAPX |

### Definitions of Terms

The various terms and phrases used in the present application have general meanings well known to those skilled in the art. Nevertheless, the present application still intends to provide a more detailed illustration and explanation of these terms and phrases herein. In the event of any discrepancy between the terms and phrases mentioned and their well-known meanings, the meanings expressed in the present disclosure shall prevail.

The terms "a" and "an" and "the" and similar referents in the context of describing the present disclosure (especially in the context of the claims) are to be construed to cover both the singular and the plural, unless otherwise indicated in the present application or clearly contradicted by context.

When the terms "for example", "such as", or variants thereof are used in the present application, these terms will not be considered as limiting terms, and will be interpreted as "including but not limited to" or "not limited to".

When the terms "comprise", "include", or variants thereof are used in the present application, the meanings of the terms "consist of" and "consist essentially of" are also provided.

As used in the present application, the term "and/or" is to be considered as a specific disclosure of each of the two or more specified features or elements, as well as any combination of the two or more features or elements. Thus, the term "and/or" as used in a phrase, such as "A and/or B" herein, is intended to include "A and B", "A or B", "A" (alone), and "B" (alone).

As used in the present application, the term "pharmaceutically acceptable salt" refers to a salt of a compound of the present disclosure that is pharmaceutically acceptable and possesses the desired pharmacological activity of the parental compound. Such salts include acid addition salts formed with inorganic or organic acids, or salts formed by replacing acidic protons present on the parental compound with metal ions, or coordination complexes formed with organic bases.

As used in the present application, the term "stereoisomer" refers to an isomer formed due to the presence of at least one asymmetric center. In compounds having one or more (e.g., 1, 2, 3, or 4) asymmetric centers, racemic mixtures, single enantiomers, mixtures of diastereoisomers, and separate diastereoisomers may be produced. The term "stereoisomer" includes conformational isomers and configurational isomers, wherein the configurational isomers mainly include cis-trans isomers and optical isomers. The compounds of the present disclosure may be present in stereoisomeric forms and thus encompass all possible stereoisomeric forms and any combination thereof or any mixture thereof, for example, single enantiomers, single diastereoisomers, or mixtures of the above. When a compound of the present disclosure contains an olefin double bond, it includes cis-isomers and trans-isomers, as well as any combination thereof, unless otherwise specified.

As used in the present application, the term "tautomer" refers to a functional group isomer resulting from the rapid movement of an atom between two positions in a molecule, such as keto-enol tautomers and imine-enamine tautomers. If tautomers of a compound of the present disclosure exist, the compound may be present in the form of a single tautomer or a mixture of tautomers, preferably in a form in which a relatively stable tautomer predominates.

As used in the present application, the term "racemic mixture" encompasses "racemate", and the term "racemate" refers to an equimolar mixture of an optically active chiral molecule and an enantiomer thereof; the term "enantiomer" refers to one of a pair of molecular entities that are mirror images of each other and are non-superimposable, and a mixture of enantiomers can be separated under, for example, chiral resolution conditions; the term "diastereoisomer" refers to a stereoisomer that is not related to mirror images; diastereoisomers are characterized by some differences in physicochemical properties, and a mixture of diastereoisomers can be separated under, for example, chromatographic or crystallization conditions.

As used in the present application, the term "solvate" refers to a substance formed by association of a compound of the present disclosure with a solvent molecule. The solvent may be an organic solvent (e.g., methanol, ethanol, propanol, or acetonitrile). For example, the compounds of the present disclosure may form ethanolates with ethanol. The compounds of the present disclosure may also form hydrates with water. The amount of the solvent may be present in a stoichiometric or non-stoichiometric ratio.

Unless otherwise defined, the direction of a divalent group is not limited. For example, for A-L-B, when L is -C(=O)-NH-, A-L-B is A-C(=O)-NH-B and/or A-NH-C(=O)-B.

As used in the present application, the term "substituted" means that a hydrogen atom in a group is replaced with a corresponding substituent. For example, when the -CH₂- in A-CH₂-B is substituted with 1 -NH₂, A-CH(NH₂)-B is obtained. It will be appreciated that a substituent is only in its possible chemical position.

As used in the present application, the term "replaced" means that the group itself is replaced with a corresponding group. For example, when the -CH₂- in A-CH₂-B is replaced with -NH-, A-NH-B is obtained.

As used in the present application, "optionally substituted with" means that the group may be unsubstituted or substituted with a substituent. For example, -(C₁-C₆)alkyl optionally substituted with halogen means that the -(C₁-C₆)alkyl may be unsubstituted or substituted with halogen, resulting in haloalkyl. It will be appreciated that when a group consists of multiple moieties, defining that one of the moieties can be substituted means that this moiety in the group can also be substituted. For example, the expression "R is selected from -(C₁-C₆)alkyl, -O-(C₁-C₆)alkyl, and -NH(C₁-C₆)alkyl, wherein the -(C₁-C₆)alkyl is optionally substituted with halogen" means that the -(C₁-C₆)alkyl, the (C₁-C₆)alkyl in the -O-(C₁-C₆)alkyl, and the (C₁-C₆)alkyl in the -NH(C₁-C₆)alkyl are all optionally substituted with halogen. For example, the expression "R is selected from methyl, -O-methyl, -NH-methyl, -S-methyl, -C(=O)-methyl, and -C(=O)NH-methyl, wherein the methyl is optionally substituted with 1 hydroxyl or amino group" means that the methyl, as well as the methyl in the -O-methyl, -NH-methyl, -S-methyl, -C(=O)-methyl, and - C(=O)NH-methyl, are all optionally substituted with 1 hydroxyl or amino group.

As used in the present application, the term "halogen" refers to fluorine, chlorine, bromine, or iodine.

As used in the present application, the term "alkyl" refers to a linear or branched monovalent saturated hydrocarbyl group. For example, (C₁-C₆)alkyl means having 1 to 6 carbon atoms, such as 1, 2, 3, 4, 5, or 6 carbon atoms; (C₁-C₄)alkyl means having 1 to 4 carbon atoms, such as 1, 2, 3, or 4 carbon atoms. Non-limiting examples of alkyl include, but are not limited to, methyl, ethyl, propyl, propyl, butyl, etc.

As used in the present application, the term "alkylene" refers to a linear or branched divalent saturated hydrocarbyl group. For example, (C₁-C₆)alkylene means having 1 to 6 carbon atoms, such as 1, 2, 3, 4, 5, or 6 carbon atoms; (C₁-C₄)alkylene means having 1 to 4 carbon atoms, such as 1, 2, 3, or 4 carbon atoms. Non-limiting examples of alkylene include, but are not limited to, methylene, ethylene, propylene, butylene, etc.

As used in the present application, the term "halo" means that the modified group is substituted with one or more halogens, for example, with 1, 2, 3, 4, 5, or 6 halogens. For example, the expression "(C₁-C₆)haloalkyl" means that the (C₁-C₆)alkyl defined above is substituted with one or more halogens; non-limiting examples include, but are not limited to, CF₃, CHF₂, CF₂CF₃, or the like.

As used in the present application, the term "alkenyl" refers to a linear or branched unsaturated hydrocarbyl group containing at least one double bond. (C₂-C₆)alkenyl means having 2 to 6 (e.g., 2, 3, 4, 5, or 6) carbon atoms, and includes (C₂-C₅)alkenyl, (C₂-C₄)alkenyl, (C₂-C₃)alkenyl, etc.; non-limiting examples include, but are not limited to, -CH=CH₂, -CH=CH-CH=CH₂, - CH=C(CH)₃-CH₃, or the like.

As used in the present application, the term "alkynyl" refers to a linear or branched unsaturated hydrocarbyl group containing at least one triple bond. For example, (C₂-C₆)alkynyl means having 2 to 6 (e.g., 2, 3, 4, 5, or 6) carbon atoms, and includes (C₂-C₅)alkynyl, (C₂-C₄)alkynyl, (C₂-C₃)alkynyl, etc.; non-limiting examples include, but are not limited to, ethynyl, propynyl, or the like.

As used in the present application, the term "cycloalkyl" refers to a monovalent saturated hydrocarbon group consisting of carbon atoms. For example, (C₃-C₆)cycloalkyl means consisting of 3-6 (e.g., 3, 4, 5, or 6) carbon atoms. The cycloalkyl includes monocyclic, bicyclic, or polycyclic systems, and includes spiro, fused, or bridged rings. Non-limiting examples include, but are not limited to, cyclobutyl, cyclopentyl, cyclohexyl, or the like.

As used in the present application, the term "cycloalkylene" refers to a divalent saturated hydrocarbon group consisting of carbon atoms. For example, (C₃-C₆)cycloalkylene means consisting of 3-6 (e.g., 3, 4, 5, or 6) carbon atoms. The cycloalkylene includes monocyclic, bicyclic, or polycyclic systems, and includes spiro, fused, or bridged rings. Non-limiting examples include, but are not limited to, cyclobutylene, cyclopentylene, cyclohexylene, or the like.

As used in the present application, the term "heterocyclyl" refers to a saturated or partially unsaturated monovalent cyclic group consisting of ring atoms, wherein 1, 2, 3, or 4 of the ring atoms are heteroatoms, and the rest are carbon atoms; preferably, the heteroatoms are selected from N, O, or S, wherein the nitrogen atom is optionally quaternized, and the nitrogen and sulfur heteroatoms may be optionally oxidized; preferably, the carbon atoms are optionally substituted with =O. For example, (4-10 membered)heterocyclyl means consisting of 4, 5, 6, 7, 8, 9, or 10 ring atoms, and (4-10 membered)heterocyclyl includes (4-9 membered)heterocyclyl, (4-8 membered)heterocyclyl, (4-7 membered)heterocyclyl, (4-6 membered)heterocyclyl, 5-membered heterocyclyl, 6-membered heterocyclyl, etc. The heterocyclyl includes monocyclic, bicyclic, or polycyclic systems, and includes spiro, fused, or bridged rings. Non-limiting examples include, but are not limited to, oxetanyl, azetidinyl, pyrrolidinyl, piperidinyl, piperazinyl, morpholinyl, or the like.

As used in the present application, the term "heterocyclylene" refers to a saturated or partially unsaturated divalent cyclic group consisting of ring atoms, wherein 1, 2, 3, or 4 of the ring atoms are heteroatoms, and the rest are carbon atoms; preferably, the heteroatoms are selected from N, O, or S, wherein the nitrogen atom is optionally quaternized, and the nitrogen and sulfur heteroatoms may be optionally oxidized; preferably, the carbon atoms are optionally substituted with =O. For example, (4-10 membered)heterocyclylene means consisting of 4-10 (e.g., 4, 5, 6, 7, 8, 9, or 10) ring atoms, and (4-10 membered)heterocyclylene includes (4-8 membered)heterocyclylene, (4-6 membered)heterocyclylene, 5-membered heterocyclylene, etc. The heterocyclylene includes monocyclic, bicyclic, or polycyclic systems, and includes spiro, fused, or bridged rings. Non-limiting examples include, but are not limited to, oxetanylene, azetidinylene, pyrrolidinylene, piperidinylene, piperazinylene, morpholinylene, or the like.

As used in the present application, the term "aryl" refers to an unsaturated carbocyclic group having a conjugated π-electron system. For example, (C₆-C₁₀)aryl consists of 6 to 10 (e.g., 6, 7, 8, 9, or 10) carbon atoms. Non-limiting examples include, but are not limited to, phenyl, etc.

As used in the present application, the term "arylene" refers to a divalent unsaturated carbocyclic group having a conjugated π-electron system. For example, (C₆-C₁₀)arylene consists of 6 to 10 (e.g., 6, 7, 8, 9, or 10) carbon atoms. Non-limiting examples include, but are not limited to, phenylene, etc.

As used in the present application, the term "heteroaryl" refers to an unsaturated group having a conjugated π-electron system and consisting of ring atoms, wherein 1, 2, 3, or 4 of the ring atoms are heteroatoms, and the rest are carbon atoms; preferably, the heteroatoms are selected from N, O, or S, wherein the nitrogen atom is optionally quaternized, and the nitrogen and sulfur heteroatoms may be optionally oxidized. For example, (5-12 membered)heteroaryl consists of 5 to 12 (e.g., 5, 6, 7, 8, 9, 10, 11, or 12) ring atoms, and includes 5-9 membered heteroaryl, 6-9 membered heteroaryl, 5-6 membered heteroaryl, etc. The heteroaryl includes monocyclic and polycyclic systems; non-limiting examples include, but are not limited to, imidazolyl, pyridinyl, or the like.

As used in the present application, the term "heteroarylene" refers to an unsaturated group having a conjugated π-electron system and consisting of ring atoms, wherein 1, 2, 3, or 4 of the ring atoms are heteroatoms, and the rest are carbon atoms; preferably, the heteroatoms are selected from N, O, or S, wherein the nitrogen atom is optionally quaternized, and the nitrogen and sulfur heteroatoms may be optionally oxidized. For example, (5-10 membered)heteroarylene consists of (5 to 10) (e.g., 5, 6, 7, 8, 9, or 10) ring atoms, and includes 5-9 membered heteroarylene, 6-9 membered heteroarylene, 5-6 membered heteroarylene, etc. The heteroarylene includes monocyclic and polycyclic systems; non-limiting examples include, but are not limited to, imidazolylene, pyridinylene, or the like.

As used in the present application, the abbreviations for amino acids have meanings well-known in the art. The amino acids are generally L-amino acids. Those skilled in the art will appreciate that they may also be replaced with D-amino acids with similar chemical properties. For example, Val represents valine, Ala represents alanine, Glu represents glutamic acid, Cit represents citrulline, Nva represents norvaline, and Lys(AC) represents acetylated lysine.

As used in the present application, the term "short peptide" refers to a chain comprising at least two contiguously linked amino acid residues. Unless otherwise stated, the direction in which a short peptide is linked is not limited to the indicated direction. For example, for A-short peptide-B, if the short peptide is Ala-Val, it may be A-Ala-Val-B or A-Val-Ala-B.

As used in the present application, the term "linking unit" refers to a fragment for linking an antibody.

As used in the present application, the term "about" generally means a variation within 0.5%-10% above or below the stated numerical value, for example, a variation within 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the stated numerical value. Unless otherwise apparent from the context, all numerical values provided herein are modified by the term "about".

The endpoints of the ranges and any values disclosed in the present application are not limited to the precise ranges or values, and these ranges or values should be interpreted as including values close to these ranges or values. For numerical ranges, the endpoint values of the ranges, the endpoint values of the ranges and the discrete point values, and the discrete point values can be combined with each other to obtain one or more new numerical ranges, and these numerical ranges should be considered to be specifically disclosed in the present application.

As used in the present application, the term "antibody" refers to any antibody form having the desired bioactivity. Thus, it is used in the broadest sense and specifically includes, but is not limited to, monoclonal antibodies (including full-length monoclonal antibodies), polyclonal antibodies, multispecific antibodies (e.g., bispecific antibodies), humanized antibodies, fully human antibodies, chimeric antibodies, and camelized single-domain antibodies.

As used in the present application, the term "monoclonal antibody" refers to an antibody obtained from a substantially homogeneous population of antibodies, which means that the antibodies comprising the population are identical except for a small number of possible naturally occurring mutations that may be present. A monoclonal antibody is highly specific and targets a single antigen epitope. In contrast, conventional (polyclonal) antibody preparations typically include a large number of antibodies targeting (or specific for) different epitopes. The modifier "monoclonal" indicates the characteristics of an antibody obtained from a substantially homogeneous population of antibodies, and shall not be interpreted as requiring any particular method to produce the antibody.

As used in the present application, the term "antigen-binding fragment" of an antibody includes fragments or derivatives of the antibody, generally including at least one fragment of an antigen-binding region or variable region (e.g., one or more CDRs) of the parental antibody that retains at least some of the binding specificity of the parental antibody. Examples of binding fragments of an antibody include, but are not limited to, Fab, Fab', F(ab')₂, and Fv fragments; bispecific antibodies; linear antibodies; single-chain antibody molecules, such as scFv; and nanobodies and multispecific antibodies formed from fragments of the antibody. A binding fragment or a derivative generally retains at least 10% of its antigen-binding activity when the antigen-binding activity is expressed on a molar concentration basis. Preferably, the binding fragment or the derivative retains at least 20%, 50%, 70%, 80%, 90%, 95%, or 100% or more of the antigen-binding affinity of the parental antibody. It is also contemplated that an antigen-binding fragment of an antibody may include conservative or non-conservative amino acid substitutions that do not significantly alter its bioactivity (referred to as "conservative variants" or "functionally conservative variants" of the antibody).

In the present application, the term "ligand-drug conjugate" generally means that a ligand is linked to a biologically active cytotoxic drug by a stable linking unit. In some embodiments of the present application, the "ligand-drug conjugate" may be an antibody-drug conjugate (ADC). The ADC may mean that a monoclonal antibody or an antigen-binding fragment is linked to a biologically active cytotoxic drug by a stable linking unit.

In the present application, the term "ligand" generally refers to small molecules, polypeptides, RNA, DNA, sugars, and macromolecular compounds capable of recognizing and binding to an antigen or receptor associated with a target cell. The role of ligands may be to present the drug to a target cell population to which the ligands bind, and these ligands include, but are not limited to, protein hormones, lectin, growth factors, antibodies, or other molecules capable of binding to cells, receptors, and/or antigens. In the present application, a ligand may be represented as Ab, a ligand antigen forms a linking bond with a linking unit (also referred to as "linker") through a heteroatom on the ligand, and the ligand may be an antibody or an antigen-binding fragment thereof, wherein the antibody may be selected from a chimeric antibody, a humanized antibody, a fully human antibody, or a murine antibody; the antibody may be a monoclonal antibody; the antibody may be a bispecific antibody. For example, the antibody may be an antibody that targets a target selected from the following targets: HER2, B7H3, TROP2, Claudin 18.2, CD30, CD33, CD70, and EGFR. For example, the antibody may be an antibody that targets a target selected from the following targets: 5T4, AGS-16, ANGPTL4, ApoE, CD19, CTGF, CXCR5, FGF2, MCPT8, MFI2, MS4A7, NCA, Sema5b, SLITRK6, STC2, TGF, 0772P, 5T4, ACTA2, ADGRE1, AG-7, AIF1, AKR1C1, AKR1C2, ASLG659, Axl, B7H3, BAFF-R, BCMA, BMPR1B, BNIP3, C1QA, C1QB, CA6, CADM1, CCD79b, CCL5, CCR5, CCR7, CD11c, CD123, CD138, CD142, CD147, CD166, CD19, CD19,CD22, CD21, CD20, CD205, CD22, CD223, CD228, CD25, CD30, CD33, CD37, CD38, CD40, CD45, CD45(PTPRC), CD46, CD47, CD49D(ITGA4), CD56, CD66e, CD70, CD71, CD72, CD74, CD79a, CD79b, CD80, CDCP1, CDH11, CDllb, CEA, CEACAM5, c-Met, COL6A3, COL7A1, CRIPTO, CSF1R, CTSD, CTSS, CXCL11, CXCL10, DDIT4, DLL3, DLL4, DRS, E16, EFNA4, EGFR, EGFRvIII, EGLN, EGLN3, EMR2, ENPP3, EpCAM, EphA2, EphB2R, ETBR, FcRH2, FcRH1, FGFR2, FGFR3, FLT3, FOLR-α, GD2, GEDA, GPC-1, GPC-3, GPNMB, GPR20, GZMB, HER2, HLA-DOB, HMOX1, IFI6, IFNG, IGF-1R, IGFBP3, IL10RA1, IL-13R, IL-2, IL20Ra, IL-3, IL-4, IL-6, IRTA2, KISS1R, KRT33A, LIV-1, LOX, LRP-1, LRRC15, LUM, LY64, LY6E, Ly86, LYPD3, MDP, MMP10, MMP14, MMP16, MPF, MSG783, MSLN, MUC-1, NaPi2b, Napi3b, Nectin-4, NOG, P2X5, pCAD, P-Cadherin, PDGFRA, PDK1, PD-L1, PFKFB3, PGF, PGK1, PIK3AP1, PIK3CD, PLOD2, PSCA, PSCAhlg, PSMA, PTK7, P-Cadherin, RNF43, ROR1, ROR2, SERPINE1, SLC39A6, SLTRK6, STAT1, STEAP1, STEAP2, TCF4, TENB2, TGFB1, TGFB2, TGFBR1, TNFRSF21, TNFSF9, Trop-2, TrpM4, Tyro7, UPK1B, VEGFA, WNT5A, ADAM9, epidermal growth factors, brevican, mesothelin, sodium phosphate cotransporter 2B, Claudin18.2, endothelin receptors, mucins (e.g., mucin 1 and mucin 16), guanylate cyclase C, integrin α4β7, integrin αvβ6, trophoblast glycoprotein, and tissue factors. As used in the present application, the term "specific binding", "specificity", or "specific for" refers to a non-random binding reaction between two molecules, such as a reaction between an antibody and an antigen that it targets. In certain embodiments, an antibody that specifically binds to an antigen (or an antibody that is specific for an antigen) means that the antibody binds to the antigen with an affinity (K_{D}) of less than about 10⁻⁵ M, e.g., less than about 10⁻⁶ M, 10⁻⁷ M, 10⁻⁸ M, 10⁻⁹ M, or 10⁻¹⁰ M or less.

The term "drug loading" generally refers to the average number of cytotoxic drugs loaded on each ligand, and may also be represented as the ratio of the number of cytotoxic drugs to the number of ligands, such as average drug/ligand ratio (DAR). The cytotoxic drug loading may be an integer or a decimal ranging from 0 to 20. In the embodiments of the present application, the drug/ligand ratio is represented as k, and may illustratively be an integer or a decimal from 1 to 2, from 2 to 3, from 3 to 4, from 4 to 5, from 5 to 6, from 6 to 7, from 7 to 8, from 8 to 9, or from 9 to 10. The drug loading of each ADC molecule after a conjugation reaction can be determined by conventional methods such as UV/visible spectroscopy, mass spectrometry, HIC, ELISA, and HPLC characterization.

As used in the present application, the term "pharmaceutically acceptable carriers and/or excipients" refers to carriers and/or excipients that are pharmacologically and/or physiologically compatible with the subject and the active ingredient, which are well-known in the art (see, e.g., Remington's Pharmaceutical Sciences. Edited by Gennaro AR, 19th ed. Pennsylvania: Mack Publishing Company, 1995). Pharmaceutically acceptable carriers and/or excipients include, but are not limited to: pH modulators, surfactants, ionic strength enhancers, diluents, agents for maintaining osmolality, agents for delaying absorption, preservatives, and stabilizers. For example, pH modulators include, but are not limited to, phosphate buffers. Surfactants include, but are not limited to, cationic, anionic, or non-ionic surfactants, such as Tween-80. Ionic strength enhancers include, but are not limited to, sodium chloride. Preservatives include, but are not limited to, various antibacterial agents and antifungal agents, such as parabens, chlorobutanol, phenol, sorbic acid, etc. Agents for maintaining osmolality include, but are not limited to, sugars, NaCl, and analogs thereof. Agents for delaying absorption include, but are not limited to, monostearates and gelatin. Diluents include, but are not limited to, water, aqueous buffers (e.g., buffered saline), alcohols, polyols (e.g., glycerol), etc. Preservatives include, but are not limited to, various antibacterial agents and antifungal agents, such as thimerosal, 2-phenoxyethanol, parabens, chlorobutanol, phenol, sorbic acid, etc. Stabilizers have the meaning generally understood by those skilled in the art, are capable of stabilizing the desired activity of the active ingredient in a drug, and include, but are not limited to, sodium glutamate, gelatin, SPGA, sugars (e.g., sorbitol, mannitol, starch, sucrose, lactose, dextran, or glucose), amino acids (e.g., glutamic acid or glycine), proteins (e.g., dried whey, albumin, or casein) or degradation products thereof (e.g., lactalbumin hydrolysates), etc.

As used in the present application, the term "treat", "treating", or "treatment" are intended to alleviate, palliate, ameliorate, or eliminate a targeted disease state or disorder. A subject is considered successfully "treated" if there is an observable and/or detectable reduction or improvement in one or more indications and symptoms of the subject after the subject receives a therapeutic amount of the ligand-drug conjugate or a racemic mixture, enantiomer, diastereoisomer, or pharmaceutically acceptable salt thereof, or a mixture of the aforementioned forms, according to the method described herein. It will also be appreciated that the treatment of the disease state or disorder includes not only complete treatment, but also achieving some biologically or medically related outcomes even if complete treatment is not attained.

As used in the present application, the term "prevent", "preventing", or "prevention" is intended to avoid, reduce, block, or delay the occurrence of a disease or disease-related symptom that has not yet appeared before the administration of a related drug. "Prevention" does not necessarily require completely blocking the onset of a disease or disease-related symptom. For example, if the administration of a related drug can reduce the subject's risk of developing the specific disease or disease-related symptom, or reduce the severity of a related symptom that appears later, the onset or development of the disease can be considered to be "prevented".

As used in the present application, the DMSO solvent used for dissolving compounds in the identification of structures by nuclear magnetic resonance is deuterated DMSO, i.e., DMSO-*d₆*.

### BRIEF DESCRIPTION OF THE DRAWINGS

FIG. 1 shows the *in vivo* inhibitory effects of the ligand-drug conjugates of the present disclosure on HPAF-II graft tumors.
FIG. 2 shows the *in vivo* inhibitory effects of the ligand-drug conjugates of the present disclosure on Panc 08.13 graft tumors.
FIG. 3 shows the *in vivo* inhibitory effects of the ligand-drug conjugates of the present disclosure (5 mg/kg) on NCI-H929 graft tumors.
FIG. 4 shows the *in vivo* inhibitory effects of the ligand-drug conjugates of the present disclosure (3 mg/kg) on NCI-H929 graft tumors.
FIG. 5 shows the *in vivo* inhibitory effects of the ligand-drug conjugates of the present disclosure on KMS11 graft tumors.
FIG. 6 shows the *in vivo* inhibitory effects of the ligand-drug conjugates of the present disclosure on SNU-5 graft tumors.
FIG. 7 shows the *in vivo* inhibitory effects of the ligand-drug conjugates of the present disclosure on Calu3 graft tumors.
FIG. 8 shows the *in vivo* inhibitory effects of the ligand-drug conjugates of the present disclosure on NCI-H1975 graft tumors.
FIG. 9 shows the *in vivo* inhibitory effects of the ligand-drug conjugates of the present disclosure on NCI-H820 graft tumors.
FIG. 10 shows the *in vivo* inhibitory effects of the ligand-drug conjugates of the present disclosure on LD1-0038-361928 graft tumors.

### Beneficial Effects

The linker-protein translation inhibitor conjugates provided in the present disclosure have one or more of the following advantages:
(1) having extremely high stability;
(2) having extremely high conjugation efficiency; and
(3) the conjugation reaction being insensitive to pH, and having relatively high conjugation efficiency at a pH value of 4.0-9.0.

The ligand-drug conjugates of the present disclosure have one or more of the following advantages:
(1) conjugates obtained according to the conjugation mode of the present disclosure being capable of effectively improving the stability of the drug molecule in circulation, thereby reducing the shedding of the drug on non-target cells; and reducing off-target toxicity;
(2) the conjugates having good tumor tissue-targeting capability; and
(3) the conjugates having good therapeutic effects on animal models of tumors.

In addition, the conjugation mode described in the present disclosure has a relatively wide application range, and can be widely used for the conjugation of biologically active molecules to antibodies or targeting small molecule ligands.

In summary, the linkers and ligand-drug conjugates of the present disclosure have great clinical value.

### DETAILED DESCRIPTION

The embodiments of the present disclosure are described in detail below with reference to examples. However, those skilled in the art will appreciate that the following examples are only used to illustrate the present disclosure and should not be construed as limiting the scope of the present disclosure. Procedures without specified conditions in the examples were conducted under conventional conditions or conditions recommended by the manufacturers. All reagents or instruments used herein without specified manufacturers were commercially available conventional products. Salts of the compounds obtained in the present disclosure can all be neutralized by conventional methods to give the corresponding free base compounds.

**Table 1**

| Abbreviation | Meaning | Abbreviation | Meaning |
|---|---|---|---|
| DMF | N,N-Dimethylformamide | HOBT | 1-Hydroxybenzotriazole |
| DIEA | Diisopropylethylamine | LC-MS | Liquid chromatography-mass spectrometry |
| HPLC | High-performance liquid chromatography | TLC | Thin-layer chromatography |
| HATU | O-(7-Azabenzotriazol-1-yl)-N,N,N',N'-tetramethyluronium hexafluorophosphate | Na₂SO₄ | Anhydrous sodium sulfate |
| EA or EtOAc | Ethyl acetate | DMSO | Dimethyl sulfoxide |
| DIPEA | N,N-Diisopropylethylamine | Pd(dba)₃ | Tris(dibenzylideneacetone)dipa lladium |
| Boc₂O | Di-tert-butyl dicarbonate | THF | Tetrahydrofuran |
| MeCN | Acetonitrile | NaBH(OAc)₃ | Sodium triacetoxyborohydride |
| Pd/C | Palladium on carbon | NaOMe | Sodium methoxide |
| Cs₂CO₃ | Cesium carbonate | Pd(PPh₃)₄ | Tetrakis(triphenylphosphine)pa lladium |
| AcOH | Acetic acid | Zn(CN)₂ | Zinc cyanide |
| DPPF | 1,1'-Bis(diphenylphosphino)fe rrocene | LiOH.H₂O | Lithium hydroxide monohydrate |
| NMP | N-Methyl-2-pyrrolidone | Pd(OH)₂/C | Palladium hydroxide on carbon |
| STAB | Sodium triacetoxyborohydride | PPh₃ | Triphenylphosphine |
| Pd(OAc)₂ | Palladium acetate | Pyridine.HF | Pyridine hydrofluoride |
| DIAD | Diisopropyl azodicarboxylate | FA | Formic acid |
| EDCI | 1-Ethyl-(3-dimethylaminopropyl)car bodiimide hydrochloride | TEA | Triethylamine |
| DMA | Dimethylamine | CHCl₃ | Trichloromethane, chloroform |
| TFE | Trifluoroethanol | t-Buxphos Pd G3 | Methanesulfonato(2-di-tert-butylphosphino-2',4',6'-triisopropyl-1,1'-biphenyl)(2'-amino-1,1'-biphenyl-2-yl)palladium(II) |
| PE | Petroleum ether | DMAc | N,N-Dimethylacetamide |
| TIPS | Triisopropylsilyl | TMP | 2,4,6-Trimethylpyridine |
| HOAT | N-Hydroxy-7-azabenzotriazole | Na(AcO)₃BH | Sodium triacetoxyborohydride |

### General chiral column resolution method

The racemate compounds of the present disclosure were dissolved in methanol and resolved on a preparative chiral chromatography column (see the table below) to give the product compounds. The absolute configurations of the compounds can be determined by methods such as optical rotation detection and X-ray single-crystal diffraction.

| | |
|---|---|
| Instrument | SFC-150 Mgm (Waters) or equivalent instrument |
| Preparative chiral chromatography columns | Daicel IC (25 × 250 mm, 10 µm), Daicel OJ (25 × 250 mm, 10 µm), Daicel AD (25 × 250 mm, 10 µm), or chromatography columns of other brands |
| Column temperature | 30 °C |
| Mobile phases | Mobile phase A: CO₂ |
| | Mobile phase B: MeOH or EtOH, with 0.2-0.5% NH₃ (7 M in MeOH) added |
| Flow rate | 100 ml/min |
| Detection wavelength | 214 nm |

### Example 1: Synthesis of Intermediates

### Example 1.1: Synthesis of Small Molecule Compounds

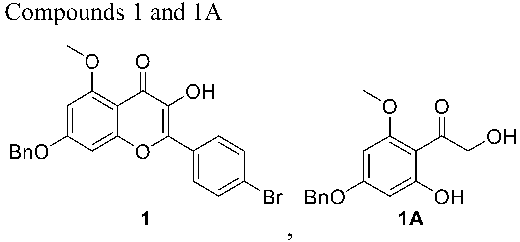

Compounds 1 and 1A were synthesized according to Liu, Tao; Nair, Somarajan J.; Lescarbeau, André; Belani, Jitendra; Peluso, Stéphane; Conley, James et al. (2012): Synthetic silvestrol analogues as potent and selective protein synthesis inhibitors. In Journal of medicinal chemistry 55 (20), pp. 8859-8878. DOI: 10.1021/jm3011542. See compound S2 on page 7 of Supporting Information.

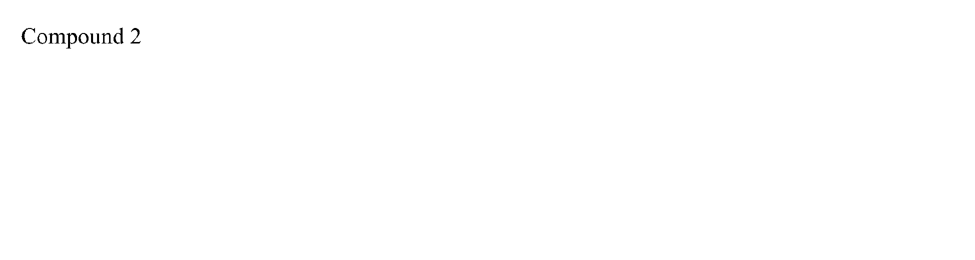

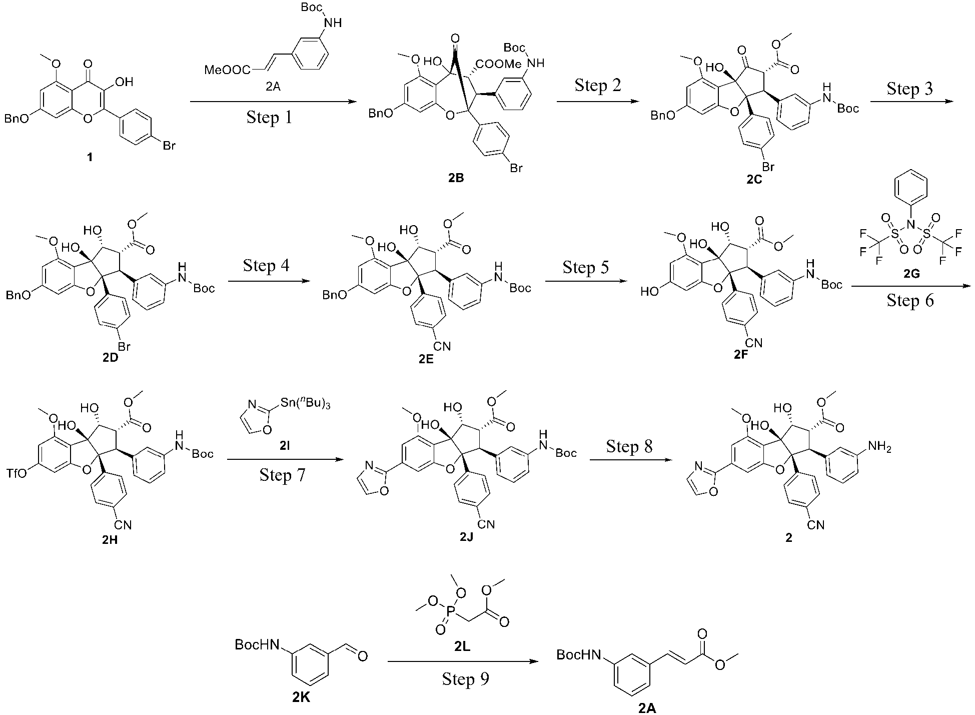

Step 1: Aftercompound1 (750 mg, 1.66 mmol) and compound 2A (2752 mg, 9.93 mmol) were dissolved in CHCl₃/TFE (7/3, 15 mL), a 20 mL syringe was used to inject the reaction mixture at a rate of 15 mL/h through a syringe pump into a coil wound around a jacketed glass cylinder equipped with a 250 W ultraviolet lamp. The temperature of the external circulation cold trap was adjusted to keep the internal reaction temperature at 0-5 °C, and the UV lamp was turned on simultaneously. The reaction mixture was irradiated for 1 h, concentrated under reduced pressure to remove the solvent, and then separated by column chromatography (EA/PE = 1/3) to remove the excess cinnamate, giving a racemate of the desired product 2B (crude, 900 mg, yield: 75%) as a yellow oil. LCMS [M+H+18]⁺ = 748.5.

Step 2: The racemate of compound 2B (5.2 g, 7.96 mmol) was mixed with MeOH (50 mL), and NaOMe (1.1 g, 19.91 mmol) was added at 0 °C. The mixture was stirred at 65 °C for 1.5 h. The solution was concentrated, sequentially washed with H₂O, an aqueous NH₄Cl solution, and saturated brine, dried over Na₂SO₄, concentrated under reduced pressure, and purified by silica gel column chromatography (0.2% FA, EA/PE = 1/3) to give a racemate of compound 2C (3.8 g, yield: 65%). LCMS [M+H-18]⁺ = 714.4.

Step 3: The racemate of compound 2C (3.8 g, 5.21 mmol) was mixed with MeCN/CHCl₃ (1/1, 100 mL/100 mL), and NaBH(OAc)₃ (5.5 g, 26.03 mmol) and AcOH (3.1 g, 52.05 mmol) were added at 0 °C. The mixture was stirred at room temperature for 2 h. The reaction solution was extracted with EA. The organic phase was sequentially washed with water and saturated brine, dried over anhydrous Na₂SO₄, and purified by silica gel column chromatography (PE:EA = 1/1) to give a racemate of compound 2D (1.9 g, yield: 50%). LCMS [M+H]⁺ = 716.5.

Step 4: A solution of the racemate of compound 2D (1.3 g, 1.78 mmol), Pd₂(dba)₃ (325.0 mg, 0.36 mmol), DPPF (393.6 mg, 0.71 mmol), and Zn(CN)₂ (519.5 mg, 4.44 mmol) in NMP (20 mL) was heated to 150 °C and heated at this temperature for 2 h. The solution was diluted with water, extracted with EA, sequentially washed with water and brine, dried over anhydrous Na₂SO₄, and purified by silica gel column chromatography (EA/PE = 1/1) to give a racemate of compound 2E (1.1 g, yield: 91%).

¹H NMR (400 MHz, CDCl₃) δ 7.48-7.40 (m, 6H), 7.37 (dt, J = 12.4, 5.3 Hz, 3H), 6.96 (t, J = 7.9 Hz, 1H), 6.85 (s, 1H), 6.51 (d, J = 7.7 Hz, 1H), 6.45 (s, 1H), 6.36 (d, J = 1.9 Hz, 1H), 6.22 (d, J = 1.9 Hz, 1H), 5.09 (d, J = 1.7 Hz, 2H), 5.01 (d, J = 6.4 Hz, 1H), 4.35 (d, J = 14.2 Hz, 1H), 3.95-3.81 (m, 4H), 3.66 (d, J = 3.8 Hz, 3H), 1.53-1.47 (m, 9H). LCMS [M+H-18]⁺ = 605.4.

Step 5: Pd(OH)₂/C (10%, 250 mg) was added to a solution of the racemate of compound 2E (500.0 mg, 0.74 mol) in EA/MeOH/THF/DCM (20:2:2:0.2, 40 mL). The mixture was purged with hydrogen three times and stirred at room temperature for 16 h. The suspension was filtered, and the filter cake was washed with MeOH/DCM (1/10). The filtrate was concentrated and purified by silica gel column chromatography (DCM/MeOH = 10/1) to give a racemate of compound 2F (300 mg, yield: 69%).

¹H NMR (400 MHz, CDCl₃) δ 7.42 (d, J = 8.6 Hz, 2H), 7.33 (d, J = 8.6 Hz, 2H), 6.98 (dd, J = 16.1, 8.2 Hz, 1H), 6.87 (s, 1H), 6.51 (d, J = 7.7 Hz, 1H), 6.33 (s, 1H), 6.24 (d, J = 1.8 Hz, 1H), 6.12 (d, J = 1.7 Hz, 1H), 4.98 (d, J = 6.3 Hz, 1H), 4.35 (d, J = 14.2 Hz, 1H), 3.90 (dd, J = 14.1, 6.4 Hz, 1H), 3.83 (d, J = 15.4 Hz, 3H), 3.67 (d, J = 4.5 Hz, 3H), 1.50 (d, J = 4.8 Hz, 9H). LCMS [M+H]⁺ = 515.4.

Step 6: The racemate of compound 2F (306.0 mg, 0.52 mmol) and K₂CO₃ (143.6 mg, 1.04 mmol) were stirred in DMF (10 mL). Compound 2G (185.8 mg, 0.52 mmol) was added. The reaction solution was stirred at 60 °C for 20 min, diluted with water, extracted with EA, sequentially washed with water and brine, dried over anhydrous Na₂SO₄, and purified by silica gel column chromatography (EA/PE = 1/1) to give a racemate of compound 2H (300 mg, yield: 80%).

¹H NMR (400 MHz, CDCl₃) δ 7.41 (d, J = 8.4 Hz, 2H), 7.33 (d, J = 8.5 Hz, 2H), 7.00-6.92 (m, 2H), 6.69 (d, J = 1.7 Hz, 1H), 6.53 (d, J = 7.6 Hz, 1H), 6.45 (d, J = 1.7 Hz, 1H), 6.39 (s, 1H), 4.99 (d, J = 5.9 Hz, 1H), 4.39 (d, J = 14.2 Hz, 1H), 3.99-3.91 (m, 4H), 3.68 (s, 3H), 1.50 (s, 9H). LCMS [M-H]⁺ = 719.2.

Step 7: A solution of the racemate of compound 2H (30 mg, 0.042 mmol) was added to DMF (3 mL), and compound 2I (60 mg, 0.167 mmol), Pd(PPh₃)₄ (4.8 mg, 0.0042 mmol), and CuI (1.6 mg, 0.0083 mmol) were added under a nitrogen atmosphere. The mixture was stirred at 100 °C for 1 h. The suspension was filtered and then purified by silica gel column chromatography (DCM/MeOH = 10/1) to give a racemate of compound 2J (15 mg, yield: 56%). LCMS [M+H]⁺ = 640.3.

Step 8: THF/6 M HCl (1/1, 4 mL) was added to a solution of the racemate of compound 2J (15 mg, 0.02 mmol), and the mixture was stirred at room temperature for 1 h. The solution was concentrated and purified by silica gel column chromatography (DCM/MeOH) to give a racemate of 2 (4.5 mg, yield: 35%) as a white solid, and the racemate was further resolved on a chiral column to give compound 2.

### LCMS [M+H]⁺ = 540.1.

¹H NMR (400 MHz, DMSO) δ 8.25 (d, *J* = 0.7 Hz, 1H), 7.50 (d, *J* = 8.6 Hz, 2H), 7.41 (d, *J* = 0.7 Hz, 1H), 7.31 (d, *J* = 8.6 Hz, 2H), 7.19 (d, *J* = 1.1 Hz, 1H), 7.14 (d, *J* = 1.0 Hz, 1H), 6.67 (t, *J* = 7.7 Hz, 1H), 6.26 (s, 1H), 6.19-6.10 (m, 2H), 5.63 (s, 1H), 5.45 (d, *J* = 5.4 Hz, 1H), 4.83 (s, 2H), 4.66 (t, *J* = 5.1 Hz, 1H), 4.27 (d, *J* = 14.0 Hz, 1H), 3.97 (dd, *J* = 13.9, 4.8 Hz, 1H), 3.83 (s, 3H), 3.58 (s, 3H).

Step 9: 60% NaH(21.87 g, 546.88 mmol)was added to THF (2.5 L) at 0 °C, and the mixture was stirred at 0 °C for 1 h. Subsequently, compound 2L (99.59 g, 546.88 mmol) was added dropwise, and the mixture was stirred at 0 °C for 2 h. A solution of compound 2K (100.00 g, 451.97 mmol) in THF (300 mL) was added dropwise. After the mixture was stirred at room temperature for 3 h, 10% HCl was added, and the mixture was then extracted with ethyl acetate. The organic phases were combined, dried over Na₂SO₄, filtered, and concentrated to give a crude product, and the crude product was slurried with MTBE to give 2A (120 g, yield: 95.73%) as a pale yellow solid. ¹H NMR (400 MHz, DMSO) δ 9.47 (s, 1H), 7.74 (s, 1H), 7.58 (d, J = 16.0 Hz, 1H), 7.49 (d, *J* = 7.2 Hz, 1H), 7.36-7.28 (m, 2H), 6.49 (d, *J* = 16.0 Hz, 1H), 3.73 (s, 3H), 1.49 (s, 9H).

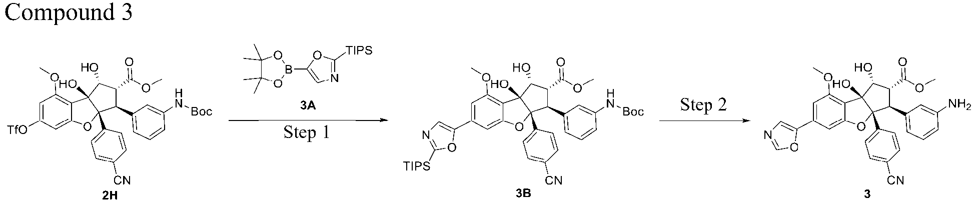

Step 1: Na₂CO₃ (48.8 mg, 0.14 mL), Pd(PPh₃)₄ (4.88 mg, 0.01 mmol), and compound 3A (28.3 mg, 0.08 mmol) were added to a solution of the racemate of compound 2H (50 mg, 0.07 mmol) in dioxane/water (2 mL/0.5 mL). The mixture was left to react at 80 °C for 2 h under a nitrogen atmosphere, filtered, and concentrated under reduced pressure, and the crude product was purified by flash column chromatography (DCM/EA = 1/1) to give a racemate of compound 3B (40 mg, yield: 76%) as a light yellow solid. LCMS: [M+H]⁺ = 796.4.

Step 2: The racemate of compound 3B (20 mg, 0.025 mmol) was dissolved in 6 M HCl (1 mL)/THF (0.5 mL), and the solution was stirred for 3 h. The reaction solution was purified by preparative HPLC (ACN/H₂O, from 0 to 100%) to give a racemate of 3 (6 mg, yield: 45%) as a white solid, and the racemate was further resolved on a chiral column to give compound 3.

LCMS: [M+H]⁺ = 540.2.

¹H NMR (400 MHz, DMSO) δ 8.46 (s, 1H), 7.79 (s, 1H), 7.51 (d, *J* = 8.4 Hz, 2H), 7.32 (d, *J* = 8.4 Hz, 2H), 7.03-6.89 (m, 3H), 6.68 (s, 3H), 4.68 (d, *J* = 4.8 Hz, 1H), 4.34 (d, *J* = 13.6 Hz, 1H), 4.09 (dd, *J* = 14.0, 4.8 Hz, 1H), 3.83 (s, 3H), 3.59 (s, 3H).

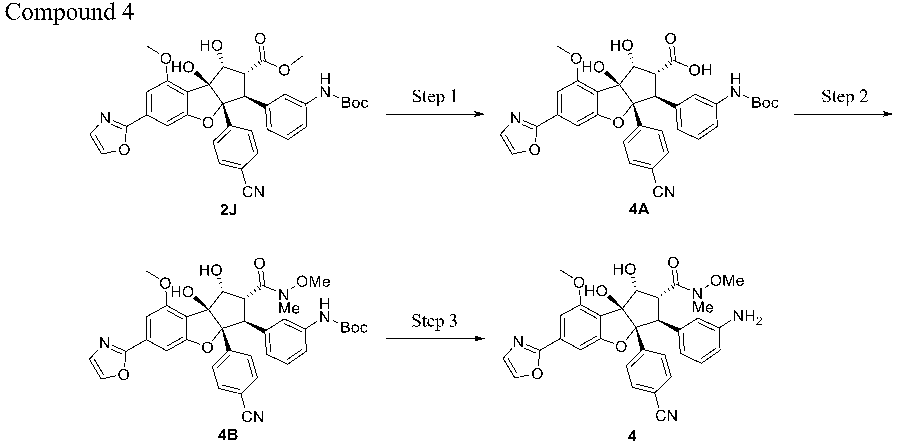

Step 1: A solution of the racemate of compound 2J (150 mg, 0.23 mmol) and LiOH (25 mg, 0.59 mmol) in THF/H₂O/MeOH (10 mL) was stirred at room temperature for 3 h. The pH was adjusted to 5 with 3 M hydrochloric acid, and the solution was concentrated and purified by silica gel column chromatography (DCM/MeOH) to give a racemate of 4A (130 mg, yield: 88%) as a white solid. LCMS [M+1]⁺ = 626.1.

Step 2: HATU (61 mg, 0.16 mmol) and DIEA (31 mg, 0.24 mmol) were added to a solution of the racemate of compound 4A (50 mg, 0.08 mmol) in DMF (3 mL), and N,O-dimethylhydroxylamine (16 mg, 0.16 mmol) was added under a N₂ atmosphere. The mixture was stirred at room temperature for 3 h, concentrated, and purified by silica gel column chromatography (DCM/MeOH) to give a racemate of 4B (20 mg, yield: 38%) as a white solid. LCMS [M+1]⁺ = 669.2

Step 3: The racemate of compound 4B (12 mg, 0.02 mmol) was dissolved in a solution of THF/6 M HCl (1/1, 4 mL). The resulting solution was stirred at room temperature for 1 h, concentrated, and purified by silica gel column chromatography (DCM/MeOH) to give a racemate of 4 (2.2 mg, yield: 22%) as a white solid, and the racemate was further resolved on a chiral column to give 4 as a white solid.

LCMS [M+H]⁺ = 569.1.

¹H NMR (400 MHz, DMSO) δ 8.25 (s, 1H), 7.53 (d, J = 8.5 Hz, 2H), 7.41 (s, 1H), 7.31 (d, J = 8.5 Hz, 2H), 7.20 (d, J = 0.9 Hz, 1H), 7.15 (s, 1H), 6.66 (t, J = 7.7 Hz, 1H), 6.21 (s, 1H), 6.16 (d, J = 7.9 Hz, 1H), 6.06 (d, J = 7.5 Hz, 1H), 5.61 (s, 1H), 5.07 (d, J = 4.8 Hz, 1H), 4.83 (s, 2H), 4.78 (t, J = 4.8 Hz, 1H), 4.32 (d, J = 13.9 Hz, 1H), 4.16 (s, 1H), 3.90 (s, 3H), 3.84 (s, 3H), 3.09 (s, 3H).

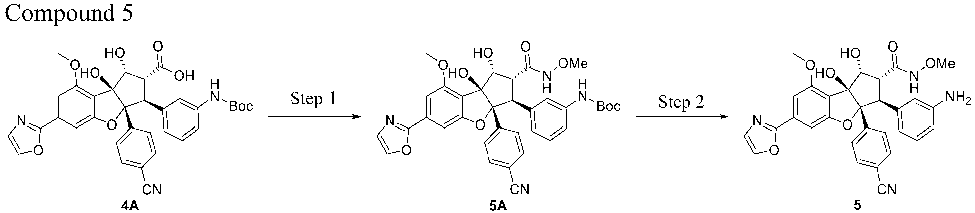

Step 1: HATU (36.4 mg, 0.1 mmol) and DIEA (18.6 mg, 0.14 mmol) were mixed with a solution of the racemate of compound 4A (30 mg, 0.05 mmol) in DMF (3 mL) under N₂, and O-methylhydroxylamine (8 mg, 0.10 mmol) was then added. The mixture was stirred at room temperature for 3 h, concentrated, and purified by silica gel column chromatography (DCM/MeOH) to give a racemate of 5A (20 mg, yield: 65%) as a white solid. LCMS [M+1]⁺ = 655.2.

Step 2: The racemate of compound 5A (12 mg, 0.03 mmol) was dissolved in a solution of THF/6 M HCl (1/1, 4 mL). The resulting solution was stirred at room temperature for 1 h, concentrated, and purified by silica gel column chromatography (DCM/MeOH) to give a racemate of 5 (2.6 mg, yield: 31%) as a white solid, and the racemate was further resolved on a chiral column to give compound 5.

LCMS [M+H]⁺ = 555.1.

¹H NMR (400 MHz, DMSO) δ 11.27 (s, 1H), 8.26 (s, 1H), 7.54 (d, J = 8.5 Hz, 2H), 7.42 (s, 1H), 7.30 (d, J = 8.3 Hz, 2H), 7.20 (s, 1H), 7.16 (s, 1H), 7.11 (t, J = 7.5 Hz, 1H), 6.87 (d, J = 18.2 Hz, 3H), 5.65 (s, 1H), 5.27 (s, 1H), 4.60 (d, J = 4.4 Hz, 1H), 4.46 (d, J = 13.9 Hz, 1H), 3.84 (s, 3H), 3.74 (dd, J = 14.1, 4.1 Hz, 1H), 3.54 (s, 3H).

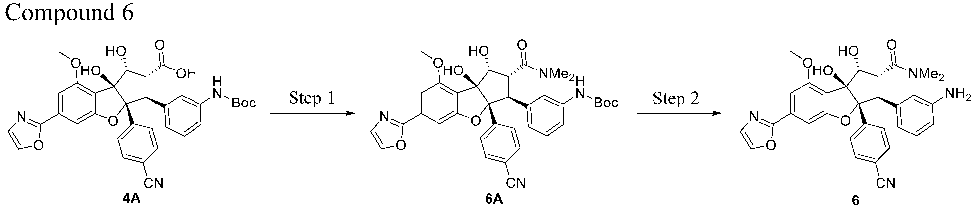

Step 1: The racemate of compound 4A (30.0 mg, 0.05 mmol), HATU (36.5 mg, 0.10 mmol), and DIEA (18.6 mg, 0.14 mmol) were stirred in DMF (3 mL) for 5 min. Dimethylamine hydrochloride (7.8 mg, 0.10 mmol) was added to the mixture, and the resulting mixture was stirred for 10 min. The solution was extracted with EA twice. The organic layer was washed with saturated brine, dried over Na₂SO₄, concentrated under reduced pressure, and purified by silica gel column chromatography (DCM/MeOH = 10/1) to give a racemate of compound 6A (16.0 mg, yield: 51%). LCMS [M+H]⁺ = 653.2.

Step 2: The racemate of compound 6A (16.0 mg, 0.02 mmol) was mixed with THF (0.5 mL), and 6 M HCl (0.5 mL) was added at 0 °C. The mixture was stirred at room temperature for 4 h, concentrated, and purified by preparative high-performance liquid chromatography to give a racemate of 6 (5.9 mg, yield: 46%) as a white solid, and the racemate was further resolved on a chiral column to give compound 6.

LCMS [M+H]⁺ = 553.2.

¹H NMR (400 MHz, DMSO) δ 8.25 (s, 1H), 7.51 (d, J = 8.4 Hz, 2H), 7.41 (s, 1H), 7.35 (d, J = 8.4 Hz, 2H), 7.18 (d, J = 19.6 Hz, 2H), 6.73 (d, J = 7.4 Hz, 1H), 6.40-6.17 (m, 3H), 5.62 (s, 1H), 4.98 (s, 1H), 4.73 (s, 1H), 4.39 (d, J = 13.4 Hz, 1H), 4.15 (dd, J = 13.3, 4.9 Hz, 1H), 3.84 (s, 3H), 3.29 (s, 3H), 2.79 (s, 3H).

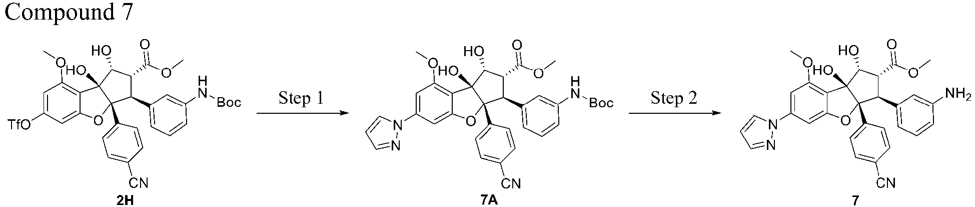

Step 1: The racemate of compound 2H (50 mg, 2.2 mmol), pyrazole (3.7 g, 13.3 mmol), tBuXPhos-Pd-G3 (4.8 mg, 0.01 mmol), tBuXPhos (12.78 mg, 0.03 mmol), and Cs₂CO₃ (67 mg, 0.21 mmol) were dissolved in 1,4-dioxane (2 mL). The reaction mixture was stirred at 100 °C for 16 h under a nitrogen atmosphere. After the reaction was complete, the reaction mixture was quenched with water and extracted with EA, followed by liquid separation. The organic phase was concentrated and then purified by silica gel column chromatography (DCM/EA = 1/1) to give a racemate of 7A (10 mg, yield: 20%) as a pale yellow solid. LCMS [M+H]⁺ = 639.2.

Step 2: The racemate of compound 7A (10 mg, 0.016 mmol) was dissolved in THF (0.5 mL), and 6 M HCl (0.5 mL) was added. The mixture was stirred at room temperature for 4 h. The reaction solution was purified by flash column chromatography (ACN-H₂O = 0-100%) to give a racemate of 7 (4 mg, yield: 47%) as a white solid, and the racemate was further resolved on a chiral column to give compound 7.

LCMS [M+H]⁺ = 539.1.

1H NMR (400 MHz, DMSO) δ 8.58 (d, J = 2.4 Hz, 1H), 7.75 (d, J = 1.6 Hz, 1H), 7.51 (d, J = 8.4 Hz, 2H), 7.31 (d, J = 8.4 Hz, 2H), 7.13 (d, J = 1.6 Hz, 1H), 7.05 (d, J = 1.6 Hz, 1H), 6.68 (t, J = 7.6 Hz, 1H), 6.58-6.54 (m, 1H), 6.23 (s, 1H), 6.14 (m, 2H), 5.54 (s, 1H), 5.39 (d, J = 5.2 Hz, 1H), 4.82 (s, 2H), 4.64 (t, J = 5.2 Hz, 1H), 4.26 (d, J = 14.0 Hz, 1H), 3.95 (m, 1H), 3.82 (s, 3H), 3.58 (s, 3H).

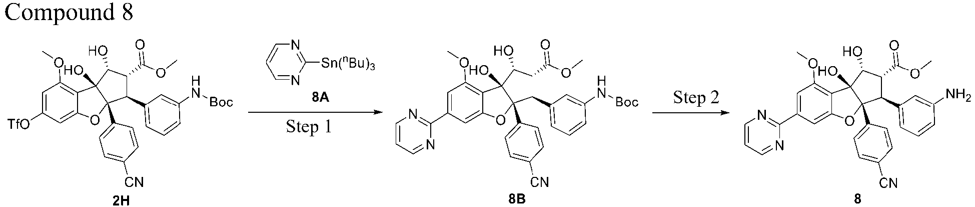

Step 1: Pd(PPh₃)₄ (9 mg, 0.0075 mmol) and CuI (28.5 mg, 0.015 mmol) were added to a solution of the racemate of compound 2H (54 mg, 0.075 mmol) and compound 8A (215 mg, 0.6 mmol) in DMF (5 mL). Then, the mixture was purged with nitrogen for 2 min, left to react under microwave conditions at 120 °C for 2 h, and concentrated, and the resulting crude product was purified by preparative column chromatography to give a racemate of 8B (20 mg, 40.9%) as a white solid.

LCMS: [M+H-C₄H₈]⁺ = 595.2.

Step 2: An aqueous HCl solution (0.5 mL, 6 M) was added to a solution of the racemate of compound 8B (20 mg, 0.0308 mmol) in THF (1 mL). The reaction solution was left to react at room temperature for 3 h and concentrated, and the resulting crude product was purified by preparative HPLC to give a racemate of 8 (9 mg, yield: 53.1%) as a white solid, and the racemate was further resolved on a chiral column to give compound 8.

LCMS: [M+H]⁺ = 551.2.

¹H NMR (400 MHz, DMSO) δ 9.53 (s, 2H), 8.94 (d, J = 4.9 Hz, 2H), 7.64 (d, J = 2.8 Hz, 2H), 7.55-7.43 (m, 3H), 7.36 (d, J = 8.6 Hz, 2H), 7.14 (t, J = 7.6 Hz, 1H), 7.06-6.82 (m, 3H), 5.66 (d, J = 58.4 Hz, 2H), 4.74 (d, J = 4.6 Hz, 1H), 4.41 (d, J = 13.8 Hz, 1H), 4.18 (d, J = 4.8 Hz, 1H), 3.86 (s, 3H), 3.60 (s, 3H).

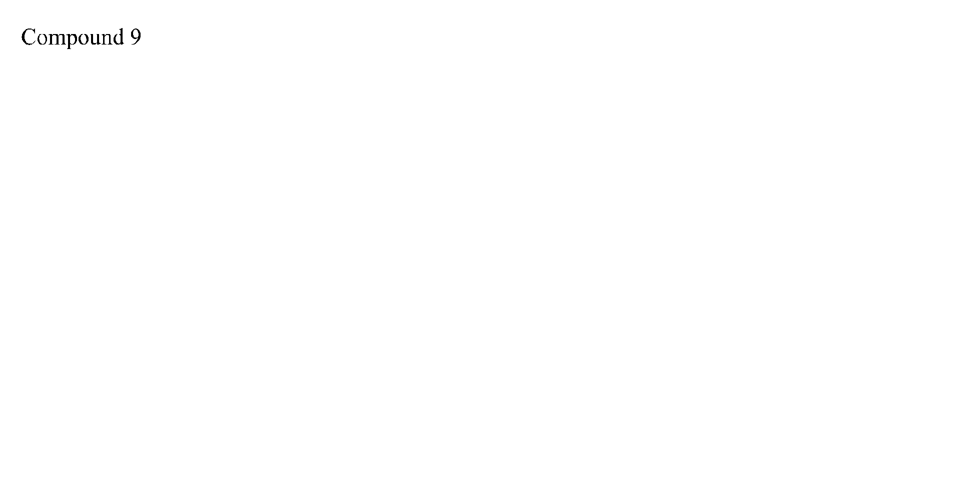

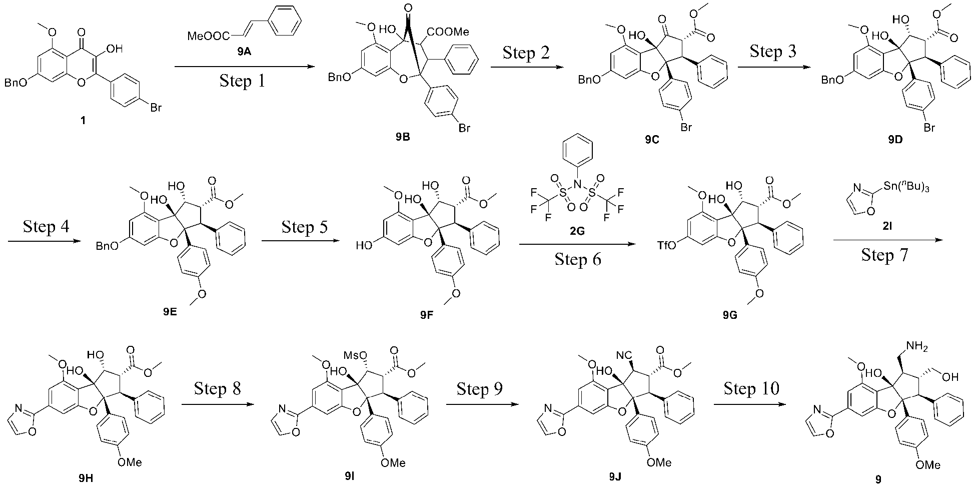

Step 1: After compound 1 (1.00 g, 2.21 mmol) and compound 9A (3.58 g, 22.08 mmol) were dissolved in a solution of TFE/trichloromethane (CHCl₃/TFE = 9/1, 18 mL), the solution was drawn into a 20 mL syringe mounted on a syringe pump, and the syringe pump was connected to an FEP tube. The FEP tube was sequentially wound around two quartz column sleeves, with a 250 W ultraviolet lamp tube in each quartz column sleeve. The two column sleeves were cooled to - 5 °C using a low-temperature circulation pump, and a room-temperature water bath was added outside the entire lamp-column sleeve system. After the ultraviolet lamps were stably activated, the syringe pump was started to inject the reaction solution into the FEP tube at 30 mL/h, and the reaction solution was subjected to stable irradiation for 1 h. After the reaction was complete, the reaction solution was concentrated and separated by column chromatography (ethyl acetate/petroleum ether = 3/1) to remove the excess cinnamate starting material, giving a crude product. The crude product was diluted with ethyl acetate, and the mixture was left to react at 65 °C for 1 h and then concentrated to give a racemate of the target product 9B (crude, 0.96 g, yield: 71%) as a yellow oil.

LCMS [M+H]⁺ = 615.5.

Step 2: The racemate of compound 9B (1.4 g, 2.27 mmol) was dissolved in methanol (100 mL), and NaOMe (0.3 g, 5.7 mmol) was then added at 0 °C. Then, the reaction solution was left to react at 65 °C for 1 h, concentrated, and then separated by column chromatography (0.5% formic acid in ethyl acetate/petroleum ether = 1/3) to give a racemate of the target product 9C (0.96 g, yield: 68%) as a yellow oil.

LCMS [M+H]⁺ = 615.1.

Step 3: The racemate of compound 9C (0.60 g, 0.977 mmol) and AcOH (1.06 g, 17.586 mmol) were dissolved in MeCN (40 mL) and DCM (40 mL), and the solution was then cooled to 0 °C and placed under a N₂ atmosphere. STAB (1.04 g, 4.89 mmol) was added in portions, and the mixture was stirred at room temperature for 1 h. The reaction solution was quenched with water. An aqueous NaHCO₃ solution was added, and the mixture was extracted with DCM twice. The organic layer was dried, concentrated under reduced pressure, and purified by silica gel column chromatography (PE:EA) to give a racemate of 9D (0.32 g, yield: 53%) as a white solid.

LCMS: [M+H]⁺ = 617.2.

Step 4: The racemate of compound 9D (2.00 g, 3.24 mmol), Cs₂CO₃ (1.58 g, 4.86 mmol), and MeOH (0.21 g, 6.48 mmol) were dissolved in toluene (30 mL), and tBuXPhos Pd G3 (51 mg, 0.06 mmol) was then added. The mixture was heated to 85 °C and left to react for 3 h. The reaction solution was concentrated, diluted with a solution of MeOH/DCM (MeOH/DCM = 1/1), and filtered. The filtrate was concentrated and then separated by column chromatography (EA/PE = 60%) to give a racemate of the target product 9E (810 mg, yield: 44%) as a brown oil.

LCMS: [M-H]⁻ = 567.0.

Step 5: The racemate of compound 9E (300 mg, 0.53 mmol) and Pd/C (10%, 150 mg) were dissolved in MeOH (15 mL), and the solution was then left to react at room temperature for 2 h under a H₂ atmosphere. The reaction solution was filtered, and the filtrate was concentrated and then separated by column chromatography to give a racemate of the target product 9F (270 mg, crude, yield not calculated) as a white solid.

LCMS: [M+H]⁺ = 479.4.

Step 6: The racemate of compound 9F (360 mg, 0.75 mmol), compound 2G (268 mg, 0.75 mmol), and K₂CO₃ (207 mg, 1.5 mmol) were added to 5 mL of DMF, and the mixture was stirred at 60 °C for 1.5 h, cooled to room temperature, and extracted with ethyl acetate and water. The organic phase was isolated, dried over anhydrous Na₂SO₄, filtered, and then concentrated. The crude product was purified by silica gel column chromatography (EA/DCM, from 0 to 50%) to give a racemate of 9G (360 mg, yield: 78%) as a yellow solid.

LCMS: [M-H]⁻ = 609.2.

Step 7: The racemate of compound 9G (340 mg, 0.56 mmol) was dissolved in DMF (5 mL), and compound 2I (1600 mg, 4.46 mmol), Pd(PPh₃)₄ (65 mg, 0.06 mmol), and CuI (21 mg, 0.11 mmol) were added. The mixture was left to react at 110 °C for 16 h under a nitrogen atmosphere. The reaction solution was cooled to room temperature and directly purified by silica gel column chromatography (PE/EA = 1/1) to give a racemate of 9H (180 mg, yield: 61%) as a white solid.

LCMS [M+H]⁺ = 530.0.

Step 8: MsCl (171 mL, 1.5 mmol) was added to a mixture of the racemate of compound 9H (80 mg, 0.15 mmol) and pyridine (0.5 mL) at 0 °C, and the resulting mixture was left to react at 0 °C for 1 h, diluted with ethyl acetate (5 mL), and washed with 1 M HCl (5 mL), followed by liquid separation. The organic phase was purified by silica gel column chromatography (DCM/EA = 1/1) to give a racemate of 9I (90 mg, yield: 97%) as a white solid.

LCMS [M+H]⁺ = 608.1.

Step 9: The racemate of compound 9I (90 mg, 0.15 mmol) was dissolved in DMF (0.5 mL), and NaCN (36 mg, 0.75 mmol) was added. The mixture was stirred at room temperature for 16 h and extracted with ethyl acetate. The organic phase was isolated, dried over anhydrous Na₂SO₄, filtered, and then concentrated. The crude product was purified by silica gel column chromatography (DCM/EA = 1/1) to give a racemate of 9J (70 mg, yield: 88%) as a white solid.

LCMS [M+H]⁺ = 539.1.

Step 10: The racemate of compound 9J (10 mg, 0.016 mmol) was dissolved in THF (0.5 mL), and a 2 M solution of lithium aluminum hydride in THF (0.08 mL) was added at 0 °C. The mixture was slowly warmed to room temperature and stirred for 1 h. The reaction solution was quenched with Na₂SO₄·10H₂O, and the resulting mixture was filtered. The filtrate was purified by preparative medium-pressure column chromatography (ACN/H₂O = 0%-100%, containing 0.1% formic acid) to give a racemate of 9 (7 mg, yield: 70%) as a white solid, and the racemate was further resolved on a chiral column to give compound 9.

LCMS [M+H]⁺ = 515.1.

¹H NMR (400 MHz, DMSO) δ 8.26 (s, 1H), 7.42 (s, 1H), 7.22 (d, J = 4.8 Hz, 2H), 7.13 (d, J = 8.4 Hz, 2H), 7.09-7.04 (m, 3H), 6.81-6.76 (m, 2H), 6.71 (d, J = 8.8 Hz, 2H), 3.97 (s, 3H), 3.66 (s, 3H), 3.23 (d, J = 13.6 Hz, 5H), 3.13 (m, 1H), 2.93 (s, 1H).

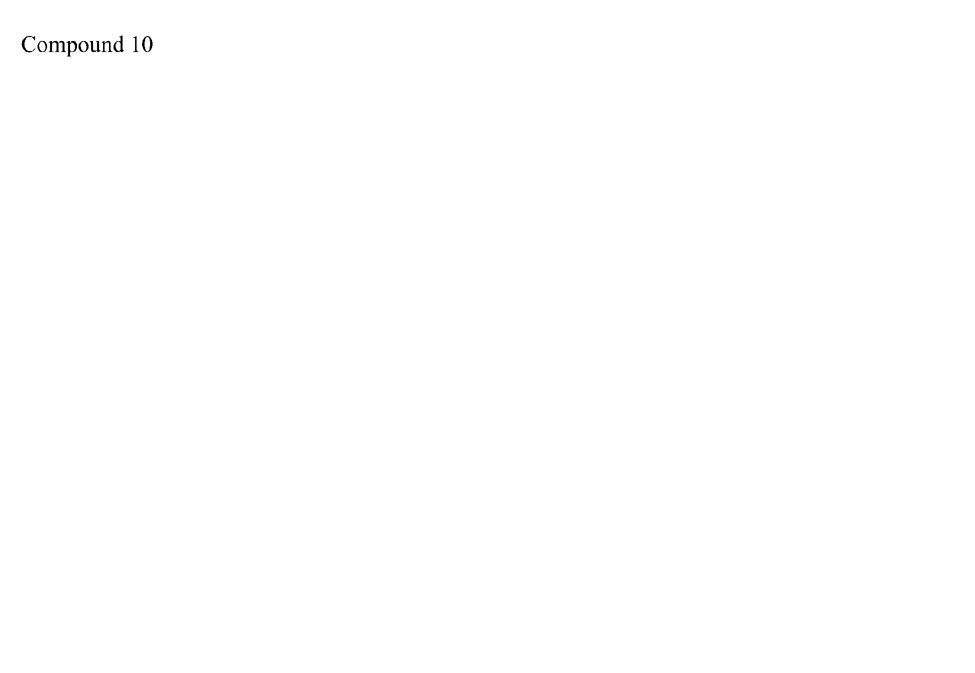

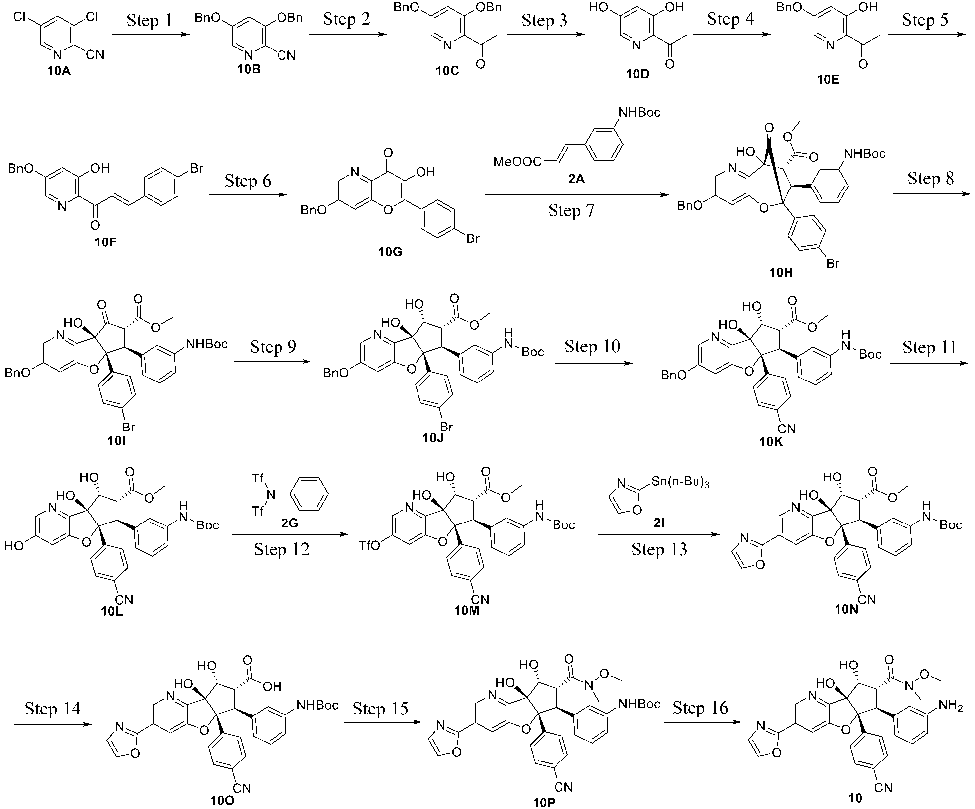

Step 1: NaH (9.06 g, 234.6 mmol) was slowly added to a solution of 10A (12.11 g, 70 mmol) in THF (120 mL) at 0 °C. The mixture was stirred at room temperature for 0.5 h. Then, BnOH (16.2 g, 150 mmol) was added, and the mixture was stirred at room temperature for 3 h, slowly quenched with water at 0 °C, and extracted with EtOAc. The organic phase was isolated, dried over anhydrous Na₂SO₄, filtered, and concentrated under reduced pressure to give compound 10B (19.6 g, yield: 89%). LCMS [M+H]⁺ = 317.2.

Step 2: A MeMgBr solution (124.9 mL, 124.9 mmol) was slowly added dropwise to a solution of 10B (13.16 g, 41.6 mmol) in THF (150 mL) at -30 °C. Then, the mixture was stirred at room temperature for 4.3 h, quenched with 6 N HCl, and stirred for another 2 h. The organic phase was washed with 1 N NaOH and water, isolated, dried over anhydrous Na₂SO₄, filtered, and concentrated under reduced pressure. The crude product was purified by silica gel column chromatography to give compound 10C (7.12 g, yield: 46%). LCMS [M+H]⁺ = 334.2.

Step 3: Compound 10C (8.5 g, 25.5 mmol) was dissolved in EA (60 mL), and Pd(OH)₂ (850 mg) was added. The mixture was stirred at room temperature for 18 h under a hydrogen atmosphere and concentrated to give 10D (3.9 g, yield: 100%) as a white solid. LCMS [M+H]⁺ = 154.2. Step 4: BnBr (4.36 g, 25.5 mmol) was added dropwise to a solution of compound 10D (3.9 g, 25.5 mmol) and K₂CO₃ (9.85 g, 71.4 mmol) in acetone (40 mL), and the mixture was stirred at room temperature for 48 h, acidified with acetic acid, diluted with water, and extracted with EA. The organic layer was concentrated and washed with petroleum ether to give 10E (4.80 g, yield: 77%) as a yellowish-white solid. LCMS [M+H]⁺ = 244.1.

Step 5: NaOH (2.368 g, 59.2 mmol) and 4-bromobenzaldehyde (3.65 g, 19.73 mol) were added to a solution of compound 10E (4800 mg, 19.73 mmol) in MeOH (60 mL). The reaction solution was stirred at 70 °C for 18 h, cooled, acidified with AcOH, and extracted with dichloromethane. The organic layer was washed with water and brine, dried, and concentrated. The crude product was slurried with PE/EA to give 10F (5.0 g, yield: 60%) as a white solid. LCMS [M+H]⁺ = 410.2. Step 6: NaOH (785 mg, 19.6 mmol, 7.0 eq) and H₂O₂ (12.5 mL) were added to a solution of compound 10F (1150 mg, 2.8 mmol, 1.0 eq) in MeOH/H₂O (130 mL/65 mL). The reaction solution was stirred at 50 °C for 3 h. The mixture was cooled and filtered. The solid was washed with a small amount of dichloromethane and acidified with AcOH, and extraction was performed with DCM. The organic layer was washed with water and brine, dried, and concentrated to give 10G (670 mg, yield: 57%) as a white solid. LCMS [M+H]⁺ = 424.0.

¹H NMR (400 MHz, DMSO) δ 9.99 (s, 1H), 8.56 (d, *J =* 2.4 Hz, 1H), 8.18 (d, *J =* 8.8 Hz, 2H), 7.94 (d, *J =* 2.4 Hz, 1H), 7.80 (d, *J* = 8.8 Hz, 2H), 7.60-7.37 (m, 5H), 5.34 (s, 2H).

Step 7: Compound 10G (2.5 g, 5.9 mmol) and compound 2A (9.8 g, 35.38 mmol) were dissolved in CHCl₃/TFE (7/3, 20 mL). A syringe was used to inject the reaction mixture at a rate of 12 mL/h through a syringe pump into a coil wound around a jacketed glass cylinder equipped with a 250 W ultraviolet lamp. The temperature of the external circulation cold trap was adjusted to keep the internal reaction temperature at 0-5 °C, and the UV lamp was turned on simultaneously. The reaction mixture was irradiated for 1 h and concentrated under reduced pressure to remove the solvent, and the residue was purified by silica gel column chromatography (EtOAc/hexane) to remove the excess cinnamate, giving a racemate of the product 10H (3.6 g, yield: 75%) as a light yellow oil. LCMS [M+H]⁺ = 700.9.

Step 8: The racemate of compound 10H (2.5 g, 3.56 mmol) was dissolved in methanol (60 mL), and NaOMe (481 mg, 8.91 mmol) was then added at 0 °C. Subsequently, the reaction solution was stirred at 65 °C for 1.5 h. After the reaction was complete, the reaction solution was concentrated, extracted with EA, washed with water, an NH₄Cl solution, and saturated brine, dried over anhydrous sodium sulfate, concentrated under reduced pressure, and finally purified by silica gel column chromatography (0.2% FA, EA/PE = 1/3) to give a racemate of compound 101 (3.0 g, crude product). LCMS [M+H]⁺ = 701.0.

Step 9: The racemate of compound 101 (3.0 g, 4.28 mmol) was dissolved in a mixed solvent of ACN/CHCl₃ (1/1, 40 mL/40 mL), and Na(AcO)₃BH (4.533 g, 21.38 mmol) and AcOH (2.566 g, 47.0 mmol) were added at 0 °C. Then, the reaction solution was stirred at room temperature for 2 h. After the reaction was complete, the reaction solution was concentrated, extracted with EA, washed with water and saturated brine, dried over anhydrous sodium sulfate, concentrated under reduced pressure, and finally purified by silica gel column chromatography (0.2% FA, EA/PE = 3/2) to give a racemate of compound 10J (500 mg). LCMS [M+H]⁺ = 703.0.

¹H NMR (400 MHz, CDCl₃) δ 8.06 (s, 1H), 7.86 (d, J = 1.9 Hz, 1H), 7.43 (d, J = 4.4 Hz, 4H), 7.40 (dd, J = 8.2, 5.0 Hz, 2H), 7.28 (s, 2H), 7.15 (d, J = 8.5 Hz, 1H), 7.10 (d, J = 2.0 Hz, 1H), 7.06 (d, J = 8.7 Hz, 2H), 7.00 (t, J = 7.9 Hz, 1H), 6.90 (s, 1H), 6.57 (d, J = 7.6 Hz, 1H), 6.35 (s, 1H), 5.14 (s, 2H), 5.06 (d, J = 6.0 Hz, 1H), 4.41 (d, J = 13.9 Hz, 1H), 3.95 (dd, J = 14.0, 6.0 Hz, 1H), 3.67 (s, 3H), 1.51 (s, 9H).

Step 10: Pd₂(dba)₃ (128 mg, 0.14 mmol) was added to a solution of the racemate of compound 10J (500 mg, 0.71 mmol), DPPF (155 mg, 0.28 mmol), and Zn(CN)₂ (208 mg, 1.80 mmol) in NMP (25 mL). The reaction solution was heated to 150 °C and left to react for 1.2 h under a N₂ atmosphere, diluted with water, and extracted with EA. Then, the organic layer was washed with water and brine, dried, concentrated, and purified by silica gel column chromatography (EA/PE = 1/1) to give aracemate of10K (300 mg, yield: 65%) as a yellow solid. LCMS: [M+H]⁺ = 650.0. Step 11: Pd(OH)₂/C (10%, 0.1 g) was added to a solution of theracemate of compound 10K (300 mg, 0.46 mol) in EA/MeOH/THF/DCM (20:2:2:0.2, 7 mL). The mixture was stirred at room temperature for 18 h under a hydrogen atmosphere and filtered to remove the solid, and the filter cake was washed with MeOH/DCM (1/10). The combined filtrates were concentrated to give a racemate of 10L (0.22 g, yield: 87.0%) as a white solid. LCMS: [M+H]⁺ = 560.2.

Step 12: K₂CO₃ (116 mg, 0.78 mmol) was added to a solution of the racemate of compound 10L (220 mg, 0.39 mmol) and compound 2G (150 mg, 0.39 mmol) in DMF (3 mL). The mixture was stirred at 60 °C for 1 h, quenched with water, and extracted with EtOAc. The organic phase was isolated, dried over anhydrous Na₂SO₄, filtered, and concentrated. The residue was purified by column chromatography (EA-PE/DCM = 0-50%) to give aracemate of10 M (221 mg, yield: 82%) as a yellow solid. LCMS [M+H-C4H8]⁺ = 636.1.

Step 13: The racemate of compound 10M (70 mg, 0.1 mmol), compound 2I (280 mg, 0.8 mmol), Pd(PPh₃)₄ (12 mg, 0.01 mmol), and CuI (4 mg, 0.02 mmol) were added to DMF (3 mL), and the mixture was then stirred under microwave conditions at 135 °C for 5 h under a N₂ atmosphere. Water was added, and the mixture was extracted with EA. The organic layer was washed with water and saturated brine and then dried and concentrated. The crude product was dissolved in DCM, and Boc₂O (2 eq), DIPEA (3 eq), and DMAP (0.2 eq) were added. The mixture was stirred at 40 °C for 18 h, concentrated, and purified by flash chromatography (DCM/EA = 1:1, DCM:MeOH = 20:1) to give a racemate of 10N (60 mg, yield: 98%) as a white solid. LCMS [M+H]⁺ = 611.2.

Step 14: H₂O (2 mL), MeOH (2 mL), and LiOH (12.6 mg, 0.30 mmol) were added to a solution of the racemate of compound 10N (60 mg, 0.1 mmol) in THF (2 mL). Then, the mixture was stirred at room temperature for 5 h and purified by reversed-phase chromatography (MeCH/HCO₂H/H₂O) to give a racemate of 10O (30 mg, yield: 51%) as a white solid. LCMS [M+H]⁺ = 597.2.

Step 15: The racemate of compound 10O (30 mg, 0.048 mmol), N,O-dimethylhydroxylamine (9 mg, 0.096 mmol), HATU (18 mg, 0.048 mmol), and TMP (24 mg, 0.192 mmol) were added to DMF (1 mL). Then, the solution was stirred at 50 °C for 18 h and purified by reversed-phase column chromatography (ACN/H₂O = 0%-100%) to give a racemate of 10P (10 mg, yield: 33%) as a white solid. LCMS: [M+H]⁺ = 640.3.

Step 16: 6 M HCl (0.5 mL) was added dropwise to a solution of the racemate of compound 10P (10 mg, 0.016 mmol) in THF (0.5 mL), and the solution was then stirred at room temperature for 4 h. The reaction solution was separated by preparative reversed-phase column chromatography (ACN/H₂O = 5%-95%, +0.1% NH₄HCO₃) to give a racemate of 10 (1.1 mg, yield: 10%) as a white solid, and the racemate was further resolved on a chiral column to give compound 10.LCMS: [M+H]⁺ = 540.2.

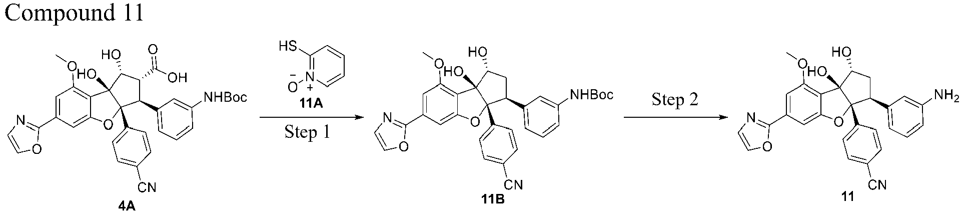

Step 1: A solution of theracemate of compound 4A (10 mg, 0.02 mmol), compound 11A (12.7 mg, 0.1 mmol),tert-dodecyl mercaptan (38.4 mg, 0.2 mmol), TMP (0.4 mg, 0.04 mmol), and zinc meso-tetraphenylporphine (1.3 mg, 0.001 mmol) in DMF (1 mL) was transferred to a microwave tube. The microwave tube was irradiated using a 25 W red light lamp, with the temperature kept at 80 °C. EDCI (36.6 mg, 0.2 mmol, dissolved in 1 mL of DMF) was added dropwise to the reaction system. The reaction solution was continuously stirred at 80 °C under 25 W red light lamp irradiation for 30 min. The reaction solutions from three parallel reactions were combined and then purified by reversed-phase column chromatography (H₂O:ACN = 0-100%) to give aracemate of11B (9 mg, yield: 33%) as a white solid. LCMS: [M+H-C₄H₈]⁺ = 526.2.

Step 2: 6 M HCl (1 mL) was added to a solution of theracemate of compound 11B (25 mg, 0.04 mmol) in THF (1 mL). The reaction solution was stirred overnight at room temperature, concentrated under reduced pressure, and then purified by reversed-phase column chromatography (H₂O:ACN = 0-100%) to give aracemate of 11 (9 mg, yield: 40%) as a white solid, and the racemate was further resolved on a chiral column to give compound 11. LCMS [M+H]⁺ = 482.0.

¹H NMR (400 MHz, DMSO) δ 8.25 (s, 1H), 7.53 (d, *J =* 8.5 Hz, 2H), 7.41 (s, 1H), 7.30 (d, *J= 8.5* Hz, 2H), 7.16 (d, *J* = 12.3 Hz, 2H), 6.73 (t, *J* = 7.7 Hz, 1H), 6.35 (s, 1H), 6.22 (t, *J* = 7.7 Hz, 2H), 5.34 (s, 1H), 4.90-4.73 (m, 3H), 4.47 (s, 1H), 4.03 (dd, *J=* 14.3, 6.1 Hz, 1H), 3.85 (s, 3H), 2.78-2.60 (m, 1H), 2.08 (dd, *J =* 12.8, 6.1 Hz, 1H).

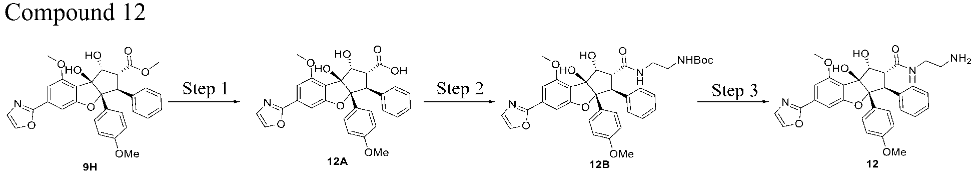

Step 1: Theracemate of compound 9H (42.3 mg, 0.08 mmol) and LiOH (16.8 mg, 0.4 mmol) were dissolved in MeOH/H₂O (5 mL/1 mL), and the solution was stirred at room temperature for 12 h. The pH was adjusted to 2-3 with a 1 M aqueous hydrochloric acid solution, and the mixture was extracted with ethyl acetate (20 mL). The organic phase was dried over sodium sulfate and concentrated to give aracemate of 12A (35 mg, yield: 84.6%) as a white solid. LCMS [M+H]⁺ = 516.2.

Step 2: The racemate of compound 12A(31 mg, 0.06 mmol) was mixed with Boc-ethylenediamine (19 mg, 0.12 mmol), 2,4,6-trimethylpyridine (22 mg, 0.18 mmol), and HATU (34 mg, 0.09 mmol) in DMF (3 mL), and the mixture was stirred at room temperature for 12 h. Then, 50 mL of water was added to the reaction solution, and the mixture was extracted with ethyl acetate, washed with saturated brine, dried, and concentrated. The crude product was purified by silica gel column chromatography (PE/EA = 1/2) to give aracemate of12B (33 mg, yield: 83.7%) as a white solid. LCMS [M+H]⁺ = 658.2.

Step 3: An aqueous HCl solution (1 mL, 6 M) was added to a solution of theracemate of compound 12B (33 mg, 0.05 mmol) in THF (3 mL), and the reaction solution was left to react at room temperature for 12 h and concentrated. The resulting crude product was purified by preparative column chromatography to give aracemate of12 (20 mg, yield: 71.4%) as a white solid, and the racemate was further resolved on a chiral column to give compound 12.

LCMS [M+H]⁺ = 558.2.

¹H NMR (400 MHz, DMSO) δ 8.45 (s, 2H), 8.24 (d, *J =* 0.6 Hz, 1H), 7.40 (d, *J =* 0.6 Hz, 1H), 7.19 (d, *J =* 1.0 Hz, 1H), 7.14 (d, *J =* 0.9 Hz, 1H), 7.10-7.01 (m, 4H), 6.97 (dd, *J =* 10.1, 7.4 Hz, 3H), 6.59 (d, *J =* 9.0 Hz, 2H), 5.32 (s, 1H), 4.65 (d, *J* = 4.8 Hz, 1H), 4.30 (d, *J =* 14.1 Hz, 1H), 3.90-3.86 (m, 1H), 3.84 (s, 3H), 3.59 (s, 3H), 3.14 (s, 2H), 2.65 (s, 2H).

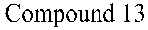

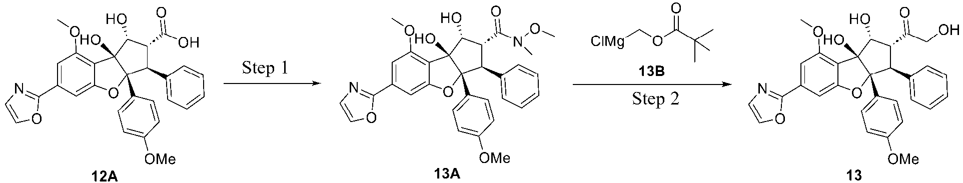

Step 1: The racemate of compound 12A (350 mg, 0.68 mmol) was dissolved in DMF (2 mL), and N,O-dimethylhydroxylamine hydrochloride (132.5 mg, 1.36 mmol), HATU (310 mg, 0.82 mmol), and DIEA (263.2 mg, 2.04 mmol) were then sequentially added. The reaction solution was stirred at room temperature for 2 h, then directly concentrated to dryness by rotary evaporation, and purified by reversed-phase chromatography (acetonitrile/water = 5%-95%, 214 nm, 20 min) to give a racemate of 13A (300 mg, yield: 79%) as a white solid. LCMS [M+Na]⁺ = 581.2.

Step 2: Iodomethyl pivalate (2000 mg, 8.26 mmol) was dissolved in a solution of anhydrous tetrahydrofuran (15 mL), and isopropylmagnesium chloride (2.0 M, 8.26 mL, 16.52 mmol) was added at -70 °C. The reaction mixture was stirred at -70 °C for 2 h to give a solution of compound 13B. The racemate of compound 13A (100 mg, 0.18 mmol) was dissolved in 1 mL of anhydrous tetrahydrofuran, and the solution of compound 13B (5 mL) was added at -70 °C. The reaction mixture was stirred overnight at room temperature, diluted with water (15 mL), extracted with dichloromethane (15 mL × 2), washed with brine (15 mL × 2), dried over anhydrous sodium sulfate, filtered, and concentrated. The crude product was purified by preparative column chromatography to give a racemate of 13 (5.5 mg, yield: 5.8%) as a white solid, and the racemate was further resolved on a chiral column to give compound 13. LCMS [M+H]⁺ = 530.0.

¹H NMR (400 MHz, DMSO-*d*₆) δ 8.25 (d, *J =* 0.8 Hz, 1H), 7.43-7.41 (m, 3H), 7.37-7.35 (m, 2H), 7.31-7.27 (m, 2H), 7.22-7.24 (m, 1H), 7.17 *(d, J=* 1.2 Hz, 1H), 7.12 (d, *J =* 1.2 Hz, 1H), 6.93-6.91 (m, 2H), 5.40 (s, 1H), 4.83 (d, *J= 4.8* Hz, 1H), 4.40-4.36 (m, 1H), 3.80 (s, 3H), 3.76-3.73 (m, 4H), 3.10 (t, *J* = 12.0 Hz, 2H).

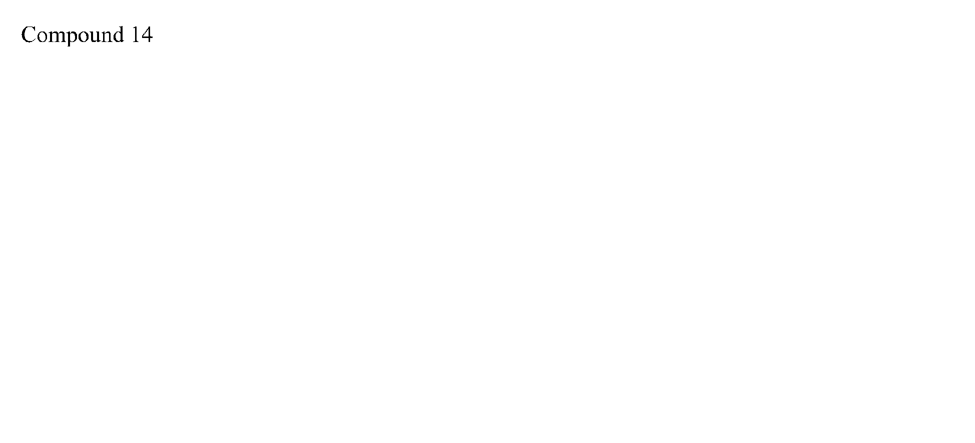

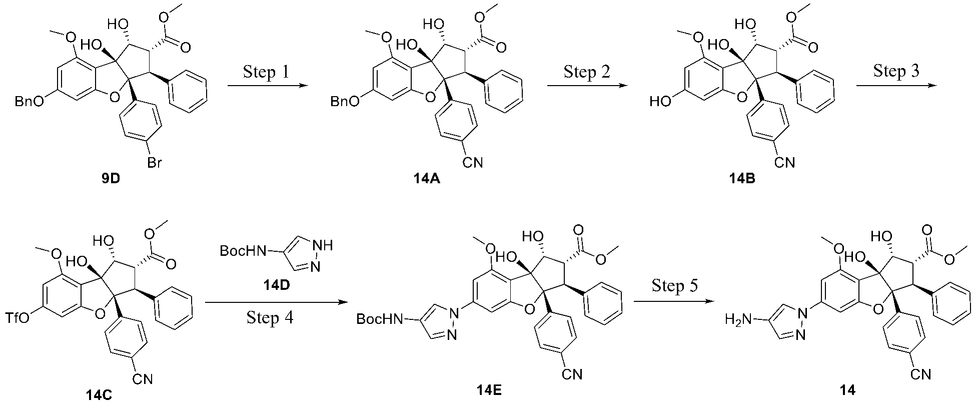

Step 1: A solution of the racemate of compound 9D (2.30 g, 3.7 mmol), Zn(CN)₂ (1.09 g, 9.3 mmol), Pd(dba)₃ (0.68 g, 0.75 mmol), and DPPF (0.83 g, 1.49 mmol) in NMP (160 mL) was stirred at 150 °C for 4 h.The mixture was extracted with H₂O and EA, and the combined organic layers were washed with brine (2 × 10 mL). The mixture was purified by flash column chromatography (PE/EA = 1/1) to give a racemate of 14A (1.9 g, yield: 90%) as a yellow solid.

LCMS: [M+H]⁺ = 564.2.

Step 2: Pd(OH)₂/C(10%, 1.4 g) was added to a solution of the racemate of compound 14A (2.8 g, 4.97 mmol) in EA/MeOH/THF/DCM (EA/MeOH/THF/DCM = 20:2:0.2:0.2, 120 mL) under a N₂ atmosphere. The suspension was stirred at room temperature for 4 h under a H₂ atmosphere. The reaction solution was filtered, and the filter cake was washed with a solution of MeOH/DCM (MeOH/DCM = 1/10). The combined filtrates were concentrated and purified by flash column chromatography (DCM/MeOH = 10/1) to give a racemate of 14B (1.6 g, yield: 68%) as a yellow solid.

LCMS: [M-H]⁺ = 474.2.

Step 3: At 0 °C, trifluoromethanesulfonic anhydride (1.1 g, 4.06 mmol, 1.2 eq) was slowly added dropwise to a solution of the racemate of compound 14B (1.6 g, 3.38 mmol) and diisopropylethylamine (0.7 g, 5.75 mmol) in 150 mL of DCM, and the mixture was stirred for 45 min and washed with 25 mL of saturated sodium bicarbonate. The organic layer was collected, and the aqueous layer was then extracted with DCM (20 mL). The combined organic layers were sequentially washed with 10% citric acid (20 mL) and water (20 mL) and dried. The filtrate was concentrated under reduced pressure and purified by flash column chromatography (DCM/EA = 10/1) to give a racemate of compound 14C (1.7 g, yield: 83%) as a white powder. LCMS: [M+H]⁺ = 606.2.

Step 4: The racemate of compound 14C (50 mg, 2.2 mmol), compound 14D (30.26 mg, 0.16 mmmol), tBuXPhos-Pd-G3 (4.8 mg, 0.01 mmol), tBuXPhos (12.78 mg, 0.03 mmol), and Cs₂CO₃ (67 mg, 0.21 mmol) were dissolved in 1,4-dioxane (2 mL). The reaction mixture was stirred at 100 °C for 16 h under a nitrogen atmosphere. After the reaction was complete, the reaction mixture was extracted with EA and concentrated to give a racemate of compound 14E (50 mg, crude product, yield not calculated). LCMS [M+H]⁺ = 639.2.

Step 5: The racemate of compound 14E (50 mg) was dissolved in a solution of THF (0.5 mL), and 6 M HCl (0.5 mL) was added. The mixture was stirred at room temperature for 4 h. The reaction solution was purified by flash column chromatography (ACN-H₂O = 0-100%) to give a racemate of 14 (6 mg, yield: 12%) as a white solid, and the racemate was further resolved on a chiral column to give compound 14.

LCMS [M+H]⁺ = 539.2.

¹H NMR (400 MHz, DMSO) δ 7.78 (s, 1H), 7.47 (d, *J =* 8.6 Hz, 2H), 7.31 (d, *J =* 8.6 Hz, 2H), 7.26 (s, 1H), 7.09-7.03 (m, 2H), 7.02-6.94 (m, 4H), 6.92 (d, *J* = 1.6 Hz, 1H), 5.51 (s, 1H), 5.39 (d, *J* = 5.3 Hz, 1H), 4.67 (t, *J* = 5.1 Hz, 1H), 4.39 (d, *J* = 13.9 Hz, 1H), 4.18 (d, *J* = 18.8 Hz, 2H), 4.15-4.07 (m, 1H), 3.80 (s, 3H), 3.58 (s, 3H).

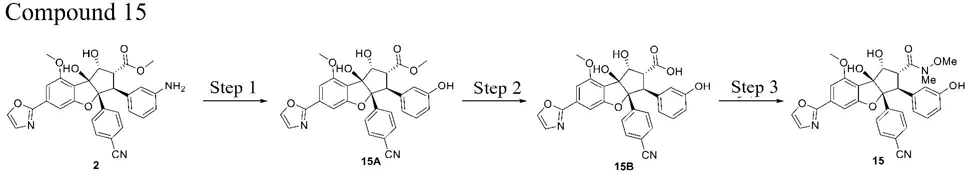

Step 1: HBF₄ (0.85 mL, 4.79 mmol) was added dropwise to a solution of the racemate of compound 2 (170 mg, 0.32 mmol) in ethanol (5 mL) at room temperature. After 15 min of stirring, the reaction solution became clear. The solution was cooled to 0 °C, and tert-butyl nitrite (65.0 mg, 0.63 mmol) was added. After 30 min, the reaction mixture was diluted with diethyl ether (8 mL) and filtered to give a solid, and the solid was washed with diethyl ether (5 mL) twice and dried. The solid was added to water (500 mL) containing cupric nitrate (17.7 g, 94.5 mmol) and cuprous oxide (45.0 mg, 0.32 mmol), and the mixture was stirred at room temperature for 1 h. The aqueous solution was filtered to give a brown solid, and the solid was purified by flash column chromatography (ACN/H₂O) to give a racemate of 15A (20 mg, yield: 12.3%) as a yellow solid. LCMS [M+H]⁺ = 541.0.

Step 2: Theracemate of compound 15A (20.0 mg, 0.04 mmol) and LiOH (34.8 mg, 0.40 mmol) were dissolved in THF/H₂O (5 mL/1 mL), and the solution was stirred at room temperature for 6 h and purified by flash column chromatography (ACN/H₂O) to give 15B (17.0 mg, yield: 85.3%) as a white solid. LCMS [M+H]⁺ = 527.0.

Step 3: Compound 15B (15.0 mg, 0.03 mmol), N,O-dimethylhydroxylamine hydrochloride (2.92 mg, 0.03 mmol), HATU (13.0 mg, 0.03 mmol), HOAT (2.25 mg, 0.03 mmol), and TMP (10.3 mg, 0.09 mmol) were added to DMF (1.0 mL). The mixture was stirred at room temperature for 16 h and purified by flash column chromatography (ACN/H₂O) to give a racemate of 15 (9.0 mg, yield: 55.4%) as a white solid, and the racemate was further resolved on a chiral column to give compound 15.

LCMS [M+H]⁺ = 570.0.

¹H NMR: (400 MHz, CDCl3) δ 9.09 (s, 1H), 8.25 (s, 1H), 7.53 (d, *J* = 8.4 Hz, 2H), 7.41 (s, 1H), 7.30 (d, *J* = 8.4 Hz, 2H), 7.21 (s, 1H), 7.15 (s, 1H), 6.85 (s, 1H), 6.83 (d, *J*=8.4 Hz, 1H), 6.56-6.36 (m, 3H), 5.63 (s, 1H), 5.11 (d, *J* = 4.8 Hz, 1H), 4.79 (t, *J =* 4.8 Hz, 1H), 4.38 (d, *J =* 7.2 Hz, 1H), 4.18-4.13 (m, 1H), 3.90 (s, 3H), 3.84 (s, 3H), 3.09 (s, 3H).

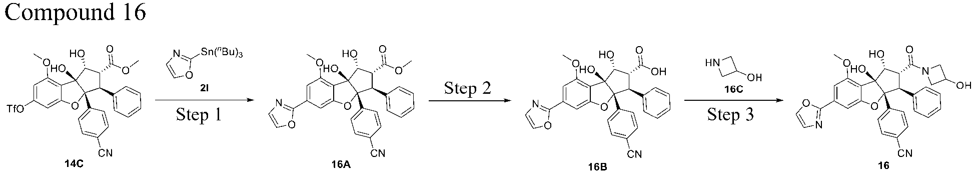

Step 1: Compound 2I (8.04 g, 22.46 mmol), Pd(PPh₃)₄ (763.35 mg, 0.6 mmol), and CuI (629.03 mg, 3.30 mmol) were added to a solution of the racemate of compound 14C(2.00 g, 3.30 mmol) in DMF (10 mL). The mixture was stirred under microwave conditions at 115 °C for 2 h under a nitrogen atmosphere and purified by silica gel column chromatography (PE/EA = 1/1) to give a racemate of 16A(1.5 g, yield: 86%) as a white solid. LCMS [M+H]⁺ = 525.0.

Steps 2 and 3: Theracemate of compound 16A (100.00 mg, 0.19 mmol) and LiOH (20.00 mg, 0.25 mmol) were dissolved in MeOH/H₂O (5 mL/1 mL), and the solution was stirred at room temperature for 12 h. The pH was adjusted to 2-3 with a 1 M aqueous hydrochloric acid solution, and the mixture was extracted with ethyl acetate (20 mL). The organic phase was dried over sodium sulfate and concentrated to give aracemate of 16B (crude product).

Theracemate of 16B (crude product) was dissolved in DMF (3 mL), and HATU (144.50 mg, 0.38 mmol), compound 16C (27.78 mg, 0.38 mmol), and DIEA (73.67 mg, 0.57 mmol) were added. The mixture was stirred at room temperature for 12 h. 50 mL of water was added to the reaction solution, and the mixture was extracted with ethyl acetate, washed with saturated brine, dried, and concentrated. The crude product was purified by silica gel column chromatography (PE/EA = 1/2) to give aracemate of16 (20 mg, yield: 17%) as a white solid, and the racemate was further resolved on a chiral column to give compound16.

LCMS [M+H]⁺ = 566.2.

¹H NMR (400 MHz, DMSO) δ 8.25 (s, 1H), 7.47 (d, *J =* 7.3 Hz, 2H), 7.41 (s, 1H), 7.32 (dd, *J =* 8.0, 5.0 Hz, 2H), 7.23 (s, 1H), 7.15 (s, 1H), 7.04 (t, *J =* 6.8 Hz, 2H), 6.96 (s, 3H), 5.77 (dd, *J =* 15.3, 5.4 Hz, 1H), 5.62 (d, *J =* 7.0 Hz, 1H), 5.06 (dd, *J =* 11.7, 5.0 Hz, 1H), 4.77 (t, *J =* 7.8 Hz, 1H), 4.67 (s, 1H), 4.62-4.50 (m, 2H), 4.47 (d, *J* = 13.8 Hz, 1H), 4.34-4.06 (m, 1H), 4.01 (dd, *J =* 16.6, 9.9 Hz, 1H), 3.93 (dd, *J* = 12.8, 6.8 Hz, 1H), 3.85 (d, *J* = 3.9 Hz, 3H), 3.59 (dd, *J* = 17.2, 7.4 Hz, 1H).

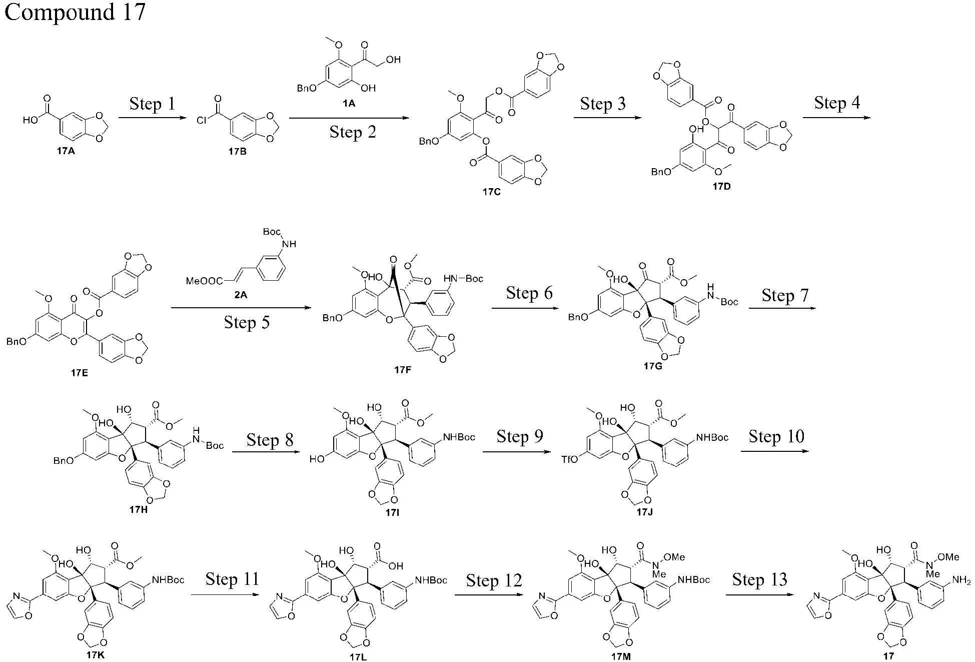

Step 1: Compound 17A (5.0 g, 30.10 mmol) was dissolved in chloroform (50 mL) under ice-water bath conditions, and N,N-dimethylformamide (0.5 mL) and thionyl chloride (5.37 g, 45.14 mmol) were sequentially added. The mixture was stirred at 75 °C for 12 h. The reaction mixture was concentrated to give 17B (6.0 g) as a yellow solid. LCMS [M+H]⁺ = 185.2.

Step 2: Compound 1A (4.0 g, 13.87 mmol) was dissolved in dichloromethane (30 mL) under ice-water bath conditions, and triethylamine (4.2 g, 41.61 mmol), DMAP (1.69 g, 13.8 mmol), and compound 17B (5.63 g, 30.52 mmol) were added. The mixture was stirred at room temperature for 1.5 h, diluted with water (30 mL), and extracted with dichloromethane (30 mL × 2). The organic phase was washed with brine (30 mL × 2), dried over anhydrous sodium sulfate, filtered, concentrated, and purified by silica gel column chromatography (PE/EA = 1/1) to give 17C (6.92 g, yield: 85%) as a white solid. LCMS [M+H]⁺ = 585.0.

Step 3: Compound 17C (3.5 g, 5.99 mmol) was dissolved in anhydrous tetrahydrofuran (30 mL), and a solution of LiHMDS in THF (1 M, 17.96 mL, 17.96 mmol) was added dropwise at -78 °C. The mixture was stirred at -78 °C for 1 h, diluted with water (50 mL), extracted with ethyl acetate (50 mL × 2), washed with brine (50 mL × 2), dried over anhydrous sodium sulfate, filtered, concentrated, and stirred with diethyl ether to give 17D (2.67 g, yield: 78%) as a white solid.

LCMS [M+H]⁺ = 585.0.

Step 4: Compound 17D (900 mg, 1.54 mmol) was dissolved in AcOH (15 mL), and sodium acetate (316 mg, 3.85 mmol) was added. The reactants were stirred overnight at 100 °C. The reaction mixture was concentrated, extracted with dichloromethane (20 mL), washed with water (20 mL × 2) and brine (20 mL × 2), dried over anhydrous sodium sulfate, filtered, concentrated, and stirred with diethyl ether to give 17E (800 mg, yield: 92%) as a yellow solid.

Step 5: Compound 17E (100 mg, 0.24 mmol) and compound 2A (397 mg, 1.44 mmol) were dissolved in CHCl₃/TFE (7/3, 5 mL). A syringe was used to inject the reaction mixture at a rate of 12 mL/h through a syringe pump into a coil wound around a jacketed glass cylinder equipped with a 250 W ultraviolet lamp. The temperature of the external circulation cold trap was adjusted to keep the internal reaction temperature at 0-5 °C, and the UV lamp was turned on simultaneously. The reaction mixture was irradiated for 1 h and concentrated under reduced pressure to remove the solvent. The residue was purified by silica gel column chromatography (EtOAc/hexane) to remove the excess compound 2A, giving a racemate of 17F (190 mg, yield: 75%) as a pale yellow solid. LCMS [M+H]⁺ = 696.1.

Step 6: The racemate of compound 17F (4.8 g, 6.91 mmol) was dissolved in MeOH (40 mL), and NaOMe (932.4 mg, 17.27 mmol) was added at 0 °C. The mixture was stirred at 65 °C for 1.5 h. The solution was concentrated, washed with H₂O, NH₄Cl (aq), and brine, dried over anhydrous Na₂SO₄, concentrated under reduced pressure, and purified by silica gel column chromatography (0.2% FA, EA/PE = 1/3) to give a racemate of compound 17G (2.8 g, yield: 58%). LCMS [M+H-H₂O]⁺ = 677.9.

Step 7: The racemate of compound 17G (2.85 g, 4.10 mmol) was dissolved in ACN/CHCl₃ (1/1, 30 mL/30 mL), and Na(AcO)₃BH (4.3 g, 20.50 mmol) and AcOH (2.5 g, 41.01 mmol) were added at 0 °C. The mixture was stirred at room temperature for 2 h, concentrated, then extracted with EA, washed with water and brine, dried over anhydrous Na₂SO₄, and purified by silica gel column chromatography (A: PE, B: 0.2% FA, EA) to give a racemate of compound 17H (1.6 g, yield: 56%). LCMS [M+H-H₂O]⁺ = 680.0.

Step 8: The racemate of compound 17H (400 mg, 0.57 mmol) was dissolved in MeOH (10 mL), and palladium hydroxide (40 mg) was added to the solution. The reactants were stirred overnight at room temperature under a hydrogen atmosphere. The reaction solution was filtered and washed with methanol (10 mL). The filtrate was concentrated to give a racemate of 17I (320 mg, yield: 91%) as a yellow solid. LCMS [M+H-H₂O]⁺ = 590.0.

Step 9: The racemate of compound 17I (320 mg, 0.53 mmol) was dissolved in DMF (5 mL), and 1,1,1-trifluoro-N-phenyl-N-((trifluoromethyl)sulfonyl)methanesulfonamide (189.3 mg, 0.53 mmol) and potassium carbonate (146.3 mg, 1.06 mmol) were added to the mixture. The reaction solution was stirred at 60 °C for 1 h and purified by reversed-phase chromatography (acetonitrile/water = 5%-95%, 214 nm, 20 min) to give a racemate of 17J (320 mg, yield: 82%) as a white solid. LCMS [M+H]⁺ = 740.2.

Step 10: The racemate of compound 17J (50 mg, 0.07 mmol) was dissolved in DMF (1.5 mL), and 2-(tributylstannyl)oxazole (190 mg, 0.54 mmol), tetrakis(triphenylphosphine)palladium (8 mg, 0.007 mmol), and CuI (2.6 mg, 0.014 mmol) were sequentially added to the solution. The solution was stirred under microwave conditions at 100 °C for 1.5 h. The reaction mixture was filtered, washed with MeOH (5 mL), and concentrated. The residue was purified by reversed-phase chromatography (acetonitrile/water = 5%-95%, 214 nm, 20 min) to give a racemate of 17K (30 mg, yield: 34%) as a yellow solid. LCMS [M+H]⁺ = 659.2.

Step 11: The racemate of compound 17K (35 mg, 0.05 mmol) was dissolved in tetrahydrofuran/water (3 mL/1 mL), and lithium hydroxide (4.20 mg, 0.1 mmol) was added to the solution. The reaction mixture was stirred at room temperature for 2 h, concentrated, and purified by reversed-phase chromatography (acetonitrile/water = 5%-95%, 214 nm, 20 min) to give a racemate of 17L (30 mg, yield: 88%) as a yellow solid. LCMS [M+H]⁺ = 645.2.

Step 12: The racemate of compound 17L (30 mg, 0.05 mmol), N,O-dimethylhydroxylamine (10 mg, 0.1 mmol), HATU (22.8 mg, 0.06 mmol), and DIEA (19.35 mg, 0.15 mmol) were sequentially added to DMF (1.5 mL). The reaction mixture was stirred at room temperature for 2 h and purified by reversed-phase chromatography (acetonitrile/water = 5%-95%, 214 nm, 20 min) to give a racemate of 17M (15 mg, yield: 47%) as a yellow solid. LCMS [M+H]⁺ = 688.2

Step 13: 6 N/HCl (0.3 mL) was added to a solution of the racemate of compound 17M (15 mg, 0.02 mmol) in tetrahydrofuran (0.5 mL). The mixture was stirred at room temperature for 12 h and purified by preparative column chromatography to give a racemate of 17 (3.8 mg, yield: 30%) as a white solid, and the racemate was further resolved on a chiral column to give compound 17.

LCMS [M+H]⁺ = 588.2.

¹H NMR (400 MHz, DMSO-*d*₆) δ 8.36 (s, 1H), 8.24 (s, 1H), 7.41 (s, 1H), 7.15 (d, J = 12.4 Hz, 2H), 6.72-6.60 (m, 4H), 6.23-6.18 (m, 3H), 6.04 (d, J = 7.6 Hz, 1H), 5.86 (d, J = 3.2 Hz, 2H), 5.37 (s, 1H), 4.92 (s, 1H), 4.83-4.73 (m, 3H), 4.16 (d, J = 14.0 Hz, 1H), 4.08 (brs, 1H), 3.88-3.85 (m, 6H), 3.06 (s, 3H).

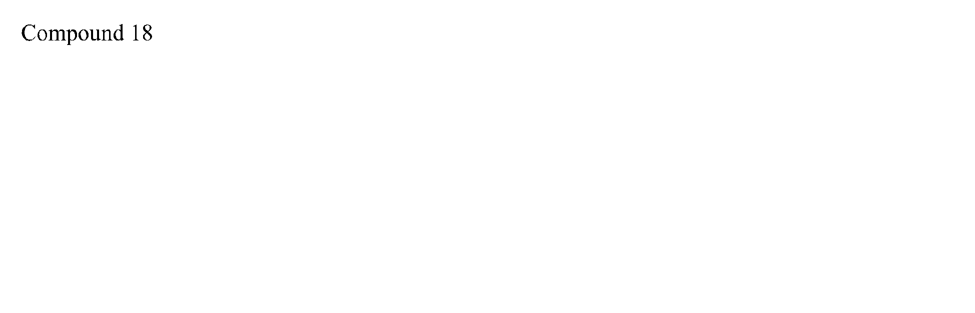

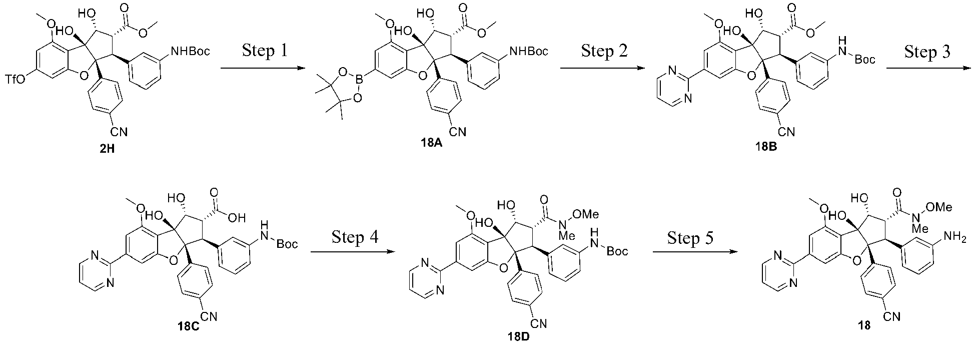

Step 1: Bis(pinacolato)diboron (35.2 mg, 0.14 mmol), potassium acetate (20.4 mg, 0.21 mmol), and Pd(dppf)Cl₂ (5.62 mg, 0.01 mmol) were added to a solution of the racemate of compound 2H (50 mg, 0.07 mmol) in dioxane (5 mL). The mixture was degassed under reduced pressure, purged with nitrogen 3 times, and stirred at 85 °C for 10 h. The reaction solution was concentrated, and the residue was purified by silica gel column chromatography (PE/EA = 5/1) to give a racemate of compound 18A (40.0 mg, yield: 83%) as a yellow solid. LC-MS: (M+H)⁺ = 699.3. ¹H NMR: (400 MHz, CDCl₃) δ 7.36 (dd, J = 8.4, 28.4 Hz, 4H), 7.22 (s, 1H), 7.14(d, J = 7.2 Hz,1H), 6.99-6.95 (m, 2H), 6.86 (s, 1H), 6.53 (d, J=7.6 Hz, 1H), 6.30 (s, 1H), 5.01 (d, J = 6.0 Hz, 1H), 4.36 (d, J = 14.4 Hz, 1H), 3.95 (s, 3H), 3.66 (s, 3H), 3.53 (s, 1H), 1.51 (s, 9H), 1.37 (s, 12H).

Step 2: 2-Bromopyrimidine (9.1 mg, 0.09 mmol), Na₂CO₃ (18.2 mg, 0.17 mmol), and Pd(dppf)Cl₂ (4.62 mg, 0.01 mmol) were added to a solution of the racemate of compound 18A (40 mg, 0.06 mmol) in dioxane/H₂O (5 mL/1 mL), and the mixture was microwaved to 100 °C and stirred for 1 h under a nitrogen atmosphere. The reaction solution was concentrated, and the residue was purified by flash column chromatography (ACN/H₂O) to give a racemate of compound 18B (30.0 mg, yield: 80.5%) as a white solid. LC-MS: (M+H)⁺ = 651.0. ¹H NMR: (400 MHz, CDCl3) δ 8.92 (d, J = 4.8 Hz, 2H), 7.92 (s, 1H), 7.86 (s, 1H), 7.40 (dd, J = 8.4, 22.0 Hz, 5H), 7.14 (d, J = 7.6 Hz, 1H), 7.01-6.96 (m, 1H), 6.91 (s, 1H), 6.57 (d, J=7.6 Hz, 1H), 6.31 (s, 1H), 5.05 (d, J = 6.0 Hz, 1H), 4.45 (d, J = 14.0 Hz, 1H), 4.07 (s, 3H), 3.97 (dd, J = 6.0, 14.0 Hz, 1H), 3.69 (s, 1H), 1.52 (s, 9H). Step 3: The racemate of compound 18B (30 mg, 0.05 mmol) and LiOH (20.2 mg, 0.23 mmol) were dissolved in THF/H₂O (1 mL/1 mL), and the solution was stirred at room temperature for 6 h and purified by flash column chromatography (ACN/H₂O) to give a racemate of compound 18C (21.0 mg, yield: 69.0%) as a white solid. LC-MS: [M+H-C₄H₈]⁺ = 581.0.

Step 4: The racemate of compound 18C (20.0 mg, 0.03 mmol), N,O-dimethylhydroxylamine hydrochloride (3.22 mg, 0.03 mmol), HATU (14.3 mg, 0.04 mmol), HOAT (2.25 mg, 0.03 mmol), and TMP (11.4 mg, 0.09 mmol) were added to DMF (5.0 mL). The mixture was stirred for 16 h and purified by flash column chromatography (ACN/H₂O) to give a racemate of compound 18D (20.0 mg, yield: 93.6%) as a white solid. LCMS [M+1]⁺ = 680.1.

Step 5: HCl (6 N, 1 mL) was added to a solution of the racemate of compound 18D (43.0 mg, 0.08 mmol) in THF (1 mL). The mixture was stirred overnight at room temperature and purified by flash column chromatography (ACN/H₂O) to give a racemate of compound 18 (70 mg, yield: 41%), and the racemate was further resolved on a chiral column to give compound 18 as a white solid. LCMS [M+H]⁺ = 580.0.

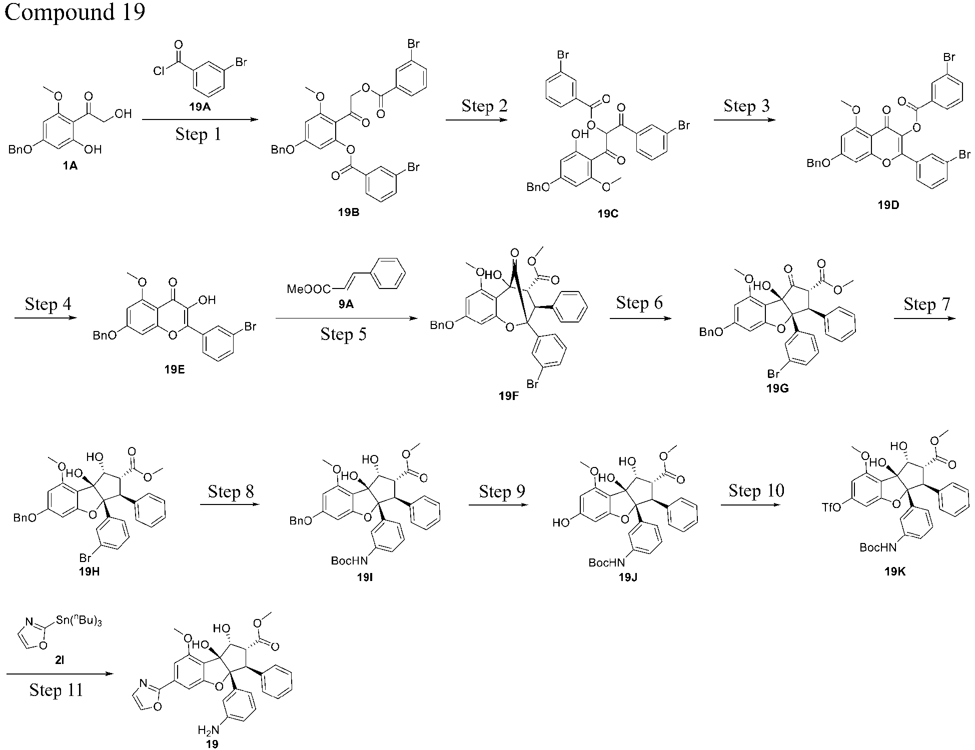

Step 1: Compound 1A (3.5 g, 12.1 mmol), DMAP (148 mg, 1.21 mmol), and TEA (3667 mg, 36.3 mmol) were dissolved in DCM (40 mL). The solution was cooled to 0 °C, and compound 19A (5860 mg, 26.7 mmol) was added in portions. The mixture was stirred at 25 °C for 1 h. The solution was concentrated, extracted with EA, washed with water and brine, dried over anhydrous Na₂SO₄, and purified by silica gel column chromatography (PE:EA = 3/1) to give 19B (5 g, yield: 63%) as a yellow solid. LCMS: [M+H]⁺ = 655.0.

Step 2: Compound 19B (5 g, 7.64 mmol) was dissolved in THF (50 mL), and the solution was cooled to -78 °C. Under a N₂ atmosphere, LiHMDS (23 mL, 23 mmol) was added dropwise to the aforementioned stirred solution, and the mixture was stirred at -78 °C for 2 h. The reaction solution was quenched with a saturated aqueous NH₄Cl solution, extracted with EA, and washed with water and saturated brine. The organic layer was dried over anhydrous Na₂SO₄, concentrated to dryness by rotary evaporation, and purified by silica gel column chromatography (PE:EA = 3/1) to give 19C (4.1 g, yield: 82%) as a yellow solid. LCMS: [M+H]⁺ = 654.9.

Step 3: Compound 19C (6.1 g, 9.32 mmol) and AcONa (1912 mg, 23.31 mmol) were dissolved in AcOH (183 mL), and the solution was stirred overnight at 100 °C. TLC showed that no starting material remained and a new spot was formed. The solution was concentrated to dryness by rotary evaporation, then extracted with EA, washed with water and brine, dried over anhydrous Na₂SO₄, concentrated, and purified by silica gel column chromatography (PE:EA = 1/1) to give 19D (4 g, yield: 67%) as a yellow solid. LCMS: [M+H]⁺ = 636.9.

Step 4: Compound 19D (4 g, 6.29 mmol) was dissolved in CHCl₃ (120 mL). The solution was cooled to 0 °C, and MeONa (3396 mg, 18.86 mmol) was added. The mixture was stirred at 25 °C for 1 h. TLC showed that no starting material remained and a new spot was formed. The pH of the reaction solution was adjusted to 5 with acetic acid, and the mixture was concentrated to dryness by rotary evaporation, extracted with EA, and washed with MeOH to give a filter cake, which was 19E (2.5 g, yield: 88%) as a gray solid.

LCMS: [M+H]⁺ = 453.0.

¹H NMR (400 MHz, CDCl3) δ 8.33 (s, 1H), 8.17 (d, *J =* 7.9 Hz, 1H), 7.55 (d, *J =* 7.9 Hz, 1H), 7.48-7.34 (m, 6H), 6.66 (d, *J* = 1.4 Hz, 1H), 6.44 (s, 1H), 5.17 (s, 2H), 3.97 (s, 3H).

Step 5: Compound 19E (1 g, 2.2 mmol) and compound 9A (2.1 g, 13.25 mmol) were dissolved in CHCl₃/TFE (7/3, 15 mL). A syringe was used to inject the reaction mixture at a rate of 12 mL/h through a syringe pump into a coil wound around a jacketed glass cylinder equipped with a 250 W ultraviolet lamp. The temperature of the external circulation cold trap was adjusted to keep the internal reaction temperature at 0-5 °C, and the UV lamp was turned on simultaneously. The reaction mixture was irradiated for 1 h and concentrated under reduced pressure to remove the solvent. The residue was purified by silica gel column chromatography (EtOAc/hexane) to remove the excess cinnamate, giving a racemate of compound 19F (2.9 g, yield: 64%) as a pale yellow solid. LCMS [M+H+H2O]⁺ = 633.1.

Step 6: A mixture of the racemate of compound 19F (2.96 g, 4.81 mmol) in MeOH (50 mL) was added to MeONa (649.76 g, 12.03 mmol). The resulting mixture was stirred at 65 °C for 2 h, concentrated, extracted with EA, washed with a saturated NH₄Cl solution and brine, dried over anhydrous Na₂SO₄, and separated and purified by silica gel column chromatography (0.5% FA, EA/PE = 31/69) to give a racemate of compound 19G (1.2 g, yield: 75%) as a pale yellow solid. LCMS [M+H]⁺ = 597.1.

Step 7: The racemate of compound 19G (1.7 g, 2.76 mmol) was mixed with MeCN/CHCl₃ (1/1, 50 mL/50 mL), and Na(AcO)₃BH (2.9 g, 13.82 mmol) and AcOH (1.7 g, 27.64 mmol) were added at 0 °C. The mixture was stirred at room temperature for 2 h. The solution was concentrated, extracted with EA, washed with water and brine, dried over anhydrous Na₂SO₄, and purified by silica gel column chromatography (A: PE, B: DCM/EA = 1/1) to give a racemate of compound 19H (919 mg, yield: 54%).

LCMS [M+H-H₂O]⁺ = 599.0.

¹H NMR (400 MHz, CDCl3) δ 7.49-7.32 (m, 6H), 7.24 (ddd, *J* = 7.9, 2.0, 1.0 Hz, 1H), 7.15-7.11 (m, 1H), 7.10-7.05 (m, 3H), 7.00 (dd, *J* = 9.1*,* 6.7 Hz, 1H), 6.89 (dd, *J =* 7.0, 2.2 Hz, 2H), 6.38 (d, *J* = 1.9 Hz, 1H), 6.23 (d, *J =* 1.9 Hz, 1H), 5.09 (s, 2H), 5.02 (d, *J =* 6.5 Hz, 1H), 4.36 (d, *J =* 14.2 Hz, 1H), 3.95-3.88 (m, 1H), 3.86 (s, 3H), 3.66 (d, *J =* 3.6 Hz, 3H).

Step 8: The racemate of compound 19H (300.0 mg, 0.49 mmol), NH₂Boc (170.7 mg, 1.46 mmol), Pd₂(dba)₃ (44.5 mg, 0.05 mmol), Xantphos (56.2 mg, 0.10 mmol), and Cs₂CO₃ (316.0 mg, 0.97 mmol) were mixed in 1,4-dioxane (20 mL), and the mixture was stirred at 100 °C for 16 h under N₂, concentrated, and purified by silica gel column chromatography (PE/EA) to give a racemate of compound 19I (219 mg, yield: 69%).

LCMS [M-H]⁻ = 652.2.

¹H NMR (400 MHz, CDCl₃) δ 7.48-7.35 (m, 6H), 7.10-7.03 (m, 4H), 6.96 (t, *J* = 1.8 Hz, 1H), 6.92-6.83 (m, 3H), 6.36 (d, *J =* 1.9 Hz, 1H), 6.22 (d, *J =* 1.9 Hz, 1H), 5.09 (s, 2H), 5.03 (d, *J* = 6.7 Hz, 1H), 4.33 (d, *J =* 14.2 Hz, 1H), 3.95 (dd, *J =* 14.2, 6.7 Hz, 1H), 3.86 (s, 3H), 3.65 (d, *J* = 3.7 Hz, 3H), 1.49 (s, 9H).

Step 9: Pd(OH)₂/C (10%, 200 mg) was added to a solution of the racemate of compound 19I (219.0 mg, 0.34 mmol) in EA/MeOH/THF/DCM (20:2:2:0.2, 10 mL). The mixture was purged with H₂ multiple times and stirred at room temperature for 16 h. The suspension was filtered, and the filter cake was washed with MeOH/DCM (1/10). The filtrates were mixed, concentrated, and dried to give a racemate of compound 19J (180 mg, yield: 95%).

¹H NMR (400 MHz, CDCl3) δ 7.23-7.02 (m, 7H), 6.95-6.86 (m, 3H), 6.47 (s, 1H), 6.11 (d, *J* = 1.5 Hz, 1H), 5.91 (s, 1H), 4.99 (d, *J* = 7.1 Hz, 1H), 4.31 (d, *J =* 14.1 Hz, 1H), 3.97 (dd, *J =* 14.1, 7.1 Hz, 1H), 3.74 (s, 3H), 3.66 (s, 3H), 1.50 (s, 9H).

LCMS [M+Na]⁺ = 586.1.

Step 10: The racemate of compound 19J (180 mg, 0.32 mmol) and K₂CO₃ (88 mg, 0.64 mmol) were stirred in DMF (10 mL). N-Phenylbis(trifluoromethanesulfonimide) (114 mg, 0.32 mmol) was added, and the mixture was stirred at 60 °C for 20 min. The solution was concentrated, extracted with EA, washed with water and brine, dried over anhydrous Na₂SO₄, and purified by silica gel column chromatography (DCM/MeOH = 10/1) to give a racemate of compound 19K (190 mg, yield: 86%). LCMS: [M+Na]⁺ = 718.0.

Step 11: Compound 2I (288.55 mg, 0.8 mmol), Pd(PPh₃)₄ (11.6 mg, 0.01 mmol), and CuI (3.84 mg, 0.02 mmol) were added to a solution of the racemate of compound 19K (70 mg, 0.1 mmol) in DMF (3 mL). The reaction solution was purged with nitrogen three times, then left to react in a microwave reactor at 100 °C for 1.5 h, and purified by reversed-phase column chromatography (H₂O:ACN = 0-100%) to give a racemate of compound 19 (2.3 mg, yield: 5%), and the racemate was further resolved on a chiral column to give compound 19 as a white solid.

LCMS [M+H]⁺ = 515.0.

¹H NMR (400 MHz, DMSO) δ 8.25 (s, 1H), 7.41 (s, 1H), 7.21-7.10 (m, 2H), 7.10-6.89 (m, 5H), 6.64 (t, *J =* 7.9 Hz, 1H), 6.48 (s, 1H), 6.26 (d, *J* = 8.0 Hz, 1H), 6.17 (d, *J* = 6.4 Hz, 1H), 5.31 (d, *J* = 5.2 Hz, 1H), 5.25 (s, 1H), 4.70 (t, *J* = 5.2 Hz, 2H), 4.20 (d, *J* = 14.1 Hz, 1H), 4.01 (dd, *J* = 14.0, 5.2 Hz, 1H), 3.85 (s, 3H), 3.55 (s, 3H).

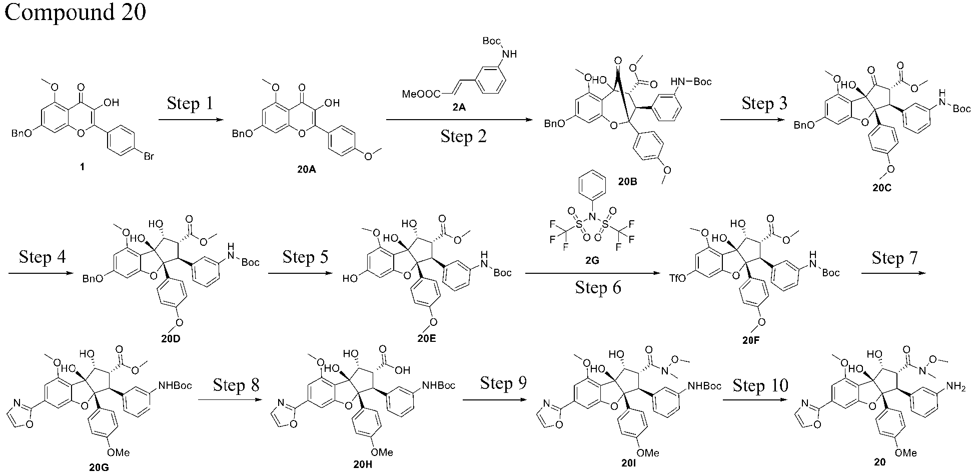

Step 1: Compound 1 (1000 mg, 2.21 mmol) and Cs₂CO₃ (1.08 g, 3.32 mmol) were dissolved in absolute methanol (10 mL) and anhydrous toluene (30 mL), and a solution of t-BuXPhosPdG3 (35.13 mg, 0.04 mmol) was added. The mixture was stirred at 85 °C for 13 h. The solution was concentrated, diluted with MeOH/DCM (1/1), and filtered. Then, the filtrate was concentrated and purified by silica gel column chromatography (DCM/MeOH = 10/1) to give 20A (0.65 g, yield: 73%) as a yellow solid.

Step 2: After compound 20A (1 g, 2.2 mmol) and compound 2A (4.1 g, 13.3 mmol) were dissolved in CHCl₃/TFE (9/1, 15 mL), a 20 mL syringe was used to inject the reaction mixture at a rate of 12 mL/h through a syringe pump into a coil wound around a jacketed glass cylinder equipped with a 250 W ultraviolet lamp. The temperature of the external circulation cold trap was adjusted to keep the internal reaction temperature at 0-5 °C, and the UV lamp was turned on simultaneously. The reaction mixture was irradiated for 1 h, concentrated under reduced pressure to remove the solvent, and then separated by column chromatography (EA/PE = 1/3) to remove the excess cinnamate, giving a racemate of the desired product 20B (crude, 3.9 g, yield: 80%) as a yellow oil. LCMS [M+H]⁺ = 682.3.

Step 3: The racemate of compound 20B (3.7 g, 5.43 mmol) was mixed with MeOH (50 mL), and NaOMe (733.5 mg, 13.58 mmol) was added at 0 °C. The mixture was stirred at 65 °C for 1.5 h. The solution was concentrated, washed with H₂O, NH₄Cl (aq), and brine, dried over Na₂SO₄, concentrated under reduced pressure, and purified by silica gel column chromatography (0.2% FA, EA/PE = 1/3) to give a racemate of compound 20C (2.5 g, yield: 67%). LCMS [M+H-H₂O]⁺ = 664.2.

Step 4: The racemate of compound 20C (2.5 g, 3.67 mmol) was dissolved in MeCN/CHCl₃ (1/1, 50 mL/50 mL), and Na(AcO)₃BH (3.9 g, 18.36 mmol) and AcOH (2.2 g, 36.71 mmol) were added at 0 °C. The mixture was stirred at room temperature for 2 h. The reaction solution was extracted with EA, washed with water and brine, dried over anhydrous Na₂SO₄, and purified by silica gel column chromatography (A: PE, B: EA = 1/1) to give a racemate of compound 20D (1.5 g, yield: 60%). LCMS [M+H-H₂O]⁺ = 666.3.

Step 5: Pd(OH)₂/C (10%, 100 mg) was added to a solution of the racemate of compound 20D (200 mg, 0.29 mmol) in EA/MeOH/THF/DCM (20:2:2:2:0.2, 10 mL). The mixture was purged with H₂ 3 times and stirred at room temperature for 16 h. The suspension was filtered, and the filter cake was washed with MeOH/DCM (1/10). The filtrate was concentrated and purified by silica gel column chromatography (DCM/MeOH = 10/1) to give a racemate of compound 20E (155 mg, yield: 89%). LCMS [M+Na]⁺ = 616.1.

Step 6: The racemate of compound 20E (155.0 mg, 0.26 mmol) and K₂CO₃ (72.1 mg, 0.52 mmol) were stirred in DMF (2 mL). Compound 2G (93.3 mg, 0.26 mmol) was added. The reaction solution was stirred at 60 °C for 20 min, diluted with water, extracted with EA, washed with water and brine, dried over anhydrous Na₂SO₄, and purified by silica gel column chromatography (EA/PE = 1/1) to give a racemate of compound 20F (160 mg, yield: 84%). LCMS: [M+Na]⁺ = 748.1.

Step 7: The racemate of compound 20F (50 mg, 0.07 mmol) was dissolved in DMF (1.5 mL), and 2-(tributylstannyl)oxazole (190 mg, 0.54 mmol), tetrakis(triphenylphosphine)palladium (8 mg, 0.007 mmol), and CuI (2.6 mg, 0.014 mmol) were sequentially added to the solution. The solution was stirred under microwave conditions at 100 °C for 1.5 h. The reaction mixture was filtered, washed with MeOH (5 mL), and concentrated. The residue was purified by reversed-phase chromatography (acetonitrile/water = 5%-95%, 214 nm, 20 min) to give a racemate of 20G (17 mg, yield: 38%) as a yellow solid. LCMS: [M+Na]⁺ = 667.2.

Step 8: The racemate of compound 20G (35 mg, 0.05 mmol) was dissolved in tetrahydrofuran/water (3 mL/1 mL), and lithium hydroxide (4.56 mg, 0.1 mmol) was then added to the solution. The reaction mixture was stirred at room temperature for 2 h, concentrated, and purified by reversed-phase chromatography (acetonitrile/water = 5%-95%, 214 nm, 20 min) to give a racemate of 20H (21 mg, yield: 61%) as a yellow solid. LCMS: [M+Na]⁺ = 653.2.

Step 9: The racemate of compound 20H (21 mg, 0.03 mmol), N,O-dimethylhydroxylamine (3.25 mg, 0.06 mmol), HATU (17.1 mg, 0.05 mmol), and DIEA (11.61 mg, 0.09 mmol) were added to DMF (1.5 mL). The reaction mixture was stirred at room temperature for 2 h and purified by reversed-phase chromatography (acetonitrile/water = 5%-95%, 214 nm, 20 min) to give a racemate of 20I (12 mg, yield: 54%) as a yellow solid. LCMS: [M+Na]⁺ = 696.3.

Step 10: 6 N/HCl (0.3 mL) was added to a solution of the racemate of compound 20I (12 mg, 0.02 mmol) in tetrahydrofuran (0.5 mL). The mixture was stirred at room temperature for 12 h and purified by preparative chromatography to give a racemate of 20 (3.2 mg, yield: 22%) as a white solid, and the racemate was further resolved on a chiral column to give compound 20. LCMS: [M+H]⁺ = 574.2.

1H NMR (400 MHz, DMSO-*d*₆) δ 8.24 (d, *J* = 0.8 Hz, 1H), 7.40 (d, *J* = 0.8 Hz, 1H), 7.17 (d, *J*= 1.2 Hz, 1H), 7.14 (d, *J =* 1.2 Hz, 1H), 7.08-7.05 (m, 2H), 6.67-6.63 (m, 3H), 6.23 (brs, 1H), 6.16 (d, *J* = 9.2 Hz, 1H), 6.01 (d, *J* = 7.6 Hz, 1H), 5.30 (s, 1H), 4.86 (d, *J* = 4.4 Hz, 1H), 4.80-4.79 (m, 3H), 4.15-4.08 (m, 2H), 3.87 (s, 3H), 3.85 (s, 3H), 3.63 (s, 3H), 3.06 (s, 3H).

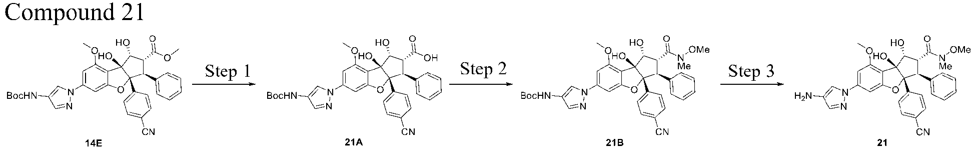

Step 1: An aqueous LiOH solution (12 mg, 0.3 mmol, 0.5 mL of H₂O) was added to a solution of theracemate of compound 14E (60 mg, 0.1 mol) in THF (2 mL). The reaction solution was stirred at room temperature for 18 h and purified by reversed-phase column chromatography (H₂O:ACN = 0-100%) to give aracemate of21A (45 mg, yield: 77.5%) as a white solid. LCMS: [M+H]⁺ = 625.2.

Step 2: TMP (35 mg, 0.28 mmol) was added to a solution of theracemate of compound 21A (45 mg, 0.07 mmol), N,O-dimethylhydroxylamine (14 mg, 0.14 mmol), HATU (54.72 mg, 0.14 mmol), and HOAT (19.58 mg, 0.14 mmol) in DMF (4 mL). The reaction solution was stirred at 50 °C for 18 h and purified by normal-phase column chromatography (DCM/MeOH = 10/1) to give aracemate of 21B (24 mg, yield: 50%) as a white solid. LCMS: [M+H]⁺ = 668.2.

Step 3: 6 M HCl (2 mL) was added to a solution of theracemate of compound 21B (24 mg, 0.03 mmol) in THF (1 mL). The reaction solution was stirred at room temperature for 18 h and purified by reversed-phase column chromatography (H₂O:ACN = 0-100%) to give aracemate of 21 (14 mg, yield: 70%) as a white solid, and the racemate was further resolved on a chiral column to give compound 21.

LCMS [M+H]⁺ = 568.2.

¹H NMR (400 MHz, DMSO) δ 7.78 (s, 1H), 7.52 (d, *J =* 8.6 Hz, 2H), 7.34-7.24 (m, 3H), 7.06 (t, *J* = 7.4 Hz, 2H), 6.99 (t, *J =* 4.4 Hz, 2H), 6.91 (dd, *J =* 18.8, 4.5 Hz, 3H), 5.50 (s, 1H), 5.01 (d, *J =* 4.8 Hz, 1H), 4.80 (t, *J =* 5.1 Hz, 1H), 4.43 (d*, J =* 13.8 Hz, 1H), 4.19 (s, 3H), 3.91 (s, 3H), 3.81 (s, 2H), 3.09 (s, 3H).

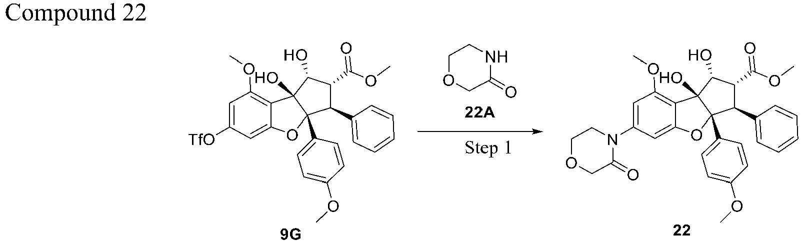

Step 1: The racemate of compound 9G (50.0 mg, 0.08 mmol), compound 22A (24.8 mg, 0.25 mmol), t-BuXphos PdG3 (13.0 mg, 0.02 mmol), t-Bune phos (7.7 mg, 0.02 mmol), and Na₂CO₃ (26.1 mg, 0.25 mmol) were dissolved in 1,4-dioxane (5 mL). The reaction solution was stirred at 100 °C for 16 h under a N₂ atmosphere, concentrated, and purified by preparative high-performance liquid chromatography to give a racemate of 22 (1.2 mg, yield: 6.7%) as a white solid, and the racemate was further resolved on a chiral column to give compound 22.

LCMS [M+H]⁺ = 562.2.

¹H NMR (400 MHz, DMSO) δ 7.11-6.94 (m, 5H), 6.89 (d, *J* = 7.4 Hz, 2H), 6.68 (d, *J* = 1.5 Hz, 1H), 6.62-6.54 (m, 3H), 5.29 (s, 1H), 5.26 (d, *J =* 5.0 Hz, 1H), 4.71 (t, *J =* 5.2 Hz, 1H), 4.23-4.15 (m, 3H), 4.00-3.92 (m, 3H), 3.78 (dd, *J =* 11.2, 6.2 Hz, 1H), 3.74 (s, 3H), 3.60 (s, 3H), 3.55 (s, 3H).

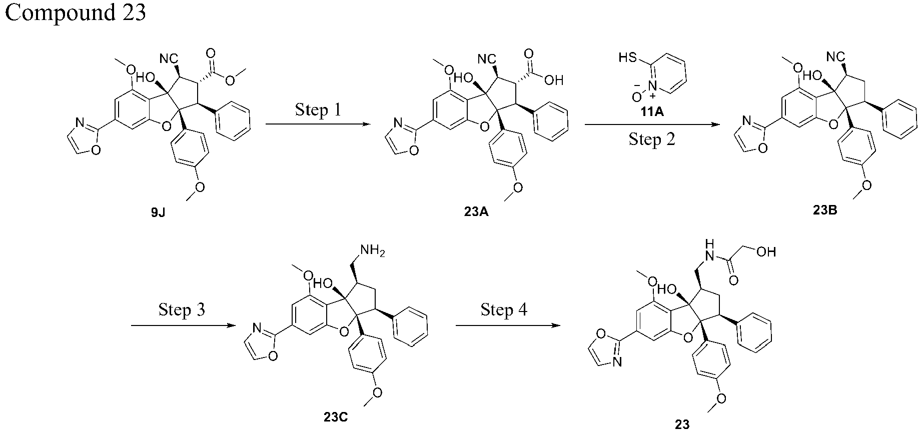

Step 1: An aqueous solution (0.5 mL) of LiOH (47 mg, 1.12 mmol) was added to a solution of the racemate of compound 9J (200 mg, 0.37 mol) in THF (5 mL). The reaction solution was stirred at room temperature for 18 h and purified by reversed-phase column chromatography (H₂O:ACN = 0-100%) to give a racemate of 23A (180 mg, yield: 92%) as a white solid. LCMS [M+H]⁺ = 525.3. Step 2: A solution of the racemate of compound 23A (10 mg, 0.02 mmol), compound 11A (24.3 mg, 0.2 mmol), tert-dodecyl mercaptan (38.4 mg, 0.2 mmol), and zinc meso-tetraphenylporphine (1.3 mg, 0.001 mmol) in DMF (1 mL) was added to a microwave tube. The microwave tube was placed in a water bath at 80 °C and irradiated with a 25 W red light lamp. EDCI (36.6 mg, 0.2 mmol, dissolved in DMF) was added dropwise to the reaction system. Subsequently, the mixture was stirred for 30 min. Three parallel reactions were conducted. The reaction solutions were combined and purified by reversed-phase column chromatography (H₂O:ACN = 0-100%) to give a racemate of 23B (10 mg, yield: 30%) as a white solid. LCMS [M+H]⁺ = 481.2.

Step 3: A solution of the racemate of compound 23B (30 mg, 0.06 mmol) in THF (2 mL) was cooled to 0 °C. LiAlH₄ (30 µL, 0.12 mmol) was added dropwise to the reaction system, and the mixture was then left to react at 0 °C for 1 h. Na₂SO₄·10H₂O was added to the reaction system, and the mixture was subsequently stirred at room temperature for half an hour. The suspension was filtered, and the filter cake was washed with MeOH/DCM (1/10) three times. The resulting filtrate was concentrated to dryness by rotary evaporation and purified by thin-layer chromatography (DCM/MeOH = 7/1) to give a racemate of 23C (5 mg, yield: 16.7%) as a white solid. LCMS [M+H]⁺ = 485.2.

Step 4: TMP (4 mg, 0.032 mmol) was added to a solution of the racemate of compound 23C (4 mg, 0.008 mmol), glycolic acid (1.2 mg, 0.008 mmol), HATU (6.4 mg, 0.016 mmol), and HOAT (2.4 mg, 0.016 mmol) in DMF (1 mL), and the mixture was left to react at room temperature for 1 h. The reaction solution was purified by reversed-phase column chromatography (H₂O:ACN = 0-100%) to give a racemate of 23 (2.2 mg, yield: 50%) as a white solid, and the racemate was further resolved on a chiral column to give compound 23.

LCMS [M+H-H₂O]⁺ = 525.0.

¹H NMR (400 MHz, DMSO) δ 8.26 (d, *J =* 0.6 Hz, 1H), 7.79 (t, *J =* 5.7 Hz, 1H), 7.41 (d, *J =* 0.7 Hz, 1H), 7.22 (dd, *J* = 8.9, 1.1 Hz, 2H), 7.14 (d, *J* = 8.9 Hz, 2H), 7.11-7.00 (m, 3H), 6.91 (d, *J* = 6.8 Hz, 2H), 6.69 (d, *J* = 9.0 Hz, 2H), 5.64 (t, *J* = 5.5 Hz, 1H), 5.42 (s, 1H), 3.96 (s, 3H), 3.91 (d, *J =* 5.3 Hz, 2H), 3.70 (t, *J =* 5.3 Hz, 2H), 3.65 (s, 3H), 3.39 (d, *J =* 6.2 Hz, 1H), 2.90 (td, *J =* 14.0, 6.8 Hz, 1H), 2.24-2.01 (m, 2H).

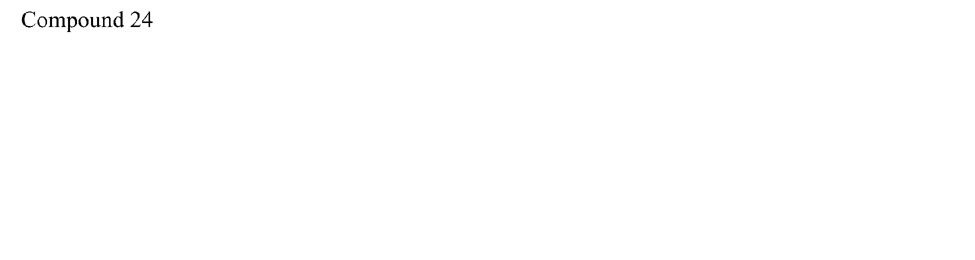

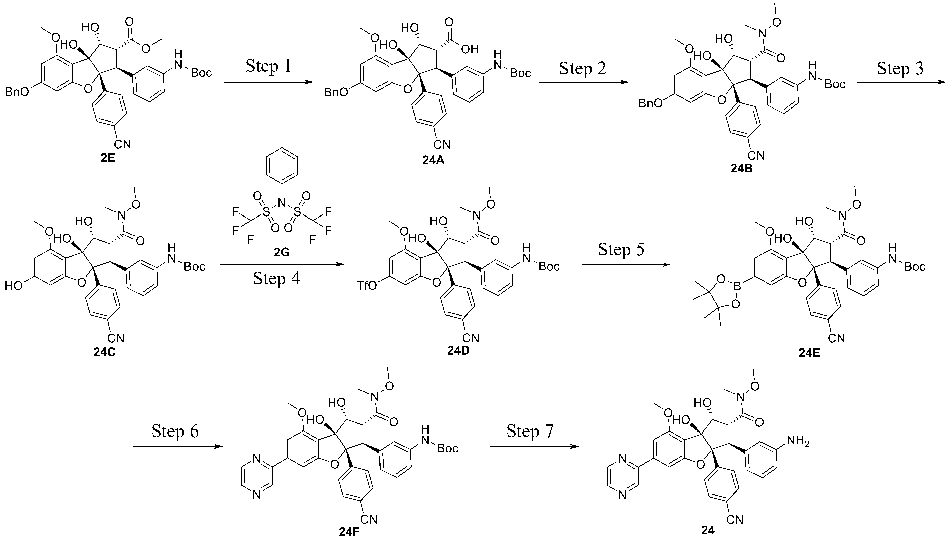

Step 1: The racemate of compound 2E (3200 mg, 4.715 mmol) was dissolved in THF (30 mL), MeOH (10 mL), and H₂O (10 mL), and LiOH (594 mg, 14.14 mmol) was added. The mixture was stirred at room temperature for 8 h under a nitrogen atmosphere. The pH of the reaction mixture was adjusted to 5 with 3 M HCl, and the mixture was concentrated to give a crude product. The crude product was purified by silica gel column chromatography (DCM/MeOH = 10/1) to give a racemate of 24A (2.8 g, yield: 89%) as a white solid. LCMS [M+H]⁺ = 665.2.

Step 2: The racemate of compound 24A (2800 mg, 4.2124 mmol) was dissolved in DMF (120 mL), and dimethylhydroxylamine hydrochloride (1233 mg, 12.6371 mmol), HATU (4802 mg, 12.6371 mmol), HOAT (1719 mg, 12.6371 mmol), and TMP (1539 mg, 12.6371 mmol) were added. The mixture was heated to 50 °C and stirred for 16 h under a nitrogen atmosphere. Water (100 mL) was added to the reaction solution, and the mixture was extracted with ethyl acetate (150 mL) twice. The organic phases were combined, washed with saturated brine (150 mL), dried over anhydrous sodium sulfate, filtered, and concentrated to give a crude product, and the crude product was purified by silica gel column chromatography (ethyl acetate/petroleum ether = 1/10) to give a racemate of 24B (2.9 g, yield: 97%) as a yellow solid. LCMS [M+H-C₄H₈]⁺ = 652.2.

Step 3: The racemate of compound 24B (2900 mg, 4.10 mmol) was dissolved in a mixed solution of EA/MeOH/THF/DCM (20:2:2:0.2) (40 mL), and Pd(OH)₂ (870 mg) was added. The mixture was stirred at room temperature for 16 h under a hydrogen atmosphere. The reaction solution was filtered and concentrated to give a crude product, and the crude product was purified by silica gel column chromatography (DCM/MeOH = 10/1) to give a racemate of 24C (1.8 g, yield: 71%) as a yellow solid. LCMS [M+H-C₄H₈]⁺ = 562.2.

Step 4: The racemate of compound 24C (1800 mg, 2.9142 mmol) was dissolved in DMF (50 mL), and compound 2G (1041 mg, 2.9142 mmol) and K₂CO₃ (804 mg, 5.8284 mmol) were added. The mixture was stirred at 60 °C for 40 min under a nitrogen atmosphere. The reaction solution was filtered, and water (100 mL) was added to the filtrate. The mixture was extracted with ethyl acetate (150 mL) twice. The organic phases were combined, washed with saturated brine (150 mL), dried over anhydrous sodium sulfate, filtered, and concentrated to give a crude product, and the crude product was purified by silica gel column chromatography (EA/PE = 1/1) to give a racemate of 24D (1.6 g, yield: 74%) as a yellow oil. LCMS [M+H-C₄H₈]⁺ = 694.1.

Step 5: The racemate of compound 24D (860 mg, 1.15 mmol) was dissolved in dioxane (12 mL), and bis(pinacolato)diboron (876 mg, 3.45 mmol), Pd(dppf)Cl₂ (188 mg, 0.23 mmol), and KOAc (432 mg, 4.60 mmol) were added. The mixture was stirred at 85 °C for 4 h under a nitrogen atmosphere. The reaction solution was filtered and concentrated to give a crude product, and the crude product was purified by silica gel column chromatography (EA/PE = 1/1) to give a racemate of 24E (700 mg, yield: 84%) as a yellow oil. LCMS [M+H-C₄H₈]⁺ = 672.2.

Step 6: The racemate of compound 24E (20 mg, 0.0300 mmol) was dissolved in dioxane (1.5 mL) and water (0.3 mL), and 2-bromopyrazine (8.74 mg, 0.0500 mmol), Pd(dppf)Cl₂ (2.24 mg, 0.0030 mmol), and Na₂CO₃ (11.65 mg, 0.1099 mmol) were added. The mixture was left to react under microwave conditions at 100 °C for 1 h under a nitrogen atmosphere. The reaction solution was filtered and concentrated to give a racemate of 24F (17 g, yield: 84%) as a yellow solid. LCMS [M+H]⁺ = 680.4.

Step 7: The racemate of compound 24F (17 mg, 0250 mmol) was dissolved in THF (1 mL), and 6 M HCl (1 mL) was added. The mixture was stirred at room temperature for 16 h under a nitrogen atmosphere. The reaction solution was filtered and concentrated to give a crude product. The crude product was purified by reversed-phase column chromatography (H₂O/ACN) to give a racemate of 24 (1.4 mg, yield: 9.6%) as a white solid, and the racemate was further resolved on a chiral column to give compound 24.

LCMS [M+H]⁺ = 580.2.

¹H NMR (400 MHz, DMSO) δ 9.38 (d, J = 0.7 Hz, 2H), 7.70 (d, J = 1.1 Hz, 1H), 7.66 (d, J = 1.0 Hz, 1H), 7.54 (d, J = 8.6 Hz, 2H), 7.33 (d, J = 8.6 Hz, 2H), 6.66 (t, J = 7.8 Hz, 1H), 6.23 (s, 1H), 6.16 (d, J = 7.8 Hz, 1H), 6.05 (d, J = 7.4 Hz, 1H), 5.65 (s, 1H), 5.09 (d, J = 4.9 Hz, 1H), 4.84-4.78 (m, 2H), 4.33 (d, J = 13.9 Hz, 1H), 4.13 (dd, J = 15.2, 9.8 Hz, 1H), 3.91 (s, 3H), 3.87 (s, 3H), 3.09 (s, 3H).

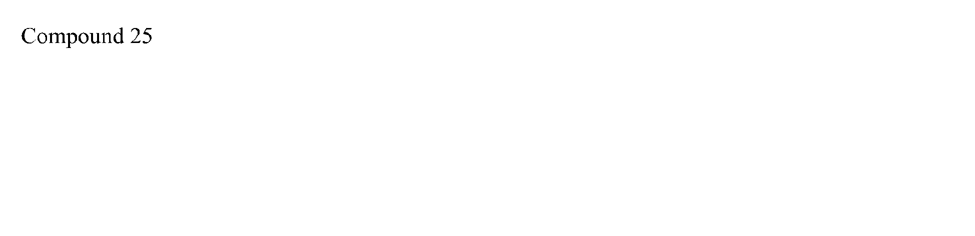

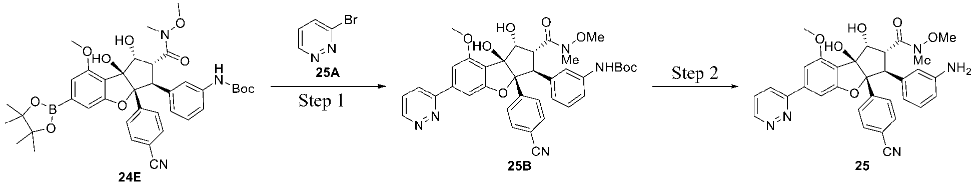

Step 1: The racemate of compound 24E (10 mg, 0.01 mmol) was dissolved in dioxane/water (5 mL/1 mL), and compound 25A (2.19 mg, 0.02 mmol), Na₂CO₃ (4.37 mg, 0.04 mmol), and Pd(dppf)Cl₂ (1.11 mg, 0.001 mmol) were added. The mixture was microwaved to 100 °C and left to react for 1 h under a nitrogen atmosphere. The reaction solution was filtered, and the filter cake was washed with ethyl acetate (2 mL × 3). After concentration, a racemate of 25B (12 mg) was obtained as a yellow solid, and the product was directly used in the next step. LCMS [M+H-C₄H₈]⁺ = 624.3.

Step 2: 6 N HCl (0.3 mL) was added to a solution of the racemate of compound 25B (12 mg, 0.02 mmol) in tetrahydrofuran (0.5 mL), and the mixture was stirred at room temperature for 12 h. The reaction solution was directly purified by preparative HPLC to give a racemate of 25 (6.3 mg, yield: 73%) as a white solid, and the racemate was further resolved on a chiral column to give compound 25.

LCMS [M+H]⁺ = 580.0.

¹H NMR (400 MHz, DMSO-*d*₆) δ 9.25-9.23 (m, 1H), 8.33-8.31 (m, 1H), 7.83-7.80 (m, 1H), 7.55 (d, *J =* 8.4 Hz, 2H), 7.40 (d, *J =* 4.8 Hz, 2H), 7.34 (d, *J =* 8.8 Hz, 2H), 6.68 (t, *J =* 7.6 Hz, 1H), 6.22-6.17 (m, 2H), 6.08 (d, *J=* 6.0 Hz, 1H), 5.59 (s, 1H), 4.80 (d, *J= 4.8* Hz, 1H), 4.33 (d, *J=* 14.0 Hz, 1H), 4.17-4.13 (m, 1H), 3.91 (s, 3H), 3.88 (s, 3H), 3.09 (s, 3H).

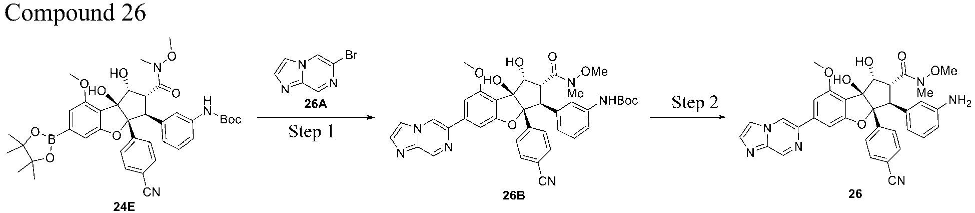

Step 1: The racemate of compound 24E (20 mg, 0.03 mmol) was dissolved in dioxane/water (5 mL/1 mL), and compound 26A (8.17 mg, 0.04 mmol), Na₂CO₃ (8.74 mg, 0.08 mmol), and Pd(dppf)Cl₂ (2.23 mg, 0.001 mmol) were added. The mixture was microwaved to 100 °C and left to react for 1 h under a nitrogen atmosphere. The reaction solution was filtered, and the filter cake was washed with ethyl acetate (2 mL × 3). After concentration, a racemate of 26B (19 mg) was obtained as a yellow solid, and the product was directly used in the next step. LCMS [M+H]⁺ = 719.3.

Step 2: 6 N HCl (0.3 mL) was added to a solution of the racemate of compound 26B (19 mg, 0.02 mmol) in tetrahydrofuran (0.5 mL), and the mixture was stirred at room temperature for 12 h. The reaction solution was directly purified by preparative HPLC to give a racemate of 26 (2.3 mg, yield: 17%) as a white solid, and the racemate was further resolved on a chiral column to give compound 26.

LCMS [M+H]⁺ = 619.2.

¹H NMR (400 MHz, DMSO-*d*₆) δ 9.32 (d, *J =* 1.6 Hz, 1H), 9.19 (s, 1H), 8.14 (s, 1H), 7.88 (d, *J =* 0.8 Hz, 1H), 7.55 (d, *J =* 8.4Hz, 2H), 7.33 (d, *J =* 8.4Hz, 3H), 7.28 (s, 1H), 6.67 (t, *J =* 7.8Hz, 1H), 6.20-6.16 (m, 2H), 6.05 (d, *J =* 7.6 Hz, 1H), 5.53 (s, 1H), 4.98 (d, *J* = 4.4 Hz, 1H), 4.82-4.80 (m, 3H), 4.30 (d, *J =* 14.0Hz, 1H), 4.15-4.11 (m, 1H), 3.90 (s, 3H), 3.87 (s, 3H), 3.09 (s, 3H).

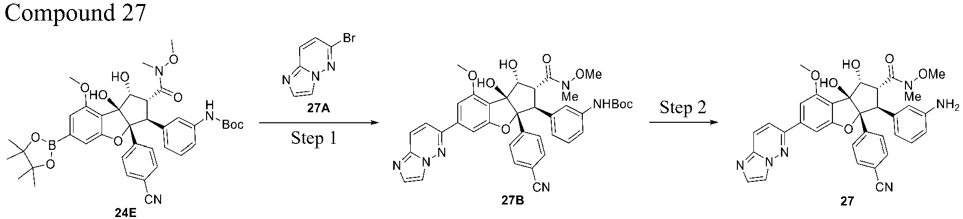

Step 1: The racemate of compound 24E (20 mg, 0.03 mmol) was dissolved in dioxane/water (5 mL/1 mL), and compound 27A (8.17 mg, 0.04 mmol), Na₂CO₃ (8.74 mg, 0.08 mmol), and Pd(dppf)Cl₂ (2.23 mg, 0.001 mmol) were added. The mixture was microwaved to 100 °C and left to react for 1 h under a nitrogen atmosphere. The reaction solution was filtered, and the filter cake was washed with ethyl acetate (2 mL × 3). After concentration, a racemate of 27B (18 mg) was obtained as a yellow solid, and the product was directly used in the next step. LCMS [M+H]⁺ = 719.3.

Step 2: 6 N HCl (0.3 mL) was added to a solution of the racemate of compound 27B (18 mg, 0.03 mmol) in tetrahydrofuran (0.5 mL), and the mixture was stirred at room temperature for 12 h. The reaction solution was directly purified by preparative HPLC to give a racemate of 27 (1.5 mg, yield: 9.7%) as a white solid, and the racemate was further resolved on a chiral column to give compound 27.

LCMS [M+H]⁺ = 619.2.

¹H NMR (400 MHz, DMSO-*d*₆) δ 8.38 (s, 1H), 8.24 (d, *J* = 9.6 Hz, 1H), 7.91 (d, *J* = 9.6 Hz, 1H), 7.84 (s, 1H), 7.55 (d, *J* = 8.4 Hz, 2H), 7.35 (s, 2H), 7.32 (s, 1H), 7.23 (s, 1H), 6.67 (t, *J* = 7.8 Hz, 1H), 6.21 (s, 1H), 6.17 (d, *J* = 7.6 Hz, 1H), 6.07 (d, *J =* 7.6 Hz, 1H), 5.60 (s, 1H), 5.05 (d, *J* = 4.8 Hz, 1H), 4.82-4.79 (m, 3H), 4.33 (d, *J =* 14.0 Hz, 1H), 4.17-4.09 (m, 1H), 3.91 (s, 3H), 3.88 (s, 3H), 3.09 (s, 3H).

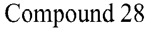

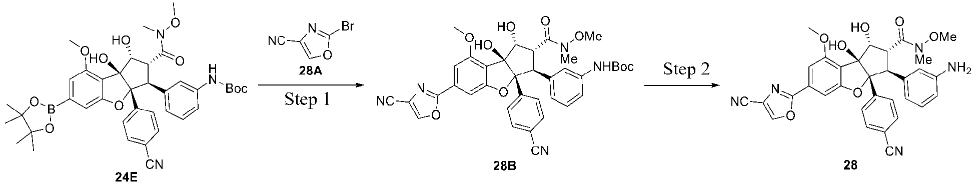

Step 1: The racemate of compound 24E (20 mg, 0.03 mmol) was dissolved in dioxane/water (3 mL/1 mL), and compound 28A (7.13 mg, 0.04 mmol), Na₂CO₃ (8.74 mg, 0.08 mmol), and Pd(dppf)Cl₂ (2.23 mg, 0.001 mmol) were added. The mixture was microwaved to 100 °C and left to react for 1 h under a nitrogen atmosphere. The reaction solution was filtered, and the filter cake was washed with ethyl acetate (2 mL × 3). After concentration, a racemate of compound 28B (crude, 20 mg) was obtained as a yellow solid, and the product was directly used in the next step. LCMS [M+H-C₄H₈]⁺ = 638.2.

Step 2: 6 N HCl (0.5 mL) was added to a solution of the racemate of compound 28B (20 mg, 0.03 mmol) in tetrahydrofuran (0.5 mL), and the mixture was stirred at room temperature for 12 h. The reaction solution was directly purified by preparative HPLC to give a racemate of 28 (3.8 mg, yield: 22%) as a white solid, and the racemate was further resolved on a chiral column to give compound 28.

LCMS [M+H]⁺ = 594.0.

¹H NMR (400 MHz, DMSO-*d*₆) δ 9.25 (s, 1H), 7.53 (d, *J =* 8.4 Hz, 2H), 7.30 (d, *J =* 8.4 Hz, 2H), 7.24 (d, *J =* 1.2 Hz, 1H), 7.16 (d, *J =* 1.2 Hz, 1H), 6.66 (t, *J* = 15.2 Hz, 1H), 6.20 (s, 1H), 6.16 (d, *J =* 8.0 Hz, 1H), 6.06 (d, *J =* 7.6 Hz, 1H), 5.67 (s, 1H), 5.13 (d, *J* = 4.8 Hz, 1H), 4.83-4.76 (m, 3H), 4.34 (d, *J* = 14.0 Hz, 1H), 4.17-4.10 (m, 1H), 3.90 (s, 3H), 3.85 (s, 3H), 3.09 (s, 3H).

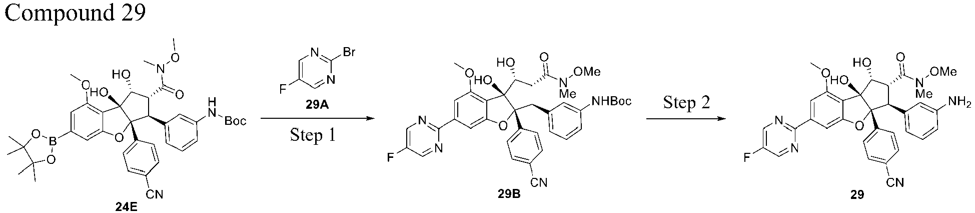

Step 1: The racemate of compound 24E (20 mg, 0.03 mmol) was dissolved in dioxane/water (3 mL/1 mL), and compound 29A (7.30 mg, 0.04 mmol), Na₂CO₃ (8.74 mg, 0.08 mmol), and Pd(dppf)Cl₂ (2.23 mg, 0.001 mmol) were added. The mixture was microwaved to 100 °C and left to react for 1 h under a nitrogen atmosphere. The reaction solution was filtered, and the filter cake was washed with ethyl acetate (2 mL × 3). After concentration, a racemate of 29B (20 mg) was obtained as a yellow solid, and the product was directly used in the next step. LCMS [M+H-C₄H₈]⁺ = 642.2.

Step 2: 6 N HCl (0.3 mL) was added to a solution of the racemate of compound 29B (20 mg, 0.03 mmol) in tetrahydrofuran (0.5 mL), and the mixture was stirred at room temperature for 12 h. The reaction solution was directly purified by preparative HPLC to give a racemate of 29 (1.5 mg, yield: 9%) as a white solid, and the racemate was further resolved on a chiral column to give compound 29.

LCMS [M+H]⁺ = 598.2.

¹H NMR (400 MHz, DMSO-*d*₆) δ 9.01 (s, 2H), 7.57-7.53 (m, 4H), 7.32 (d, *J* = 8.8 Hz, 2H), 6.66 (t, *J =* 7.6 Hz, 1H), 6.23 (s, 1H), 6.17 (d, *J =* 12.0 Hz, 1H), 6.04 (d, *J* = 7.6 Hz, 1H), 5.59 (s, 1H), 5.03 (d, *J* = 4.8 Hz, 1H), 4.87-4.79 (m, 3H), 4.30 (d, *J =* 14.0 Hz, 1H), 4.16-4.09 (m, 1H), 3.90 (s, 3H), 3.85 (s, 3H), 3.09 (s, 3H).

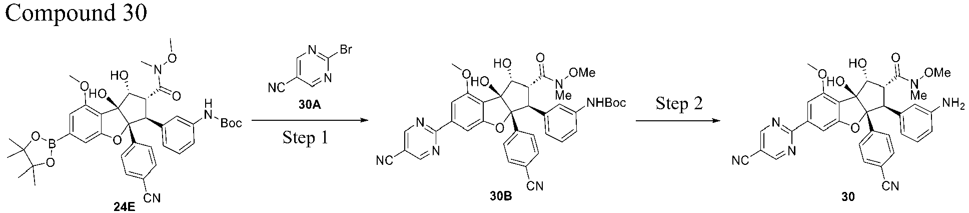

Step 1: The racemate of compound 24E (20 mg, 0.03 mmol) was dissolved in dioxane/water (3 mL/1 mL), and compound 30A (7.59 mg, 0.04 mmol), Na₂CO₃ (8.74 mg, 0.08 mmol), and Pd(dppf)Cl₂ (2.23 mg, 0.001 mmol) were added. The mixture was microwaved to 100 °C and left to react for 1 h under a nitrogen atmosphere. The reaction solution was filtered, and the filter cake was washed with ethyl acetate (2 mL × 3). After concentration, a racemate of 30B (20 mg) was obtained as a yellow solid, and the product was directly used in the next step. LCMS [M+H-C₄H₈]⁺ = 649.2.

Step 2: 6 N HCl (0.3 mL) was added to a solution of the racemate of compound 30B (20 mg, 0.03 mmol) in tetrahydrofuran (0.5 mL), and the mixture was stirred at room temperature for 12 h. The reaction solution was directly purified by preparative HPLC to give a racemate of 30 (1.1 mg, yield: 6.4%) as a white solid, and the racemate was further resolved on a chiral column to give compound 30.

LCMS [M+H]⁺ = 605.2.

¹H NMR (400 MHz, DMSO-*d*₆) δ 8.68 (s, 2H), 7.55-7.53 (m, 4H), 7.33 (d, *J* = 8.8 Hz, 2H), 6.66 (t, *J =* 7.6 Hz, 1H), 6.22 (s, 1H), 6.17-6.15 (m, 1H), 6.03 (d, *J =* 8.0 Hz, 1H), 5.54 (s, 1H), 4.98 (d, *J =* 4.8 Hz, 1H), 4.83-4.79 (m, 3H), 4.29 (d, *J =* 14.0 Hz, 1H), 4.16-4.10 (m, 1H), 3.98 (s, 3H), 3.90 (s, 3H), 3.85 (s, 3H), 3.08 (s, 3H).

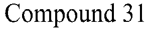

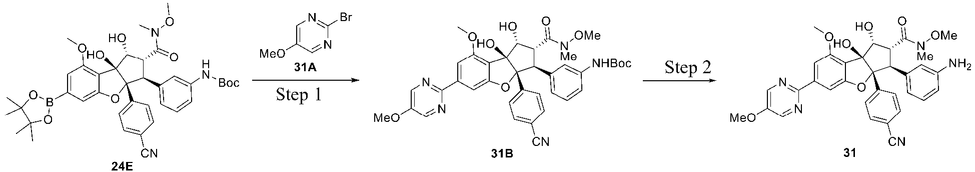

Step 1: The racemate of compound 24E (20 mg, 0.03 mmol) was dissolved in dioxane/water (3 mL/1 mL), and compound 31A (7.80 mg, 0.04 mmol), Na₂CO₃ (8.74 mg, 0.08 mmol), and Pd(dppf)Cl₂ (2.23 mg, 0.001 mmol) were added. The mixture was microwaved to 100 °C and left to react for 1 h under a nitrogen atmosphere. The reaction solution was filtered, and the filter cake was washed with ethyl acetate (2 mL × 3). After concentration, a racemate of 31B (20 mg) was obtained as a yellow solid, and the product was directly used in the next step. LCMS [M+H-C₄H₈]⁺ = 654.2.

Step 2: 6 N HCl (0.3 mL) was added to a solution of the racemate of compound 31B (20 mg, 0.03 mmol) in tetrahydrofuran (0.5 mL), and the mixture was stirred at room temperature for 12 h. The reaction solution was directly purified by preparative HPLC to give a racemate of 31 (3.3 mg, yield: 19%) as a white solid, and the racemate was further resolved on a chiral column to give compound 31.

LCMS [M+H]⁺ = 610.2.

¹H NMR (400 MHz, DMSO-*d*₆) δ 8.68 (s, 2H), 7.55-7.53 (m, 4H), 7.33 (d, *J =* 8.8 Hz, 2H), 6.66 (t*, J =* 7.6 Hz, 1H), 6.22 (s, 1H), 6.17-6.15 (m, 1H), 6.03 (d, *J* = 8.0 Hz, 1H), 5.54 (s, 1H), 4.98 (d, *J* = 4.8 Hz, 1H), 4.83-4.79 (m, 3H), 4.29 (d, *J =* 14.0 Hz, 1H), 4.16-4.10 (m, 1H), 3.98 (s, 3H), 3.90 (s, 3H), 3.85 (s, 3H), 3.08 (s, 3H).

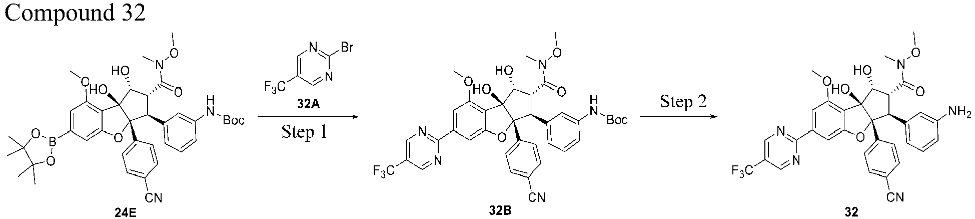

Step 1: The racemate of compound 24E (20 mg, 0.0300 mmol) was dissolved in dioxane (2.0 mL) and water (0.5 mL), and compound 32A (9.00 mg, 0.0550 mmol), Pd(dppf)Cl₂ (2.24 mg, 0.0027 mmol), and Na₂CO₃ (12.00 mg, 0.1099 mmol) were added. The mixture was microwaved to 100 °C and left to react for 1 h under a nitrogen atmosphere. The reaction solution was filtered and concentrated to give a racemate of 32B (17 mg, yield: 84%) as a yellow solid. LCMS [M+H-C₄H₈]⁺ = 692.2.

Step 2: The racemate of compound 32B (17 mg, 0269 mmol) was dissolved in THF (1 mL), and 6 M HCl (1 mL) was added. The mixture was stirred at room temperature for 16 h. The reaction solution was filtered and concentrated to give a crude product. The crude product was purified by preparative reversed-phase column chromatography [H₂O (containing 0.1% FA)/ACN] to give a racemate of 32 (1.1 mg, yield: 7.5%) as a white solid, and the racemate was further resolved on a chiral column to give compound 32.

LCMS [M+H]⁺ = 648.2.

¹H NMR (400 MHz, DMSO) δ 9.38 (d, J = 0.7 Hz, 2H), 7.70 (d, J = 1.1 Hz, 1H), 7.66 (d, J = 1.0 Hz, 1H), 7.54 (d, J = 8.6 Hz, 2H), 7.33 (d, J = 8.6 Hz, 2H), 6.66 (t, J = 7.8 Hz, 1H), 6.23 (s, 1H), 6.16 (d, J = 7.8 Hz, 1H), 6.05 (d, J = 7.4 Hz, 1H), 5.65 (s, 1H), 5.09 (d, J = 4.9 Hz, 1H), 4.84-4.78 (m, 2H), 4.33 (d, J = 13.9 Hz, 1H), 4.13 (dd, J = 15.2, 9.8 Hz, 1H), 3.91 (s, 3H), 3.87 (s, 3H), 3.09 (s, 3H).

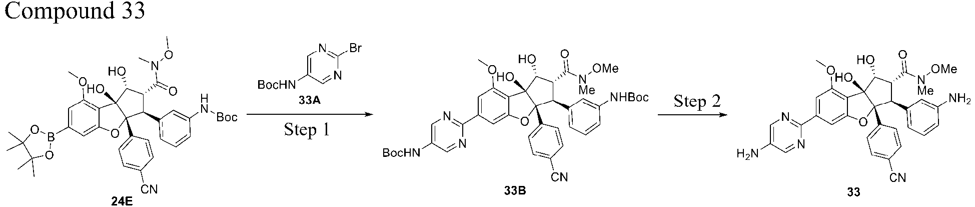

Step 1: The racemate of compound 24E (20 mg, 0.03 mmol) was dissolved in dioxane/water (3 mL/1 mL), and compound 33A (11.26 mg, 0.04 mmol), Na₂CO₃ (8.74 mg, 0.08 mmol), and Pd(dppf)Cl₂ (2.23 mg, 0.001 mmol) were added. The mixture was microwaved to 100 °C and left to react for 1 h under a nitrogen atmosphere. The reaction solution was filtered, and the filter cake was washed with ethyl acetate (2 mL × 3). After concentration, a racemate of 33B (23 mg) was obtained as a yellow solid, and the product was directly used in the next step. LCMS [M+H-C₄H₈]⁺ = 739.3.

Step 2: 6 N HCl (0.5 mL) was added to a solution of the racemate of compound 33B (23 mg, 0.03 mmol) in tetrahydrofuran (0.5 mL), and the mixture was stirred at room temperature for 12 h. The reaction solution was directly purified by preparative HPLC to give a racemate of 33 (1.1 mg, yield: 6.4%) as a white solid, and the racemate was further resolved on a chiral column to give compound 33.

LCMS [M+H]⁺ = 595.2.

¹H NMR (400 MHz, DMSO-*d*₆) δ 8.22 (s, 2H), 7.54 (d, *J* = 8.8 Hz, 2H), 7.44 (d, *J* = 4.8 Hz, 2H), 7.32 (d, *J =* 8.8 Hz, 2H), 6.65 (t, *J =* 7.8 Hz, 1H), 6.22 (s, 1H), 6.16 (d, *J* = 8.0 Hz, 1H), 6.01 (d, *J* = 7.6 Hz, 1H), 5.75 (s, 2H), 5.47 (s, 1H), 4.89 (d, *J =* 4.4 Hz, 1H), 4.84-4.77 (m, 3H), 4.26 (d, *J =* 13.6 Hz, 1H), 3.89 (s, 3H), 3.83 (s, 3H), 3.08 (s, 3H).

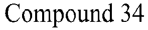

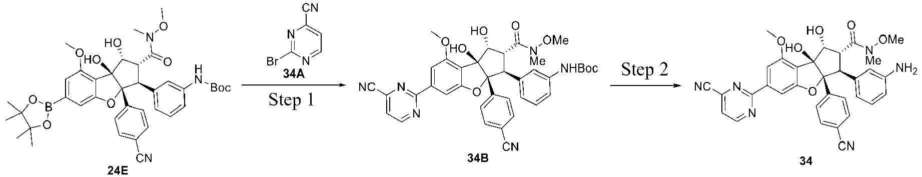

Step 1: The racemate of compound 24E (20 mg, 0.03 mmol) was dissolved in dioxane/water (3 mL/1 mL), and compound 34A (6.51 mg, 0.04 mmol), Na₂CO₃ (5.83 mg, 0.08 mmol), and Pd(dppf)Cl₂ (4.89 mg, 0.001 mmol) were added. The mixture was microwaved to 100 °C and left to react for 1 h under a nitrogen atmosphere. The reaction solution was filtered, and the filter cake was washed with ethyl acetate (2 mL × 3). After concentration, a racemate of 34B (20 mg) was obtained as a yellow solid, and the product was directly used in the next step. LCMS [M+H-C₄H₈]⁺ = 649.2.

Step 2: 6 N HCl (0.5 mL) was added to a solution of the racemate of compound 34B (20 mg, 0.03 mmol) in tetrahydrofuran (0.5 mL), and the mixture was stirred at room temperature for 12 h. The reaction solution was directly purified by preparative HPLC to give a racemate of 34 (4.6 mg, yield: 27%) as a white solid, and the racemate was further resolved on a chiral column to give compound 34.

LCMS [M+H]⁺ = 605.2.

¹H NMR (400 MHz, DMSO-*d*₆) δ 9.26 (d, *J* = 4.8 Hz, 1H), 8.10 (d, *J* = 4.8 Hz, 1H), 7.62 (d, *J =* 1.0 Hz, 1H), 7.58-7.52 (m, 3H), 7.33 (d, *J* = 8.4 Hz, 2H), 6.66 (t, *J =* 7.6 Hz, 1H), 6.23 (s, 1H), 6.16 (d*, J =* 8.0 Hz, 1H), 6.04 (d*, J =* 7.6 Hz, 1H), 5.64 (s, 1H), 5.08 (d, *J* = 4.8 Hz, 1H), 4.93-4.76 (m, 3H), 4.31 (d, *J =* 14.0 Hz, 1H), 4.17 (s, 1H), 3.91(S,3H), 3.89 (s, 3H), 3.09 (s, 3H).

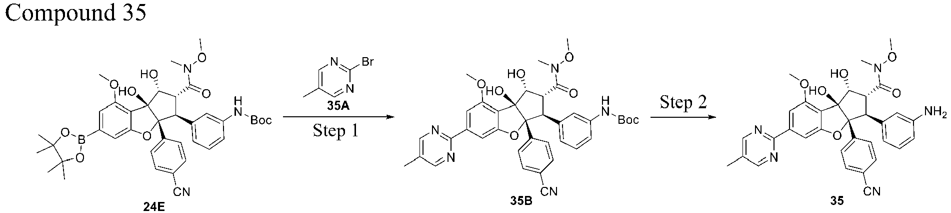

Step 1: The racemate of compound 24E (20 mg, 0.0275 mmol) was dissolved in dioxane (2.0 mL) and water (0.4 mL), and compound 35A (9.00 mg, 0.0550 mmol), Pd(dppf)Cl₂ (2.24 mg, 0.0027 mmol), and Na₂CO₃ (12.00 mg, 0.1099 mmol) were added. The mixture was microwaved to 100 °C and left to react for 1 h under a nitrogen atmosphere. The reaction solution was filtered and concentrated to give a crude product, and the crude product was purified by reversed-phase column chromatography (H₂O/ACN) to give a racemate of 35B (12 mg, yield: 63%) as a yellow solid. LCMS [M+H-C₄H₈]⁺ = 638.2.

Step 2: Compound 35B (12 mg, 0.0173 mmol) was dissolved in THF (1 mL), and 6 M HCl (1 mL) was added. The mixture was stirred at room temperature for 16 h. The reaction solution was filtered and concentrated to give a crude product. The crude product was purified by reversed-phase column chromatography [H₂O (containing 0.1% FA)/ACN] to give 35 (2.6 mg, yield: 25%) as a white solid, and the product was further resolved on a chiral column to give compound 35.

LCMS [M+H]⁺ = 594.2.

¹H NMR (400 MHz, DMSO) δ 9.38 (d, J = 0.7 Hz, 2H), 7.70 (d, J = 1.1 Hz, 1H), 7.66 (d, J = 1.0 Hz, 1H), 7.54 (d, J = 8.6 Hz, 2H), 7.33 (d, J = 8.6 Hz, 2H), 6.66 (t, J = 7.8 Hz, 1H), 6.23 (s, 1H), 6.16 (d, J = 7.8 Hz, 1H), 6.05 (d, J = 7.4 Hz, 1H), 5.65 (s, 1H), 5.09 (d, J = 4.9 Hz, 1H), 4.84-4.78 (m, 2H), 4.33 (d, J = 13.9 Hz, 1H), 4.13 (dd, J = 15.2, 9.8 Hz, 1H), 3.91 (s, 3H), 3.87 (s, 3H), 3.09 (s, 3H).

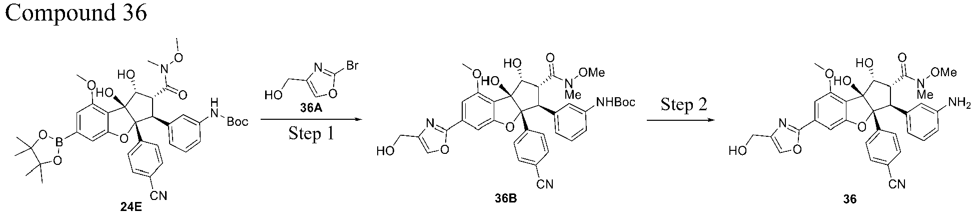

Step 1: Compound 36A (9.8 mg, 0.05 mmol), Na₂CO₃ (8.8 mg, 0.08 mmol), and Pd(dppf)Cl₂ (2.5 mg, 0.00 mmol) were added to a solution of the racemate of compound 24E (20 mg, 0.03 mmol) in dioxane/H₂O (2.5 mL/0.5 mL), and the mixture was microwaved to 100 °C and left to react for 1 h under a nitrogen atmosphere. The reaction solution was concentrated, and the crude product was purified by reversed-phase column chromatography (ACN/H₂O) to give a racemate of 36B (10.0 mg, yield: 52.1%) as a white solid. LCMS [M+H-C₄H₈]⁺ = 643.2.

Step 2: HCl (6 N, 2 mL) was added to a solution of the racemate of compound 36B (10.0 mg, 0.01 mmol) in THF (2 mL), and the mixture was stirred overnight at room temperature. The reaction solution was directly purified by reversed-phase column chromatography (ACN/H₂O) to give a racemate of 36 (6.4 mg, yield: 74.7%) as a white solid, and the racemate was further resolved on a chiral column to give compound 36. LCMS [M+H]⁺ = 599.3.

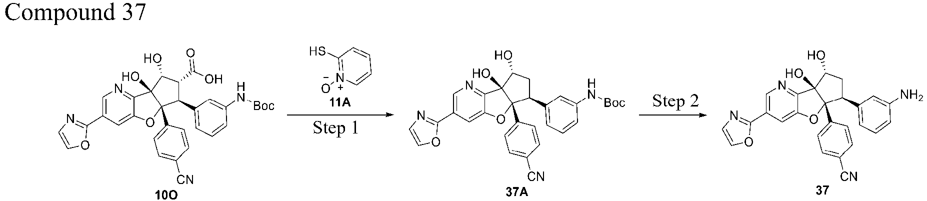

Step 1: The racemate of compound 10O (20 mg, 0.03 mmol), compound 11A (42.6 mg, 0.3 mmol), HOAT (6.12 mg, 0.045 mmol), tert-dodecyl mercaptan (60.6 mg, 0.3 mmol), TMP (5.48 mg, 0.045 mmol), zinc meso-tetraphenylporphine (2.03 mg, 0.003 mmol), and DMF (2 mL) were added to a microwave tube. After the reaction system was purged with nitrogen, the microwave tube was placed in a hot water bath at 80 °C, and the system was stirred while being irradiated with 25 W red light. A solution of EDCI (57.3 mg, 0.3 mmol) in DMF (2 mL) was slowly added dropwise to the reaction solution, and the mixture was then stirred for 30 min. After the reaction was complete, the reaction solution was purified by silica gel column chromatography (H2O/ACN = 40%:60%) to give a racemate of compound 37A (8.6 mg, yield: 46%). LCMS [M+H]⁺ = 553.2.

Step 2: The racemate of compound 37A (8.6 mg, 0.015 mmol) was dissolved in THF (1 mL), and 6 M HCl (2 mL) was added. The reaction solution was stirred at room temperature (20 °C) for 2 h, concentrated to dryness by rotary evaporation, and purified by silica gel column chromatography (H2O/ACN = 55:45) to give a racemate of compound 37 (3.7 mg, yield: 52%), and the racemate was further resolved on a chiral column to give compound 37.

LCMS [M+H]⁺ = 453.2.

¹H NMR (400 MHz, DMSO) δ 8.80 (d, *J* = 1.7 Hz, 1H), 8.33 (s, 1H), 7.87 (d, *J =* 1.7 Hz, 1H), 7.54 (d, *J* = 8.6 Hz, 2H), 7.47 (d, *J* = 0.6 Hz, 1H), 7.30 (d, *J* = 8.6 Hz, 2H), 6.88 (s, 1H), 6.55 (s, 1H), 6.47 (d, *J =* 23.6 Hz, 2H), 6.03 (s, 1H), 5.21 (s, 1H), 4.51 (d, *J =* 3.9 Hz, 1H), 4.24 (dd, *J* = 14.0, 6.0 Hz, 1H), 2.80 (td, *J =* 13.4, 4.3 Hz, 1H), 2.16 (dd, *J* = 12.6, 6.3 Hz, 1H).

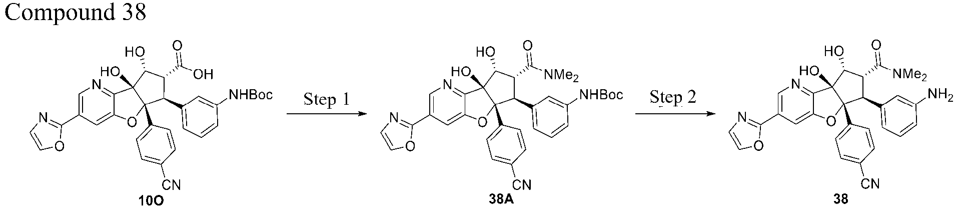

Step 1: A dimethylamine solution (0.1 mL, 0.2 mmol), HATU (15 mg, 0.04 mmol), and TMP (5 mg, 0.04 mmol) were added to a solution of the racemate of compound 10O (24 mg, 0.04 mmol) in DMF (2 mL), and the mixture was stirred at 20 °C for 2 h. The reaction solution was directly purified by reversed-phase column chromatography (MeCN/H₂O/FA) to give a racemate of 38A (12 mg, yield: 48%) as a white solid. LCMS [M+H]⁺ = 624.3.

Step 2: 6 M HCl (2 mL) was added to a solution of the racemate of compound 38A (12 mg, 0.02 mmol) in THF (2 mL), and the mixture was stirred at 25 °C for 18 h. The reaction solution was directly purified by reversed-phase column chromatography (MeCN/H₂O/FA) to give a racemate of 38 (4.7 mg, yield: 47%) as a white solid, and the racemate was further resolved on a chiral column to give compound 38.

LCMS [M+H]⁺ = 524.2.

¹H NMR (400 MHz, DMSO) δ 8.78 (d, *J* = 1.7 Hz, 1H), 8.33 (s, 1H), 7.89 (d, *J =* 1.7 Hz, 1H), 7.53 (s, 1H), 7.51 (s, 1H), 7.47 (s, 1H), 7.37 (d, *J =* 8.5 Hz, 2H), 6.66 (t, *J =* 7.8 Hz, 1H), 6.22 (s, 1H), 6.20 (s, 1H), 6.14 (dd, *J* = 17.3, 7.6 Hz, 2H), 5.28 (d, *J* = 5.5 Hz, 1H), 4.79 (s, 2H), 4.73 (t, *J* = 5.3 Hz, 1H), 4.54 (d, *J* = 13.3 Hz, 1H), 4.21 (dd, *J* = 13.3, 5.3 Hz, 1H), 3.28 (s, 3H), 2.79 (s, 3H).

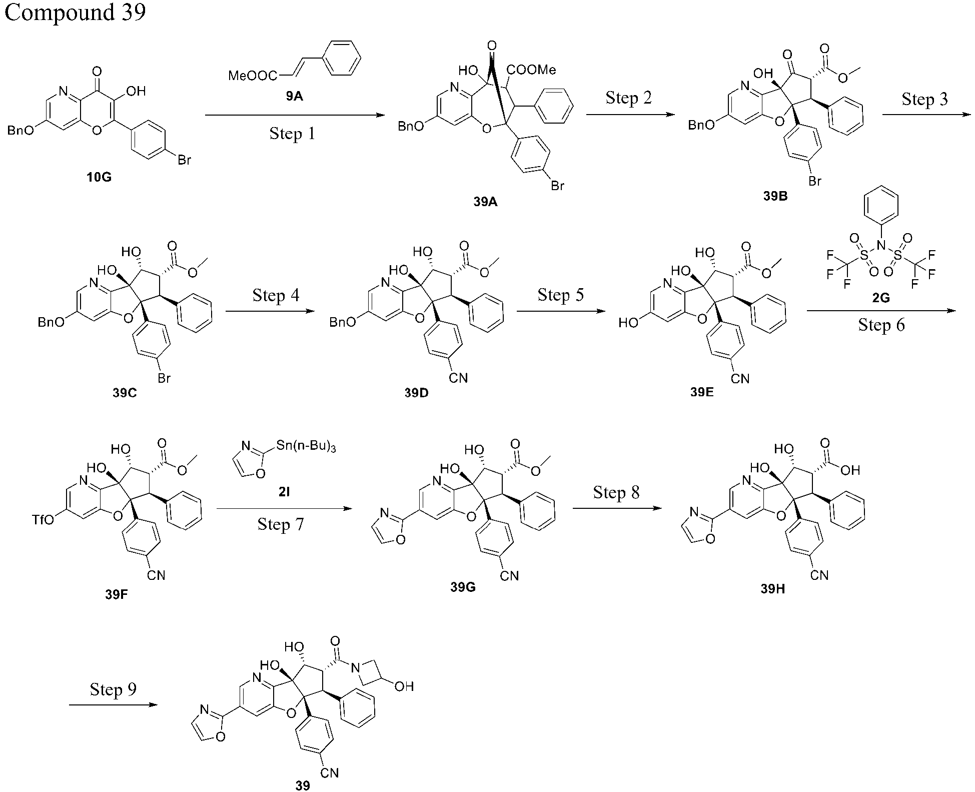

Step 1: After compound 10G (1 g, 2.36 mmol) and compound 9A (2.3 g, 14.14 mmol) were dissolved in CHCl₃/TFE (7/3, 15 mL), a 20 mL syringe was used to inject the reaction mixture at a rate of 12 mL/h through a syringe pump into a coil wound around a jacketed glass cylinder equipped with a 250 W ultraviolet lamp. The temperature of the external circulation cold trap was adjusted to keep the internal reaction temperature at 0-5 °C, and the UV lamp was turned on simultaneously. The reaction mixture was irradiated for 1 h, concentrated under reduced pressure to remove the solvent, and then separated by column chromatography (EA/PE = 1/3) to remove the excess cinnamate, giving a racemate of 39A (7 g, yield: 51%) as a light yellow solid. LCMS [M+H-H₂O]⁺ = 568.1.

Step 2: The racemate of compound 39A (4.9 g, 8.37 mmol) was dissolved in methanol (60 mL), and NaOMe (1.135 g, 20.94 mmol) was added at 0 °C. The mixture was stirred at 65 °C for 1.5 h. The reaction solution was concentrated, extracted with EA, washed with water, an NH₄Cl solution, and saturated brine, dried over anhydrous sodium sulfate, filtered, then concentrated, and purified by silica gel column chromatography (0.2% FA in EA/PE = 1/3) to give a racemate of compound 39B (3.6 g, 73%). LCMS [M+H]⁺ = 586.1.

Step 3: The racemate of compound 39B (3.6 g, 6.15 mmol) was dissolved in a mixed solvent of ACN/CHCl₃ (1/1, 40 mL/40 mL), and Na(AcO)₃BH (6.525 g, 30.77 mmol) and AcOH (3.695 g, 61.53 mmol) were added at 0 °C. The mixture was stirred at room temperature for 2 h. The reaction solution was concentrated, extracted with EA, washed with water and saturated brine, dried over anhydrous sodium sulfate, filtered, then concentrated, and purified by silica gel column chromatography (0.2% FA in EA/PE = 3/2) to give a racemate of compound 39C (1.9 g, yield: 53%). LCMS [M+H]⁺ = 588.1.

Step 4: Under a nitrogen atmosphere, a solution of the racemate of compound 39C (1500 mg, 2.55 mmol), Zn(CN)₂ (7745.6 mg, 6.37 mmol), Pd₂(dba)₃ (468 mg, 0.5 mmol), and DPPF (564 mg, 1.02 mmol) in DMP (30 mL) was stirred at 150 °C for 1 h. The reaction solution was diluted with water and extracted with EA three times (50 mL × 3). The organic phase was concentrated, and the crude product was purified by FLASH column chromatography (preparative low- to medium-pressure flash liquid chromatography) (DCM/MEOH = 0-10%) to give a racemate of 39D (1100 mg, yield: 80.7%) as a yellow solid. LCMS: [M+H]⁺ = 535.6.

Step 5: Theracemate of39D (1100 mg, 2.06 mmol) and Pd/C (700 mg) were added to a solution of EA/MeOH (20/20 mL), and the mixture was purged with hydrogen three times and stirred at room temperature for 16 h. The reaction solution was purified by FLASH column chromatography (preparative low- to medium-pressure flash liquid chromatography) (MeOH/DCM = 0-10%) to give aracemate of compound 39E (350 mg, yield: 38.2%). LCMS: [M+H]⁺ = 445.6.

Step 6: Theracemate of compound 39E (350 mg, 0.79 mmol) and K₂CO₃ (108 mg, 0.79 mmol) were added to DMF (5 mL), and compound 2G (281 mg, 0.79 mmol) was then added. The mixture was stirred at 60 °C for 1.5 h. The reaction solution was diluted with EA (30 mL), washed with H₂O (20 mL × 2), and then purified by FLASH column chromatography (preparative low- to medium-pressure flash liquid chromatography) (MeOH/DCM = 0-10%) to give aracemate of compound 39F (310 mg, yield: 68.3%). LCMS [M+H]⁺ = 577.6.

Step 7: Theracemate of compound 39F (300 mg, 0.7 mmol), compound 2I (756 mg, 2.1 mmol), and Pd(PPh₃)₄ (81 mg, 0.05 mmol) were added to DMF (4 mL), and CuI (26.5 mg, 0.14 mmol) was added. The mixture was stirred under microwave conditions at 135 °C for 5 h. The reaction solution was diluted with EA (30 mL), and the resulting mixture was washed with H₂O (20 mL × 2). The solution was purified by FLASH column chromatography (preparative low- to medium-pressure flash liquid chromatography) (MeOH/DCM = 0-15%) to give aracemate of39G (250 mg, yield: 71%) as a yellow solid. LCMS [M+H]⁺ = 496.2.

Step 8: The racemate of compound 39G (250 mg, 0.5 mmol) and LiOH·H₂O (60 mg, 1.5 mmol) were added to a mixed solution of THF/MeOH/H₂O (1.5/1.5/1.5 mL), and the mixture was stirred at 20 °C for 5 h. The reaction solution was purified by FLASH column chromatography (preparative low- to medium-pressure flash liquid chromatography) (ACN-H₂O) to give a racemate of 39H (170 mg, yield: 70%) as a yellow solid. LCMS [M+H]⁺ = 482.7.

Step 9: Theracemate of compound 39H (20 mg, 0.04 mmol), HATU (16 mg, 0.04 mmol), and TMP (15 mg, 0.12 mmol) were dissolved in DMF (2 mL), and 3-hydroxyazetidine (5.8 mg, 0.08 mmol) was added. The mixture was stirred at 20 °C for 2 h. The reaction solution was purified by FLASH column chromatography (preparative low- to medium-pressure flash liquid chromatography)

(MeOH/DCM = 0-10%) to give a racemate of 39 (19 mg, yield: 86%) as a white solid, and the racemate was further resolved on a chiral column to give compound 39. LCMS [M+H]⁺ = 537.7.

¹H NMR (400 MHz, DMSO) δ 8.79 (s, 1H), 8.44 (s, 1H), 8.33 (s, 1H), 7.93 (d, *J* = 1.6 Hz, 1H), 7.48 (d, *J =* 6.1 Hz, 3H), 7.37-7.30 (m, 2H), 7.06 (t, *J =* 6.8 Hz, 2H), 7.02-6.93 (m, 3H), 6.28 (d, *J* = 10.8 Hz, 1H), 5.82 (s, 1H), 5.41 (m, 1H), 4.67 (m, 2H), 4.53 (m, 2H), 4.07-3.96 (m, 3H), 3.62-3.55 (m, 1H).

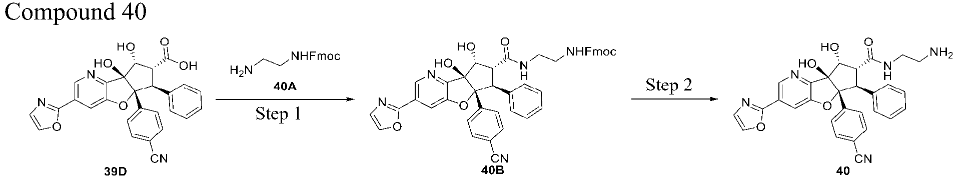

Step 1: The racemate of compound 39D (20 mg, 0.04 mmol), HATU (12 mg, 0.04 mmol), and TMP (14.5 mg, 0.12 mmol) were stirred in DMF (2 mL). Compound 40A (12 mg, 0.04 mmol) was added, and the mixture was stirred at 20 °C for 2 h and purified by normal-phase column chromatography (MeOH/DCM = 0-10%) to give a racemate of 40B (30 mg, yield: 94%) as a white solid. LCMS [M+H]⁺ = 747.0.

Step 2: The racemate of compound 40B (30 mg, 0.04 mmol) and piperidine (10 mg, 0.12 mol) were dissolved in DMF (1 mL), and the mixture was left to react at 20 °C for 1 h. The reaction solution was lyophilized to give a racemate of 40 (20 mg, yield: 87%) as a white solid, and the racemate was further resolved on a chiral column to give compound 40.

LCMS [M+H]⁺ = 524.2.

¹H NMR (400 MHz, DMSO) δ 8.80 (d, *J =* 1.7 Hz, 1H), 8.41 (t, *J* = 5.6 Hz, 1H), 8.34 (s, 1H), 7.93 (d, *J =* 1.7 Hz, 1H), 7.52 (d, *J =* 8.6 Hz, 2H), 7.48 (s, 1H), 7.30 (d, *J =* 8.5 Hz, 2H), 7.04 (ddd, *J =* 21.9, 14.9, 7.3 Hz, 6H), 6.26 (s, 1H), 4.67 (dd, *J =* 18.3, 9.1 Hz, 2H), 3.25 (dd, *J =* 12.6, 6.5 Hz, 3H), 2.75 (t, *J =* 6.6 Hz, 2H).

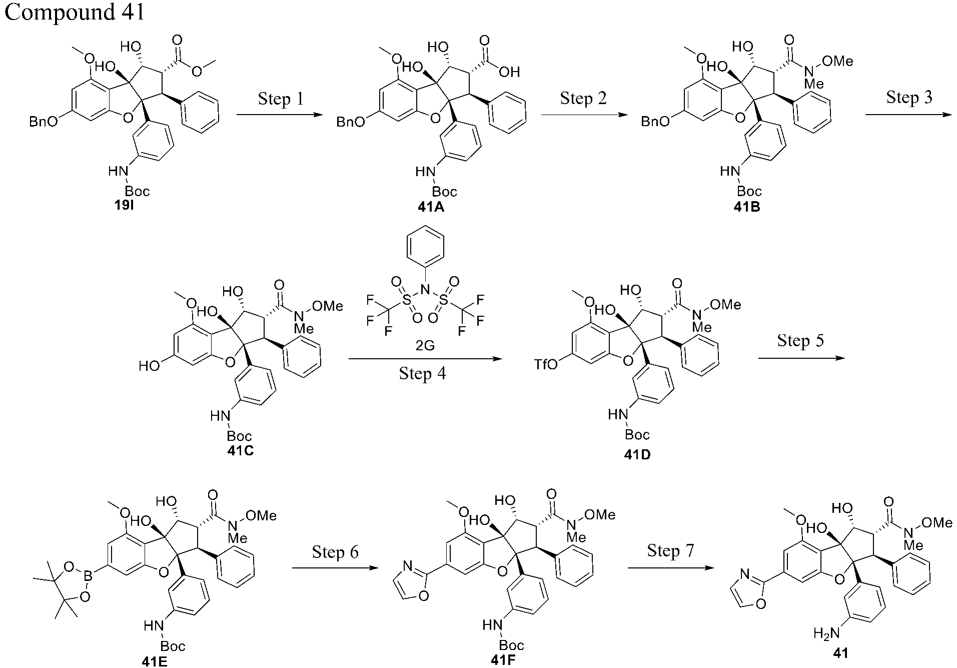

Step 1: LiOH·H₂O (19.3 mg, 0.46 mmol) was added to a solution of the racemate of compound 19I (100.0 mg, 0.15 mmol) in THF/H₂O/MeOH (3 mL/1 mL/1 mL), and the mixture was stirred at 40 °C for 3 h. The pH of the reaction solution was adjusted to about 5 with 3 M HCl, and the reaction solution was concentrated to dryness by rotary evaporation and purified by normal-phase column chromatography (DCM/MeOH) to give a racemate of compound 41A A (75 mg, yield: 77%). LCMS [M-H]⁻ = 638.2.

Step 2: TMP (56.8 mg, 0.47 mmol) was added to a solution of the racemate of compound 41A (75.0 mg, 0.12 mmol), dimethylhydroxylamine hydrochloride (22.8 mg, 0.23 mmol), HOAT (16.0 mg, 0.12 mmol), and HATU (89.2 mg, 0.23 mmol) in DMF (mL). The mixture was stirred at 40 °C for 16 h. The reaction solution was purified by normal-phase column chromatography (DCM/MeOH) to give a racemate of compound 41B (85 mg, yield: 110%). LCMS [M+H]⁺ = 683.1. Step 3: Pd(OH)₂/C (10%, 50 mg) was added to a mixed solution of the racemate of compound 41B (85.0 mg, 0.13 mmol) in EA/MeOH/THF/DCM (20:2:2:0.2, 10 mL), and the mixture was purged with hydrogen three times and stirred at room temperature for 16 h. The reaction solution was filtered, and the filter cake was rinsed with MeOH/DCM (1/10) three times. The filtrate was concentrated to dryness by rotary evaporation to give a racemate of compound 41C (50 mg, crude product, yield not calculated), and the product was directly used in the next step. LCMS [M+H]⁺ = 593.1.

Step 4: Compound 2G (30.2 mg, 0.0.84 mmol) was added to a solution of the racemate of compound 41C (50.0 mg, 0.08 mmol) and K₂CO₃ (23.3 mg, 0.169 mmol) in DMF (2 mL), and the mixture was stirred at 60 °C for 1 h. The reaction solution was extracted with water and ethyl acetate. The organic phase was dried over anhydrous sodium sulfate, filtered, and then concentrated, and the resulting crude product was purified by normal-phase column chromatography (PE/EA) to give a racemate of compound 41D (45 mg, yield: 73%). LCMS: [M+H]+ = 725.1.

Step 5: The racemate of compound 41D (33.0 mg, 0.05 mmol) was dissolved in a solution of dioxane (5 mL), and Pin₂B₂ (34.5 mg, 0.14 mmol), Pd(dppf)Cl₂ (7.4 mg, 0.01 mmol), and KOAc (13.4 mg, 0.18 mmol) were added. The mixture was stirred at 85 °C for 16 h under a N₂ atmosphere. The reaction solution was filtered, and the filter cake was washed with EA. The filtrate was concentrated and then purified by silica gel column chromatography (DCM/MeOH) to give a racemate of compound 41E (43 mg, crude product, yield not calculated), and the product was directly used in the next step. LCMS [M+H]⁺ = 703.3.

Step 6: The racemate of compound 41E (43.0 mg, 0.06 mmol), 2-bromooxazole (13.6 mg, 0.09 mmol), Na₂CO₃ (19.5 mg, 0.184 mmol), and Pd(dppf)Cl₂ (5.0 mg, 0.01 mmol) were added to a solution of dioxane/water (2 mL/1 mL). The reaction mixture was degassed under reduced pressure, purged with N₂ three times, and stirred under microwave conditions at 100 °C for 2 h. The reaction solution was concentrated and then purified by silica gel column chromatography (DCM/MeOH) to give a racemate of compound 41F (7 mg, yield: 17.7%). LCMS [M+H]⁺ = 644.1.

Step 7: The racemate of compound 41F (7.0 mg, 0.01 mmol) was mixed with THF (1 mL), and 6 M HCl (2 mL) was added at 0 °C. The mixture was stirred at 10 °C for 16 h. The reaction solution was concentrated and purified by preparative HPLC to give a racemate of 41 (0.8 mg, yield: 13.5%) as a white solid, and the racemate was further resolved on a chiral column to give compound 41. LCMS [M+H]⁺ = 544.2.

¹H NMR (400 MHz, DMSO) δ 8.25 (s, 1H), 7.41 (s, 1H), 7.17 (d, *J* = 14.2 Hz, 2H), 7.04 (t, *J* = 7.4 Hz, 2H), 6.98 (d, *J =* 7.2 Hz, 1H), 6.89 (d, *J =* 7.6 Hz, 2H), 6.69 (t, *J =* 7.8 Hz, 1H), 6.47 (s, 1H), 6.28 (d, *J =* 7.9 Hz, 1H), 6.19 (d, *J* = 8.2 Hz, 1H), 5.23 (s, 1H), 4.90 (s, 1H), 4.84 (s, 1H), 4.76 (s, 1H), 4.21 (s, 1H), 3.89 (s, 3H), 3.86 (s, 3H), 3.06 (s, 3H).

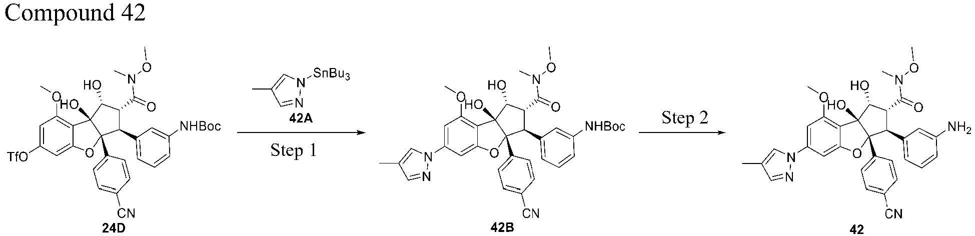

Step 1: The racemate of compound 24D (50 mg, 0.07 mmol), t-BuXPhos-PdG3 (7.94 mg, 0.01 mmol), and K₂CO₃ (450 mg, 3.26 mmol) were added to DMF (3 mL), and compound 42A (582 mg, 1.63 mmol) was added. The mixture was left to react at 120 °C for 5 h. The reaction solution was diluted with water and extracted with EA three times (10 mL × 3). The organic phase was concentrated and purified by preparative HPLC (ACN/H₂O) to give a racemate of 42B (8 mg, yield: 18%) as a white solid. LCMS [M+H]⁺ = 682.1.

Step 2: The racemate of compound 42B (10 mg, 0.01 mmol) and 6 M HCl (1.5 mol) were added to THF (1 mL), and the mixture was left to react at 40 °C for 5 h. The reaction solution was lyophilized to give a racemate of 42 (8 mg, yield: 94%) as a white solid. LCMS [M+H]⁺ = 582.1. The racemate was further resolved on a chiral column to give compound 42.

¹H NMR (400 MHz, DMSO) δ 8.36 (s, 1H), 7.63-7.50 (m, 3H), 7.31 (d, J = 8.5 Hz, 2H), 7.13-6.98 (m, 3H), 6.80 (s, 3H), 5.60 (s, 1H), 4.81 (s, 1H), 4.42 (d, J = 13.4 Hz, 1H), 4.17 (d, J = 12.4 Hz, 1H), 3.92 (s, 3H), 3.83 (s, 3H), 3.08 (s, 3H), 2.12 (s, 3H).

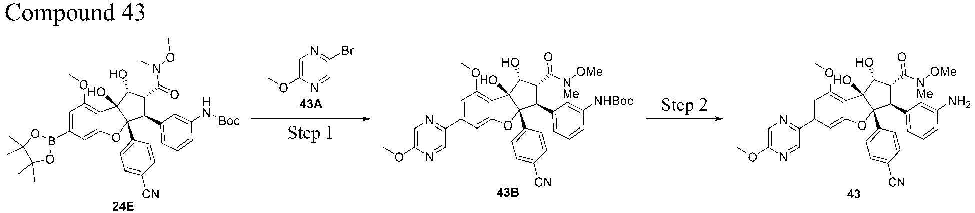

Step 1: The racemate of compound 24E (20 mg, 0.03 mmol) was dissolved in dioxane/water (3 mL/1 mL), and compound 43A (7.79 mg, 0.04 mmol), Na₂CO₃ (5.83 mg, 0.06 mmol), and Pd(dppf)Cl₂ (4.89 mg, 0.001 mmol) were added. The mixture was microwaved to 100 °C and left to react for 1 h under a nitrogen atmosphere. The reaction solution was filtered, and the filter cake was washed with ethyl acetate (2 mL × 3). After concentration, a racemate of 43B (20 mg) was obtained as a yellow solid, and the product was directly used in the next step. LCMS [M+H]⁺ = 710.2.

Step 2: 6 N HCl (0.5 mL) was added to a solution of the racemate of compound 43B (20 mg, 0.03 mmol) in tetrahydrofuran (0.5 mL), and the mixture was stirred at room temperature for 12 h. The reaction solution was directly purified by preparative HPLC to give a racemate of 43 (3.1 mg, yield: 18%) as a white solid, and the racemate was further resolved on a chiral column to give compound 43.

LCMS [M+H]+ = 610.2.

¹H NMR (400 MHz, DMSO-*d*₆) δ 8.89 (d, *J =* 1.2 Hz, 1H), 8.41 (d, *J* = 1.2 Hz, 1H), 7.54 (d, *J* = 8.4 Hz, 2H), 7.33 (s, 1H), 7.31 (s, 2H), 7.24 (d, *J =* 0.8 Hz, 1H), 6.67 (t, *J =* 7.8 Hz, 1H), 6.22-6.15 (m, 2H), 6.05 (d, *J =* 8.0 Hz, 1H), 5.51 (s, 1H), 4.98 (d, *J =* 4.4 Hz, 1H), 4.82-4.78 (m, 3H), 4.30 (d, *J* = 14.0 Hz, 1H), 4.13 (d, *J =* 14.8 Hz, 1H), 4.03 (s, 3H), 3.90 (s, 3H), 3.85 (s, 3H).

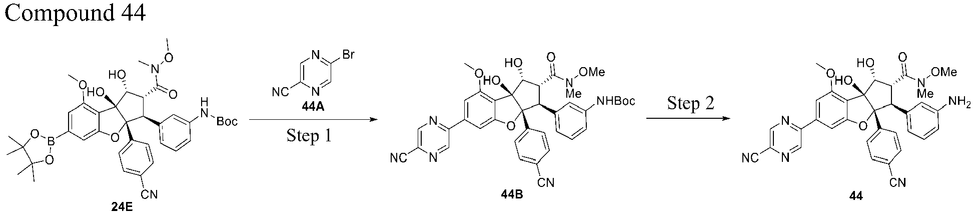

Step 1: The racemate of compound 24E (20 mg, 0.03 mmol) was dissolved in dioxane/water (3 mL/1 mL), and compound 44A (7.59 mg, 0.04 mmol), Na₂CO₃ (5.83 mg, 0.08 mmol), and Pd(dppf)Cl₂ (4.89 mg, 0.001 mmol) were added. The mixture was microwaved to 100 °C and left to react for 1 h under a nitrogen atmosphere. The reaction solution was filtered, and the filter cake was washed with ethyl acetate (2 mL × 3). After concentration, a racemate of 44B (20 mg) was obtained as a yellow solid, and the product was directly used in the next step. LCMS [M+H]⁺ = 705.0.

Step 2: 6 N HCl (0.5 mL) was added to a solution of the racemate of compound 44B (20 mg, 0.03 mmol) in tetrahydrofuran (0.5 mL), and the mixture was stirred at room temperature for 12 h. The reaction solution was directly purified by preparative HPLC to give a racemate of 44 (1.4 mg, yield: 8.2%) as a white solid, and the racemate was further resolved on a chiral column to give compound 44.

LCMS [M+H]+ = 605.2.

¹H NMR (400 MHz, DMSO-*d*₆) δ9.57 (d, *J* = 1.6 Hz, 1H), 9.28 (d, *J* = 1.2 Hz, 1H), 7.55-7.53 (m, 3H), 7.42 (s, 1H), 7.33-7.31 (m, 2H), 6.67 (t, *J =* 7.8 Hz, 1H), 6.20-6.15 (m, 2H), 6.07 (d, *J =* 8.0 Hz, 1H), 5.64 (s, 1H), 5.10 (d, *J =* 5.2 Hz, 1H), 4.82-4.78 (m, 3H), 4.34 (d, *J=* 13.6 Hz, 1H), 4.15 (d, *J =* 18.8 Hz, 1H), 3.90 (s, 3H), 3.87 (s, 3H), 3.09 (s, 3H).

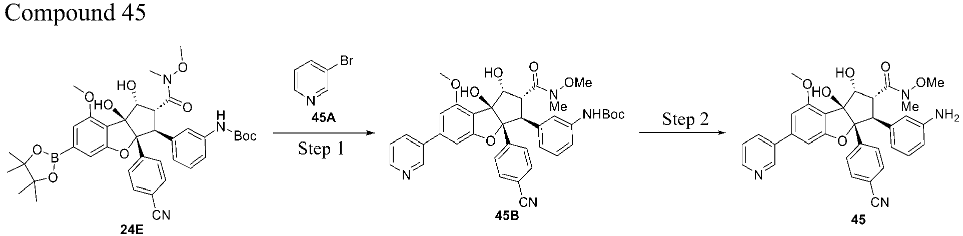

Step 1: The racemate of compound 24E (20 mg, 0.03 mmol) was dissolved in dioxane/water (3 mL/1 mL), and compound 45A (6.51 mg, 0.04 mmol), Na₂CO₃ (5.83 mg, 0.08 mmol), and Pd(dppf)Cl₂ (4.89 mg, 0.001 mmol) were added. The mixture was microwaved to 100 °C and left to react for 1 h under a nitrogen atmosphere. The reaction solution was filtered, and the filter cake was washed with ethyl acetate (2 mL × 3). After concentration, a racemate of 45B (20 mg) was obtained as a yellow solid, and the product was directly used in the next step.

Step 2: 6 N HCl (0.5 mL) was added to a solution of the racemate of compound 45B (20 mg, 0.03 mmol) in tetrahydrofuran (0.5 mL), and the mixture was stirred at room temperature for 12 h. The reaction solution was directly purified by preparative HPLC to give a racemate of 45 (1.5 mg, yield: 8.8%) as a white solid, and the racemate was further resolved on a chiral column to give compound 45. LCMS [M+H]⁺ = 579.2

¹H NMR (400 MHz, DMSO-*d*₆) δ 8.96 (d, *J* = 2.0 Hz, 1H), 8.59 (d, *J =* 5.6 Hz, 1H), 8.14 (d, *J* = 8.0 Hz, 1H), 7.70-7.46 (m, 3H), 7.33 (d, *J =* 8.4 Hz, 2H), 7.00 (s, 1H), 6.88 (s, 1H), 6.79-6.57 (m, 1H), 6.23-6.12 (m, 2H), 6.06 (d, *J =* 7.6 Hz, 1H), 5.51 (s, 1H), 4.99 (d, *J =* 4.4 Hz, 1H), 4.88-4.64 (m, 3H), 4.30 (d, *J =* 13.9 Hz, 1H), 4.14 (d, *J =* 10.5 Hz, 1H), 3.90 (s, 3H), 3.88 (s, 3H), 3.09 (s, 3H).

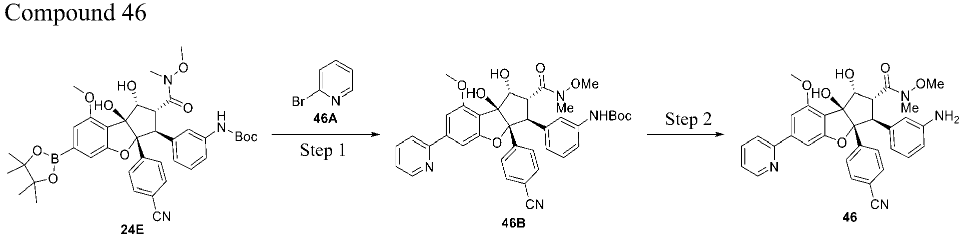

Step 1: The racemate of compound 24E (20 mg, 0.03 mmol) was dissolved in dioxane/water (3 mL/1 mL), and compound 46A (6.51 mg, 0.04 mmol), Na₂CO₃ (5.83 mg, 0.08 mmol), and Pd(dppf)Cl₂ (4.89 mg, 0.001 mmol) were added. The reaction mixture was left to react under microwave conditions at 100 °C for 1 h. The reaction solution was filtered, and the filter cake was washed with ethyl acetate (2 mL × 3). The filtrates were combined and concentrated to give a racemate of 46B (20 mg) as a yellow solid, and the product was directly used in the next step. LCMS [M+H]⁺ = 679.0.

Step 2: 6 N HCl (0.5 mL) was added to a solution of the racemate of compound 46B (20 mg, 0.03 mmol) in tetrahydrofuran (0.5 mL), and the mixture was stirred at room temperature for 12 h. The reaction solution was directly purified by preparative HPLC to give a racemate of 46 (1.4 mg, yield: 8.2%) as a white solid, and the racemate was further resolved on a chiral column to give compound 46.

LCMS [M+H]⁺ = 579.1.

¹H NMR (400 MHz, DMSO-*d*₆) δ 8.69-8.68 (m, 1H), 8.04 (d, *J =* 8.0 Hz, 1H), 7.92-7.88 (m, 1H), 7.54 (d, *J* = 8.8 Hz, 2H), 7.40-7.32 (m, 5H), 6.66 (t, *J =* 7.8 Hz, 1H), 6.20-6.15 (m, 2H), 6.05 (d, *J* = 7.2 Hz, 1H), 5.52 (s, 1H), 4.97 (d, *J* = 4.4 Hz, 1H), 4.82-4.79 (m, 3H), 4.30 (d, *J =* 14.0 Hz, 1H), 4.16-4.11 (m, 1H), 3.90 (s, 3H), 3.86 (s, 3H), 3.09 (s, 3H).

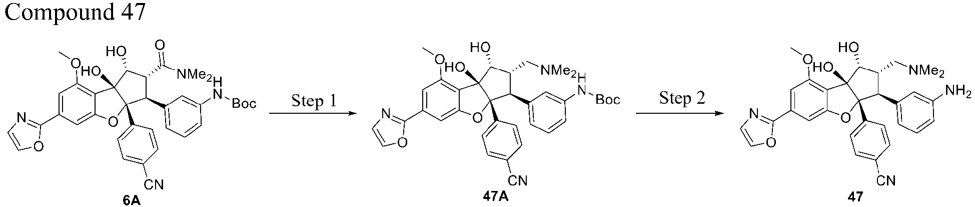

Step 1: BH₃·DMS (70 µL, 0.14 mmol) was added to a solution of the racemate of compound 6A (30 mg, 0.05 mmol) in tetrahydrofuran (5 mL). The reaction system was stirred at 60 °C for 2 h. Methanol (15 mL) was added to the reaction system, and the mixture was stirred at 60 °C for another 18 h. The reaction solution was concentrated to dryness by rotary evaporation and subsequently purified by normal-phase column chromatography (DCM:MeOH) to give a racemate of 47A (20 mg, yield: 69%) as a white solid. LCMS [M+H]⁺ = 639.2.

Step 2: 6 M HCl (1 mL) was added to a solution of the racemate of compound 47A (20 mg, 0.03 mmol) in THF (1 mL). The reaction system was stirred at room temperature for 18 h. The reaction solution was purified by reversed-phase column chromatography (H₂O/ACN) to give a racemate of 47 (14 mg, yield: 69%) as a white solid, and the racemate was further resolved on a chiral column to give compound 47.

LCMS [M+H]⁺ = 539.2.

¹H NMR (400 MHz, DMSO) δ 9.68 (s, 1H), 8.25 (s, 1H), 7.57-7.36 (m, 5H), 7.18 (s, 2H), 6.85 (s, 1H), 6.45 (d, *J* = 39.4 Hz, 3H), 5.74 (s, 1H), 5.30 (d, *J* = 5.8 Hz, 1H), 4.68 (s, 1H), 3.87 (s, 3H), 3.68 (d, *J =* 13.2 Hz, 1H), 3.24 (d, *J =* 11.1 Hz, 1H), 2.95 (d, *J =* 3.3 Hz, 3H), 2.84 (t, *J =* 12.9 Hz, 4H), 1.24 (s, 1H).

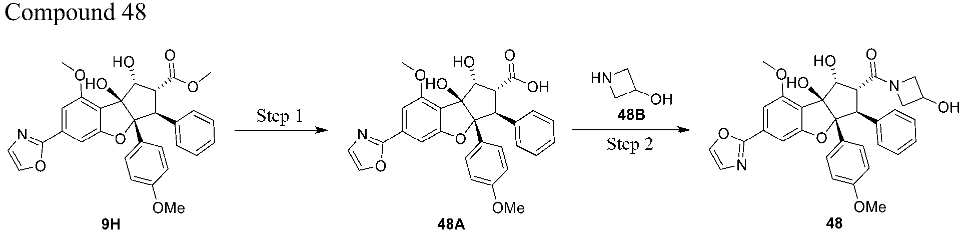

Step 1: The racemate of compound 9H (210 mg, 0.4 mmol) and LiOH·H₂O (48 mg, 1.19 mmol) were added to THF/MeOH/H₂O (1.5/1.5/1.5 mL). The mixture was stirred at 20 °C for 16 h. The solution was purified by reversed-phase column chromatography (MeCN/H₂O/FA) to give a racemate of 48A (150 mg, yield: 75%) as a white solid. LCMS [M+H]⁺ = 516.7.

Step 2: The racemate of compound 48A (20 mg, 0.04 mmol), HATU (15 mg, 0.04 mmol), and TMP (14 mg, 0.12 mmol) were stirred in DMF (2 mL). The racemate of compound 48B (9 mg, 0.08 mmol) was added to the mixture. The resulting mixture was stirred at 20 °C for 2 h and then purified by silica gel column chromatography (MeOH/DCM = 0-10%) to give 48 (10 mg, yield: 50%) as a white solid, and the product was further resolved on a chiral column to give compound 48.

LCMS [M+H]⁺ = 571.2.

¹H NMR (400 MHz, DMSO) δ 8.25 (s, 1H), 7.41 (s, 1H), 7.17 (d, J = 20.4 Hz, 2H), 7.05 (m, 4H), 7.00-6.94 (m, 1H), 6.91 (d, J = 7.2 Hz, 2H), 6.58 (m, 2H), 5.77 (m, 1H), 5.34 (d, J = 7.2 Hz, 1H), 4.91 (t, J = 4.4 Hz, 1H), 4.72-4.45 (m, 3H), 4.31 (m, 1H), 4.23-4.05 (m, 1H), 4.03-3.93 (m, 1H), 3.88-3.76 (m, 4H), 3.61-3.50 (m, 4H).

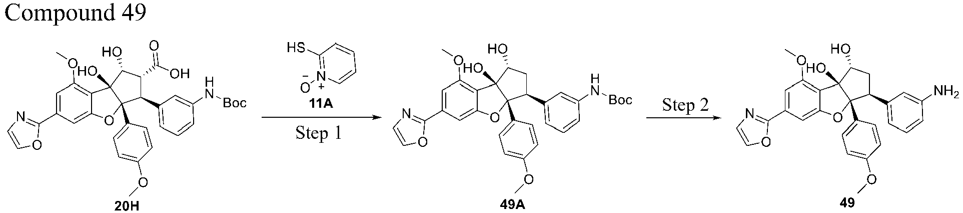

Step 1: The racemate of compound 20H (25 mg, 0.04 mmol), compound 11A (50.38 mg, 0.4 mmol), HOAT (8.09 mg, 0.06 mmol), tert-dodecyl mercaptan (80.13 mg, 0.40 mmol), TMP (7.25 mg, 0.06 mmol), zinc meso-tetraphenylporphine (2.69 mg, 0.001 mmol), and DMF (2 mL) were mixed in a microwave tube. After the reaction system was purged with nitrogen, the microwave tube was placed in hot water at 80 °C, and the system was stirred while being irradiated with 25 W red light. EDCI (75.77 mg, 0.40 mmol, dissolved in 2 mL of DMF) was slowly added dropwise to the reaction solution, and the mixture was then stirred for 30 min. After the reaction was complete, the reaction solution was purified by silica gel column chromatography (H2O/ACN = 40%:60%) to give a racemate of the target product 49A (16 mg, yield: 69%). LCMS [M+H-C₄H₈-H₂O]⁺ = 513.2. Step 2: The racemate of compound 49A (13 mg, 0.02 mmol) was dissolved in THF (2 mL), and 6 M HCl (4 mL) was added. The reaction solution was stirred at room temperature (20 °C) for 4 h, concentrated to dryness by rotary evaporation, and purified by silica gel column chromatography (H₂O/ACN = 55:45) to give a racemate of compound 49 (10 mg, yield: 93%), and the racemate was further resolved on a chiral column to give compound 49.

LCMS [M+H]⁺ = 487.2.

¹H NMR (400 MHz, DMSO) δ 8.24 (d, *J* = 0.7 Hz, 1H), 7.40 (d*, J =* 0.7 Hz, 1H), 7.14 (dd, *J* = 5.1*,* 1.1 Hz, 2H), 7.04 (d, *J =* 9.0 Hz, 2H), 6.75 (t, *J =* 7.7 Hz, 1H), 6.62 (d, *J =* 9.0 Hz, 2H), 6.40 (s, 1H), 6.26 (t, *J =* 6.3 Hz, 2H), 5.06 (s, 1H), 5.01 (s, 1H), 4.65 (d, *J =* 3.7 Hz, 1H), 4.47 (s, 1H), 3.93-3.87 (m, 1H), 3.86 (s, 3H), 3.62 (s, 3H), 2.64 (td, *J* = 13.4, 4.9 Hz, 1H), 2.00 (dd, *J* = 12.7, 6.1 Hz, 1H).

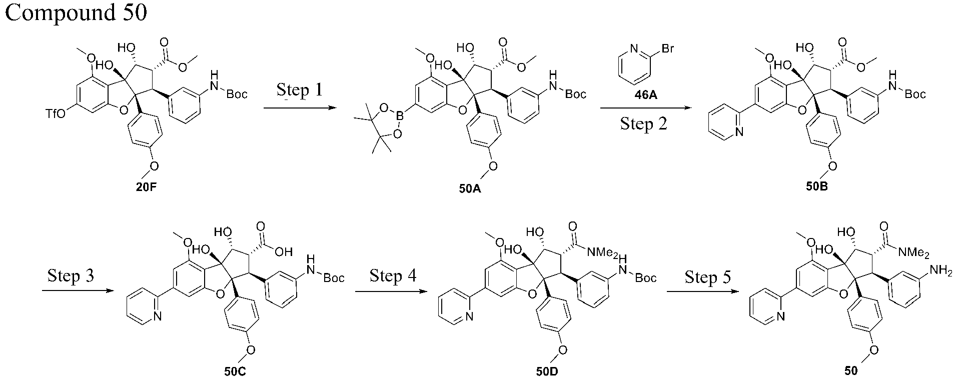

Step 1: The racemate of compound 20F (2.8 g, 3.86 mmol) was dissolved in dioxane (30 mL), and Pin₂B₂ (2.9 g, 11.59 mmol), Pd(dppf)Cl₂ (630.3 mg, 0.77 mmol), and KOAc (1.1 g, 15.45 mmol) were added. The mixture was purged with nitrogen three times and stirred at 85 °C for 16 h. The reaction solution was filtered, and the filter cake was washed with EA. The filtrate was concentrated and then purified by silica gel column chromatography (DCM/MeOH) to give a racemate of compound 50A (1.8 g, yield: 66%). LCMS [M+Na]⁺ = 726.8.

Steps 2 and 3: The racemate of compound 50A (200.0 mg, 0.28 mmol) was dissolved in dioxane/water (2 mL/1 mL), and compound 46A (67.4 mg, 0.43 mmol), Na₂CO₃ (90.5 mg, 0.85 mmol), and Pd(dppf)Cl₂ (23.0 mg, 0.03 mmol) were added. The reaction solution was purged with nitrogen three times, stirred at 130 °C for 2 h, concentrated, and then purified by silica gel column chromatography (DCM/MeOH) to give a racemate of compound 50C (110 mg, two-step yield: 60%). LCMS [M+H]⁺ = 641.8.

Step 4: HATU (26.7 mg, 0.07 mmol), DIEA (30.2 mg, 0.24 mmol), and the racemate of compound 50C (30.0 mg, 0.05 mmol) were dissolved in DMF (2 mL), and the mixture was stirred at 10 °C for 10 min. Dimethylamine (0.1 mL, 0.23 mmol, 2 M in THF) was added to the mixture, and the resulting mixture was left to react at 10 °C for 1 h. The solution was diluted with water, extracted with EA, washed with water and brine, dried over anhydrous Na₂SO₄, filtered, concentrated, and then purified by silica gel column chromatography (DCM/MeOH) to give a racemate of compound 50D (20 mg, yield: 64%). LCMS [M+H]⁺ = 669.0.

Step 5: The racemate of compound 50D (20.0 mg, 0.02 mmol) was dissolved in THF (1 mL), and 6 M HCl (1 mL) was added at 0 °C. The mixture was stirred at 25 °C for 16 h, concentrated, and then purified by preparative HPLC to give a racemate of 50 (4.1 mg, yield: 24%) as a white solid, and the racemate was further resolved on a chiral column to give compound 50.

LCMS [M+H]⁺ = 568.2.

¹H NMR (400 MHz, DMSO) δ 8.69 (dd, *J =* 4.7, 0.9 Hz, 1H), 8.05 (d, *J* = 8.1 Hz, 1H), 7.90 (td, *J* = 7.8, 1.8 Hz, 1H), 7.41-7.34 (m, 1H), 7.32 (dd, *J* = 6.4, 1.1 Hz, 2H), 7.10 *(d, J=* 8.9 Hz, 2H), 6.69 (t, *J* = 7.8 Hz, 1H), 6.64 (d, *J* = 9.0 Hz, 2H), 6.32 (s, 1H), 6.25 (d, *J* = 6.7 Hz, 1H), 6.10 (d, *J* = 7.0 Hz, 1H), 5.23 (s, 1H), 4.79 (d, *J* = 5.5 Hz, 1H), 4.73 (d, *J =* 3.6 Hz, 1H), 4.20 (d, *J* = 13.4 Hz, 1H), 4.01 (dd, *J=* 13.4, 6.1 Hz, 1H), 3.88 (s, 3H), 3.63 (s, 3H), 3.25 (s, 3H), 2.76 (s, 3H).

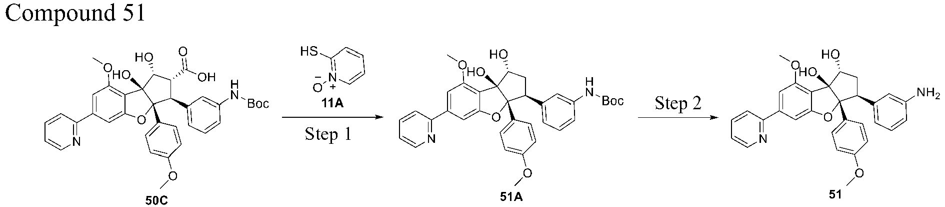

Step 1: The racemate of compound 50C (25 mg, 0.04 mmol), compound 11A (49.59 mg, 0.39 mmol), HOAT (7.97 mg, 0.06 mmol), tert-dodecyl mercaptan (78.88 mg, 0.39 mmol), TMP (7.13 mg, 0.06 mmol), zinc meso-tetraphenylporphine (2.65 mg, 0.004 mmol), and DMF (2 mL) were mixed in a microwave tube. After the reaction system was purged with nitrogen, the microwave tube was placed in hot water at 80 °C, and the system was stirred while being irradiated with 25 W red light. A solution of EDCI (74.58 mg, 0.39 mmol) in DMF (2 mL) was slowly added dropwise to the reaction solution, and the mixture was then stirred for 30 min. After the reaction was complete, the reaction solution was purified by silica gel column chromatography (H₂O/ACN = 40:60) to give a racemate of the target product 51A (14 mg, yield: 60%). LCMS [M+H]⁺ = 597.2. Step 2: The racemate of compound 51A (12 mg, 0.02 mmol) was dissolved in THF (2 mL), and 6 M HCl (4 mL) was added. The reaction solution was stirred at room temperature (20 °C) for 4 h, concentrated to dryness by rotary evaporation, and purified by silica gel column chromatography (H₂O/ACN = 55%:45%) to give a racemate of the target product 51 (3 mg, yield: 30%), and the racemate was further resolved on a chiral column to give compound 51.

LCMS [M+H]⁺ = 497.2.

¹H NMR (400 MHz, DMSO) δ 8.68 (d, J = 3.9 Hz, 1H), 8.03 (d, J = 8.1 Hz, 1H), 7.89 (td, J = 7.8, 1.8 Hz, 1H), 7.40-7.35 (m, 1H), 7.33-7.27 (m, 2H), 7.07 (d, J = 9.0 Hz, 2H), 6.73 (t, J = 7.7 Hz, 1H), 6.63 (d, J = 9.0 Hz, 2H), 6.37 (s, 1H), 6.23 (d, J = 7.8 Hz, 2H), 5.33 (t, J = 4.6 Hz, 1H), 4.92 (s, 1H), 4.80 (s, 1H), 4.57 (d, J = 3.6 Hz, 1H), 4.50 (d, J = 4.1 Hz, 1H), 3.87 (s, 3H), 3.63 (s, 3H), 2.00 (dd, J = 15.0, 7.3 Hz, 3H).

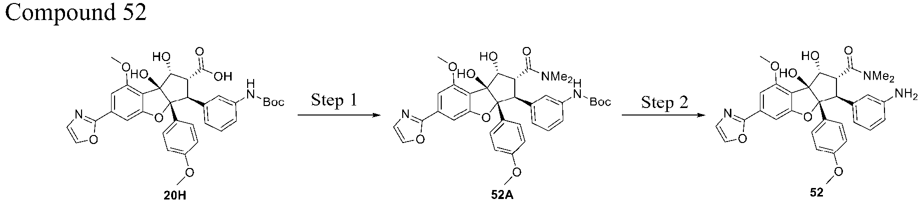

Step 1: The racemate of compound 20H (30 mg, 0.048 mmol) was dissolved in DMF (3 mL), and a dimethylamine solution (0.12 mL, 0.24 mmol, 2 M in THF), HATU (36.18 mg, 0.095 mmol), HOAT (12.95 mg, 0.095 mmol), and TMP (17.27 mg, 0.14 mmol) were added. The mixture was stirred at 25 °C for 2 h. Water was added to quench the reaction, and the mixture was extracted with ethyl acetate twice. The organic phase was concentrated and then purified by silica gel column chromatography (EA/PE) to give a racemate of 52A (20 mg, yield: 65%) as a yellow solid. LCMS [M+H]⁺ = 658.2.

Step 2: The racemate of compound 52A (20 mg, 0.030 mmol) was dissolved in THF (1 mL), and 6 M HCl (1 mL) was added. The mixture was stirred at 25 °C for 16 h. The reaction solution was filtered and purified by reversed-phase column chromatography (ACN:0.1% FA-H₂O) to give a racemate of 52 (9.4 mg, yield: 55%) as a yellow solid, and the racemate was further resolved on a chiral column to give compound 52.

LCMS [M+H]⁺ = 558.2.

¹H NMR (400 MHz, DMSO) δ 8.24 (s, 1H), 7.40 (s, 1H), 7.16 (d, *J* = 15.0 Hz, 2H), 7.08 (d, *J* = 8.9 Hz, 2H), 6.63 (t, *J* = 8.4 Hz, 3H), 6.23 (s, 1H), 6.15 (d, *J* = 7.9 Hz, 1H), 6.01 (d, *J* = 7.6 Hz, 1H), 5.28 (s, 1H), 4.80-4.69 (m, 4H), 4.20 (d, *J* = 13.4 Hz, 1H), 4.02 (dd, *J* = 13.5, 5.5 Hz, 1H), 3.85 (s, 3H), 3.62 (s, 3H), 3.25 (s, 3H), 2.77 (s, 3H).

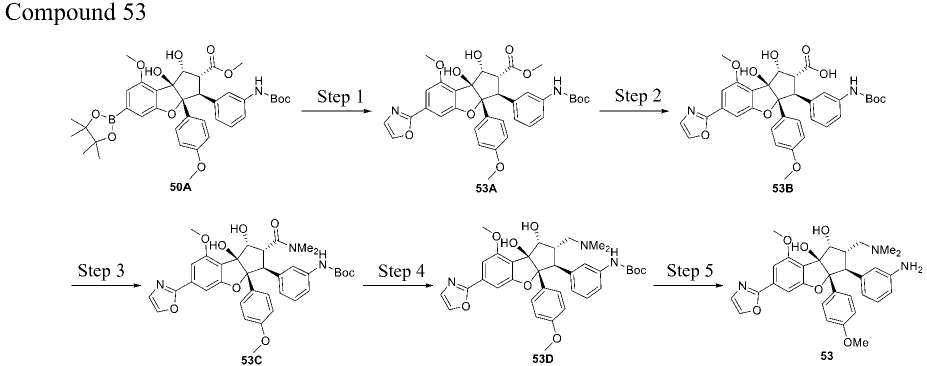

Step 1: The racemate of compound 50A (250 mg, 0.3553 mmol) was dissolved in dioxane (3 mL) and water (0.6 mL), and 2-bromooxazole (525.73 mg, 3.5532 mmol), Pd(dppf)Cl₂ (28.99 mg, 0.0355 mmol), and Na₂CO₃ (150.78 mg, 1.4225 mmol) were added. The mixture was stirred under microwave conditions at 130 °C for 2.5 h. The reaction solution was filtered, concentrated, and then purified by silica gel column chromatography (EA/PE) to give a racemate of 53A (200 mg, yield: 87%) as a yellow solid. LCMS [M+H-C₄H₈]⁺ = 589.2.

Step 2: The racemate of compound 53A (200 mg, 0.3102 mmol) was dissolved in THF (6 mL) and H₂O (2 mL), and LiOH (40 mg, 0.9307 mmol) was added. The mixture was stirred at 20 °C for 16 h. The pH of the reaction solution was adjusted to 5 with 3 M HCl. After concentration, the residue was purified by silica gel column chromatography (DCM:MeOH = 10:1) to give a racemate of 53B (150 mg, yield: 77%) as a yellow solid. LCMS [M-C₄H₈+H]⁺ = 575.1.

Step 3: The racemate of compound 53B (150 mg, 0.238 mmol) was dissolved in DMF (5 mL), and a dimethylamine solution (0.6 mL, 1.2 mmol, 2 M in THF), HATU (180.99 mg, 0.476 mmol), HOAT (64.79 mg, 0.476 mmol), and TMP (86.43 mg, 0.714 mmol) were added. The mixture was stirred at 25 °C for 2 h. The reaction solution was filtered, concentrated, and then purified by reversed-phase column chromatography (ACN:0.1% FA-H₂O) to give a racemate of 53C (120 mg, yield: 77%) as a yellow solid. LCMS [M+H]⁺ = 658.2.

Step 4: The racemate of compound 53C (60 mg, 0.091 mmol) was dissolved in THF (2 mL). Under a nitrogen atmosphere, BH3·THF (0.45 mL) was added, and the mixture was stirred at 60 °C for 3 h. Then, MeOH was added, and the mixture was stirred at 60 °C for 16 h. The reaction solution was concentrated and purified by reversed-phase column chromatography (ACN:0.1% FA-H₂O) to give a racemate of 53D (30 mg, yield: 52%) as a yellow solid. LCMS [M+H]⁺ = 644.2.

Step 5: The racemate of compound 53D (30 mg, 0.05 mmol) was dissolved in THF (1 mL), and 6 M HCl (1 mL) was added. The mixture was stirred at 25 °C for 16 h. The reaction solution was directly purified by reversed-phase column chromatography (ACN:0.1%FA-H₂O) to give a racemate of 53 (17 mg, yield: 68%) as a white solid, and the racemate was further resolved on a chiral column to give compound 53.

LCMS [M+H]⁺ = 544.2.

¹H NMR (400 MHz, DMSO) δ 8.25 (d, J = 0.7 Hz, 1H), 7.41 (d, J = 0.8 Hz, 1H), 7.19-7.15 (m, 4H), 6.88 (s, 3H), 6.64 (d, J = 9.0 Hz, 3H), 5.41 (d, J = 111.7 Hz, 2H), 4.71 (s, 1H), 3.87 (s, 3H), 3.62 (s, 3H), 3.24 (s, 1H), 2.95 (d, J = 4.3 Hz, 4H), 2.81 (d, J = 4.7 Hz, 4H), 1.24 (s, 1H).

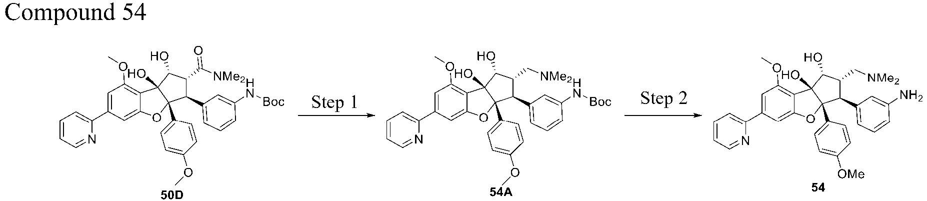

Step 1: The racemate of compound 50D (25.0 mg, 0.04 mmol) was dissolved in THF (1 mL), and BH3·DMS (0.04 mL, 0.11 mmol) was added. The mixture was stirred at 60 °C for 2 h. Methanol (1 mL) was added, and the mixture was stirred at 60 °C for 18 h. The reaction solution was concentrated and purified by silica gel column chromatography (DCM/MeOH) to give a racemate of compound 54A (10 mg, yield: 41%). LCMS [M+H]⁺ = 654.2.

Step 2: The racemate of compound 54A (10.0 mg, 0.015 mmol) was mixed with THF (1 mL), and 6 M HCl (1 mL) was added at 0 °C. The mixture was stirred at 25 °C for 16 h. The reaction solution was concentrated and then purified by preparative HPLC to give a racemate of 54 (1.2 mg, yield: 14%) as a white solid, and the racemate was further resolved on a chiral column to give compound 54.

LCMS [M+H]+ = 554.2.

¹H NMR (400 MHz, DMSO) δ 8.67 (d, *J* = 3.8 Hz, 1H), 8.01 (d, *J* = 8.0 Hz, 1H), 7.89 (t, *J* = 7.8 Hz, 1H), 7.39-7.33 (m, 1H), 7.27 (s, 2H), 7.18 (d, *J* = 8.9 Hz, 2H), 6.70 (d, *J* = 7.8 Hz, 1H), 6.62 (d, *J* = 9.0 Hz, 2H), 6.30 (s, 1H), 6.20 (d, *J* = 8.3 Hz, 1H), 6.10 (d, *J* = 7.6 Hz, 1H), 5.33 (d, *J* = 4.7 Hz, 2H), 5.14 (s, 1H), 4.81 (s, 1H), 4.54 (s, 1H), 3.86 (s, 3H), 3.62 (s, 3H), 3.48 (d, *J* = 13.7 Hz, 1H), 2.04-1.98 (m, 8H), 1.29-1.29 (m, 1H).

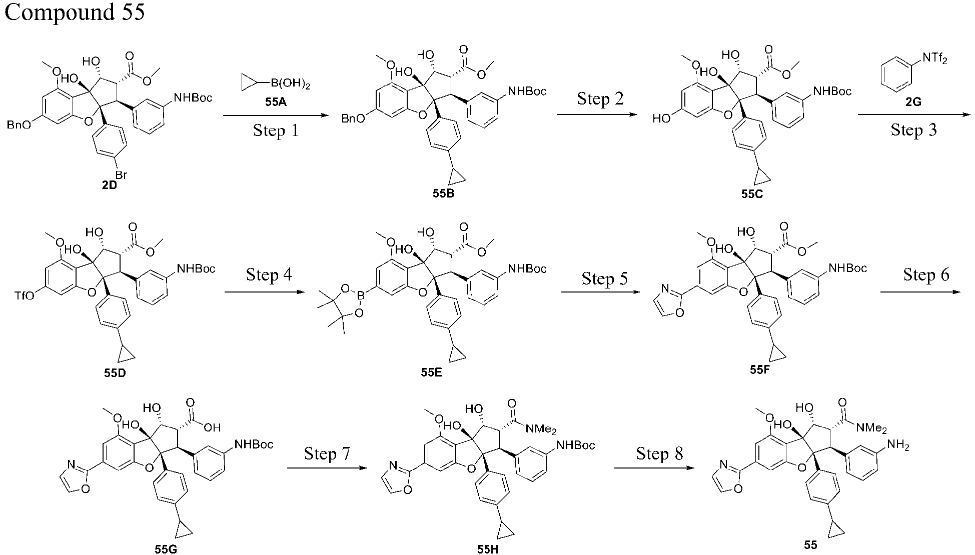

Step 1: Compound 55A (212 mg, 2.46 mmol), K₃PO₄ (695 mg, 3.28 mmol), Cy₃P (92 mg, 0.33 mmol), and Pd(OAc)₂ (37 mg, 0.164 mmol) were added to a mixture of the racemate of compound 2D (600 mg, 0.82 mmol) in toluene (25 mL) and H₂O (10 mL), and the reaction solution was stirred at 120 °C for 3 h under a nitrogen atmosphere. The reactants were poured into water (20 mL) and extracted with EA (50 mL). The organic phase was washed with 20 mL of brine, dried, concentrated, and purified by silica gel column chromatography (PE/EA = 2/1) to give a racemate of 55B (450 mg, yield: 79%) as a yellow oil. LCMS [M+Na]⁺ = 716.4.

Step 2: Pd(OH)₂/C (10%, 100 mg) was added to a mixed solution of the racemate of compound 55B (450 mg, 0.65 mol) in EA/MeOH (1:1, 25 mL). The mixture was purged with hydrogen three times and stirred at 25 °C for 18 h. This procedure was repeated three times until the starting materials were completely consumed. The reaction solution was filtered, and the filter cake was washed with MeOH/DCM (1/10). The combined filtrates were concentrated to give a racemate of 55C (380 mg, yield: 97%) as a white solid. LCMS [M+Na]⁺ = 626.2.

Step 3: K₂CO₃ (174 mg, 1.26 mmol) was added to a solution of the racemate of compound 55C (380 mg, 0.63 mmol) and compound 2G (225 mg, 0.63 mmol) in DMF (15 mL), and the mixture was stirred at 60 °C for 2 h, poured into H₂O (15 mL), and extracted with EtOAc (30 mL). The organic phase was washed with water (15 mL) and brine (15 mL), dried over anhydrous Na₂SO₄, filtered, and then concentrated, and the crude product was purified by silica gel column chromatography (DCM/EA = 0-30%) to give a racemate of 55D (350 mg, yield: 76%) as a white solid. LCMS [M+Na]⁺ = 758.0.

Step 4: Under a nitrogen atmosphere, bis(pinacolato)diboron (69 mg, 0.272 mmol), potassium acetate (40 mg, 0.408 mmol), and Pd(dppf)Cl₂·DCM (17 mg, 0.02 mmol) were added to a solution of the racemate of compound 55D (50 mg, 0.068 mmol) in dioxane (20 mL), and the mixture was stirred at 105 °C for 18 h. The reaction solution was concentrated and purified by silica gel column chromatography (DCM/EA = 3/1) to give a racemate of 55E (40 mg, yield: 83%) as an oil. LCMS [M+Na]⁺ = 736.4.

Step 5: Under a nitrogen atmosphere, 2-bromooxazole (50 mg, 0.34 mmmol), Pd(dppf)Cl₂·DCM (14 mg, 0.017 mmol), and Na₂CO₃ (27 mg, 0.25 mmol) were added to a mixture of the racemate of compound 55E (58 mg, 0.084 mmol) in dioxane (4 mL) and H₂O (2 mL), and the resulting mixture was stirred under microwave conditions at 90 °C for 2 h. Water (10 mL) was added, and the mixture was extracted with EA (50 mL). The organic phase was washed with water (20 mL) and brine (20 mL), dried, and concentrated. The crude product was purified by flash silica gel column chromatography (DCM/EA = 2:1) to give a racemate of 55F (38 mg, yield: 70%) as a white solid. LCMS [M+Na]⁺ = 677.3.

Step 6: H₂O (2 mL), MeOH (2 mL), and LiOH (8 mg, 0.174 mmol) were added to a solution of the racemate of compound 55F (38 mg, 0.058 mmol) in THF (2 mL), and the mixture was stirred at 25 °C for 18 h. The reaction solution was directly purified by reversed-phase column chromatography (MeCN/FA/H₂O) to give a racemate of 55G (25 mg, yield: 68%) as a white solid. LCMS [M+Na]⁺ = 663.3.

Step 7: A dimethylamine solution (0.1 mL, 0.2 mmol, 2 M in THF), HATU (15 mg, 0.04 mol), and TMP (10 mg, 0.08 mmol) were added to a solution of the racemate of compound 55G (25 mg, 0.04 mmol) in DMF (2 mL), and the mixture was stirred at 20 °C for 2 h. The reaction solution was directly purified by reversed-phase column chromatography (MeCN/H₂O/FA) to give a racemate of 55H (18 mg, yield: 69%) as a white solid. LCMS [M+H]⁺ = 668.3.

Step 8: The racemate of compound 55H (18 mg, 0.027 mmol) was dissolved in THF (2 mL), and 6 M HCl (2 mL) was added. The mixture was stirred at 25 °C for 18 h. The reaction solution was purified by reversed-phase column chromatography (MeCH/FA/H₂O) to give a racemate of 55 (6.0 mg, yield: 40%) as a white solid, and the racemate was further resolved on a chiral column to give compound 55.

LCMS [M+H]⁺ = 568.3.

¹H NMR (400 MHz, DMSO) δ 8.25 (s, 1H), 7.41 (s, 1H), 7.18 (s, 1H), 7.14 (s, 1H), 7.04 (d, *J* = 8.3 Hz, 2H), 6.76 (d, *J* = 8.3 Hz, 2H), 6.63 (t, *J* = 7.7 Hz, 1H), 6.24 (s, 1H), 6.15 (d, *J* = 7.5 Hz, 1H), 6.02 (d, *J* = 7.4 Hz, 1H), 5.29 (s, 1H), 4.78 (d, *J* = 4.3 Hz, 2H), 4.74 (d, *J* = 5.2 Hz, 1H), 4.22 (d, *J* = 13.4 Hz, 1H), 4.08-4.00 (m, 1H), 3.85 (s, 3H), 3.26 (s, 3H), 2.77 (s, 3H), 1.74 (d, *J* = 4.9 Hz, 1H), 0.87-0.81 (m, 2H), 0.53 (d, *J* = 5.1 Hz, 2H).

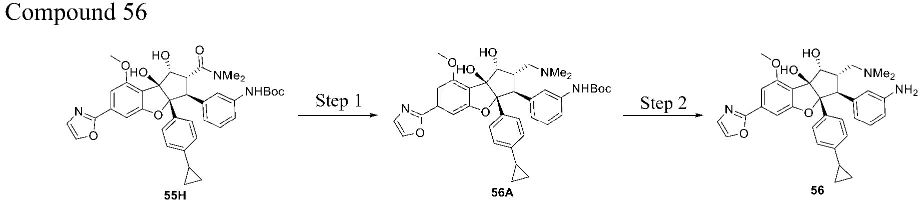

Step 1: A 1 M DIBAL-H solution (0.1 mL, 0.1 mmol) was added to a mixture of the racemate of compound 55H (3 mg, 0.004 mmol) in THF (1 mL). The resulting mixture was stirred at 20 °C for 5 h. The DIBAL-H solution (0.1 mL, 0.1 mmol) was further added, and the mixture was stirred at 60 °C for another 4 h. The reaction was quenched with methanol, and the reaction solution was concentrated. The crude product was purified by preparative TLC (DCM/MeOH) to give a racemate of 56A (2.5 mg, yield: 80%) as a white solid. LCMS [M+H]⁺ = 654.3.

Step 2: 6 M HCl (2 mL) was added to a solution of the racemate of compound 56A (3 mg, 0.005 mmol) in THF (2 mL), and the mixture was stirred at 25 °C for 18 h. The reaction solution was purified by reversed-phase column chromatography (MeCN/FA/H₂O) to give a racemate of 56 (1.5 mg, yield: 60%) as a white solid, and the racemate was further resolved on a chiral column to give compound 56.

LCMS: [M+H]⁺ = 554.2.

¹H NMR (400 MHz, DMSO) δ 8.37 (s, 4H), 8.22 (s, 1H), 7.39 (s, 1H), 7.21 (s, 1H), 7.10 (d, *J* = 9.0 Hz, 4H), 6.75 (d, *J* = 8.5 Hz, 2H), 6.68 (t, *J* = 7.7 Hz, 2H), 6.28 (s, 1H), 6.18 (d, *J* = 7.6 Hz, 1H), 6.11 (d, *J* = 7.9 Hz, 1H), 5.33 (t, *J* = 4.6 Hz, 1H), 5.16 (s, 1H), 4.77 (s, 1H), 4.46 (d, *J* = 4.4 Hz, 1H), 3.83 (s, 3H), 3.53 (s, 2H), 2.20 (s, 6H), 1.97 (s, 1H), 1.73 (d, *J* = 5.4 Hz, 1H), 0.85-0.83 (m, 2H), 0.53-0.51 (m, 2H).

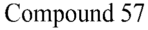

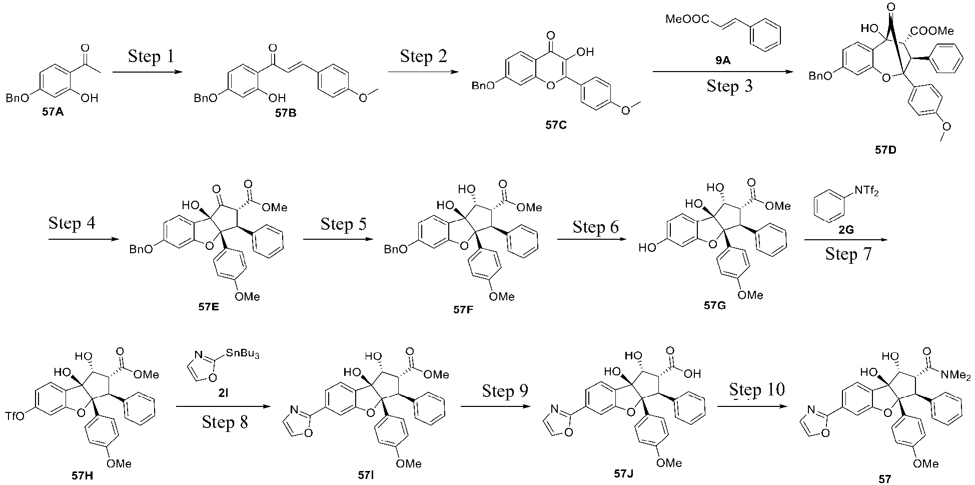

Step 1: Compound 57A (24 g, 99.06 mmol) and 4-methoxybenzaldehyde (16.19 g, 118.87 mmol) were dissolved in EtOH (1 L), and 50% NaOH (80 mL) was added at 50 °C. The mixture was stirred at 50 °C for 12 h. The reaction solution was concentrated, and the resulting crude product was diluted with H₂O (500 mL), extracted with DCM (500 mL × 2), washed with brine (500 mL × 2), dried over Na₂SO₄, filtered, and concentrated to give 57B (19.87 g, yield: 56%) as a yellow solid. LCMS [M+H]⁺ = 361.2.

Step 2: Compound 57B (18.8 g, 52.16 mmol) was dissolved in MeOH/H₂O (100 mL/50 mL), and NaOH (14.61 g, 365.10 mmol) and H₂O₂ (80 mL) were added. The mixture was stirred at 50 °C for 12 h. The reaction solution was filtered, and the filter cake was washed with diethyl ether (100 mL) and dissolved in 200 mL of DCM. The pH was adjusted to 5 with 1 N HCl (100 mL), and the solution was washed with brine (100 mL × 2), dried over Na₂SO₄, filtered, and concentrated to give 57C (8.9 g, yield: 46%) as a yellow solid. LCMS [M+H]⁺ = 375.0.

Step 3: Compound 57C (2 g, 5.35 mmol) and compound 9A (5.197 g, 32.09 mmol) were dissolved in CHCl₃/TFE (7/3, 15 mL × 2). A syringe was used to inject the reaction mixture (12 mL/h) through a syringe pump into a coil wound around a jacketed glass cylinder equipped with a 250 W ultraviolet lamp. The temperature was adjusted to keep the internal reaction temperature at 0-5 °C, and the UV lamp was turned on simultaneously. The reaction mixture was irradiated for 1 h and concentrated under reduced pressure to remove the solvent. The residue was purified using a silica gel plug (EtOAc/hexane) to remove the excess cinnamate, giving a racemate of 57D (8.1 g, total yield: 75%) as a pale yellow solid. LCMS [M+H]⁺ = 537.7.

Step 4: The racemate of compound 57D (8.6 g, 16.03 mmol) was dissolved in methanol (120 mL), and solid sodium methoxide (2.163 g, 40.07 mmol) was slowly added under ice-water bath conditions (0 °C). The reaction solution was placed in an oil bath at 65 °C and stirred for 1.5 h.

After the reaction was complete, the reaction solution was concentrated to dryness by rotary evaporation to remove the methanol, and then extracted with ethyl acetate (200 mL × 3). The organic phase was washed once with an ammonium chloride solution (100 mL) and once with saturated brine (100 mL), dried over anhydrous sodium sulfate, filtered, concentrated, and then purified by silica gel column chromatography (0.2% FA in EA/PE (65%/35%)) to give a racemate of compound 57E (7.0 g, yield: 81%). LCMS [M+H-H₂O]⁺ = 519.7.

Step 5: The racemate of compound 57E (7.0 g, 9.39 mmol) was dissolved in a mixed solution of acetonitrile/chloroform (80 mL/80 mL), and acetic acid (7.827 g, 130.46 mmol) and STAB (13.828 g, 65.23 mmol) were slowly added under ice-water bath conditions (0 °C). The reaction solution was stirred at room temperature for 2 h. After the reaction was complete, the reaction solution was concentrated to dryness by rotary evaporation to remove the solvent, and then extracted with ethyl acetate (200 mL × 3). The organic phase was washed with saturated brine (100 mL), dried over anhydrous sodium sulfate, filtered, concentrated, and then purified by silica gel column chromatography (0.2% FA in EA/PE = 50%/50%) to give a racemate of compound 57F (3.8 g, yield: 54%). LCMS [M+H-H₂O]⁺ = 521.8.

Step 6: The racemate of compound 57F (3.8 g, 7.06 mmol) was dissolved in methanol (50 mL), and 10% Pd/C (1.14 g) was added. The mixture was purged with hydrogen three times and stirred at 20 °C for 16 h. The reaction solution was filtered, concentrated, and purified by silica gel column chromatography (PE:EA = 40:60) to give a racemate of compound 57G (3.0 g, yield: 85%). LCMS [ M+H-H2O]⁺ = 431.2.

Step 7: Under a nitrogen atmosphere, the racemate of compound 57G (2.8 g, 6.24 mmol) was dissolved in DMF (30 mL), and compound 2G (2.23 g, 6.24 mmol) and K₂CO₃ (1.723 g, 12.49 mmol) were added. The mixture was heated at 60 °C for 1 h. After the reaction was complete, the reaction solution was quenched with water (100 mL) and extracted with EA (200 mL) three times. The organic phase was washed with saturated brine, dried over anhydrous sodium sulfate, filtered, concentrated, and then purified by silica gel column chromatography (PE:EA = 60:40) to give a racemate of compound 57H (2.7 g, yield: 75%). LCMS [M+H-H₂O]⁺ = 563.0.

Step 8: Under a nitrogen atmosphere, the racemate of compound 57H (200.0 mg, 0.34 mmol) was dissolved in DMF (3 mL), and compound 2I (333.3 mg, 0.93 mmol), Pd(PPh₃)₄ (139.9 mg, 0.03 mmol), and CuI (13.1 mg, 0.069 mmol) were added. The mixture was stirred at 100 °C for 1 h. The reaction solution was quenched with water and extracted with EA. The organic layer was washed with water and brine, dried over anhydrous Na₂SO₄, filtered, concentrated, and then purified by silica gel column chromatography (DCM/MeOH = 20:1) to give a racemate of compound 57I (100 mg, yield: 58%). LCMS [M+H]+ = 500.7.

Step 9: The racemate of compound 57I (100.0 mg, 0.20 mmol) was dissolved in THF/H₂O/MeOH (3 mL/1 mL/1 mL), and LiOH (25.3 mg, 0.60 mmol) was added. The mixture was stirred at 40 °C for 3 h. The pH was adjusted to 5 with 3 M hydrochloric acid. After concentration, the residue was purified by silica gel column chromatography (DCM/MeOH) to give a racemate of compound 57J (90 mg, yield: 92%). LCMS [M+H]⁺ = 486.7.

Step 10: The racemate of compound 57J (90.0 mg, 0.19 mmol), HATU (105.8 mg, 0.28 mmol), and DIEA (119.7 mg, 0.93 mmol) were dissolved in DMF (2 mL), and the solution was stirred at 25 °C for 10 min. DMA (0.6 µL, 0.93 mmol, 2 M in THF) was added, and the mixture was left to react at 25 °C for 1 h. The solution was diluted with water, extracted with EA, washed with water and brine, dried over anhydrous Na₂SO₄, filtered, concentrated, and then purified by silica gel column chromatography (DCM/MeOH) to give a racemate of compound 57 (6.5 mg, yield: 6.8%), and the racemate was further resolved on a chiral column to give compound 57.

LCMS [M+H]+ = 513.8.

¹H NMR (400 MHz, DMSO) δ 8.25 (s, 1H), 7.73-7.50 (m, 3H), 7.41 (s, 1H), 7.12 (d, *J* = 8.8 Hz, 2H), 7.06-6.95 (m, 3H), 6.83 (d, *J* = 7.1 Hz, 2H), 6.68 (d, *J* = 8.9 Hz, 2H), 4.94 (d, *J* = 8.1 Hz, 1H), 4.19 (d, *J* = 13.2 Hz, 1H), 4.02 (dd, *J* = 13.1, 8.1 Hz, 1H), 3.65 (s, 3H), 3.20 (s, 3H), 2.72 (s, 3H).

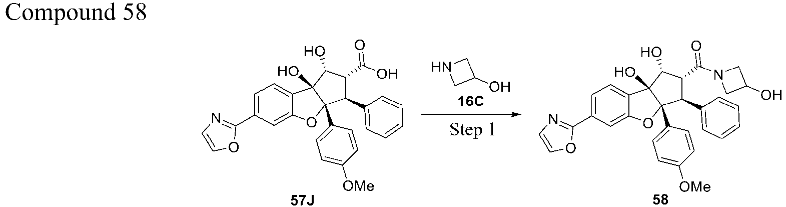

Step 1: The racemate of compound 57J (30.0 mg, 0.06 mmol), HATU (70.5 mg, 0.19 mmol), and DIEA (39.9 mg, 0.31 mmol) were dissolved in DMF (2 mL), and the solution was stirred at 25 °C for 10 min. Compound 16C (34.0 mg, 0.31 mmol) was added, and the mixture was left to react at 25 °C for 1 h. The reaction solution was diluted with water, extracted with EA, washed with water and brine, dried over anhydrous Na₂SO₄, and purified by silica gel column chromatography (DCM/MeOH) to give a racemate of compound 58 (14.1 mg, yield: 42%), and the racemate was further resolved on a chiral column to give compound 58.

LCMS [M+H]⁺ = 541.2.

¹H NMR (400 MHz, DMSO) δ 8.24 (s, 1H), 7.58 (d, *J* = 14.3 Hz, 3H), 7.40 (s, 1H), 7.05 (ddd, *J* = 21.5, 11.8, 6.0 Hz, 5H), 6.86 (d, *J* = 7.2 Hz, 2H), 6.67 (dd, *J* = 8.6, 7.0 Hz, 2H), 5.73 (dd, *J* = 10.7, 5.7 Hz, 1H), 5.59 (dd, *J* = 5.8, 2.5 Hz, 1H), 5.53 (d, *J* = 3.3 Hz, 1H), 4.83 (dt, *J* = 10.3, 7.7 Hz, 1H), 4.53 (dt, *J* = 40.9, 11.6 Hz, 2H), 4.15 (dd, *J* = 13.2, 7.7 Hz, 1H), 4.10-4.03 (m, 1H), 3.93 (ddd, *J* = 25.7, 13.2, 8.3 Hz, 1H), 3.78-3.66 (m, 1H), 3.65 (t, *J* = 5.7 Hz, 3H), 3.50 (ddd, *J* = 33.5, 10.0, 4.2 Hz, 1H).

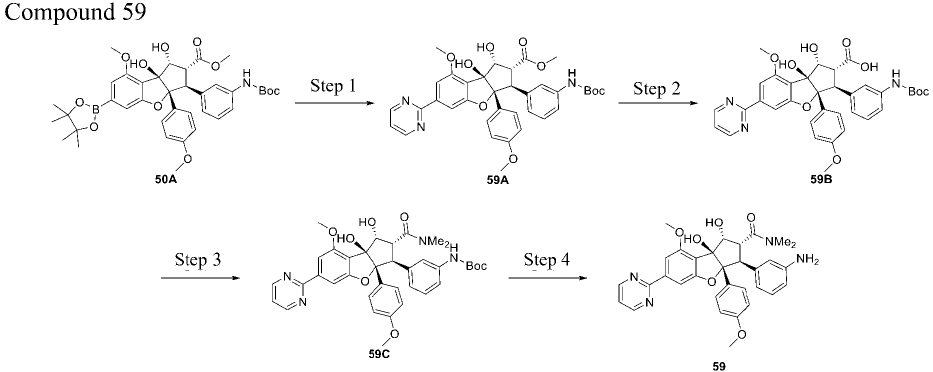

Step 1: The racemate of compound 50A (100 mg, 0.1421 mmol) was dissolved in dioxane (2 mL) and water (0.4 mL), and 2-bromopyrimidine (112.98 mg, 0.7106 mmol), Pd(dppf)Cl₂ (11.60 mg, 0.0142 mmol), and Na₂CO₃ (60.26 mg, 0.5685 mmol) were added. The mixture was stirred under microwave conditions at 130 °C for 2 h. The reaction solution was filtered, concentrated, and then purified by silica gel column chromatography (EA/PE) to give a racemate of 59A (70 mg, yield: 75%) as a yellow solid. LCMS [M+H-H₂O]⁺ = 638.2.

Step 2: The racemate of compound 59A (60 mg, 0.09 mmol) was dissolved in THF (2 mL) and H₂O (0.7 mL), and LiOH (11.5 mg, 0.27 mmol) was added. The mixture was stirred at 20 °C for 16 h. The pH of the reaction solution was adjusted to 5 with 3 M HCl. After concentration, the residue was purified by silica gel column chromatography (DCM:MeOH = 10:1) to give a racemate of 59B (40 mg, yield: 69%) as a yellow solid. LCMS [M+H-C₄H₈]⁺ = 586.2.

Step 3: The racemate of compound 59B (40 mg, 0.062 mmol) was dissolved in DMF (2 mL), and a dimethylamine solution (0.16 mL, 0.32 mmol, 2 M in THF), HATU (47.40 mg, 0.312 mmol), HOAT (16.97 mg, 0.125 mmol), and TMP (22.62 mg, 0.187 mmol) were added. The mixture was stirred at 25 °C for 2 h. The reaction solution was filtered, concentrated, and then purified by reversed-phase column chromatography (ACN:0.1% FA-H₂O) to give a racemate of 59C (31 mg, yield: 76%) as a yellow solid. LCMS [M+H]⁺ = 669.2.

Step 4: The racemate of compound 59C (7 mg, 0.01 mmol) was dissolved in THF (1 mL), and 6 M HCl (0.5 mL) was added. The mixture was stirred at 20 °C for 16 h. The reaction solution was directly purified by reversed-phase column chromatography (ACN:0.1% FA-H₂O) to give a racemate of 59 (4.0 mg, yield: 67%) as a white solid, and the racemate was further resolved on a chiral column to give compound 59.

LCMS [M+H]⁺ = 569.2.

¹H NMR (400 MHz, DMSO) δ 8.94 (d, J = 4.9 Hz, 2H), 7.65-7.61 (m, 2H), 7.48 (t, J = 4.9 Hz, 1H), 7.10 (d, J = 8.9 Hz, 2H), 6.65-6.60 (m, 3H), 6.27 (s, 1H), 6.17 (d, J = 7.1 Hz, 1H), 6.00 (d, J = 7.8 Hz, 1H), 5.26 (s, 1H), 4.78 (d, J = 5.8 Hz, 1H), 4.74 (d, J = 4.0 Hz, 1H), 4.19 (d, J = 13.5 Hz, 1H), 4.01 (dd, *J* = 13.6, 6.0 Hz, 1H), 3.87 (s, 3H), 3.63 (s, 3H), 3.25 (s, 3H), 2.76 (s, 3H).

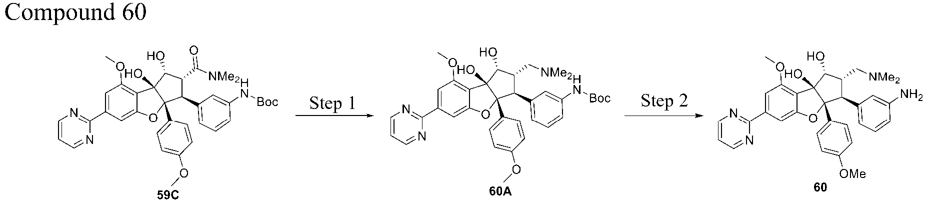

Step 1: The racemate of compound 59C (10 mg, 0.015 mmol) was dissolved in THF (1.5 mL), and DIBAL-H (0.045 mL, 0.045 mmol) was added at 0 °C under a nitrogen atmosphere. The mixture was left to react at 22 °C for 1 h. DIBAL-H (0.045 mL, 0.045 mmol) was then added at 0 °C under a nitrogen atmosphere, and the mixture was left to react at 22 °C for 1 h. DIBAL-H (0.045 mL, 0.045 mmol) was then added at 0 °C under a nitrogen atmosphere, and the mixture was left to react at 22 °C for 1 h. The reaction solution was quenched with iced water, concentrated, and then purified by reversed-phase column chromatography (ACN:0.1% FA-H₂O) to give a racemate of 60A (5 mg, yield: 51%) as a yellow solid. LCMS [M+H]⁺ = 655.5.

Step 2: The racemate of compound 60A (5 mg, 0.008 mmol) was dissolved in THF (1 mL), and 6 M HCl (1 mL) was added. The mixture was stirred at 25 °C for 16 h. The reaction solution was directly purified by reversed-phase column chromatography (ACN:0.1% FA-H₂O) to give a racemate of 60 (2.5 mg, yield: 59%) as a white solid, and the racemate was further resolved on a chiral column to give compound 60.

LCMS [M+H]⁺ = 555.5.

¹H NMR (400 MHz, ) δ 8.92 (d, J = 4.8 Hz, 2H), 8.13 (s, 1H), 7.57 (d, J = 16.0 Hz, 2H), 7.46 (t, J = 4.8 Hz, 1H), 7.17 (d, J = 8.9 Hz, 2H), 6.69 (t, J = 7.7 Hz, 1H), 6.62 (d, J = 8.9 Hz, 2H), 6.28 (s, 1H), 6.20 (d, J = 7.7 Hz, 1H), 6.07 (d, J = 7.2 Hz, 1H), 5.20 (s, 1H), 4.81 (s, 2H), 4.54 (s, 1H), 3.86 (s, 3H), 3.62 (s, 3H), 3.47 (d, J = 13.8 Hz, 1H), 3.06 (s, 2H), 2.37 (s, 6H).

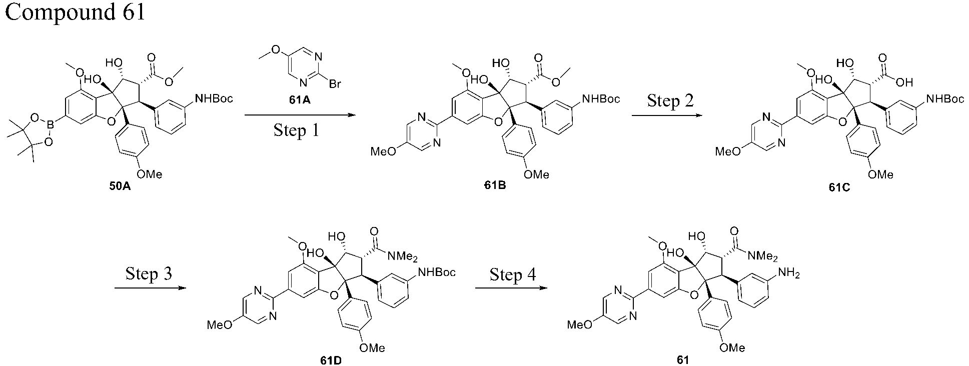

Step 1: Compound 61A (189 mg, 0.71 mmol), Pd(dppf)Cl₂ (11.6 mg, 0.014 mmol), and an aqueous solution (0.4 mL) of Na₂CO₃ (30.13 mg, 0.2842 mmol) were added to a solution of the racemate of compound 50A (100 mg, 0.142 mmol) in dioxane (2 mL). Under a nitrogen atmosphere, the mixture was stirred at 100 °C for 16 h. The reaction solution was directly purified by silica gel column chromatography (PE/EA) to give a racemate of compound 61B (50 mg, yield: 50%). LCMS [M+H]⁺ = 687.0.

Step 2: The racemate of compound 61B (50 mg, 0.07 mmol) and LiOH·H₂O (9 mg, 0.22 mmol) were added to a mixed solution of THF/MeOH/H₂O (1/1/1, 3 mL), and the mixture was stirred at 20 °C for 16 h. The reaction solution was purified by reversed-phase column chromatography (ACN-H₂O) (FA) to give a racemate of 61C (34 mg, yield: 69%) as a white solid. LCMS [M+H]⁺ = 673.0.

Step 3: The racemate of compound 61C (34 mg, 0.05 mmol), HATU (19 mg, 0.05 mmol), and TMP (14 mg, 0.12 mmol) were stirred in DMF (2 mL). Dimethylamine (11 mg, 0.1 mmol) was added to the mixture, and the resulting mixture was stirred at 20 °C for 2 h. The reaction solution was purified by reversed-phase column chromatography (ACN/H₂O = 0-100%) to give a racemate of 61D (10 mg, yield: 50%) as a white solid. LCMS [M+H]⁺ = 700.1.

Step 4: The racemate of compound 61D (6 mg, 0.0086 mmol) was dissolved in THF (1 mL), and 6 M HCl (1 mL) was added. The mixture was stirred at 20 °C for 5 h. The reaction solution was directly purified by reversed-phase column chromatography (ACN-H₂O) to give a racemate of 61 (3.8 mg, yield: 70%) as a white solid, and the racemate was further resolved on a chiral column to give compound 61. LCMS [M+H]⁺ = 600.0.

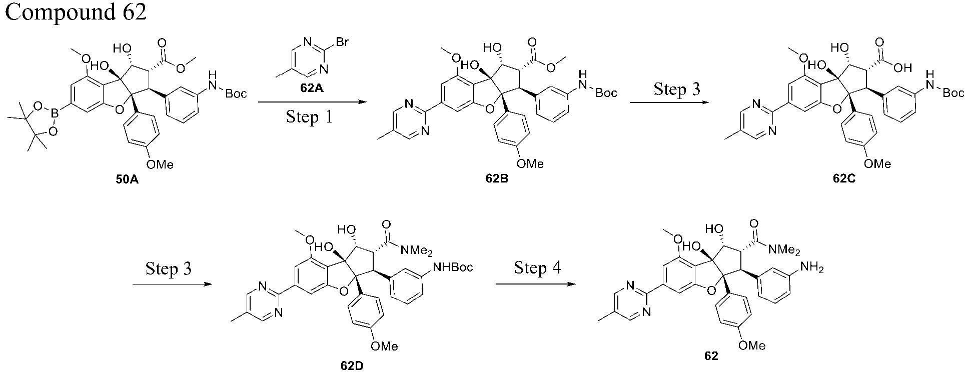

Step 1: Compound 62A (25.82 mg, 0.15 mmol), Na₂CO₃ (31.64 mg, 0.3 mmol), and Pd(dppf)Cl₂ (8.06 mg, 0.01 mmol) were added to a solution of the racemate of compound 50A (70 mg, 0.1 mmol) in dioxane/water (3 mL/0.6 mL). The reaction system was purged with nitrogen three times and left to react under microwave conditions at 130 °C for 1 h. The reaction solution was purified by reversed-phase column chromatography ((H₂O + 0.1% FA)/ACN) to give a racemate of 62B (50 mg, yield: 75%) as a yellow solid. LCMS [M+H]⁺ = 670.9.

Step 2: LiOH·H₂O (6.41 mg, 0.15 mmol) was added to a solution of the racemate of compound 62B (50 mg, 0.08 mmol) in THF/H₂O (1/1, 2 mL), and the mixture was stirred at room temperature for 18 h. The reaction solution was purified by reversed-phase column chromatography (H2O/ACN) to give a racemate of 62C (25 mg, yield: 51%) as a white solid. LCMS [M+H-C₄H₈]⁺ = 600.9.

Step 3: DIEA (7.88 mg, 0.06 mmol) was added to a solution of the racemate of compound 62C (20 mg, 0.03 mmol), dimethylamine (12.44 mg, 0.15 mmol), and HATU (17.4 mg, 0.05 mmol) in DMF (1 mL), and the mixture was stirred at room temperature for 1 h. The reaction solution was purified by reversed-phase column chromatography (H₂O/ACN) to give a racemate of 62D (25 mg, yield: 90%) as a white solid. LCMS [M+H]⁺ = 684.1.

Step 4: 6 M HCl (1 mL) was added to a solution of the racemate of compound 62D (5 mg, 0.01 mmol) in THF (1 mL), and the mixture was stirred at room temperature for 5 h. The reaction solution was purified by reversed-phase column chromatography (H₂O/ACN) to give a racemate of 62 (2.5 mg, yield: 60%) as a white solid, and the racemate was further resolved on a chiral column to give compound 62.

LCMS [M+H]⁺ = 583.2.

¹H NMR (400 MHz, DMSO) δ 8.78 (s, 2H), 7.59 (d, *J* = 4.3 Hz, 2H), 7.10 (d, *J* = 8.8 Hz, 2H), 6.63 (t, *J* = 7.8 Hz, 3H), 6.25 (s, 1H), 6.16 (d, *J* = 7.6 Hz, 1H), 5.98 (d, *J* = 7.4 Hz, 1H), 5.25 (s, 1H), 4.86-4.63 (m, 4H), 4.17 (d, *J* = 13.5 Hz, 1H), 3.99 (dd, *J* = 13.4, 6.0 Hz, 1H), 3.87 (s, 3H), 3.63 (s, 3H), 3.25 (s, 3H), 2.76 (s, 3H), 2.34 (s, 3H).

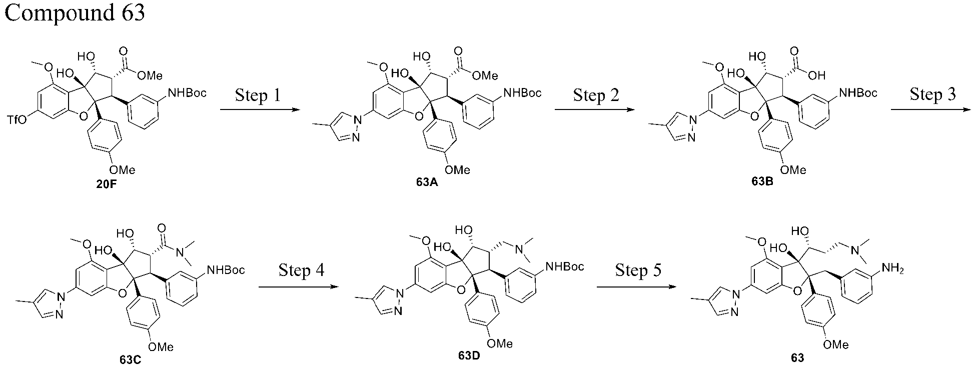

Step 1: The racemate of compound 20F (200 mg, 0.28 mmol) was dissolved in 1,4-dioxane (20 mL), and K₂CO₃ (77.28 mg, 1.38 mmol), 4-methyl-1H-pyrazole (113.70 mg, 1.38 mol), Pd₂(dba)₃ (51.28 mg, 0.01 mmol), and t-Buxphos (67.31 mg, 0.14 mmol) were then added. The mixture was stirred at 120 °C for 12 h. The reaction solution was concentrated, and the resulting crude product was purified by FLASH column chromatography (preparative low- to medium-pressure flash liquid chromatography) (PE/EA = 2/1) to give a racemate of 63A (133.7 mg, yield: 73%) as a yellow solid. LCMS [M+H-C₄H₈]⁺ = 602.2.

Step 2: The racemate of compound 63A (133.7 mg, 0.20 mmol) was dissolved in THF/H₂O (3 mL/1 mL), and LiOH·H₂O (25.60 mg, 0.60 mmol) was added. The mixture was left to react at *15* °C for 12 h. The reaction solution was purified by preparative HPLC (H₂O/ACN) to give a racemate of 63B (92.9 mg, yield: 71%) as a yellow solid. LCMS [M+H-C₄H₈]⁺ = 588.2.

Step 3: The racemate of compound 63B (92.9 mg, 0.14 mmol) was dissolved in DMF (2 mL), and dimethylamine/THF (0.1 mL, 0.29 mmol), HATU (65.86 mg, 0.17 mmol), HOAT (23.57 mg, 0.17 mol), and TMP (52.47 mg, 0.43 mmol) were added. The mixture was left to react at room temperature for 2 h. The reaction solution was purified by preparative HPLC (H₂O/ACN) to give a racemate of 63C (71.9 mg, yield: 74%) as a white solid. LCMS [M+H]⁺ = 671.3.

Step 4: BH₃/DMS (0.13 mL, 0.33 mmol) and TFA (12.50 mg, 0.11 mmol) were added to a solution of the racemate of compound 63C (72 mg, 0.11 mmol) in THF (2.5 mL), and the mixture was left to react at 20 °C for 12 h. The reaction solution was purified by preparative HPLC (H₂O/ACN) to give a racemate of 63D (28.4 mg, yield: 40%) as a yellow oil. LC-MS: [M+H]⁺ = 657.2.

Step 5: 6 N HCl (1 mL) was added to a solution of the racemate of compound 63D (28.3 mg, 0.04 mmol) in THF (1 mL), and the mixture was left to react at 15 °C for 12 h. The reaction solution was purified by preparative HPLC (H₂O/ACN) to give a racemate of 63 (5.8 mg, yield: 24%) as a white solid, and the racemate was further resolved on a chiral column to give compound 63.

LCMS [M+H]⁺ = 557.2.

¹H NMR (400 MHz, DMSO-*d*₆) δ 8.31 (s, 1H), 8.24 (s, 1H), 7.54 (s, 1H), 7.14 (d, *J* = 8.8 Hz, 2H), 6.95 (s, 2H), 6.68 (t, *J* = 7.8 Hz, 1H), 6.62 (d, *J* = 9.2 Hz, 2H), 6.25 (s, 1H), 6.19-6.17 (m, 1H), 6.07 (d, *J* = 7.6 Hz, 1H), 5.05 (s, 1H), 4.80 (brs, 1H), 4.45 (d, *J* = 4.4 Hz, 1H), 3.82 (s, 1H), 3.62 (s, 3H), 3.48-3.44 (m, 2H), 3.02-2.95 (m, 1H), 2.33 (s, 6H), 2.11 (s, 3H).

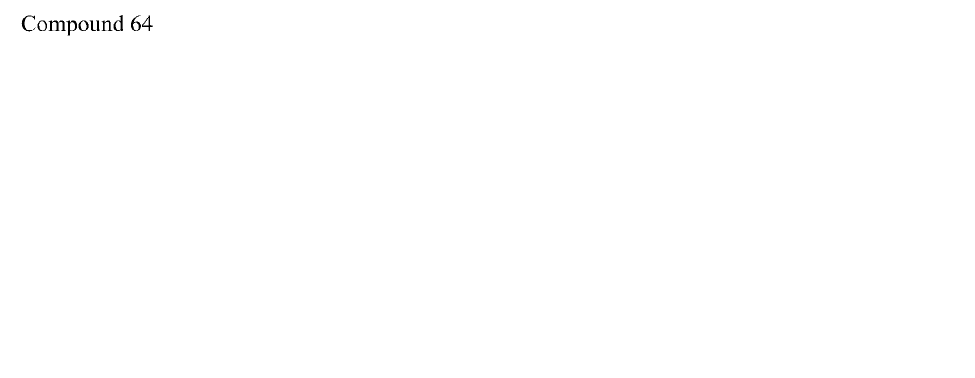

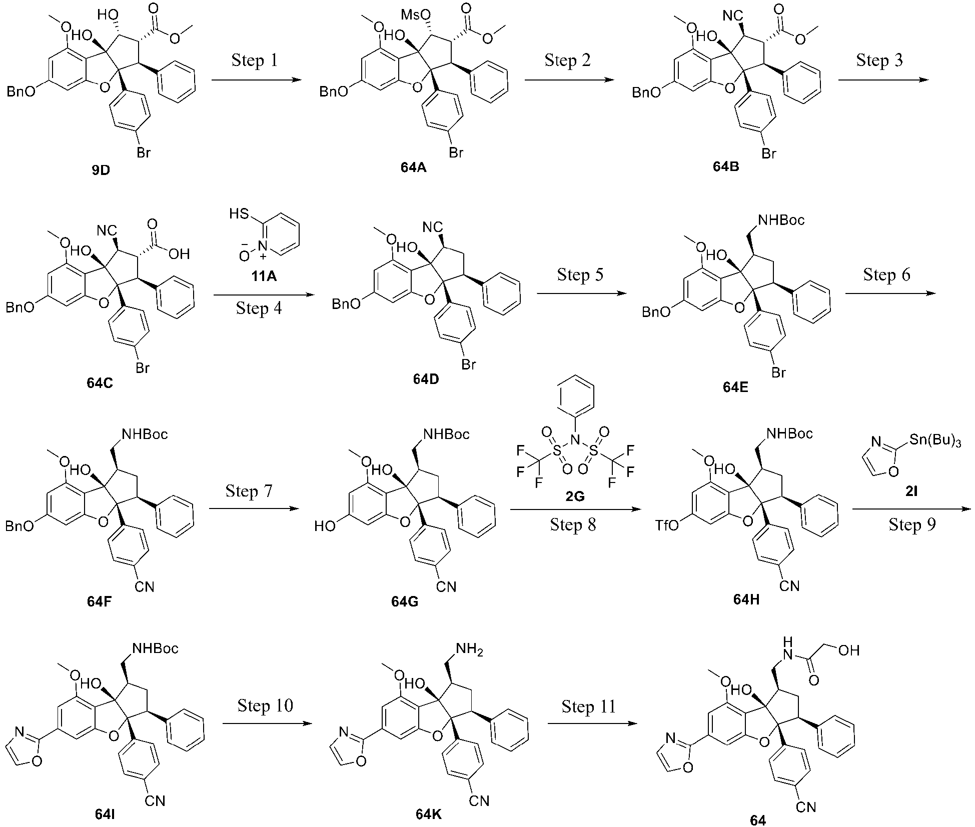

Step 1: MsCl (4250 mg, 37.3 mmol) was added to a mixture of the racemate of compound 9D (2300 mg, 3.73 mmol) in pyridine (30 mL) at 0 °C, and the resulting mixture was left to react at 0 °C for 3 h. EA (50 mL) was added to the reaction solution, and the mixture was washed with water (20 mL). The organic phase was dried, filtered, and concentrated, and the crude product was then purified by silica gel column chromatography (PE/EA = 2/1) to give a racemate of 64A (2400 mg, yield: 93%) as a white solid. LCMS [M+Na]⁺ = 717.1.

Step 2: NaCN (423 mg, 8.63 mmol) was added to a solution of the racemate of compound 64A (2.4 g, 3.45 mmol) in DMF (20 mL), and the mixture was left to react at 25 °C for 18 h. EA (30 mL) was added to the reaction solution, and the mixture was washed with water (20 mL) and then saturated brine. The organic phase was dried, concentrated, and purified by FLASH column chromatography (preparative low- to medium-pressure flash liquid chromatography) (PE/EA = 2/1) to give a racemate of 64B (2200 mg, yield: 100%) as a white solid. LCMS [M+H]⁺ = 626.1.

Step 3: H₂O (5 mL), MeOH (5 mL), and LiOH·H₂O (443 mg, 10.54 mmol) were added to a solution of the racemate of compound 64B (2200 mg, 3.51 mmol) in THF (5 mL), and the mixture was left to react at 25 °C for 18 h. The reaction solution was purified by reversed-phase column chromatography (MeCN/FA/H₂O) to give a racemate of 64C (1.9 g, yield: 88%) as a white solid.

LCMS [M+H]⁺ = 612.2.

Step 4: The racemate of compound 64C (100 mg, 0.16 mmol), compound 11A (207.3 mg, 1.63 mmol), HOAT (33.3 mg, 0.24 mmol), tert-dodecyl mercaptan (329.8 mg, 1.63 mmol), TMP (29.8 mg, 0.24 mmol), zinc meso-tetraphenylporphine (11.0 mg, 0.02 mmol), and DMF (2 mL) were mixed in a microwave tube. After the reaction system was purged with nitrogen, the microwave tube was placed in hot water at 80 °C, and the system was stirred while being irradiated with 25 W red light. A solution of EDCI (311.8 mg, 1.63 mmol) in DMF (4 mL) was slowly added dropwise to the reaction solution, and the mixture was stirred for 30 min and purified by reversed-phase column chromatography (H₂O/ACN = 40%-60%) to give a racemate of 64D (35 mg, yield: 38%) as a white solid. LCMS [M+H-H₂O]⁺ = 550.4.

Step 5: A 10 M BH₃·DMS solution (0.4 mL, 3.7 mmol) was added to a mixture of the racemate of compound 64D (210 mg, 0.37 mmol) in THF (10 mL), and the resulting mixture was stirred at 70 °C for 3 h. The reaction solution was cooled, and MeOH was slowly added. The mixture was concentrated to give a crude product. The crude product was dissolved in DCM (15 mL), and DIPEA (90 mg, 0.70 mmol) and Boc₂O (150 mg, 0.70 mmol) were added. The mixture was stirred at 25 °C for 5 h. The reaction solution was concentrated and purified by FLASH column chromatography (preparative low- to medium-pressure flash liquid chromatography) (PE:EA = 5:1) to give a racemate of 64E (200 mg, yield: 85%) as a white solid. LCMS [M+Na]⁺ = 694.2.

Step 6: The racemate of compound 64E (160 mg, 0.24 mmol), Pd₂(dba)₃ (46 mg, 0.05 mmol), DPPF (55 mg, 0.1 mmol), and Zn(CN)₂ (74 mg, 0.63 mmol) were added to NMP (20 mL), and the mixture was heated to 140 °C and stirred for 2 h under a N₂ atmosphere. The reaction solution was diluted with water and extracted with EA. The organic phase was washed with water and then brine, dried, and concentrated. The crude product was purified by FLASH column chromatography (preparative low- to medium-pressure flash liquid chromatography) (PE:EA = 2:1) to give a racemate of 64F (130 mg, yield: 88%) as a white solid. LCMS: [M+H-H₂O-C₄H₈]⁺ = 545.3.

Step 7: Pd(OH)₂/C (10%, 50 mg) was added to a solution of the racemate of compound 64F (130 mg, 0.21 mol) in EA/MeOH (1:1, 25 mL), and the mixture was purged with H₂ several times and stirred at 25 °C for 5 h. The reaction solution was filtered, and the filter cake was washed with MeOH/DCM (1/10). The combined filtrates were concentrated and purified by FLASH column chromatography (preparative low- to medium-pressure flash liquid chromatography) to give a racemate of 64G (60 mg, yield: 54%) as a white solid. LCMS: [M+Na]⁺ = 551.0.

Step 8: K₂CO₃ (32 mg, 0.228 mmol) was added to a solution of the racemate of compound 64G (60 mg, 0.114 mmol) and compound 2G (41 mg, 0.114 mol) in DMF (5 mL), and the mixture was stirred at 60 °C for 1 h, poured into water, and extracted with EA. The organic phase was washed with water and brine, dried, and concentrated. The concentrate was purified by silica gel column chromatography (EA-PE.DCM/0-50%), and the resulting crude product was further purified by reversed-phase column chromatography to give a racemate of 64H (40 mg, yield: 53%) as a white solid. LCMS [M+H-CO₂-C₄H₈]⁺ = 561.0.

Step 9: Compound 2I (11 mg, 0.03 mmol), Pd(PPh₃)₄ (3 mg, 0.002 mmol), and CuI (1 mg, 0.004 mmol) were added to a solution of the racemate of compound 64H (5 mg, 0.007 mmol) in DMF (1 mL), and the mixture was stirred at 100 °C for 2 h under a nitrogen atmosphere. EA (10 mL) was added, and the mixture was washed with water (10 mL) and then brine (10 mL). The organic phase was dried and concentrated. The crude product was purified through a preparative silica gel plate to give a racemate of 64I (3 mg, yield: 75%) as a white solid. LCMS [M+Na]⁺ = 602.2. Step 10: The racemate of compound 64I (3 mg, 0.044 mmol) was dissolved in 6 M HCl (1 mL) and THF (1 mL), and the solution was stirred at 25 °C for 5 h. The reaction solution was purified by reversed-phase column chromatography (MeCN/FA/H₂O) to give a racemate of 64K (3 mg, yield: 100%) as a white solid. LCMS [M+Na]⁺ = 480.2.

Step 11: Hydroxyacetic acid (4 mg, 0.05 mmol), HATU (4 mg, 0.01 mmol), and TMP (3 mg, 0.02 mmol) were added to a mixture of the racemate of compound 64K (3 mg, 0.01 mmol) in DMF (1 mL), and the resulting mixture was stirred at 20 °C for 2 h. The reaction solution was purified by reversed-phase column chromatography (MeCN/H₂O/FA) to give a racemate of 64 (2 mg, yield: 59%) as a white solid, and the racemate was further resolved on a chiral column to give compound 64.

LCMS [M+Na]⁺ = 560.0.

¹H NMR (400 MHz, DMSO) δ 8.41 (s, 1H), 8.27 (s, 1H), 7.79 (s, 1H), 7.59 (d, *J* = 8.4 Hz, 2H), 7.45-7.37 (m, 3H), 7.26 (d, *J* = 16.1 Hz, 2H), 7.07 (td, *J* = 14.1, 7.0 Hz, 3H), 6.95 (d, *J* = 7.2 Hz, 2H), 5.66 (s, 2H), 5.33 (d, *J* = 4.5 Hz, 1H), 3.98-3.90 (m, 3H), 3.92 (d, *J* = 5.0 Hz, 2H), 3.79 (d, *J* = 6.8 Hz, 1H), 3.73-3.66 (m, 1H), 2.29-2.20 (m, 1H), 2.19-2.13 (m, 1H).

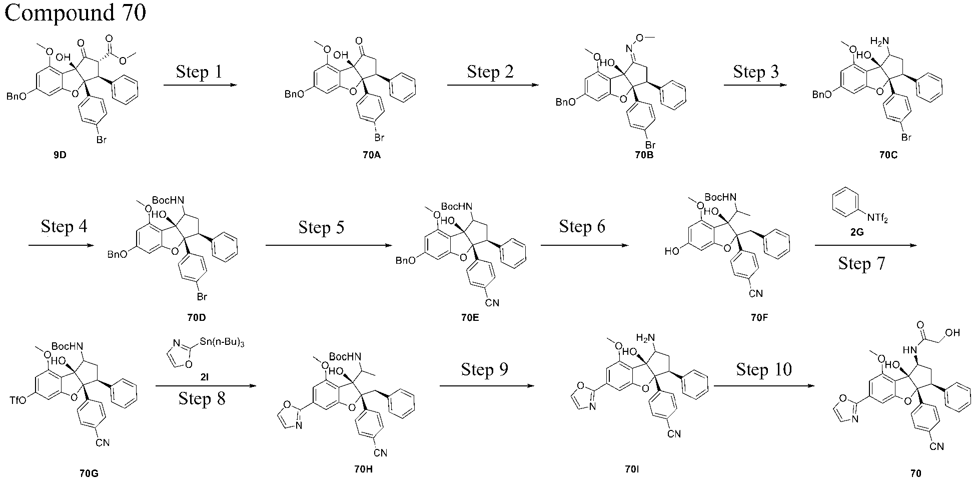

Step 1: DMAP (113 mg, 0.93 mmol) was added to a mixture of the racemate of compound 9D (1900 mg, 3.18 mmol) in H₂O/toluene (6 mL/30 mL), and the solution was then stirred at 110 °C for 2 h. The mixture was concentrated and purified by silica gel column chromatography (PE/EA = 2/1) to give a racemate of the target product 70A (1200 mg, yield: 70%). LCMS [M+H]⁺ = 557.2. Step 2: The racemate of compound 70A (1000 mg, 1.8 mmol) and dimethylhydroxylamine hydrochloride (500 mg, 5.4 mmol) were dissolved in pyridine (20 mL) and EtOH (20 mL), and the solution was heated to 70 °C and stirred for 4 h under a nitrogen atmosphere, diluted with water, and extracted with EA. The organic phase was washed with brine, dried, concentrated, and purified by silica gel column chromatography (EA/PE = 0-1/2) to give a racemate of 70B (650 mg, yield: 71%) as a yellow solid. LCMS [M+H-H₂O]⁺ = 568.1.

Step 3: A BH₃·DMS solution (2.78 mL, 5.55 mmol) was added to a solution of the racemate of compound 70B (650 mg, 0.17 mmol) in THF (10 mL), and the mixture was stirred at 65 °C for 4 h. The reaction solution was cooled to room temperature, slowly quenched with MeOH, and concentrated to give a racemate of 70C (420 mg, yield: 68%) as a white solid. LCMS [M+H]⁺ = 558.2.

Step 4: DIPEA (291 mg, 2.26 mmol) and Boc₂O (194 mg, 0.9 mmol) were added to a solution of the racemate of compound 70C (420 mg, 0.75 mmol) in DCM (10 mL), and the mixture was stirred at 25 °C for 4 h, concentrated, and purified by flash chromatography to give a racemate of 70D (300 mg, yield: 61%) as a white solid. LCMS [M+Na]⁺ = 680.2.

Step 5: The racemate of compound 70D (345 mg, 0.52 mmol), Pd₂(dba)₃ (92 mg, 0.1 mmol), DPPF (111 mg, 0.2 mmol), and Zn(CN)₂ (152 mg, 1.3 mmol) were suspended in NMP (20 mL), and the suspension was heated to 130 °C and stirred for 2 h under a nitrogen atmosphere, diluted with water, and extracted with EA. The organic phase was washed with water and brine, dried, then concentrated, and purified by silica gel column chromatography (EA/DCM = 0-1/1) to give a racemate of 70E (280 mg,yield: 89%) as a yellow solid. LCMS [M+H-H₂O]⁺ = 587.2.

Step 6: A mixture of the racemate of compound 70E (230 mg, 0.38 mmol) and Pd(OH)₂ (80 mg) in EA (10 mL) and MeOH (10 mL) was stirred at 25 °C for 3 h under a hydrogen atmosphere. The mixture was filtered, and the filtrate was concentrated to give a racemate of 70F (170 mg, yield: 87%) as a white solid. LCMS [M+Na]⁺ = 537.2.

Step 7: The racemate of compound 70F (200 mg, 0.39 mmol), K₂CO₃ (135 mg, 0.98 mmol), and compound 2G (167 mg, 0.47 mmol) were added to DMF (10 mL), and the mixture was stirred at 60 °C for 2 h. 30 mL of EA was added, and the mixture was washed with 10 mL of water. The organic phase was washed with 10 mL of water and 10 mL of brine, dried, concentrated, and purified by flash column chromatography (PE/EA = 3/1) to give a racemate of70G (150 mg, yield: 60%) as a white solid. LCMS [M+Na]⁺ = 669.0.

Step 8: Compound 2I (78 mg, 0.216 mmmol), Pd(PPh₃)₄ (12 mg, 0.01 mmol), and CuI (4 mg, 0.02 mmol) were added to a mixture of the racemate of compound 70G (35 mg, 0.054 mmol) in DMF (3 mL), and the mixture was stirred at 100 °C for 1.5 h under a nitrogen atmosphere. The reaction solution was cooled to room temperature and poured into 30 mL of EA, and the mixture was washed with 10 mL of water. The organic phase was then washed with 10 mL of water and 10 mL of brine, dried, concentrated, and purified by preparative TLC to give a racemate of 70H (25 mg, yield: 83%) as a white solid. LCMS [M+Na]⁺ = 588.2.

Step 9: The racemate of compound 70H (25 mg, 0.044 mmol) and 6 M HCl (1 mL) were added to THF (1 mL), and the mixture was stirred at 25 °C for 5 h. The reaction solution was directly purified by reversed-phase column chromatography (MeCN/FA/H₂O) to give a racemate of 70I (25 mg, yield: 100%) as a white solid. LCMS [M+Na]⁺ = 466.0.

Step 10: Glycolic acid (4 mg, 0.05 mmol), HATU (4 mg, 0.01 mmol), and TMP (3 mg, 0.02 mmol) were added to a mixture of the racemate of compound 70I (5 mg, 0.01 mmol) in DMF (1 mL). The resulting mixture was stirred at 20 °C for 2 h. The reaction solution was purified by reversed-phase column chromatography (MeCN/H₂O/FA) to give a racemate of 70 (4 mg, yield: 67%)as a white solid, and the racemate was further resolved on a chiral column to give compound 70.

LCMS [M+Na]⁺ = 546.0.

¹H NMR (400 MHz, DMSO) δ 8.26 (s, 1H), 8.02 (d, *J* = 6.9 Hz, 1H), 7.58 (d, *J* = 8.5 Hz, 2H), 7.42 (s, 1H), 7.37 (d, *J* = 8.5 Hz, 2H), 7.29 (d, *J* = 0.8 Hz, 1H), 7.19 (s, 1H), 7.12-7.07 (m, 2H), 7.03 (dd, *J* = 15.2, 7.3 Hz, 3H), 6.15 (s, 1H), 5.81 (t, *J* = 5.5 Hz, 1H), 4.65-4.52 (m, 1H), 3.95 (d, *J* = 5.5 Hz, 2H), 3.82 (s, 3H), 3.68 (dd, *J* = 14.3, 5.7 Hz, 1H), 2.40-2.38 (m, 1H), 2.03-1.97 (m, 1H).

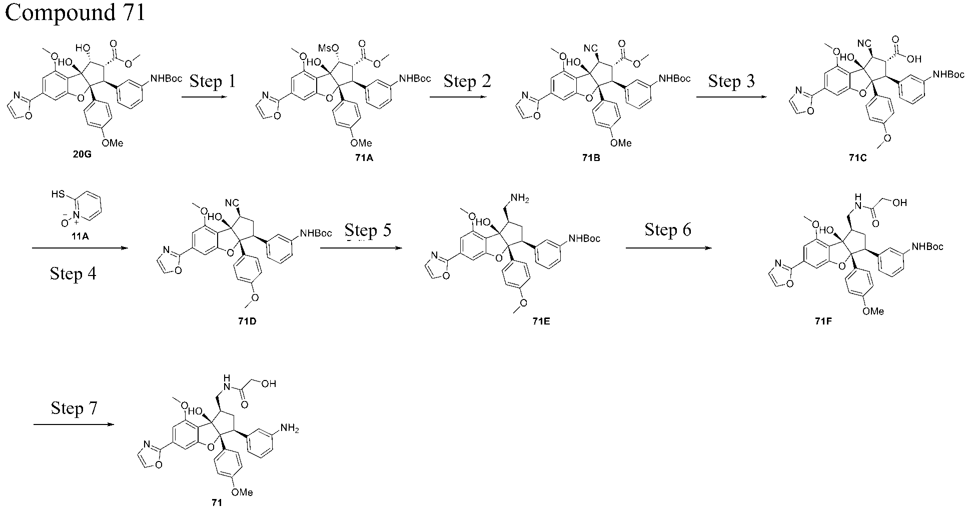

Step 1: The racemate of compound 20G (197 mg, 0.31 mmol) was dissolved in pyridine (2 mL), and MsCl (1.07 g, 9.30 mmol) was added under ice-water bath conditions. The reaction solution was stirred at 10 °C for 3 h, diluted with 6 N HCl (20 mL), extracted with EA (20 mL × 2), washed with brine (20 mL), and purified by preparative HPLC (H₂O (0.1% FA)/ACN) to give a racemate of 71A (150 mg, yield: 68%) as a white solid. LCMS [M+H]⁺ = 723.2.

Step 2: The racemate of compound 71A (150 mg, 0.21 mmol) was dissolved in DMF (2 mL), and NaCN (51.45 mg, 1.05 mmol) was added. The reaction solution was stirred at 10 °C for 12 h, diluted with a NaHCO₃ solution (20 mL), extracted with EA (20 mL × 2), washed with brine (20 mL), and purified by preparative HPLC (H₂O (0.1% FA)/ACN) to give a racemate of 71B (115 mg, yield: 85%) as a yellow solid. LCMS [M+H]⁺ = 654.2.

Step 3: The racemate of compound 71B (115 mg, 0.18 mmol) was dissolved in a solution of THF (2 mL), and LiOH·H₂O (22.68 mg, 0.54 mmol) was added. The reaction solution was stirred at 10°Cfor 3 h, concentrated, and purified by preparative HPLC (H2O (0.1% FA)/ACN) to give a racemate of 71C (95 mg, yield: 84%) as a yellow solid. LCMS [M+H-C₄H₈]⁺ = 584.7.

Step 4: A solution of the racemate of compound 71C (10 mg, 0.02 mmol), compound 11A (19.85 mg, 0.16 mmol), tert-dodecyl mercaptan (31.58 mg, 0.16 mmol), TMP (2.86 mg, 0.02 mmol), and zinc meso-tetraphenylporphine (1.06 mg, 0.001 mmol) in DMF (1 mL) was placed in a Schlenk tube. The reaction system was irradiated with 25 W red light, with the reaction temperature kept at 80 °C. EDCI (29.86 mg, 0.16 mmol) was dissolved in 1 mL of DMF, and the solution was added dropwise to the reaction system. The reaction solution was stirred for 0.5 h and purified by reversed-phase column chromatography (H₂O/ACN) to give a racemate of 71D (20 mg, yield: 24.7%) as a white solid. LCMS [M+H-C₄H₈]⁺ = 541.2.

Step 5: A solution of the racemate of compound 71D (15 mg, 0.03 mmol) in THF (1 mL) was cooled to 0 °C. LiAlH₄ (12.6 µL, 0.05 mmol) was added dropwise to the reaction system, and the mixture was stirred at 0 °C for 1 h. Na₂SO₄·10H₂O was added to the reaction system, and the mixture was stirred at room temperature for another 0.5 h. The reaction solution was filtered, and the filter cake was washed with DMF. The resulting filtrate was concentrated to dryness by rotary evaporation to give a racemate of 71E (20 mg, crude product, yield not calculated) as a yellow solid, and the product was directly used in the next step. LCMS [M+H]⁺ = 600.9.

Step 6: TMP (14.64 mg, 0.121 mmol) was added to a solution of the racemate of compound 71E (18 mg, 0.03 mmol), glycolic acid (4.57 mg, 0.06 mmol), HATU (22.84 mg, 0.06 mmol), and HOAT (8.17 mg, 0.06 mmol) in DMF (1 mL), and the mixture was stirred at room temperature for 1 h. The reaction solution was purified by reversed-phase column chromatography (H₂O/ACN) to give a racemate of 71F (4 mg, yield: 13.8%) as a white solid. LCMS [M+H-H₂O]⁺ = 640.9.

Step 7: 6 M HCl (1 mL) was added to a solution of the racemate of compound 71F (4 mg, 0.01 mmol) in THF (1 mL), and the mixture was stirred overnight at room temperature. The reaction solution was purified by reversed-phase column chromatography (H₂O/ACN) to give a racemate of 71 (1.2 mg, yield: 36.4%) as a white solid, and the racemate was further resolved on a chiral column to give compound 71.

LCMS [M+H-H₂O]⁺ = 540.9.

¹H NMR (400 MHz, DMSO) δ 8.25 (s, 1H), 7.76 (s, 1H), 7.41 (s, 1H), 7.19 (dd, *J* = 12.7, 6.6 Hz, 4H), 6.73 (dd, *J* = 18.4, 7.8 Hz, 3H), 6.42-6.24 (m, 2H), 6.16 (s, 1H), 5.63 (s, 1H), 5.35 (s, 1H), 3.95 (s, 3H), 3.90 (s, 3H), 3.66 (s, 5H), 2.86 (s, 1H), 2.15-1.98 (m, 2H).

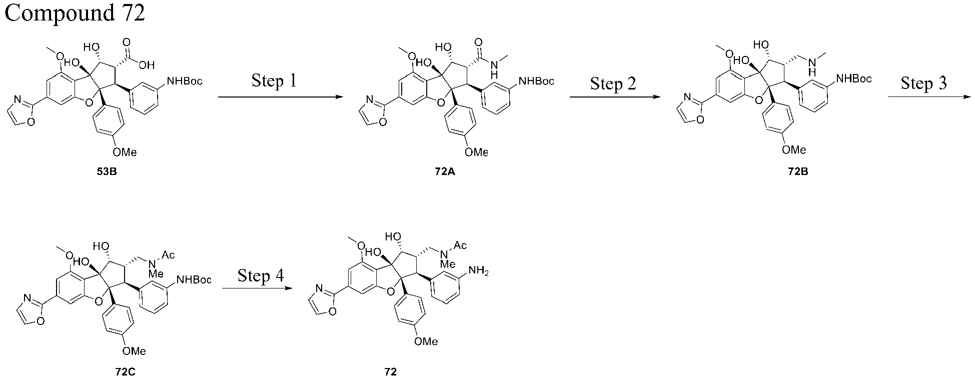

Step 1: The racemate of 53B (100 mg, 0.16 mmol), HATU (90 mg, 0.24 mmol), HOAT (32 mg, 0.24 mmol), and TMP (58 mg, 0.48 mmol) were added to a solution of DMF (5 mL), and the mixture was stirred at 17 °C for 0.5 h. Methylamine hydrochloride (21 mg, 0.32 mmol) was added, and the mixture was stirred at 28 °C for 1 h. The reaction solution was purified by silica gel column chromatography (DCM/MeOH) to give a racemate of 72A (90 mg, yield: 82%) as a yellow solid. LCMS [M+H]⁺ = 644.4.

Step 2: The racemate of compound 72A (80 mg, 0.124 mmol) was dissolved in a solution of THF (1 mL), and a solution of BH₃·DMS (0.19 mL, 2 M in THF) was added. The mixture was stirred at 60 °C for 4 h under a nitrogen atmosphere. MeOH (3 mL) was added, and the mixture was stirred at 60 °C for 16 h. The reaction solution was purified by silica gel column chromatography (DCM/MeOH) to give a racemate of 72B (15 mg, yield: 19%) as a yellow solid. LCMS [M+H]⁺ = 630.4.

Step 3: Acetic anhydride (3.7 mg, 0.035 mmol) was added to a solution of the racemate of compound 72B (15 mg, 0.023 mmol) and TEA (7.06 mg, 0.070 mmol) in DMF (1 mL), and the mixture was stirred at 27 °C for 2 h. The reaction solution was purified by silica gel column chromatography (DCM/MeOH) to give a racemate of 72C (8 mg, yield: 54%) as a yellow solid. LCMS [M+H]⁺ = 672.3.

Step 4: The racemate of compound 72C (8 mg, 0.008 mmol) was added to THF/6 M HCl (1/1, 3 mL), and the mixture was stirred at 28 °C for 5 h. The reaction solution was concentrated and purified by reversed-phase column chromatography (H₂O/ACN) to give a racemate of 72 (2.9 mg, yield: 39%) as a white solid, and the racemate was further resolved on a chiral column to give compound 72. LCMS [M+H]⁺ = 572.4.

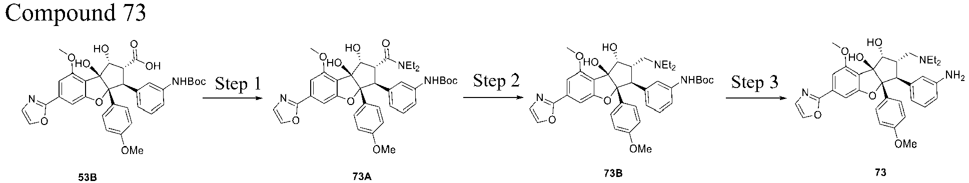

Step 1: The racemate of compound 53B (50 mg, 0.08 mmol), HATU (60.29 mg, 0.16 mmol), and DIEA (30.74 mg, 0.24 mmol) were dissolved in DMF (2 mL), and diethylamine (5.8 mg, 0.08 mmol) was then added. The mixture was stirred at room temperature for 0.5 h. The reaction solution was concentrated to dryness by rotary evaporation and purified by reversed-phase silica gel column chromatography (FA-H₂O/ACN = 45:55) to give a racemate of the target product 73A (35 mg, yield: 74%). LCMS [M+H]⁺ = 686.3.

Step 2: The racemate of compound 73A (25 mg, 0.04 mmol) was dissolved in THF (2 mL), and BH₃·DMS (0.12 mL, 2 M in THF) was added. The mixture was stirred at 60 °C for 2 h. MeOH (2 mL) was added, and the mixture was stirred for 14 h. The reaction solution was concentrated to dryness by rotary evaporation and then purified by silica gel column chromatography (DCM:MeOH = 85:15) to give a racemate of compound 73B (20 mg, yield: 74.5%). LCMS [M+H]⁺ = 672.3.

Step 3: The racemate of compound 73B (15 mg, 0.02 mmol) was dissolved in THF (3 mL), and 6 M HCl (3 mL) was added. The mixture was stirred at room temperature for 2 h. The reaction solution was concentrated to dryness by rotary evaporation and purified by reversed-phase silica gel column chromatography (FA-H₂O/ACN = 70:30) to give a racemate of the target product 73 (1 mg, yield: 8.7%), and the racemate was further resolved on a chiral column to give compound 73.

LCMS [M+H]⁺ = 572.2.

1H NMR (400 MHz, DMSO) δ 8.23 (s, 1H), 8.16 (s, 1H), 7.39 (d, J = 0.7 Hz, 1H), 7.16 (d, J = 8.8 Hz, 2H), 7.11 (s, 2H), 6.69 (t, J = 7.7 Hz, 1H), 6.62 (d, J = 8.9 Hz, 2H), 6.29 (s, 1H), 6.19 (d, J = 8.1 Hz, 1H), 6.10 (d, J = 7.2 Hz, 1H), 5.22 (s, 1H), 4.81 (s, 1H), 4.51 (s, 1H), 3.85 (s, 3H), 3.62 (s, 3H), 3.05 (s, 2H), 2.74 (s, 2H), 1.24 (s, 4H), 1.00 (d, J = 5.2 Hz, 6H).

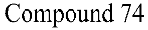

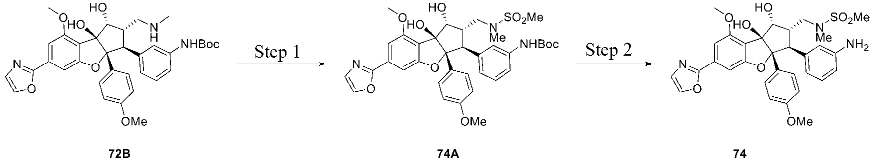

Step 1: MsCl (1.5 mg, 0.014 mmol) was added to a solution of the racemate of 72B (6 mg, 0.009 mmol) and TEA (2.82 mg, 0.028 mmol) in DMF (1 mL), and the mixture was stirred at 27 °C for 2 h. The reaction solution was concentrated and purified by preparative TLC (DCM/MeOH = 10/1) to give a racemate of 74A (3.3 mg, yield: 50%) as a yellow solid. LCMS [M+H]⁺ = 708.5.

Step 2: The racemate of compound 74A (3 mg, 0.004 mmol) was dissolved in THF/6 M HCl (1/1, 3 mL), and the solution was stirred at 28 °C for 5 h. The reaction solution was concentrated and purified by reversed-phase column chromatography (H₂O/ACN) to give a racemate of 74 (1 mg, yield: 39%) as a white solid, and the racemate was further resolved on a chiral column to give compound 74.

LCMS [M+H]⁺ = 608.5.

¹H NMR (400 MHz, DMSO) δ 8.23 (s, 1H), 7.40 (s, 1H), 7.21 (s, 1H), 7.14 (s, 1H), 7.11 (d, *J* = 2.1 Hz, 3H), 6.70 (t, *J* = 7.8 Hz, 2H), 6.61 (d, *J* = 9.0 Hz, 2H), 6.32 (s, 1H), 6.22-6.18 (m, 2H), 5.33 (d, *J* = 4.7 Hz, 1H), 5.27 (s, 1H), 4.78 (d, *J* = 5.0 Hz, 3H), 4.47 (s, 1H), 3.85 (s, 3H), 3.61 (s, 3H), 2.90 (s, 3H), 2.83 (s, 3H), 2.00 (dd, *J* = 14.4, 6.8 Hz, 6H), 1.46 (d, *J* = 7.4 Hz, 3H).

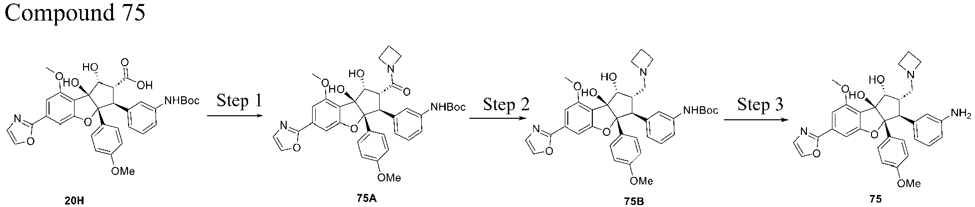

Step 1: The racemate of compound 20H (50 mg, 0.08 mmol), HATU (30.15 mg, 0.08 mmol), HOAT (10.79 mg, 0.08 mmol), and TMP (38.43 mg, 0.32 mmol) were dissolved in DMF (2 mL), and cyclobutylamine (4.53 mg, 0.08 mmol) was added. The mixture was stirred at room temperature for 2 h. The reaction solution was concentrated to dryness by rotary evaporation and purified by reversed-phase silica gel column chromatography (H₂O/ACN = 50:50) to give a racemate of the target product 75A (25 mg, yield: 70%). LCMS [M+H]⁺ = 670.2.

Step 2: The racemate of compound 75A (25 mg, 0.04 mmol) was dissolved in THF (2 mL), and BH₃·DMS (0.12 mL, 2 N in THF) was added. The mixture was stirred at 60 °C for 2 h. MeOH (2 mL) was added, and the mixture was stirred for 14 h. The reaction solution was concentrated to dryness by rotary evaporation and then purified by silica gel column chromatography (DCM:MeOH = 85:15) to give a racemate of compound 75B (9 mg, yield: 39%). LCMS [M+H]⁺ = 656.2.

Step 3: The racemate of compound 75B (9 mg, 0.01 mmol) was dissolved in THF (1 mL), and 6 M HCl (1 mL) was added. The mixture was stirred at room temperature for 2 h. The reaction solution was concentrated to dryness by rotary evaporation and purified by reversed-phase silica gel column chromatography (H₂O/ACN = 70:30) to give a racemate of compound 75 (1 mg, yield: 18%), and the racemate was further resolved on a chiral column to give compound 75.

LCMS [M+H]⁺ = 556.2.

1H NMR (400 MHz, DMSO) δ 8.23 (d, J = 0.7 Hz, 1H), 8.21 (s, 1H), 7.39 (d, J = 0.7 Hz, 1H), 7.15-7.08 (m, 4H), 6.70 (t, J = 7.7 Hz, 1H), 6.61 (d, J = 9.0 Hz, 2H), 6.27 (s, 1H), 6.19 (d, J = 7.8 Hz, 1H), 6.10 (d, J = 7.6 Hz, 1H), 5.17 (s, 1H), 4.79 (s, 1H), 4.43 (d, J = 4.2 Hz, 1H), 3.84 (s, 3H), 3.61 (d, J = 2.2 Hz, 3H), 3.51 (d, J = 14.1 Hz, 1H), 2.82 (d, J = 12.6 Hz, 1H), 2.69 (dd, J = 17.5, 6.7 Hz, 1H), 2.42 (d, J = 10.4 Hz, 1H), 2.03-1.92 (m, 3H), 1.24 (s, 5H).

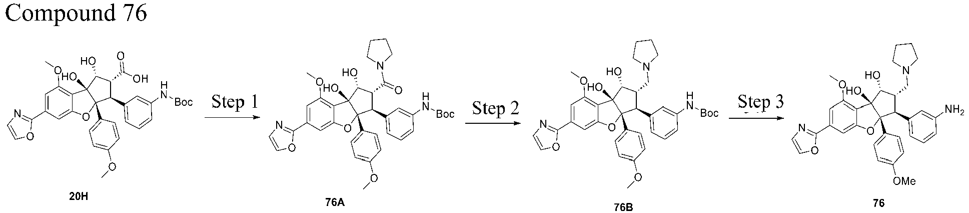

Step 1: TMP (19.2 mg, 0.16 mmol) was added to a solution of the racemate of compound 20H (50.0 mg, 0.08 mmol), cyclopentylamine (5.6 mg, 0.08 mmol), HATU (120.6 mg, 0.32 mmol), and HOAT (21.6 mg, 0.16 mmol) in DMF (2 mL), and the mixture was stirred at 25 °C for 2 h. The reaction solution was purified by silica gel column chromatography (DCM/MeOH) to give a racemate of compound 76A (40 mg, yield: 74%). LCMS [M+H]⁺ = 684.6.

Step 2: BH₃·DMS (0.09 mL, 0.18 mmol, 2 N in THF) was added to a mixture of the racemate of compound 76A (40.0 mg, 0.06 mmol) in THF (2 mL), and the mixture was stirred at 60 °C for 2 h. Methanol (8 mL) was added, and the mixture was stirred at 60 °C for 18 h. The reaction solution was concentrated and purified by silica gel column chromatography (DCM/MeOH) to give a racemate of compound 76B (30 mg, yield: 76%). LCMS [M+H]⁺ = 670.2.

Step 3: The racemate of compound 76B (30.0 mg, 0.04 mmol) was dissolved in THF (1 mL), and 6 M HCl (2 mL) was added at 0 °C. The mixture was stirred at 25 °C for 1 h. The reaction solution was purified by silica gel column chromatography (DCM/MeOH) to give a racemate of compound 76 (23.3 mg, yield: 91%), and the racemate was further resolved on a chiral column to give compound 76.

LCMS [M+H]⁺ = 570.2.

¹H NMR (400 MHz, DMSO) δ 10.57 (s, 1H), 10.01 (s, 2H), 8.25 (d, *J* = 0.7 Hz, 1H), 7.41 (d, *J* = 0.7 Hz, 1H), 7.21-7.09 (m, 6H), 7.09-6.94 (m, 2H), 6.62 (d, *J* = 9.0 Hz, 2H), 5.53 (s, 1H), 5.37 (s, 1H), 4.74 (d, *J* = 3.4 Hz, 1H), 3.86 (s, 3H), 3.79 (s, 1H), 3.70 (d, *J* = 13.6 Hz, 1H), 3.62 (s, 3H), 3.32 (dd, *J* = 16.9, 6.7 Hz, 4H), 3.06 (s, 1H), 2.96-2.86 (m, 1H), 2.10-1.91 (m, 4H).

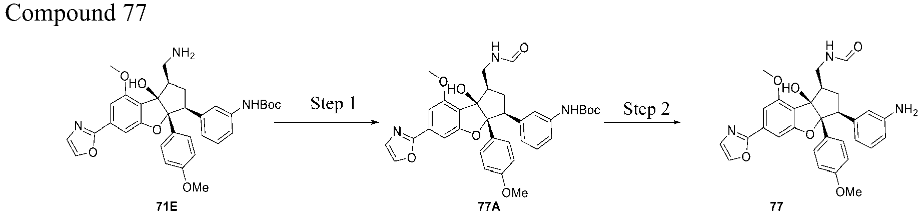

Step 1: The racemate of compound 71E (10 mg, 0.017 mmol) and ethyl formate (63 mg, 0.85 mmol) were dissolved in DMF (5 mL), and the solution was stirred at 60 °C for 18 h and purified by reversed-phase column chromatography (MeCN/FA/H₂O) to give a racemate of 77A (2.5 mg, yield: 23%) as a white solid. LCMS [M+H-H₂O]⁺ = 610.2.

Step 2: A mixture of the racemate of compound 77A (2.5 mg, 0.034 mmol) and formic acid (4 mL) in THF (0.5 mL) was stirred at 25 °C for 5 h and purified by reversed-phase column chromatography (MeCN/FA/H₂O) to give a racemate of 77 (1.5 mg, yield: 71%) as a white solid, and the racemate was further resolved on a chiral column to give compound 77.

LCMS [M+Na]⁺ = 550.2.

¹H NMR (400 MHz, DMSO) δ 8.25 (s, 1H), 8.14 (s, 1H), 7.86 (s, 1H), 7.41 (s, 1H), 7.19 (d, *J* = 6.0 Hz, 2H), 7.15 (d, *J* = 8.9 Hz, 2H), 6.69 (t, *J* = 7.9 Hz, 4H), 6.28 (s, 1H), 6.23 (d, *J* = 7.8 Hz, 1H), 6.09 (d, *J* = 8.0 Hz, 1H), 5.32 (t, *J* = 4.6 Hz, 1H), 5.21 (s, 1H), 4.80 (s, 1H), 3.93 (s, 3H), 3.66 (s, 3H), 2.09 (d, *J* = 8.2 Hz, 1H), 2.00 (d, *J* = 7.5 Hz, 3H).

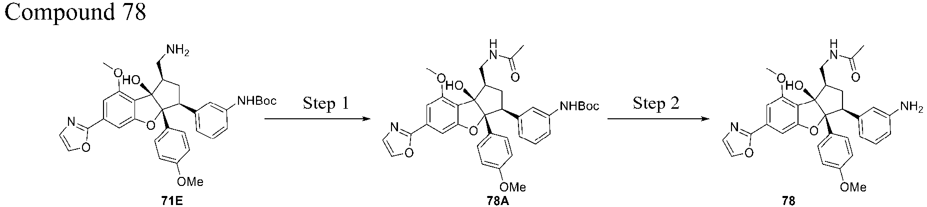

Step 1: A mixture of the racemate of compound 71E (10 mg, 0.017 mmol) and Ac₂O (48 mg, 0.034 mmol) in DMF (5 mL) was stirred at 25 °C for 18 h and purified by reversed-phase column chromatography (MeCN/FA/H₂O) to give a racemate of 78A (5 mg, yield: 45%) as a white solid. LCMS [M+Na]⁺ = 664.3.

Step 2: A mixture of the racemate of compound 78A (5 mg, 0.008 mmol) and 6 M HCl (1 mL) in THF (1 mL) was stirred at 25 °C for 5 h and purified by reversed-phase column chromatography (MeCN/FA/H₂O) to give a racemate of 78 (2 mg, yield: 50%) as a white solid, and the racemate was further resolved on a chiral column to give compound 78.

LCMS [M+Na]⁺ = 564.2.

¹H NMR (400 MHz, DMSO) δ 8.25 (s, 1H), 7.65 (s, 1H), 7.41 (s, 1H), 7.20 (d, *J* = 4.5 Hz, 3H), 7.15 (d, *J* = 8.9 Hz, 2H), 6.69 (dd, *J* = 8.3, 5.0 Hz, 4H), 6.29 (s, 1H), 6.23 (d, *J* = 7.8 Hz, 1H), 6.10 (d, *J* = 7.2 Hz, 1H), 5.33 (t, *J* = 4.6 Hz, 1H), 5.16 (s, 1H), 4.80 (s, 1H), 3.93 (s, 3H), 3.66 (s, 3H), 3.51 (s, 2H), 2.00 (d, *J* = 7.6 Hz, 2H), 1.87 (s, 3H).

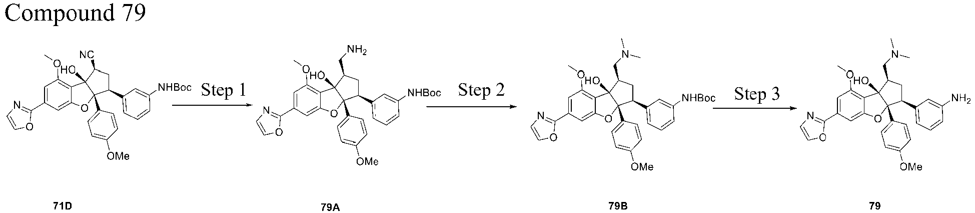

Step 1: Aqueous ammonia (1 drop) was added to a mixture of the racemate of compound 71D (20 mg, 0.034 mmol) and Raney nickel (100 mg) in DMF (5 mL), and the mixture was stirred at 25 °C for 18 h. The reaction solution was filtered, and the filtrate was concentrated to give a racemate of 79A (20 mg, white crude product, yield not calculated), and the product was directly used in the next step. LCMS [M+H]⁺ = 600.2.

Step 2: Sodium triacetoxyborohydride (20 mg, 0.09 mmol) was added to a solution of the racemate of compound 79A (18 mg, 0.03 mmol), AcOH (18 mg, 0.3 mmol), and 30% formaldehyde (150 mg, 1.5 mmol) in DMF (2 mL), and the mixture was stirred at 25 °C for 18 h and purified by reversed-phase column chromatography (MeCN/FA/H₂O) to give a racemate of 79B (8 mg, yield: 42%) as a white solid. LCMS [M+H]⁺ = 628.2.

Step 3: 6 M HCl (1 mL) was added to a solution of the racemate of compound 79B (8 mg, 0.013 mmol) in THF (0.5 mL). The mixture was stirred at 25 °C for 5 h and purified by reversed-phase column chromatography (MeCN/FA/H₂O) to give a racemate of 79 (3 mg, yield: 45%) as a white solid, and the racemate was further resolved on a chiral column to give compound 79.

LCMS [M+H]⁺ = 528.2.

¹H NMR (400 MHz, DMSO) δ 8.25 (s, 1H), 8.22 (s, 1H), 7.41 (s, 1H), 7.19 (d, *J* = 4.5 Hz, 2H), 7.15 (d, *J* = 8.9 Hz, 2H), 6.73-6.66 (m, 4H), 6.32 (s, 1H), 6.23 (d, *J* = 7.7 Hz, 1H), 6.13 (d, *J* = 7.4 Hz, 1H), 5.32 (s, 1H), 4.80 (s, 1H), 3.93 (s, 3H), 3.66 (s, 3H), 2.89 (s, 1H), 2.37 (s, 6H), 2.20-2.18 (m, 1H), 2.08-1.95 (m, 3H).

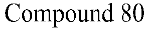

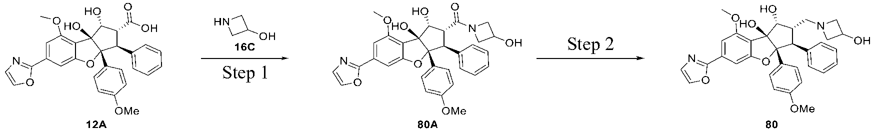

Step 1: DIEA (46.94 mg, 0.388 mmol) was added to a solution of the racemate of compound 12A (50 mg, 0.097 mol), compound 16C (12.75 mg, 0.116 mmol), HATU (55.28 mg, 0.145 mmol), and HOAt (19.79 mg, 0.145 mmol) in DMF (5 mL), and the mixture was stirred at 20 °C for 1 h. The reaction solution was diluted with water (100 mL) and extracted with ethyl acetate (50 mL × 3). The organic phase was dried over anhydrous sodium sulfate, filtered, concentrated, and then purified by silica gel column chromatography (DCM:MeOH = 10:1) to give a racemate of 80A (45 mg, yield: 73.7%) as a white solid. LCMS [M+H]⁺ = 571.

Step 2: A solution of BH₃·DMS (0.8 mL, 1.665 mmol, 2 N in THF) and trifluoroacetic acid (63 mg, 0.549 mmol) in THF (1 mL) was added to a solution of the racemate of compound 80A (95 mg, 0.166 mol) in THF (10 mL), and the mixture was stirred at 0 °C for 1 h. MeOH (20 mL) was added to the reaction solution, and the mixture was stirred for 1 h. The reaction solution was concentrated and purified by preparative reversed-phase column chromatography (H₂O (0.1% FA)/CH₃CN) to give a racemate of compound 80 (80 mg, yield: 86.3%), and the racemate was further resolved on a chiral column to give compound 80.

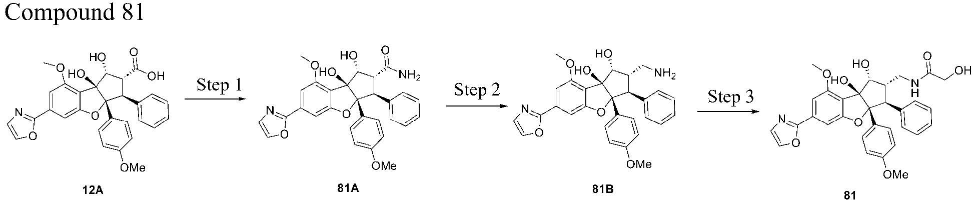

Step 1: The racemate of compound 12A (100.0 mg, 0.19 mmol), HATU (88.5 mg, 0.23 mmol), and DIEA (30.1 mg, 0.23 mmol) were dissolved in DMF (2 mL), and NH₄Cl (20.58 mg, 0.39 mmol) was added. The mixture was stirred at 25 °C for 2 h, diluted with water, and extracted with DCM. The organic phase was washed with water and brine, dried over anhydrous Na₂SO₄, filtered, concentrated, and then purified by silica gel column chromatography (DCM/MeOH) to give a racemate of compound 81A (90 mg, yield: 90%). LCMS [M+H]⁺ = 515.5.

Step 2: The racemate of compound 81A (70.0 mg, 0.14 mmol) was dissolved in THF (2 mL), and TFA (15.5 mg, 0.14 mmol) was added. BH₃·DMS (0.21 mL, 0.41 mmol) was added, and the mixture was stirred at 25 °C for 2 h. The solution was diluted with an aqueous NaOH solution, stirred at 25 °C for 30 min, concentrated, and then purified by silica gel column chromatography (DCM/MeOH) to give a racemate of compound 81B (23 mg, yield: 33%). LCMS [M+H]⁺ = 501.2. Step 3: TMP (9.7 mg, 0.08 mmol) was added to a solution of the racemate of compound 81B (10.0 mg, 0.02 mmol), glycolic acid (1.5 mg, 0.02 mmol), HATU (15.2 mg, 0.04 mmol), and HOAT (5.4 mg, 0.04 mmol) in DMF (0.5 mL). The mixture was stirred at 25 °C for 2 h and purified by reversed-phase column chromatography (0.02% FA/H₂O:acetonitrile = 0-100%) to give a racemate of compound 81 (1.1 mg, yield: 9.8%), and the racemate was further resolved on a chiral column to give compound 81.

LCMS [M+H]⁺ = 559.2.

¹H NMR (400 MHz, DMSO) δ 8.23 (d, *J* = 0.6 Hz, 1H), 8.05 (s, 1H), 7.40 (d, *J* = 0.7 Hz, 1H), 7.15-7.07 (m, 7H), 7.05-6.93 (m, 2H), 6.57 (t, *J* = 8.1 Hz, 2H), 5.69 (s, 1H), 5.32 (t, *J* = 4.7 Hz, 1H), 5.27 (s, 1H), 5.09 (d, *J* = 4.4 Hz, 1H), 4.39 (t, *J* = 4.1 Hz, 1H), 3.91-3.82 (m, 5H), 3.65 (d, *J* = 13.6 Hz, 1H), 3.59 (s, 3H), 3.27-3.25 (m, 1H), 1.29 (s, 1H).

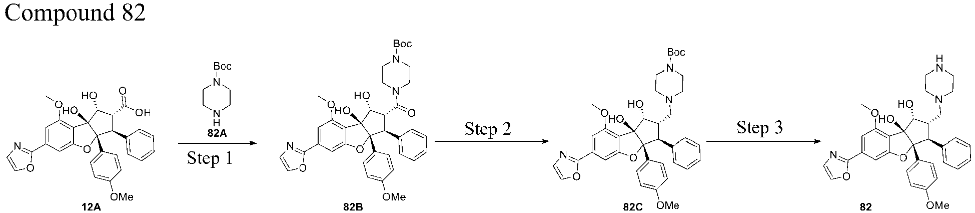

Step 1: The racemate of compound 12A (25 mg, 0.05 mmol), HATU (36.88 mg, 0.05 mmol), and DIEA (18.8 mg, 0.15 mmol) were dissolved in DMF (2 mL), and compound 82A (9.03 mg, 0.05 mmol) was then added. The mixture was stirred at room temperature for 0.5 h. The reaction solution was concentrated to dryness by rotary evaporation and purified by reversed-phase silica gel column chromatography (H₂O/ACN = 50:50) to give a racemate of the target product 82B (20 mg, yield: 60%). LCMS [M+H]⁺ = 684.5.

Step 2: The racemate of compound 82B (10 mg, 0.015 mmol) was dissolved in THF (0.5 mL), and BH₃·DMS (3.4 mg, 2 N in THF) was added. The mixture was stirred at room temperature for 2 h. The reaction solution was concentrated to dryness by rotary evaporation and then purified by preparative TLC (DCM:MeOH = 15:1) to give a racemate of compound 82C (11.5 mg, yield: 65%). LCMS [M+H]⁺ = 670.3.

Step 3: The racemate of compound 82C (11.5 mg, 0.02 mmol) was dissolved in THF (2 mL), and 6 M HCl (2 mL) was added. The mixture was stirred at room temperature for 2 h. The reaction solution was concentrated to dryness by rotary evaporation and purified by reversed-phase silica gel column chromatography (FA-H₂O/ACN = 80:20) to give a racemate of compound 82 (3 mg, yield: 26.3%), and the racemate was further resolved on a chiral column to give compound 82. LCMS [M+H]⁺ = 570.2.

1H NMR (400 MHz, DMSO) δ 8.23 (d, J = 0.7 Hz, 1H), 8.20 (s, 1H), 7.39 (d, J = 0.8 Hz, 1H), 7.16-7.04 (m, 7H), 7.00 (d, J = 7.3 Hz, 1H), 6.96 (d, J = 7.3 Hz, 2H), 6.59 (d, J = 9.0 Hz, 2H), 5.26 (s, 1H), 4.79 (s, 1H), 4.50 (d, J = 3.8 Hz, 1H), 3.85 (s, 3H), 3.67 (d, J = 13.9 Hz, 1H), 3.59 (s, 3H), 3.13 (s, 2H), 2.94 (s, 4H), 2.44-2.35 (m, 4H), 2.05 (d, J = 9.7 Hz, 1H).

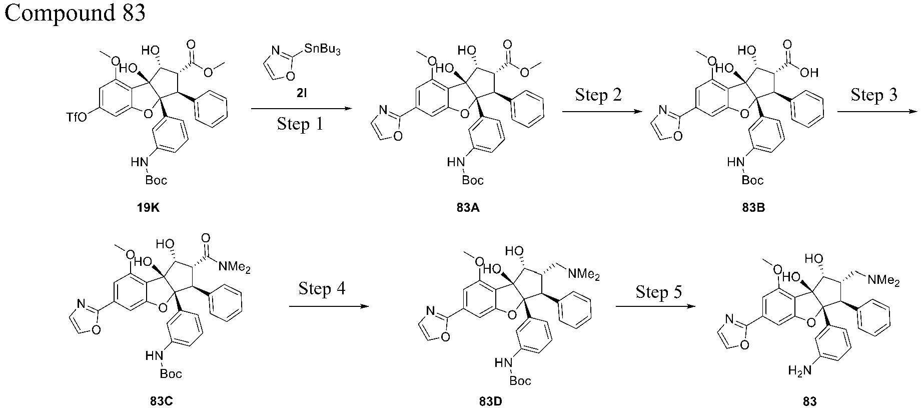

Step 1: Compound 2I (695 mg, 1.9 mmol), Pd(PPh₃)₄ (83 mg, 0.07 mmol), and CuI (27 mg, 0.14 mmol) were added to a solution of the racemate of compound 19K (500 mg, 0.72 mmol) in DMF (5 mL), and the mixture was stirred at 100 °C for 1 h. The reaction solution was filtered, concentrated, and then purified by silica gel column chromatography (DCM/MeOH = 10/1) to give a racemate of 83A (370 mg, yield: 84%) as a grayish-yellow solid. LCMS [M+H]⁺ = 615.6.

Step 2: The racemate of compound 83A (400 mg, 0.65 mmol) and LiOH (68 mg, 1.63 mmol) were dissolved in THF/H₂O/MeOH (3/1/1, 10 mL), and the solution was stirred at 30 °C for 3 h. The pH of the reaction solution was adjusted to 5 with 3 M HCl. After concentration, the residue was purified by silica gel column chromatography (DCM/MeOH) to give a racemate of 83B (250 mg, yield: 64%) as a white solid. LCMS [M+Na]⁺ = 623.6.

Step 3: The racemate of compound 83B (100 mg, 0.17 mmol), HATU (95 mg, 0.25 mmol), HOAT (34 mg, 0.25 mmol), and TMP (60 mg, 0.50 mmol) were added to DMF (5 mL), and the mixture was stirred at 25 °C for 0.5 h. Dimethylamine (27 mg, 0.33 mmol) was added, and the mixture was stirred at 28 °C for 1 h. The reaction solution was concentrated and then purified by silica gel column chromatography (DCM/MeOH) to give a racemate of 83C (43 mg, yield: 41%) as a yellow solid. LCMS [M+H]⁺ = 628.7.

Step 4: The racemate of compound 83C (20 mg, 0.032 mmol) was dissolved in THF (1 mL), and TFA (3.6 mg, 0.032 mmol) and BH₃·DMS (0.048 mL, 2 N in THF) were added. The mixture was stirred at 25 °C for 2 h under a nitrogen atmosphere. A saturated aqueous NaOH solution (1 mL) was added, and the mixture was stirred at 25 °C for 2 h. The reaction solution was purified by preparative TLC (DCM/MeOH = 10/1) to give a racemate of 83D (10 mg, yield: 51%) as a white solid. LCMS [M+H]⁺ = 614.2.

Step 5: The racemate of compound 83D (10 mg, 0.016 mmol) was dissolved in a solution of THF/6 M HCl (1/1, 3 mL), and the mixture was stirred at 28 °C for 5 h. The reaction solution was concentrated and then purified by reversed-phase column chromatography (H₂O/ACN) to give a racemate of 83 (4.6 mg, yield: 55%) as a white solid, and the racemate was further resolved on a chiral column to give compound 83.

LCMS [M+H]⁺ = 514.5.

¹H NMR (400 MHz, DMSO) δ 9.46 (s, 1H), 8.25 (s, 1H), 7.41 (s, 1H), 7.18-7.05 (m, 5H), 7.01 (d, *J* = 7.4 Hz, 2H), 6.72 (t, *J* = 7.9 Hz, 1H), 6.55 (s, 1H), 6.45 (d, *J* = 7.7 Hz, 1H), 6.22 (d, *J* = 7.6 Hz, 1H), 5.37 (s, 1H), 5.10 (s, 1H), 4.67 (s, 2H), 3.88 (s, 3H), 3.61 (d, *J* = 13.9 Hz, 1H), 2.91 (s, 7H).

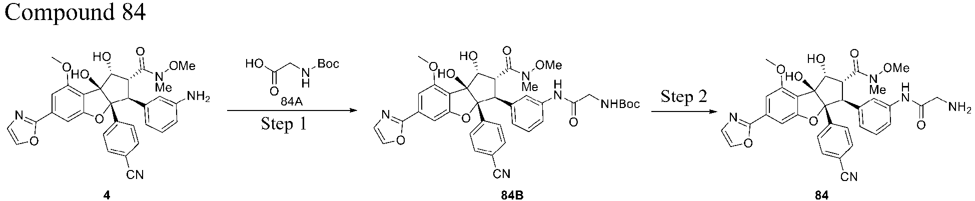

Step 1: The racemate of compound 4 (25 mg, 0.044 mmol) was dissolved in DMF (2 mL), and compound 84A (11.54 mg, 0.066 mmol), HATU (33.44 mg, 0.088 mmol), HOAT (11.97 mg, 0.088 mmol), and TMP (15.96 mg, 0.132 mmol) were added. The mixture was stirred at 25 °C for 1 h under a nitrogen atmosphere. The reaction solution was concentrated and purified by reversed-phase column chromatography (ACN/0.1% FA-H₂O) to give a racemate of 84B (25 mg, yield: 78%) as a white solid. LCMS [M+H]⁺ = 726.6.

Step 2: The racemate of compound 84B (25 mg, 0.034 mmol) was dissolved in THF (2 mL), and 6 M HCl (2 mL) was added. The mixture was stirred at 25 °C for 2 h under a nitrogen atmosphere. The reaction solution was concentrated and purified by reversed-phase column chromatography (ACN/0.1% FA-H₂O) to give a racemate of 84 (19.4 mg, yield: 90%) as a white solid, and the racemate was further resolved on a chiral column to give compound 84.

LCMS [M+H]⁺ = 626.2.

¹H NMR (400 MHz, DMSO) δ 9.74 (s, 1H), 8.25 (d, J = 0.6 Hz, 1H), 7.52 (d, J = 8.6 Hz, 2H), 7.41 (d, J = 0.7 Hz, 1H), 7.36-7.29 (m, 3H), 7.23 (dd, J = 10.3, 4.4 Hz, 2H), 7.16 (d, J = 1.1 Hz, 1H), 6.97 (t, J = 7.9 Hz, 1H), 6.58 (d, J = 7.7 Hz, 1H), 5.68 (s, 1H), 5.17 (d, J = 4.2 Hz, 1H), 4.83 (s, 1H), 4.43 (d, J = 13.8 Hz, 1H), 4.20 (d, J = 9.8 Hz, 1H), 3.93 (s, 3H), 3.85 (s, 3H), 3.35 (s, 2H), 3.09 (s, 3H).

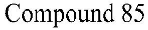

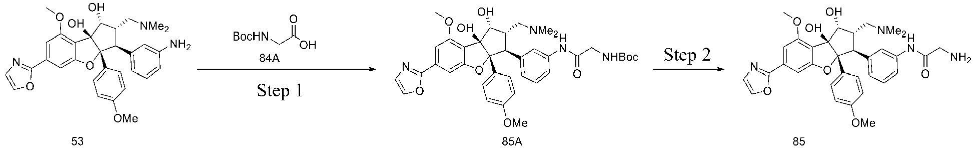

Step 1: TMP (20.19 mg, 0.17 mmol) was added to a solution of the racemate of compound 53 (45 mg, 0.08 mmol), compound 84A (29.01 mg, 0.17 mmol), HATU (62.98 mg, 0.17 mmol), and HOAT (22.54 mg, 0.17 mmol) in DMF (3 mL), and the mixture was stirred at room temperature for 1 h. The reaction solution was extracted with water and ethyl acetate. The organic phase was washed with saturated brine, dried over anhydrous sodium sulfate, filtered, concentrated, and then purified by silica gel column chromatography (DCM/MeOH = 10/1) to give a racemate of 85A (36 mg, yield: 62%) as a yellow solid. LCMS [M+H]⁺ = 701.6.

Step 2: 6 M HCl (3 mL) was added to a solution of the racemate of compound 85A (36 mg, 0.05 mmol) in THF (3 mL), and the mixture was stirred overnight at room temperature. The reaction solution was purified by reversed-phase column chromatography (H₂O/ACN) to give a racemate of 85 (12 mg, yield: 38.7%) as a white solid, and the racemate was further resolved on a chiral column to give compound 85.

LCMS [M+H]⁺ = 601.5.

¹H NMR (400 MHz, DMSO) δ 8.26 (d, *J* = 5.0 Hz, 2H), 8.23 (s, 1H), 7.41 (d, *J* = 8.7 Hz, 1H), 7.31 (dd, *J* = 21.2, 10.2 Hz, 2H), 7.20-7.08 (m, 5H), 7.01 (td, *J* = 7.9, 4.0 Hz, 1H), 6.71-6.63 (m, 2H), 6.60 (d, *J* = 8.9 Hz, 2H), 4.53 (d, *J* = 4.2 Hz, 1H), 3.85 (s, 3H), 3.79 (d, *J* = 3.1 Hz, 1H), 3.64 (s, 1H), 3.62-3.58 (m, 3H), 3.52 (s, 2H), 3.03 (s, 1H), 2.31 (d, *J* = 8.3 Hz, 6H), 2.10 (s, 3H).

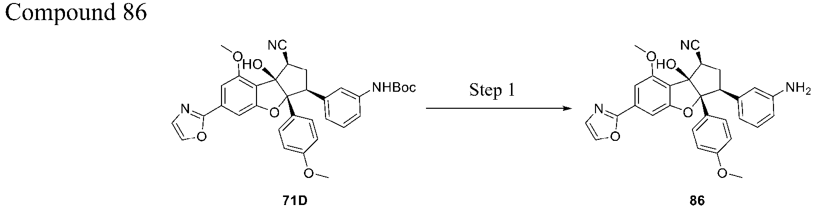

Step 1: The racemate of compound 71D (18 mg, 0.02 mmol) was dissolved in THF (2 mL), and 6 M HCl (2 mL) was added. The mixture was stirred at room temperature for 2 h. The reaction solution was concentrated and purified by reversed-phase silica gel column chromatography (FAH₂O/ACN = 65%:35%) to give a racemate of the target product 86 (6.1 mg, yield: 37%), and the racemate was further resolved on a chiral column to give compound 86.

LCMS [M+H]⁺ = 496.2.

¹H NMR (400 MHz, DMSO) δ 8.27 (s, 1H), 7.43 (d, *J* = 0.6 Hz, 1H), 7.24 (d, *J* = 1.7 Hz, 2H), 7.17 (d, *J* = 8.9 Hz, 2H), 6.76 (d, *J* = 9.0 Hz, 2H), 6.71 (t, *J* = 7.7 Hz, 1H), 6.28 (d, *J* = 8.1 Hz, 1H), 6.25 (s, 1H), 6.01 (d, *J* = 7.1 Hz, 2H), 4.94 (s, 2H), 4.04 (dd, *J* = 10.8, 8.4 Hz, 1H), 3.97 (s, 3H), 3.68 (s, 3H), 3.28-3.22 (m, 1H), 2.41 (dd, *J* = 12.4, 8.3 Hz, 1H), 1.24 (s, 1H).

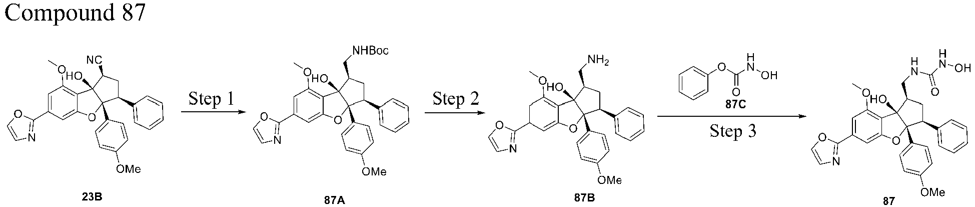

Step 1: The racemate of compound 23B (60 mg, 0.54 mmol), Raney-Ni (21.75 mg, 0.375 mmol), TEA (25 mg, 0.25 mmol), and (Boc)₂O (136 mg, 0.625 mmol) were dissolved in DMF/THF (2.0 mL/2.0 mL), and the solution was stirred at 25 °C for 12 h under a hydrogen atmosphere. The reaction solution was filtered. Water was added, and the mixture was extracted with ethyl acetate twice. The organic phase was concentrated and purified by normal-phase column chromatography (DCM/MeOH) to give a racemate of 87A (40 mg, yield: 55%) as a white solid. LCMS [M+H-H₂O-C₄H₈]⁺ = 511.3.

Step 2: The racemate of compound 87A (40 mg, 0.068 mmol) was dissolved in THF (2 mL), and 6 M HCl (2 mL) was added. The mixture was stirred at 25 °C for 2 h under a nitrogen atmosphere. The reaction solution was concentrated and purified by reversed-phase column chromatography (ACN:0.1% FA-H₂O) to give a racemate of 87B (27 mg, yield: 82%) as a white solid. LCMS [M+H-H₂O]⁺ = 467.3.

Step 3: The racemate of compound 87B (10.0 mg, 0.021 mmol) was dissolved in THF (2 mL), and compound 87C (9.47 mg, 0.062 mmol) and imidazole (14 mg, 0.21 mmol) were added. The mixture was stirred at 70 °C for 5 h under a nitrogen atmosphere. The reaction solution was concentrated and purified by reversed-phase column chromatography (ACN/0.1% FA-H₂O) to give a racemate of 87 (27 mg, yield: 24%) as a white solid, and the racemate was further resolved on a chiral column to give compound 87.

LCMS [M+H]⁺ = 526.2.

¹H NMR (400 MHz, DMSO) δ 8.63 (s, 1H), 8.41 (s, 1H), 8.25 (d, J = 0.7 Hz, 1H), 7.41 (d, J = 0.7 Hz, 1H), 7.22 (dd, J = 2.8, 1.1 Hz, 1H), 7.21-7.19 (m, 1H), 7.14 (t, J = 6.2 Hz, 2H), 7.10-7.03 (m, 3H), 6.91 (d, J = 6.7 Hz, 2H), 6.78 (t, J = 5.7 Hz, 1H), 6.70-6.66 (m, 2H), 5.42 (s, 1H), 3.95 (s, 3H), 3.68-3.61 (m, 5H), 3.42-3.34 (m, 1H), 2.89 (dd, J = 11.2, 5.7 Hz, 1H), 2.23-2.12 (m, 1H), 2.10-2.01 (m, 1H).

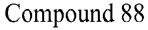

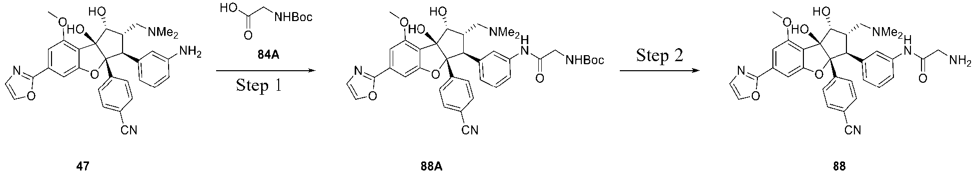

Step 1: The racemate of compound 47 (18 mg, 0.03 mmol), HATU (12.72 mg, 0.03 mmol), HOAT (4.55 mg, 0.03 mmol), and TMP (16.21 mg, 0.13 mmol) were dissolved in DMF (3 mL), and compound 84A (5.86 mg, 0.03 mmol) was added. The mixture was stirred at room temperature for 2 h. The reaction solution was concentrated to dryness by rotary evaporation and purified by reversed-phase silica gel column chromatography (ACN:FA-H₂O = 30%:70%) to give a racemate of the target product 88A (18 mg, yield: 75%). LCMS [M+H]⁺ = 696.3.

Step 2: The racemate of compound 88A (16 mg, 0.02 mmol) was dissolved in THF (3 mL), and 6 M HCl (3 mL) was added. The mixture was stirred at room temperature for 2 h. The reaction solution was concentrated to dryness by rotary evaporation and purified by reversed-phase silica gel column chromatography (ACN:FA-H₂O = 30:70) to give a racemate of the target product 88 (14 mg, yield: 93%), and the racemate was further resolved on a chiral column to give compound 88.

LCMS [M+H]⁺ = 596.2.

1H NMR (400 MHz, DMSO) δ 9.76 (s, 1H), 8.24 (d, J = 0.7 Hz, 1H), 8.20 (s, 2H), 7.49 (d, J = 8.7 Hz, 2H), 7.40 (dd, J = 4.7, 3.9 Hz, 3H), 7.36 (s, 1H), 7.29 (d, J = 8.2 Hz, 1H), 7.15-7.12 (m, 2H), 7.02 (t, J = 7.9 Hz, 1H), 6.72 (d, J = 8.0 Hz, 1H), 5.57 (s, 1H), 4.51 (d, J = 4.1 Hz, 1H), 3.84 (s, 3H), 3.81-3.75 (m, 1H), 3.44 (s, 2H), 3.16 (s, 2H), 2.62 (t, J = 11.3 Hz, 1H), 2.25 (s, 7H), 2.04 (d, J = 11.4 Hz, 1H).

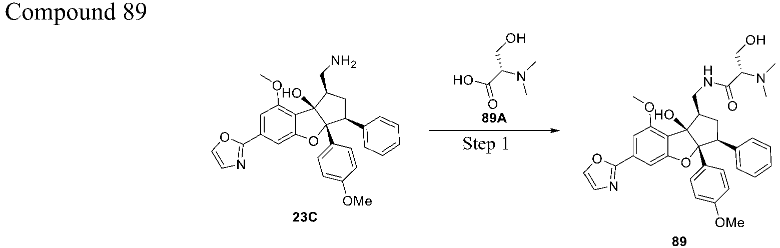

Step 1: TMP (15.0 mg, 0.12 mmol) was added to a solution of the racemate of compound 23C (15.0 mg, 0.03 mmol), compound 89A (4.1 mg, 0.03 mmol), HATU (23.6 mg, 0.06 mmol), and HOAT (8.4 mg, 0.06 mmol) in DMF (0.5 mL), and the mixture was stirred at 25 °C for 2 h. The reaction solution was directly purified by reversed-phase column chromatography (H₂O:acetonitrile = 0-100%) to give a racemate of compound 89 (2.8 mg, yield: 15%), and the racemate was further resolved on a chiral column to give compound 89.

LCMS [M+H]⁺ = 600.2.

¹H NMR (400 MHz, DMSO) δ 8.25 (d, *J* = 0.6 Hz, 1H), 7.41 (d, *J* = 0.6 Hz, 1H), 7.22 (d, *J* = 7.2 Hz, 2H), 7.17-7.00 (m, 5H), 6.92 (d, *J* = 6.6 Hz, 2H), 6.68 (dd, *J* = 9.0, 2.6 Hz, 2H), 5.25 (dd, *J* = 5.5, 2.0 Hz, 1H), 3.97 (d, *J* = 3.6 Hz, 3H), 3.79 (dd, *J* = 18.1, 9.4 Hz, 2H), 3.64 (s, 4H), 3.35 (s, 2H), 2.84 (s, 1H), 2.66 (dd, *J* = 6.7, 4.8 Hz, 1H), 2.32 (dd, *J* = 5.6, 3.8 Hz, 6H), 2.22 (d, *J* = 4.0 Hz, 2H).

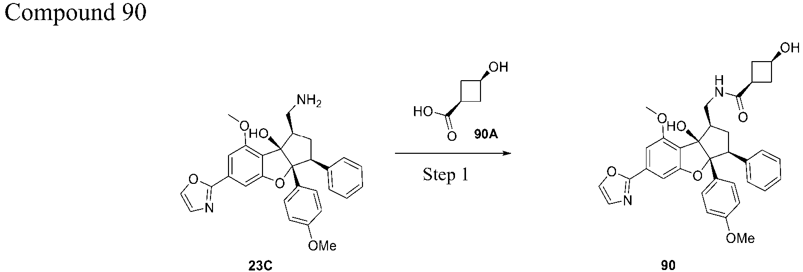

Step 1: TMP (15.0 mg, 0.12 mmol) was added to a solution of the racemate of compound 23C (15.0 mg, 0.03 mmol), compound 90A (3.6 mg, 0.03 mmol), HATU (23.6 mg, 0.06 mmol), and HOAT (8.4 mg, 0.06 mmol) in DMF (0.5 mL), and the mixture was stirred at 25 °C for 2 h. The reaction solution was directly purified by reversed-phase column chromatography (H₂O:acetonitrile = 0-100%) to give a racemate of compound 90 (4.7 mg, yield: 26%), and the racemate was further resolved on a chiral column to give compound 90.

LCMS [M+H]⁺ = 565.2.

¹H NMR (400 MHz, DMSO) δ 8.25 (d, *J* = 0.6 Hz, 1H), 7.55 (t, *J* = 5.5 Hz, 1H), 7.41 (d, *J* = 0.7 Hz, 1H), 7.21 (dd, *J* = 8.9, 1.0 Hz, 2H), 7.14-7.01 (m, 5H), 6.90 (d, *J* = 6.9 Hz, 2H), 6.68 (d, *J* = 9.0 Hz, 2H), 5.26 (s, 1H), 5.09 (d, *J* = 7.1 Hz, 1H), 3.98-3.87 (m, 4H), 3.70 (dt, *J* = 13.3, 5.2 Hz, 1H), 3.65 (s, 3H), 3.62-3.53 (m, 1H), 3.37 (d, *J* = 6.4 Hz, 1H), 2.81 (d, *J* = 8.0 Hz, 1H), 2.47-2.37 (m, 1H), 2.37-2.25 (m, 2H), 2.10 (dt, *J* = 13.1, 9.1 Hz, 2H), 1.99 (dd, *J* = 18.2, 9.5 Hz, 2H).

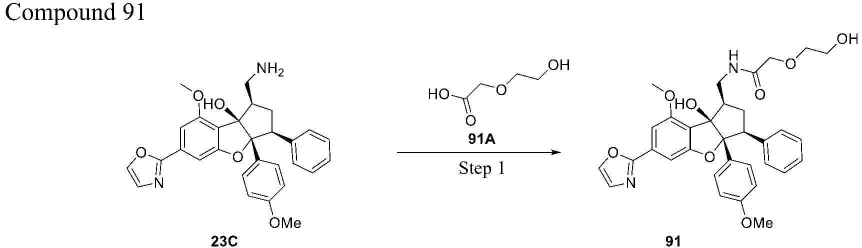

Step 1: TMP (15.0 mg, 0.12 mmol) was added to a solution of the racemate of compound 23C (15.0 mg, 0.03 mmol), compound 91A (3.7 mg, 0.03 mmol), HATU (23.6 mg, 0.06 mmol), and HOAT (8.4 mg, 0.06 mmol) in DMF (0.5 mL), and the mixture was stirred at 25 °C for 2 h. The reaction solution was directly purified by reversed-phase column chromatography (H₂O:acetonitrile = 0-100%) to give a racemate of compound 91 (4.8 mg, yield: 26%), and the racemate was further resolved on a chiral column to give compound 91.

LCMS [M+H]⁺ = 569.2.

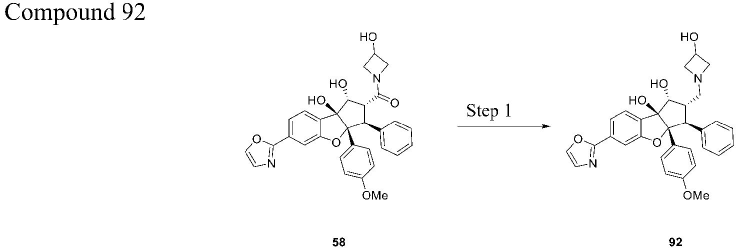

Step 1: The racemate of compound 58 (10 mg, 0.018 mmol) was dissolved in THF (1 mL), and BH₃·DMS (0.028 mL, 2 N in THF) and TFA (2.0 mg, 0.018 mmol) were added. The mixture was stirred at 25 °C for 2 h under a nitrogen atmosphere. The reaction solution was quenched with an aqueous NaOH solution (0.5 mL), filtered, concentrated, and then purified by reversed-phase column chromatography (ACN:0.1% FA-H₂O) to give a racemate of 92 (3.2 mg, yield: 51%) as a white solid, and the racemate was further resolved on a chiral column to give compound 92.

LCMS [M+1]⁺ = 527.2.

¹H NMR (400 MHz, DMSO) δ 8.26 (s, 1H), 8.23 (d, J = 0.7 Hz, 1H), 7.57 (dd, J = 7.8, 1.4 Hz, 1H), 7.49 (dd, J = 10.1, 4.5 Hz, 2H), 7.39 (d, J = 0.7 Hz, 1H), 7.09 (dd, J = 8.1, 5.9 Hz, 4H), 7.03 (t, J = 7.2 Hz, 1H), 6.87 (d, J = 7.1 Hz, 2H), 6.64 (d, J = 9.0 Hz, 2H), 5.55 (s, 1H), 4.52 (d, J = 5.7 Hz, 1H), 4.10 (s, 1H), 3.61 (d, J = 7.7 Hz, 5H), 3.53 (d, J = 13.9 Hz, 2H), 2.85 (s, 2H), 2.62 (s, 2H), 2.22 (d, J = 11.9 Hz, 1H).

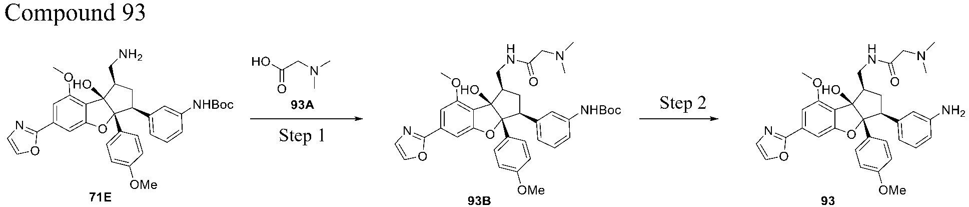

Step 1: Compound 93A (2.89 mg, 0.03 mmol), HATU (12.19 mg, 0.03 mmol), HOAT (4.36 mg, 0.03 mmol), and TMP (9.71 mg, 0.08 mmol) were added to a solution of the racemate of compound 71E (16.0 mg, 0.03 mmol) in DMF (1 mL), and the mixture was stirred at 30 °C for 2 h. The reaction solution was directly purified by reversed-phase column chromatography (H₂O/ACN) to give a racemate of 93B (8.0 mg, yield: 38.4%) as a white solid. LC-MS: (M+H)⁺ = 685.7.

Step 2: 6 N HCl (0.5 mL) was added to a solution of the racemate of compound 93B (8.0 mg, 2.21 mmol) in THF (0.5 mL), and the mixture was stirred at 30 °C for 2 h. The reaction solution was purified by reversed-phase column chromatography (H₂O/ACN) to give a racemate of 93 (3.0 mg, yield: 43.9%) as a white solid, and the racemate was further resolved on a chiral column to give compound 93.

LCMS [M+H]⁺ = 585.5.

¹H NMR (400 MHz, DMSO) δ 9.79 (s, 1H), 8.56 (s, 1H), 8.26 (s, 1H), 7.42 (d, *J* = 0.6 Hz, 1H), 7.22 (s, 2H), 7.15 (d, *J* = 8.8 Hz, 2H), 7.04 (s, 1H), 6.71 (d, *J* = 8.9 Hz, 4H), 5.31 (s, 1H), 3.96 (s, 5H), 3.90-3.86 (m, 1H), 3.66 (s, 3H), 3.63-3.57 (m, 1H), 2.83 (s, 7H), 2.19-2.10 (m, 2H), 1.24 (s, 1H).

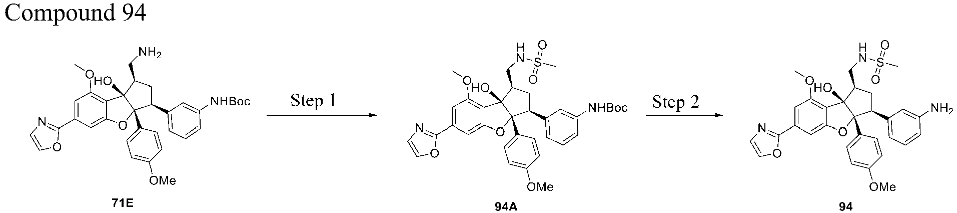

Step 1: TMP (0.1 mL) was added to a solution of the racemate of compound 71E (16.0 mg, 0.03 mmol) in DCM (1 mL), and MsCl (0.1 mL) was then slowly added dropwise under ice bath conditions. The mixture was stirred at 0 °C for 1 h. The reaction solution was purified by reversed-phase column chromatography (H₂O/ACN) to give a racemate of 94A (2.0 mg, yield: 29.4%) as a white solid. LC-MS: [M+H-H₂O]⁺ = 660.6.

Step 2: HCl (6 N, 0.1 mL) was added to a solution of the racemate of compound 94A (4.0 mg, 0.01 mmol) in THF (0.1 mL), and the mixture was stirred at 25 °C for 2 h. The reaction solution was concentrated and purified by preparative TLC to give a racemate of 94 (2.0 mg, yield: 58.6%) as a white solid, and the racemate was further resolved on a chiral column to give compound 94.

LCMS [M+H]⁺ = 579.2.

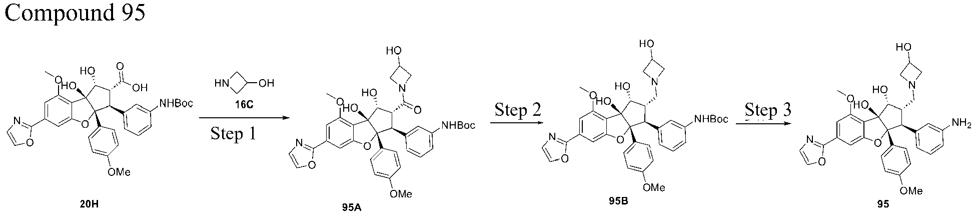

Step 1: The racemate of compound 20H (200 mg, 0.32 mmol), HATU (181 mg, 0.48 mmol), HOAT (65 mg, 0.48 mmol), and TMP (115 mg, 0.95 mmol) were added to DMF (10 mL), and the mixture was stirred at 25 °C for 0.5 h. Compound 16C (69 mg, 0.63 mmol) was added, and the mixture was stirred at 28 °C for 1 h. The reaction solution was concentrated and purified by silica gel column chromatography (DCM/MeOH) to give a racemate of 95A (150 mg, yield: 69%) as a yellow solid. LCMS [M+H]⁺ = 686.6.

Step 2: The racemate of compound 95A (10 mg, 0.015 mmol) was dissolved in a solution of THF (1 mL), and TFA (1.7 mg, 0.015 mmol) and BH₃·DMS (0.022 mL, 2 N in THF) were added. The mixture was stirred at 25 °C for 2 h under a nitrogen atmosphere. A saturated aqueous NaOH solution (1 mL) was added, and the mixture was stirred at 25 °C for 2 h. The reaction solution was concentrated and then purified by silica gel column chromatography (DCM/MeOH = 10/1) to give a racemate of 95B (5 mg, yield: 51%) as a white solid. LCMS [M+H]⁺ = 672.2.

Step 3: The racemate of compound 95B (5 mg, 0.007 mmol) was dissolved in THF/6 M HCl (1/1, 3 mL), and the solution was stirred at 28 °C for 5 h. The reaction solution was concentrated and purified by reversed-phase column chromatography (H₂O/ACN) to give a racemate of 95 (3.2 mg, yield: 74%) as a white solid, and the racemate was further resolved on a chiral column to give compound 95.

LCMS [M+H]⁺ = 572.5.

¹H NMR (400 MHz, DMSO) δ 8.24 (d, *J* = 0.6 Hz, 1H), 7.40 (s, 1H), 7.13 (t, *J* = 6.9 Hz, 4H), 6.71 (t, *J* = 7.7 Hz, 1H), 6.62 (d, *J* = 8.8 Hz, 2H), 6.32 (s, 1H), 6.22 (d, *J* = 7.8 Hz, 1H), 6.14 (d, *J* = 6.9 Hz, 1H), 5.31 (d, *J* = 8.2 Hz, 2H), 4.85 (s, 2H), 4.49 (s, 1H), 4.36 (s, 1H), 4.16 (s, 2H), 3.85 (s, 3H), 3.62 (s, 3H), 3.47 (d, *J* = 13.5 Hz, 1H), 3.07 (s, 2H), 2.80 (s, 1H).

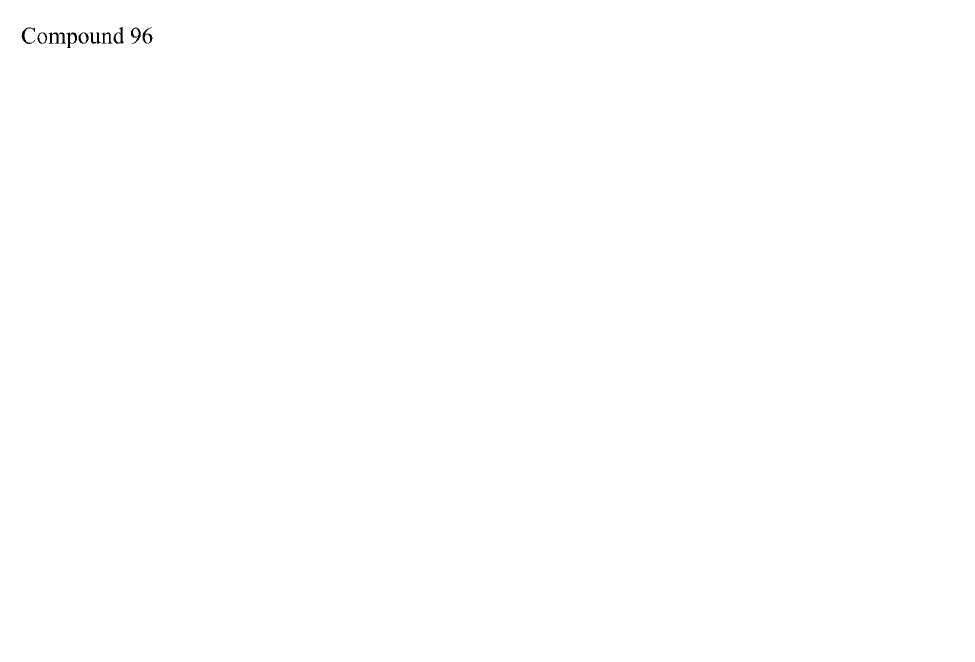

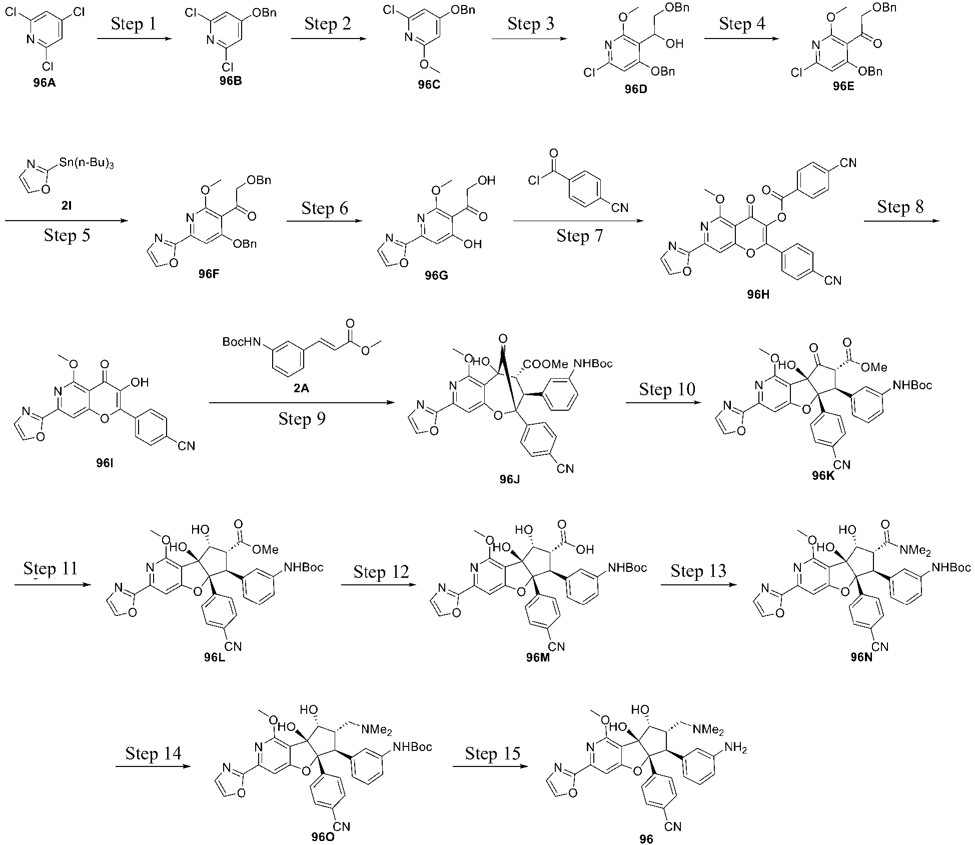

Step 1: At 0 °C, 60% NaH (50.00 g, 301.49 mmol) was added to a solution of compound 96A (50.00 g, 274.08 mmol) in DMF (250 mL), and the mixture was stirred for 20 min. Benzyl alcohol (31.12 g, 287.78 mmol) was added dropwise, and the mixture was stirred for another 3 h. The reaction solution was poured into water, and a large amount of white solid precipitated. The mixture was filtered, and the filter cake was slurried with petroleum ether. After filtration, 96B (50 g, yield: 72%) was obtained as a white solid. LCMS [M+H]⁺ = 254.0.

Step 2: Compound 96B (118.00 g, 464.37 mmol) was dissolved in toluene (300 mL), and sodium methoxide (67.99 g, 1.26 mol) was added. The mixture was stirred at 60 °C for 5 h. The reaction solution was quenched with water and extracted with ethyl acetate. The organic phases were combined, concentrated, and then slurried with petroleum ether to give compound 96C (90.0 g, yield: 77%). LCMS [M+H]⁺ = 250.2.

Step 3: Compound 96C (10.00 g, 40.05 mmol) was dissolved in diethyl ether (300 mL), and n-butyllithium (16.82 mL, 42.05 mmol, 2.50 M) was added dropwise at -78 °C. The mixture was stirred at -78 °C for 2 h. 2-(Benzyloxy)acetaldehyde (6.62 g, 44.05 mmol) was dissolved in diethyl ether, and the resulting solution was added dropwise to the above reaction solution. The mixture was stirred overnight, quenched with water, extracted, concentrated to dryness by rotary evaporation, and purified by silica gel column chromatography to give compound 96D (5.30 g, yield: 33.1%). LCMS [M+H]⁺ = 400.0.

Step 4: Compound 96D (10.00 g, 25.01 mmol) was dissolved in DMSO (60 mL). At 10 °C, acetic anhydride (39.57 g, 387.63 mmol) was added, and the mixture was stirred overnight. The reaction solution was quenched with water, extracted, concentrated to dryness by rotary evaporation, and purified by column chromatography to givecompound 96E (6.0 g, yield: 60%). LCMS [M+H]⁺ = 398.2.

Step 5: Compound 96E (4000 mg, 10.054 mmol) was dissolved in a solution of NMP (40 mL), and the compound was added. The mixture was stirred at 150 °C for 4 h. The reaction solution was concentrated to dryness by rotary evaporation. Water (150 mL) was added, and the mixture was extracted with EA (200 mL) three times. The organic phase was washed with saturated brine (150 mL), dried over anhydrous sodium sulfate, concentrated to dryness by rotary evaporation, and then purified by silica gel column chromatography (EA/PE = 50%/50%) to give compound 96F (1000 mg, yield: 23%). LCMS [M+H]⁺ = 431.3

Step 6: Compound 96F (800.0 mg, 1.858 mmol) was dissolved in a solution of DCM (10 mL), and BCl₃ (11.15 mL) was slowly added at -10 °C. The mixture was stirred at -10 °C for 1 h. The reaction solution was concentrated to dryness by rotary evaporation. MeOH (10 mL) was added, and the mixture was concentrated to dryness by rotary evaporation and purified by reversed-phase column chromatography (ACN/0.1 % FA-H₂O = 40%/60%) to give compound 96G (260 mg, yield: 45%). LCMS [M+H]⁺ = 251.2.

Step 7: Compound 96G (150 mg, 0.5995 mmol) was dissolved in a solution of DCM (10 mL), and DBU (274 mg, 1.8 mmol) was added. Then, 4-cyanobenzoyl chloride (248 mg, 1.5 mmol) was added in portions, and the mixture was stirred at room temperature (25 °C) for 16 h. The reaction solution was concentrated to dryness by rotary evaporation and purified by silica gel column chromatography (DCM:EA = 0:1) to give compound 96H (150 mg, yield: 51%). LCMS [M+H]⁺ = 491.2.

Step 8: Compound 96H (250 mg, 0.51 mmol) was dissolved in a solution of MeOH (6 mL), and sodium methoxide (83 mg, 1.53 mmol) was added. The mixture was stirred at 60 °C for 1 h. The pH of the reaction solution was adjusted to <7 with acetic acid. After filtration, the filter cake was collected to give compound 96I (147 mg, yield: 80%). LCMS [M+H]⁺ = 362.3

Step 9: Compound 96I (170 mg, 0.47 mmol) and compound 2A (781.99 mg, 2.82 mmol) were dissolved in CHCl₃/TFE (7/3, 15 mL). A syringe was used to inject the reaction mixture (12 mL/h) into a coil wound around a jacketed glass cylinder equipped with a 250 W ultraviolet lamp. The jacket temperature was adjusted to keep the internal reaction temperature at 0-5 °C, and the UV lamp was turned on simultaneously. The reaction mixture was irradiated for 1 h and concentrated under reduced pressure to remove the solvent. The residue was purified using a silica gel plug (EtOAc/hexane) to remove the excess cinnamate, giving a racemate of 96J (120 mg, yield: 75%) as a pale yellow solid. LCMS [M+H]⁺ = 639.5.

Step 10: The racemate of compound 96J (130 mg, 0.20 mmol) was dissolved in methanol (5 mL), andsodium methoxide(27.48 mg, 0.51 mmol) was added at 0 °C. The reaction solution was stirred at 65 °C for 1.5 h, concentrated to dryness by rotary evaporation, and directly purified by silica gel column chromatography (0.2% FA in EA/PE = 60%/40%) to give a racemate of compound 96K (68 mg, yield: 52%). LCMS: [M+H]⁺ = 639.6.

Step 11: The racemate of compound 96K (68 mg, 0.11 mmol) was dissolved in a mixed solution of ACN/CHCl₃ (4 mL/4 mL), and AcOH (63.89 mg, 1.06 mmol) and STAB (112.87 mg, 0.53 mmol) were slowly added at 0 °C. The mixture was stirred at room temperature (20 °C) for 2 h. The reaction solution was concentrated to dryness by rotary evaporation. Water (30 mL) was added, and the mixture was extracted with EA (100 mL) three times. The organic phase was washed with saturated brine (50 mL), dried over anhydrous sodium sulfate, concentrated to dryness by rotary evaporation, and then purified by silica gel column chromatography (0.2% FA in EA/PE = 55%/45%) to give a racemate of the product 96L (35 mg, yield: 51%). LCMS: [M+H]⁺ = 641.7. Step 12: LiOH·H₂O (6 mg, 0.02 mmol) was added to a solution of the racemate of compound 96L (30 mg, 0.05 mmol) in THF/MeOH/H₂O (3/1/1, 2 mL), and the mixture was stirred at room temperature for 1 h. The pH of the reaction solution was adjusted to about 5 with 3 M HCl, and the mixture was concentrated to dryness by rotary evaporation and purified through a preparative silica gel plate (DCM/MeOH = 8:1) to give a racemate of 96M (30 mg, yield: 100%) as a white solid. LCMS [M+H]⁺ = 627.5.

Step 13: DIEA (14.42 mg, 0.11 mmol) was added to a solution of the racemate of compound 96M (28 mg, 0.04 mmol), DMA (4.03 mg, 0.09 mmol), and HATU (33.99 mg, 0.09 mmol) in DMF (1 mL), and the mixture was stirred at room temperature for 1 h. The reaction solution was extracted with water and ethyl acetate. The organic phase was washed with saturated brine, dried over anhydrous sodium sulfate, filtered, and concentrated, and the crude product was then purified through a preparative silica gel plate (DCM/MeOH = 14/1) to give a racemate of 96N (30 mg, yield: 96.15%) as a white solid. LCMS: [M+H]= 654.6.

Step 14: BH₃·DMS (60 µL, 0.12 mmol) was added to a solution of the racemate of compound 96N (26 mg, 0.04 mmol) in THF (1 mL), and the mixture was stirred at room temperature for 2 h. Methanol was added to the reaction solution, and the mixture was stirred for another 2 h. The reaction solution was purified by normal-phase column chromatography (PE/EA = 0-100%) to give a racemate of 96Q (7 mg, yield: 27.48%) as a white solid. LCMS: [M+H]⁺ = 640.6.

Step 15: 6 M HCl (1 mL) was added to a solution of the racemate of compound 96Q (7 mg, 0.01 mmol) in THF (1 mL), and the mixture was stirred at room temperature for 1 h. The reaction solution was concentrated to dryness by rotary evaporation, and the resulting mixture was slurried with diethyl ether three times to give a racemate of compound 96 (5 mg, yield: 84.7%), and the racemate was further resolved on a chiral column to give compound 96. LCMS: [M+H]⁺ = 540.2.

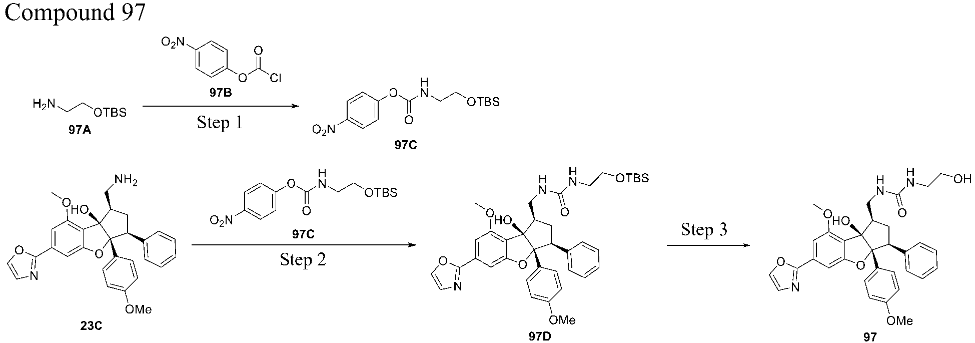

Step 1: The racemate of compound 97A (50 mg, 0.285 mmol) was dissolved in DCM (8 mL), and compound 97B (86 mg, 0.428 mmol) and TEA (87 mg, 0.86 mmol) were added. Under a nitrogen atmosphere, the mixture was stirred at 25 °C for 3 h. The reaction solution was concentrated under reduced pressure and purified by normal-phase column chromatography (PE:EA = 2:1) to give a racemate of 97C (60 mg, yield: 66%) as a white solid. LCMS [M+H-56]⁺ = 341.3.

Step 2: The racemate of compound 23C (10.0 mg, 0.021 mmol) was dissolved in THF (1.5 mL), and 97C (8.4 mg, 0.025 mmol) and TEA (4.1 mg, 0.041 mmol) were added. Under a nitrogen atmosphere, the mixture was stirred at 25 °C for 2 h. The reaction solution was concentrated under reduced pressure and purified by reversed-phase column chromatography (ACN:0.1% FA-H₂O) to give a racemate of 97D (10 mg, yield: 72%) as a white solid. LCMS [M+H-C₄H₈]⁺ = 668.2. Step 3: The racemate of compound 97D (9.0 mg, 0.013 mmol) was dissolved in THF (0.5 mL), and formic acid (0.5 mL) was added. Under a nitrogen atmosphere, the mixture was stirred at 25 °C for 1 h. The reaction solution was concentrated under reduced pressure and purified by reversed-phase column chromatography (ACN:0.1% FA-H₂O) to give a racemate of 97 (2.6 mg, yield: 35%) as a white solid, and the racemate was further resolved on a chiral column to give compound 97. LCMS [M+H]⁺ = 572.5.

¹H NMR (400 MHz, DMSO) δ 8.25 (d, *J* = 0.7 Hz, 1H), 7.41 (d, *J* = 0.7 Hz, 1H), 7.20 (dd, *J* = 11.3, 1.1 Hz, 2H), 7.16-7.00 (m, 5H), 6.90 (d, *J* = 6.8 Hz, 2H), 6.68 (d, *J* = 9.0 Hz, 2H), 6.14 (t, *J* = 5.6 Hz, 1H), 5.97 (t, *J* = 5.8 Hz, 1H), 5.33 (s, 1H), 4.69 (t, *J* = 5.1 Hz, 1H), 3.93 (s, 3H), 3.73-3.61 (m, 4H), 3.50 (dt, *J* = 13.1, 6.5 Hz, 1H), 3.42 (dd, *J* = 10.8, 5.6 Hz, 2H), 3.37 (d, *J* = 6.3 Hz, 1H), 3.12 (qd, *J* = 5.9, 2.0 Hz, 2H), 2.82-2.71 (m, 1H), 2.23-2.00 (m, 2H).

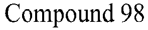

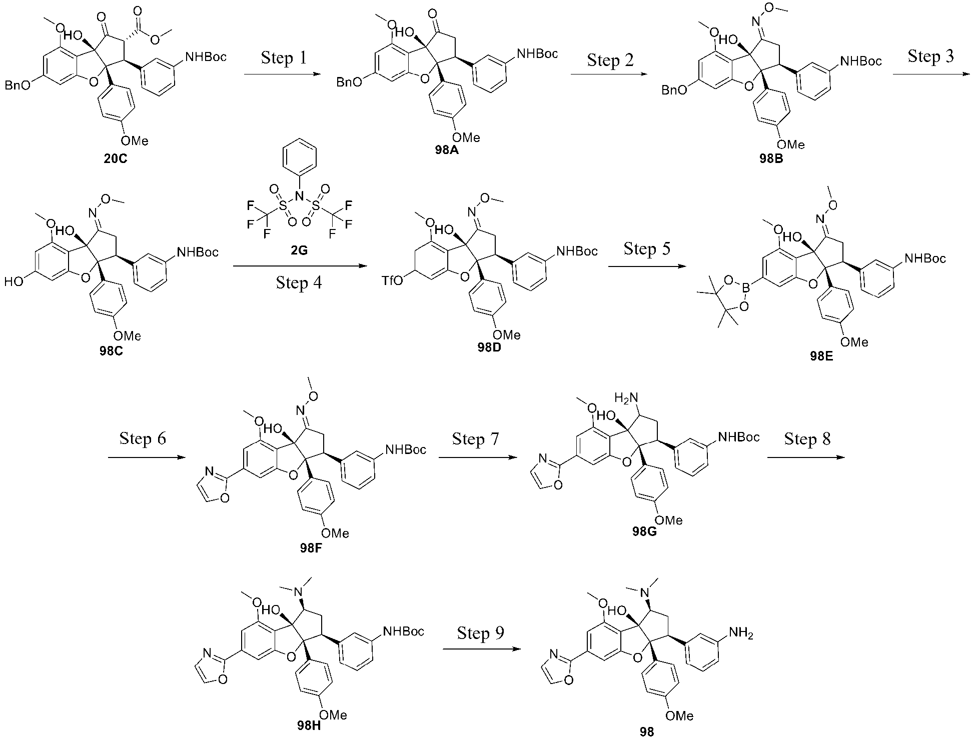

Step 1: DMAP (268 mg, 2.2 mmol) was added to a solution of the racemate of compound 20C (5 g, 7.33 mmol) in H₂O/toluene (10/50 mL), and the mixture was stirred at 60 °C for 1 h. The reaction solution was concentrated to dryness by rotary evaporation and extracted with water and ethyl acetate. The organic phases were combined, washed with saturated brine, dried over anhydrous sodium sulfate, filtered, concentrated, and then purified by normal-phase column chromatography (A: PE, B: EA) to give a racemate of 98A (4 g, yield: 87.45%) as a yellow solid. LCMS: [M+Na]⁺ = 646.6.

Step 2: A solution of the racemate of compound 98A (200 mg, 0.32 mmol) and O-methylhydroxylamine hydrochloride (134 mg, 1.61 mmol) in EtOH/pyridine (1/1 mmL) was heated to 60 °C and stirred for 2 h. The reaction solution was concentrated to dryness by rotary evaporation and purified by normal-phase column chromatography (PE, EA) to give a racemate of 98B (120 mg, yield: 57.4%) as a white solid. LCMS: [M+Na]⁺ = 675.7.

Step 3: Pd(OH)₂/C (300 mg) was added to a solution of the racemate of compound 98B (600 mg, 0.92 mmol) in AcOH (10 mL), and the mixture was purged with hydrogen three times and stirred at room temperature for 1 h. The reaction solution was filtered, and the filtrate was directly concentrated to dryness by rotary evaporation to give a racemate of compound 98C (600 mg, crude product, yield not calculated), and the product was directly used in the next step. LCMS: [M+H]⁺ = 563.6.

Step 4: K₂CO₃ (674.96 mg, 1.78 mmol) was added to a solution of the racemate of compound 98C (500 mg, 0.89 mmol) and compound 2G (317.05 mg, 0.89 mmol) in DMF, and the mixture was left to react at 60 °C for 0.5 h. The reaction solution was concentrated to dryness by rotary evaporation and extracted with water and ethyl acetate. The organic phases were combined, washed with saturated brine, dried over anhydrous sodium sulfate, filtered, concentrated, and then purified by normal-phase column chromatography (PE:EA) to give a racemate of 98D (330 mg, yield: 47.55%) as a yellow solid. LCMS: [M+H]= 695.6.

Step 5: Bis(pinacolato)diboron (351.27 mg, 1.38 mmol), Pd(dppf)Cl₂·DCM (75.25 mg, 0.09 mmol), and KOAc (173.63 mg, 1.84 mmol) were added to a solution of the racemate of compound 98D (320 mg, 0.46 mmol) in 1,4-dioxane (5 mL), and the mixture was stirred at 85 °C for 5 h. The reaction solution was filtered, and the filtrate was purified by normal-phase column chromatography (PE/EA = 1/1) to give a racemate of 98E (300 mg, yield: 96.77%) as a yellow solid. LCMS [M+H-H₂O]⁺ = 655.2.

Step 6: 2-Bromooxazole (231.08 mg, 1.56 mmol), Pd(dppf)Cl₂ (25.49 mg, 0.03 mmol), and Na₂CO₃ (66.21 mg, 0.62 mmol) were added to a mixed solution of the racemate of compound 98E (210 mg, 0.31 mmol) in 1,4-dioxane (10 mL) and H₂O (2 mL), and the mixture was left to react in a microwave reactor at 135 °C for 2.5 h. The reaction solution was filtered, and the filtrate was purified by normal-phase column chromatography (PE/EA = 1/1) to give a racemate of 98F (90 mg, yield: 50.33%) as a yellow solid. LCMS [M+H]⁺ = 614.6.

Step 7: Raney Ni (30 mg) was added to a solution of the racemate of compound 98F (70 mg, 0.11 mmol) in AcOH (5 mL), and the mixture was purged with hydrogen three times and stirred at room temperature for 1 h. The reaction solution was filtered, and the filtrate was concentrated to give a racemate of 98G (60 mg, crude product, yield not calculated) as a yellow solid, and the product was directly used in the next step. LCMS: [M+H]+ = 600.6.

Step 8: An aqueous formaldehyde solution (1 mL) was added to a solution of the racemate of compound 98G (20 mg, 0.03 mmol) in DMF (1 mL), and the mixture was stirred at room temperature for 1 h. Na(AcO)₃BH (36.23 mg, 0.17 mmol) was added to the reaction solution, and the mixture was stirred for another 1 h. The reaction solution was concentrated to dryness by rotary evaporation and purified by normal-phase column chromatography (PE, EA) to give a racemate of 98H (15 mg, yield: 71.4%) as a white solid. LCMS [M+H]⁺ = 614.6.

Step 9: A solution of HCl in 1,4-dioxane (1 mL) was added to a solution of the racemate of compound 98H (10 mg, 0.02 mmol) in DCM (1 mL), and the mixture was left to react at room temperature for 1 h. The reaction solution was purified by reversed-phase column chromatography (H₂O/ACN) to give a racemate of 98 (1.1 mg, yield: 13.75%) as a white solid, and the racemate was further resolved on a chiral column to give compound 98.

LCMS [M+H]⁺ = 514.2

¹H NMR (400 MHz, DMSO) δ 8.23 (s, 1H), 7.39 (s, 1H), 7.06 (dd, *J* = 29.7, 11.4 Hz, 4H), 6.74 (t, *J* = 7.9 Hz, 1H), 6.58 (d, *J* = 8.9 Hz, 2H), 6.39 (s, 1H), 6.25 (d, *J* = 7.7 Hz, 2H), 5.08 (s, 1H), 4.82 (s, 2H), 3.83 (s, 3H), 3.71 (s, 1H), 3.61 (s, 3H), 2.19 (s, 6H), 1.20 (d, *J* = 31.1 Hz, 2H).

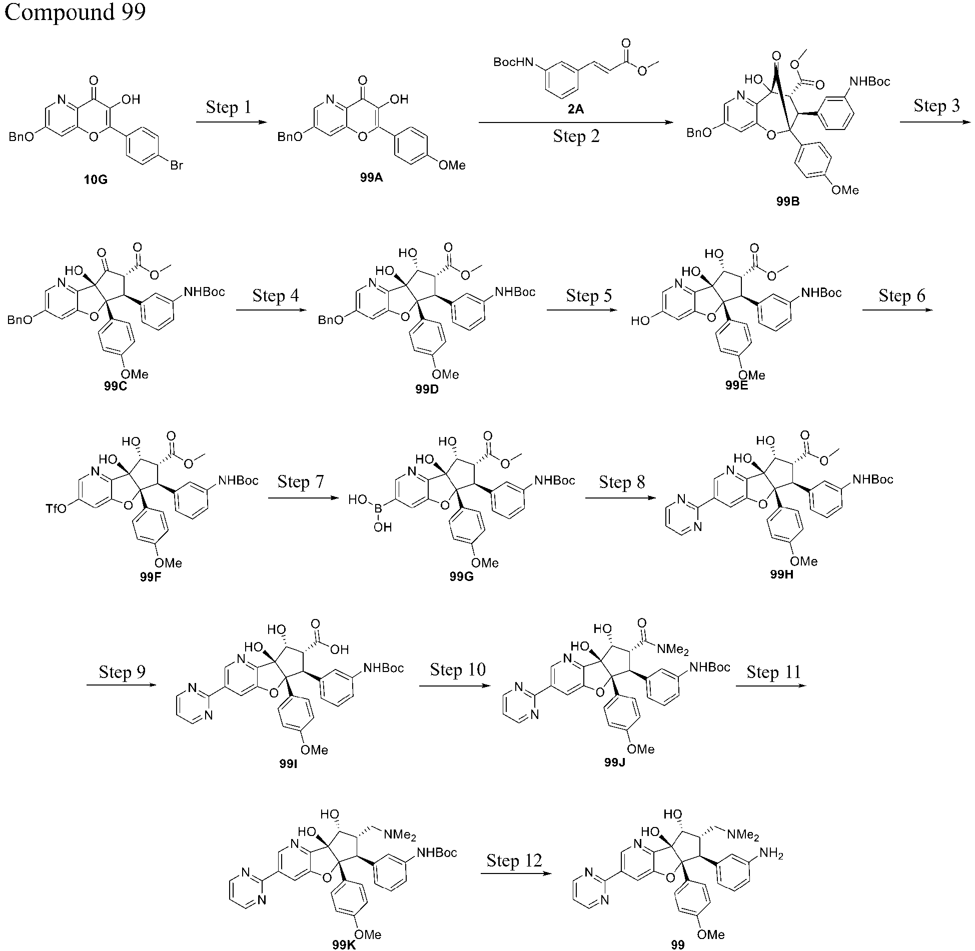

Step 1: At room temperature, tBuXPhos Pd G3 (225.0 mg, 0.28 mmol) and Cs₂CO₃ (6.936 g, 21.28 mmol) were added to a solution of compound 10G (6.0 g, 14.18 mmol) in MeOH (45 mL) and toluene (90 mL). The mixture was purged with nitrogen 3 times and left to react at 85 °C for 5 h. The reaction solution was filtered and purified by normal-phase column chromatography to give 99A (2.9 g, yield: 54.5%) as a yellow solid. LC-MS: [M+H]⁺ = 376.3.

Steps 2-4: A racemate of compound 99D was synthesized according to a synthesis method similar to that in steps 9-11 for compound 96. LCMS: [M+H]⁺ = 655.6.

Step 5: Pd/C (10%, 0.2 g) and AcOH (0.22 g, 3.67 mmol) were added to a solution of the racemate of compound 99D (0.8 g, 1.22 mmol) in MeOH (10 mL), and the mixture was purged with hydrogen three times and stirred at room temperature for 12 h. The reaction solution was filtered, and the filter cake was rinsed with DCM/MeOH (10:1). The filtrate was concentrated to give a racemate of 99E (0.5 g, yield: 72.5%) as a white solid. LCMS: [M+H]⁺ = 565.2.

Step 6: Compound 2G (316.49 mg, 0.89 mmol) and K₂CO₃ (244.68 mg, 1.77 mmol) were added to a solution of the racemate of compound 99E (0.5 g, 0.89 mmol) in DMF (7 mL), and the mixture was stirred at 60 °C for 2 h. The reaction solution was extracted with water and ethyl acetate, dried, and concentrated. The resulting crude product was purified by normal-phase column chromatography to give a racemate of 99F (0.5 g, yield: 81.0%) as a white solid. LCMS: [M+H]⁺ = 697.5

Step 7: Bis(pinacolato)diboron (545.26 mg, 2.16 mmol), Pd(dppf)Cl₂ (117.24 mg, 0.14 mmol), and KOAc (270.11 mg, 2.87 mmol) were added to a solution of the racemate of compound 99F (0.5 g, 0.72 mmol) in 1,4-dioxane (10 mL). Under a nitrogen atmosphere, the mixture was stirred at 85 °C for 24 h. The reaction solution was filtered, and the filter cake was washed with ethyl acetate. The filtrate was concentrated and purified by normal-phase column chromatography to give a racemate of 99G (0.2 g, yield: 47.0%) as a white solid. LCMS: [M+H]⁺ = 593.5.

Step 8: 2-Bromopyrimidine (63.6 mg, 0.4 mmol), Pd(PPh₃)₄ (34.65 mg, 0.03 mmol), and Na₂CO₃ (71.44 mg, 0.67 mmol) were added to a solution of the racemate of compound 99G (0.2 g, 0.337 mmol) in ACN (1 mL) and H₂O (0.25 mL). Nitrogen was bubbled through the mixture for 1 min, and the mixture was stirred at 110 °C for 0.5 h. The reaction solution was filtered, and the filter cake was washed with ethyl acetate. The filtrate was concentrated and purified by normal-phase column chromatography to give a racemate of 99H (0.1 g, yield: 47.2%) as a white solid. LCMS: [M+H]⁺ = 627.6.

Step 9: LiOH·H₂O (20.03 mg, 0.477 mmol) was added to a solution of the racemate of compound 99H (0.1 g, 0.159 mmol) in THF/H₂O/MeOH (1 mL/0.25 mL/0.25 mL), and the mixture was stirred at 40 °C for 3 h. The pH of the reaction solution was adjusted to 5 with 1 M HCl. After concentration, the crude product was purified by normal-phase column chromatography to give a racemate of 99I (90.0 mg, yield: 92.0%) as a white solid. LCMS: [M+H]⁺ = 613.5

Step 10: Dimethylamine (9.9 mg, 0.22 mmol), HATU (64.6 mg, 0.17 mmol), HOAT (23.12 mg, 0.17 mmol), and TMP (53.2 mg, 0.44 mmol) were added to a solution of the racemate of compound 99I (90.0 mg, 0.147 mmol) in DMF (1 mL), and the mixture was stirred at room temperature for 2 h. The reaction solution was purified by reversed-phase column chromatography to give a racemate of 99J (70.0 mg, yield: 74.4%) as a white solid. LCMS: [M+H]⁺ = 640.6.

Step 11: Under a nitrogen atmosphere, DIBAL-H (0.36 mg, 0.36 mmol) was slowly added dropwise to a solution of the racemate of compound 99J (60.0 mg, 0.09 mmol) in THF (0.5 mL) at 0 °C, and the mixture was stirred at room temperature for 2 h. The reaction solution was quenched with water and purified by reversed-phase column chromatography to give a racemate of 99K (4.0 mg, yield: 6.8%) as a white solid. LCMS: [M+H]⁺ = 626.6.

Step 12: 6 N HCl (0.5 mL) was added dropwise to a solution of the racemate of compound 99K (4.0 mg, 0.01 mmol) in THF (0.5 mL), and the mixture was stirred at room temperature for 2 h. The reaction solution was purified through a preparative silica gel plate to give a racemate of 99 (0.8 mg, yield: 23.8%) as a white solid, and the racemate was further resolved on a chiral column to give compound 99. LCMS: [M+H]⁺ = 526.2.

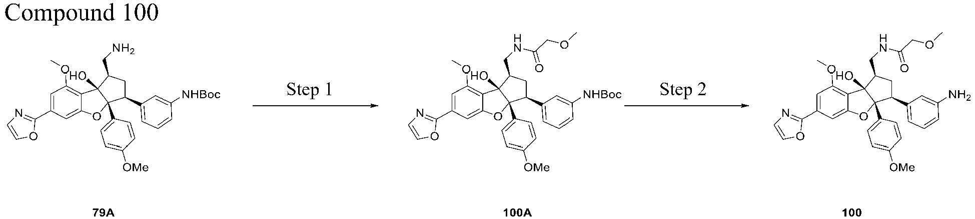

Step 1: Methoxyacetic acid (5 mg, 0.051 mmol), HATU (7 mg, 0.018 mmol), and TMP (4 mg, 0.034 mmol) were added to a mixture of the racemate of compound 79A (10 mg, 0.017 mmol) in DMF (1 mL), and the mixture was stirred at 20 °C for 2 h. The reaction solution was purified by reversed-phase column chromatography (MeCN/H₂O/FA) to give a racemate of 100A (8 mg, yield: 73%) as a white solid. LCMS [M+H-H₂O]⁺ = 654.2.

Step 2: The racemate of compound 100A (8 mg, 0.012 mmol) was dissolved in a mixed solution of 6 M HCl (1 mL) and THF (1 mL), and the mixture was stirred at 25 °C for 5 h. The reaction solution was purified by reversed-phase column chromatography (MeCN/FA/H₂O) to give a racemate of 100 (3 mg, yield: 43%) as a white solid, and the racemate was further resolved on a chiral column to give compound 100.

LCMS [M+H-H₂O]⁺ = 554.0.

¹H NMR (400 MHz, DMSO) δ 8.25 (s, 1H), 7.65 (s, 1H), 7.41 (s, 1H), 7.21 (d, *J* = 3.0 Hz, 2H), 7.17 (s, 1H), 7.15 (s, 1H), 6.70 (d, *J* = 8.9 Hz, 3H), 6.31 (s, 1H), 6.26 (s, 1H), 6.13 (s, 1H), 5.33 (d, *J* = 4.8 Hz, 2H), 3.94 (s, 3H), 3.89 (d, *J* = 4.8 Hz, 2H), 3.64-3.66 (m, 4H), 3.38 (s, 3H), 2.02-1.97 (m, 3H).

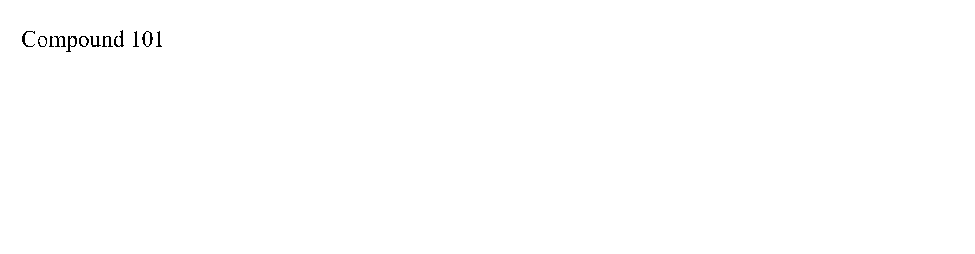

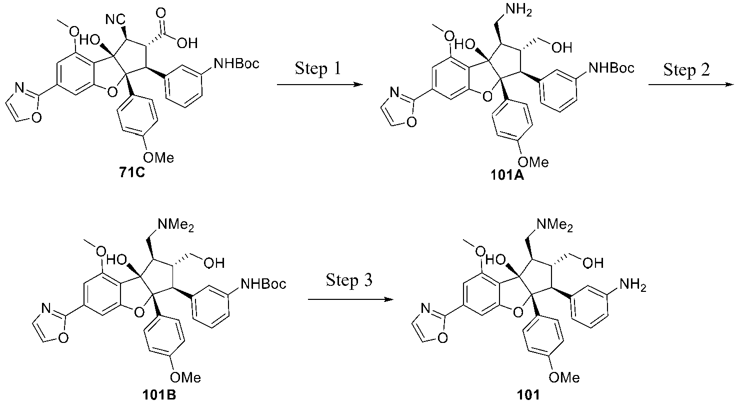

Step 1: BH₃·THF (1 M, 2 mL) was added to a solution of the starting material, the racemate of 71C (100 mg, 0.156 mmol), in THF (2 mL) at 0 °C, and the mixture was left to react at 0 °C to 20 °C for 4 h. MeOH (2 mL) was slowly added dropwise to the reaction solution, and the mixture was stirred at room temperature for another 1 h. The reaction solution was concentrated to give 101A (100 mg, crude product, yield not calculated), and the product was directly used in the next step. LCMS [M+H]⁺ = 630.6.

Step 2: Pd/C (100 mg) and an aqueous formaldehyde solution (3 mL, 37%) were added to a solution of the racemate of compound 101A (100 mg, 0.156 mmol) in MeOH (3 mL), and the mixture was purged with hydrogen three times and left to react at 20 °C for 8 h. The reaction solution was filtered to remove palladium on carbon and purified by reversed-phase column chromatography (MeCN/0.1% FA/H₂O) to give a racemate of 101B (65 mg, yield: 63%) as a yellow solid. LCMS [M+H]⁺ = 658.2.

Step 3: A solution of HCl in 1,4-dioxane (4 N, 1 mL) was added to the racemate of compound 101B (65 mg, 0.099 mmol) in DCM (1 mL), and the mixture was left to react at 20 °C for 2 h. The reaction solution was concentrated and slurried with EA twice to give a racemate of 101 (45 mg, yield: 69%) as a yellow solid, and the racemate was further resolved on a chiral column to give compound 101.

LCMS [M+H]⁺ = 658.2

1H NMR (400 MHz, DMSO) δ 10.34 (s, 2H), 8.89 (s, 1H), 8.29 (s, 1H), 7.44 (s, 1H), 7.30-7.24 (m, 2H), 7.20-7.10 (m, 4H), 7.03 (s, 1H), 6.74 (d, J = 8.9 Hz, 2H), 6.66 (d, J = 7.2 Hz, 1H), 6.02 (s, 1H), 4.01 (s, 3H), 3.69 (s, 3H), 3.54-3.43 (m, 3H), 3.28-3.14 (m, 4H), 3.10 (d, J = 4.7 Hz, 3H), 3.00 (d, J = 4.6 Hz, 3H), 2.72-2.59 (m, 1H), 1.23 (s, 1H).

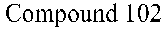

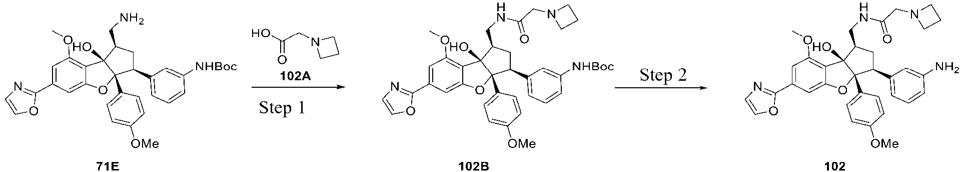

Step 1: Compound 102A (6 mg, 0.051 mmol), HATU (7 mg, 0.018 mmol), and TMP (4 mg, 0.034 mmol) were added to a solution of the racemate of compound 71E (10 mg, 0.017 mmol) in DMF (1 mL), and the mixture was stirred at 20 °C for 2 h. The reaction solution was purified by reversed-phase column chromatography (MeCN/H₂O/FA) to give a racemate of 102B (7 mg, yield: 58%) as a white solid. LCMS [M+H]⁺ = 697.2.

Step 2: The racemate of compound 102B (7 mg, 0.01 mmol) was dissolved in a mixed solution of 6 M HCl (1 mL) and THF (1 mL), and the mixture was stirred at 25 °C for 5 h. The reaction solution was purified by reversed-phase column chromatography (MeCN/FA/H₂O) to give a racemate of 102 (3 mg, yield: 50%) as a white solid, and the racemate was further resolved on a chiral column to give compound 102.

LCMS [M+H]⁺ = 597.0.

¹H NMR (400 MHz, DMSO) δ 8.26 (s, 2H), 7.68 (s, 1H), 7.42 (s, 1H), 7.21 (s, 2H), 7.16 (d, *J* = 8.8 Hz, 2H), 6.70 (dd, *J* = 8.0, 6.0 Hz, 3H), 6.30 (s, 1H), 6.23 (d, *J* = 7.8 Hz, 1H), 6.10 (d, *J* = 7.3 Hz, 1H), 5.29 (s, 1H), 3.99 (s, 3H), 3.66-3.60 (m, 4H), 3.24-3.20 (m, 4H), 3.08 (d, *J* = 5.0 Hz, 2H), 2.84 (s, 1H), 2.06-2.00 (m, 5H).

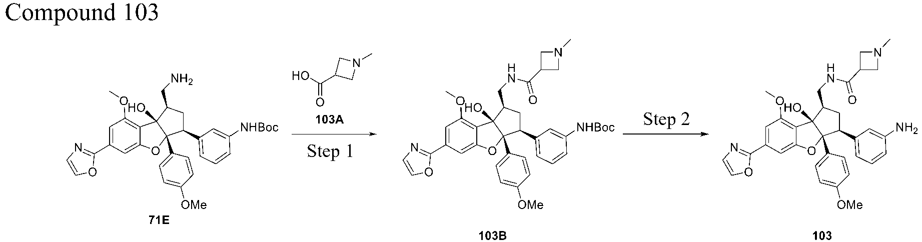

Step 1: Compound 103A (6 mg, 0.051 mmol), HATU (7 mg, 0.018 mmol), and TMP (4 mg, 0.034 mmol) were added to a solution of the racemate of compound 71E (10 mg, 0.017 mmol) in DMF (1 mL), and the mixture was stirred at 20 °C for 2 h. The reaction solution was purified by reversed-phase column chromatography (MeCN/H₂O/FA) to give a racemate of 103B (8 mg, yield: 67%) as a white solid. LCMS [M+H]⁺ = 697.2.

Step 2: The racemate of compound 103B (8 mg, 0.012 mmol) was dissolved in a mixed solution of 6 M HCl (1 mL) and THF (1 mL), and the mixture was stirred at 25 °C for 5 h. The reaction solution was purified by reversed-phase chromatography (MeCN/FA/H₂O) to give a racemate of 103 (3 mg, yield: 43%) as a white solid, and the racemate was further resolved on a chiral column to give compound 103.

LCMS [M+H]+ = 597.2.

¹H NMR (400 MHz, DMSO) δ 8.25 (s, 1H), 8.17 (s, 1H), 7.77 (s, 1H), 7.41 (s, 1H), 7.19 (d, *J* = 3.5 Hz, 2H), 7.15 (d, *J* = 8.9 Hz, 2H), 6.69-6.60 (m, 3H), 6.28 (s, 1H), 6.23 (d, *J* = 8.0 Hz, 1H), 6.08 (d, *J* = 7.7 Hz, 1H), 5.32 (s, 1H), 5.17 (s, 1H), 4.79 (s, 1H), 3.97-3.90 (m, 4H), 3.71 (s, 1H), 3.68-3.60 (m, 4H), 3.61 (s, 3H), 2.74 (s, 1H), 2.37 (s, 3H), 2.01-1.97 (m, 2H).

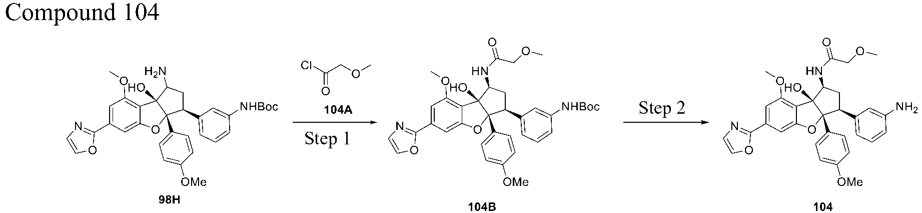

A racemate of compound 104 was synthesized according to a synthesis method similar to that for compound 100, and the racemate was further resolved on a chiral column to give compound 104. LCMS: [M+Na]⁺ = 580.2

¹H NMR (400 MHz, DMSO) δ 8.27 (s, 1 H), 8.07 (d, J = 7.4 Hz, 1 H), 7.43 (s, 1 H), 7.21 (d, J = 6.2 Hz, 2 H), 7.11 (d, J = 8.8 Hz, 2 H), 6.75 (t, J = 7.8 Hz, 1 H), 6.67 (d, J = 8.8 Hz, 2 H), 6.35 (s, 1 H), 6.30-6.23 (m, 2 H), 5.45 (s, 1 H), 5.32 (s, 1 H), 3.88 (s, 3 H), 3.81 (d, J = 4.3 Hz, 2 H), 3.65 (s, 3 H), 3.29 (s, 3 H), 2.03-1.99 (m, 2 H).

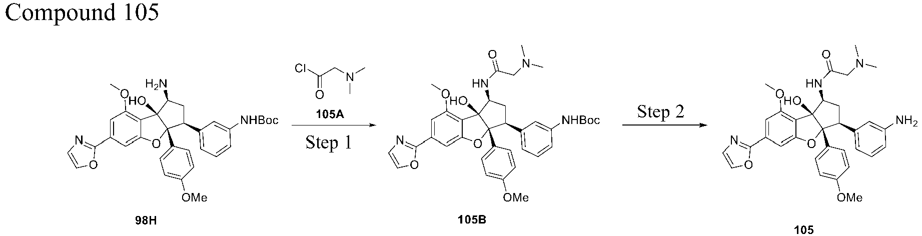

A racemate of compound 105 was synthesized according to a synthesis method similar to that for compound 100, and the racemate was further resolved on a chiral column to give compound 105. LCMS: [M+H]⁺ = 571.2.

¹H NMR (400 MHz, DMSO) δ 8.33 (s, 1 H), 8.26 (d, *J* = 6.5 Hz, 1 H), 8.16 (d, *J* = 7.1 Hz, 1 H), 7.42 (d, *J* = 5.0 Hz, 1 H), 7.21 (d, *J* = 8.1 Hz, 2 H), 7.10 (d, *J* = 8.8 Hz, 2 H), 6.76 (t, *J* = 7.8 Hz, 1 H), 6.66 (d, *J* = 8.9 Hz, 2 H), 6.36 (s, 1 H), 6.30 (d, *J* = 7.8 Hz, 1 H), 6.24 (d, *J* = 7.9 Hz, 1 H), 5.45 (s, 1 H), 4.76 (dd, *J* = 13.8, 6.9 Hz, 1 H), 3.88 (d, *J* = 5.8 Hz, 3 H), 3.64 (s, 3 H), 2.83 (s, 2 H), 2.13 (s, 6 H), 2.00 (d, *J* = 7.6 Hz, 1 H), 1.89 (dd, *J* = 12.8, 6.9 Hz, 1 H).

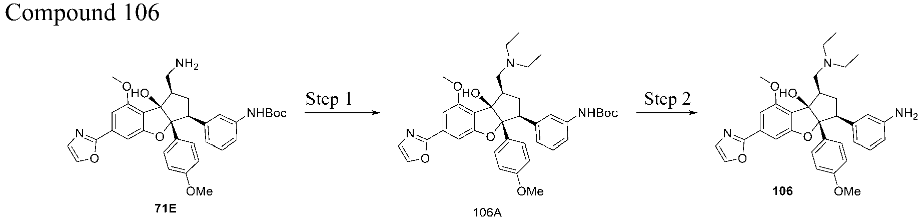

A racemate of compound 106 was synthesized according to a synthesis method similar to that for compound 79, and the racemate was further resolved on a chiral column to give compound 106. LCMS [M+H]⁺ = 557.2.

¹H NMR (400 MHz, DMSO) δ 8.34 (s, 1H), 8.25 (s, 1H), 7.41 (s, 1H), 7.17 (m 5H), 6.70 (d, *J* = 8.8 Hz, 3H), 6.34 (s, 1H), 6.23 (m, 1H), 6.17 (d, *J* = 7.6 Hz, 1H), 4.80 (s, 1H), 3.92 (s, 3H), 3.66 (s, 3H), 3.00 (dd, *J* = 12.8, 5.4 Hz, 1H), 2.91-2.83 (m, 1H), 2.63-2.58 (m, 2H), 2.23-1.97 (m, 4H), 1.01 (t, *J* = 7.2 Hz, 6H).

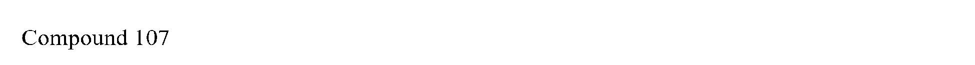

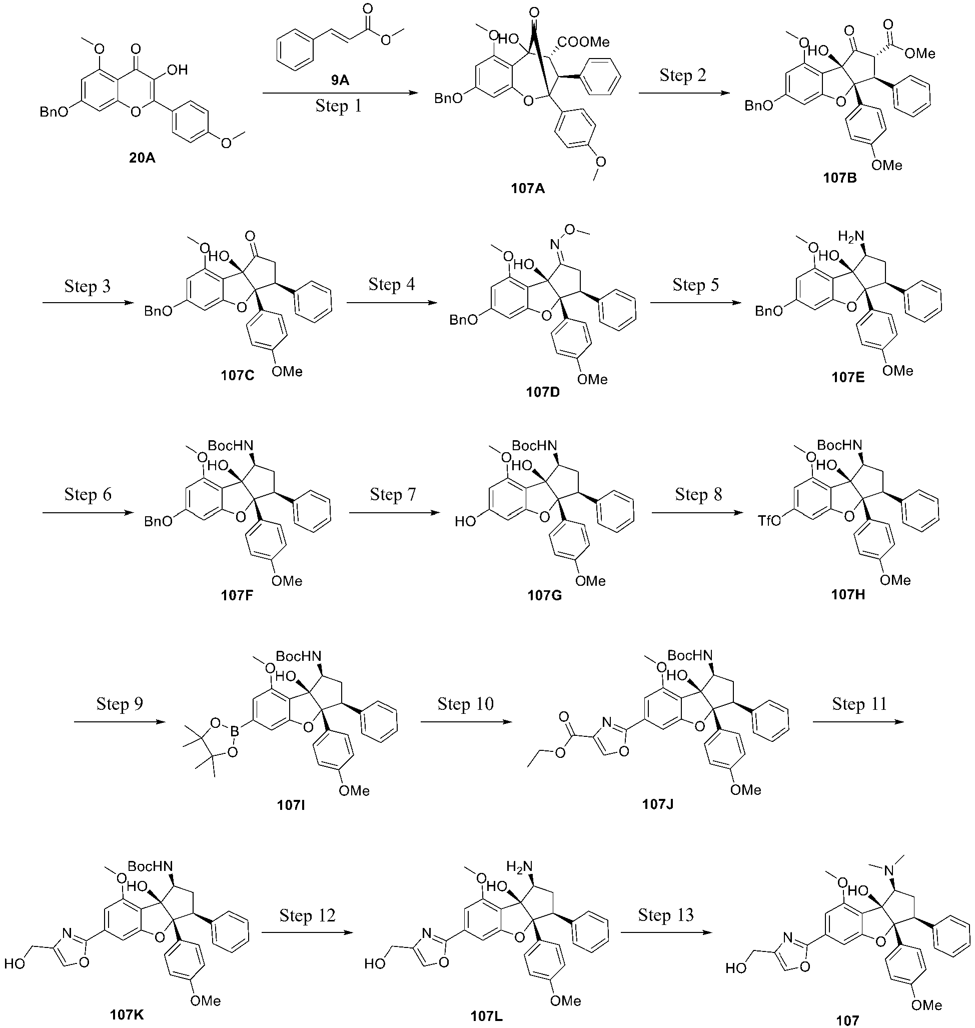

Steps 1 and 2: A racemate of compound 107B was synthesized according to a synthesis method similar to that in steps 9 and 10 for compound 96. LCMS [M+H]⁺ = 549.6.

Steps 3 and 4: A racemate of compound 107D was synthesized according to a synthesis method similar to that in steps 1 and 2 for compound 98.LCMS: [M+H-H₂O]⁺ = 520.5.

Step 5: 10 M BH3·DMS (1.9 mL) was added to a solution of the racemate of compound 107D (1 g, 1.86 mmol) in THF (20 mL), and the mixture was stirred at 65 °C for 6 h. The reaction solution was quenched with methanol and concentrated to give a racemate of compound 107E, and the product was directly used in the next step. LCMS [M+H]⁺ = 510.5.

Step 6: Boc₂O (1.01 g, 4.65 mmol) was added to a solution of the racemate of compound 107E (948 mg, 1.86 mmol) and TEA (752 mg, 7.4 mmol) in DCM (20 mL), and the mixture was stirred at room temperature for 2 h. The reaction solution was concentrated and extracted with water and ethyl acetate. The organic phases were combined, washed with saturated brine, dried over anhydrous sodium sulfate, filtered, concentrated, and then purified by normal-phase column chromatography (PE/EA) to givea racemate of 107F(350 mg, yield: 31%) as a white solid. LCMS [M-H₂O-t-Bu+H]⁺ = 536.5.

Steps 7-10: A racemate of compound 107Jwas synthesized according to a synthesis method similar to that in steps 3-6 for compound 98. LCMS [M+H-C₄H₈]⁺ = 587.4.

Step 11: A solution of the racemate of compound 107J (30 mg, 0.046 mmol) in THF was cooled to 0 °C, and a solution of LiAlH₄ (0.48 mL, 0.46 mmol, 1 N in THF) was slowly added dropwise to the reaction solution. The mixture was stirred at 0 °C for 2 h. Methanol was added to the reaction solution to quench the reaction, and the reaction solution was purified by normal-phase column chromatography (DCM/MeOH = 10/1) to give a racemate of107K(20 mg, yield: 71.4%) as a white solid. LCMS [M+H]⁺ = 601.2.

Step 12: 4 M HCl (0.3 mL) was added to a solution of the racemate of compound 107K (20 mg, 0.033 mmol) in THF (1 mL), and the mixture was stirred at room temperature for 2 h.The reaction solution was purified by reversed-phase column chromatography to give a racemate of 107L (15 mg, yield: 90.4%) as a white solid.LCMS [M+H]⁺ = 501.5.

Step 13: Two drops of acetic acid were added to a solution of the racemate of compound 107L (15 mg, 0.03 mmol) and an aqueous formaldehyde solution (30%, 0.3 mL) in DMF (2 mL), and the mixture was stirred at room temperature for 2 h.NaBH(AcO)₃ (19 mg, 0.09 mmol) was added to the reaction solution, and the mixture was stirred at room temperature for another 2 h. The reaction solution was purified by reversed-phase column chromatography (H₂O/ACN) to give a racemate of 107 (1 mg, yield: 13.1%) as a white solid, and the racemate was further resolved on a chiral column to give compound 107.

LCMS [M+H]⁺ = 529.2.

¹H NMR (400 MHz, DMSO) δ 8.05 (s, 1H), 7.21 (t, *J* = 6.8 Hz, 2H), 7.10 (dd, *J =* 9.5, 7.0 Hz, 5H), 7.00 (d, *J* = 6.8 Hz, 2H), 6.69 (t, *J* = 6.8 Hz, 2H), 5.31 (dd, *J* = 6.6, 4.5 Hz, 2H), 4.46 (d, *J* = 5.6 Hz, 2H), 3.94 (d, *J =* 12.0 Hz, 3H), 3.66 (s, 3H), 2.53 (s, 6H), 2.31-2.24 (m, 1H), 1.97 (s, 1H).

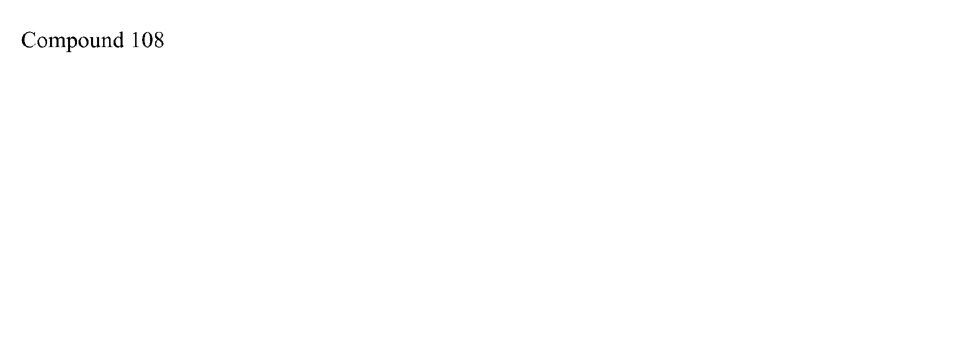

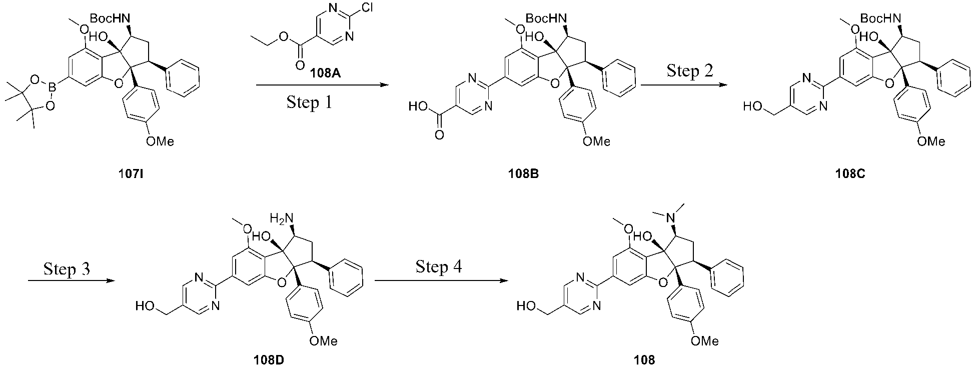

Step 1: The racemate of compound 107I (20.0 mg, 31.8 µmol), compound 108A (17.8 mg, 95.3 µmol), Pd(PPh₃)₄ (3.7 mg, 3.2 µmol), and K₂CO₃ (13.17 mg, 95.3 µmol) were dissolved in a mixed solution of 1,4-dioxane (1.5 mL) and H₂O (0.5 mL). Under a nitrogen atmosphere, the mixture was microwaved to 70 °C and left to react for 30 min. The reaction solution was purified by preparative HPLC (CH₃CN/H₂O, 0.1% FA) to give a racemate of 108B (15.0 mg, yield: 75.5%) as a white solid. LCMS: [M/2+H]⁺ = 626.5.

Step 2: The racemate of compound 108B (14.0 mg, 22.4 µmol) was dissolved in THF (1 mL), and LiAlH₄ (2 drops) was added at 0 °C. The mixture was stirred at 0 °C for 2 h. Water (1 drop), an aqueous NaOH solution (1 drop), and water (1 drop) were added. The resulting mixed solution was dried over anhydrous sodium sulfate, filtered, and concentrated to give a racemate of compound 108C (crude product), and the product was directly used in the next step. LCMS: [M+H]⁺ = 566.5.

Step 3: The racemate of compound 108C (crude product) was dissolved in DCM (1 mL), and TFA (1 mL) was added with stirring. The mixture was stirred at 20 °C for 1 h. The reaction solution was concentrated to dryness by rotary evaporation to give a racemate of compound 108D (crude product), and the product was directly used in the next step. LCMS: [M+H]⁺ = 512.5.

Step 4: The racemate of compound 108E (crude product) was dissolved in MeOH (1.5 mL) and formaldehyde (1.5 mL). After stirring, Pd/C (10.0 mg) was added, and the mixture was purged with hydrogen three times and stirred at 20 °C for 2 h. The reaction solution was filtered. The filtrate was concentrated and purified by preparative HPLC (CH₃CN/H₂O, 0.1% FA) to give a racemate of 108E (1.0 mg) as a white solid, and the racemate was further resolved on a chiral column to give compound 108. LCMS: [M+H]⁺ = 540.5.

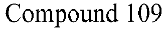

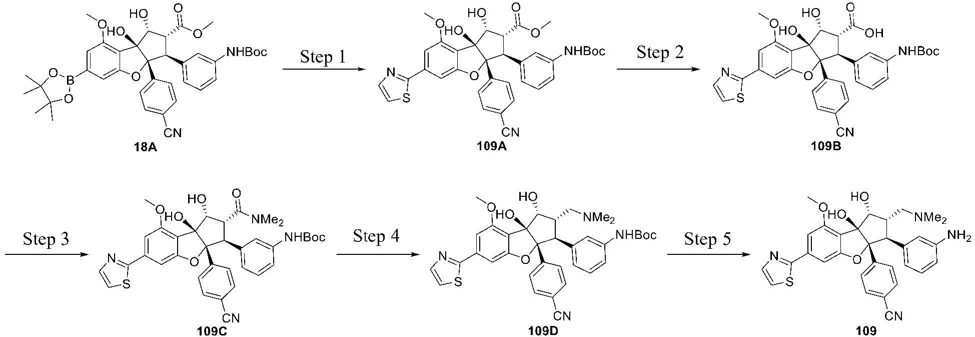

Step 1: The racemate of compound 18A (30.0 mg, 0.04 mmol) was dissolved in dioxane/water (1 mL/2 mL), and 2-bromothiazole (10.6 mg, 0.06 mmol), Na₂CO₃ (13.7 mg, 0.13 mmol), and Pd(dppf)Cl₂ (3.5 mg, 0.004 mmol) were added. The mixture was purged with nitrogen three times and stirred at 85 °C for 16 h. The reaction solution was concentrated and purified by silica gel column chromatography (DCM/MeOH) to give a racemate of compound 109A (20.0 mg, yield: 35%). LCMS [M+H]⁺ = 600.5.

Step 2: The racemate of compound 109A (20.0 mg, 0.03 mmol) and LiOH·H₂O (5.1 mg, 0.12 mmol) were dissolved in THF/H₂O/MeOH (1 mL/0.1 mL/0.1 mL), and the solution was stirred at 40 °C for 3 h. The pH of the reaction solution was adjusted to 5 with 3 M hydrochloric acid, and the mixture was purified by silica gel column chromatography (DCM/MeOH) to give a racemate of compound 109B (10 mg, yield: 52%). LCMS [M+H]⁺ = 642.7.

Step 3: The racemate of compound 109B (10.0 mg, 0.02 mmol), HATU (17.8 mg, 0.05 mmol), and DIEA (10.1 mg, 0.08 mmol) were added to DMF (1 mL), and the mixture was stirred at 25 °C for 10 min. DMA (0.04 mL, 0.08 mmol, 2 M in THF) was added, and the mixture was stirred at 25 °C for 1 h. The reaction solution was diluted with water and extracted with EA. The organic phase was washed with water and saturated brine, dried over anhydrous Na₂SO₄, filtered, concentrated, and then purified by silica gel column chromatography (DCM/MeOH) to give a racemate of compound 109C (8 mg, yield: 80%). LCMS [M+H]⁺ = 669.3.

Step 4: The racemate of compound 109C (8.0 mg, 0.01 mmol) and TFA (1.4 mg, 0.012 mmol) were added to THF (1 mL), and the mixture was stirred at 60 °C for 30 min. BH₃·DMS (0.04 mL, 0.036 mmol) was added, and the mixture was stirred at 25 °C for 2 h. The reaction solution was diluted with NaOH (aq), stirred at 25 °C for 30 min, concentrated, and then purified by silica gel column chromatography (DCM/MeOH) to give a racemate of compound 109D (6 mg, yield: 77%). LCMS: [M+H]⁺ = 655.7.

Step 5: The racemate of compound 109D (6.0 mg, 0.01 mmol) was mixed with THF (1 mL), and 6 M HCl (0.5 mL) was added at 0 °C. The mixture was stirred at 25 °C for 1 h. The reaction solution was purified by silica gel column chromatography (DCM/MeOH) to give a racemate of compound 109 (0.8 mg, yield: 15%), and the racemate was further resolved on a chiral column to give compound 109.

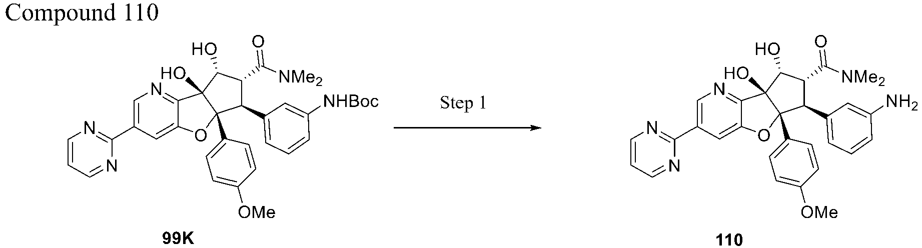

Step 1: 6 N HCl (0.5 mL) was added dropwise to a solution of the racemate of compound 99K (5.0 mg, 0.01 mmol) in THF (0.5 mL), and the mixture was stirred at room temperature for 2 h. The reaction solution was purified through a preparative silica gel plate to give 110 (3.0 mg, yield: 71.1%) as a white solid, and the product was further resolved on a chiral column to give compound 110.

LCMS: [M+H]⁺ = 540.2.

¹HNMR (400 MHz, DMSO-d₆) δ 9.18 (d, *J* = 1.3 Hz, 1H), 8.99 (d, *J* = 4.8 Hz, 2H), 8.26 (d, J= 1.3 Hz, 1H), 7.56 (t, *J* = 4.8 Hz, 1H), 7.22-6.96 (m, 6H), 6.66 (d, *J* = 8.8 Hz, 2H), 4.88 (d, *J=* 5.6 Hz, 1H), 4.55-4.51 (m, 1H), 4.27-4.22 (m, 1H), 3.62 (s, 3H), 3.39 (s, 1H), 3.28 (s, 3H), 2.78 (s, 3H), 1.77 (s, 1H).

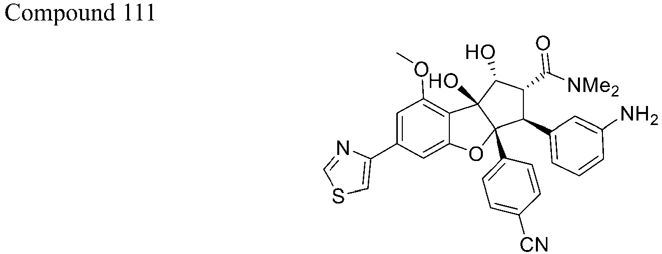

A racemate of compound 111 was synthesized according to a synthesis method similar to that for compound 18, and the racemate was further resolved on a chiral column to give compound 111. LCMS [M+H]⁺ = 569.2.

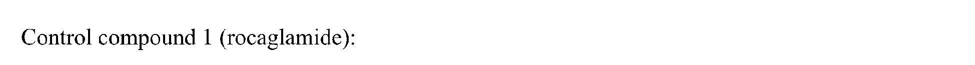

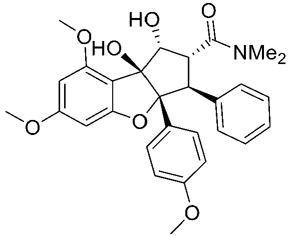

CAS: 84573-16-0, purchased from MedChemExpress LLC.

Santagata S, Mendillo ML, Tang YC, Subramanian A, Perley CC, Roche SP, Wong B, Narayan R, Kwon H, Koeva M, Amon A, Golub TR, Porco JA Jr, Whitesell L, Lindquist S. Tight coordination of protein translation and HSF1 activation supports the anabolic malignant state. Science. 2013 Jul 19;341(6143):1238303. doi: 10.1126/science.1238303.

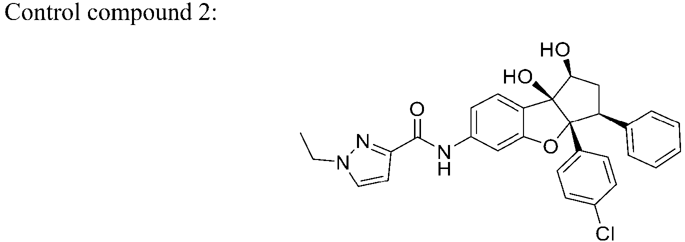

Synthesized with reference to Patent No. WO2005113529A2.

### Example 1.2: Synthesis of Linkers

### Example 1.2.1: Synthesis of Compound L1

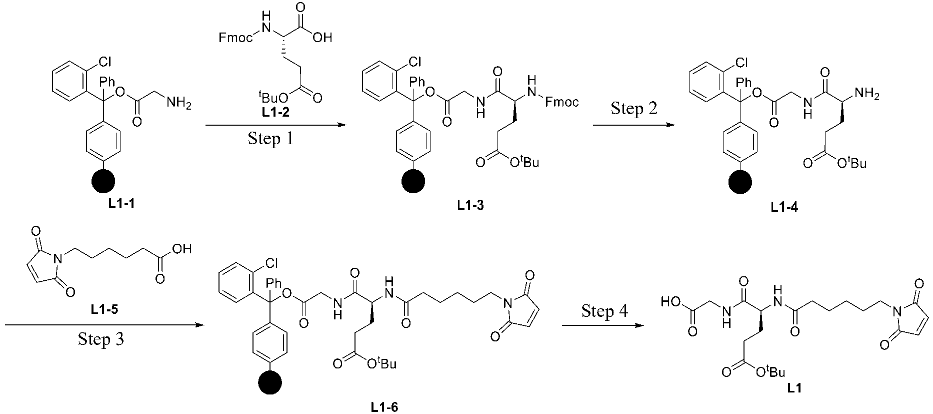

Step 1: DCM (100 mL) was added to solid 2-Cl-Gly resin L1-1 (10 g), and N₂ was bubbled through the mixture for 30 min. After filtration, the resin was washed with DMF (50 mL) three times. Amino acid L1-2 (5.26 g, 12.36 mmol), HATU (4.79 g, 12.36 mmol), and DMF (80 mL) were added to 10 g of the resin. N₂ was bubbled through the mixture, and DIEA (2.1 mL, 12.36 mmol) was then added. N₂ was bubbled through the mixture for 2 h. After filtration, the resin was washed with DMF (10 mL) 6 times to give resin L1-3.

Step 2: 20% piperidine-DMF (5 mL) was added to resin L1-3. After N₂ was bubbled through the mixture for 5 min, the mixture was filtered, and the resin was washed with DMF once. Then, 20% piperidine-DMF (5 mL) was added. After N₂ was bubbled through the mixture for 15 min, the mixture was filtered, and the resin was washed with DMF 6 times. 10 g of the resin was sequentially washed with DMF (50 mL × 3), DCM (50 mL × 3), and MTBE (50 mL × 3) and then dried to give resin L1-4.

Step 3: Compound L1-5 (2.61 g, 12.36 mmol), HATU (4.79 g, 12.36 mmol), and DMF (80 mL) were added to resin L1-4. N₂ was bubbled through the mixture, and DIEA (2.1 mL, 12.36 mmol) was then added. N₂ was bubbled through the mixture for 2 h. After filtration, the resin was washed with DMF (10 mL) 6 times to give resin L1-6.

Step 4: Resin L1-6 was immersed in a cleavage solution (60% DCM + 20% acetic acid + 20% trifluoroethanol). The mixture was stirred at -20 °C for 2 h and purified by reversed-phase silica gel column chromatography (ACN/H₂O = 1/3) to give 900 mg of compound L1.

LCMS [M+H]⁺ = 454.2.

¹H NMR (400 MHz, DMSO) δ 12.56 (s, 1H), 8.15 (t, J = 5.8 Hz, 1H), 7.95 (d, J = 8.2 Hz, 1H), 7.00 (s, 2H), 4.32-4.23 (m, 1H), 3.73 (qd, J = 17.5, 5.8 Hz, 2H), 3.37 (t, J = 7.1 Hz, 2H), 2.23 (t, J = 8.0 Hz, 2H), 2.10 (td, J = 7.3, 3.9 Hz, 2H), 1.87 (td, J = 13.6, 8.0 Hz, 1H), 1.69 (dq, J = 16.3, 8.3 Hz, 1H), 1.47 (dt, J = 14.9, 7.3 Hz, 4H), 1.38 (s, 9H), 1.24-1.13 (m, 2H).

The following compounds were synthesized with reference to the synthesis method for compound L1.

| No. | Structure | LCMS[M+H]⁺ |
|---|---|---|
| L1-A | 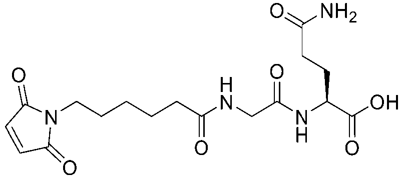 | 397.2 |
| L1-B | 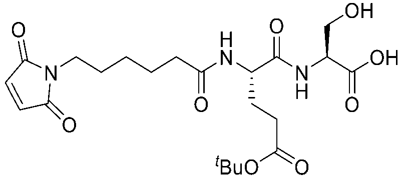 | 484.2 |
| L1-C | 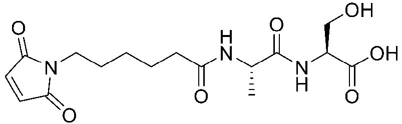 | 370.2 |
| L1-D | 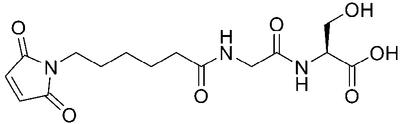 | 356.2 |
| L1-E | 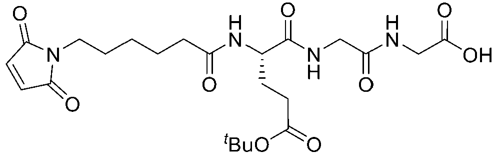 | 511.2 |
| L1-F | 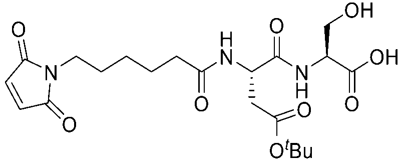 | 470.2 |
| L1-G | 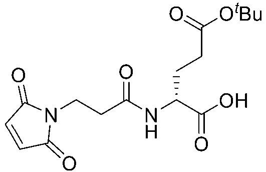 | 355.2 |
| L1-H | 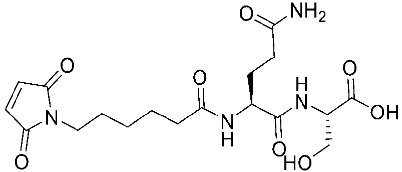 | 427.2 |
| L1-I | 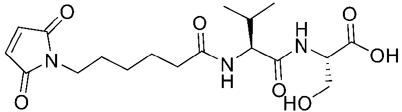 | 398.2 |
| L1-K | 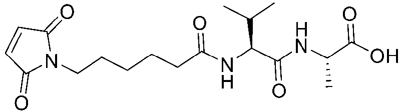 | 382.2 |
| L1-L | 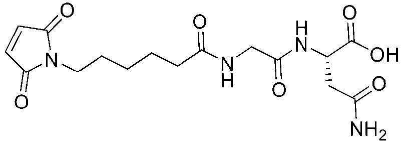 | 383.2 |
| L1-M | 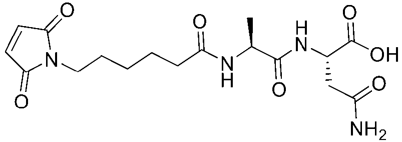 | 397.2 |

### Example 1.2.2: Synthesis of Compound L2

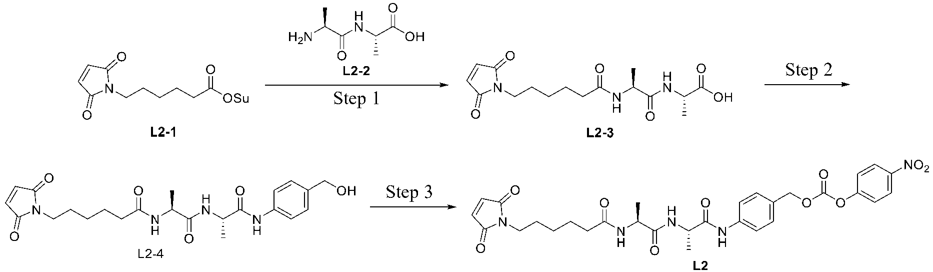

Step 1: NaHCO₃ (2.73 g, 32.44 mmol) and L2-1 (10 g, 32.44 mmol) were added to a mixed solution of L2-2 (4.33 g, 27.03 mmol) in acetone/H₂O (60 mL/60 mL). The mixed solution was stirred at room temperature for 16 h. After the reaction was complete, the reaction solution was concentrated, extracted with EA, washed with water and saturated brine, and dried over anhydrous sodium sulfate. The crude product was recrystallized from EA to give compound L2-3 (5.8 g, yield: 50.8%). LCMS [M+H]⁺ = 354.0.

¹H NMR (400 MHz, DMSO) δ 12.50 (s, 1H), 8.10 (d, J = 7.3 Hz, 1H), 7.92 (d, J = 7.7 Hz, 1H), 7.01 (s, 2H), 4.35-4.25 (m, 1H), 4.17 (t, J = 7.3 Hz, 1H), 3.37 (t, J = 7.1 Hz, 2H), 2.07 (t, J = 7.4 Hz, 2H), 1.53-1.40 (m, 4H), 1.26 (d, J = 7.3 Hz, 3H), 1.18 (dd, J = 15.0, 7.5 Hz, 5H).

Step 2: 4-Aminobenzyl alcohol (173 mg, 1.4 mmol) was dissolved in a mixed solution of DCM and MeOH (20 mL/10 mL), and EEDQ (690 mg, 2.8 mmol) and compound L2-3 (500 mg, 1.4 mmol) were then added to the solution. The mixture was stirred at room temperature for 16 h. LCMS analysis showed that the reaction had been complete. The reaction solution was concentrated to dryness by rotary evaporation and then extracted with EA three times, washed with saturated brine, and dried over anhydrous sodium sulfate. The organic phase was concentrated under reduced pressure and then purified by silica gel column chromatography (MeOH/DCM = 62%:38%) to give compound L2-4 (775 mg, yield: 85%).

LCMS [M+H]⁺ = 459.0.

¹H NMR (400 MHz, DMSO) δ 9.82 (s, 1H), 8.03 (dd, *J* = 19.5, 7.2 Hz, 2H), 7.56 (d, *J* = 8.4 Hz, 2H), 7.23 (d, *J =* 8.4 Hz, 2H), 7.00 (s, 2H), 5.11 (s, 1H), 4.42 (s, 2H), 4.40-4.33 (m, 1H), 4.25 (t, *J =* 7.1 Hz, 1H), 3.37 (d, *J* = 7.1 Hz, 2H), 2.10 (t, *J =* 7.3 Hz, 2H), 1.48 (dd, *J =* 14.8, 7.4 Hz, 4H), 1.30 (d, *J =* 7.1 Hz, 3H), 1.19 (d, *J =* 7.1 Hz, 5H).

Step 3: L2-4 (100 mg, 0.22 mmol) and bis(p-nitrophenyl) carbonate (200 mg, 0.66 mmol) were added to DMF (3 mL), and the mixture was then stirred at room temperature for 16 h. The resulting mixture was purified by reversed-phase column chromatography (ACN/H₂O = 30%-70%) to give L2 (50 mg, yield: 50%) as a white solid. LCMS [M+H]+ = 624.0.

The following compounds were synthesized with reference to the synthesis method for compound L2.

| No. | Structure | LCMS[M+H]⁺ |
|---|---|---|
| L2-A | 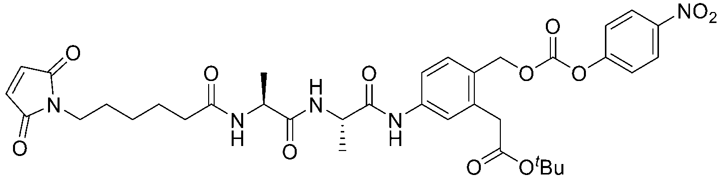 | 738.3 |
| L2-B | 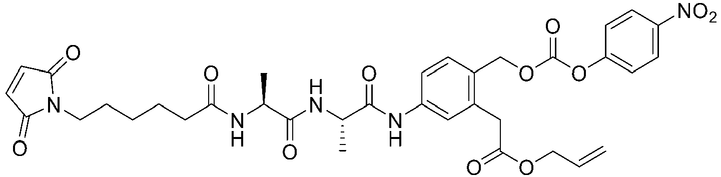 | 722.3 |

### Example 1.2.3: Synthesis of Compound L3

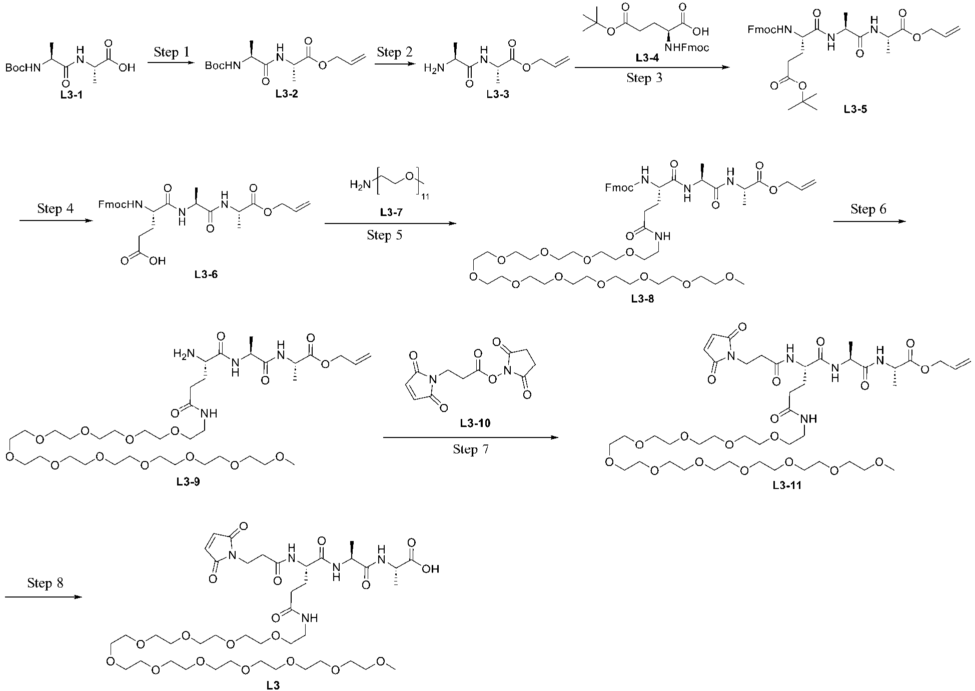

Step 1: DMF (200 mL), compound L3-1 (14.0 g, 53.79 mmol), and HATU (30.7 g, 80.68 mmol) were added to a 500 mL three-necked flask at 0 °C, and DIEA (20.9 g, 161.36 mmol) was added dropwise to the above reaction system. Allyl alcohol (9.4 g, 161.36 mmol) was then added dropwise. After the dropwise addition, the mixture was stirred at room temperature for 2 h. After the reaction was complete, the reaction solution was quenched with 500 mL of water, extracted with 600 mL of ethyl acetate, dried, concentrated, and purified by silica gel column chromatography (PE/EA) to give compound L3-2 (11.0 g, yield: 68%). LCMS: [M+H]⁺ = 301.2. Step 2: Compound L3-2 (15.0 g, 49.94 mmol) was dissolved in dichloromethane (100 mL) at 0 °C, and HCl (400 mmol, 100 mL, 4 N in 1,4-dioxane) was added dropwise. The mixture was stirred at room temperature for 2 h and concentrated to give compound L3-3 (15 g, crude product, yield not calculated). LCMS: [M+H]⁺ = 201.2.

Step 3: DMF (200 mL), compound L3-4 (21.2 g, 49.94 mmol), and HATU (19.0 g, 49.94 mmol) were added to a 500 mL three-necked flask at 0 °C, and DIEA (34.8 mL, 199.76 mmol) was added dropwise to the above reaction system. Compound L3-3 (11.8 g, 49.94 mmol) was then added to the above system, and the mixture was stirred at room temperature for 1 h. After the reaction was complete, the reaction solution was quenched with 400 mL of water, extracted with 400 mL of ethyl acetate, dried, concentrated, and purified by silica gel column chromatography (mobile phase: PE to EA) to give compound L3-5 (25.0 g, yield: 82%). LCMS: [M+H]⁺ = 608.5

Step 4: Compound L3-5 (20.0 g, 32.9 mmol) was dissolved in dichloromethane (100 mL) at 0 °C, and TFA (50 mL) was added dropwise. The mixture was stirred at room temperature for 2 h and concentrated to give L3-6 (20 g, crude product, yield not calculated). LCMS: [M+H]⁺ = 552.3. Step 5: DMF (200 mL), compound L3-6 (8.0 g, 14.50 mmol), and HATU (6.0 g, 15.82 mmol) were added to a 500 mL three-necked flask at 0 °C, and DIEA (6.9 mL, 39.55 mmol) was added dropwise to the above reaction system. Compound L3-7 (6.8 g, 13.18 mmol) was then added to the above system, and the mixture was stirred at room temperature for 1 h. After the reaction was complete, the reaction solution was quenched with 400 mL of water, extracted with 400 mL of ethyl acetate, dried, concentrated, and purified by silica gel column chromatography (mobile phase: PE to EA) to give compound L3-8 (10.0 g, yield: 66%). LCMS: [M+H]⁺ = 1050.

Step 6: Under a N₂ atmosphere, DMA (10 mL) was added to a solution of compound L3-8 (1.0 g, 1.0 mmol) in THF (10 mL). The mixture was stirred at room temperature for 1 h and concentrated to give compound L3-9 (crude, 1 g, yield: 95%) as a white solid.

Step 7: Under a N₂ atmosphere, DIEA (120 mg, 0.9 mmol) was added to a solution of compound L3-9 (500 mg, 0.6 mmol) and compound L3-10 (160 mg, 0.6 mol) in DMF (5 mL).The mixture was stirred at room temperature for 1 h and extracted with EtOAc and H₂O. The resulting organic phase was dried over anhydrous Na₂SO₄ and filtered, and the filtrate was concentrated under reduced pressure. The mixture was purified by reversed-phase column chromatography (H₂O/acetonitrile = 0-100%) (214 nm) to give compound L3-11 (210 mg, yield: 36%) as a white solid. LCMS: [M+H]⁺ = 978.8.

Step 8: Under a N₂ atmosphere, TEA (20 mg, 0.2 mmol), FA (10 mg, 0.2 mol), and Pd(PPh₃)₄ (6 mg, 0.004 mmol) were added to a solution of compound L3-11 (40 mg, 0.04 mmol) in THF (2 mL). The mixture was stirred at room temperature for 18 h and purified by reversed-phase column chromatography (H₂O:acetonitrile = 0-100%) (214 nm) to give compound L3 (20 mg, yield: 53%) as a white solid. LCMS: [M+H]⁺ = 938.9.

The following compounds were synthesized with reference to the synthesis method for compound L3.

| No. | Structure | LCMS[M+H]⁺ |
|---|---|---|
| L3-A | 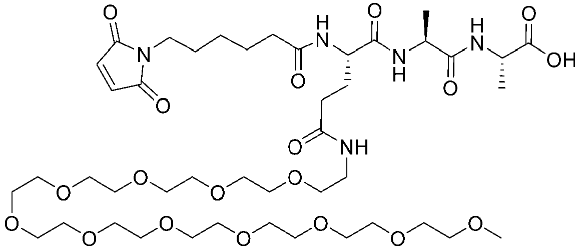 | 980.5 |
| L3-B | 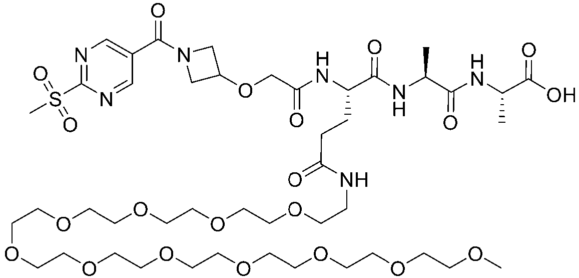 | 1084.5 |

### Example 1.2.4: Synthesis of Compound L4

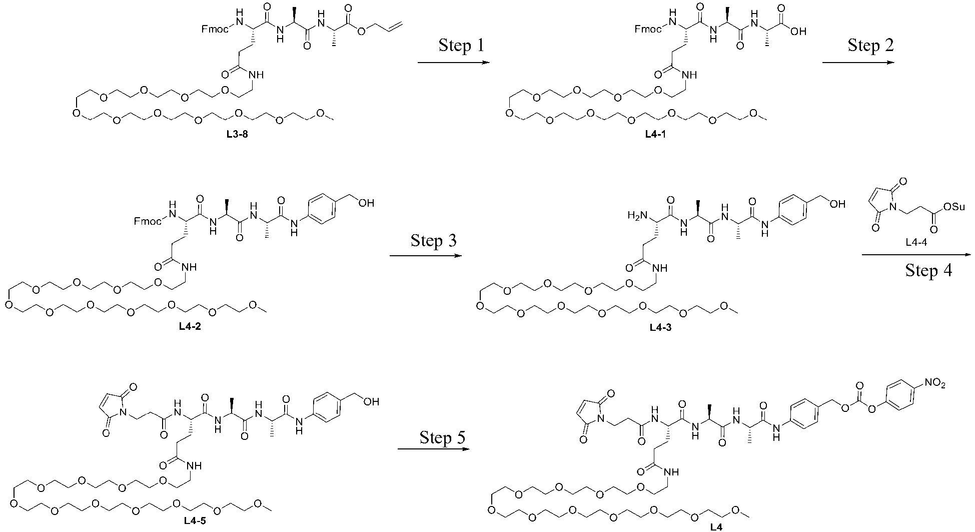

Step 1: Compound L3-8 (2 g, 1.9 mmol), PhSiH₃ (2.3 mL, 19 mol), and Pd(PPh₃)₄ (110 mg, 0.095 mmol) were dissolved in a mixed solution of THF (20 mL), DCM (20 mL), and DMF (2.5 mL), and the solution was stirred at room temperature for 1 h. The reaction solution was concentrated to dryness by rotary evaporation and purified by silica gel column chromatography (DCM:(THF:MeOH = 1:1)) to give L4-1 (1.42 g, yield: 74%) as a brown gel. LCMS: [M+H]⁺ = 1010.

¹H NMR (400 MHz, DMSO) δ 12.59 (s, 1H), 8.20-7.98 (m, 2H), 7.98-7.92 (m, 1H), 7.89 (d, *J =* 7.5 Hz, 2H), 7.73 (dd, *J* = 7.2, 4.5 Hz, 2H), 7.60-7.51 (m, 1H), 7.42 (t, *J* = 7.4 Hz, 2H), 7.33 (t, *J* = 7.4 Hz, 2H), 4.36-4.28 (m, 1H), 4.25 (td, *J =* 11.2, 5.5 Hz, 3H), 4.14 (dt, *J=* 14.1, 7.0 Hz, 1H), 3.98 (dd, *J* = 13.8, 7.7 Hz, 1H), 3.57-3.44 (m, 40H), 3.44-3.39 (m, 4H), 3.23 (s, 3H), 3.19 (dd, *J =* 11.7, 5.9 Hz, 2H), 2.17-2.12 (m, 1H), 1.89 (d, *J* = 13.4 Hz, 1H), 1.23 (ddd, *J=* 11.1, 7.1, 3.4 Hz, 6H).

Step 2: Compound L4-1 (1.32 g, 1.3 mmol), HATU (597 mg, 1.57 mmol), 4-aminobenzyl alcohol (193 mg, 1.57 mol), and DIEA (335 mg, 2.6 mmol) were dissolved in a mixed solution of THF (10 mL), DCM (10 mL), and DMF (2.5 mL), and the solution was stirred at room temperature for 1 h. The reaction solution was concentrated to dryness by rotary evaporation and purified by silica gel column chromatography (DCM:(MeOH:THF = 1:1)) to give L4-2 (1.5 g, yield: 75%) as a yellow solid. LCMS: [M+H]⁺ = 1115.

Step 3: Compound L4-2 (1.45 g, 1.3 mmol) was dissolved in a solution of dimethylamine in THF (30 mL), and the solution was stirred at room temperature for 1 h. The reaction solution was concentrated to dryness by rotary evaporation and slurried with diethyl ether to give L4-3 (1.27 g, crude product) as a yellow oil. LCMS: [M+H]⁺ = 893

Step 4: Compound L4-3 (1.22 g, 1.37 mmol), compound L4-4 (728 mg, 2.74 mmol), and DIEA (353 mg, 2.74 mmol) were dissolved in a mixed solution of THF (50 mL) and DMF (1 mL), and the solution was stirred at room temperature for 1 h. The pH of the reaction solution was adjusted to 6 with 4 M HCl, and the mixture was concentrated to dryness by rotary evaporation and purified by silica gel column chromatography (DCM:THF and DCM:(THF:MeOH = 1:1)) twice to give L4-5 (1.37 g, crude product) as a yellow oil.

LCMS: [M+H]⁺ = 1044.

¹H NMR (400 MHz, DMSO) δ 9.81 (d, *J =* 58.4 Hz, 1H), 9.41 (s, 2H), 8.25 (dd, *J =* 10.7, 5.5 Hz, 2H), 8.08 (d, *J =* 7.1 Hz, 1H), 7.92-7.83 (m, 1H), 7.58 (dd, *J =* 16.9, 8.4 Hz, 2H), 7.23 (d, *J=* 8.0 Hz, 2H), 7.00 (t, *J =* 6.4 Hz, 2H), 6.76 (d, *J* = 82.2 Hz, 1H), 5.13 (t, *J* = 5.6 Hz, 1H), 4.42 (d, *J* = 5.5 Hz, 2H), 4.39 (t, *J* = 5.1 Hz, 4H), 4.24 (dt, *J =* 15.9, 7.1 Hz, 1H), 4.17-4.10 (m, 1H), 3.48 (d, *J* = 12.7 Hz, 40H), 3.43 (d, *J* = 2.2 Hz, 2H), 3.42 (s, 3H), 3.23 (s, 3H), 3.17 (d, *J* = 3.4 Hz, 2H), 2.42-2.39 (m, 1H), 2.10 (d, *J* = 6.6 Hz, 1H), 1.30 (s, 6H).

Step 5: Compound L4-5 (400 mg, 0.38 mmol), bis(p-nitrophenyl) carbonate (175 mg, 0.575 mmol), and DIEA (98 mg, 0.76 mmol) were dissolved in a mixed solution of THF (3 mL), DCM (3 mL), and DMF (0.5 mL), and the solution was stirred at room temperature for 16 h. The reaction solution was concentrated to dryness by rotary evaporation and purified by silica gel column chromatography (PE:(DCM:THF = 1:1) and DCM:(THF:MeOH = 1:1)) twice to give L4 (250 mg, yield: 49%) as a white gel.

LCMS: [M+H]⁺ = 1209.

¹H NMR (400 MHz, DMSO) δ 9.91 (d, *J =* 62.8 Hz, 1H), 8.32 (d, *J =* 9.1 Hz, 2H), 8.28-8.19 (m, 2H), 8.09 (t, *J* = 7.6 Hz, 1H), 7.89-7.81 (m, 1H), 7.71 (d, *J* = 8.5 Hz, 1H), 7.66 (d, *J* = 8.5 Hz, 1H), 7.60-7.53 (m, 2H), 7.42 (d, *J =* 8.3 Hz, 2H), 6.99 (d, *J =* 5.6 Hz, 2H), 5.25 (s, 2H), 4.39 (dt, *J =* 9.2, 7.1 Hz, 1H), 4.31-4.19 (m, 1H), 4.14 (dt, *J =* 16.7, 8.3 Hz, 1H), 3.50 (d, *J* = 4.5 Hz, 40H), 3.42 (dd, *J =* 5.9, 3.6 Hz, 2H), 3.40-3.34 (m, 3H), 3.31 (s, 1H), 3.23 (s, 3H), 3.22-3.14 (m, 2H), 2.40 (dt, *J* = 6.9, 4.7 Hz, 2H), 2.10 (d, *J* = 7.7 Hz, 2H), 1.27-1.22 (m, 6H).

The following compounds were synthesized with reference to the synthesis method for compound L4.

| No. | Structure | LCMS[M+H]⁺ |
|---|---|---|
| L4-A | 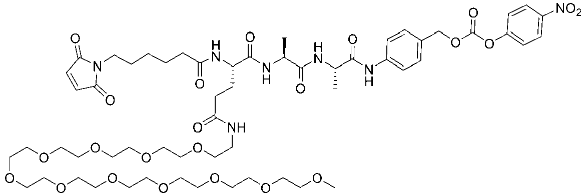 | 1250.6 |
| L4-B | 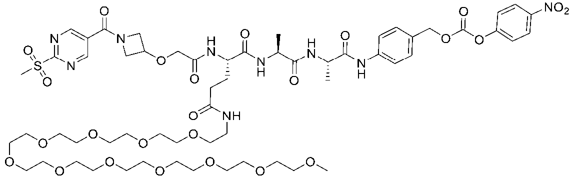 | 1354.6 |

### Example 1.2.5: Synthesis of Compound L5

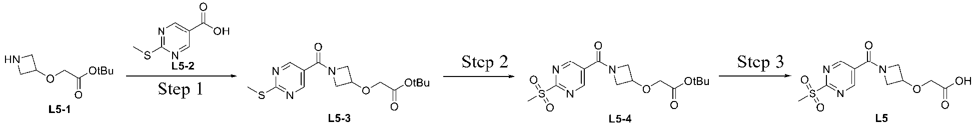

Step 1: Under a nitrogen atmosphere, DIEA (0.51 g, 3.96 mmol) was added to a solution of L5-1 (0.37 g, 1.98 mol), L5-2 (0.33 g, 1.98 mmol), and HATU (1.13 g, 2.97 mmol) in DMF (5 mL). The reaction solution was stirred at room temperature for 1 h. Saturated brine was added to the reaction solution, and the mixture was extracted with ethyl acetate. The organic phase was dried over anhydrous sodium sulfate, filtered, and concentrated under reduced pressure to give a mixture. The mixture was purified by column chromatography (PE/EA) to give L5-3 (0.4 g, yield: 74%) as a white solid.

LCMS: [M+H]⁺ = 340.2.

Step 2: Potassium peroxymonosulfate (1.96 g, 3.08 mmol, water, 20 mL) was added to a solution of L5-3 (0.36 g, 1.06 mmol) in THF (20 mL). The reaction solution was stirred at room temperature for 1 h under a nitrogen atmosphere and extracted with water and ethyl acetate. The resulting organic phase was washed with saturated brine, dried over anhydrous sodium sulfate, and filtered to give a mixed solution. The mixed solution was concentrated under reduced pressure to give L5-4 (0.27 g, yield: 68%) as a yellow oil.

LCMS: [M+H]⁺ = 372.3.

Step 3: A solution of L5-4 (0.1 g, 0.26 mmol) in DCM/TFA (3/1, 6 mL) was stirred at room temperature for 1 h. The reaction solution was concentrated under reduced pressure to give a mixture. The mixture was purified by reversed-phase column chromatography to give L5 (30 mg, yield: 35%) as a white solid.

LCMS: [M+H]⁺ = 316.1.

### Example 1.3: Synthesis of Linker-Small Molecule Compounds

### Example 1.3.1: Compound III-D2

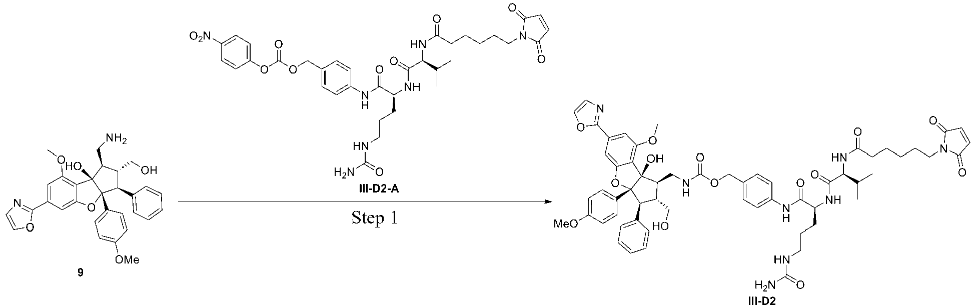

Step 1: Compound 9 (12 mg, 0.023 mmol) was dissolved in DMF (1 mL), and compound III-D2-A (CAS: 159857-81-5) (17 mg, 0.023 mmol) and DIEA (10.9 mg, 0.09 mmol) were added. The mixture was stirred at room temperature for 2 h. The reaction solution was purified by preparative medium-pressure liquid chromatography (ACN/H₂O = 0% to 100%) to give III-D2 (12 mg, yield: 46%) as a white solid.

LCMS [M+H]⁺ = 1113.3.

¹H NMR (400 MHz, DMSO) δ 10.03 (s, 1H), 8.25 (s, 1H), 8.11 (d, J = 7.6 Hz, 1H), 7.82 (d, J = 8.4 Hz, 1H), 7.63 (d, J = 8.4 Hz, 2H), 7.44-7.33 (m, 3H), 7.21 (d, J = 10.2 Hz, 2H), 7.06 (dd, J = 27.4, 12.8 Hz, 7H), 6.69 (dd, J = 30.1, 6.8 Hz, 5H), 5.98 (s, 1H), 5.51 (s, 1H), 5.42 (s, 2H), 5.05 (dd, J = 35.2, 12.0 Hz, 2H), 4.57 (t, J = 4.4 Hz, 1H), 4.43-4.35 (m, 1H), 4.23-4.16 (m, 1H), 3.91 (s, 3H), 3.71-3.61 (m, 6H), 3.16-2.83 (m, 5H), 2.38 (t, J = 18.4 Hz, 1H), 2.22-2.06 (m, 2H), 1.96 (dd, J = 13.4, 6.8 Hz, 1H), 1.78-1.29 (m, 9H), 1.28-1.12 (m, 3H), 0.84 (dd, J = 13.6, 6.8 Hz, 6H).

The following compounds were synthesized with reference to the synthesis method for compound III-D2.

| No. | Structure | LCMS [M+H]⁺ |
|---|---|---|
| I-D5 | 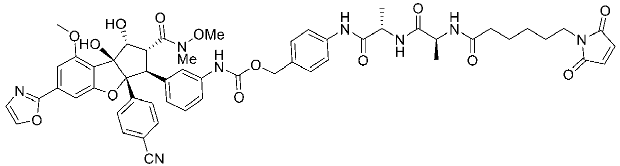 | 1053.4 |
| III-D3 | 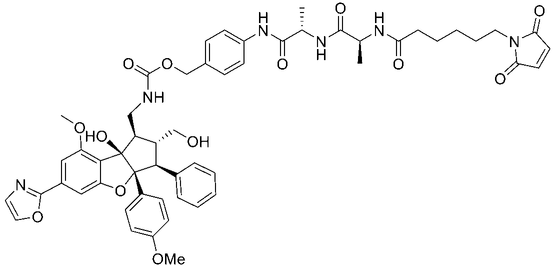 | 999.4 |
| III-D4 | 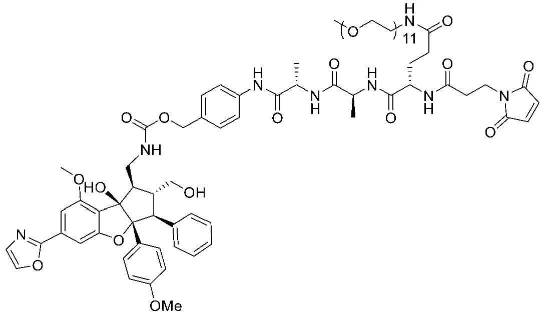 | 1583.6 |
| II-D2 | 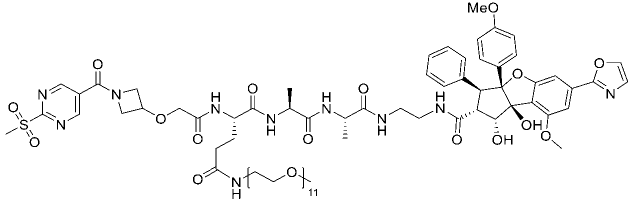 | 1623.7 |
| II-D6 | 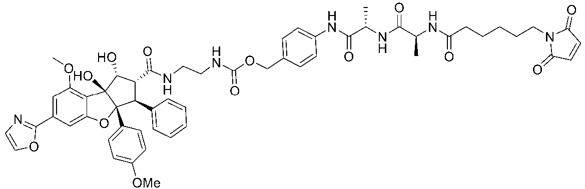 | 1042.4 |
| II-D7 | 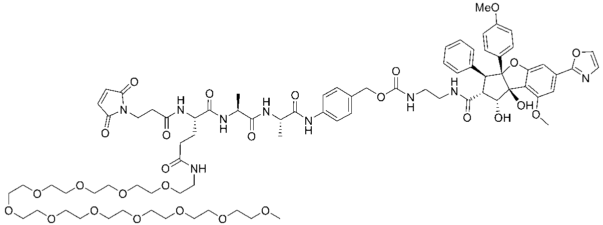 | 1626.7 |
| I-D16 | 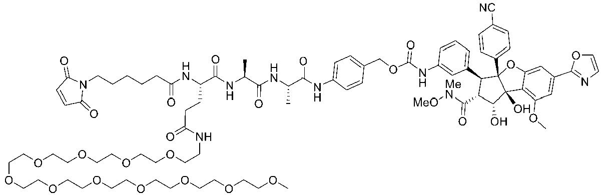 | 1679.8 |
| I-D17 | 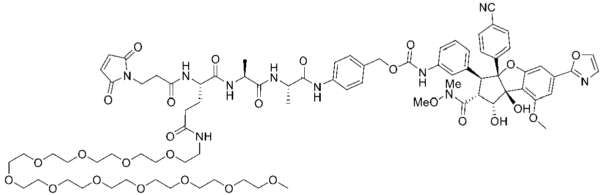 | 1637.7 |
| I-D18 | 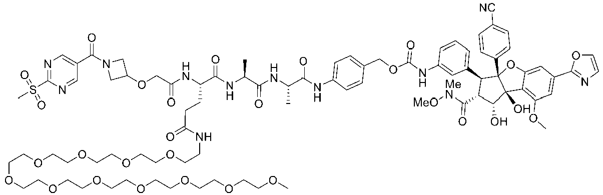 | 1783.7 |
| I-D19 | 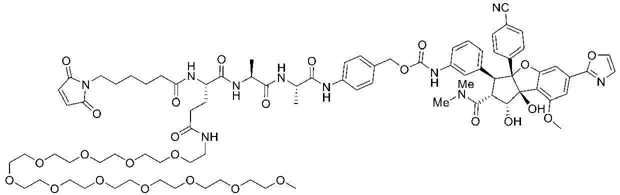 | 1663.8 |
| I-D20 | 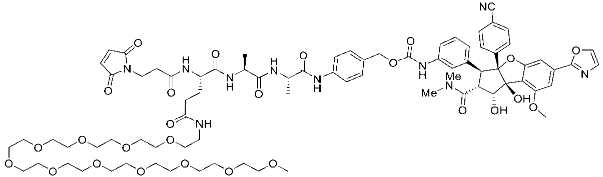 | 1621.7 |
| I-D21 | 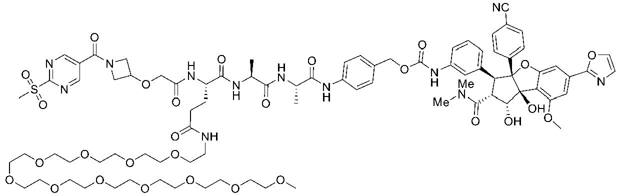 | 1767.7 |
| I-D26 | 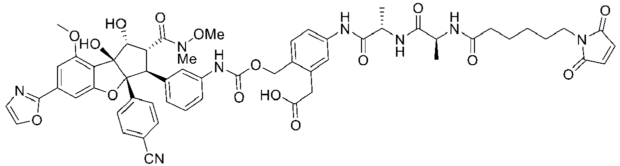 | 1111.4 |
| I-D27 | 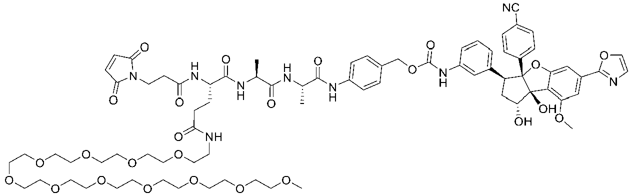 | 1550.7 |
| I-D28 | 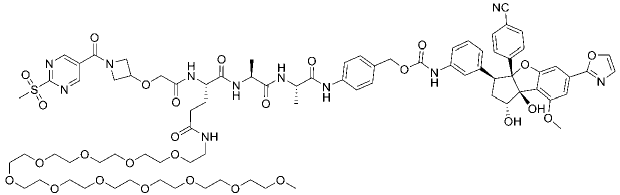 | 1696.7 |
| V-D8 | 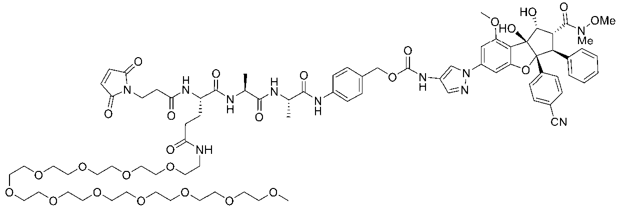 | 1636.7 |

### Example 1.3.2: Compound I-D1

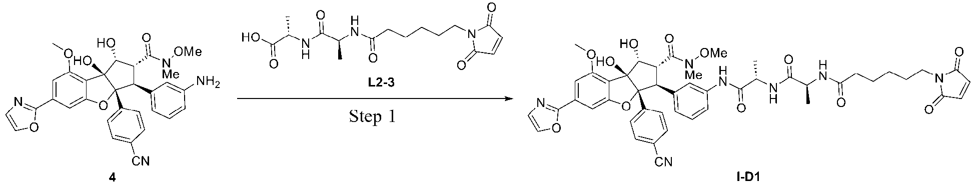

Step 1: A mixture of compound 4 (10 mg, 0.017 mmol), compound L2-3 (6 mg, 0.017 mmol), HATU (6.4 mg, 0.017 mmol), HOAT (2.3 mg, 0.017 mmol), and TMP (8.2 mg, 0.07 mmol) was added to DMF (1 mL), and the mixture was then stirred at room temperature for 2 h. The solution was purified by flash ACN/H₂O (0.001 FA) = (0%-30%) and ACN/H₂O (30%-100%) to give I-D1 (12 mg, yield: 46%) as a white solid.

LCMS [M+H]⁺ = 904.2.

¹H NMR (400 MHz, DMSO) δ 11.03 (s, 1H), 9.04 (t, *J =* 5.6 Hz, 1H), 8.11 (s, 1H), 8.03 (d, *J =* 8.0 Hz, 1H), 7.85 (d, *J =* 8.0 Hz, 1H), 7.16 (t, *J =* 1.6 Hz, 1H), 7.14-7.10 (m, 1H), 6.86 (t, *J* = 2.0 Hz, 1H), 5.76 (s, 2H), 5.16 (dd, *J =* 13.2, 5.1 Hz, 1H), 4.71 (d, *J =* 5.6 Hz, 2H), 4.48 (dd, *J* = 53.5, 17.6 Hz, 2H), 2.99-2.86 (m, 1H), 2.61 (d, *J =* 17.2 Hz, 1H), 2.45-2.38 (m, 1H), 2.09-1.98 (m, 1H).

The following compounds were synthesized with reference to the method for compound I-D1.

| No. | Structure | LCMS [M+H]⁺ |
|---|---|---|
| I-D3 | 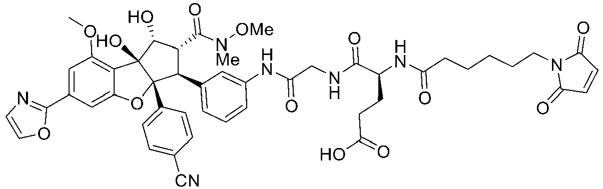 | 1053.4 |
| I-D4 | 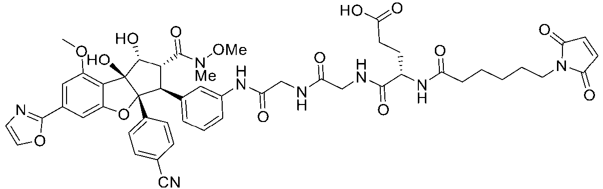 | 999.4 |
| I-D6 | 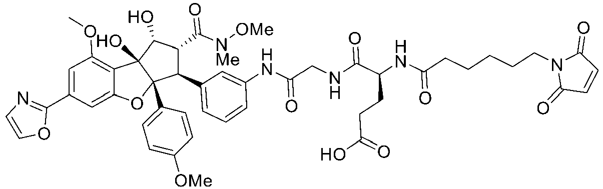 | 953.3 |
| I-D7 | 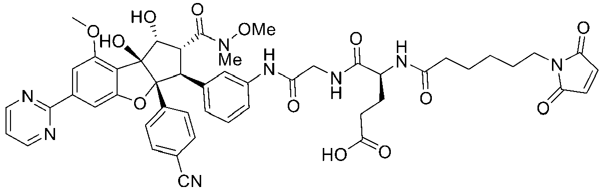 | 959.3 |
| I-D9 | 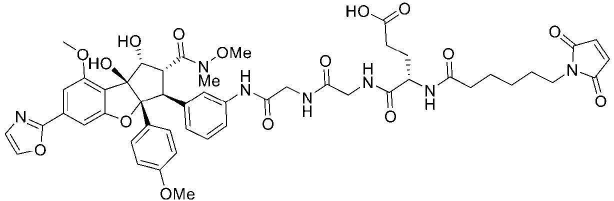 | 1010.4 |
| I-D10 | 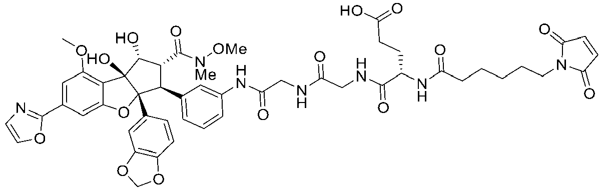 | 1024.3 |
| I-D11 | 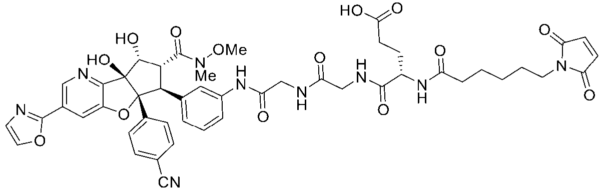 | 976.3 |
| IV-D1 | 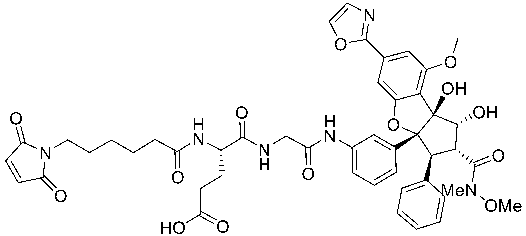 | 923.3 |
| IV-D2 | 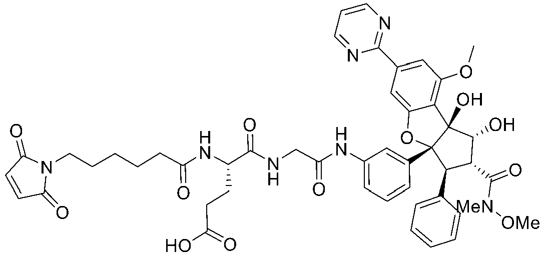 | 934.4 |
| V-D1 | 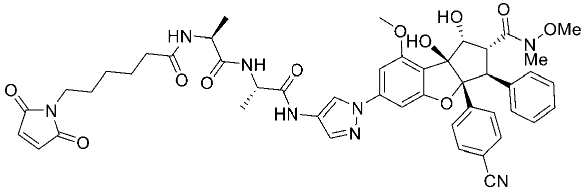 | 903.4 |
| I-D22 | 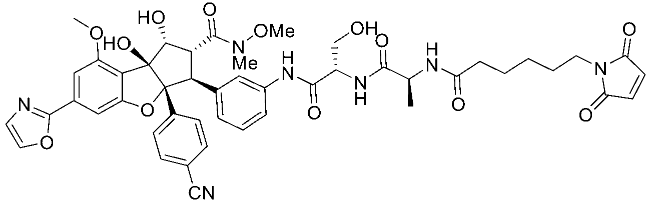 | 920.3 |
| I-D23 | 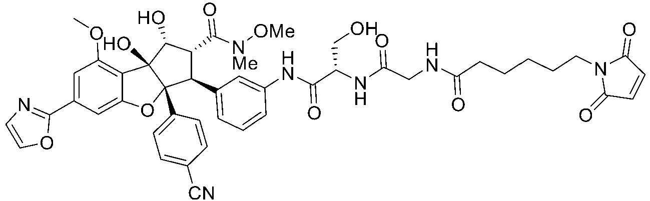 | 906.3 |
| I-D24 | 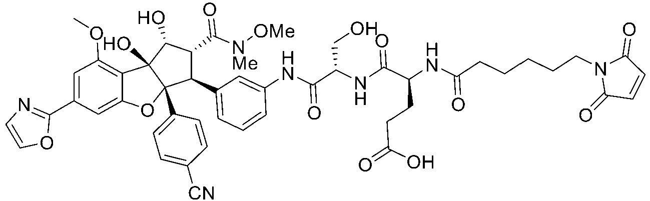 | 978.3 |
| I-D25 | 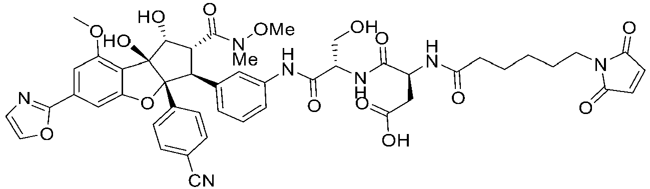 | 964.3 |
| I-D31 | 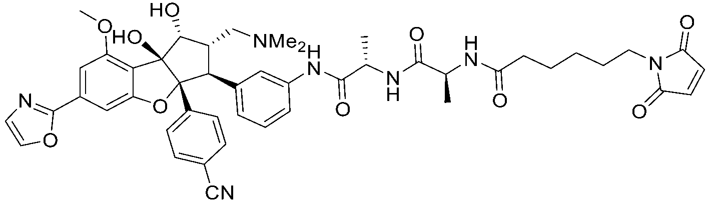 | 874.4 |
| I-D32 | 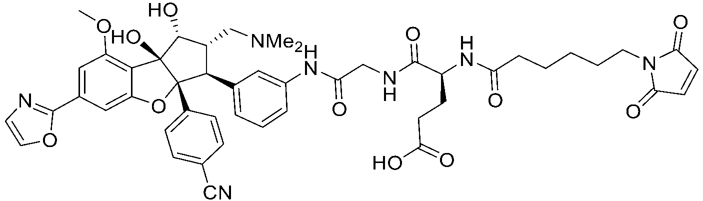 | 918.4 |
| I-D33 | 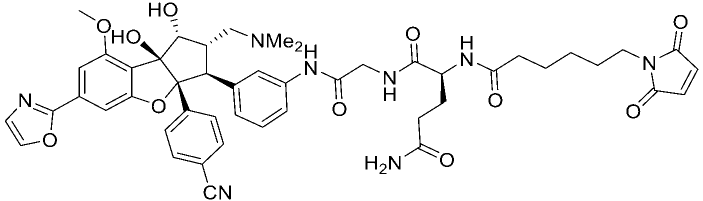 | 917.4 |
| I-D34 | 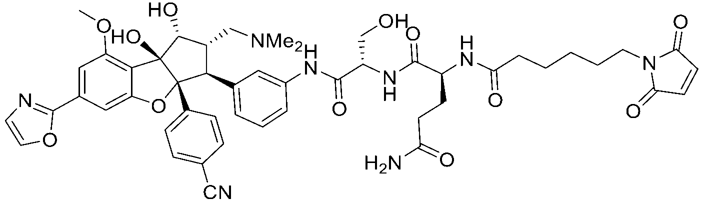 | 947.4 |
| I-D35 | 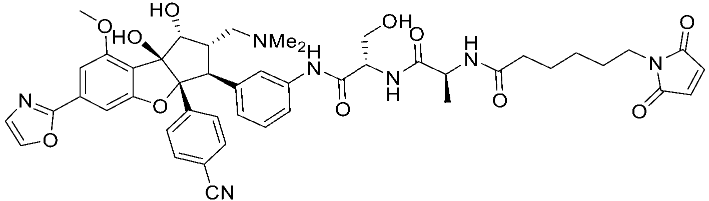 | 890.4 |
| I-D36 | 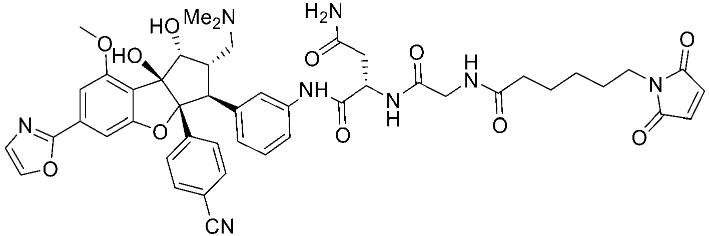 | 903.4 |
| I-D37 | 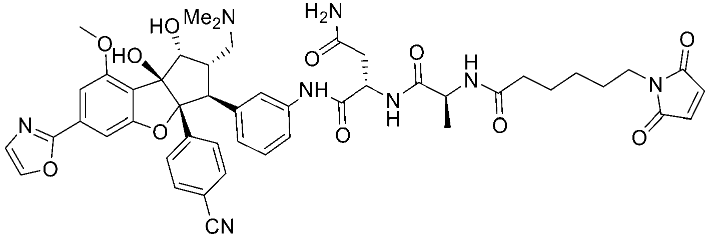 | 917.4 |
| I-D42 | 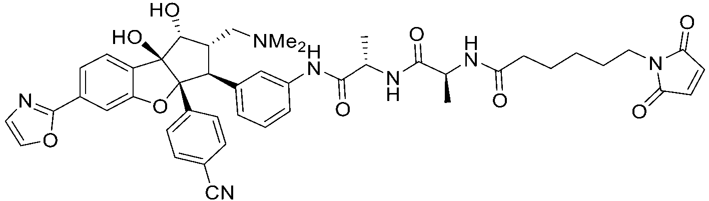 | 844.4 |
| I-D43 | 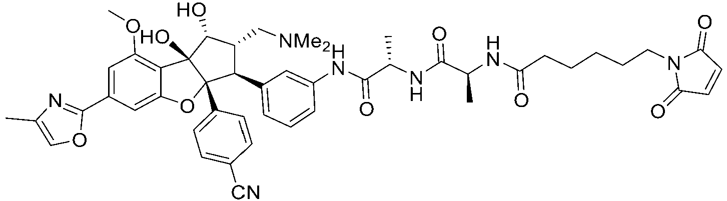 | 888.4 |
| I-D44 | 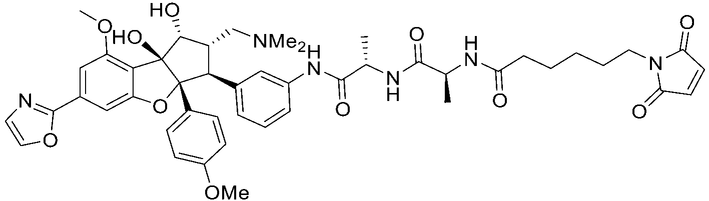 | 879.4 |
| I-D46 | 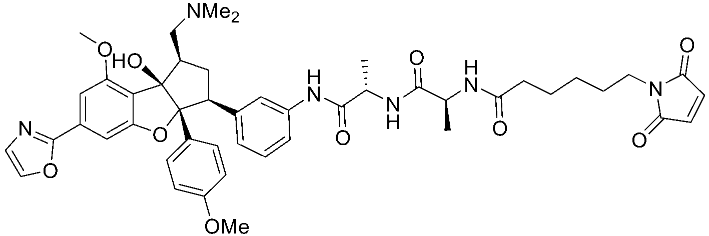 | 863.4 |
| I-D51 | 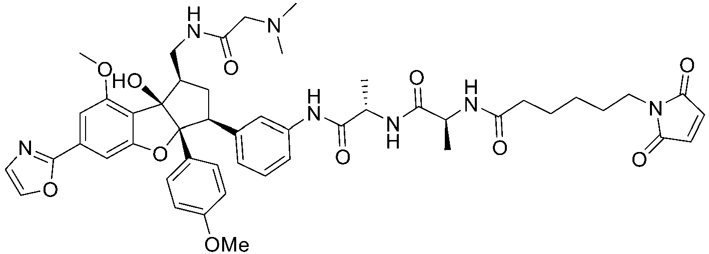 | 920.5 |
| I-D52 | 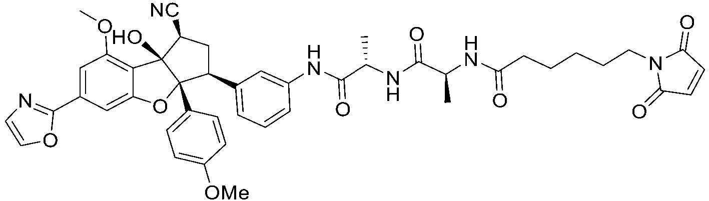 | 831.3 |
| I-D55 | 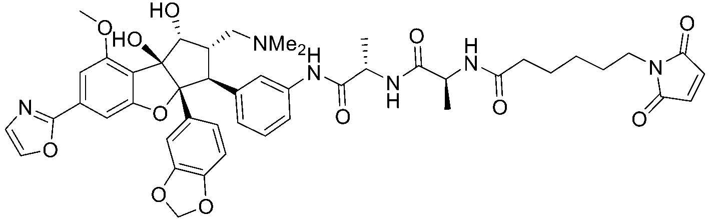 | 893.4 |

### Example 1.3.3: Synthesis of Compound I-D41

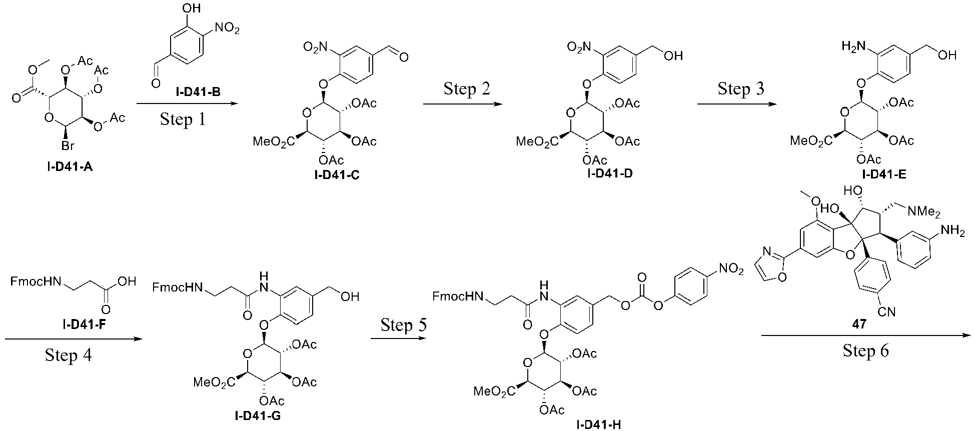

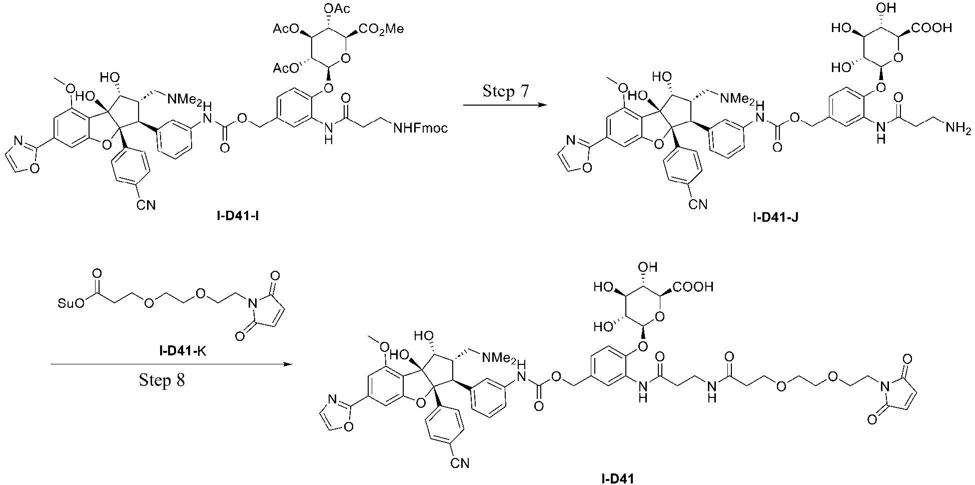

Step 1: Compound I-D41-A (20 g, 50.51 mmol), compound I-D41-B (8.43 g, 50.51 mmol), and silver oxide (46.7 g, 202.02 mmol) were dissolved in acetonitrile (300 mL), and the solution was stirred at room temperature for 16 h. The reaction solution was purified by normal-phase column chromatography (PE/EA= 20%/80%) to give I-D41-C (19 g, yield: 77.5%) as a white solid. LCMS: [M+Na]⁺ = 506.0.

Step 2: Compound I-D41-C (5.17 g, 10.7 mmol) and silica gel (3.85 g, 64.22 mmol) were dissolved in isopropanol/chloroform (20 mL/80 mL), and NaBH₄ (406 mg, 10.7 mmol) was added under ice bath conditions. The mixture was stirred at room temperature for 2 h. The reaction solution was filtered and concentrated under reduced pressure to give I-D41-D (4.3 g, yield: 82.8%) as a white solid. LCMS: [M+NH⁴]⁺ = 503.2.

Step 3: Compound I-D41-D (4.3 g, 8.87 mmol) was dissolved in EA/THF (50 mL/50 mL), and Pd/C (1.25 g, 10%) was added. The mixture was stirred at room temperature for 2 h. The reaction solution was filtered and concentrated under reduced pressure to give I-D41-E (4 g, yield: 99.2%) as a brown solid. LCMS: [M+H]⁺ = 456.2.

Step 4: Compound I-D41-E (4.1 g, 9.01 mmol), HATU (3.76 g, 9.91 mmol), HOAT (1.35 g, 9.91 mmol), TMP (3.27 g, 27.03 mmol), and compound I-D41-F (2.8 g, 9.01 mmol) were dissolved in DMF (10 mL), and the solution was stirred at room temperature for 2 h. The reaction solution was purified by reversed-phase column chromatography (water/acetonitrile = 30%/70%) to give I-D41-G (5.8 g, yield: 86.1%) as a yellow solid. LCMS: [M+H]⁺ = 749.2.

Step 5: A solution of compound I-D41-G (1.0 g, 1.34 mmol), bis(p-nitrophenyl) carbonate (487.7 mg, 1.60 mmol), and DIEA (517.4 mg, 4.01 mmol) in DMF (10 mL) was stirred at 25 °C for 1 h. The reaction solution was purified by reversed-phase column chromatography (H₂O:acetonitrile = 0-100%) to give compound I-D41-H (700 mg, yield: 70%).

Step 6: DIEA (24.0 mg, 0.18 mmol) was added to a solution of compound I-D41-H (70.0 mg, 0.09 mmol), compound 47 (50.1 mg, 0.09 mmol), and HOAT (25.3 mg, 0.19 mmol) in DMF (3 mL), and the mixture was stirred at 25 °C for 16 h. The reaction solution was diluted with water and extracted with EA. The organic layer was concentrated and purified by silica gel column chromatography (PE/EA) to give compound I-D41-I (10 mg, yield: 10.0%).

LCMS: [M+H]⁺ = 1314.2.

Step 7: Compound I-D41-I (10.0 mg, 0.038 mmol) and LiOH·H₂O (6.4 mg, 0.15 mmol) were stirred in DMF/MeOH (2 mL/0.5 mL) for 3 h. The mixture was purified by reversed-phase column chromatography (0.1% FA/H2O:acetonitrile = 0-100%) to give compound I-D41-J (4 mg, yield: 57%). LCMS [M+H]⁺ = 951.2.

Step 8: Compound I-D41-J (4.0 mg, 0.01 mmol), compound I-D41-K (3.7 mg, 0.01 mmol), and DIEA (4.1 mg, 0.04 mmol) were stirred in DMF (1 mL) at 25 °C for 2 h. The mixture was purified by reversed-phase column chromatography (0.1% FA/H₂O:MeOH = 0-100%) to give compound I-D41 (2 mg, yield: 40%).

LCMS: 1/2[M+2H-H₂O]⁺ = 586.8.

¹H NMR (400 MHz, DMSO) δ 9.59 (s, 1H), 9.18 (s, 1H), 8.24 (s, 2H), 8.02 (t, *J* = 5.5 Hz, 1H), 7.51 (d, *J* = 8.5 Hz, 2H), 7.41 (d, *J* = 9.6 Hz, 3H), 7.23 (s, 1H), 7.14 (dd, *J* = 13.5, 8.0 Hz, 5H), 7.02-6.95 (m, 3H), 6.63 (d, *J* = 7.7 Hz, 1H), 5.78 (s, 1H), 5.63 (s, 1H), 5.22 (s, 1H), 5.04 (t, *J* = 8.2 Hz, 2H), 4.76 (d, *J* = 7.1 Hz, 1H), 4.57 (d, *J* = 3.8 Hz, 1H), 3.85 (s, 5H), 3.71 (dd, *J* = 34.5, 10.7 Hz, 7H), 3.53 (d, *J* = 5.7 Hz, 11H), 2.87 (d, *J* = 14.2 Hz, 2H), 2.58 (d, *J* = 7.4 Hz, 3H), 2.47 (s, 4H), 2.28 (t, *J* = 6.5 Hz, 2H), 2.03-1.95 (m, 1H).

The following compounds were synthesized with reference to the synthesis method for compound I-D41.

| No. | Structure | LCMS [M+H]⁺ |
|---|---|---|
| I-D56 | 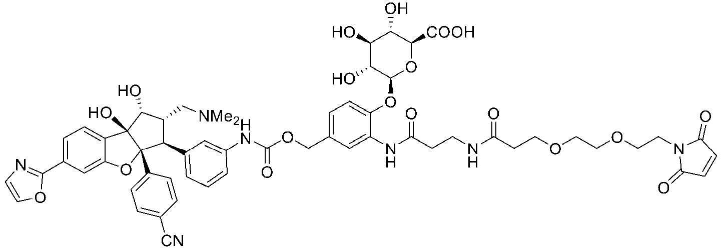 | 1160.4 |
| I-D57 | 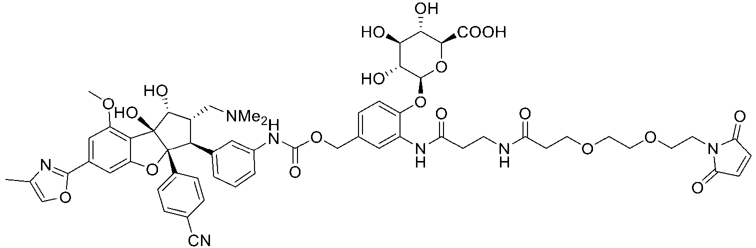 | 1204.4 |
| I-D58 | 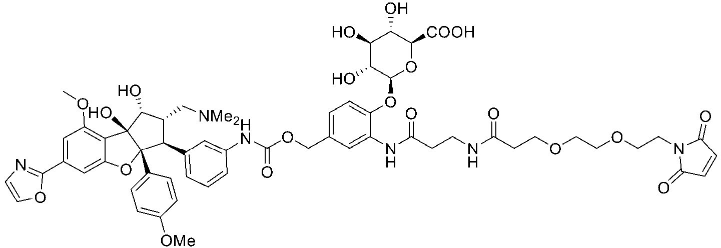 | 1195.4 |
| I-D59 | 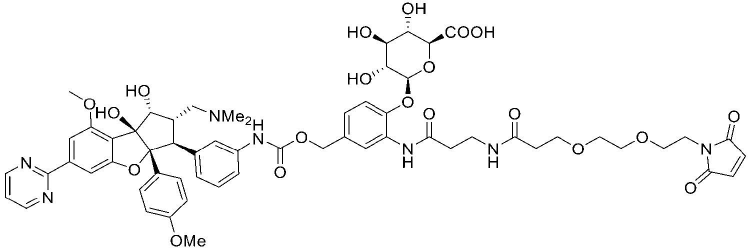 | 1206.4 |
| I-D61 | 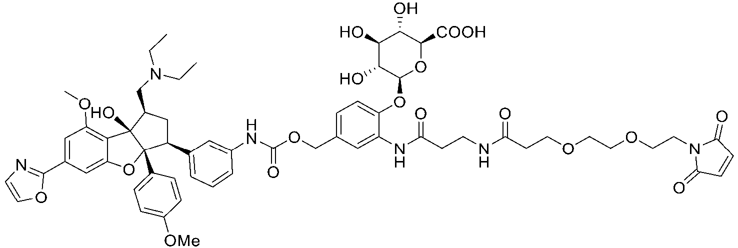 | 1207.4 |
| I-D62 | 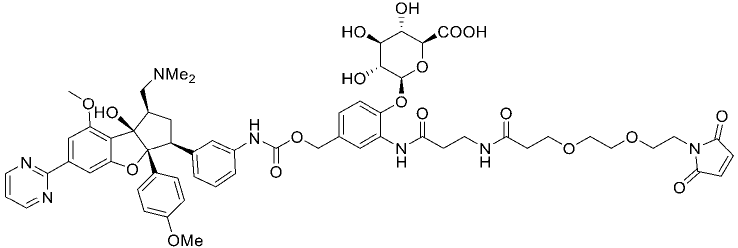 | 1190.4 |
| I-D63 | 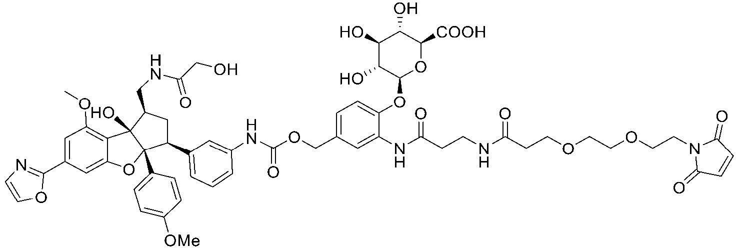 | 1209.4 |
| I-D64 | 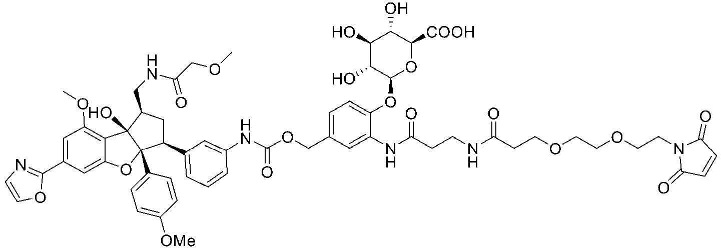 | 1223.4 |
| I-D66 | 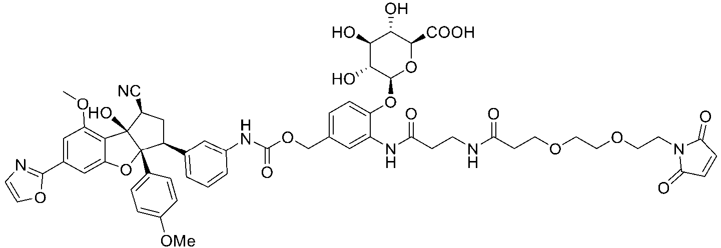 | 1147.4 |
| I-D67 | 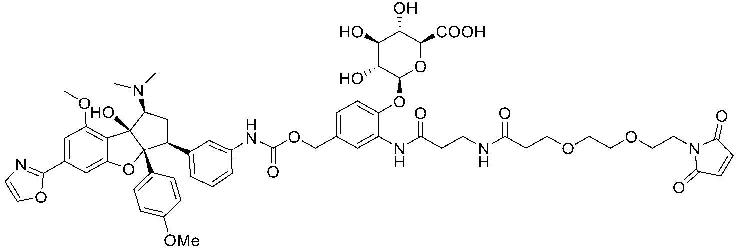 | 1165.4 |
| I-D68 | 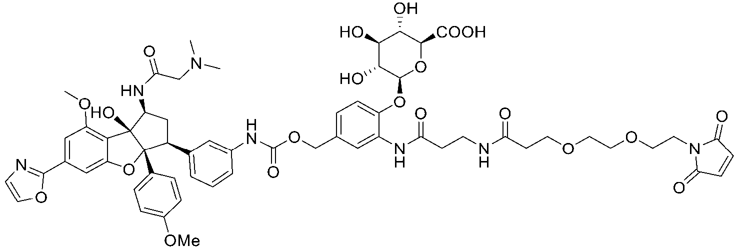 | 1222.4 |
| I-D69 | 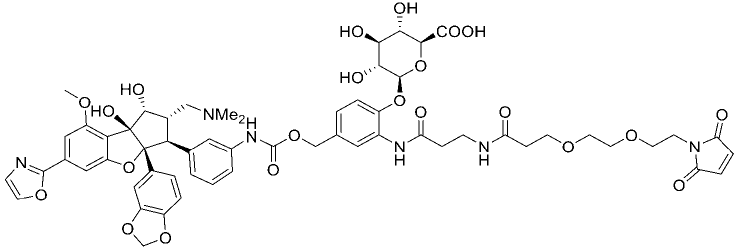 | 1209.4 |
| II-D20 | 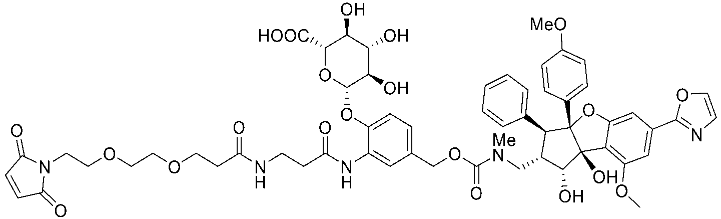 | 1166.4 |
| II-D21 | 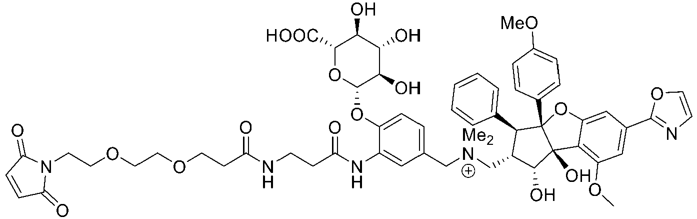 | 1137.4 |
| III-D11 | 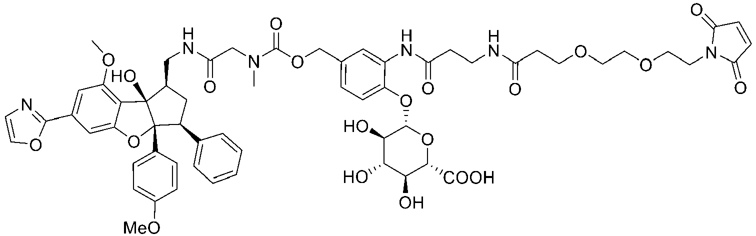 | 1207.4 |
| III-D12 | 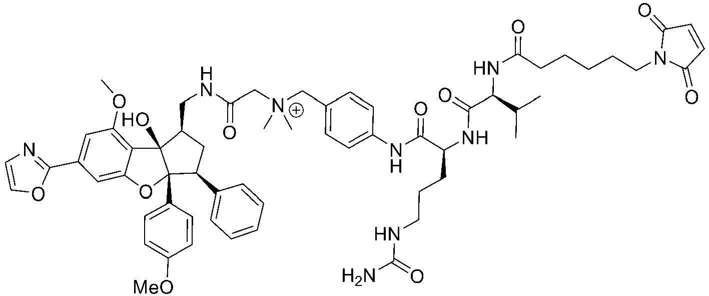 | 1125.5 |
| IV-D18 | 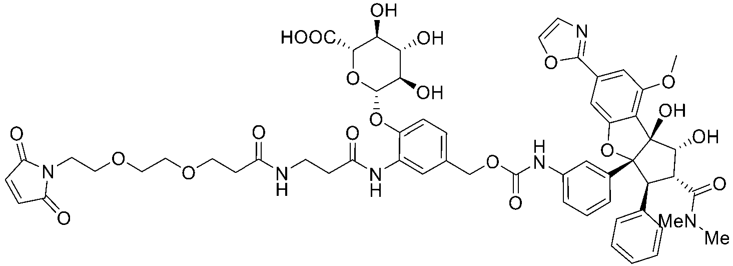 | 1179.4 |
| IV-D20 | 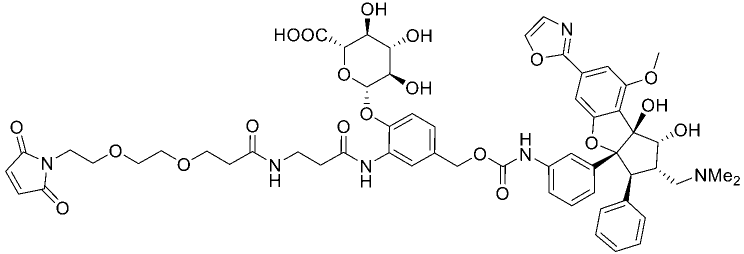 | 1165.4 |
| V-D9 | 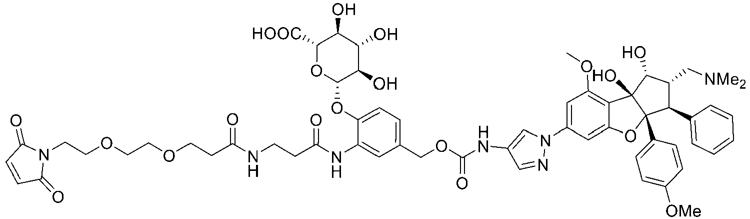 | 1194.4 |

### Example 1.3.4: Synthesis of Compound I-D60

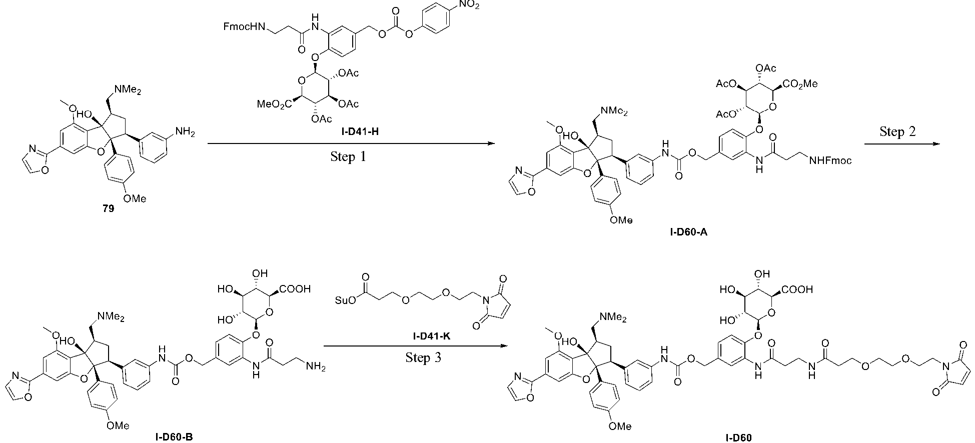

Step 1: Compound 79 (42 mg, 0.081 mmol) was stirred in DMF (2 mL), and compound I-D41-H (74 mg, 0.011 mmol), HOAT (12.1 mg, 0.0891 mmol), and DIEA (231.3 mg, 0.243 mmol) were then added. The mixture was stirred at 20 °C for 16 h to give compound I-D60-A. The reaction solution was directly used in the next step without any treatment. LCMS [M+H]⁺ = 1302.8.

Step 2: An aqueous LiOH solution (1.5 mL, 1 mol/L) was added to the solution of compound I-D60-A in DMF obtained in step 1, and the mixture was stirred at 20 °C for 20 min. The reaction solution was purified by reversed-phase column chromatography (water-acetonitrile) to give compound I-D60-B (40 mg, yield: 79%). LCMS [M+H]⁺ = 940.2.

Step 3: Compound I-D60-B (40 mg, mmol) and compound I-D41-K (15 mg, 0.0426 mmol) were added to DMF (1.5 mL), and DIEA (16.5 mg, 0.128 mmol) was then added. The mixture was stirred at 20 °C for 20 min. The reaction solution was purified by reversed-phase column chromatography (water-acetonitrile) to give I-D60 (30 mg, yield: 60%) as a white solid. LCMS [M+H]⁺ = 1179.2.

¹H NMR (400 MHz, DMSO-d₆) δ 9.55 (s, 1H), 9.26-9.08 (m, 1H), 8.29-8.15 (m, 2H), 8.08-7.94 (m, 1H), 7.41 (s, 1H), 7.28-7.19 (m, 3H), 7.17-7.03 (m, 5H), 7.00 (s, 2H), 6.95 (t, J = 7.8 Hz, 1H), 6.73-6.65 (m, 2H), 6.58-6.48 (m, 1H), 5.93-5.62 (m, 1H), 5.17 (s, 1H), 5.03 (s, 2H), 4.69 (d, J = 7.2 Hz, 1H), 3.96 (s, 3H), 3.64 (s, 3H), 3.60-3.49 (m, 5H), 3.49-3.22 (m, 17H), 3.21-3.10 (m, 1H), 2.99 (s, 1H), 2.70-2.62 (m, 5H), 2.60-2.53 (m, 2H), 2.27 (t, J = 6.6 Hz, 3H), 2.12 (q, J = 12.5 Hz, 1H).

### Example 1.3.5: Synthesis of Compound I-D65

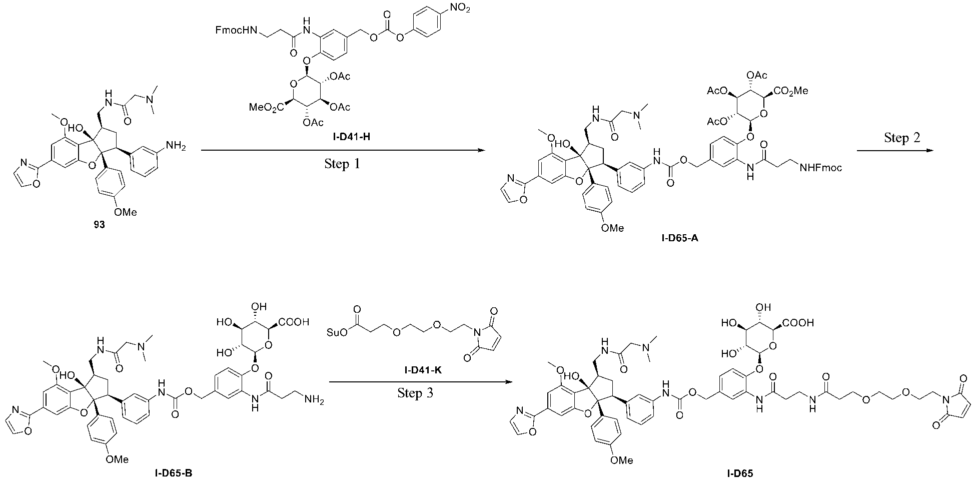

Step 1: Compound 93 (39 mg, 0.0471 mmol), HOAT (8.7 mg, 0.0642 mmol), and DIEA (16 mg, 0.128 mmol) were added to a solution of compound I-D41-H (25 mg, 0.0428 mmol) in DMF (1.5 mL), and the mixture was stirred at 20 °C for 20 h to give compound I-D65-A. The reaction solution was directly used in the next step without any treatment. LCMS [M+H]⁺ = 1359.2.

Step 2: An aqueous LiOH solution (1.5 mL, 1 M) was added to the solution of compound I-D65-A in DMF (1.5 mL) obtained in step 1, and the mixture was stirred at 20 °C for 20 min. The reaction solution was purified by reversed-phase column chromatography (water:acetonitrile) to give compound I-D65-B (15 mg, yield: 68%). LCMS [M+H]⁺ = 997.2.

Step 3: Compound I-D65-B (15 mg, 0.015 mmol) was added to DMF (2 mL), and compound I-D41-K (5.3 mg, 0.015 mmol) and DIEA (5.8 mg, 0.045 mmol) were subsequently added. The mixture was stirred at 20 °C for 20 min. The reaction solution was purified by reversed-phase column chromatography (water:acetonitrile) to give I-D65 (6 mg, yield: 32%) as a white solid. LCMS: [1/2M+H]⁺ = 618.8.

¹H NMR (400 MHz, DMSO-*d*₆) δ 9.56 (s, 1H), 9.18-9.07 (m, 1H), 8.28-8.21 (m, 2H), 8.03-7.93 (m, 1H), 7.86-7.77 (m, 1H), 7.41 (d, *J* = 0.8 Hz, 1H), 7.23-7.17 (m, 4H), 7.16-7.07 (m, 4H), 7.01 (s, 2H), 6.94 (t, *J =* 8.1 Hz, 1H), 6.72-6.65 (m, 2H), 6.52 (d, *J =* 7.7 Hz, 1H), 5.85-5.76 (m, 1H), 5.32 (s, 1H), 5.28-5.20 (m, 1H), 5.03 (s, 2H), 4.79 (d, *J =* 7.3 Hz, 1H), 3.96 (s, 3H), 3.80-3.71 (m, 2H), 3.64 (s, 3H), 3.63-3.57 (m, 1H), 3.56-3.51 (m, 4H), 3.50-3.38 (m, 8H), 3.09-2.97 (m, 2H), 2.92-2.81 (m, 1H), 2.60-2.54 (m, 2H), 2.31-2.24 (m, 8H), 2.20-2.04 (m, 2H).

### Example 1.3.6: Synthesis of Compound I-D73

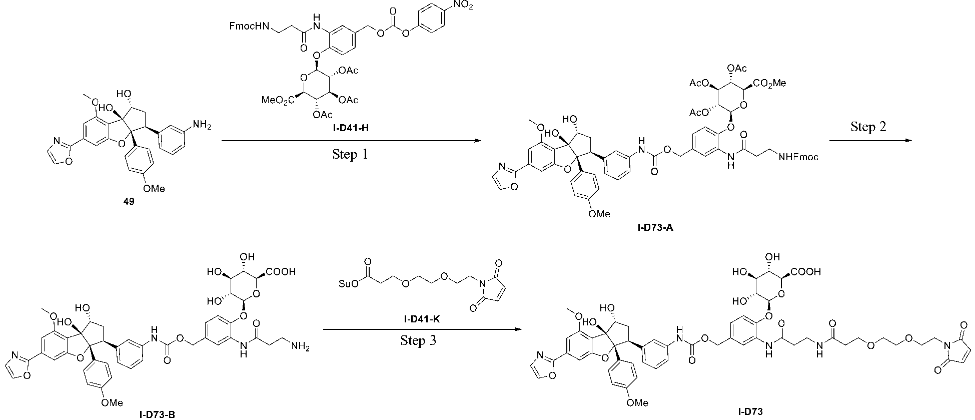

Step 1: Compound 49 (30 mg, 0.06 mmol), I-D41-H (56.4 mg, 0.06 mmol), and HOAT (16.8 mg, 0.12 mmol) were dissolved in DMF (2 mL), and DIEA (15.9 mg, 0.12 mmol) was added to the solution. The mixture was stirred at 20 °C for 16 h. The reaction solution was concentrated and purified by silica gel column chromatography (MeOH/DCM) to give compound I-D73-A (30 mg, yield: 39%). LCMS: [M+H]⁺=1261.3.

Step 2: Compound I-D73-A (30 mg, 0.02 mmol) was dissolved in a mixed solution of THF (3 mL)/MeOH (1 mL)/H₂O (1 mL), and LiOH (3.0 mg, 0.07 mmol) was added. The mixture was stirred at 20 °C for 1.5 h. The reaction solution was directly purified by reversed-phase silica gel column chromatography (H₂O/CH₃CN) to give compound I-D73-B (10 mg, yield: 47%). LCMS: [M+H]⁺=899.8.

Step 3: Compound I-D73-B (15.0 mg, 0.017 mmol) and compound I-D41-K (6.0 mg, 0.017 mmol) were dissolved in DMF (1 mL), and DIEA (5.5 mg, 0.043 mmol) was added. The mixture was stirred at 20 °C for 2 h. The reaction solution was directly purified by reversed-phase silica gel column chromatography (H₂O/CH₃CN) to give I-D73 (5.3 mg, yield: 28%) as a white solid.

LCMS [M+H]⁺=1138.2.

¹H NMR (400 MHz, DMSO) δ 9.57 (s, 1H), 9.14 (s, 1H), 8.24 (s, 2H), 7.99 (s, 1H), 7.40 (s, 1H), 7.26 (s, 1H), 7.19 (d, J = 7.6 Hz, 1H), 7.17-7.13 (m, 2H), 7.11 (s, 2H), 7.03 (d, J = 9.0 Hz, 2H), 7.01 (s, 2H), 6.97 (t, J = 7.9 Hz, 1H), 6.64 (d, J = 7.8 Hz, 1H), 6.61 (d, J = 9.0 Hz, 2H), 5.81 (s, 1H), 5.26 (s, 1H), 5.09 (s, 1H), 5.04 (s, 2H), 4.81 (s, 1H), 4.70 (d, J = 3.9 Hz, 1H), 4.50 (t, J = 4.0 Hz, 1H), 3.96 (dd, J = 14.3, 6.1 Hz, 1H), 3.86 (s, 3H), 3.60 (s, 3H), 3.54 (t, J = 6.3 Hz, 4H), 3.48 (d, J = 5.2 Hz, 2H), 3.46-3.36 (m, 8H), 2.57 (s, 2H), 2.28 (t, J = 6.5 Hz, 2H), 2.05-1.96 (m, 2H), 1.24 (s, 4H).

### Example 1.3.7: Synthesis of Compound I-D74

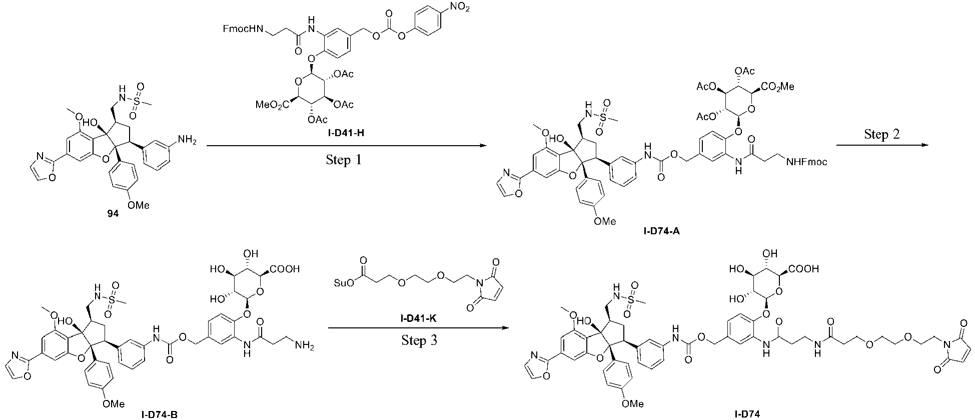

Step 1: DIEA (13.0 mg, 0.1 mmol) was added to a solution of compound 94 (29 mg, 0.05 mmol), compound I-D41-H (45.9 mg, 0.05 mmol), and HOAt (13.7 mg, 0.1 mmol) in DMF (1.5 mL), and the mixture was stirred at 20 °C for 20 h to give compound I-D74-A. The reaction solution was directly used in the next step without any treatment. LCMS [M+H]⁺ = 1352.7.

Step 2: An aqueous LiOH solution (1.5 mL, 1 M) was added to the solution of compound I-D74-A in DMF obtained in step 1, and the mixture was stirred at 20 °C for 1.5 h. The reaction solution was purified by reversed-phase column chromatography (water:acetonitrile) to give I-D74-B (40 mg, yield: 62%) as a white solid. LCMS [M+H]⁺ = 990.7.

Step 3: DIEA (15.6 mg, 0.12 mmol) was added to a solution of compound I-D74-B (40 mg, 0.04 mmol) and compound I-D41-K (14.3 mg, 0.04 mmol) in DMF (2 mL), and the mixture was stirred at room temperature for 1 h. The reaction solution was purified by reversed-phase column chromatography (water:acetonitrile) to give I-D74 (15 mg, yield: 32%) as a white solid.

LCMS [M+H]⁺ = 1229.3

¹H NMR (400 MHz, DMSO) δ 9.56 (s, 1H), 9.10 (s, 1H), 8.33-8.14 (m, 2H), 7.97 (d, J = 5.4 Hz, 1H), 7.41 (d, J = 0.7 Hz, 1H), 7.21 (dd, J = 9.2, 3.5 Hz, 4H), 7.14-7.08 (m, 4H), 7.01 (s, 2H), 6.95 (t, J = 7.8 Hz, 1H), 6.83 (t, J = 6.0 Hz, 1H), 6.68 (d, J = 9.0 Hz, 2H), 6.52 (d, J = 7.8 Hz, 1H), 5.85 (d, J = 4.0 Hz, 1H), 5.30 (d, J = 4.9 Hz, 2H), 5.04 (s, 2H), 4.85 (d, J = 7.2 Hz, 1H), 3.94 (s, 3H), 3.87 (d, J = 8.9 Hz, 1H), 3.80-3.69 (m, 1H), 3.63 (s, 3H), 3.54 (t, J = 6.4 Hz, 4H), 3.50-3.35 (m, 10H), 3.27 (s, 1H), 2.99 (s, 3H), 2.78 (d, J = 21.7 Hz, 1H), 2.55 (dd, J = 14.2, 7.2 Hz, 4H), 2.31-2.19 (m, 3H), 2.18-2.05 (m, 1H).

### Example 1.3.8: Compound II-D5

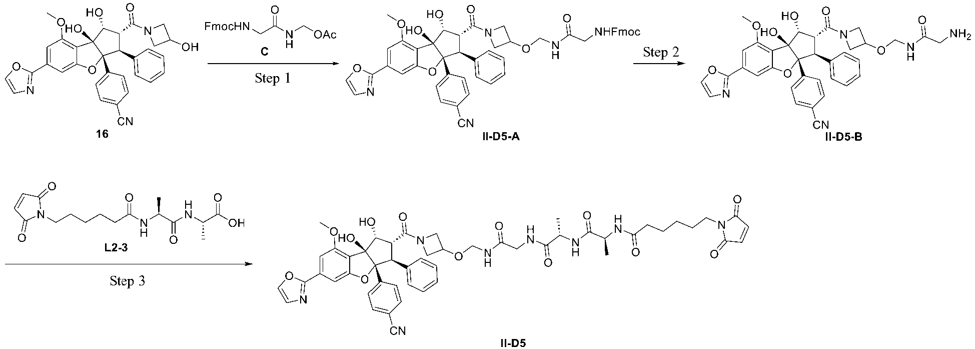

Step 1: Compound 16 (18.0 mg, 0.03 mmol), compound C (58.6 mg, 0.16 mmol), and TsOH (2.74 mg, 0.02 mmol) were added to THF (1 mL), and the mixture was stirred for 2 days and purified by flash chromatography (ACN/H₂O) to give compound II-D5-A (14.0 mg, yield: 50.3%) as a white solid. LCMS [M+H]⁺ = 874.1.

Step 2: Compound II-D5-A (14.0 mg, 0.02 mmol) and piperidine (5.5 mg, 0.06 mmol) were added to DMF (1 mL), and the mixture was stirred for 15 min and purified by flash chromatography (ACN/H₂O) to give compound II-D5-B (9.0 mg, yield: 90%) as a white solid. LCMS [M+1]⁺ = 652.0.

Step 3: Compound II-D5-B (3.0 mg, 0.00 mmol), compound L2-3 (1.7 mg, 0.00 mmol), HATU (2.1 mg, 0.01 mmol), HOAT (0.75 mg, 0.01 mmol), and TMP (1.67 mg, 0.01 mmol) were added to DMF (1.0 mL), and the mixture was stirred for 16 h and purified by flash chromatography (ACN/H₂O) to give II-D5 (1.0 mg, yield: 22.0%) as a white solid. LCMS [M+H]⁺ = 987.1.

The following compounds were synthesized with reference to the synthesis method for compound II-D5.

| No. | Structure | LCMS [M+H]⁺ |
|---|---|---|
| II-D4 | 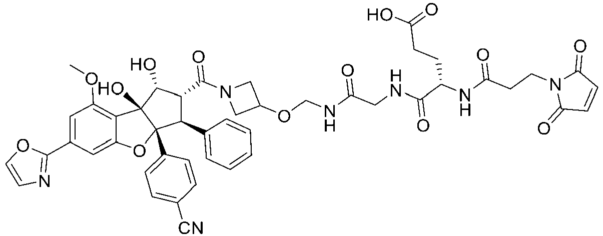 | 932.3 |
| II-D9 | 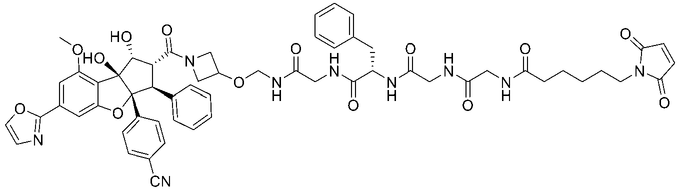 | 1106.4 |

### Reference linker-payload 1

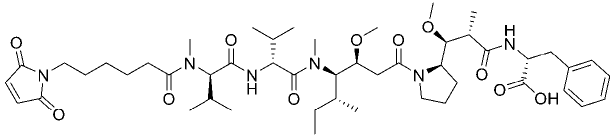

Reference linker-payload 1 was purchased from MCE, CAS: 863971-19-1.

### Reference linker-payload 2

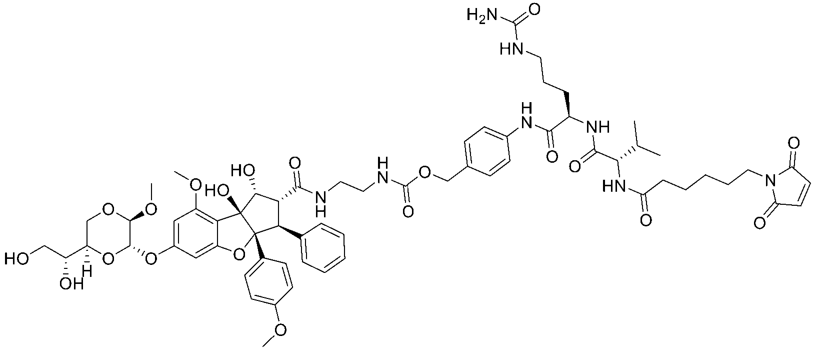

Reference linker-payload 2 was synthesized according to Patent No. WO2017214024A1.

### Reference linker-payload 3

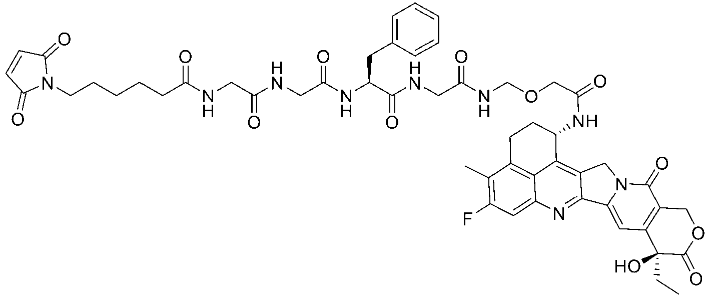

Reference linker-payload 3 was purchased from MCE, CAS: 1599440-13-7.

### Reference linker-payload 4

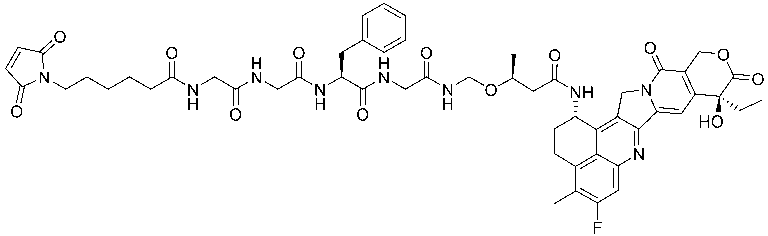

Reference linker-payload 4 was synthesized according to Patent No. WO2022068878A1.

### Example 2: Synthesis of Ligand-Drug Conjugates

In the present application, the 20 mM aqueous histidine buffer solution (pH 5.5) was obtained by adjusting the pH of a 20 mM aqueous histidine solution to 5.5 with HOAc. Stock solution 1: a 10 mM EDTA buffer, pH = 4.7; stock solution 2: an 800 mM aqueous sodium citrate buffer solution, pH = 8.4; stock solution 3: a 500 mM aqueous urea solution; stock solution 4: a 10 mM aqueous tris(2-carboxyethyl)phosphine solution.

The protein purity of the ADCs in the present application was determined by SEC. The parameters are shown in Table 2 below.

**Table 2**

| Instrument | | Agilent Technologies 1260 |
|---|---|---|
| Chromatography column | | TOSON - G3000SWXL, 300*7.8mm, 5µm |
| Column temperature | | 22 °C |
| Wavelength | | 280 nm |
| Injection volume | | 30 ~ 50 µg |
| Mobile phase | 0.2 M K₂HPO₄/KH₂PO₄, 0.25 M KCl, 15% (v/v) 2-propanol, pH 7.0 | |
| Gradient | Time (min) | Flow rate (mL/min) |
| | 0.0 | 0.75 |
| | 18.0 | 0.75 |

The DAR values of the ADCs in the present application can be determined by LC-MS. The experimental procedure was as follows: 75.0 µL of 8.0 mol/L guanidine hydrochloride (Gdn-HCl), 5.0 µL of 1.0 mol/L Tris-HCl, and 2.0 µL of 1 mol/L DTT were added to a solution of 40.0 µg of an ADC sample. The solution was diluted to 100.0 µL with ultrapure water, mixed well, and incubated at 22 °C for 30 min. The average drug/antibody ratio (DAR) was then analyzed using LC-MS. The LC parameters are shown in Table 3 below.

**Table 3**

| | | | | |
|---|---|---|---|---|
| Instrument | Agilent Technologies 1260 | | | |
| Reversed-phase chromatography column | Agilent, PLRP-S 2.1*50 mm, 8 µm | | | |
| Column temperature | 80°C | | | |
| Sample temperature | 2~8°C | | | |
| Detection wavelength | 280nm | | | |
| Injection volume | 10.0 µL | | | |
| Mobile phases | Mobile phase A: 0.025% TFA, and ultrapure water containing 0.1% FA | | | |
| | Mobile phase B: 0.025% TFA, and ACN containing 0.1% FA | | | |
| Gradient | Time (min) | %A | %B | Flow rate (mL/min) |
| | 0.0 | 75 | 25 | 0.5 |
| | 0.0 | 75 | 25 | |
| | 0.7 | 66 | 34 | |
| | 5.0 | 55 | 45 | |
| | 6.0 | 10 | 90 | |
| | 7.0 | 10 | 90 | |
| | 7.1 | 75 | 25 | |
| | 10.0 | 75 | 25 | |

The MS parameters are shown in Table 4 below.

**Table 4**

| Instrument | Agilent Technologies 6224 TOF LC/MS |
|---|---|
| Ionization mode | Positive |
| Gas temperature | 350 °C |
| Gas flow | 13 L/min |
| Atomizer pressure | 45 psig |
| Capillary voltage | 5000 V |
| Fragmentation voltage | 250 V |
| Cone voltage | 65 V |
| Peak-to-peak value of radio frequency voltage on octupole | 750 V |
| Acquisition mode | MS (seg) |
| Mass range | 500-8000 m/z |
| Acquisition rate | 1 spectra/s |
| Acquisition time | 1.4-7.0 min |

The DAR values of the ADCs in the present application can also be determined by HIC. The parameters are shown in Table 5 below.

**Table 5**

| | | | |
|---|---|---|---|
| Chromatography column | TSKgel Butyl-NPR, 4.6mm *3.5cm, 2.5µm | | |
| Column temperature | 25 °C | | |
| Detection wavelength | 280 nm | | |
| Injection volume | 8 µL | | |
| Mobile phases | Mobile phase A: 1.5 M (NH₄)₂SO₄, 0.05 M K₂HPO₄.3H₂O (pH = 7.0) | | |
| | Mobile phase B: 21.3 mM KH₂PO₄, 28.6 mM K₂HPO₄.3H₂O, 25% (V/V) IPA (pH = 7.0) | | |

| Gradient | Time (min) | % B | Flow rate (mL/min) |
|---|---|---|---|
| | 0.0 | 0.0 | |
| | 2.0 | 0.0 | |
| | 15.0 | 100.0 | 0.6 |
| | 16.0 | 100.0 | |
| | 17.0 | 0.0 | |
| | 20.0 | 0.0 | |

The antibodies used as the ligands were prepared according to a conventional method. For example, vectors can be constructed and then transfected into eukaryotic cells, such as HEK293 cells and CHO cells, and the antibodies are expressed and purified. Ligand-drug conjugates were prepared with the anti-CEACAM5 antibody tusamitamab (prepared with reference to WO2014079886), anti-HER3 antibody DB1001 (patritumab, prepared with reference to WO2007077028A1), anti-GPC-3 antibody DB1002 (prepared with reference to WO2006006693), anti-ADAM9 antibody DB1003 (prepared with reference to WO2024199111), anti-BCMA antibody DB1004 (prepared with reference to US9273141), and anti-c-MET antibody DB1005 (prepared with reference to US20160161500A1).

The amino acid sequence of the heavy chain of the antibody tusamitamab is set forth in SEQ ID NO: 1, and the amino acid sequence of its light chain is set forth in SEQ ID NO: 2. The amino acid sequence of the heavy chain of anti-HER3 antibody DB1001 is set forth in SEQ ID NO: 3, and the amino acid sequence of its light chain is set forth in SEQ ID NO: 4. The amino acid sequence of the heavy chain of anti-GPC-3 antibody DB1002 is set forth in SEQ ID NO: 5, and the amino acid sequence of its light chain is set forth in SEQ ID NO: 6. The amino acid sequence of the heavy chain of anti-ADAM9 antibody DB1003 is set forth in SEQ ID NO: 25, and the amino acid sequence of its light chain is set forth in SEQ ID NO: 26. The amino acid sequence of the heavy chain of anti-BCMA antibody DB1004 is set forth in SEQ ID NO: 35, and the amino acid sequence of its light chain is set forth in SEQ ID NO: 36. The amino acid sequence of the heavy chain of anti-c-MET antibody DB1005 is set forth in SEQ ID NO: 15, and the amino acid sequence of its light chain is set forth in SEQ ID NO: 16.

### Example 2.1: Preparation of Conjugate II-E6

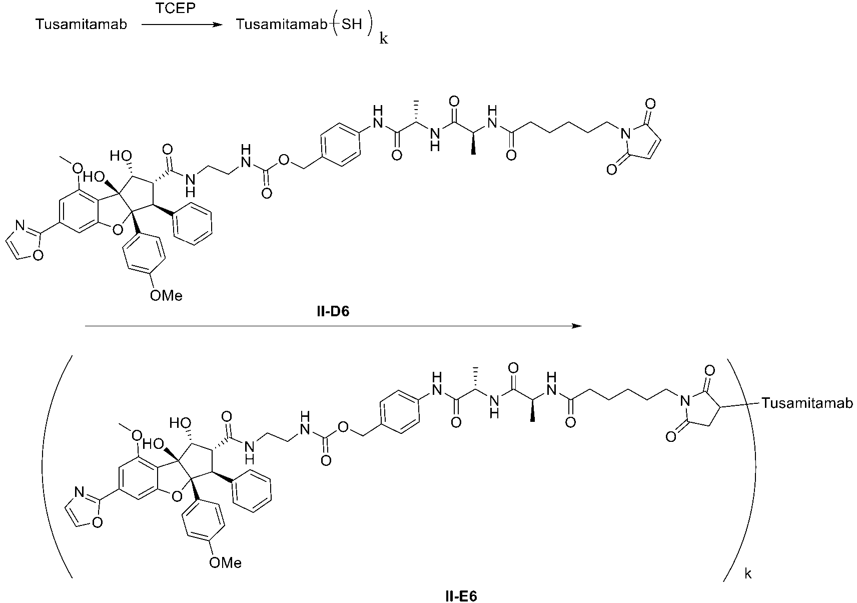

A prepared aqueous tris(2-carboxyethyl)phosphine solution (10 mM, 0.040 mL, 0.400 µmol) was added to a solution of the antibody tusamitamab in an aqueous PBS buffer solution (0.2 mL, 15 mg/mL, 0.020 µmol) at 22 °C. The mixture was left to react in a water bath shaker at 22 °C for 3 h, and the reaction was stopped.

II-D6 (0.31 mg, 0.30 µmol) was dissolved in 0.03 mL of DMAc, and the resulting solution was added to the above solution. The mixture was left to react in a water bath shaker at 22 °C for 3 h, and the reaction was stopped. The reaction solution was purified with activated carbon (eluent phase: a 0.02 M aqueous histidine/hydrochloric acid buffer solution, pH 5.5) to give conjugate II-E6 (1.63 mg, 3.20 mg/mL). Yield: 54%.

The DAR value was 7.91, as determined by LC-MS.

The following compounds were synthesized with reference to the method for conjugate II-E6.

| No. | Structure | DAR value |
|---|---|---|
| I-E1 | 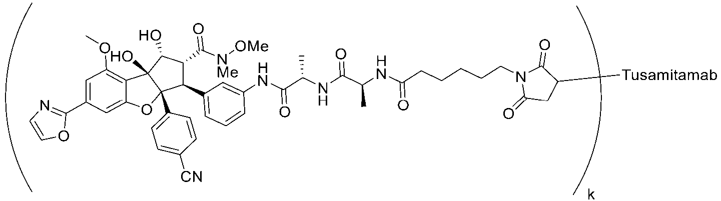 | 7.92 |
| I-E3 | 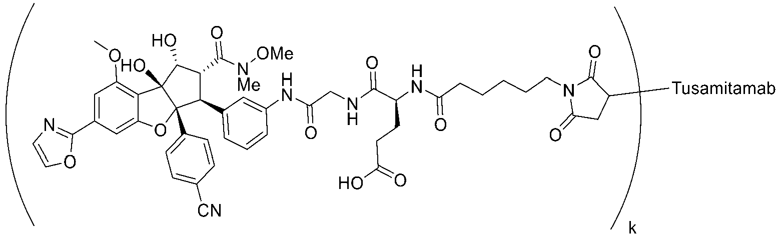 | 8.02 |
| I-E5 | 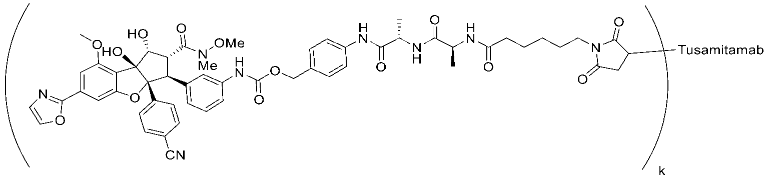 | 7.91 |
| I-E16 | 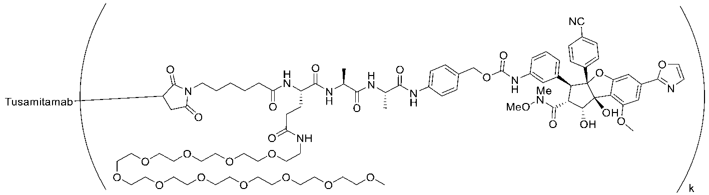 | 7.78 |
| I-E17 | 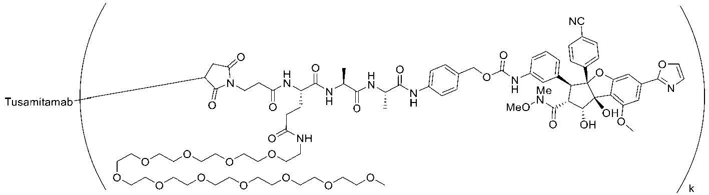 | 7.87 |
| I-E19 | 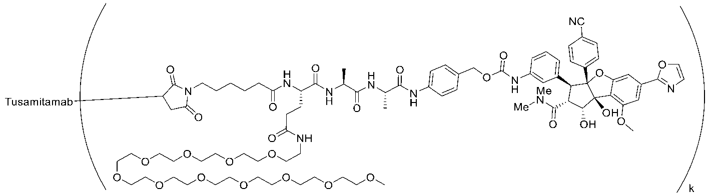 | 7.69 |
| I-E20 | 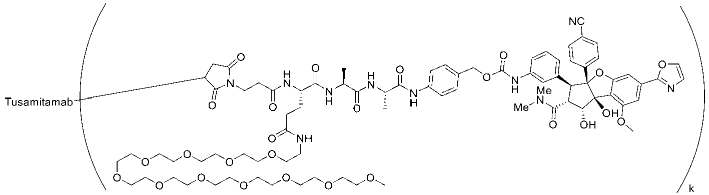 | 7.76 |
| I-E27 | 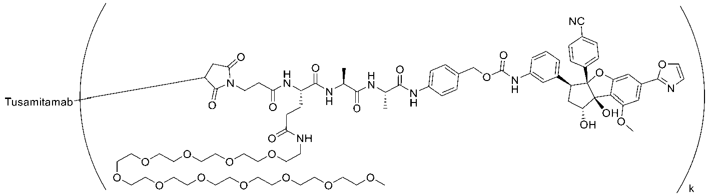 | 7.84 |
| I-E43 | 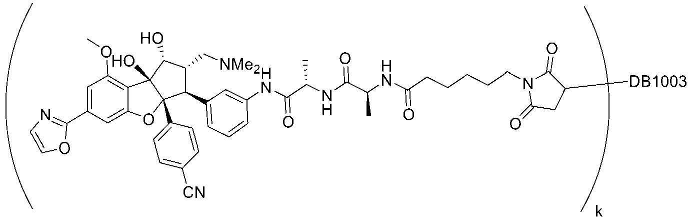 | 7.97 |
| I-E48 | 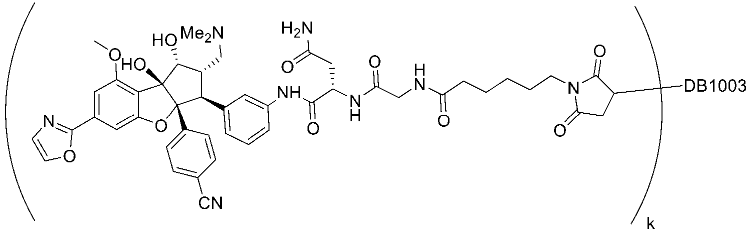 | 7.89 |
| I-E58 | 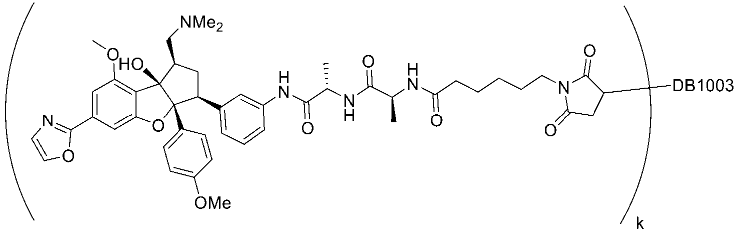 | 7.96 |
| I-E62 | 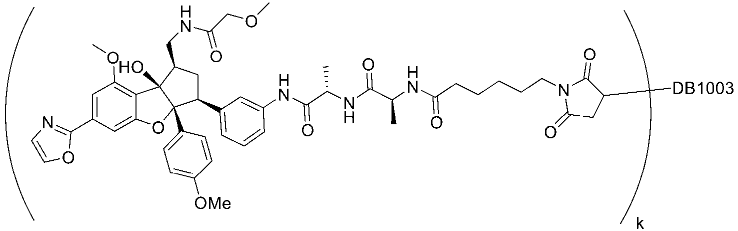 | 8.00 |
| I-E63 | 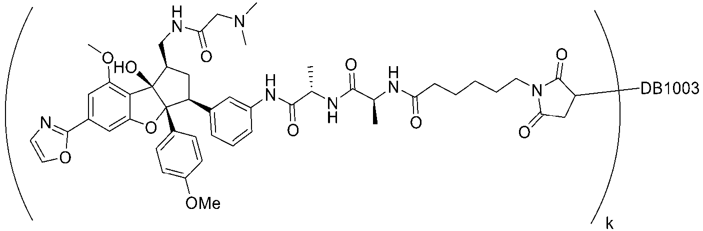 | 7.99 |
| I-E64 | 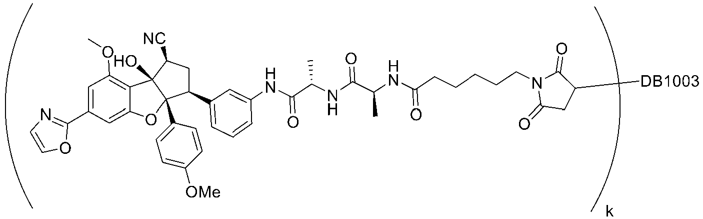 | 7.88 |
| I-E65 | 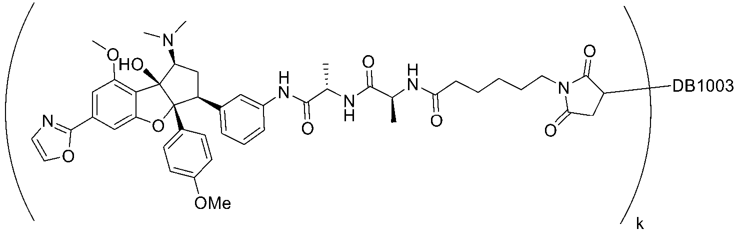 | 7.91 |
| I-E68 | 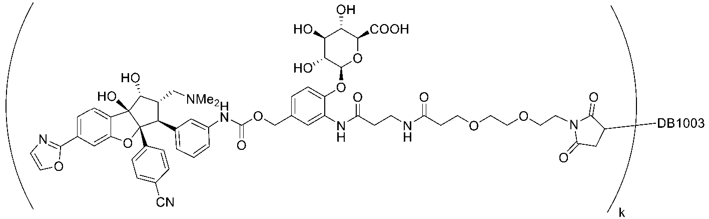 | 7.94 |
| I-E76 | 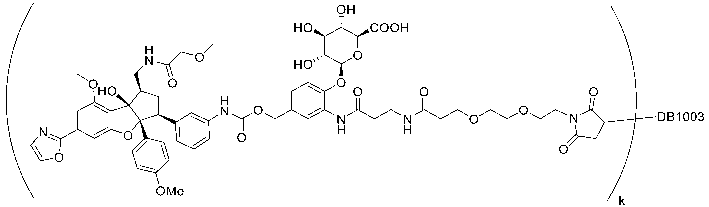 | 7.87 |
| I-E77 | 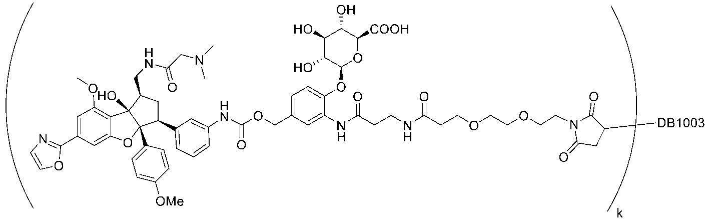 | 7.81 |
| I-E78 | 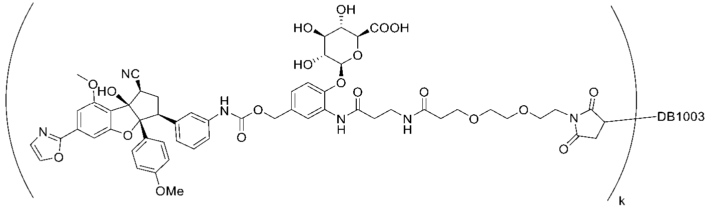 | 7.98 |
| I-E79 | 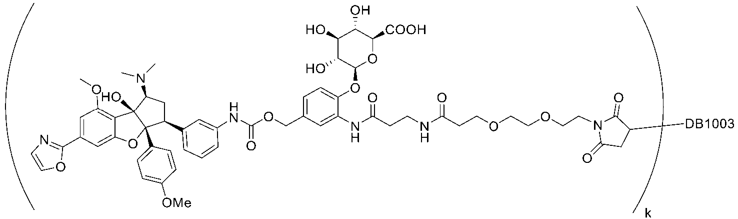 | 7.90 |
| I-E86 | 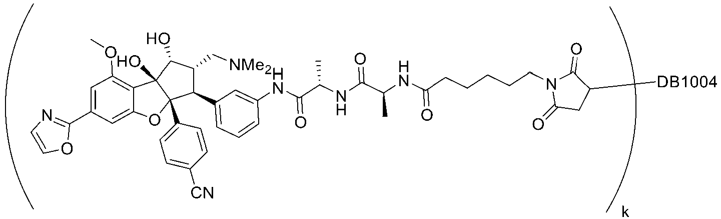 | 7.83 |
| I-E87 | 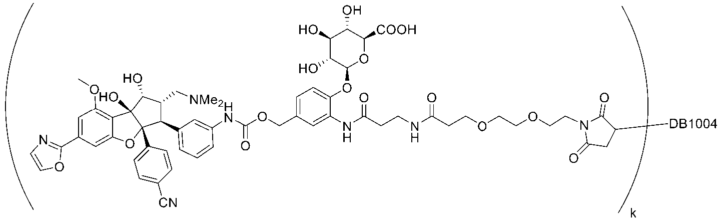 | 8.03 |
| I-E88 | 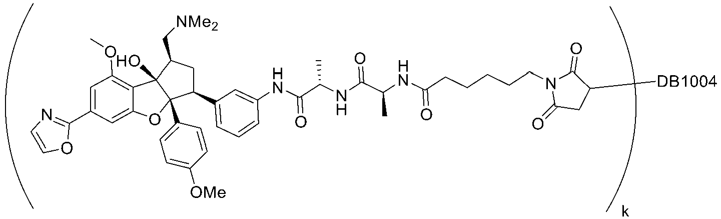 | 7.95 |
| I-E89 | 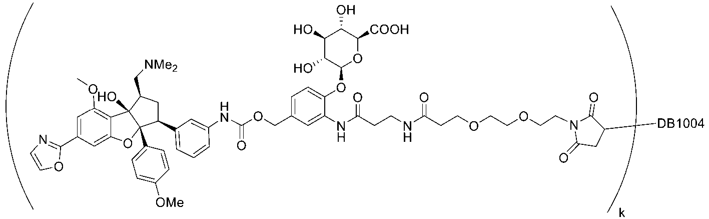 | 7.89 |
| I-E90 | 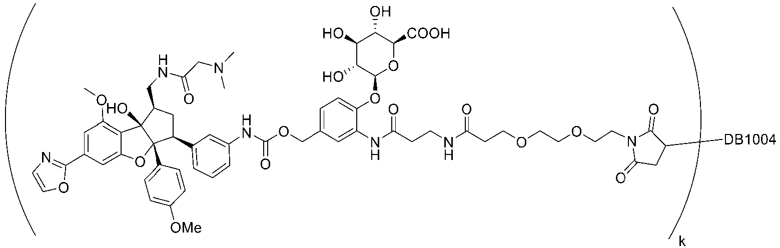 | 7.87 |
| I-E91 | 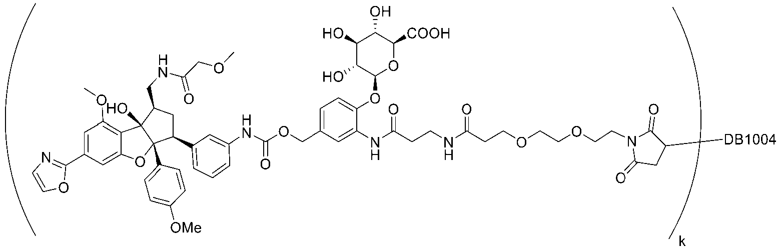 | 7.95 |
| I-E92 | 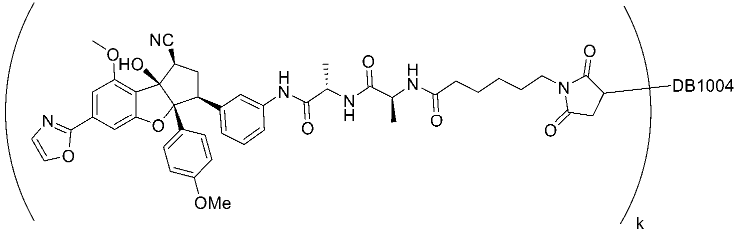 | 7.96 |
| I-E93 | 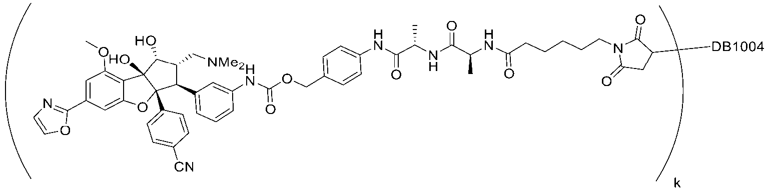 | 7.96 |
| I-E94 | 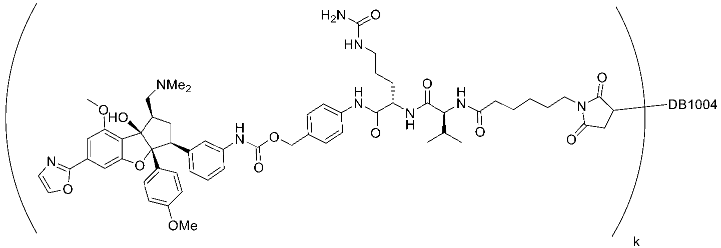 | 8.01 |
| I-E95 | 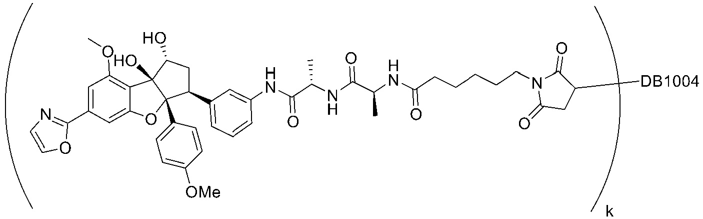 | 7.88 |
| I-E96 | 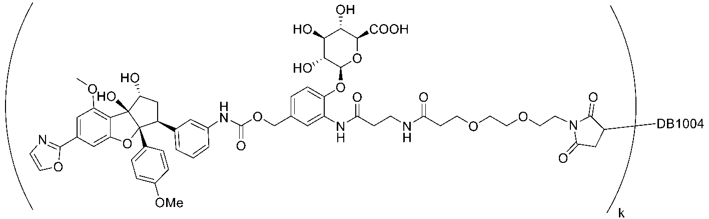 | 7.94 |
| I-E97 | 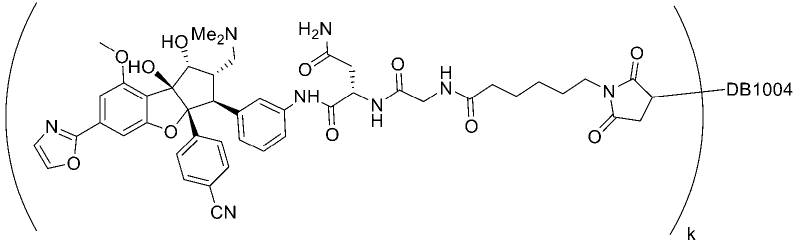 | 8.00 |
| I-E98 | 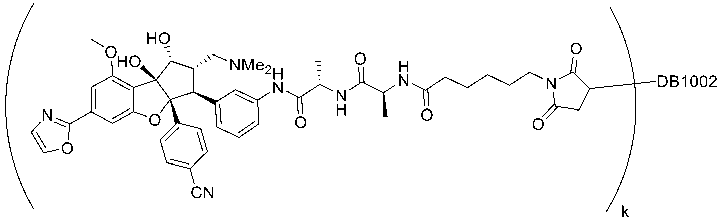 | 7.96 |
| I-E99 | 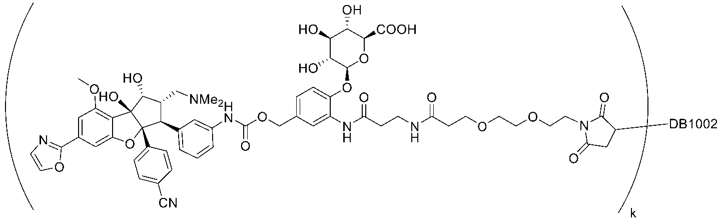 | 7.83 |
| I-E100 | 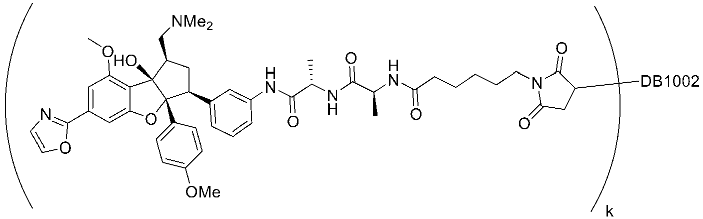 | 7.99 |
| I-E101 | 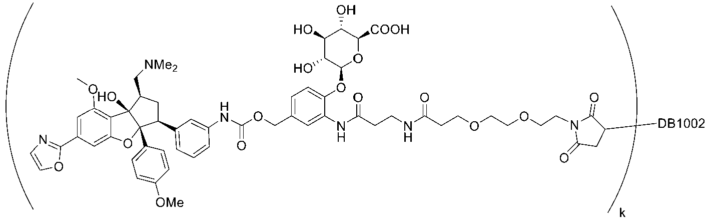 | 7.97 |
| I-E102 | 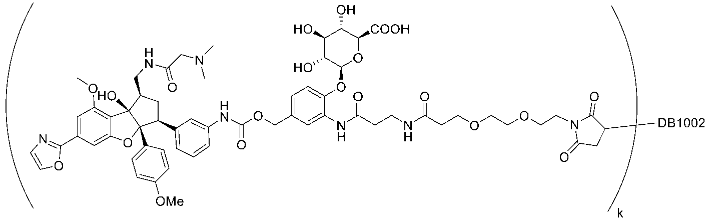 | 8.02 |
| II-E3 | 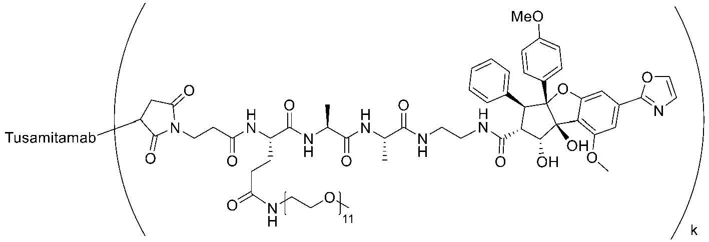 | 7.89 |
| II-E5 | 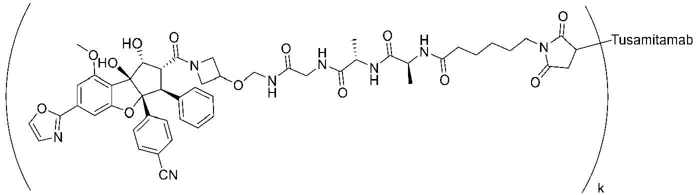 | 7.91 |
| III-E3 | 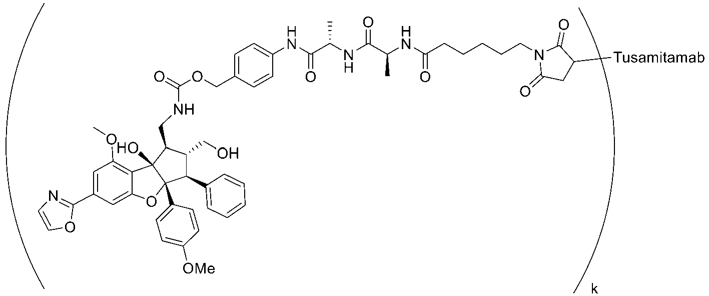 | 7.75 |
| I-E70 | 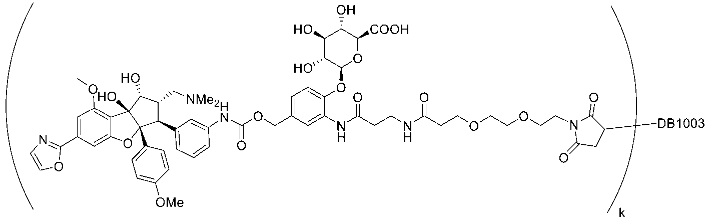 | 7.98 |
| I-E72 | 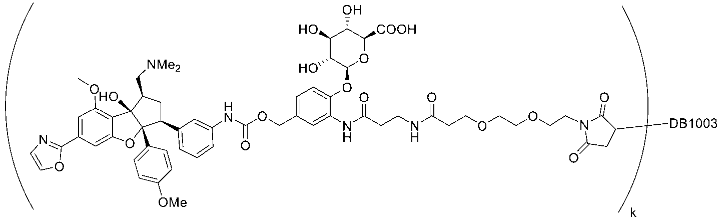 | 8.02 |
| I-E103 | 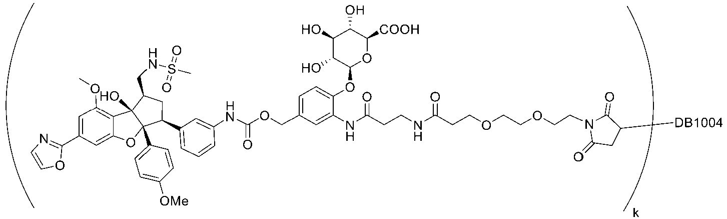 | 7.85 |
| I-E104 | 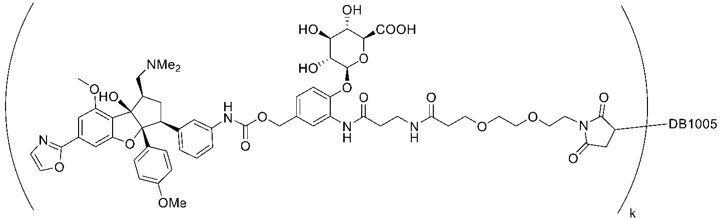 | 7.49 |
| I-E105 | 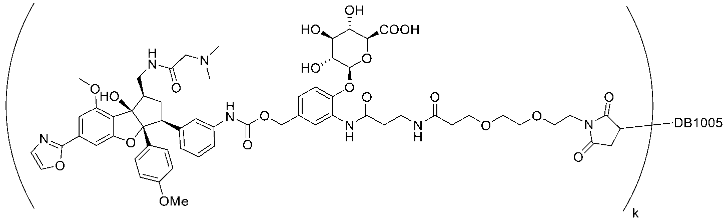 | 7.88 |
| I-E106 | 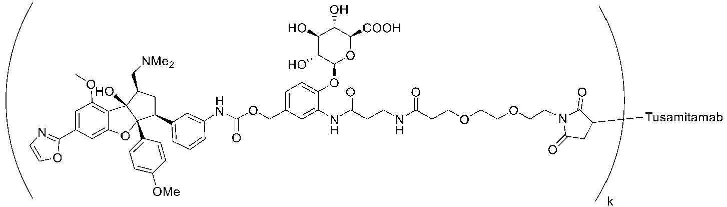 | 7.83 |
| I-E107 | 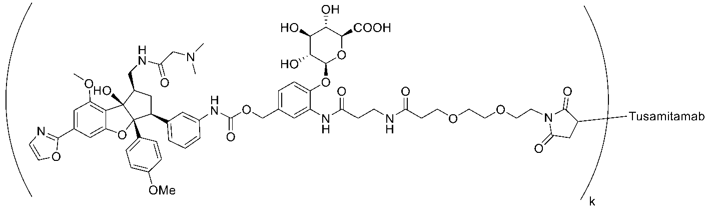 | 7.92 |
| I-E109 | 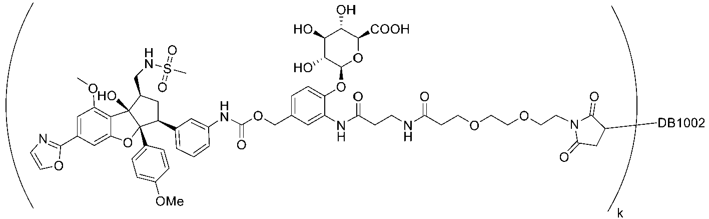 | 7.86 |
| Control ADC1 | 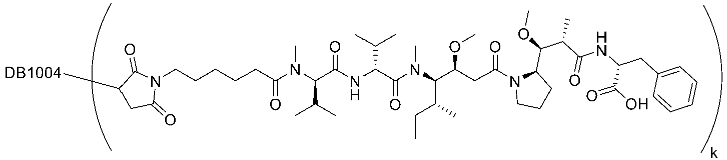 | 4.36 |
| Control ADC2 | 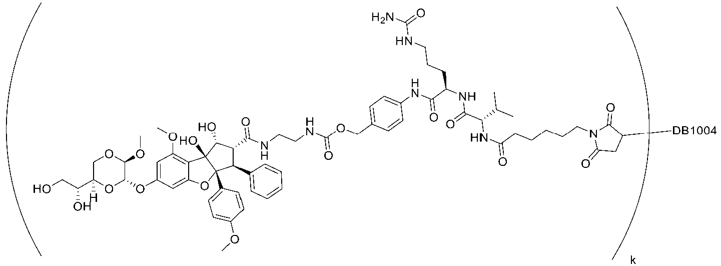 | 3.85 |
| Control ADC3 | 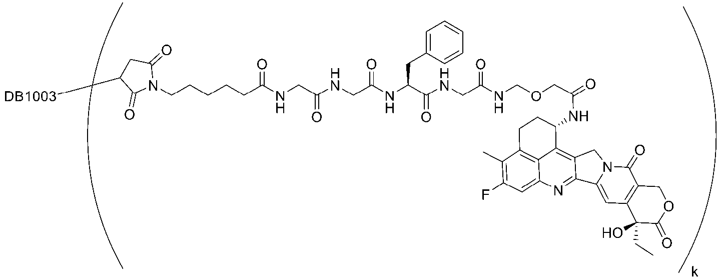 | 7.99 |
| Control ADC4 | 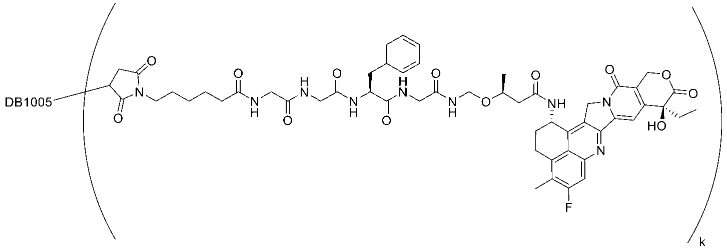 | 7.46 |

### Example 2.2: Preparation of Conjugate II-E2

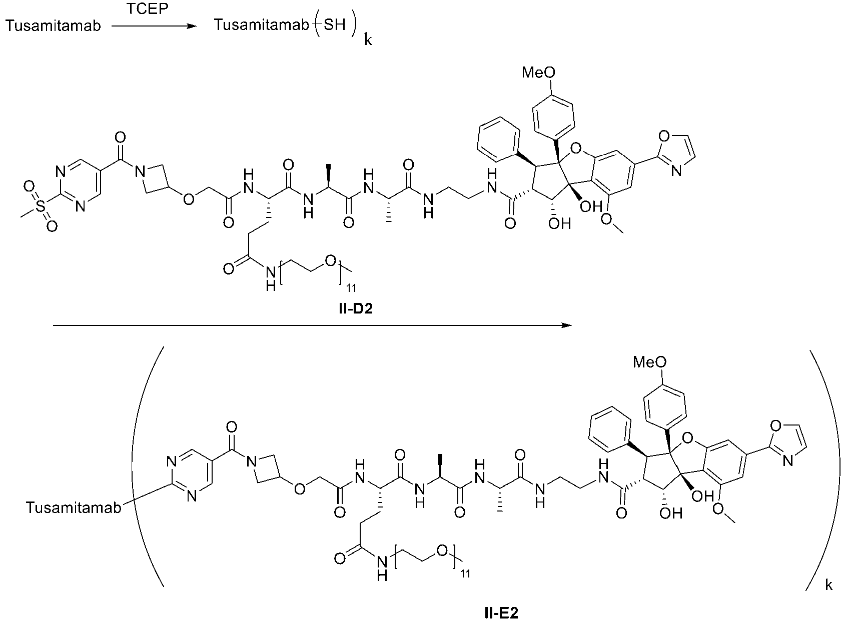

Stock solution 1 was added to a solution of the antibody tusamitamab in an aqueous buffer solution (0.02 M His-HCl, pH = 6.0), so that the buffer system contained 2 mM EDTA.

A prepared aqueous tris(2-carboxyethyl)phosphine solution (10 mM, 0.070 mL, 0.705 µmol) was added to a solution of the antibody tusamitamab in an aqueous buffer solution (0.47 mL, 15 mg/mL, 0.047 µmol) at 22 °C. The mixture was left to react in a water bath shaker at 22 °C for 3 h, and the reaction was stopped.

Compound II-D2 (0.92 mg, 0.564 µmol) was dissolved in 0.09 mL of DMAc, and the resulting solution was added to the above solution. The mixture was left to react in a water bath shaker at 22 °C for 3 h, and the reaction was stopped. The reaction solution was purified with activated carbon and an Amicon^{®} Ultra ultrafiltration centrifuge tube (4 mL, 10K) (mobile phase: a 0.02 M aqueous histidine/hydrochloric acid buffer solution, pH 5.5) to give conjugate II-E2 (3.8 mg, 3.82 mg/mL). Yield: 54%.

The DAR value was 7.75, as determined by LC-MS.

The following compounds were synthesized with reference to the method for conjugate II-E2.

| No. | Structure | DAR value |
|---|---|---|
| I-E18 | 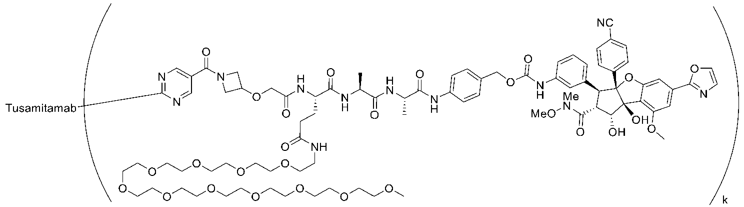 | 7.96 |
| I-E21 | 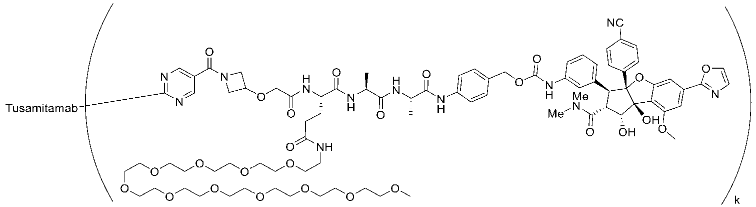 | 7.85 |
| I-E28 | 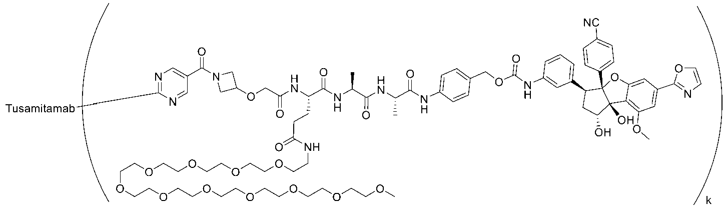 | 7.82 |

### Example 3: Inhibition of In Vitro Proliferation of Tumor Cells by Small Molecule Compounds (Toxins)

Objective: To assess the inhibitory effects of the drug compounds on tumor cell proliferation, for example, their inhibitory activity against the *in vitro* proliferation of NCI-H716, NCI-H1975, NCI-H1373, NCI-H647, NCI-H520, BT474, DLD-1, NCI-N87, NUGC-4, SNU-423, MKN-45, SK-OV-3, OVCAR3, Ls174T, HCC1954, and ES2 tumor cells. The cells were treated with the compounds at different concentrations *in vitro.* After 5 days of culture, the proliferation of the cells was assessed using the CTG (CellTiter-Glo^{®} Luminescent Cell Viability Assay, Promega, Cat. No. G7558) reagent, and the *in vitro* activity of the compounds was evaluated based on their IC₅₀ values.
1. Cell suspension preparation: NCI-H716, NCI-H1975, NCI-H1373, NCI-H647, NCI-H520, BT474, DLD-1, NCI-N87, NUGC-4, SNU-423, MKN-45, SK-OV-3, OVCAR3, Ls174T, HCC1954, and ES2 cells in the log phase were collected, washed with PBS once, and then digested with 2-3 mL of trypsin for 2-3 min. After the cells were digested completely, 10-15 mL of a cell culture medium was added to wash off the digested cells. The cells were centrifuged at 1000 rpm for 5 min, and the supernatant was discarded. Subsequently, the cells were resuspended in 10-20 mL of the cell culture medium to prepare single-cell suspensions.
2. Cell plating: The BT474, DLD-1, NCI-N87, NUGC-4, SNU-423, MKN-45, SK-OV-3, Ls174T, and HCC1954 single-cell suspensions were each mixed well, and their viable cell density was adjusted to 6 × 10⁴ cells/mL with the cell culture medium. After the density adjustment, the resulting cell suspensions were each mixed well and added to 96-well cell culture plates at 50 µL/well. The culture plates were incubated overnight in an incubator (37 °C, 5% CO₂).
3. Compound solution preparation: The compounds were dissolved in DMSO to prepare stock solutions with an initial concentration of 10 mM. For the small molecule compounds, a total of 6 concentrations were prepared: 1000 nM, 333 nM, 111 nM, 37.03 nM, 12.34 nM, and 4.11 nM.
4. Sample addition: The prepared test samples with different concentrations were added to the culture plates, with two replicate wells set for each sample. The culture plates were incubated in an incubator for 5 days (37 °C, 5% CO₂).
5. Color development: The 96-well cell culture plates were taken out, 50 µL of the CTG reagent was added to each well, and the plates were incubated at room temperature for 10 min.
6. Plate reading: The 96-well cell culture plates were taken out and placed in a microplate reader, and the chemiluminescence was measured using a microplate reader.
7. Data analysis: The cell survival rates were calculated according to the formula: (Lum_{test drug} - Lum_{blank control})/(Lum_{solvent blank control} - Lum_{blank control}) × 100%. Sigmoid dose-survival rate curves were plotted using a nonlinear regression model in GraphPad Prism software, and the IC50 values were calculated. The results are shown in Table 7-1.

**Table 7-1. IC₅₀ values for the inhibition of the in vitro proliferation of tumor cells by the small molecule compounds of the present application**

| Compound No. | Proliferation inhibitory activity IC₅₀ (nM) | | | | | | | | |
|---|---|---|---|---|---|---|---|---|---|
| | BT474 | DLD-1 | NCI-N87 | NUGC-4 | SNU-423 | MKN-45 | SK-OV-3 | Ls174T | HCC19 54 |
| Rocaglamide | 5.4861 | 8.1856 | 9.3239 | 8.2530 | 14.4404 | 5.7543 | 8.5247 | 6.0846 | 2.6620 |
| Racemate of control compound 2 | >100 | >100 | / | / | / | / | / | / | / |
| Racemate of compound 2 | 3.3268 | / | 2.0679 | 1.9845 | 3.6709 | / | 2.0485 | / | / |
| Racemate of compound 3 | / | / | 3.6290 | 3.5126 | 8.1778 | / | / | / | / |
| Racemate of compound 4 | 3.0366 | / | / | / | / | 1.4044 | / | / | / |
| Racemate of compound 8 | / | 5.0368 | / | / | / | 2.1198 | / | / | / |
| Racemate of compound 9 | 0.8287 | / | / | / | / | 0.6949 | / | / | / |
| Racemate of compound 11 | / | / | / | / | / | 1.4427 | / | / | / |
| Racemate of compound 14 | / | / | / | / | / | 0.8174 | / | / | / |
| Racemate of compound 15 | / | / | / | / | / | 2.6460 | / | / | / |
| Racemate of compound 16 | / | / | / | / | / | 2.3389 | / | / | / |
| Racemate of compound 18 | / | / | / | / | / | 1.9069 | / | / | / |
| Racemate of compound 21 | / | / | / | / | / | 1.1019 | / | / | / |
| Racemate of compound 23 | 1.8021 | 1.2848 | 0.8970 | / | / | 1.5023 | / | 1.1043 | / |
| Racemate of compound 29 | / | / | / | / | / | 1.2286 | / | / | / |
| Racemate of compound 30 | / | / | / | / | / | 1.5008 | / | / | / |
| Racemate of compound 31 | / | / | / | / | / | 1.3219 | / | / | / |
| Racemate of compound 32 | / | / | / | / | / | / | / | 5.4461 | / |
| Racemate of compound 35 | / | / | / | / | / | / | / | 3.0302 | / |
| Racemate of compound 41 | 4.649 | / | / | / | / | / | / | / | / |
| Racemate of compound 43 | / | / | / | / | / | 2.0029 | / | / | / |
| Racemate of compound 47 | 4.4376 | / | 2.0096 | / | / | 3.1216 | / | / | / |
| Racemate of compound 48 | 2.2505 | / | / | / | / | / | / | / | / |
| Racemate of compound 49 | 7.887 | / | / | / | / | / | / | / | / |
| Racemate of compound 53 | 4.438 | / | / | / | / | / | / | / | 1.879 |
| Racemate of compound 57 | / | 2.3515 | / | / | / | / | / | / | / |
| Racemate of compound 64 | / | 1.5363 | / | / | / | / | / | / | / |
| Racemate of compound 70 | / | 2.0437 | / | / | / | / | / | / | / |
| Racemate of compound 75 | 4.25 | / | / | / | / | / | / | / | / |
| Racemate of compound 79 | / | 2.486 | / | / | / | / | / | / | 0.7217 |
| Racemate of compound 86 | / | 2.989 | / | / | / | / | / | / | 2.1475 |
| Racemate of compound 87 | / | 2.839 | / | / | / | / | / | / | / |
| Racemate of compound 89 | / | 1.35 | / | / | / | / | / | / | / |
| Racemate of compound 90 | / | 2.747 | / | / | / | / | / | / | / |
| Racemate of compound 91 | / | 2.161 | / | / | / | / | / | / | / |
| Racemate of compound 93 | / | 11.80 | / | / | / | / | / | / | 1.4969 |
| Racemate of compound 97 | / | 3.083 | / | / | / | / | / | / | / |
| Racemate of compound 107 | / | 0.9205 | / | / | / | / | / | / | / |
| Racemate of compound 100 | / | 7.219 | / | / | / | / | / | / | / |
| Racemate of compound 111 | / | / | / | / | / | / | / | / | 2.981 |

| | | | | | | | | | |
|---|---|---|---|---|---|---|---|---|---|
| "/" indicates that no assay was performed. | | | | | | | | | |

**Table 7-2. IC₅₀ values for the inhibition of the in vitro proliferation of tumor cells by the small molecule compounds of the present application**

| Compound No. | Proliferation inhibitory activity IC₅₀ (nM) | |
|---|---|---|
| | BT474 | DLD-1 |
| Control compound 2 | >50 | >50 |
| Compound 4 | 1.29 | <10 |
| Compound 11 | <10 | / |
| Compound 47 | 1.16 | / |
| Compound 49 | 4.85 | / |
| Compound 79 | 1.34 | 1.36 |
| Compound 93 | 1.32 | 3.86 |
| Compound 98 | <10 | <10 |
| Compound 100 | 4.07 | 1.88 |
| Compound 106 | <10 | <10 |

| | | |
|---|---|---|
| "/" indicates that no assay was performed. | | |

Conclusion: According to the results in Tables 7-1 and 7-2,the small molecule compounds of the present application exhibited significantly enhanced proliferation inhibitory activity against BT474, DLD-1, NCI-N87, NUGC-4, SNU-423, MKN-45, SK-OV-3, Ls174T, and HCC1954.

### Example 4: Inhibition of In Vitro Proliferation of Tumor Cells by Ligand-Drug Conjugates

### Example 4.1: Inhibition of In Vitro Proliferation of Tumor Cells by ADCs

The cell proliferation inhibitory effects of anti-Her3 ADCs on the cancer cells with positive Her3 expression, NCI-H1975, NCI-N87, BT474, FADU, MDA-MB-468, and MCF-7, were assessed using the CellTiter-Glo^{®} chemiluminescence cell viability assay (i.e., the CTG method).

Cells in the log phase were collected and plated at a density of 5000 cells/well. The cell plates were incubated overnight in an incubator at 37 °C with 5% CO₂. On day two of the experiment, the test samples were diluted 3-fold with the complete culture medium to obtain 8 concentration gradients (the 3-fold dilution started from the highest concentration 2000 nM) and then added to the cell culture plates at 50 µL/well. The complete culture medium was used as a blank control, and 2 replicate wells were set. The plates were incubated in the incubator at 37 °C with 5% CO₂ for another 7 days. After the incubation, the cell culture plates were taken out and equilibrated to room temperature. Subsequently, the CTG assay reagent (Promega, Cat#: G7573) was added at 50 µL/well. The plates were shaken for thorough mixing and then left to stand in a dark place for 10 min, and the signal values were read using a microplate reader. Sigmoid dose-response curves were plotted using a nonlinear regression model in GraphPad Prism software, and the IC₅₀ values were calculated. The cell survival rates were calculated according to the formula: (Lum_{test drug} - Lum_{blank control})/(Lum_{solvent blank} control - Lum_{blank} control) × 100%.

Conclusion: The ligand-drug conjugates of the present application exhibited significant proliferation inhibitory activity against NCI-H1975, NCI-N87, BT474, FADU, MDA-MB-468, and MCF-7 cells.

### Example 4.2: Inhibition of In Vitro Proliferation of Tumor Cells by ADCs

The cell proliferation inhibitory effects of anti-GPC-3 ADCs on the cancer cells with positive GPC-3 expression, HepG2, Huh7, and SK-HEP-1, were assessed using the CellTiter-Glo^{®} chemiluminescence cell viability assay (i.e., the CTG method).

Cells in the log phase were collected and plated at a density of 5000 cells/well. The cell plates were incubated overnight in an incubator at 37 °C with 5% CO₂. On day two of the experiment, the ADCs were diluted 3-fold with the complete culture medium to obtain 8 concentration gradients (the 2-fold dilution started from the highest concentration 2000 nM) and then added to the cell culture plates at 50 µL/well. The complete culture medium was used as a blank control, and 2 replicate wells were set. The plates were incubated in the incubator at 37 °C with 5% CO₂ for another 7 days. After the incubation, the cell culture plates were taken out and equilibrated to room temperature. Subsequently, the CTG assay reagent (Promega, Cat#: G7573) was added at 50 µL/well. The plates were shaken for thorough mixing and then left to stand in a dark place for 10 min, and the signal values were then read using a microplate reader. Sigmoid dose-response curves were plotted using a nonlinear regression model in GraphPad Prism software, and the IC₅₀ values were calculated. The cell survival rates were calculated according to the formula: (Lum_{test drug} - Lum_{blank control})/(Lum_{solvent blank} control - Lum_{blank} control) × 100%.

Conclusion: The ligand-drug conjugates of the present application exhibited significant proliferation inhibitory activity against HepG2, Huh7, and SK-HEP-1 cells.

### Example 4.3: Inhibition of In Vitro Proliferation of Tumor Cells by ADCs

The cell proliferation inhibitory effects of anti-ADAM9 ADCs on the cancer cells with positive ADAM9 expression, Calu3, LS174T, and Colo205, were assessed using the CellTiter-Glo^{®} chemiluminescence cell viability assay (i.e., the CTG method).

Cells in the log phase were collected and plated at a density of 5000 cells/well. The cell plates were incubated overnight in an incubator at 37 °C with 5% CO₂. On day two of the experiment, the ADCs were diluted 3-fold with the complete culture medium to obtain 8 concentration gradients (the 2-fold dilution started from the highest concentration 2000 nM) and then added to the cell culture plates at 50 µL/well. The complete culture medium was used as a blank control, and 2 replicate wells were set. The plates were incubated in the incubator at 37 °C with 5% CO₂ for another 7 days. After the incubation, the cell culture plates were taken out and equilibrated to room temperature. Subsequently, the CTG assay reagent (Promega, Cat#: G7573) was added at 50 µL/well. The plates were shaken for thorough mixing and then left to stand in a dark place for 10 min, and the signal values were then read using a microplate reader. Sigmoid dose-response curves were plotted using a nonlinear regression model in GraphPad Prism software, and the IC₅₀ values were calculated. The cell survival rates were calculated according to the formula: (Lum_{test drug} - Lum_{blank control})/(Lum_{solvent blank} control - Lum_{blank} control) × 100%.

Conclusion: The ligand-drug conjugates of the present application exhibited significant proliferation inhibitory activity against Calu3, LS174T, and Colo205 cells.

### Example 4.4: Inhibition of In Vitro Proliferation of Tumor Cells by ADCs

The cell proliferation inhibitory effects of anti-CEACAM5 ADCs on cancer cells with positive CEACAM5 expression, such as MKN45, SNU16, and LS174T, were assessed using the Cell Titer-Glo^{®} chemiluminescence cell viability assay (i.e., the CTG method).

Cells in the log phase were collected and plated at a density of 5000 cells/well. The cell plates were incubated overnight in an incubator at 37 °C with 5% CO₂. On day two of the experiment, the ADCs were diluted 3-fold with the complete culture medium to obtain 8 concentration gradients (the 2-fold dilution started from the highest concentration 2000 nM) and then added to the cell culture plates at 50 µL/well. The complete culture medium was used as a blank control, and 2 replicate wells were set. The plates were incubated in the incubator at 37 °C with 5% CO₂ for another 7 days. After the incubation, the cell culture plates were taken out and equilibrated to room temperature. Subsequently, the CTG assay reagent (Promega, Cat#: G7573) was added at 50 µL/well. The plates were shaken for thorough mixing and then left to stand in a dark place for 10 min, and the signal values were then read using a microplate reader. Sigmoid dose-response curves were plotted using a nonlinear regression model in GraphPad Prism software, and the IC₅₀ values were calculated. The cell survival rates were calculated according to the formula: (Lum_{test drug} - Lum_{blank control})/(Lum_{solvent blank} control - Lum_{blank} control) × 100%.

The proliferation inhibitory effects on MKN45 cells are shown in Table 8 below:

**Table 8.IC₅₀ values for the inhibition of the in vitro proliferation of tumor cells by the ligand-drug conjugates of the present application**

| Compound | IC₅₀(nM) |
|---|---|
| I-E1 | 0.84 |
| I-E3 | 1.24 |
| III-E3 | 0.35 |
| I-E5 | 1.27 |
| II-E5 | 0.49 |
| II-E6 | 0.26 |

Conclusion: The ligand-drug conjugates of the present application exhibited significant proliferation inhibitory activity against MKN45, SNU16, and LS174T cells.

### Example 4.5: Inhibition of In Vitro Proliferation of Tumor Cells by ADCs

The cell proliferation inhibitory effects of anti-BCMA ADCs on the cancer cells with positive BCMA expression, NCI-H929 and MM.1R, were assessed using the CellTiter-Glo^{®} chemiluminescence cell viability assay (i.e., the CTG method).

Cells in the log phase were collected and plated at a density of 5000 cells/well. The cell plates were incubated overnight in an incubator at 37 °C with 5% CO₂. On day two of the experiment, the ADCs were diluted 3-fold with the complete culture medium to obtain 8 concentration gradients (the 2-fold dilution started from the highest concentration 2000 nM) and then added to the cell culture plates at 50 µL/well. The complete culture medium was used as a blank control, and 2 replicate wells were set. The plates were incubated in the incubator at 37 °C with 5% CO₂ for another 7 days. After the incubation, the cell culture plates were taken out and equilibrated to room temperature. Subsequently, the CTG assay reagent (Promega, Cat#: G7573) was added at 50 µL/well. The plates were shaken for thorough mixing and then left to stand in a dark place for 10 min, and the signal values were then read using a microplate reader. Sigmoid dose-response curves were plotted using a nonlinear regression model in GraphPad Prism software, and the IC₅₀ values were calculated. The cell survival rates were calculated according to the formula: (Lum_{test drug} - Lum_{blank control})/(Lum_{solvent blank} control - Lum_{blank} control) × 100%.

The proliferation inhibitory effects on NCI-H929 cells are shown in Table 9-1 below:

**Table 9-1.IC₅₀ values for the inhibition of the in vitro proliferation of tumor cells by the ligand-drug conjugates of the present application**

| Compound | IC₅₀ (nM) |
|---|---|
| I-E97 | 0.6154 |
| I-E92 | 2.087 |

The proliferation inhibitory effects on MM.1R cells are shown in Table 9-2 below:

**Table 9-2-IC₅₀ values for the inhibition of the in vitro proliferation of tumor cells by the ligand-drug conjugates of the present application**

| Compound | IC₅₀ (nM) |
|---|---|
| I-E97 | 1.225 |

Conclusion: The ligand-drug conjugates of the present application exhibited significant proliferation inhibitory activity against NCI-H929 and MM.1R cells.

### Example 5: Bystander Killing Activity Experiment

The bystander killing activity of the ligand-drug conjugates of the present application was assessed using NCI-N87 cells (purchased from the Cell Bank of the Chinese Academy of Sciences, Cat. No. TCHu130), which have high CEACAM5 expression, and Raji-luciferase cells (Raji-luc cells, purchased from Nanjing Cobioer Biosciences, Cat. No. CBP30043L), which have negative CEACAM5 expression and carry luciferase.

The specific experimental procedure was as follows: First, the 6-day killing effects of the ligand-drug conjugates of the present application on NCI-N87 cells and Raji-luc cells were investigated using the CTG method. The experimental results show that the ligand-drug conjugates of the present application killed NCI-N87 cells, which have high CEACAM5 expression, in a dose-dependent manner, but exhibited little killing activity against Raji-luc cells, which have negative CEACAM5 expression. Subsequently, NCI-N87 cells and Raji-luc cells were co-incubated in a 1:1 ratio and treated with the compounds for 6 days, and the bystander killing effects of the ligand-drug conjugates of the present application on cells with negative CEACAM5 expression were then assessed by determining luciferase values using a microplate reader (refer to the steps described in the instructions for the Steady-Glo^{®} Luciferase Assay kit [Promega Cat. No. E2510] for the determination steps, followed by the measurement of chemiluminescence with the microplate reader). The experimental results show that the ligand-drug conjugates of the present application released toxins after entering NCI-N87 cells, which are CEACAM5-positive, and then killed Raji-luc cells, which are CEACAM5 -negative, demonstrating that the ligand-drug conjugates of the present application have strong bystander killing effects.

### Example 6: Evaluation of Efficacy of Ligand-Drug Conjugate in Mice with HPAF-II and Panc 08.13 Graft Tumors

To study the inhibitory effect of the ligand-drug conjugate of the present disclosure on tumors formed *in vivo,* graft tumors were formed in mice using HPAF-II and Panc 08.13, and the anti-tumor effect of the ligand-drug conjugate was then assessed.

### 1. Test compound and materials

### Negative control group: normal saline

Treatment group 1: I-E43, 2 mg/kg (HPAF-II graft tumor model)
Treatment group 2: I-E43, 3 mg/kg (Panc 08.13 graft tumor model)

### 2. Preparation method: All samples were prepared by dilution with normal saline.

### 3. Test animals: 8-week-old female BALB/c mice, purchased from Jiangsu GemPharmatech Co., Ltd.

### 4. Test method:

1 × 10⁷ HPAF-II cells (ATCC, CRL-1997) or Panc 08.13 cells (ATCC, CRL-2551) were subcutaneously inoculated into the right anterior scapula of each 8-week-old female BALB/c nude mouse. When the tumors grew to about 150 mm³, the tumor-bearing mice were randomly grouped using a Study Director TM, and administration was started by intravenous (i.v.) injection on the day of grouping (day 0). A total of 1 injection was administered. The administration dose for the HPAF-II graft tumor model was 2 mg/kg, and the administration dose for the Panc 08.13 graft tumor model was 3 mg/kg. The experiment ended 21 days after the administration. Tumor volume and body weight were measured twice weekly, and the measurements were recorded. There were 5 mice in the control group or each treatment group. Tumor growth inhibition rates were calculated by measuring tumor volume. Tumor growth inhibition rate (TGI%) = 100% - (the tumor volume of the treatment group on the day of measurement - the tumor volume of the treatment group on day 0)/(the tumor volume of the control group on the day of measurement - the tumor volume of the control group on day 0). In the case of tumor regression (the tumor volume at the time of measurement was smaller than the tumor volume at the time of the first dose), tumor growth inhibition rate (TGI%) = [1 - (the tumor volume of the treatment group on the day of measurement ÷ the tumor volume of the treatment group on day 0) ÷ (the tumor volume of the control group on the day of measurement ÷ the tumor volume of the control group on day 0)] ÷ [ 1 - (the tumor volume of the control group on day 0 ÷ the tumor volume of the control group on the day of measurement)] × 100%.

**Table 10-1. The inhibitory effect of the ligand-drug conjugate of the present application on tumors in HPAF-II tumor-bearing mice**

| Group | TGI% (day 6) | TGI% (day 13) | TGI% (day 21) |
|---|---|---|---|
| I-E43, 2mpk | 114.14% | 122.82% | 129.90% |

**Table 10-2. The inhibitory effect of the ligand-drug conjugate of the present application on tumors in Panc 08.13 tumor-bearing mice**

| Group | TGI% (day 6) | TGI% (day 13) |
|---|---|---|
| I-E43, 3mpk | 103.74% | 119.71% |

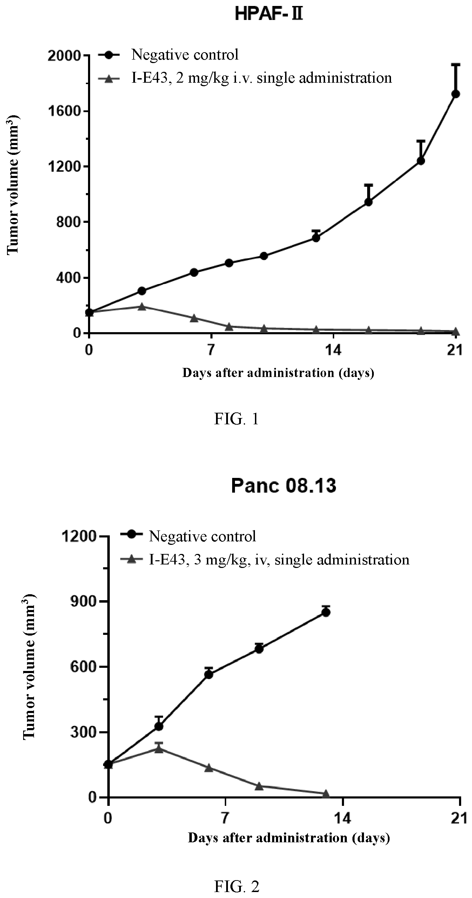

The results are shown in Tables 10-1 and 10-2 and FIGs. 1 and 2. The tumor growth inhibition rates in Tables 10-1 and 10-2 were calculated according to the calculation formula for tumor growth inhibition rate in the case of regression.

Conclusion: The ligand-drug conjugate of the present application exhibited significant anti-tumor activity after a single administration.

### Example 7.1: Evaluation of Efficacy of Ligand-Drug Conjugates in NCI-H929 Human Myeloma Cell Tumor-Bearing Mice

### Objective

To evaluate the anti-tumor effects of the ligand-drug conjugates of the present application in NCI-H929 tumor-bearing mice, with female NOG mice as test animals.

### 1. Test compounds and materials

### Negative control (negative control group): normal saline

Control ADC1 (positive control group): 5 mg/kg, single administration
I-E87 (treatment group): 5 mg/kg, single administration
I-E89 (treatment group): 5 mg/kg, single administration

### 2. Preparation method: All were prepared by dilution with normal saline.

### 3. Test animals: 6- to 8-week-old NOG mice, purchased from Vital River Laboratory Animal Technology Co., Ltd.

4. Test method: 1 × 10^7 NCI-H929 cells (ATCC, CRL-3580) resuspended in 0.2 mL of PBS and Matrigel (1:1) were subcutaneously inoculated into the right dorsal side of each experimental mouse. Tumor growth was observed regularly. When the tumors grew to a mean volume of 150 mm³, the mice were randomly grouped according to tumor size and mouse body weight and dosed, with 5 animals in each group. The day of grouping and administration was defined as day 0. Administration was performed once by tail vein injection, and the experiment ended 21 days after the administration. Tumor volume and body weight were measured 2-3 times weekly, and the measurements were recorded. At the end of the experiment, the mice were euthanized, and the tumor growth inhibition rates TGI% (TGI = (1 - (Tᵢ - T₀)/(Vᵢ - V₀))) were calculated. Tᵢ: the mean tumor volume of the treatment group or the positive control group on day i post-dose; T₀: the mean tumor volume of the treatment group or the positive control group on day 0 post-dose; Vᵢ: the mean tumor volume of the negative control group on day i post-dose; V₀: the mean tumor volume of the negative control group on day 0 post-dose.

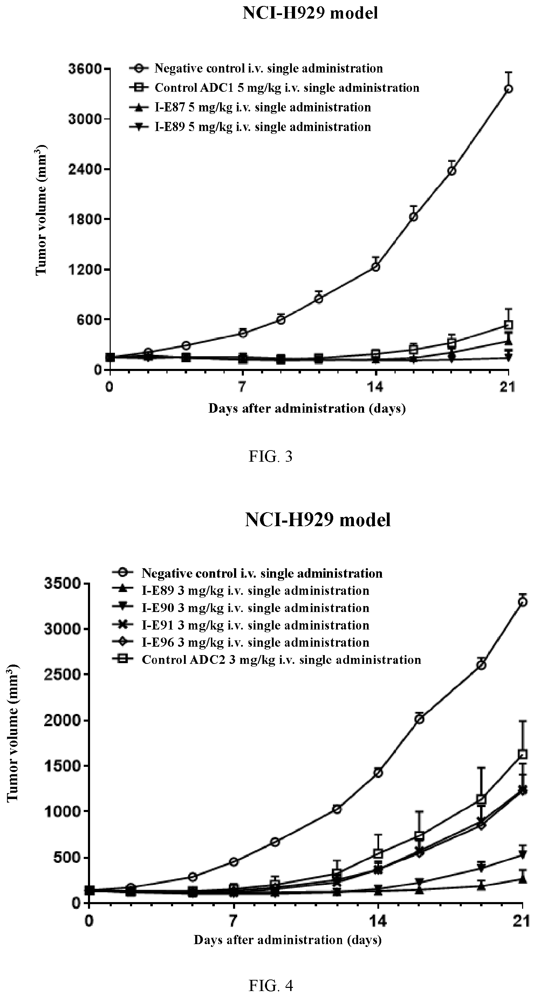

The experimental results are shown in Table 11 and FIG. 3. At the end of the experiment, the mean tumor volume of the negative control group was 3362.56 mm³.

**Table 11. The inhibitory effects of the ligand-drug conjugates of the present application on tumors in NCI-H929 tumor-bearing mice**

| Group | Administration | Dose | Day 21 | |
|---|---|---|---|---|
| | | (mg/kg) | Tumor volume (mean ± SEM, mm³) | TGI (%) |
| 1 | Negative control | - | 3362.56 ± 198.47 | - |
| 2 | Control ADC1 | 5 | 538.59 ± 191.01 | 87.91% |
| 3 | I-E87 | 5 | 343.62 ± 105.67 | 93.96% |
| 4 | I-E89 | 5 | 145.03 ± 40.95 | 100.19% |

Conclusion: The ligand-drug conjugates of the present application significantly reduced tumor volume, and the ligand-drug conjugates of the present application, I-E87 and I-E89, exhibited better inhibitory effects on tumors than the control ADC1 at the same concentration.

### Example 7.2: Evaluation of Efficacy of Ligand-Drug Conjugates in NCI-H929 Human Myeloma Cell Tumor-Bearing Mice

### Objective

To evaluate the anti-tumor effects of the ligand-drug conjugates of the present application in NCI-H929 tumor-bearing mice, with female NOG mice as test animals.

### 1. Test compounds and materials

Negative control (negative control group): normal saline
I-E89 (treatment group): 3 mg/kg, single administration
I-E90 (treatment group): 3 mg/kg, single administration
I-E91 (treatment group): 3 mg/kg, single administration
I-E96 (treatment group): 3 mg/kg, single administration
Control ADC2 (treatment group): 3 mg/kg, single administration

2. Preparation method: All were prepared by dilution with normal saline.

3. Test animals: 6- to 8-week-old NOG mice, purchased from Vital River Laboratory Animal Technology Co., Ltd.

4. Test method: 1 × 10^7 NCI-H929 cells (ATCC, CRL-3580) resuspended in 0.2 mL of PBS and Matrigel (1:1) were subcutaneously inoculated into the right dorsal side of each experimental mouse. Tumor growth was observed regularly. When the tumors grew to a mean volume of 140 mm³, the mice were randomly grouped according to tumor size and mouse body weight and dosed, with 5 animals in each group. The day of grouping and administration was defined as day 0. Administration was performed once by tail vein injection, and the experiment ended 21 days after the administration. Tumor volume and body weight were measured 2-3 times weekly, and the measurements were recorded. At the end of the experiment, the mice were euthanized, and the tumor growth inhibition rates TGI% (TGI = (1 - (Ti - T0)/(Vi - V0))) were calculated. Ti: the mean tumor volume of the treatment group or the positive control group on day i post-dose; T0: the mean tumor volume of the treatment group or the positive control group on day 0 post-dose; Vi: the mean tumor volume of the negative control group on day i post-dose; V0: the mean tumor volume of the negative control group on day 0 post-dose.

The experimental results are shown in Table 12 and FIG. 4. At the end of the experiment, the mean tumor volume of the negative control group was 3298.68 mm³.

**Table 12. The inhibitory effects of the ligand-drug conjugates of the present application on tumors in NCI-H929 tumor-bearing mice**

| Group | Administration | Dose (mg/kg) | Day 21 | |
|---|---|---|---|---|
| | | | Tumor volume (mean ± SEM, mm³) | TGI (%) |
| 1 | Negative control | - | 3298.68 ± 83.42 | - |
| 2 | I-E89 | 3 | 265.18 ± 95.99 | 96.01 % |
| 3 | I-E90 | 3 | 528.62 ± 104.20 | 87.68 % |
| 4 | I-E91 | 3 | 1240.86 ± 165.97 | 65.10 % |
| 5 | I-E96 | 3 | 1232.97 ± 292.86 | 65.36 % |
| 6 | Control ADC2 | 3 | 1629.37 ± 162.20 | 52.82 % |

Conclusion: The ligand-drug conjugates of the present application significantly reduced tumor volume, and the ligand-drug conjugates of the present application, I-E89, I-E90, I-E91, and I-E96, exhibited better inhibitory effects on tumors than the control ADC2 at the same concentration.

### Example 8: Evaluation of Efficacy of Ligand-Drug Conjugates in KMS11 Human Myeloma Cell Tumor-Bearing Mice

### Objective

To evaluate the anti-tumor effects of the ligand-drug conjugates of the present application in KMS11 tumor-bearing mice, with female CB17 SCID mice as test animals.

### 1. Test compounds and materials

Negative control (negative control group): normal saline
I-E89 (treatment group): 5 mg/kg, single administration
I-E90 (treatment group): 5 mg/kg, single administration
I-E103 (treatment group): 5 mg/kg, single administration

2. Preparation method: All were prepared by dilution with normal saline.

3. Test animals: 6- to 8-week-old CB17 SCID mice, purchased from Shanghai Lingchang Biotechnology Co., Ltd.

4. Test method: 1 × 10^7 KMS11 cells (JCRB, JCRB1179) resuspended in 0.2 mL of PBS and Matrigel (1:1) were subcutaneously inoculated into the right dorsal side of each experimental mouse. Tumor growth was observed regularly. When the tumors grew to a mean volume of 120 mm³, the mice were randomly grouped according to tumor size and mouse body weight and dosed, with 5 animals in each group. The day of grouping and administration was defined as day 0. Administration was performed once by tail vein injection, and the experiment ended 21 days after the administration. Tumor volume and body weight were measured twice weekly, and the measurements were recorded. At the end of the experiment, the mice were euthanized, and the tumor growth inhibition rates TGI% (TGI = (1 - (Ti - T0)/(Vi - V0))) were calculated. Ti: the mean tumor volume of the treatment group or the positive control group on day i post-dose; T0: the mean tumor volume of the treatment group or the positive control group on day 0 post-dose; Vi: the mean tumor volume of the negative control group on day i post-dose; V0: the mean tumor volume of the negative control group on day 0 post-dose.

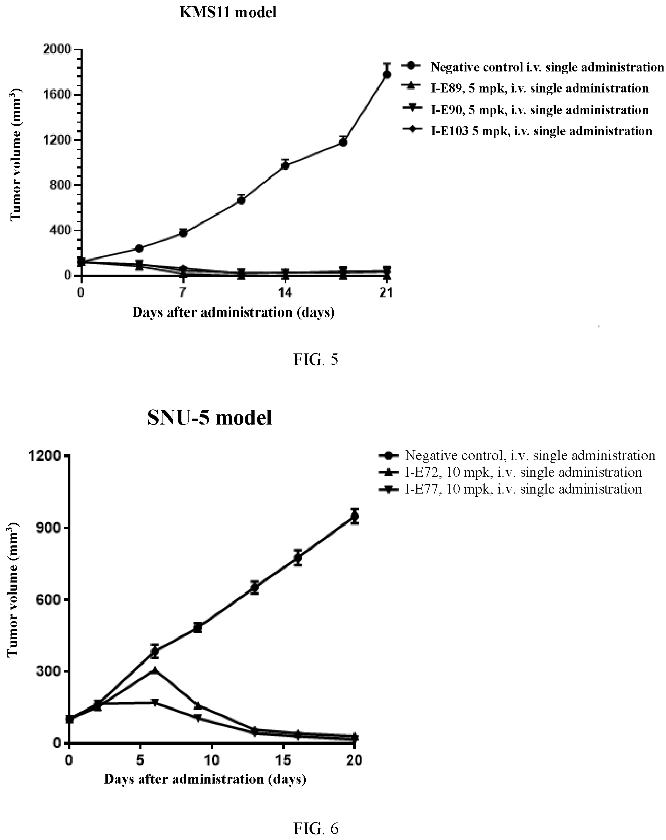

The experimental results are shown in Table 13 and FIG. 5. At the end of the experiment, the mean tumor volume of the negative control group was 1779.42 mm³.

**Table 13. The inhibitory effects of the ligand-drug conjugates of the present application on tumors in KMS11 tumor-bearing mice**

| Group | Administration | Dose (mg/kg) | Day 21 | |
|---|---|---|---|---|
| | | | Tumor volume (mean ± SEM, mm³) | TGI (%) |
| 1 | Negative control | - | 1779.42±97.70 | - |
| 2 | I-E89 | 5 | 0±0 | 107.47 |
| 3 | I-E90 | 5 | 40.87±40.87 | 104.98 |
| 4 | I-E103 | 5 | 34.57±34.57 | 105.37 |

Conclusion: The ligand-drug conjugates of the present application significantly reduced tumor volume.

### Example 9: Evaluation of Efficacy of Ligand-Drug Conjugates in SNU-5 Human Gastric Cancer Cell Tumor-Bearing Mice

### Objective

To evaluate the anti-tumor effects of the ligand-drug conjugates of the present application in SNU-5 tumor-bearing mice, with female NCG mice as test animals.

### 1. Test compounds and materials

Negative control (negative control group): normal saline
I-E72 (treatment group): 10 mg/kg, single administration
I-E77 (treatment group): 10 mg/kg, single administration

2. Preparation method: All were prepared by dilution with normal saline.

3. Test animals: 6- to 8-week-old NCG mice, purchased from Jiangsu GemPharmatech Co., Ltd.

4. Test method: 5 × 10^6 SNU-5 cells (ATCC, CRL-5973) resuspended in 0.2 mL of PBS and Matrigel (1:1) were subcutaneously inoculated into the right dorsal side of each experimental mouse. Tumor growth was observed regularly. When the tumors grew to a mean volume of 100 mm³, the mice were randomly grouped according to tumor size and mouse body weight and dosed, with 5 animals in each group. The day of grouping and administration was defined as day 0. Administration was performed once by tail vein injection, and the experiment ended 21 days after the administration. Tumor volume and body weight were measured 2-3 times weekly, and the measurements were recorded. At the end of the experiment, the mice were euthanized, and the tumor growth inhibition rates TGI% (TGI = (1 - (Ti - T0)/(Vi - V0))) were calculated. Ti: the mean tumor volume of the treatment group or the positive control group on day i post-dose; T0: the mean tumor volume of the treatment group or the positive control group on day 0 post-dose; Vi: the mean tumor volume of the negative control group on day i post-dose; V0: the mean tumor volume of the negative control group on day 0 post-dose.

The experimental results are shown in Table 14 and FIG. 6. At the end of the experiment, the mean tumor volume of the negative control group was 948.10 mm³.

**Table 14. The inhibitory effects of the ligand-drug conjugates of the present application on tumors in SUN-5 tumor-bearing mice**

| Group | Administration | Dose (mg/kg) | Day 20 | |
|---|---|---|---|---|
| | | | Tumor volume (mean ± SEM, mm³) | TGI (%) |
| 1 | Negative control | - | 948.10±29.56 | - |
| 2 | I-E72 | 10 | 29.78±2.63 | 96.87% |
| 3 | I-E77 | 10 | 15.92±4.65 | 98.34% |

Conclusion: The ligand-drug conjugates of the present application significantly reduced tumor volume.

### Example 10: Evaluation of Efficacy of Ligand-Drug Conjugate in Calu3 Human Lung Cancer Cell Tumor-Bearing Mice

### Objective

To evaluate the anti-tumor effect of the ligand-drug conjugate of the present application in Calu3 tumor-bearing mice, with female CB-17 SCID mice as test animals.

### 1. Test compound and materials

Negative control (negative control group): normal saline
I-E77 (treatment group): 8 mg/kg, single administration

2. Preparation method: All were prepared by dilution with normal saline.

3. Test animals: 5- to 8-week-old CB-17 SCID mice, purchased from Vital River Laboratory Animal Technology Co., Ltd.

4. Test method: 5 × 10^6 Calu3 cells (ATCC, HTB-55) resuspended in 0.2 mL of PBS and Matrigel (1:1) were subcutaneously inoculated into the right dorsal side of each experimental mouse. Tumor growth was observed regularly. When the tumors grew to a mean volume of 130 mm³, the mice were randomly grouped according to tumor size and mouse body weight and dosed, with 5 animals in each group. The day of grouping and administration was defined as day 0. Administration was performed once by tail vein injection, and the experiment ended 21 days after the administration. Tumor volume and body weight were measured 2-3 times weekly, and the measurements were recorded. At the end of the experiment, the mice were euthanized, and the tumor growth inhibition rates TGI% (TGI = (1 - (Ti - T0)/(Vi - V0))) were calculated. Ti: the mean tumor volume of the treatment group or the positive control group on day i post-dose; T0: the mean tumor volume of the treatment group or the positive control group on day 0 post-dose; Vi: the mean tumor volume of the negative control group on day i post-dose; V0: the mean tumor volume of the negative control group on day 0 post-dose.

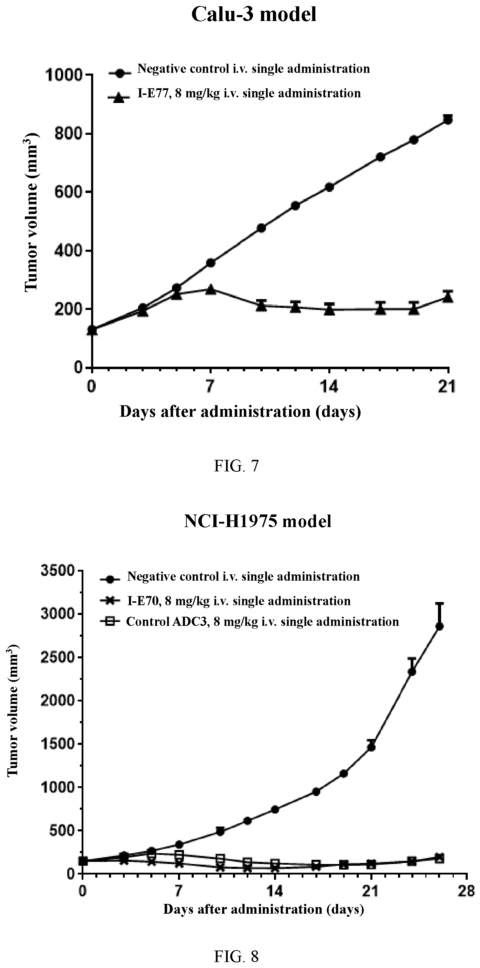

The experimental results are shown in Table 15 and FIG. 7. At the end of the experiment, the mean tumor volume of the negative control group was 847.25 mm³.

**Table 15. The inhibitory effect of the ligand-drug conjugate of the present application on tumors in Calu3 tumor-bearing mice**

| Group | Administration | Dose (mg/kg) | Day 21 | |
|---|---|---|---|---|
| | | | Tumor volume (mean ± SEM, mm³) | TGI (%) |
| 1 | Negative control | - | 847.25±14.29 | - |
| 2 | I-E77 | 8 | 241.55±20.11 | 84.48% |

Conclusion: The ligand-drug conjugate of the present application significantly reduced tumor volume.

### Example 11: Evaluation of Efficacy of Ligand-Drug Conjugate in NCI-H1975 Human Lung Cancer Cell Tumor-Bearing Mice

### Objective

To evaluate the anti-tumor effect of the ligand-drug conjugate of the present application in NCI-H1975 tumor-bearing mice, with female BALB/c nude mice as test animals.

### 1. Test compound and materials

Negative control (negative control group): normal saline
I-E70 (treatment group): 8 mg/kg, single administration
Control ADC3 (positive control group): 8 mg/kg, single administration

2. Preparation method: All were prepared by dilution with normal saline.

3. Test animals: 6- to 8-week-old BALB/c nude mice, purchased from Vital River Laboratory Animal Technology Co., Ltd.

4. Test method: 5 × 10^6 NCI-H1975 cells (ATCC, CRL-5908) resuspended in 0.2 mL of PBS were subcutaneously inoculated into the right dorsal side of each experimental mouse. Tumor growth was observed regularly. When the tumors grew to a mean volume of 150 mm³, the mice were randomly grouped according to tumor size and mouse body weight and dosed, with 6 animals in each group. The day of grouping and administration was defined as day 0. Administration was performed once by tail vein injection, and the experiment ended 26 days after the administration. Tumor volume and body weight were measured 2-3 times weekly, and the measurements were recorded. At the end of the experiment, the mice were euthanized, and the tumor growth inhibition rates TGI% (TGI = (1 - (Ti - T0)/(Vi - V0))) were calculated. Ti: the mean tumor volume of the treatment group or the positive control group on day i post-dose; T0: the mean tumor volume of the treatment group or the positive control group on day 0 post-dose; Vi: the mean tumor volume of the negative control group on day i post-dose; V0: the mean tumor volume of the negative control group on day 0 post-dose.

The experimental results are shown in Table 16 and FIG. 8. At the end of the experiment, the mean tumor volume of the negative control group was 2856.79 mm³.

**Table 16. The inhibitory effect of the ligand-drug conjugate of the present application on tumors in NCI-H1975 tumor-bearing mice**

| Group | Administration | Dose (mg/kg) | Day 26 | |
|---|---|---|---|---|
| | | | Tumor volume (mean ± SEM, mm³) | TGI (%) |
| 1 | Negative control | - | 2856.79±263.99 | - |
| 2 | I-E70 | 8 | 200.43±36.55 | 98.23% |
| 3 | Control ADC3 | 8 | 180.24±38.83 | 98.97% |

Conclusion: The ligand-drug conjugate of the present application significantly reduced tumor volume.

### Example 12: Evaluation of Efficacy of Ligand-Drug Conjugate in NCI-H820 Human Lung Cancer Cell Tumor-Bearing Mice

### Objective

To evaluate the anti-tumor effect of the ligand-drug conjugate of the present application in NCI-H820 tumor-bearing mice, with female BALB/c nude mice as test animals.

### 1. Test compound and materials

Negative control (negative control group): normal saline
I-E104 (treatment group): 3 mg/kg, single administration
Control ADC4 (control group): 3 mg/kg, single administration

2. Preparation method: All were prepared by dilution with normal saline.

3. Test animals: 6- to 8-week-old BALB/c nude mice, purchased from Vital River Laboratory Animal Technology Co., Ltd.

4. Test method: 10 × 10^6 NCI-H820 cells (ATCC, HTB-181) resuspended in 0.2 mL of PBS and Matrigel (1:1) were subcutaneously inoculated into the right dorsal side of each experimental mouse. Tumor growth was observed regularly. When the tumors grew to a mean volume of 150 mm³, the mice were randomly grouped according to tumor size and mouse body weight and dosed, with 6 animals in each group. The day of grouping and administration was defined as day 0. Administration was performed once by tail vein injection, and the experiment ended 21 days after the administration. Tumor volume and body weight were measured twice weekly, and the measurements were recorded. At the end of the experiment, the mice were euthanized, and the tumor growth inhibition rates TGI% (TGI = (1 - (Ti - T0)/(Vi - V0))) were calculated. Ti: the mean tumor volume of the treatment group or the positive control group on day i post-dose; T0: the mean tumor volume of the treatment group or the positive control group on day 0 post-dose; Vi: the mean tumor volume of the negative control group on day i post-dose; V0: the mean tumor volume of the negative control group on day 0 post-dose.

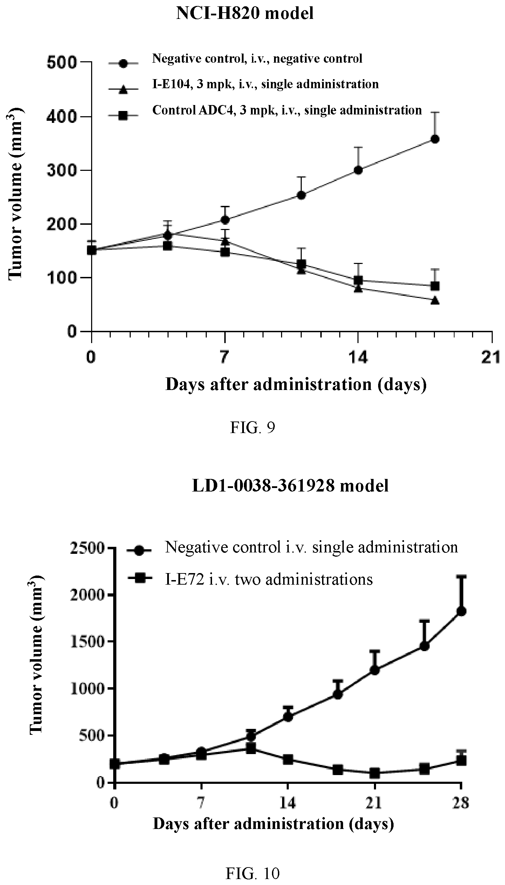

The experimental results are shown in Table 17 and FIG. 9. At the end of the experiment, the mean tumor volume of the negative control group was 358 mm³.

**Table 17. The inhibitory effect of the ligand-drug conjugate of the present application on tumors in NCI-H820 tumor-bearing mice**

| Group | Administration | Dose (mg/kg) | Day 21 | |
|---|---|---|---|---|
| | | | Tumor volume (mean ± SEM, mm³) | TGI (%) |
| 1 | Negative control | - | 358±124 | - |
| 2 | I-E104 | 3 | 58±7 | 145% |
| 3 | Control ADC4 | 3 | 84 ±31 | 132% |

Conclusion: The ligand-drug conjugate of the present application significantly reduced tumor volume.

### Example 13: Evaluation of Efficacy of Ligand-Drug Conjugate in LD1-0038-361928 Human Colorectal Cancer PDX Tumor-Bearing Mice

### Objective

To evaluate the anti-tumor effect of the ligand-drug conjugate of the present application in LD1-0038-361928 human colorectal cancer PDX tumor-bearing mice, with female NU/NU mice as test animals.

### 1. Test compound and materials

Negative control (negative control group): normal saline
I-E72 (treatment group): intravenously injected at 5 mg/kg on D0 and intravenously injected at 10 mg/kg on D7
FP4+3-passage LD1-0038-361928 tumor mass: The LD1-0038-361928 tumor mass was derived from patients with (rectal) ulcerative adenocarcinoma, grade II, accompanied by lymph node metastasis; "FP4+3-passage" means that the cells at passage 4 were thawed and then passaged 3 times.

2. Preparation method: All were prepared by dilution with normal saline.

3. Test animals: 6- to 8-week-old NU/NU mice, purchased from Vital River Laboratory Animal Technology Co., Ltd.

4. Test method: The successfully thawed FP4+3-passage LD1-0038-361928 human rectal cancer *in vivo* graft tumor mass was cut into small pieces measuring approximately 3 mm × 3 mm × 3 mm. The small pieces were subcutaneously inoculated into the right dorsal side of each NU/NU mouse using a tumor mass inoculation needle (the efficacy experiment passage number was FP4+4). Each animal was inoculated with about 30-60 mg of tumor tissue mixed with 10 µL of Matrigel. Tumor growth was observed regularly. When the tumors grew to a mean volume of 196 mm³, the mice were randomly grouped according to tumor size and mouse body weight and dosed, with 5 animals in each group. The day of grouping and administration was defined as day 0. Administration was performed twice by tail vein injection, and the experiment ended 21 days after the administration. Tumor volume and body weight were measured twice weekly, and the measurements were recorded. At the end of the experiment, the mice were euthanized, and the tumor growth inhibition rates TGI% (TGI = (1 - (Ti - T0)/(Vi - V0))) were calculated. Ti: the mean tumor volume of the treatment group or the positive control group on day post-dose; T0: the mean tumor volume of the treatment group or the positive control group on day 0 post-dose; Vi: the mean tumor volume of the negative control group on day i post-dose; V0: the mean tumor volume of the negative control group on day 0 post-dose.

The experimental results are shown in Table 18 and FIG. 10. At the end of the experiment, the mean tumor volume of the negative control group was 1827.98 mm³.

**Table 18. The inhibitory effect of the ligand-drug conjugate of the present application on tumors in NCI-H820 tumor-bearing mice**

| Group | Administration | Dose (mg/kg) | Day 28 | |
|---|---|---|---|---|
| | | | Tumor volume (mean ± SEM, mm³) | TGI (%) |
| 1 | Negative control | - | 1827.98±368.54 | - |
| 2 | I-E72 | D0 5mpk, D7 10mpk | 232.93±100.52 | 97.74% |

Conclusion: The ligand-drug conjugate of the present application significantly reduced tumor volume.

### Example 14: Evaluation of Efficacy of Ligand-Drug Conjugate in MKN45 Human Gastric Cancer Cell Tumor-Bearing Mice

### Objective

To evaluate the anti-tumor effect of the ligand-drug conjugate of the present application in MKN45 tumor-bearing mice, with female CB17 SCID mice as test animals.

### 1. Test compound and materials

Negative control (negative control group): normal saline
I-E107 (treatment group): 5 mg/kg, single administration

2. Preparation method: All were prepared by dilution with normal saline.

3. Test animals: 6- to 8-week-old CB17 SCID mice, purchased from Vital River Laboratory Animal Technology Co., Ltd.

4. Test method: 5 × 10^6 MKN45 cells (Cell Resource Center, Institute of Basic Medical Sciences, Chinese Academy of Medical Sciences, IM-H088) resuspended in 0.2 mL of PBS and Matrigel (1:1) were subcutaneously inoculated into the right dorsal side of each experimental mouse. Tumor growth was observed regularly. When the tumors grew to a mean volume of 150 mm³, the mice were randomly grouped according to tumor size and mouse body weight and dosed, with 5 animals in each group. The day of grouping and administration was defined as day 0. Administration was performed once by tail vein injection, and the experiment ended 21 days after the administration. Tumor volume and body weight were measured 2-3 times weekly, and the measurements were recorded. At the end of the experiment, the mice were euthanized, and the tumor growth inhibition rates TGI% (TGI = (1 - (Ti - T0)/(Vi - V0))) were calculated. Ti: the mean tumor volume of the treatment group or the positive control group on day i post-dose; T0: the mean tumor volume of the treatment group or the positive control group on day 0 post-dose; Vi: the mean tumor volume of the negative control group on day i post-dose; V0: the mean tumor volume of the negative control group on day 0 post-dose.

Conclusion: The ligand-drug conjugate of the present application significantly reduced tumor volume.

### Example 15: Evaluation of Efficacy of Ligand-Drug Conjugate in HCC827 Human Gastric Cancer Cell Tumor-Bearing Mice

### Objective

To evaluate the anti-tumor effect of the ligand-drug conjugate of the present application in HCC827 tumor-bearing mice, with female CB17 SCID mice as test animals.

### 1. Test compound and materials

Negative control (negative control group): normal saline
Tusamitamab-LP566 (treatment group): 5 mg/kg, single administration

2. Preparation method: All were prepared by dilution with normal saline.

3. Test animals: 6- to 8-week-old CB17 SCID mice, purchased from Vital River Laboratory Animal Technology Co., Ltd.

4. Test method: 5 × 10^6 HCC827 cells (ATCC, CRL-2868) resuspended in 0.2 mL of PBS and Matrigel (1:1) were subcutaneously inoculated into the right dorsal side of each experimental mouse. Tumor growth was observed regularly. When the tumors grew to a mean volume of 150 mm³, the mice were randomly grouped according to tumor size and mouse body weight and dosed, with 5 animals in each group. The day of grouping and administration was defined as day 0. Administration was performed once by tail vein injection, and the experiment ended 21 days after the administration. Tumor volume and body weight were measured 2-3 times weekly, and the measurements were recorded. At the end of the experiment, the mice were euthanized, and the tumor growth inhibition rates TGI% (TGI = (1 - (Ti - T0)/(Vi - V0))) were calculated. Ti: the mean tumor volume of the treatment group or the positive control group on day i post-dose; T0: the mean tumor volume of the treatment group or the positive control group on day 0 post-dose; Vi: the mean tumor volume of the negative control group on day i post-dose; V0: the mean tumor volume of the negative control group on day 0 post-dose.

Conclusion: The ligand-drug conjugate of the present application significantly reduced tumor volume.

### Example 16: Evaluation of Efficacy of Ligand-Drug Conjugates in HUH7 Human Gastric Cancer Cell Tumor-Bearing Mice

### Objective

To evaluate the anti-tumor effects of the ligand-drug conjugates of the present application in HUH7 tumor-bearing mice, with female NCG mice as test animals.

### 1. Test compounds and materials

Negative control (negative control group): normal saline
I-E102 (treatment group): 5 mg/kg, single administration
I-E109 (treatment group): 5 mg/kg, single administration

2. Preparation method: All were prepared by dilution with normal saline.

3. Test animals: 6- to 8-week-old NCG mice, purchased from Jiangsu GemPharmatech Co., Ltd.

4. Test method: 5 × 10^6 HUH7 cells resuspended in 0.2 mL of PBS were subcutaneously inoculated into the right dorsal side of each experimental mouse. Tumor growth was observed regularly. When the tumors grew to a mean volume of 130 mm³, the mice were randomly grouped according to tumor size and mouse body weight and dosed, with 5 animals in each group. The day of grouping and administration was defined as day 0. Administration was performed once by tail vein injection, and the experiment ended 21 days after the administration. Tumor volume and body weight were measured 2-3 times weekly, and the measurements were recorded. At the end of the experiment, the mice were euthanized, and the tumor growth inhibition rates TGI% (TGI = (1 - (Ti - T0)/(Vi - V0))) were calculated. Ti: the mean tumor volume of the treatment group or the positive control group on day i post-dose; T0: the mean tumor volume of the treatment group or the positive control group on day 0 post-dose; Vi: the mean tumor volume of the negative control group on day i post-dose; V0: the mean tumor volume of the negative control group on day 0 post-dose.

Conclusion: The ligand-drug conjugates of the present application significantly reduced tumor volume.

### Example 17: GSH Exchange Stability Test

The test compounds were each placed in a PBS buffer (pH 7.4), and HSA (human serum albumin) in GSH simulated plasma was added. The mixtures were incubated at 40 °C under a nitrogen atmosphere, and the DAR values of the ADC compounds were determined at 0 h, 1 d, and 4 d to simulate the assessment of the stability of the linkers of the ADC compounds in plasma.

The ADC concentration was 0.001 µmol/mL, and the GSH concentration was 0.954 µmol/mL (equivalent to 1.5 times the HSA concentration in human plasma (0.636 µmol/mL)).

50 µL of an ADC solution was added to a 4 mL EP tube, 3000 µL of a 20 mM PBS solution was added, and 90 µL of a 10 mg/mL GSH solution was then added. After the feeding, the mixture was fully shaken for thorough mixing and aliquoted into six 2-mL cryopreservation tubes at 500 µL per tube. One tube of the sample at T0 was stored in a freezer at -80 °C, and the remaining 5 tubes of the sample were protected from light with aluminum foil and then incubated in a thermostatic incubator at 40 °C. One tube was taken out at 1 d and another at 4 d for analysis. The results show that the ADC compounds all exhibited relatively good stability, and their DAR values remained unchanged.

Conclusion: The ligand-drug conjugates of the present application exhibited good long-term stability in GSH.

### Example 18: Plasma Stability Test

To assess the plasma stability of the ligand-drug conjugates of the present disclosure, the ligand-drug conjugates of the present disclosure were incubated in human, rat, and monkey plasma for 21 days, and samples were taken at 0 h, 8 h, 1 d, 4 d, 7 d, 14 d, and 21 d for DAR value determination and free drug detection.

| Reagent | Supplier | Cat. No. |
|---|---|---|
| Water | Millipore Milli-Q | NA (Homemade ultra-pure water) |
| Acetonitrile | MERCK | JB112730 |
| Formic acid | Sigma | SHBN5849 |
| Formic acid | Fisher | A117-50 |
| Streptavidin magnetic beads | Promega | 473016 |
| PBS buffer | Life Technologies Corporation | 2379439 |
| Dithiothreitol | Sigma | SLCH5653 |
| Cynomolgus monkey plasma | Guangdong Chunsheng | NA |
| Human plasma | BioreclamationIVT | HUMANPLNHP2N |

Incubation of ligand-drug conjugates in plasma

The ligand-drug conjugates of the present disclosure were diluted with plasma to a final concentration of 150 µg/mL and incubated in a dark place at 37 °C for 21 days. Samples were collected at T0 (the samples were diluted within 30 min and then immediately frozen to -70 °C), 2 h, 8 h, 1 d (24 h), 4 d, 7 d, 14 d, and 21 d. All samples were stored in a freezer at -70 °C before analysis. For the samples collected at 2 h and 8 h, only their DAR values were analyzed.
Free drug analysis by LC-MS/MS
Protein precipitation

The plasma samples were thawed, and 291.9 µL of each sample was taken for free drug analysis. 400 µL of a precipitant (a solution in acetonitrile containing 0.1% formic acid, 200 ng/mL tolbutamide, and 200 ng/mL labetalol) was added to each sample, and the mixtures were well mixed. The mixtures were shaken for at least 20 min to precipitate proteins from the plasma. The samples were centrifuged at 4 °Cat 4000 rpm for 20 min, and 150 µL of the supernatant was taken for LC-MS/MS analysis.

The devices used for free drug analysis are shown in the table below:

| Instrument | |
|---|---|
| UPLC: | Shimadzu LC 30-AD |
| MS: | Triple QuadTM 6500+ |
| Autosampler: | CTC PAL |

The LC and MS parameters are shown below:

| | | |
|---|---|---|
| Mobile phases | A: 0.1% formic acid in water | |
| | B: 0.1% formic acid in acetonitrile | |
| Chromatography column | ACQUITY UPLC HSS T3 1.8 µm 2.1 × 100 mm column, part model: 186003539 | |
| LC gradient Flow rate: 0.50 mL/min | Time/min | Mobile phase B% |
| | 0.01 | 15 |
| | 0.80 | 45 |
| | 2.30 | 50 |
| | 2.50 | 100 |
| | 2.90 | 100 |
| | 2.91 | 15 |
| | 3.00 | End |
| MS parameters | CUR | 40 psi |
| | CAD | 10 psi |
| | IS | 5500 V |
| | TEM | 500 °C |
| | GS1 | 50 psi |
| | GS2 | 60 psi |
| | EP | 10V |
| | CXP | 11 V |

### Drug-to-ligand conjugation rate determination by LC-MS

### Ligand-drug conjugate capture by immobilization magnetic beads

25 µL of streptavidin magnetic beads was added to each well of a 96-well plate, and the stock buffer was discarded. The beads were washed with 200 µL of PBS and treated with 80 µL of a biotin-labeled antigen protein and another 100 µL of PBS for 120 min at room temperature. After the antigen protein was completely immobilized, the beads were washed with 200 µL of PBS twice. 20 µL of a ligand-drug conjugate plasma sample and 180 µL of PBS were added to each well. Subsequently, the plate was shaken at room temperature for 120 min to ensure that the ligand-drug conjugate in the plasma was completely captured, and the supernatants were then removed. The beads were washed with PBS twice and then treated with 50 µL of a wash buffer (1% formic acid in water) at room temperature for 20 min. Subsequently, 5 µL of a neutralizing buffer (1 M NH₄HCO₃, pH 8.5) and 5 µL of DTT (1 M) were added. The captured ligand-drug conjugate was reduced at room temperature for 60 min, and analysis was then performed by LC-MS.

Drug-to-ligand conjugation rate analysis

The LC-MS parameters are shown below:

| | | |
|---|---|---|
| Instrument | LC-MS/MS-BF (Waters VION QTof) | |
| Mobile phases | A: 0.1% Formic Acid in Water | |
| | B: 0.1% Formic Acid in Acetonitrile | |
| Chromatography column | ACQUITY UPLC HSS T3 1.8 µm 2.1 × 100 mm column, part model: 186003539 | |
| LC gradient Flow rate: 0.50 mL/min | Time | Mobile phase B% |
| | 0.00 | 20 |
| | 0.50 | 20 |
| | 6.00 | 40 |
| | 6.20 | 65 |
| | 6.40 | 20 |
| | 7.00 | 80 |
| | 7.40 | 80 |
| | 7.60 | 20 |
| | 8.00 | 20 |
| MS parameters | Input m/z range 1 | 1000-2400 |
| | Output m/z range 1 | 20000-30000 |
| | Input m/z range 2 | 850-2200 |
| | Output m/z range 2 | 50000-55000 |

The results show that after the ligand-drug conjugates of the present disclosure were incubated in plasma for 21 days, no drug or only an extremely tiny amount of drug came off, and their drug-to-ligand conjugation rates did not change significantly. The ligand-drug conjugates of the present disclosure exhibited extremely high plasma stability, suggesting that the ligand-drug conjugates of the present disclosure have better safety.

### Example 19: Toxicology Experiment on Cynomolgus Monkeys

A female monkey and a male monkey, both aged 3 to 5 years, were selected. Before administration, their body temperature was measured, and clinical pathology examinations were performed. After a single intravenous injection of a ligand-drug conjugate of the present disclosure was administered, clinical symptoms and food intake were observed daily; clinical pathology examinations were performed on D3, D7, D14, and D21; body weight was measured once weekly; and body temperature was measured at 4 h, 24 h, 48 h, and 72 h post-dose. On day 22, gross anatomical dissections were performed, and tissues were collected and histopathologically examined. Moreover, the toxicokinetic properties were also assessed.

The experiment showed that the tolerated doses of the ligand-drug conjugates of the present disclosure in monkeys were relatively high.

Finally, it should be noted that the above examples are only used to illustrate the technical solutions of the present disclosure and are not intended to limit the present disclosure. Although the present disclosure has been described in detail with reference to preferred examples, those of ordinary skill in the art will appreciate that modifications to the specific embodiments of the present disclosure or equivalent replacements of some of the technical features of the present disclosure can still be made without departing from the spirit of the technical solutions of the present disclosure, and all such modifications and equivalent replacements shall fall within the scope of the technical solutions claimed by the present disclosure.

## Claims

1. A ligand-drug conjugate or a racemic mixture, tautomer, stereoisomer, enantiomer, or diastereoisomer thereof, or a mixture thereof, or a pharmaceutically acceptable salt or solvate thereof, wherein the ligand-drug conjugate consists of a ligand, a linking group, and a drug group, and the drug group is selected from a structure represented by formula I-A, II-A', III-A', IV-A, or V-A: 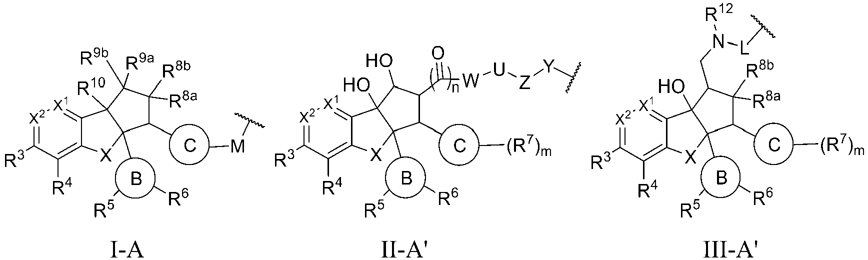 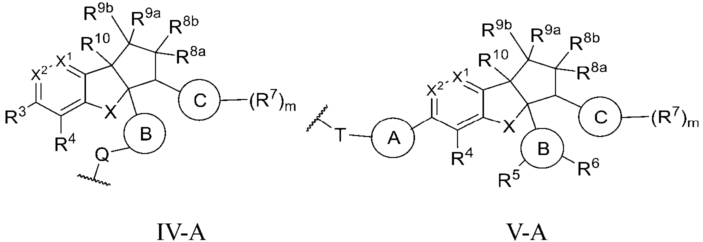 wherein:
M is selected from -N(R^{m})-, -O-, -(C₁-C₆)alkylene-O-, and -(C₁-C₆)alkylene-N(R^{m})-, wherein the -(C₁-C₆)alkylene-O- and -(C₁-C₆)alkylene-N(R^{m})- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and -N[(C₁-C₄)alkyl]₂;
n is 0 or 1;
W is absent or is selected from -(C₁-C₆)alkylene-, -O-, and -N(R^{w})-, wherein the -(C₁-C₆)alkylene-is optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and -N[(C₁-C₄)alkyl]₂;
U is selected from -(C₁-C₆)alkylene-, -(C₃-C₆)cycloalkylene-, and -(4-10 membered)heterocyclylene-, wherein the -(C₁-C₆)alkylene-, -(C₃-C₆)cycloalkylene-, and -(4-10 membered)heterocyclylene- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and - N[(C₁-C₄)alkyl]₂;
Z is absent or is selected from -(C₁-C₆)alkylene-, -(C₃-C₆)cycloalkylene-, and -(4-10 membered)heterocyclylene-;
Y is absent or is selected from -O-, -N(R^{y})-, and -N⁺(R^{y})₂-, wherein R^{y} is selected from hydrogen, -(C₁-C₆)alkyl, -O(C₁-C₆)alkyl, -(C₁-C₆)haloalkyl, and -O-(C₁-C₆)haloalkyl, or R^{y} and Z form -(4-10 membered)heterocyclylene-, wherein the -(4-10 membered)heterocyclylene- is optionally substituted with one or more (e.g., 1, 2, 3, 4, 5, 6, or 7) groups selected from hydrogen, halogen, - OH, -O-(C₁-C₄)alkyl, -NH₂, -NH(C₁-C₄)alkyl, and -N[(C₁-C₄)alkyl]₂;
R¹² is selected from hydrogen, -(C₁-C₆)alkyl, -O-(C₁-C₆)alkyl, -(C=O)-(C₁-C₆)alkyl, -(C₃-C₆)cycloalkyl, -O-(C₃-C₆)cycloalkyl, -(4-10 membered)heterocyclyl, and -O-(4-10 membered)heterocyclyl, wherein the -(C₁-C₆)alkyl, -O-(C₁-C₆)alkyl, -(C=O)-(C₁-C₆)alkyl, -(C₃-C₆)cycloalkyl, -O-(C₃-C₆)cycloalkyl, -(4-10 membered)heterocyclyl, and -O-(4-10 membered)heterocyclyl are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and - N[(C₁-C₄)alkyl]₂;
L is absent or is selected from -C(=O)-(C₁-C₆)alkylene-O-, -C(=O)-(C₁-C₆)alkylene-N(R^{L})-, - C(=O)-(C₁-C₆)alkylene-N⁺(R^{L})₂-, -C(=O)NH-(C₁-C₆)alkylene-O-, -C(=O)NH-(C₁-C₆)alkylene-N(R^{L})-, and -C(=O)NH-(C₁-C₆)alkylene-N⁺(R^{L})₂-, wherein R^{L} is selected from hydrogen, -(C₁-C₆)alkyl, -O-(C₁-C₆)alkyl, -(C₁-C₆)haloalkyl, and -O-(C₁-C₆)haloalkyl, wherein the -C(=O)-(C₁-C₆)alkylene-O-, -C(=O)-(C₁-C₆)alkylene-N(R^{L})-, -C(=O)-(C₁-C₆)alkylene-N⁺(R^{L})₂-, -C(=O)NH-(C₁-C₆)alkylene-O-, -C(=O)NH-(C₁-C₆)alkylene-N(R^{L})-, and -C(=O)NH-(C₁-C₆)alkylene-N⁺(R^{L})₂- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and -N[(C₁-C₄)alkyl]₂;
Q is selected from -N(R⁹)-, -O-, -(C₁-C₆)alkylene-O-, and -(C₁-C₆)alkylene-N(R⁹)-, wherein the - (C₁-C₆)alkylene-O- and -(C₁-C₆)alkylene-N(R⁹)- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and -N[(C₁-C₄)alkyl]₂;
T is selected from -N(R^{t})-, -O-, -(C₁-C₆)alkylene-O-, and -(C₁-C₆)alkylene-N(R^{t})-, wherein the - (C₁-C₆)alkylene-O- and -(C₁-C₆)alkylene-N(R⁹)- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and -N[(C₁-C₄)alkyl]₂;
R^{m}, R^{w}, R^{q}, and R^{t} are each independently selected from hydrogen, -(C₁-C₆)alkyl, -O-(C₁-C₆)alkyl, -(C₁-C₆)haloalkyl, and -O-(C₁-C₆)haloalkyl;
X¹ is selected from N and C(R¹), wherein R¹ is selected from hydrogen, halogen, cyano, -OH, - (C₁-C₆)alkyl, and -O-(C₁-C₆)alkyl;
X² is selected from N and C(R²), wherein R² is selected from hydrogen, halogen, cyano, -OH, - (C₁-C₆)alkyl, and -O-(C₁-C₆)alkyl;
R³ is selected from -(C₃-C₆)cycloalkyl, -(3-8 membered)heterocyclyl, -(C₆-C₁₀)aryl, and -(5-12 membered)heteroaryl, wherein the -(C₃-C₆)cycloalkyl, -(3-8 membered)heterocyclyl, -(C₆-C₁₀)aryl, and -(5-12 membered)heteroaryl are optionally substituted with one or more R³¹;
ring A is selected from -(C₃-C₆)cycloalkylene-, -(3-8 membered)heterocyclylene-, -(C₆-C₁₀)arylene-, and -(5-12 membered)heteroarylene-, wherein the -(C₃-C₆)cycloalkylene-, -(3-8 membered)heterocyclylene-, -(C₆-C₁₀)arylene-, and -(5-12 membered)heteroarylene- are optionally substituted with one or more R³¹;
each R³¹ is independently selected from oxo, hydrogen, halogen, cyano, nitro, -OH, -SH, -NH₂, - (C₁-C₆)alkyl, -(C₁-C₆)alkylene-OH, -(C₁-C₆)alkylene-NH₂, -(C₁-C₆)alkylene-NH[(C₁-C₆)alkyl], - (C₁-C₆)alkylene-N[(C₁-C₆)alkyl]₂, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, - (C₆-C₁₀)aryl, -(5-12 membered)heteroaryl, -S(=O)₂-NH₂, -S(=O)₂-NH(C₁-C₆)alkyl, -S(=O)₂-N[(C₁-C₆)alkyl]₂, -NH-C(=O)H, -NH-C(=O)-(C₁-C₆)alkyl, -N[(C₁-C₆)alkyl]-C(=O)-(C₁-C₆)alkyl, N[(C₁-C₆)alkyl]-C(=O)H, -NH-S(=O)₂-(C₁-C₆)alkyl, -N[(C₁-C₆)alkyl]-S(=O)₂-(C₁-C₆)alkyl, - P(=O)[(C₁-C₆)alkyl]₂, -P(=O)[(C₁-C₆)alkyl]-NH₂, -P(=O)[(C₁-C₆)alkyl]-NH(C₁-C₆)alkyl, and - P(=O)[(C₁-C₆)alkyl]-N[(C₁-C₆)alkyl]₂, wherein the -(C₁-C₆)alkyl, -(C₁-C₆)alkylene-OH, -(C₁-C₆)alkylene-NH₂, -(C₁-C₆)alkylene-NH[(C₁-C₆)alkyl], -(C₁-C₆)alkylene-N[(C₁-C₆)alkyl]₂, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH(C₁-C₆)alkyl, - C(=O)-N[(C₁-C₆)alkyl]₂, -(C₆-C₁₀)aryl, -(5-12 membered)heteroaryl, -S(=O)₂-NH₂, -S(=O)₂-NH(C₁-C₆)alkyl, -S(=O)₂-N[(C₁-C₆)alkyl]₂, -NH-C(=O)H, -NH-C(=O)-(C₁-C₆)alkyl, -N[(C₁-C₆)alkyl]-C(=O)-(C₁-C₆)alkyl, N[(C₁-C₆)alkyl]-C(=O)H, -NH-S(=O)₂-(C₁-C₆)alkyl, -N[(C₁-C₆)alkyl]-S(=O)₂-(C₁-C₆)alkyl, -P(=O)[(C₁-C₆)alkyl]₂, -P(=O)[(C₁-C₆)alkyl]-NH₂, -P(=O)[(C₁-C₆)alkyl]-NH(C₁-C₆)alkyl, and -P(=O)[(C₁-C₆)alkyl]-N[(C₁-C₆)alkyl]₂ are optionally substituted with one or more groups selected from hydrogen, halogen, -OH, -O-(C₁-C₄)alkyl, -(C₁-C₆)alkylene-OH, -NH₂, -NH(C₁-C₄)alkyl, -N[(C₁-C₄)alkyl]₂, -(C₁-C₆)alkylene-NH₂, -(C₁-C₆)alkylene-NH[(C₁-C₆)alkyl], -(C₁-C₆)alkylene-N[(C₁-C₆)alkyl]₂, -(C₆-C₁₀)aryl, -(5-12 membered)heteroaryl, -(C₁-C₆)alkylene-O(C₁-C₆)alkyl, and oxo;
R⁴ is selected from hydrogen, halogen, cyano, -OH, -NH₂, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4-10 membered)heterocyclyl, -O-(4-10 membered)heterocyclyl, -(C₆-C₁₀)aryl, -O-(C₆-C₁₀)aryl, -(5-12 membered)heteroaryl, and -O-(5-12 membered)heteroaryl;
X is selected from O, S, NH, N[(C₁-C₆)alkyl], N[C(=O)-R^{c}], C(R^{a})(R^{b}), C(=O), C[=C(R^{a})R^{b}], S(=O), and S(=O)₂;
R^{a} and R^{d} are each independently selected from hydrogen, halogen, cyano, -OR^{c}, -SR^{c}, -N(R^{c})R^{c}, -(C₁-C₆)alkyl, and -(C₁-C₆)haloalkyl;
R^{c} is selected from hydrogen, -(C₁-C₆)alkyl, -(C₁-C₆)haloalkyl, -O-(C₁-C₆)alkyl, -O-(C₁-C₆)haloalkyl, -(C₃-C₆)cycloalkyl, -O-(C₃-C₆)cycloalkyl, -(4-10 membered)heterocyclyl, -O-(4-10 membered)heterocyclyl, -(C₆-C₁₀)aryl, -O-(C₆-C₁₀)aryl, -(5-12 membered)heteroaryl, and -O-(5-12 membered)heteroaryl;
ring B is selected from -(C₆-C₁₀)aryl and -(5-12 membered)heteroaryl, or ring B is selected from - (C₆-C₁₀)arylene- and -(5-12 membered)heteroarylene-, wherein the -(C₆-C₁₀)arylene- and -(5-12 membered)heteroarylene- are optionally substituted with one or more groups selected from R⁵ and R⁶;
R⁵ and R⁶ are each independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, -C(=O)-H, and -C(=N-OH)-H, wherein the -(C₁-C₆)alkyl, -(C₂-C₆)alkenyl, -(C₂-C₆)alkynyl, -(C₁-C₆)haloalkyl, -O-(C₁-C₆)alkyl, - O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, - (4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH(C₁-C₆)alkyl, and -C(=O)-N[(C₁-C₆)alkyl]₂ are optionally substituted with one or more groups selected from hydrogen, halogen, oxo, -OH, -O-(C₁-C₄)alkyl, -NH₂, -NH(C₁-C₄)alkyl, and N[(C₁-C₄)alkyl]₂; or
R⁵ and R⁶, together with the carbon atoms to which they are attached, form 5-membered heterocyclyl;
ring C is selected from -(C₆-C₁₀)aryl and -(5-12 membered)heteroaryl, or ring C is selected from - (C₆-C₁₀)arylene- and -(5-12 membered)heteroarylene-, wherein the -(C₆-C₁₀)arylene- and -(5-12 membered)heteroarylene- are optionally substituted with one or more R⁷;
each R⁷ is independently selected from hydrogen, halogen, cyano, nitro, -OH, -SH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH₂, -C(=O)-NH(C₁-C₆)alkyl, -C(=O)-N[(C₁-C₆)alkyl]₂, and -NHC(=O)-(C₁-C₆)alkyl, wherein the -(C₁-C₆)alkyl, -(C₂-C₆)alkenyl, -(C₂-C₆)alkynyl, -(C₁-C₆)haloalkyl, -O-(C₁-C₆)alkyl, - O-(C₁-C₆)haloalkyl, -S-(C₁-C₆)alkyl, -NH(C₁-C₆)alkyl, -N[(C₁-C₆)alkyl]₂, -(C₃-C₆)cycloalkyl, - (4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH(C₁-C₆)alkyl, -C(=O)-N[(C₁-C₆)alkyl]₂, and -NHC(=O)-(C₁-C₆)alkyl are optionally substituted with one or more groups selected from hydrogen, halogen, oxo, -OH, -O-(C₁-C₄)alkyl, -NH₂, -NH(C₁-C₄)alkyl, and -N[(C₁-C₄)alkyl]₂;
m is 1, 2, 3, 4, or 5;
R^{8a}, R^{8b}, R^{9a}, and R^{9b} are each independently selected from hydrogen, halogen, cyano, -(C₁-C₆)alkyl, -(C₁-C₆)haloalkyl, -(C₂-C₆)alkenyl, -(C₂-C₆)alkynyl, -OR, -N(R)R, -[(C₁-C₈)alkylene]R, -[(C₁-C₈)alkylene]OR, -[(C₁-C₈)alkylene]N(R)R, -[(C₁-C₈)alkylene]N(R)C(=O)R, -[(C₁-C₈)alkylene]N(R)C(=O)N(R)R, -C(=O)R, -C(=O)N(R)R, -C(=O)[(C₁-C₈)alkylene]N(R)R, - C(=O)OR, -C(=S)N(R)R, -SR, -S(=O)R, -S(=O)₂R, -S(=O)₂N(R)R, -N(R)C(=O)R, - N(R)C(=O)N(R)R, -P(=O)(OR)(OR), -(C₆-C₁₀)aryl, -(5-12 membered)heteroaryl, -(C₃-C₆)cycloalkyl, and -(4-10 membered)heterocyclyl,
wherein each R is independently selected from hydrogen, -OH, -NH₂, -(C₁-C₆)alkyl, -(C₂-C₆)alkenyl, -(C₂-C₆)alkynyl, -(C₁-C₆)haloalkyl, -O-(C₁-C₆)alkyl, -(C₁-C₄)alkylene-O-(C₁-C₆)alkyl, -O-(C₁-C₆)haloalkyl, -S-(C₁-C₆)alkyl, -S(=O)₂-(C₁-C₆)alkyl, -(C₃-C₆)cycloalkyl, -(4-10 membered)heterocyclyl, -O-(C₃-C₆)cycloalkyl, -O-(4-10 membered)heterocyclyl, -C(=O)-H, - C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, -C(=O)-NH₂, -C(=O)-NH(C₁-C₆)alkyl, -C(=O)-N[(C₁-C₆)alkyl]₂, -[(C₁-C₆)alkylene]-(C₃-C₆)cycloalkyl,-[(C₁-C₆)alkylene]-(4-10 membered)heterocyclyl, -(C₆-C₁₀)aryl,-[(C₁-C₆)alkylene]-(C₆-C₁₀)aryl, and -(5-12 membered)heteroaryl, wherein the -(C₁-C₆)alkyl, -(C₂-C₆)alkenyl, -(C₂-C₆)alkynyl, -(C₁-C₆)haloalkyl, -O-(C₁-C₆)alkyl, -(C₁-C₄)alkylene-O-(C₁-C₆)alkyl, -O-(C₁-C₆)haloalkyl, -S-(C₁-C₆)alkyl, -S(=O)₂-(C₁-C₆)alkyl, -(C₃-C₆)cycloalkyl, -(4-10 membered)heterocyclyl, -O-(C₃-C₆)cycloalkyl, -O-(4-10 membered)heterocyclyl, -C(=O)-(C₁-C₆)alkyl, -C(=O)-(C₃-C₆)cycloalkyl, -C(=O)-O-(C₁-C₆)alkyl, -C(=O)-O-(C₃-C₆)cycloalkyl, - C(=O)-NH(C₁-C₆)alkyl, -C(=O)-N[(C₁-C₆)alkyl]₂, -[(C₁-C₆)alkylene]-(C₃-C₆)cycloalkyl, -[(C₁-C₆)alkylene]-(4-10 membered)heterocyclyl, -(C₆-C₁₀)aryl, -[(C₁-C₆)alkylene]-(C₆-C₁₀)aryl, and - (5-12 membered)heteroaryl are optionally substituted with one or more groups selected from hydrogen, halogen, -OH, -O-(C₁-C₄)alkyl, -NH₂, -NH(C₁-C₄)alkyl, N[(C₁-C₄)alkyl]₂, and (C₁-C₄)alkyl;
or R^{8a} and R^{8b} are combined to form oxo, -(C₂-C₆)alkenyl, -(C₃-C₆)cycloalkyl, or -(4-10 membered)heterocyclyl;
or R^{9a} and R^{9b} are combined to form oxo, -(C₂-C₆)alkenyl, -(C₃-C₆)cycloalkyl, or -(4-10 membered)heterocyclyl;
or R^{8a} and R^{9a}, together with the carbon atoms to which they are attached, form (C₃-C₆)cycloalkyl, (4-10 membered)heterocyclyl, or (5-10 membered)heteroaryl, wherein the aforementioned group is optionally substituted with one or more groups selected from hydrogen, halogen, -OH, -O-(C₁-C₄)alkyl, -NH₂, -NH(C₁-C₄)alkyl, -N[(C₁-C₄)alkyl]₂, -(C₃-C₆)cycloalkyl, -(4-8 membered)heterocyclyl, -(5-6 membered)heteroaryl, and -(C₆-C₁₀)aryl;
or R^{8b} and R^{9b}, together with the carbon atoms to which they are attached, form (C₃-C₆)cycloalkyl, (4-10 membered)heterocyclyl, or (5-10 membered)heteroaryl, wherein the aforementioned group is optionally substituted with one or more groups selected from hydrogen, halogen, -OH, -O-(C₁-C₄)alkyl, -NH₂, -NH(C₁-C₄)alkyl, -N[(C₁-C₄)alkyl]₂, -(C₃-C₆)cycloalkyl, -(4-8 membered)heterocyclyl, -(5-6 membered)heteroaryl, and -(C₆-C₁₀)aryl;
R¹⁰ is selected from hydrogen, cyano, -OH, and -NH₂, or R¹⁰ and R^{9a}, together with the atoms to which they are attached, form (5-6 membered)heterocyclyl, wherein the (5-6 membered)heterocyclyl is optionally substituted with one or more groups selected from hydrogen, halogen, -OH, -(C₁-C₄)alkyl, -(C₃-C₆)cycloalkyl, -O-(C₁-C₄)alkyl, -NH₂, -NH(C₁-C₄)alkyl, and - N[(C₁-C₄)alkyl]₂.

2. The ligand-drug conjugate or the racemic mixture, tautomer, stereoisomer, enantiomer, or diastereoisomer thereof, or the mixture thereof, or the pharmaceutically acceptable salt or solvate thereof according to claim 1, wherein:
R^{m}, R^{w}, R^{y}, R^{q}, and R^{t} are each independently selected from hydrogen, -(C₁-C₄)alkyl, -O-(C₁-C₄)alkyl, -(C₁-C₄)haloalkyl, and -O-(C₁-C₄)haloalkyl;
preferably, R^{m}, R^{w}, R^{y}, R^{q}, and R^{t} are each independently selected from hydrogen, methyl, ethyl, propyl, -O-methyl, -O-ethyl, and -O-propyl;
preferably, R^{m}, R^{w}, R^{y}, R^{q}, and R^{t} are each independently hydrogen and methyl;
preferably, R^{m}, R^{w}, R^{y}, R^{q}, and R^{t} are each independently hydrogen.

3. The ligand-drug conjugate or the racemic mixture, tautomer, stereoisomer, enantiomer, or diastereoisomer thereof, or the mixture thereof, or the pharmaceutically acceptable salt or solvate thereof according to claim 1 or 2, wherein:
M is selected from -NH-, -O-, -(C₁-C₆)alkylene-O-, and -(C₁-C₆)alkylene-NH-, wherein the -(C₁-C₆)alkylene-O- and -(C₁-C₆)alkylene-NH- are optionally substituted with 1, 2, or 3 groups selected from hydrogen, halogen, -OH, -O-methyl, -NH₂, -NH-methyl, -N(methyl)₂, and -N(ethyl)₂;
preferably, M is selected from -NH-, -O-, -(C₁-C₆)alkylene-O-, and -(C₁-C₆)alkylene-NH-;
preferably, M is selected from -NH-, -O-, -(C₁-C₄)alkylene-O-, and -(C₁-C₄)alkylene-NH-;
preferably, M is selected from -NH-, -O-, -CH₂O-, and -CH₂NH-;
preferably, M is selected from -NH-, -O-, *-CH₂O-, and *-CH₂NH-, wherein the * end is attached to ring C;
preferably, M is selected from -NH-.

4. The ligand-drug conjugate or the racemic mixture, tautomer, stereoisomer, enantiomer, or diastereoisomer thereof, or the mixture thereof, or the pharmaceutically acceptable salt or solvate thereof according to claim 1 or 2, wherein:
W is absent or is selected from -(C₁-C₆)alkylene-, -NH-, -N[(C₁-C₆)alkyl]-, and -N[O(C₁-C₆)alkyl]-;
preferably, W is absent or is selected from -(C₁-C₄)alkylene, -NH-, and -N[-(C₁-C₄)alkyl]-;
preferably, W is absent or is selected from methylene, ethylene, -NH-, -N(Me)-, and -N(Et)-;
preferably, W is absent or is selected from -NH- and -N(Me)-; U is selected from -(C₁-C₆)alkylene-, -(C₃-C₆)cycloalkylene-, and -(4-10 membered)heterocyclylene-;
preferably, U is selected from -(C₁-C₄)alkylene-, -(C₃-C₆)cycloalkylene-, and -(4-6 membered)heterocyclylene-;
preferably, U is selected from methylene, ethylene, cyclopropylene, azetidinylene, and pyrrolidinylene;
preferably, U is selected from methylene, ethylene, and 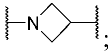
preferably, U is selected from methylene, ethylene, 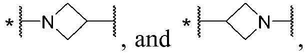 wherein the * end is attached to W, and when W is absent, the * end is attached to carbonyl;
Z is absent or is selected from -(C₁-C₄)alkylene-, -(C₃-C₆)cycloalkylene-, and -(4-6 membered)heterocyclylene-;
preferably, Z is absent or is selected from methylene, ethylene, cyclopropylene, azetidinylene, and pyrrolidinylene;
preferably, Z is absent;
Y is absent or is selected from -O-, -N(R^{y})-, and -N⁺(R^{y})₂-, wherein R^{y} is selected from hydrogen, -(C₁-C₆)alkyl, and -O(C₁-C₆)alkyl, or R^{y} and Z form -(4-10 membered)heterocyclylene-;
preferably, Y is absent or is selected from -O-, -N(R^{y})-, and -N⁺(R^{y})₂-, wherein R^{y} is selected from hydrogen, -(C₁-C₄)alkyl, and -O(C₁-C₄)alkyl, or R^{y} and Z form -(4-6 membered)heterocyclylene-;
preferably, Y is absent or is selected from -O-, -N(R^{y})-, and -N⁺(R^{y})₂-, wherein R^{y} is selected from hydrogen, methyl, ethyl, and -O-methyl;
preferably, Y is selected from -O-, -NH-, -N(CH₃)-, and -N⁺(CH₃)₂-.

5. The ligand-drug conjugate or the racemic mixture, tautomer, stereoisomer, enantiomer, or diastereoisomer thereof, or the mixture thereof, or the pharmaceutically acceptable salt or solvate thereof according to claim 1 or 2, wherein:
the structural unit -W-U-Z-Y- is selected from -N(R^{w})-(C₁-C₆)alkylene-N(R^{y})-, -N(R^{w})-(C₁-C₆)alkylene-N⁺(R^{y})₂-, -N(R^{w})-(C₁-C₆)alkylene-O-, -(C₁-C₆)alkylene-N(R^{y})-, -(C₁-C₆)alkylene-O-, -(C₃-C₆)cycloalkylene-N(R^{y})-, -(C₁-C₆)alkylene-N⁺(R^{y})₂-, -(C₃-C₆)cycloalkylene-O-, -(4-6 membered)heterocyclylene-O-, -(4-6 membered)heterocyclylene-N(R^{y})-, and -N(R^{w})-(4-6 membered)heterocyclylene-, wherein the -N(R^{w})-(C₁-C₆)alkylene-N(R^{y})-, -N(R^{w})-(C₁-C₆)alkylene-N⁺(R^{y})₂-, -N(R^{w})-(C₁-C₆)alkylene-O-, -(C₁-C₆)alkylene-N(R^{y})-, -(C₁-C₆)alkylene-N⁺(R^{y})₂-, -(C₁-C₆)alkylene-O-, -(C₃-C₆)cycloalkylene-N(R^{y})-, -(C₃-C₆)cycloalkylene-O-, -(4-6 membered)heterocyclylene-O-, -(4-6 membered)heterocyclylene-N(R^{y})-, and -N(R^{w})-(4-6 membered)heterocyclylene- are optionally substituted with one or more (e.g., 1, 2, 3, 4, 5, 6, or 7) groups selected from hydrogen, halogen, -OH, -O-(C₁-C₄)alkyl, -NH₂, -NH(C₁-C₄)alkyl, and - N[(C₁-C₄)alkyl]₂;
preferably, the structural unit -W-U-Z-Y- is selected from -N(R^{w})-(C₁-C₆)alkylene-N(R^{y})-, -N(R^{w})-(C₁-C₆)alkylene-N⁺(R^{y})₂-, -N(R^{w})-(C₁-C₆)alkylene-O-, -(C₁-C₆)alkylene-N(R^{y})-, -(C₁-C₆)alkylene-N⁺(R^{y})₂-, -(C₁-C₆)alkylene-O-, -(4-6 membered)heterocyclylene-O-, -(4-6 membered)heterocyclylene-N(R^{y})-, and -N(R^{w})-(4-6 membered)heterocyclylene-;
preferably, the structural unit -W-U-Z-Y- is selected from *-NH-CH₂-CH₂-NH-, *-N(CH₃)-CH₂-CH₂-NH-, *-NH-CH₂-CH₂-O-, *-CH₂-O-, *-CH₂-NH-, *-CH₂-N(CH₃)-, *-CH₂-N⁺(CH₃)₂-, 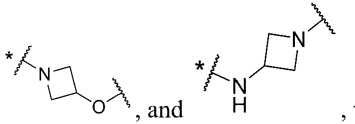 wherein the * end is attached to carbonyl.

6. The ligand-drug conjugate or the racemic mixture, tautomer, stereoisomer, enantiomer, or diastereoisomer thereof, or the mixture thereof, or the pharmaceutically acceptable salt or solvate thereof according to claim 1 or 2, wherein:
R¹² is selected from hydrogen, -(C₁-C₆)alkyl, -O-(C₁-C₆)alkyl, -(C=O)-(C₁-C₆)alkyl, -(C₃-C₆)cycloalkyl, -O-(C₃-C₆)cycloalkyl, -(4-10 membered)heterocyclyl, and -O-(4-10 membered)heterocyclyl;
preferably, R¹² is selected from hydrogen, -(C₁-C₄)alkyl, -O-(C₁-C₄)alkyl, -(C=O)-(C₁-C₄)alkyl, - (C₃-C₆)cycloalkyl, and -O-(C₃-C₆)cycloalkyl;
preferably, R¹² is selected from hydrogen, -(C₁-C₄)alkyl, -O-(C₁-C₄)alkyl, and -(C₃-C₆)cycloalkyl;
preferably, R¹² is selected from hydrogen and -(C₁-C₄)alkyl;
preferably, R¹² is selected from hydrogen, methyl, and ethyl;
preferably, R¹² is selected from hydrogen and methyl;
preferably, R¹² is selected from hydrogen;
L is absent or is selected from -C(=O)-(C₁-C₄)alkylene-O-, -C(=O)-(C₁-C₄)alkylene-N(R^{L})-, - C(=O)-(C₁-C₄)alkylene-N⁺(R^{L})₂-, -C(=O)NH-(C₁-C₄)alkylene-O-, -C(=O)NH-(C₁-C₄)alkylene-N(R^{L})-, and -C(=O)NH-(C₁-C₄)alkylene-N⁺(R^{L})₂-, wherein R^{L} is selected from hydrogen, -(C₁-C₄)alkyl, and -(C₁-C₄)haloalkyl;
preferably, L is absent or is selected from -C(=O)-(C₁-C₄)alkylene-O-, -C(=O)-(C₁-C₄)alkylene-N(R^{L})-, and -C(=O)-(C₁-C₄)alkylene-N⁺(R^{L})₂-, wherein R^{L} is selected from hydrogen, methyl, and ethyl;
preferably, L is absent or is selected from -C(=O)CH₂O-, -C(=O)CH₂CH₂O-, -C(=O)CH₂N(R^{L})-, -C(=O)CH₂CH₂N(R^{L})-, -C(=O)CH₂N⁺(R^{L})₂-, and -C(=O)CH₂CH₂N⁺(R^{L})₂-, wherein R^{L} is selected from hydrogen, methyl, and ethyl;
preferably, L is absent or is selected from *-C(=O)CH₂O-, *-C(=O)CH₂N(CH₃)-, and *-C(=O)CH₂N⁺(CH₃)₂-, wherein the * end is attached to N.

7. The ligand-drug conjugate or the racemic mixture, tautomer, stereoisomer, enantiomer, or diastereoisomer thereof, or the mixture thereof, or the pharmaceutically acceptable salt or solvate thereof according to claim 1 or 2, wherein:
Q is selected from -NH-, -O-, -(C₁-C₆)alkylene-O-, and -(C₁-C₆)alkylene-NH-, wherein the -(C₁-C₆)alkylene-O- and -(C₁-C₆)alkylene-NH- are optionally substituted with 1, 2, or 3 groups selected from hydrogen, halogen, -OH, -O-methyl, -NH₂, -NH-methyl, -N(methyl)₂, and -N(ethyl)₂;
preferably, Q is selected from -NH-, -O-, -(C₁-C₆)alkylene-O-, and -(C₁-C₆)alkylene-NH-;
preferably,Q is selected from -NH-, -O-, -(C₁-C₄)alkylene-O-, and -(C₁-C₄)alkylene-NH-;
preferably,Q is selected from -NH-, -O-, -CH₂O-, and -CH₂NH-;
preferably,Q is selected from -NH-, -O-, *-CH₂O-, and *-CH₂NH-, wherein the * end is attached to ring B;
preferably, Q is selected from -NH-.

8. The ligand-drug conjugate or the racemic mixture, tautomer, stereoisomer, enantiomer, or diastereoisomer thereof, or the mixture thereof, or the pharmaceutically acceptable salt or solvate thereof according to claim 1 or 2, wherein:
T is selected from -NH-, -O-, -(C₁-C₆)alkylene-O-, and -(C₁-C₆)alkylene-NH-, wherein the -(C₁-C₆)alkylene-O- and -(C₁-C₆)alkylene-NH- are optionally substituted with 1, 2, or 3 groups selected from hydrogen, halogen, -OH, -O-methyl, -NH₂, -NH-methyl, -N(methyl)₂, and -N(ethyl)₂;
preferably, T is selected from -NH-, -O-, -(C₁-C₆)alkylene-O-, and -(C₁-C₆)alkylene-NH-;
preferably, T is selected from -NH-, -O-, -(C₁-C₄)alkylene-O-, and -(C₁-C₄)alkylene-NH-;
preferably, T is selected from -NH-, -O-, -CH₂O-, and -CH₂NH-;
preferably, T is selected from -NH-, -O-, *-CH₂O-, and *-CH₂NH-, wherein the * end is attached to ring A;
preferably, T is selected from -NH-, *-CH₂O-, and *-CH₂NH-, wherein the * end is attached to ring A;
preferably, T is selected from -NH- and *-CH₂NH-, wherein the * end is attached to ring A.

9. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-8, wherein R¹ is selected from hydrogen, halogen, cyano, -OH, -(C₁-C₄)alkyl, and -O-(C₁-C₄)alkyl;
preferably, R¹ is selected from hydrogen, fluorine, chlorine, cyano, -OH, methyl, ethyl, propyl, - O-methyl, -O-ethyl, and -O-propyl;
preferably, R¹ is selected from hydrogen and -O-methyl;
preferably, X¹ is selected from N, CH, and C(OCH₃).

10. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-9, wherein R² is selected from hydrogen, halogen, cyano, -OH, -(C₁-C₄)alkyl, and -O-(C₁-C₄)alkyl;
preferably, R² is selected from hydrogen, fluorine, chlorine, cyano, -OH, methyl, ethyl, propyl, - O-methyl, -O-ethyl, and -O-propyl;
preferably, R² is hydrogen;
preferably, X² is selected from N and CH.

11. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-10, wherein:
R³ is selected from -(C₃-C₆)cycloalkyl, -(4-6 membered)heterocyclyl, -(C₆-C₁₀)aryl, and -(5-12 membered)heteroaryl, wherein the -(C₃-C₆)cycloalkyl, -(4-6 membered)heterocyclyl, -(C₆-C₁₀)aryl, and -(5-12 membered)heteroaryl are optionally substituted with one or more R³¹;
ring A is selected from -(C₃-C₆)cycloalkylene-, -(4-6 membered)heterocyclylene-, -(C₆-C₁₀)arylene-, and -(5-12 membered)heteroarylene-, wherein the -(C₃-C₆)cycloalkylene-, -(4-6 membered)heterocyclylene-, -(C₆-C₁₀)arylene-, and -(5-12 membered)heteroarylene- are optionally substituted with one or more R³¹;
R³¹ is selected from oxo, hydrogen, halogen, cyano, nitro, -OH, -SH, -NH₂, -(C₁-C₄)alkyl, -(C₁-C₄)alkylene-OH, -(C₁-C₄)alkylene-NH₂, -(C₁-C₄)alkylene-NH(C₁-C₄)alkyl, -(C₁-C₄)alkylene-N[(C₁-C₄)alkyl]₂, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, -O-(4-8 membered)heterocyclyl, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, - C(=O)-NH₂, -C(=O)-NH(C₁-C₄)alkyl, and -C(=O)-N[(C₁-C₄)alkyl]₂;
preferably, R³¹ is selected from oxo, hydrogen, halogen, cyano, -OH, -SH, -NH₂, -(C₁-C₄)alkyl, - (C₁-C₄)haloalkyl, -O-(C₁-C₄)alkyl, -NH-(C₁-C₄)alkyl, -(C₁-C₄)alkylene-OH, -(C₁-C₄)alkylene-NH₂, -(C₁-C₄)alkylene-NH(C₁-C₄)alkyl, and -(C₁-C₄)alkylene-N[(C₁-C₄)alkyl]₂;
preferably, R³¹ is selected from oxo, hydrogen, fluorine, chlorine, cyano, -OH, -SH, -NH₂, methyl, halomethyl, -O-methyl, -CH₂OH, -CH₂NH₂, and -CH₂CH₂N(CH₃)₂;
preferably, R³¹ is selected from hydrogen, -NH₂, -CH₂OH, and -CH₂NH₂;
preferably, R³¹ is selected from hydrogen;
preferably, R³ is selected from -(C₃-C₆)cycloalkyl and -(4-6 membered)heterocyclyl, wherein the -(C₃-C₆)cycloalkyl and -(4-6 membered)heterocyclyl are optionally substituted with one or more R³¹, wherein R³¹ is defined as described in the present claim;
preferably, R³ is selected from cyclopropyl, oxetanyl, tetrahydrofuranyl, azetidinyl, pyrrolidinyl, morpholinyl, morpholinonyl, piperidinyl, piperazinyl, and piperazinonyl, wherein the cyclopropyl, oxetanyl, tetrahydrofuranyl, azetidinyl, pyrrolidinyl, morpholinyl, morpholinonyl, piperidinyl, piperazinyl, and piperazinonyl are optionally substituted with 1, 2, or 3 R³¹, wherein R³¹ is defined as described in the present claim;
preferably, R³ is selected from 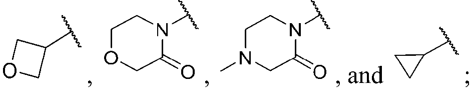
preferably, R³ is selected from phenyl, naphthyl, and -(5-10 membered)heteroaryl, wherein the phenyl, naphthyl, and -(5-10 membered)heteroaryl are optionally substituted with one or more R³¹,
wherein R³¹, is defined as described in the present claim;
preferably, R³ is selected from -(5-9 membered)heteroaryl, wherein the -(5-9 membered)heteroaryl is optionally substituted with 1, 2, 3, or 4 R³¹, wherein R³¹, is defined as described in the present claim;
preferably, R³ is selected from oxazolyl, isoxazolyl, thiazolyl, isothiazolyl, pyrazolyl, imidazolyl, triazolyl, tetrazolyl, pyridinyl, pyrazinyl, pyridazinyl, pyrimidinyl, pyridazinoimidazolyl, pyrazinoimidazolyl, and pyrimidinonyl, wherein the oxazolyl, isoxazolyl, thiazolyl, isothiazolyl, pyrazolyl, imidazolyl, triazolyl, tetrazolyl, pyridinyl, pyrazinyl, pyridazinyl, pyrimidinyl, pyridazinoimidazolyl, pyrazinoimidazolyl, and pyrimidinonyl are optionally substituted with 1, 2,
or 3 R³¹, wherein R³¹, is defined as described in the present claim;
preferably, R³ is selected from 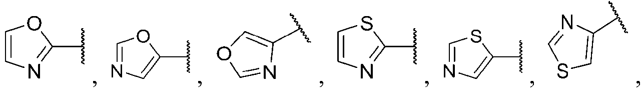 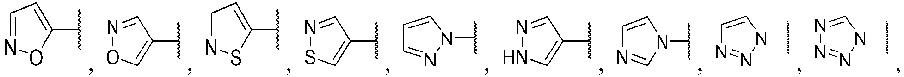 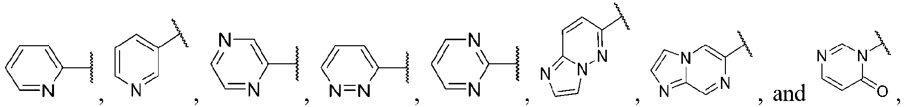 wherein the 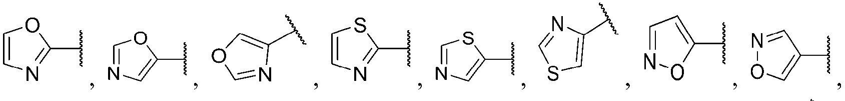 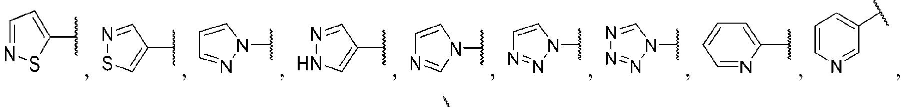 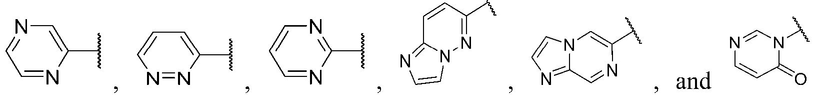 are optionally substituted with 1, 2, or 3 R³¹, wherein R³¹, is defined as described in the present claim;
preferably, R³ is selected from 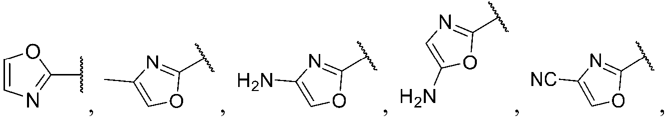 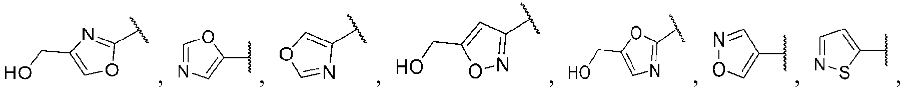 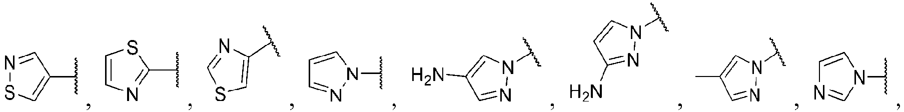 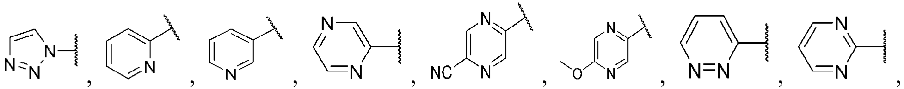 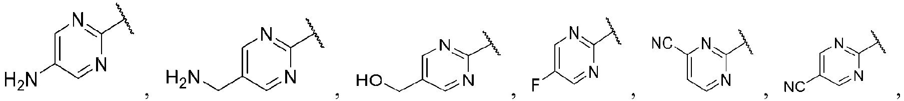 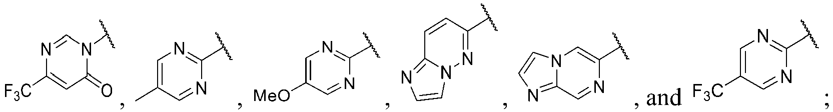
more preferably, R³ is selected from 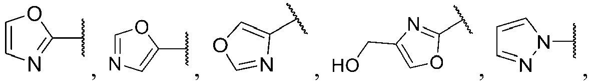 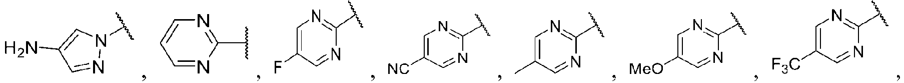 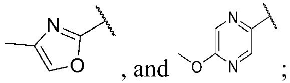
more preferably, R³ is selected from 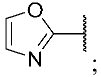
preferably, ring A is selected from phenylene, naphthylene, and -(5-10 membered)heteroarylene-, wherein the phenylene, naphthylene, and -(5-10 membered)heteroarylene- are optionally substituted with one or more R³¹, wherein R³¹, is defined as described in the present claim;
preferably, ring A is selected from -(5-9 membered)heteroarylene-, wherein the -(5-9 membered)heteroarylene- is optionally substituted with 1, 2, 3, or 4 R³¹, wherein R³¹ is defined as described in the present claim;
preferably, ring A is selected from -(5-6 membered)heteroarylene-, wherein the -(5-6 membered)heteroarylene- is optionally substituted with 1, 2, 3, or 4 R³¹, wherein R³¹, is defined as described in the present claim;
preferably, ring A is selected from oxazolylene, isoxazolylene, thiazolylene, isothiazolylene, pyrazolylene, imidazolylene, triazolylene, tetrazolylene, pyridinylene, pyrazinylene, pyridazinylene, pyrimidinylene, pyridazinoimidazolylene, pyrazinoimidazolylene, and
pyrimidinonylene, wherein the oxazolylene, isoxazolylene, thiazolylene, isothiazolylene, pyrazolylene, imidazolylene, triazolylene, tetrazolylene, pyridinylene, pyrazinylene, pyridazinylene, pyrimidinylene, pyridazinoimidazolylene, pyrazinoimidazolylene, and pyrimidinonylene are optionally substituted with 1, 2, or 3 R³¹, wherein R³¹, is defined as described in the present claim;
preferably, ring A is selected from 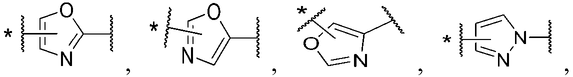 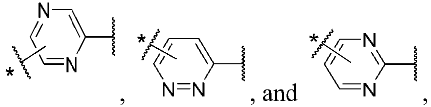 wherein the * end is attached to T;
preferably, ring A is selected from 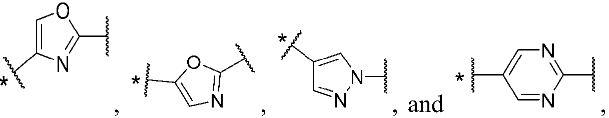 wherein the * end is attached to T.

12. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-11, wherein R⁴ is selected from hydrogen, halogen, cyano, -OH, -NH₂, -(C₁-C₄)alkyl, -(C₁-C₄)haloalkyl, -O-(C₁-C₄)alkyl, and -O-(C₁-C₄)haloalkyl;
preferably, R⁴ is selected from hydrogen, fluorine, chlorine, cyano, -OH, -NH₂, and methyl;
preferably, R⁴ is hydrogen.

13. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-12, wherein X is selected from O, S, NH, N[(C₁-C₄)alkyl], N[C(=O)-R^{c}], C(R^{a})(R^{b}), C(=O), C[=C(R^{a})R^{b}], S(=O), and S(=O)₂, wherein R^{a} and R^{b} are each independently selected from hydrogen, halogen, cyano, -OR^{c}, -SR^{c}, -N(R^{c})R^{c}, -(C₁-C₄)alkyl, and -(C₁-C₄)haloalkyl, wherein R^{c} is selected from hydrogen, -(C₁-C₄)alkyl, -(C₁-C₄)haloalkyl, -O-(C₁-C₄)alkyl, -O-(C₁-C₄)haloalkyl, and -(C₃-C₆)cycloalkyl;
preferably, X is selected from O, S, NH, and N[(C₁-C₄)alkyl];
preferably, X is selected from O, S, and NH;
more preferably, X is selected from O.

14. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-13, wherein ring B is selected from -(C₆-C₁₀)aryl and (5-10 membered)heteroaryl, or ring B is selected from -(C₆-C₁₀)arylene- and (5-10 membered)heteroarylene-, wherein the -(C₆-C₁₀)arylene- and (5-10 membered)heteroarylene- are optionally substituted with 1, 2, or 3 groups selected from R⁵ and R⁶;
preferably, ring B is selected from phenyl, naphthyl, and (5-6 membered)heteroaryl;
preferably, the structural unit 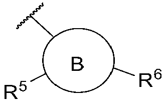
is 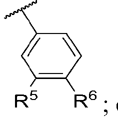
or
preferably, ring B is selected from phenylene, naphthylene, and (5-6 membered)heteroarylene,
wherein the phenylene, naphthylene, and (5-6 membered)heteroarylene are optionally substituted with 1 or 2 groups selected from R⁵ and R⁶;
preferably, ring B is phenylene, wherein the phenylene is optionally substituted with 1 R⁵ or R⁶;
preferably, ring B is 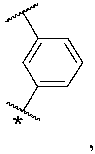
wherein the * end is attached to Q;
R⁵ and R⁶ are each independently selected from hydrogen, halogen, cyano, nitro, -OH, -SH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, and -O-(4-8 membered)heterocyclyl, wherein the -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, and -O-(4-8 membered)heterocyclyl are optionally substituted with 1, 2, 3, 4, or 5 groups selected from hydrogen, halogen, -OH, -O-(C₁-C₄)alkyl, - NH₂, -NH(C₁-C₄)alkyl, and N[(C₁-C₄)alkyl]₂;
preferably, R⁵ and R⁶ are each independently selected from hydrogen, halogen, cyano, -OH, -NH₂, -(C₁-C₄)alkyl, -(C₁-C₄)alkyl-OH, -(C₁-C₄)alkyl-NH₂, -(C₁-C₄)haloalkyl, -O-(C₁-C₄)alkyl, -O-(C₁-C₄)haloalkyl, and -(C₃-C₆)cycloalkyl;
preferably, R⁵ is selected from hydrogen, fluorine, chlorine, bromine, cyano, -OH, -NH₂, and - CH₂NH₂;
preferably, R⁵ is selected from hydrogen, -OH, -NH₂, and -CH₂NH₂;
preferably, R⁶ is selected from hydrogen, fluorine, chlorine, bromine, cyano, -OH, -NH₂, -O-methyl, and cyclopropyl;
or R⁵ and R⁶, together with the atoms to which they are attached, form 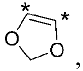
wherein * indicates the position of the attached atom.

15. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-14, wherein ring C is selected from -(C₆-C₁₀)aryl and (5-10 membered)heteroaryl, or ring C is selected from -(C₆-C₁₀)arylene and (5-10 membered)heteroarylene, wherein the -(C₆-C₁₀)arylene and (5-10 membered)heteroarylene are optionally substituted with 1, 2, or 3 R⁷;
preferably, ring C is selected from phenyl, naphthyl, and (5-6 membered)heteroaryl;
preferably, the structural unit 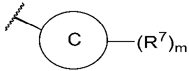
is 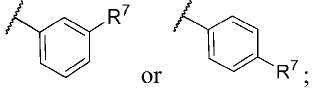
or
preferably, ring C is selected from phenylene, naphthylene, and (5-6 membered)heteroarylene, wherein the phenylene, naphthylene, and (5-6 membered)heteroarylene are optionally substituted with 1 or 2 R⁷;
preferably, ring C is phenylene, wherein the phenylene is optionally substituted with 1 R⁷;
preferably, ring C is 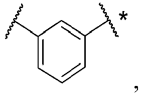
wherein the * end is attached to M;
each R⁷ is independently selected from hydrogen, halogen, cyano, nitro, -OH, -SH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, -O-(4-8 membered)heterocyclyl, and -NHC(=O)-(C₁-C₄)alkyl, wherein the -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -S-(C₁-C₄)alkyl, -NH(C₁-C₄)alkyl, -N[(C₁-C₄)alkyl]₂, -(C₃-C₆)cycloalkyl, -(4-8 membered)heterocyclyl, -O-(C₃-C₆)cycloalkyl, -0-(4-8 membered)heterocyclyl, and -NHC(=O)-(C₁-C₄)alkyl are optionally substituted with 1, 2, 3, 4, or 5 groups selected from hydrogen, halogen, -OH, -O-(C₁-C₄)alkyl, -NH₂, -NH(C₁-C₄)alkyl, and - N[(C₁-C₄)alkyl]₂;
preferably, each R⁷ is independently selected from hydrogen, halogen, cyano, -OH, -NH₂, -(C₁-C₄)alkylene-OH, -(C₁-C₄)alkylene-NH(C₁-C₄)alkyl, -(C₁-C₄)alkylene-N[(C₁-C₄)alkyl]₂, -(C₁-C₄)alkylene-NH₂, and -NHC(=O)-(C₁-C₄)alkylene-NH₂;
preferably, each R⁷ is independently selected from hydrogen, fluorine, chlorine, bromine, -OH, - NH₂, -CH₂OH, -CH₂NH₂, -NHC(=O)CH₂NH₂, and -NHC(=O)CH₂CH₂NH₂;
preferably, each R⁷ is independently selected from hydrogen;
preferably, m is 1, 2, or 3.

16. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-15, wherein R^{8a}, R^{8b}, R^{9a}, and R^{9b} are each independently selected from hydrogen, halogen, cyano, -(C₁-C₄)alkyl, -(C₁-C₄)haloalkyl, -(C₂-C₄)alkenyl, -(C₂-C₄)alkynyl, -OR, -N(R)R, -[(C₁-C₆)alkylene]R, -[(C₁-C₆)alkylene]OR, -[(C₁-C₆)alkylene]N(R)R, -[(C₁-C₆)alkylene]N(R)C(=O)R, -[(C₁-C₆)alkylene]N(R)C(=O)N(R)R, -C(=O)R, -C(=O)N(R)R, - C(=O)[(C₁-C₆)alkylene]N(R)R, -C(=O)OR, -C(=S)N(R)R, -SR, -S(=O)R, -S(=O)₂R, - S(=O)₂N(R)R, -N(R)C(=O)R, -N(R)C(=O)N(R)R, and -P(=O)(OR)(OR); each R is independently selected from hydrogen, -OH, -NH₂, -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -(C₁-C₄)alkylene-O-(C₁-C₄)alkyl, -S-(C₁-C₄)alkyl, -S(=O)₂-(C₁-C₄)alkyl, -(C₃-C₆)cycloalkyl, -(4-8 membered)heterocyclyl, -O-(C₃-C₆)cycloalkyl, -O-(4-8 membered)heterocyclyl, -C(=O)-H, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, -C(=O)-NH₂, -C(=O)-NH(C₁-C₄)alkyl, -[(C₁-C₄)alkylene]-(C₃-C₆)cycloalkyl, -C(=O)-N[(C₁-C₄)alkyl]₂, -[(C₁-C₄)alkylene]-(4-8 membered)heterocyclyl, -(C₆-C₁₀)aryl, -[(C₁-C₄)alkylene]-(C₆-C₁₀)aryl, and - (5-10 membered)heteroaryl, wherein the -(C₁-C₄)alkyl, -(C₂-C₄)alkenyl, -(C₂-C₄)alkynyl, -(C₁-C₄)haloalkyl, -O-(C₁-C₄)alkyl, -O-(C₁-C₄)haloalkyl, -(C₁-C₄)alkylene-O-(C₁-C₄)alkyl, -S-(C₁-C₄)alkyl, -S(=O)₂-(C₁-C₄)alkyl, -(C₃-C₆)cycloalkyl, -(4-8 membered)heterocyclyl, -O-(C₃-C₆)cycloalkyl, -O-(4-8 membered)heterocyclyl, -C(=O)-(C₁-C₄)alkyl, -C(=O)-(C₃-C₆)cycloalkyl, -C(=O)-O-(C₁-C₄)alkyl, -C(=O)-O-(C₃-C₆)cycloalkyl, -C(=O)-NH(C₁-C₄)alkyl, -[(C₁-C₄)alkylene]-(C₃-C₆)cycloalkyl, -C(=O)-N[(C₁-C₄)alkyl]₂, -[(C₁-C₄)alkylene]-(4-8 membered)heterocyclyl, -(C₆-C₁₀)aryl, -[(C₁-C₄)alkylene]-(C₆-C₁₀)aryl, and -(5-10 membered)heteroaryl are optionally substituted with 1, 2, 3, 4, 5, 6, or 7 groups selected from hydrogen, halogen, -OH, -O-(C₁-C₄)alkyl, -NH₂, -NH(C₁-C₄)alkyl, N[(C₁-C₄)alkyl]₂, and (C₁-C₄)alkyl;
preferably, each R is independently selected from hydrogen, -OH, -NH₂, -(C₁-C₄)alkyl, -(C₁-C₄)haloalkyl, -O-(C₁-C₄)alkyl, -(C₁-C₄)alkylene-O-(C₁-C₆)alkyl, -O-(C₁-C₄)haloalkyl, -S(=O)₂-(C₁-C₄)alkyl, -(C₃-C₆)cycloalkyl, -(4-8 membered)heterocyclyl, -C(=O)-H, -C(=O)-(C₁-C₄)alkyl, -C(=O)-(C₃-C₆)cycloalkyl, -[(C₁-C₄)alkylene]-(C₃-C₆)cycloalkyl, and -[(C₁-C₄)alkylene]-(4-8 membered)heterocyclyl, wherein the -(C₁-C₄)alkyl, -(C₁-C₄)haloalkyl, -O-(C₁-C₄)alkyl, -(C₁-C₄)alkylene-O-(C₁-C₆)alkyl, -O-(C₁-C₄)haloalkyl, -S(=O)₂-(C₁-C₄)alkyl, -(C₃-C₆)cycloalkyl, -(4-8 membered)heterocyclyl, -C(=O)-(C₁-C₄)alkyl, -C(=O)-(C₃-C₆)cycloalkyl, -[(C₁-C₄)alkylene]-(C₃-C₆)cycloalkyl, and -[(C₁-C₄)alkylene]-(4-8 membered)heterocyclyl are optionally substituted with 1, 2, 3, 4, or 5 groups selected from hydrogen, halogen, -OH, -O-(C₁-C₄)alkyl, -NH₂, -NH(C₁-C₄)alkyl, N[(C₁-C₄)alkyl]₂, methyl, and ethyl;
preferably, each R is independently selected from hydrogen, -OH, -NH₂, -CH₃, -CH₂CH₃, -CH₂OH, -CH₂CH₂OH, -CH₂OCH₃, -CH₂NH₂, -CH₂CH₂NH₂, -CH₂CH₂NHCH₃, -CH₂N(CH₃)₂, - CH(N(CH₃)₂)CH₂OH, -CH₂OCH₂CH₂OH, -OCH₃, -C(=O)-H, -C(=O)CH₃, -C(=O)CH₂OH, - S(=O)₂CH₃, cyclobutyl-OH, oxetanyl, azetidinyl, azetidinyl-OH, pyrrolidinyl, piperazinyl, azetidinyl-CH₃, piperazinyl-CH₃, and -CH₂-azetidinyl;
more preferably, each R is independently selected from hydrogen, -OH, -NH₂, -(C₁-C₄)alkyl, -O-(C₁-C₄)alkyl, and -(4-8 membered)heterocyclyl, wherein the -(C₁-C₄)alkyl, -O-(C₁-C₄)alkyl, and - (4-8 membered)heterocyclyl are optionally substituted with 1, 2, 3, 4, or 5 groups selected from hydrogen, halogen, -OH, -NH₂, -NH(C₁-C₄)alkyl, -N[(C₁-C₄)alkyl]₂, and -O-(C₁-C₄)alkyl;
more preferably, each R is independently selected from hydrogen, -OH, -(C₁-C₄)alkyl, -O-(C₁-C₄)alkyl, and -(4-8 membered)heterocyclyl, wherein the -(C₁-C₄)alkyl, -O-(C₁-C₄)alkyl, and -(4-8 membered)heterocyclyl are optionally substituted with 1, 2, or 3 groups selected from hydrogen, halogen, -OH, -NH₂, -NH-methyl, and -N(methyl)₂;
more preferably, each R is independently selected from methyl, ethyl, propyl, -O-methyl, -O-ethyl, -O-propyl, oxetanyl, and azetidinyl, wherein the aforementioned groups are each independently and optionally substituted with 1, 2, or 3 groups selected from -OH and -NH₂;
preferably, R^{8a}, R^{8b}, R^{9a}, and R^{9b} are each independently selected from hydrogen, cyano, -OR, - N(R)R, -[(C₁-C₄)alkylene]R, -[(C₁-C₄)alkylene]OR, -[(C₁-C₄)alkylene]N(R)R, -[(C₁-C₄)alkylene]N(R)C(=O)R, -[(C₁-C₄)alkylene]N(R)C(=O)N(R)R, -C(=O)R, -C(=O)N(R)R, - C(=O)[(C₁-C₄)alkylene]N(R)R, -C(=O)OR, -C(=S)N(R)R, -S(=O)R, -S(=O)₂R, -S(=O)₂N(R)R, - N(R)C(=O)R, and -N(R)C(=O)N(R)R, wherein R is defined as described in the present claim; preferably, R^{8a} and R^{8b} are each independently selected from hydrogen, -methylene-R, -ethylene-R, -propylene-R, -methylene-OR, -ethylene-OR, -propylene-OR, -methylene-N(R)R, -ethylene-N(R)R, -propylene-N(R)R, -C(=O)R, -C(=O)N(R)R, and -C(=O)OR, wherein R is defined as described in the present claim;
preferably, R^{8a} and R^{8b} are each independently selected from hydrogen, -methylene-R, -methylene-OR, -methylene-N(R)R, -C(=O)R, -C(=O)OR, and -C(=O)N(R)R, wherein R is defined as described in the present claim; for example, each R is independently selected from hydrogen, -OH, -NH₂, -(C₁-C₄)alkyl, -O-(C₁-C₄)alkyl, -C(=O)-(C₁-C₄)alkyl, -S(=O)₂(C₁-C₄)alkyl, and -(4-8 membered)heterocyclyl, wherein the -(C₁-C₄)alkyl and -(4-8 membered)heterocyclyl are each optionally substituted with 1, 2, 3, 4, or 5 groups selected from hydrogen, halogen, -OH, -NH₂, - NHCH₃, and CH₃;
preferably, R^{8a} is hydrogen, preferably 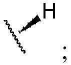
preferably, R^{8b} is selected from hydrogen, 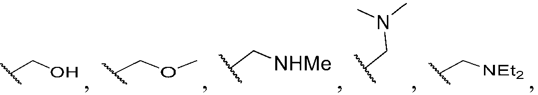 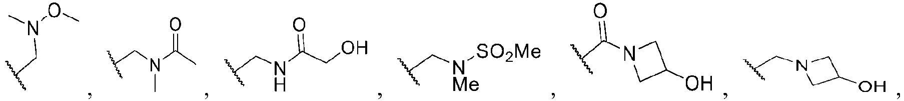 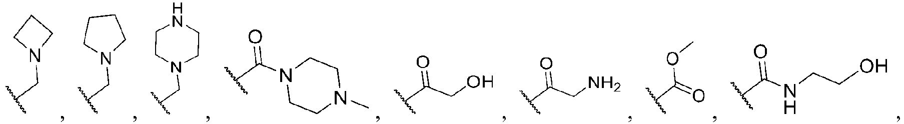 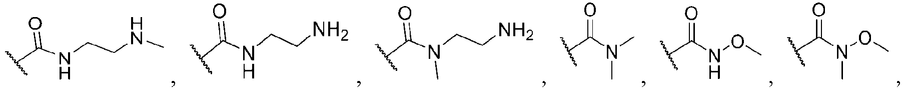 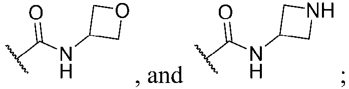
preferably, R^{8b} is selected from hydrogen, 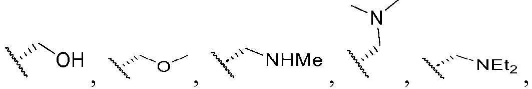 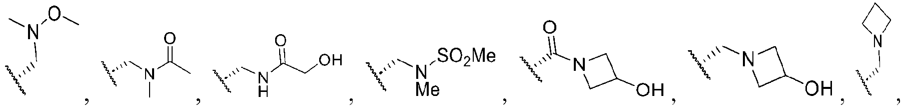 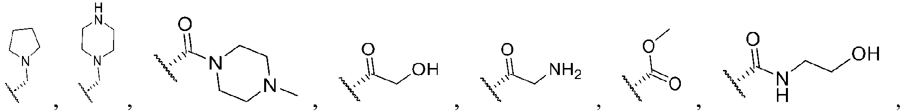 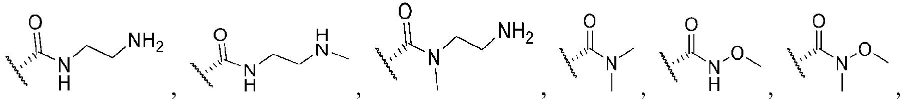 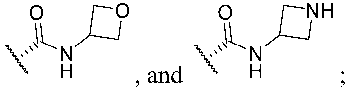
preferably, R^{9a} and R^{9b} are each independently selected from hydrogen, -CN, -OR, -NHR, -N(R)R, -NHC(=O)R, -methylene-R, -ethylene-R, -propylene-R, -methylene-N(R)R, -ethylene-N(R)R, - propylene-N(R)R, -methylene-NHC(=O)R, and -methylene-NHC(=O)NHR, wherein R is defined as described in the present claim;
preferably, R^{9a} and R^{9b} are each independently selected from hydrogen, -CN, -OR, -N(R)R, - NHC(=O)R, -methylene-N(R)R, -methylene-NHC(=O)R, and -methylene-NHC(=O)NHR, wherein R is defined as described in the present claim; for example, each R is independently selected from hydrogen, -OH, -(C₁-C₄)alkyl, -(C₁-C₄)alkylene-O-(C₁-C₄)alkyl, -S(=O)₂(C₁-C₄)alkyl, -(C₃-C₆)cycloalkyl, -(4-8 membered)heterocyclyl, -methylene-(C₃-C₆)cycloalkyl, and - methylene-(4-8 membered)heterocyclyl, wherein the -(C₁-C₄)alkyl, -(C₃-C₆)cycloalkyl, and -(4-8 membered)heterocyclyl are each optionally substituted with 1, 2, 3, 4, or 5 groups selected from hydrogen, halogen, -OH, -NH₂, -NH(C₁-C₄)alkyl, -N[(C₁-C₄)alkyl]₂, -O-(C₁-C₄)alkyl, and -(C₁-C₄)alkyl;
preferably, R^{9a} is selected from hydrogen, -CN, -N(R)R, -NHC(=O)R, -methylene-N(R)R, - methylene-NHC(=O)R, and -methylene-NHC(=O)NHR, wherein R is defined as described in the present claim; for example, each R is independently selected from hydrogen, -OH, methyl, ethyl, -methylene-O-ethyl, -S(=O)₂-methyl, cyclobutyl, azetidinyl, -methylene-cyclobutyl, and - methylene-azetidinyl, wherein the aforementioned groups are each independently and optionally substituted with 1, 2, or 3 groups selected from -OH, OCH₃, NHCH₃, N(CH₃)₂, and CH₃; preferably, R^{9a} is selected from hydrogen, -CN, -NHCH₃, -N(CH₃)₂, -NHC(=O)H, - NHC(=O)CH₂OH, -NHC(=O)CH₂OCH₃, -NHC(=O)CH₂NHCH₃, -NHC(=O)CH₂N(CH₃)₂, - CH₂NH₂, -CH₂NHCH₃, -CH₂N(CH₃)₂, -CH₂N(CH₂CH₃)₂, -CH₂N(CH₃)(CH₂CH₃), - CH₂NHC(=O)CH₂OH, -CH₂NHC(=O)CH₂OCH₃, -CH₂NHC(=O)CH₂NHCH₃, - CH₂NHC(=O)CH₂N(CH₃)₂, -CH₂NHC(=O)H, -CH₂NHC(=O)CH₃, -CH₂NHC(=O)NHOH, - CH₂NHC(=O)NHCH₂CH₂OH, -CH₂NHC(=O)CH(N(CH₃)₂)CH₂OH, -CH₂NHC(=O)CH₂NHCH₃, -CH₂NHC(=O)CH₂N(CH₃)₂, -CH₂NHC(=O)CH₂N(CH₂CH₃)₂, -CH₂NHC(=O)CH₂OCH₂CH₂OH, -CH₂NHS(=O)₂CH₃, -CH₂NHC(=O)cyclobutyl-OH, -CH₂NHC(=O)-azetidinyl-CH₃, and - CH₂NHC(=O)CH₂-azetidinyl;
preferably, R^{9a} is selected from hydrogen, 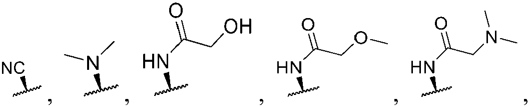 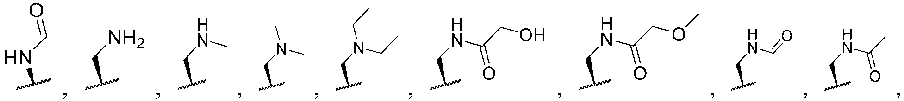 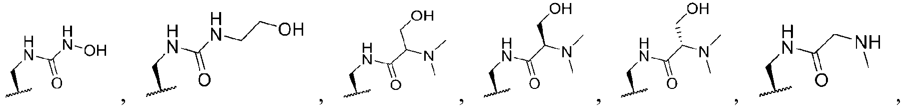 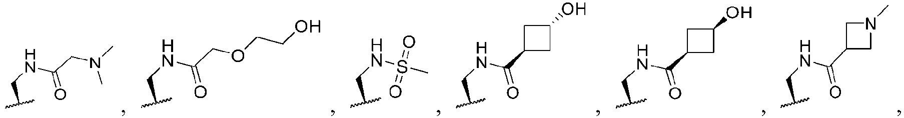 and 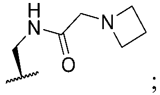
preferably, R^{9a} is 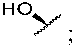 preferably, R^{9b} is selected from hydrogen and 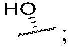
preferably, R^{9b} is -OH, preferably 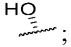 or R^{8a} and R^{8b} are combined to form oxo, -(C₂-C₄)alkenyl, -(C₃-C₆)cycloalkyl, or -(4-8 membered)heterocyclyl;
or R^{9a} and R^{9b} are combined to form oxo, -(C₂-C₄)alkenyl, -(C₃-C₆)cycloalkyl, or -(4-8 membered)heterocyclyl;
or R^{8a} and R^{9a}, together with the carbon atoms to which they are attached, form (C₃-C₆)cycloalkyl, (4-8 membered)heterocyclyl, or (5-9 membered)heteroaryl, wherein the aforementioned group is optionally substituted with 1, 2, 3, 4, 5, 6, or 7 groups selected from hydrogen, halogen, -OH, -O-(C₁-C₄)alkyl, -NH₂, -NH(C₁-C₄)alkyl, -N[(C₁-C₄)alkyl]₂, -(C₃-C₆)cycloalkyl, and -(C₆-C₁₀)aryl;
or R^{8b} and R^{9b}, together with the carbon atoms to which they are attached, form (C₃-C₆)cycloalkyl, (4-8 membered)heterocyclyl, or (5-9 membered)heteroaryl, wherein the aforementioned group is optionally substituted with 1, 2, 3, 4, 5, 6, or 7 groups selected from hydrogen, halogen, -OH, -O-(C₁-C₄)alkyl, -NH₂, -NH(C₁-C₄)alkyl, -N[(C₁-C₄)alkyl]₂, -(C₃-C₆)cycloalkyl, and -(C₆-C₁₀)aryl; preferably, R^{8a} and R^{9a} are absent, and R^{8b} and R^{9b}, together with the atoms to which they are attached, form 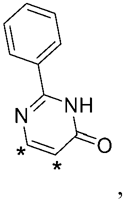 wherein * indicates the position of the attached atom.

17. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-16, wherein R¹⁰ is selected from -OH, or R¹⁰ and R^{9a}, together with the atoms to which they are attached, form 5-membered nitrogen-containing heterocyclyl, wherein the 5-membered nitrogen-containing heterocyclyl is optionally substituted with 1, 2, 3, 4, or 5 groups selected from hydrogen, halogen, -OH, -(C₁-C₄)alkyl, -NH₂, -NH(C₁-C₄)alkyl, and -N[(C₁-C₄)alkyl]₂;
preferably, R¹⁰ is -OH, or R¹⁰ and R^{9a}, together with the atoms to which they are attached, form imidazolinyl, wherein the imidazolinyl is optionally substituted with 1, 2, or 3 groups selected from halogen, -OH, methyl, -NH₂, -NH-methyl, and -N(methyl)₂;
preferably, R¹⁰ is -OH, or R¹⁰ and R^{9a}, together with the atoms to which they are attached, form 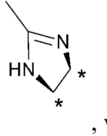 wherein * indicates the position of the attached atom;
preferably, R¹⁰ is -OH, preferably

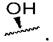

18. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-17, wherein the drug group is selected from:
a structure represented by formula I-A-1, II-A-1', III-A-1', IV-A-1, or V-A-1: 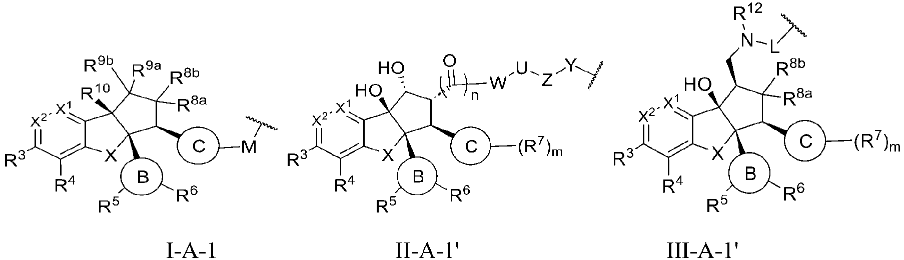 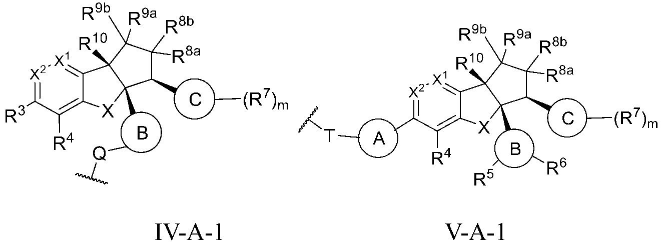
wherein X, X¹, X², ring A, ring B, ring C, R³, R⁴, R⁵, R⁶, R⁷, R^{8a}, R^{8b}, R^{9a}, R^{9b}, R¹⁰, R¹², m, n, M, W, U, Z, Y, L, Q, and T are defined as described in any one of claims 1-17;
or a structure represented by formula I-A-5, II-A-5, III-A-5, IV-A-5, or V-A-5: 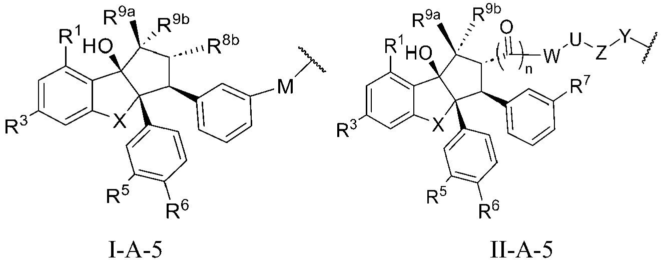 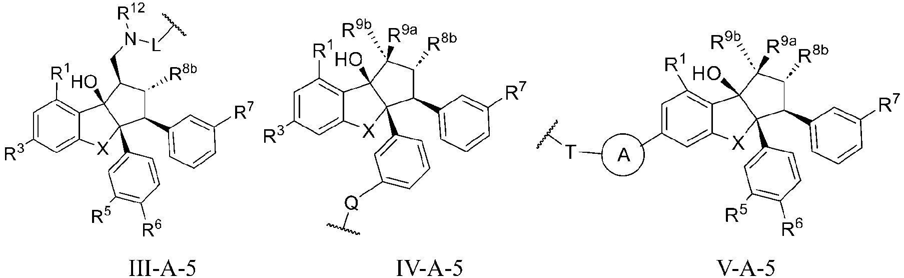
wherein X, R¹, ring A, R³, R⁵, R⁶, R⁷, R^{8b}, R^{9a}, R^{9b}, R¹², n, M, W, U, Z, Y, L, Q, and T are defined as described in any one of claims 1-17;
or structures represented by formulas I-A-6 and I-A-7: 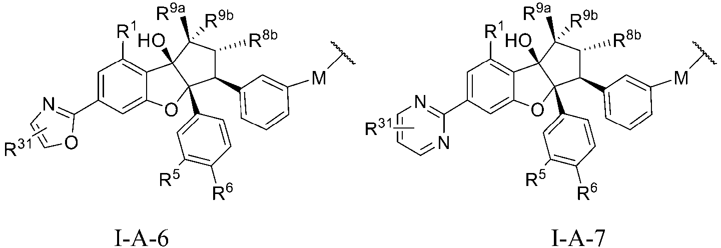
wherein R¹, R³¹, R⁵, R⁶, R^{8b}, R^{9a}, R^{9b}, and M are defined as described in any one of claims 1-17.

19. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-18, wherein the drug group is selected from the structures shown in Table A.

20. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-19, wherein the linking group is -Tr-L³-L²-L¹-, and the linking group and the drug group are linked to form a structure selected from a structure represented by formula I-B, II-B', III-B', IV-B, or V-B: 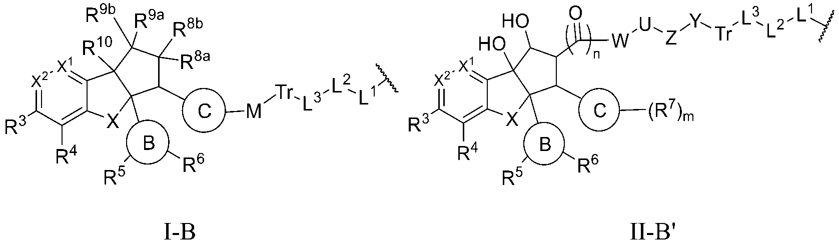 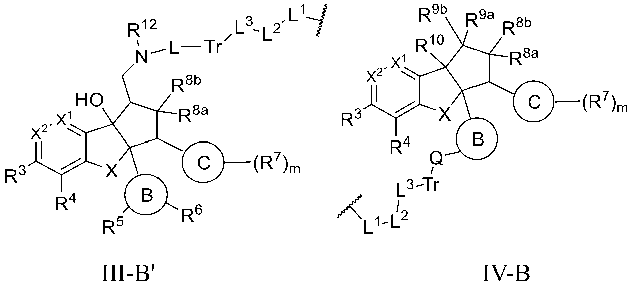 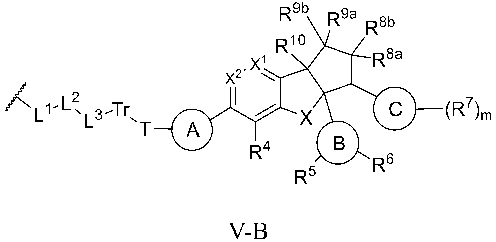 wherein:
X, X¹, X², ring A, ring B, ring C, R³, R⁴, R⁵, R⁶, R⁷, R^{8a}, R^{8b}, R^{9a}, R^{9b}, R¹⁰, R¹², m, n, M, W, U, Z, Y, L, Q, and T are defined as described in any one of claims 1-19;
Tr is absent, or Tr is any group;
L³ is absent or is selected from a polypeptide fragment and a peptide fragment analog;
L² is absent or is selected from a linker fragment;
L¹ is a linking unit;
the wavy line indicates linking to the ligand by a nitrogen atom or a carbon atom on the L¹ group.

21. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-20, wherein:
L¹ is a linking unit;
L² is -(C(R^{L21})₂)ᵣ-, wherein r is a natural number from 0 to 50;
any C(R^{L21})₂ unit in L² may be independently replaced with the following structural unit: -Cy-, - C(=O)-, -NR^{L22}-, -O-, -S-, -S(=O)-, -S(=O)₂-, -P(R^{L22})-, -P(=O)(R^{L22})-, -C(=S)-, -C(=NR^{L22})-,-N=N-, -CH=N-, -N=CH-, 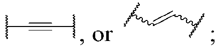 -Cy- is selected from -phenylene-, -(5-8 membered)heteroarylene-, -(4-10 membered)heterocyclylene-, and -(C₃-C₁₀)cycloalkylene-, and -Cy- is unsubstituted, or each -Cy-is independently substituted with one or more R^{Cx};
R^{L21}, R^{L22}, and R^{Cx} are each independently selected from hydrogen, deuterium, halogen, nitro, - CN, -OR^{L2a}, -SR^{L2a}, -N(R^{L2a})₂, -N⁺(R^{L2a})₃, -C(=O)R^{L2a}, -C(=O)OR^{L2a}, -C(=O)-C(=O)R^{L2a}, - C(=O)CH₂C(=O)R^{L2a}, -S(=O)R^{L2a}, -S(=O)₂R^{L2a}, -C(=O)N(R^{L2a})₂, -S(=O)₂N(R^{L2a})₂, -OC(=O)R^{L2a}, -N(R^{L2a})S(=O)₂R^{L2b}, -N(R^{L2a})C(=O)R^{L2b}, -(CH₂)_{y}-C(=O)-(N(Me)CH₂C(=O))ₛ-OR^{L2a}, -(CH₂)_{y}-C(=O)-(N(Me)CH₂C(=O))ₛ-NHR^{L2a}, -(CH₂)_{y}-C(=O)-(N(Me)CH₂C(=O))ₛ-N⁺(R^{L2a})₃, -(CH₂)_{y}-NHC(=O)CH₂(OCH₂CH₂)OR^{L2a}, -(CH₂)_{y}-NH(C(=O)CH₂(N(Me))ₛ-R^{L2a}, -(CH₂)_{y}-C(=O)NH-(CH₂CH₂O)ₛ-R^{L2a}, -(CH₂)_{y}-NHC(=O)-(CH₂CH₂O)ₛ-R^{L2a}, -(CH₂CH₂O)ₛ-R^{L2a}, - (C(=O)CH₂N(Me))ₛ-R^{L2a}, -C(=O)CH₂(OCH₂CH₂)ₛ-OR^{L2a}, -C(=O)-(CH₂CH₂O)ₘ-R^{L2a}, -C(=O)-(CH₂)_{y}-C(=O)NH-(CH₂CH₂O)ₛ-R^{L2a}, -C(=O)-(CH₂)_{y}-NHC(=O)-(CH₂CH₂O)ₛ-R^{L2a}, -(C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl, wherein the -(C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl are optionally substituted with one or more R^{L2a};
s and y are each independently a natural number from 0 to 50;
R^{L2a} and R^{L2b} are each independently selected from hydrogen, deuterium, halogen, nitro, -CN, - OH, -SH, -NH₂, -N(Me)₂, -C(=O)OH, -S(=O)₂Me, -S(=O)₂OH, -C(=O)NH₂, -S(=O)₂NH₂, -(C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl;
L³ is absent or is selected from an amino acid residue, a short peptide consisting of 2-10 amino acid residues, -C(=O)-(C₁-C₆)alkylene-NH-, 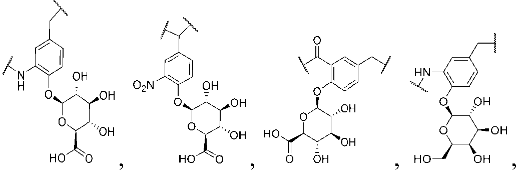 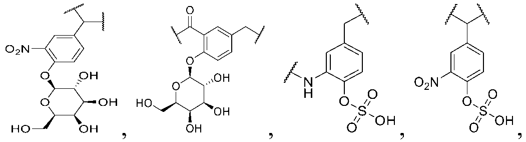 and any combination of the aforementioned groups, wherein the amino acid residues are natural amino acid residues or non-natural amino acid residues, and L³ is optionally modified by one or more protecting groups; preferably, the protecting groups are selected from a polyethylene glycol fragment and -OtBu, wherein the polyethylene glycol fragment is linked to L³ by the structural unit -NH-;
Tr is absent or is selected from 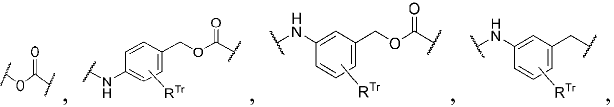 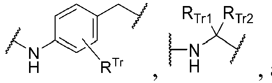 and any combination of the aforementioned groups;
R^{Tr}, R^{Tr1}, and R^{Tr2} are each independently selected from hydrogen, deuterium, halogen, nitro, -CN, -OH, -NH₂, -SH, -OC(=O)NH₂, -C(=O)OH, -C(=O)NH₂, -S(=O)₂OH, -S(=O)₂NH₂, -CH₂C(=O)-(N(Me)CH₂C(=O))_{z}-OR^{Tra}, -CH₂C(=O)-(N(Me)CH₂C(=O))_{z}-NHR^{Tra}, -(CH₂CH₂O)_{z}-R^{Tra}, - C(=O)NH-(CH₂CH₂O)_{z}-R^{Tra}, -(C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl, wherein the - (C₁-C₆)alkyl, -(C₂-C₆)alkenyl, -(C₂-C₆)alkynyl, -(C₃-C₈)cycloalkyl, -(4-10 membered)heterocycloalkyl, -(C₆-C₁₀)aryl, and -(5-10 membered)heteroaryl are optionally substituted with one or more R^{Tra};
each R^{Tra} is independently hydrogen, deuterium, halogen, nitro, -CN, -OH, -NH₂, -SH, -N(Me)₂, - C(=O)OH, -C(=O)NH₂, -S(=O)₂OH, -S(=O)₂NH₂, -S(=O)₂Me, -(C₁-C₆)alkyl, -(C₂-C₆)alkenyl, - (C₂-C₆)alkynyl, -(C₃-C₈)cycloalkyl, -(4-10 membered)heterocycloalkyl, -(C₆-C₁₀)aryl, and -(5-10 membered)heteroaryl;
each z is independently a natural number from 0 to 50.

22. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-21, wherein:
L¹ is selected from carbonyl, -(C₁-C₈)alkylene-C(=O)-, -(4-8 membered)heterocyclylene-, -(5-10 membered)heteroarylene-, and -(C₁-C₈)alkylene-(5-10 membered)heteroarylene-, wherein the - (C₁-C₈)alkylene-C(=O)-, -(4-8 membered)heterocyclylene-, -(5-10 membered)heteroarylene-, and -(C₁-C₈)alkylene-(5-10 membered)heteroarylene- are optionally substituted with 1, 2, or 3 oxo, - (C₁-C₄)alkylene-, and -(C₁-C₄)alkyl groups;
preferably, L¹ is selected from carbonyl, -(C₁-C₄)alkylene-C(=O)-, -(5-6 membered)heterocyclylene-, -(5-6 membered)heteroarylene-, and -(C₁-C₄)alkylene-(5-6 membered)heteroarylene-, wherein the -(C₁-C₄)alkylene-C(=O)-, -(5-6 membered)heterocyclylene-, -(5-6 membered)heteroarylene-, and -(C₁-C₄)alkylene-(5-6 membered)heteroarylene- are optionally substituted with 1, 2, or 3 oxo, -(C₁-C₄)alkylene-, and-(C₁-C₄)alkyl groups;
preferably, L¹ is selected from 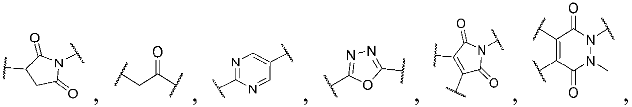 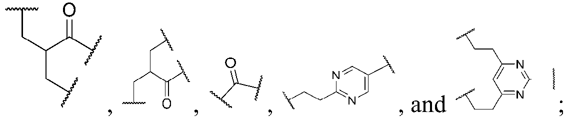
preferably, L¹ is selected from 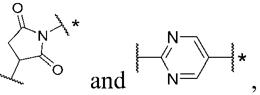 wherein the * end is attached to L².

23. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-22, wherein:
L² is -(C(R^{L21})₂)ᵣ-, wherein r is a natural number from 0 to 10 (e.g., 1, 2, 3, 4, 5, 6, 7, 8, or 9);
any C(R^{L21})₂ unit in L² may be independently replaced with the following structural unit: -Cy-, - C(=O)-, -NR^{L22}-, -O-, -S-, or -S(=O)-;
-Cy- is selected from -phenylene-, -4-6 membered heterocyclylene-, and -(C₃-C₆)cycloalkylene-, and -Cy- is unsubstituted, or each -Cy- is independently substituted with 1, 2, 3, 4, 6, or 7 R^{Cx};
R^{L21}, R^{L22}, and R^{Cx} are each independently selected from hydrogen, deuterium, halogen, nitro, - CN, -OR^{L2a}, -SR^{L2a}, -N(R^{L2a})₂, -C(=O)R^{L2a}, -C(=O)OR ^{L2a}, -C(=O)-C(=O)R^{L2a}, - C(=O)CH₂C(=O)R^{L2a}, -S(=O)R^{L2a}, =S(=O)₂R^{L2a}, -C(=O)N(R^{L2a})₂, -S(=O)₂N(R^{L2a})₂, -(CH₂)₂-C(=O)NH-(CH₂CH₂O)ₜ-R^{L2a}, -(C₁-C₆)alkyl, and -(C₂-C₆)alkenyl, wherein the -(C₁-C₆)alkyl and - (C₂-C₆)alkenyl are optionally substituted with 1, 2, 3, 4, 5, 6, or 7 R^{L2a};
t is a natural number from 0 to 30;
R^{L2a} is selected from hydrogen, deuterium, fluorine, chlorine, bromine, iodine, nitro, -CN, -OH, - SH, -NH₂, -N(Me)₂, -C(=O)OH, -S(=O)₂Me, -S(=O)₂OH, -C(=O)₂OH, -C(=O)NH₂, -S(=O)₂NH₂, methyl, ethyl, and vinyl;
preferably, L² is -(C(R^{L21})₂)ᵣ-, wherein r is 2, 3, 4, 5, 6, 7, 8, or 9;
any C(R^{L21})₂ unit in L² may be independently replaced with the following structural unit: -Cy-, - C(=O)-, -NR^{L22}-, -O-, -S-, or -S(=O)-;
-Cy- is selected from cyclopropylene, cyclobutylene, cyclopentylene, azetidinylene, pyrrolidinylene, piperazinylene, or piperidinylene, and -Cy- is unsubstituted, or each -Cy- is independently substituted with 1, 2, 3, 4, or 5 R^{Cx}; for example, -Cy- is 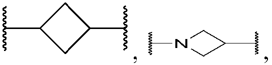 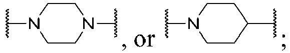 R^{L21}, R^{L22}, and R^{Cx} are each independently selected from hydrogen, deuterium, fluorine, chlorine, bromine, iodine, nitro, -CN, -OH, -SH, -NH₂, -C(=O)OH, -C(=O)NH, -(CH₂)₂-C(=O)NH-(CH₂CH₂O)₁₁-CH₃, methyl, ethyl, propyl, deuterated methyl, deuterated ethyl, halomethyl, haloethyl, methyl-OH, ethyl-OH, propyl-OH, vinyl-OH, vinyl, deuterated vinyl, and halovinyl;
preferably, L² is selected from 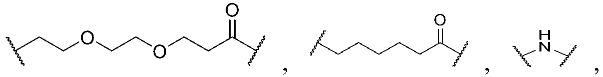 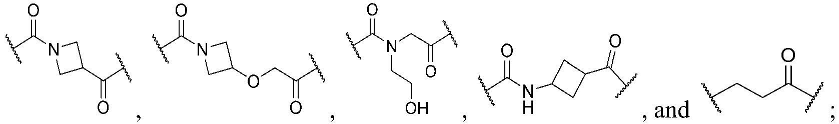
preferably, L² is selected from 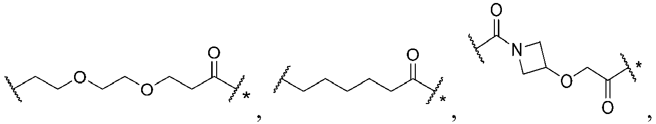 and 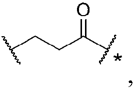 wherein the * end is attached to L₃.

24. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-23, wherein:
-L¹-L²- is selected from 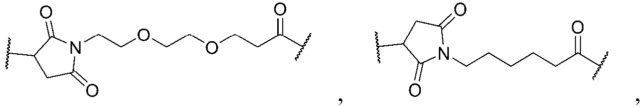 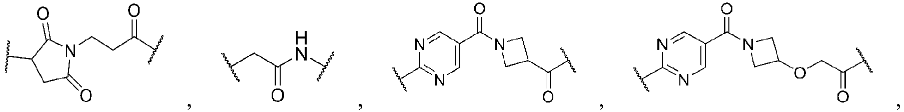 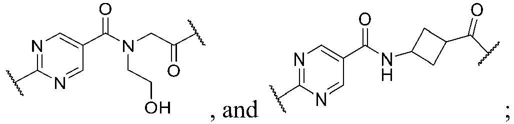
preferably, -L¹-L²- is selected from 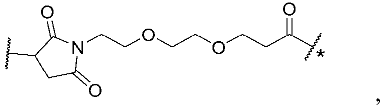 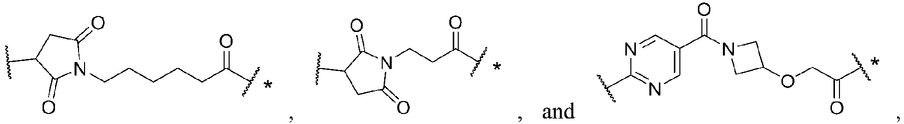 wherein the * end is attached to L₃.

25. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-24, wherein:
L³ is selected from Val, D-Val, Phe, Lys, Leu, Ile, Gly, Ala, D-Ala, Cit, Asp, Asn, Glu, Gln, Arg, Ser, -Arg-Val-, -Arg-Leu-, -Val-Cit-, -Val-Ala-, -Val-Lys-, -Val-Lys(AC)-, -Phe-Lys-, -Phe-Lys(AC)-, -Leu-Lys-, -Leu-Lys(AC)-, -Ala-Ala-, -Ala-Lys-, -(D-Ala)-Ala-, -Gly-Glu-, -Gly-Asp-, -Gly-Asn-, -Val-Glu-, -Val-Asp-, -Asn-Asn-, -Asp-Asp-, -Asp-Glu-, -Ser-Val-, -Ser-Ala-, -Ser-Gly-, -Ser-Glu-, -Ser-Gln-, -Ser-Asp-, -Gly-Gln-, -Asn-Gly-, -Asn-Ala-, -Ala-Ala-Glu-, -Gly-Gly-Glu-, -Gly-Gly-Asp-, -Gly-Gly-Asn-, -Gly-Ala-Ala-, -Gly-Val-Ala-, -Gly-Val-Cit-, -Glu-Val-Cit-, -Ala-Ala-Ala-, -Ala-(D-Ala)-Ala-, -Ala-Ala-Asn-, -Ala-(D-Ala)-Asn-, -Ala-Ala-Asp-, -Val-Lys-Gly-, -(D-Val)-Leu-Lys-, -Gly-Gly-Arg-, -Gly-Gly-Gly-, -Lys-Ala-Asn-, -Gly-Phe-Gly-, -Gly-Gly-Phe-, -Asn-Pro-Val-, -Ala-Lys-Gly-, -Gly-Lys-Gly-, -Gly-Gly-Gly-Gly-, -Gly-Gly-Phe-Gly-, -Gly-Gly-Glu-Gly-, -Lys-Ala-Ala-Asn-, -Lys-Ala-Ala-Asp-, -Ala-Ala-Pro-Val-, -Ala-Ala-Pro-Nva-, 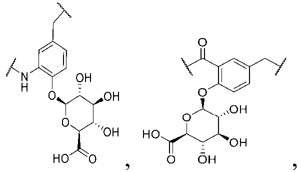 -C(=O)-(C₁-C₄)alkylene-NH-, and any combination of the aforementioned groups, wherein Asp and Glu are each independently and optionally modified by -(CH₂CH₂O)ₜ-R^{L3}, wherein -(CH₂CH₂O)ₜ-R^{L3} is linked to Asp or Glu by the structural unit -NH-, R^{L3} is -(C₁-C₄)alkyl, and t is an integer from 6 to 30 (e.g., 8, 9, 10, 11, 12, 13, 14, 15, 20, 21, 22, 23, 24, or 25);
preferably, L³ is selected from -Val-Cit-, -Arg-Val-, -Ala-Ala-, -Val-Ala-, -Gly-Glu-, -Ser-Val-, - Ser-Ala-, -Ser-Gly-, -Ser-Glu-, -Ser-Gln-, -Ser-Asp-, -Gly-Gln-, -Asn-Gly-, -Asn-Ala-, -Ala-Ala-Glu-, -Gly-Ala-Ala-, -Gly-Gly-Glu-, -Gly-Gly-Phe-Gly-, -Ala-Ala-Glu(NH-(CH₂CH₂O)ₜ-R^{L3})-, and -C(=O)-CH₂CH₂-NH-, wherein R^{L3} is -(C₁-C₄)alkyl, and t is an integer from 6 to 30; an example is -Ala-Ala-Glu(NH-(CH₂CH₂O)₁₁-CH₃)- or -Ala-Ala-Glu(NH-(CH₂CH₂O)₂₄-CH₃)-;
preferably, L³ is selected from -Val-Cit-*, *-Ala-Ala-, -Val-Ala-*, *-Gly-Glu-, *-Ser-Val-, *-Ser-Ala-, *-Ser-Gly-, *-Ser-Glu-, *-Ser-Gln-, *-Ser-Asp-, -Gly-Gln-*, *-Gly-Gln-, *-Asn-Gly-, *-Asn-Ala-, *-Gly-Ala-Ala-, *-Gly-Gly-Glu-, -Gly-Gly-Phe-Gly-*, *Ala-Ala-C(=O)-CH[CH₂CH₂-C(=O)-NH-(CH₂CH₂O)₁₁-CH₃]-NH-, and 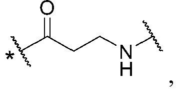 wherein the * end is attached to Tr.

26. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-25, wherein:
Tr is absent or is selected from 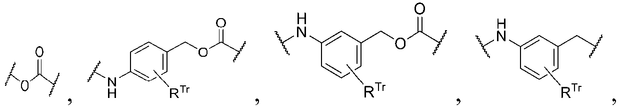 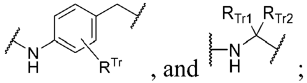
R^{Tr}, R^{Tr1}, and R^{Tr2} are each independently selected from hydrogen, deuterium, halogen, nitro, -CN, -OH, -NH₂, -SH, -OC(=O)NH₂, -C(=O)OH, -C(=O)NH₂, -S(=O)₂OH, -S(=O)₂NH₂, -(CH₂CH₂O)_{z}-R^{Tra}, -C(=O)NH-(CH₂CH₂O)_{z}-R^{Tra}, gluconolactone, glucuronic acid, -(C₁-C₄)alkyl, and -(C₂-C₄)alkenyl, wherein the -(C₁-C₄)alkyl and -(C₂-C₄)alkenyl are optionally substituted with 1, 2, 3, 4, or 5 R^{Tra};
each R^{Tra} is independently hydrogen, deuterium, halogen, nitro, -CN, -OH, -NH₂, -SH, -N(Me)₂, - C(=O)OH, -C(=O)NH₂, -S(=O)₂OH, -S(=O)₂NH₂, -S(=O)₂Me, -(C₁-C₄)alkyl, or -(C₂-C₄)alkenyl; each z is independently an integer from 6 to 30;
preferably, R^{Tr}, R^{Tr1}, and R^{Tr2} are each independently selected from hydrogen, deuterium, fluorine, chlorine, bromine, iodine, nitro, -CN, -OH, -NH₂, -SH, -OC(=O)NH₂, -C(=O)OH, -C(=O)NH₂, - S(=O)₂OH, -S(=O)₂NH₂, -(CH₂CH₂O)_{z}-R^{Tra}, -C(=O)NH-(CH₂CH₂O)_{z}-R^{Tra}, gluconolactone, glucuronic acid, methyl, ethyl, -ethylene-OH, and -CH₂-COOH;
each R^{Tra} is independently hydrogen, deuterium, fluorine, chlorine, bromine, iodine, -CN, -OH, - NH₂, -SH, -N(Me)₂, -C(=O)OH, -C(=O)NH₂, methyl, or ethyl;
each z is independently an integer from 6 to 30 (e.g., 8, 9, 10, 11, 12, 13, 14, 15, 20, 21, 22, 23, 24, or 25);
preferably, R^{Tr} is selected from hydrogen, -CH₂-COOH, and 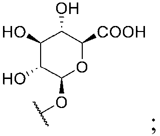
preferably, Tr is absent or is selected from 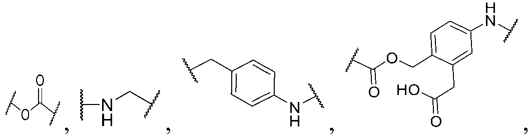 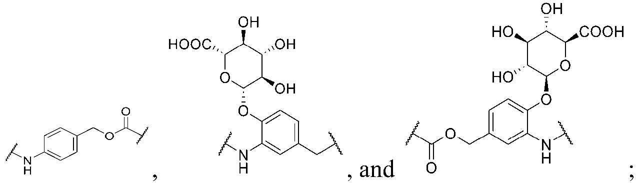 preferably, Tr is absent or is selected from 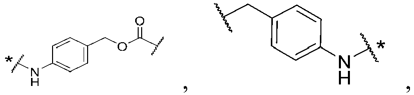 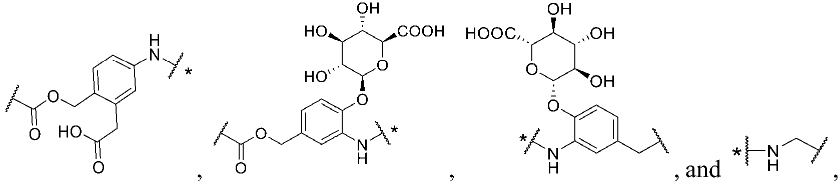 wherein the * end is attached to L³.

27. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-26, wherein: -L³-L²-L¹- is selected from 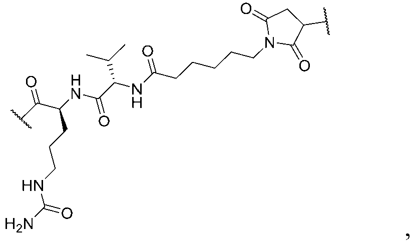 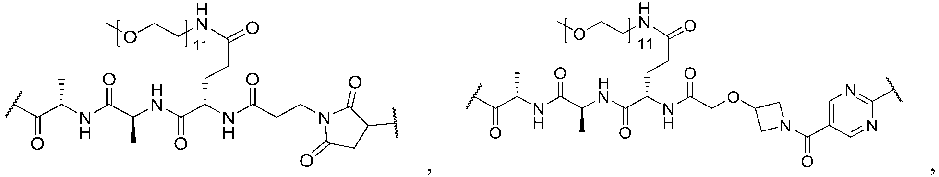 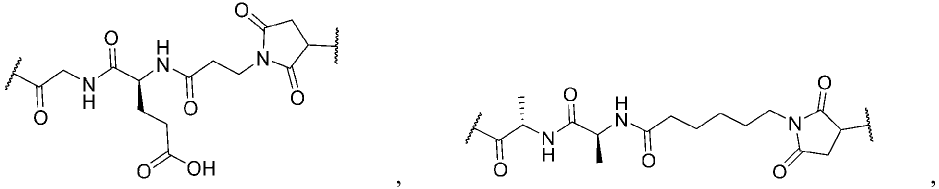 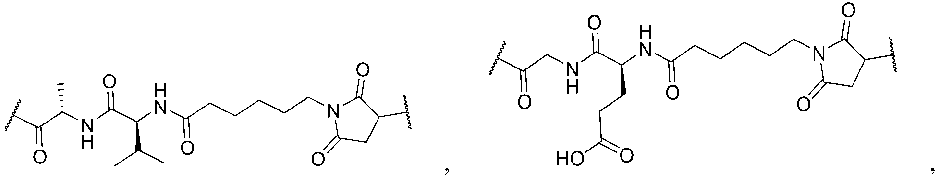 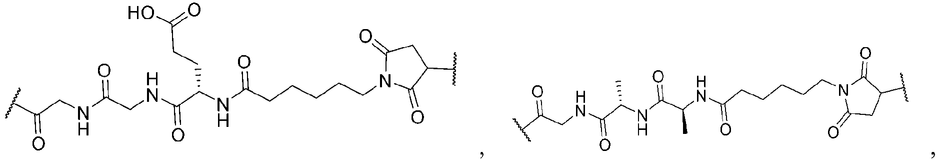 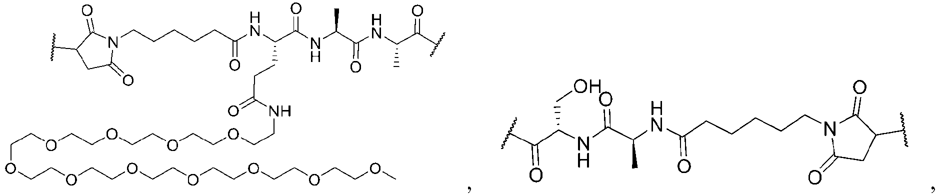 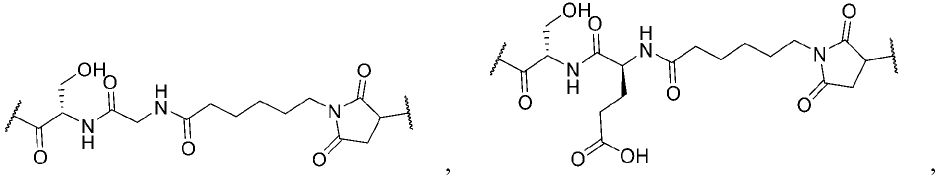 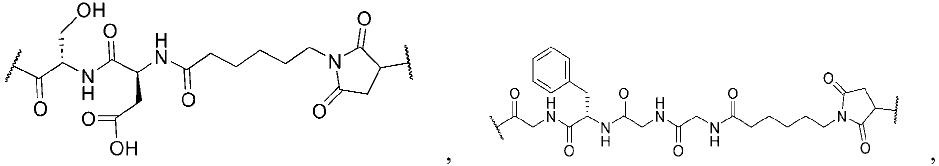 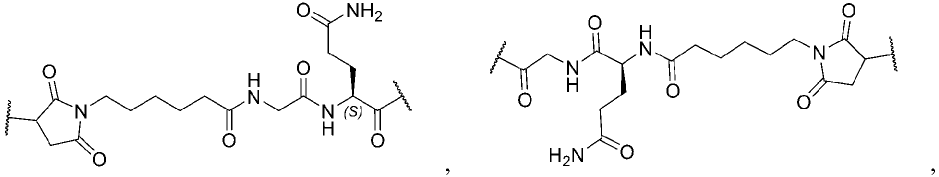 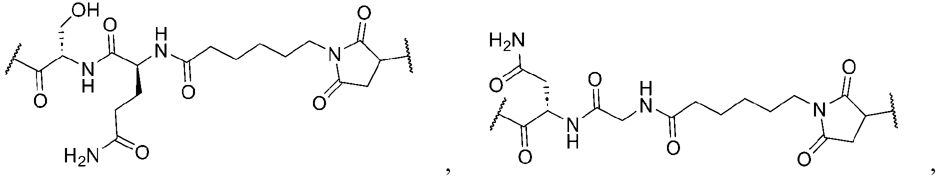 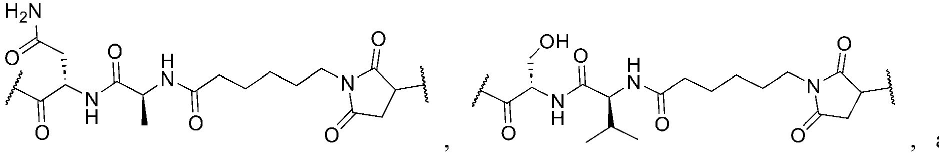 and

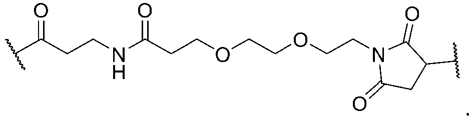

28. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-27, wherein the linking group and the drug group are linked to form a structure selected from:
a structure represented by formula I-B-1, II-B-1', III-B-1', IV-B-1, or V-B-1: 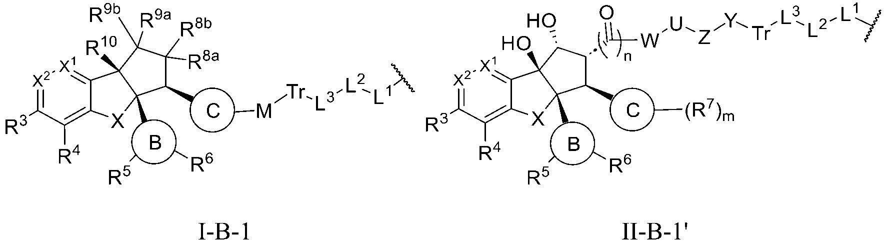 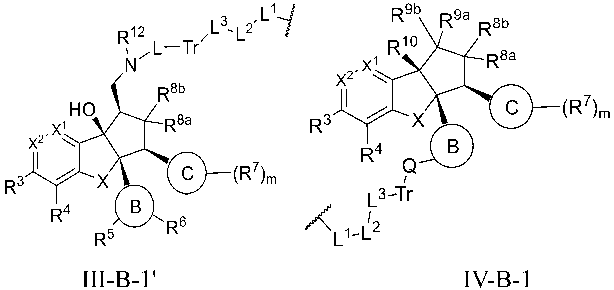 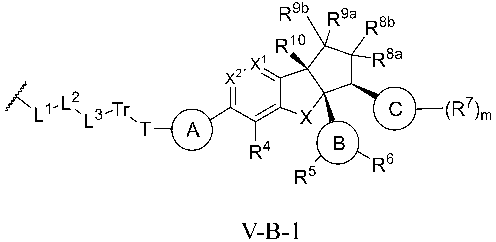
wherein X, X¹, X², ring A, ring B, ring C, R³, R⁴, R⁵, R⁶, R⁷, R^{8a}, R^{8b}, R^{9a}, R^{9b}, R¹⁰, R¹², m, n, M, W, U, Z, Y, L, Q, T, Tr, L¹, L², and L³ are defined as described in any one of claims 1-27;
or a structure represented by formula I-B-5, II-B-5, III-B-5, IV-B-5, or V-B-5: 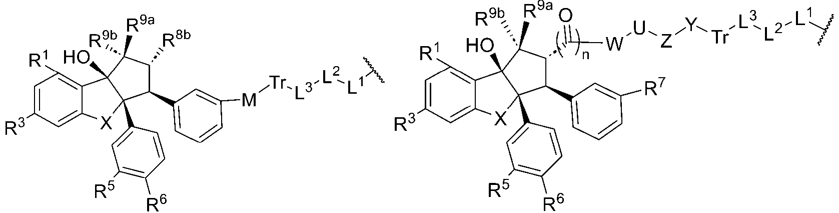 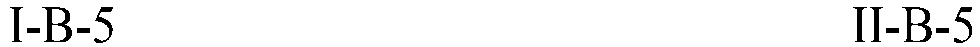 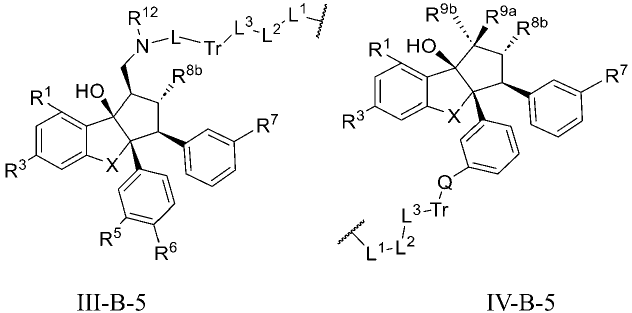 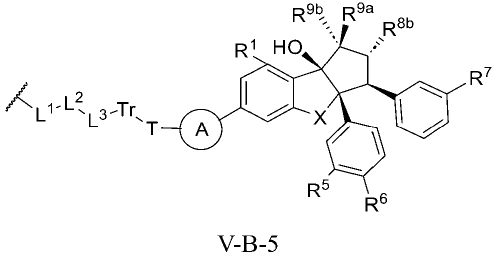
wherein X, R¹, ring A, R³, R⁵, R⁶, R⁷, R^{8b}, R^{9a}, R^{9b}, R¹², n, M, W, U, Z, Y, L, Q, T, Tr, L¹, L², and L³ are defined as described in any one of claims 1-27;
or structures represented by formulas I-B-6 and I-B-7: 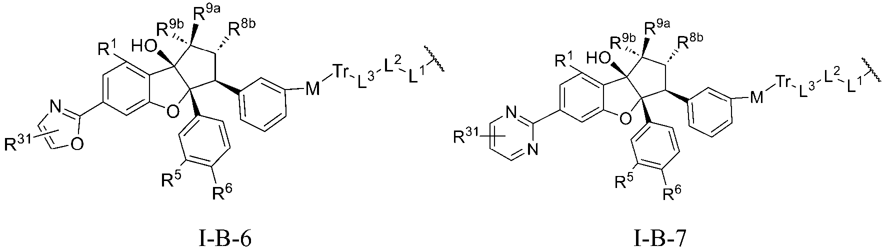
wherein R¹, R³¹, R⁵, R⁶, R^{8b}, R^{9a}, R^{9b}, M, Tr , L¹, L², and L³ are defined as described in any one of claims 1-27.

29. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-28, wherein the linking group and the drug group are linked to form a structure selected from the structures shown in Table B.

30. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-29, wherein the ligand-drug conjugate is selected from a compound represented by formula I-C, II-C', III-C', IV-C, or V-C: 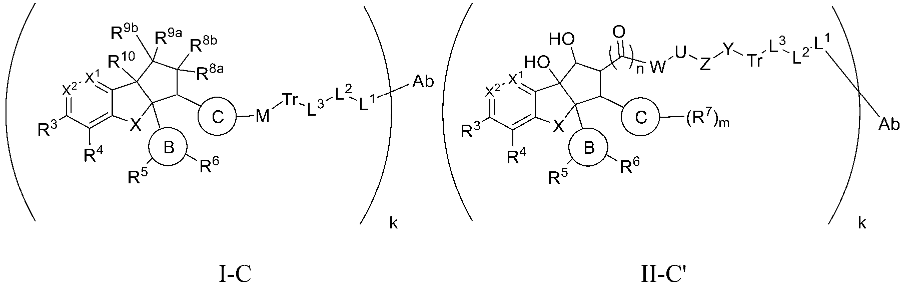 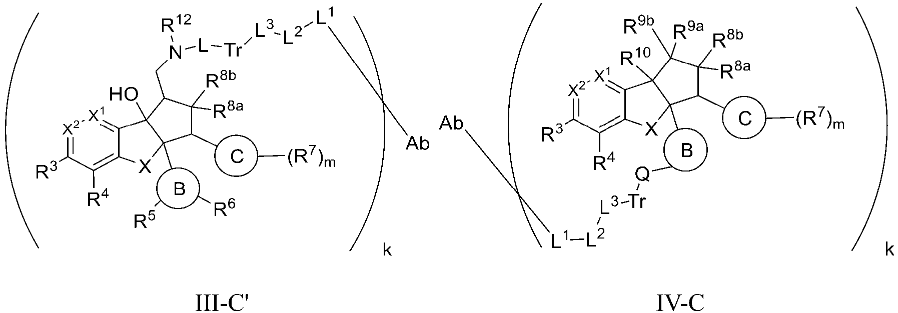 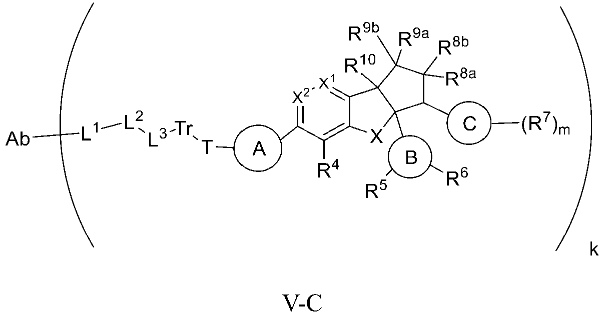 wherein:
X, X¹, X², ring A, ring B, ring C, R³, R⁴, R⁵, R⁶, R⁷, R^{8a}, R^{8b}, R^{9a}, R^{9b}, R¹⁰, R¹², m, n, M, W, U, Z, Y, L, Q, T, Tr, L¹, L², and L³ are defined as described in any one of claims 1-29;
Ab is a ligand that binds to a target;
k is a drug loading and is an integer or a decimal from 1 to 16.

31. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-30, wherein Ab is conjugated to L¹ by an S atom; preferably, Ab is a polypeptide, or an antibody or an antigen-binding fragment thereof, particularly an antibody or an antigen-binding fragment thereof, capable of specifically binding to a target;
preferably, the antibody is selected from one or more of the following:
(1) a fully human antibody, a humanized antibody, a murine antibody, and a chimeric antibody,
(2) a probody,
(3) a bispecific antibody and a multispecific antibody,
(4) a monoclonal antibody and a polyclonal antibody, and
(5) an IgG antibody;
preferably, the antigen-binding fragment is selected from an antigen-binding fragment of a Fab, a Fab', a F(ab')₂, an Fv, an scFv, an Fd, a dAb, a VHH, and a complementarity-determining region (CDR) fragment;
preferably, Ab is a monoclonal antibody, particularly a monoclonal antibody that takes sulfhydryl as a conjugation site, or a monoclonal antibody that has undergone site-directed mutagenesis or modification and takes sulfhydryl as a conjugation site;
preferably, Ab targets an antigen selected from the following group: HER2, HER3, B7H3, B7H4, DLL3, TROP2, Claudin18.2, CD30, CD33, CD70, EGFR, GPC-3, ADAM9, BCMA, CEACAM5, and c-met;
preferably, Ab is selected from antibodies and antigen-binding fragments thereof against HER2, HER3, B7H3, TROP2, Claudin18.2, CD30, CD33, CD70, GPC-3, ADAM9, BCMA, CEACAM5, and c-met;
preferably, Ab is selected from tusamitamab, DB1001, DB1002, DB1003, DB1004, DB1005, cetuximab, and panitumumab, and biosimilars of the antibodies and antigen-binding fragments thereof;
preferably, Ab is selected from tusamitamab, DB1001, DB1002, DB1003, DB1004, and DB1005, andantigen-binding fragments of the antibodies;
preferably, Ab comprises the sequences of a CDR1, a CDR2, and a CDR3 contained in a heavy chain set forth in SEQ ID NO: 1, and/or the sequences of a CDR1, a CDR2, and a CDR3 contained in a light chain set forth in SEQ ID NO: 2; or Ab comprises the sequences of a CDR1, a CDR2, and a CDR3 contained in a heavy chain set forth in SEQ ID NO: 3, and/or the sequences of a CDR1, a CDR2, and a CDR3 contained in a light chain set forth in SEQ ID NO: 4; or Ab comprises the sequences of a CDR1, a CDR2, and a CDR3 contained in a heavy chain set forth in SEQ ID NO: 5, and/or the sequences of a CDR1, a CDR2, and a CDR3 contained in a light chain set forth in SEQ ID NO: 6; or Ab comprises the sequences of a CDR1, a CDR2, and a CDR3 contained in a heavy chain set forth in SEQ ID NO: 25, and/or the sequences of a CDR1, a CDR2, and a CDR3 contained in a light chain set forth in SEQ ID NO: 26; or Ab comprises the sequences of a CDR1, a CDR2, and a CDR3 contained in a heavy chain set forth in SEQ ID NO: 35, and/or the sequences of a CDR1, a CDR2, and a CDR3 contained in a light chain set forth in SEQ ID NO: 36; or Ab comprises the sequences of a CDR1, a CDR2, and a CDR3 contained in a heavy chain set forth in SEQ ID NO: 15, and/or the sequences of a CDR1, a CDR2, and a CDR3 contained in a light chain set forth in SEQ ID NO: 16;
preferably, Ab comprises a heavy chain variable region CDR1 set forth in SEQ ID NO: 7, a heavy chain variable region CDR2 set forth in SEQ ID NO: 8, and a heavy chain variable region CDR3 set forth in SEQ ID NO: 9, and/or a light chain variable region CDR1 set forth in SEQ ID NO: 10, a light chain variable region CDR2 set forth in SEQ ID NO: 11, and a light chain variable region CDR3 set forth in SEQ ID NO: 12; or, a heavy chain variable region CDR1 set forth in SEQ ID NO: 17, a heavy chain variable region CDR2 set forth in SEQ ID NO: 18, and a heavy chain variable region CDR3 set forth in SEQ ID NO: 19, and/or a light chain variable region CDR1 set forth in SEQ ID NO: 20, a light chain variable region CDR2 set forth in SEQ ID NO: 21, and a light chain variable region CDR3 set forth in SEQ ID NO: 22; or, a heavy chain variable region CDR1 set forth in SEQ ID NO: 27, a heavy chain variable region CDR2 set forth in SEQ ID NO: 28, and a heavy chain variable region CDR3 set forth in SEQ ID NO: 29, and/or a light chain variable region CDR1 set forth in SEQ ID NO: 30, a light chain variable region CDR2 set forth in SEQ ID NO: 31, and a light chain variable region CDR3 set forth in SEQ ID NO: 32; or, a heavy chain variable region CDR1 set forth in SEQ ID NO: 37, a heavy chain variable region CDR2 set forth in SEQ ID NO: 38, and a heavy chain variable region CDR3 set forth in SEQ ID NO: 39, and/or a light chain variable region CDR1 set forth in SEQ ID NO: 40, a light chain variable region CDR2 set forth in SEQ ID NO: 41, and a light chain variable region CDR3 set forth in SEQ ID NO: 42; or, a heavy chain variable region CDR1 set forth in SEQ ID NO: 45, a heavy chain variable region CDR2 set forth in SEQ ID NO: 46, and a heavy chain variable region CDR3 set forth in SEQ ID NO: 47, and/or a light chain variable region CDR1 set forth in SEQ ID NO: 48, a light chain variable region CDR2 set forth in SEQ ID NO: 49, and a light chain variable region CDR3 set forth in SEQ ID NO: 50; or, a heavy chain variable region CDR1 set forth in SEQ ID NO: 53, a heavy chain variable region CDR2 set forth in SEQ ID NO: 54, and a heavy chain variable region CDR3 set forth in SEQ ID NO: 55, and/or a light chain variable region CDR1 set forth in SEQ ID NO: 56, a light chain variable region CDR2 set forth in SEQ ID NO: 57, and a light chain variable region CDR3 set forth in SEQ ID NO: 58;
preferably, Ab comprises the sequence of a heavy chain variable region contained in a heavy chain set forth in SEQ ID NO: 1, and/or the sequence of a light chain variable region contained in a light chain set forth in SEQ ID NO: 2; or Ab comprises the sequence of a heavy chain variable region contained in a heavy chain set forth in SEQ ID NO: 3, and/or the sequence of a light chain variable region contained in a light chain set forth in SEQ ID NO: 4; or Ab comprises the sequence of a heavy chain variable region contained in a heavy chain set forth in SEQ ID NO: 5, and/or the sequence of a light chain variable region contained in a light chain set forth in SEQ ID NO: 6; or Ab comprises the sequence of a heavy chain variable region contained in a heavy chain set forth in SEQ ID NO: 25, and/or the sequence of a light chain variable region contained in a light chain set forth in SEQ ID NO: 26; or Ab comprises the sequence of a heavy chain variable region contained in a heavy chain set forth in SEQ ID NO: 35, and/or the sequence of a light chain variable region contained in a light chain set forth in SEQ ID NO: 36; or Ab comprises the sequence of a heavy chain variable region contained in a heavy chain set forth in SEQ ID NO: 15, and/or the sequence of a light chain variable region contained in a light chain set forth in SEQ ID NO: 16; preferably, Ab comprises the sequence of a heavy chain variable region set forth in SEQ ID NO: 14 and/or the sequence of a light chain variable region set forth in SEQ ID NO: 13; or Ab comprises the sequence of a heavy chain variable region set forth in SEQ ID NO: 24 and/or the sequence of a light chain variable region set forth in SEQ ID NO: 23; or Ab comprises the sequence of a heavy chain variable region set forth in SEQ ID NO: 34 and/or the sequence of a light chain variable region set forth in SEQ ID NO: 33; or Ab comprises the sequence of a heavy chain variable region set forth in SEQ ID NO: 44 and/or the sequence of a light chain variable region set forth in SEQ ID NO: 43; or Ab comprises the sequence of a heavy chain variable region set forth in SEQ ID NO: 52 and/or the sequence of a light chain variable region set forth in SEQ ID NO: 51; or Ab comprises the sequence of a heavy chain variable region set forth in SEQ ID NO: 60 and/or the sequence of a light chain variable region set forth in SEQ ID NO: 59;
preferably, the amino acid sequence of a heavy chain of Ab is set forth in SEQ ID NO: 1, and the amino acid sequence of a light chain of Ab is set forth in SEQ ID NO: 2; or the amino acid sequence of a heavy chain of Ab is set forth in SEQ ID NO: 3, and the amino acid sequence of a light chain of Ab is set forth in SEQ ID NO: 4; or the amino acid sequence of a heavy chain of Ab is set forth in SEQ ID NO: 5, and the amino acid sequence of a light chain of Ab is set forth in SEQ ID NO: 6; or the amino acid sequence of a heavy chain of Ab is set forth in SEQ ID NO: 25, and the amino acid sequence of a light chain of Ab is set forth in SEQ ID NO: 26; or the amino acid sequence of a heavy chain of Ab is set forth in SEQ ID NO: 35, and the amino acid sequence of a light chain of Ab is set forth in SEQ ID NO: 36; or the amino acid sequence of a heavy chain of Ab is set forth in SEQ ID NO: 15, and the amino acid sequence of a light chain of Ab is set forth in SEQ ID NO: 16;
preferably, Ab is an anti-CEACAM5 antibody or an antigen-binding fragment thereof, e.g., tusamitamab or an antigen-binding fragment thereof or a variant thereof;
preferably, Ab comprises a heavy chain and a light chain of the anti-CEACAM5 antibody or the antigen-binding fragment thereof, wherein the amino acid sequence of the heavy chain is a sequence having at least 80%, 85%, 90%, 95%, 96%, 97%, 98%, or 99% identity to SEQ ID NO: 1 or is set forth in SEQ ID NO: 1, and the amino acid sequence of the light chain is a sequence having at least 80%, 85%, 90%, 95%, 96%, 97%, 98%, or 99% identity to SEQ ID NO: 2 or is set forth in SEQ ID NO: 2; or
preferably, Ab is an anti-HER3 antibody or an antigen-binding fragment thereof, e.g., DB1001 or an antigen-binding fragment thereof or a variant thereof;
preferably, Ab comprises a heavy chain and a light chain of the anti-HER3 antibody or the antigen-binding fragment thereof, wherein the amino acid sequence of the heavy chain is a sequence having at least 80%, 85%, 90%, 95%, 96%, 97%, 98%, or 99% identity to SEQ ID NO: 3 or is set forth in SEQ ID NO: 3, and the amino acid sequence of the light chain is a sequence having at least 80%, 85%, 90%, 95%, 96%, 97%, 98%, or 99% identity to SEQ ID NO: 4 or is set forth in SEQ ID NO: 4; or
preferably, Ab is an anti-GPC-3 antibody or an antigen-binding fragment thereof, e.g., DB1002 or an antigen-binding fragment thereof or a variant thereof;
preferably, Ab comprises a heavy chain and a light chain of the anti-GPC-3 antibody or the antigen-binding fragment thereof, wherein the amino acid sequence of the heavy chain is a sequence having at least 80%, 85%, 90%, 95%, 96%, 97%, 98%, or 99% identity to SEQ ID NO: 5 or is set forth in SEQ ID NO: 5, and the amino acid sequence of the light chain is a sequence having at least 80%, 85%, 90%, 95%, 96%, 97%, 98%, or 99% identity to SEQ ID NO: 6 or is set forth in SEQ ID NO: 6; or
preferably, Ab is an anti-ADAM9 antibody or an antigen-binding fragment thereof, e.g., DB1003 or an antigen-binding fragment thereof or a variant thereof;
preferably, Ab comprises a heavy chain and a light chain of the anti-ADAM9 antibody or the antigen-binding fragment thereof, wherein the amino acid sequence of the heavy chain is a sequence having at least 80%, 85%, 90%, 95%, 96%, 97%, 98%, or 99% identity to SEQ ID NO: 25 or is set forth in SEQ ID NO: 25, and the amino acid sequence of the light chain is a sequence having at least 80%, 85%, 90%, 95%, 96%, 97%, 98%, or 99% identity to SEQ ID NO: 26 or is set forth in SEQ ID NO: 26; or
preferably, Ab is an anti-BCMA antibody or an antigen-binding fragment thereof, e.g., DB1004 or an antigen-binding fragment thereof or a variant thereof;
preferably, Ab comprises a heavy chain and a light chain of the anti-BCMA antibody or the antigen-binding fragment thereof, wherein the amino acid sequence of the heavy chain is a sequence having at least 80%, 85%, 90%, 95%, 96%, 97%, 98%, or 99% identity to SEQ ID NO: 35 or is set forth in SEQ ID NO: 35, and the amino acid sequence of the light chain is a sequence having at least 80%, 85%, 90%, 95%, 96%, 97%, 98%, or 99% identity to SEQ ID NO: 36 or is set forth in SEQ ID NO: 36; or
preferably, Ab is an anti-c-met antibody or an antigen-binding fragment thereof, e.g., DB1005 or an antigen-binding fragment thereof or a variant thereof;
preferably, Ab comprises a heavy chain and a light chain of the anti-c-met antibody or the antigen-binding fragment thereof, wherein the amino acid sequence of the heavy chain is a sequence having at least 80%, 85%, 90%, 95%, 96%, 97%, 98%, or 99% identity to SEQ ID NO: 15 or is set forth in SEQ ID NO: 15, and the amino acid sequence of the light chain is a sequence having at least 80%, 85%, 90%, 95%, 96%, 97%, 98%, or 99% identity to SEQ ID NO: 16 or is set forth in SEQ ID NO: 16.

32. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-31, wherein k is selected from integers or decimals from 1 to 10, preferably integers or decimals from 2 to 8, e.g., integers or decimals from 4 to 8 (e.g., about 4, about 4.5, about 5, about 5.5, about 6, about 6.5, about 7, about 7.5, or about 8), e.g., integers or decimals from 6 to 8 (e.g., about 6.1, about 6.3, about 6.5, about 6.7, about 6.9, about 7.1, about 7.3, about 7.5, about 7.7, or about 8), e.g., integers or decimals from 7 to 8 (e.g., about 7.1, about 7.2, about 7.3, about 7.4, about 7.5, about 7.6, about 7.7, about 7.8, about 7.9, or about 8).

33. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-32, wherein the ligand-drug conjugate is selected from:
a compound represented by formula I-C-1, II-C-1', **III-C-1',** IV-C-1, or V-C-1: 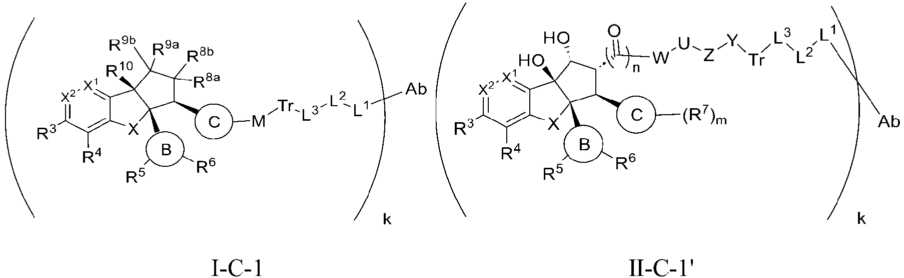 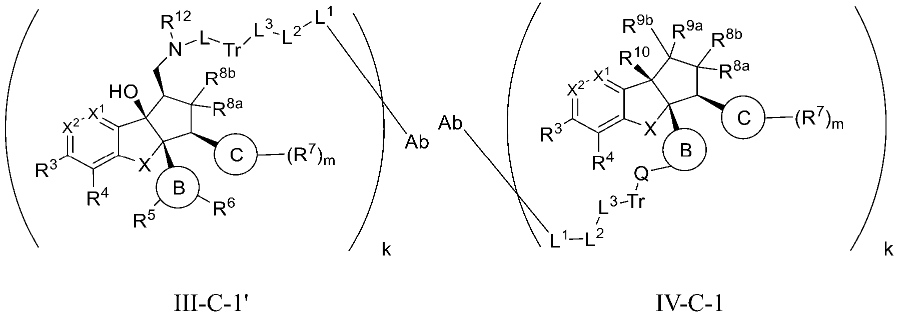 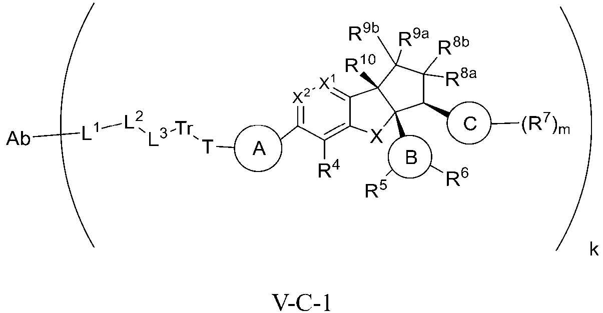
wherein X, X¹, X², ring A, ring B, ring C, R³, R⁴, R⁵, R⁶, R⁷, R^{8a}, R^{8b}, R^{9a}, R^{9b}, R¹⁰, R¹², m, n, M, W, U, Z, Y, L, Q, T, Tr, L¹, L², L³, Ab, and k are defined as described in any one of claims 1-32;
or a compound represented by formula I-C-5, II-C-5, III-C-5, IV-C-5, or V-C-5: 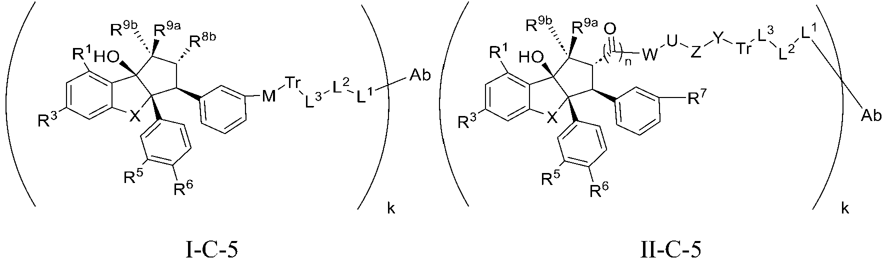 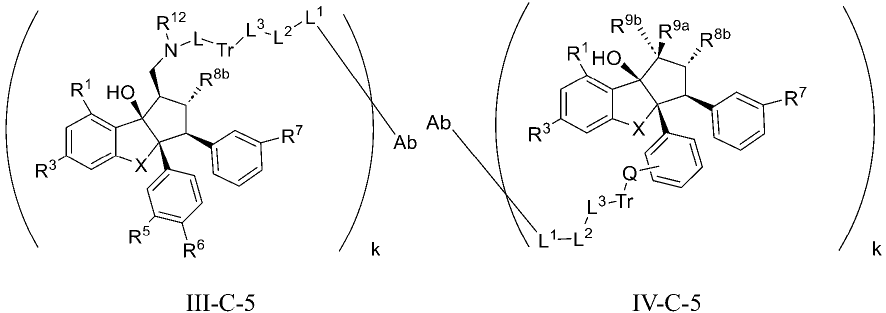 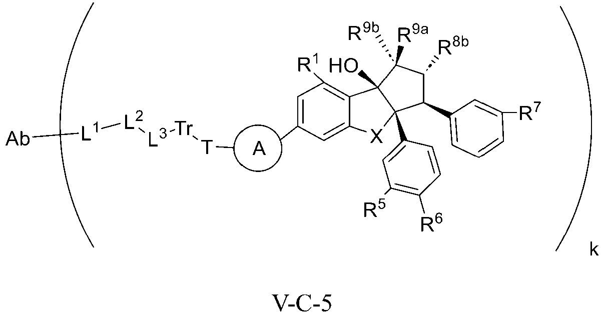
wherein X, R¹, ring A, R³, R⁵, R⁶, R⁷, R^{8b}, R^{9a}, R^{9b}, R¹², n, M, W, U, Z, Y, L, Q, T, Tr, L¹, L², L³,
Ab, and k are defined as described in any one of claims 1-32;
or compounds represented by formulas I-C-6 and I-C-7: 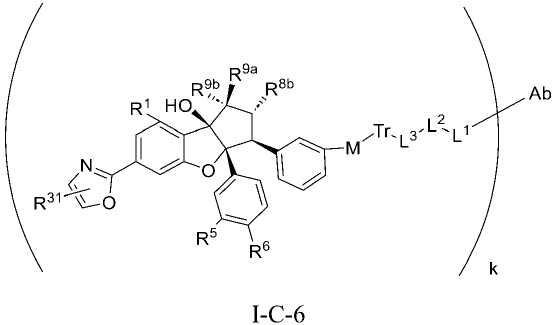 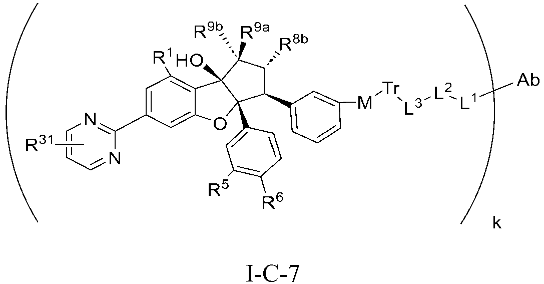
wherein R¹, R³¹, R⁵, R⁶, R^{8b}, R^{9a}, R^{9b}, M, Tr, L¹, L², L³, Ab, and k are defined as described in any one of claims 1-32.

34. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-33, wherein the ligand-drug conjugate is selected from the structures shown in Table C.

35. The ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-34, wherein the ligand-drug conjugate is selected from the structures shown in Table E.

36. A mixture of ligand-drug conjugates, comprising or consisting of the ligand-drug conjugate, the tautomer thereof, the enantiomer thereof, the diastereoisomer thereof, the pharmaceutically acceptable salt thereof, or the solvate thereof according to any one of claims 1-35, wherein the ligand-drug conjugate has one, two, or more k values.

37. A compound or a racemic mixture, tautomer, stereoisomer, enantiomer, or diastereoisomer thereof, or a mixture thereof, or a pharmaceutically acceptable salt or solvate thereof, wherein the compound is selected from a structure represented by formula I-D, II-D', III-D', IV-D, or V-D: 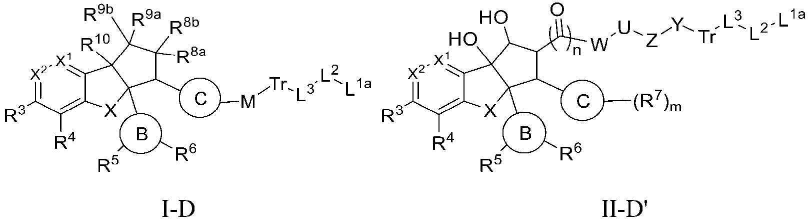 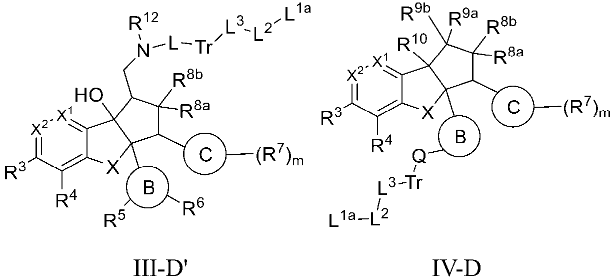 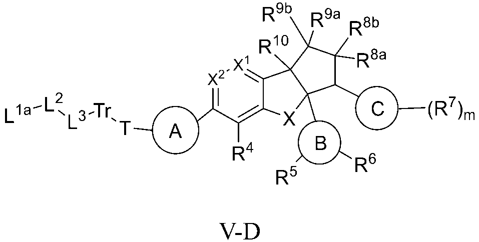 wherein:
X, X¹, X², ring A, ring B, ring C, R³, R⁴, R⁵, R⁶, R⁷, R^{8a}, R^{8b}, R^{9a}, R^{9b}, R¹⁰, R¹², m, n, M, W, U, Z, Y, L, Q, T, Tr, L², and L³ are defined as described in any one of claims 1-28;
L^{1a} is a linking unit.

38. The compound or the racemic mixture, tautomer, stereoisomer, enantiomer, or diastereoisomer thereof, or the mixture thereof, or the pharmaceutically acceptable salt or solvate thereof according to claim 37, wherein:
L^{1a} is selected from -C(=O)-(C₁-C₈)alkyl, -C(=O)O-(4-8 membered)heterocyclyl, -C(=O)O-(C₆-C₁₀)aryl, (4-8 membered)heterocyclyl, and (5-10 membered)heteroaryl, wherein the -C(=O)-(C₁-C₈)alkyl, -C(=O)O-(4-8 membered)heterocyclyl, -C(=O)O-(C₆-C₁₀ membered)aryl, (4-8 membered)heterocyclyl, and (5-10 membered)heteroaryl are optionally substituted with 1, 2, 3, 4,
or 5 oxo, halogen, =CH₂, -(C₂-C₄)alkenyl, -S-CH₂-phenyl, -S(=O)₂-(C₁-C₄)alkyl, -O-S(=O)₂-phenyl-(C₁-C₄)alkyl, and -(C₁-C₄)alkyl groups;
L^{1a} is selected from -C(=O)-(C₁-C₄)alkyl, -C(=O)O-(5-6 membered)heterocyclyl, -C(=O)O-phenyl, (5-6 membered)heterocyclyl, and (5-6 membered)heteroaryl, wherein the -C(=O)-(C₁-C₄)alkyl, -C(=O)O-(5-6 membered)heterocyclyl, -C(=O)O-phenyl, (5-6 membered)heterocyclyl,
and (5-6 membered)heteroaryl are optionally substituted with 1, 2, 3, 4, or 5 oxo, fluorine, bromine, =CH₂, vinyl, -S-CH₂-phenyl, -S(=O)₂CH₃, -O-S(=O)₂-phenyl-CH₃, and methyl groups;
preferably, L^{1a} is selected from 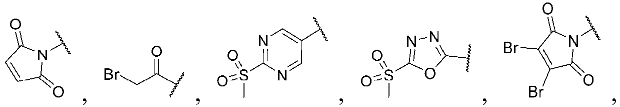 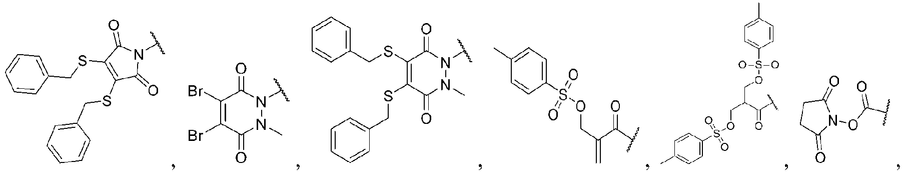 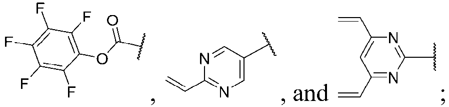
preferably, L^{1a} is selected fron

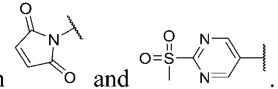

39. The compound or the racemic mixture, tautomer, stereoisomer, enantiomer, or diastereoisomer thereof, or the mixture thereof, or the pharmaceutically acceptable salt or solvate thereof according to claim 37 or 38, wherein the compound is selected from the structures shown in Table D.

40. Use of the compound or the racemic mixture, tautomer, stereoisomer, enantiomer, or diastereoisomer thereof, or the mixture thereof, or the pharmaceutically acceptable salt or solvate thereof according to any one of claims 37-39 in the manufacture of the ligand-drug conjugate according to any one of claims 1-36.

41. A pharmaceutical composition, comprising at least one of the ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-35, or at least one of the mixture of ligand-drug conjugates according to claim 36, or at least one of the compound or the racemic mixture, tautomer, stereoisomer, enantiomer, or diastereoisomer thereof, or the mixture thereof, or the pharmaceutically acceptable salt or solvate thereof according to any one of claims 37-39, and one or more pharmaceutically acceptable carriers and/or excipients.

42. Use of the ligand-drug conjugate or the racemic mixture, tautomer, stereoisomer, enantiomer, or diastereoisomer thereof, or the mixture thereof, or the pharmaceutically acceptable salt or solvate thereof according to any one of claims 1-35, or the mixture of ligand-drug conjugates according to claim 36, or the compound or the racemic mixture, tautomer, stereoisomer, enantiomer, or diastereoisomer thereof or the mixture thereof according to any one of claims 37-39, or the pharmaceutical composition according to claim 41 in the manufacture of a medicament for treating and/or preventing a disease or disorder or reducing the severity of the disease or disorder, wherein the disease or disorder is a tumor or cancer;
preferably, the tumor or cancer is selected from leukemia, malignant lymphoma, multiple myeloma, gastric cancer, colorectal cancer, pancreatic cancer, colon cancer, breast cancer, ovarian cancer, liver cancer, and lung cancer.
